# EUROPEAN PATENT SPECIFICATION

(11) **EP 3 344 275 B1**
(45) Date of publication and mention of the grant of the patent: **08.03.2023**
(21) Application number: 16843105.4
(22) Date of filing: 02.09.2016
(51) Int. Cl.: A61K 31/337, A61K 31/357, A61K 31/7068, A61K 38/12, A61K 38/15, A61K 38/21, A61K 38/50, A61K 45/06, A61P 35/00, A61K 31/436, A61K 31/437, A61K 31/44, A61K 31/475, A61K 31/506, A61K 31/519, A61K 31/553, A61K 31/565, A61K 31/573, A61K 31/664, A61K 31/706, A61K 31/555

(54) **PEPTIDOMIMETIC MACROCYCLES AND USES THEREOF**
PEPTIDOMIMETISCHE MAKROZYKLEN UND VERWENDUNGEN DAVON
MACROCYCLES PEPTIDOMIMÉTIQUES ET LEURS UTILISATIONS

(30) Priority: 03.09.2015 US 201562214142 P; 18.03.2016 US 201662310254 P; 02.06.2016 US 201662344651 P; 02.06.2016 US 201662344791 P
(43) Date of publication of application: 11.07.2018
(73) Proprietor: Aileron Therapeutics, Inc., Cambridge, MA 02139 (US)
(72) Inventor: AIVADO, Manuel, Chester Springs, Pennsylvania 19425 (US); GUERLAVAIS, Vincent, Arlington, Massachusetts 02476 (US); OLSON, Karen, Waltham, Massachusetts 02451 (US)
(74) Representative: Avidity IP
(86) International application number: PCT/US2016/050194
(87) International publication number: WO 2017/040990

(56) References cited:
- WO-A1-2012/021874
- WO-A1-2012/021875
- WO-A1-2013/123266
- WO-A1-2016/049355
- WO-A1-2016/049359
- WO-A1-2016/154058
- WO-A2-2012/021876
- CHANG, Y. S. ET AL.: 'Stapled a-helical peptide drug development: A potent dual inhibitor of MDM2 and MDMX for p53-dependent cancer therapy' PROCEEDINGS OF THE NATIONAL ACADEMY OF SCIENCES vol. 110, no. 36, 2013, pages E3445 - E3454, XP055333079
- BROWN, C. J. ET AL.: 'Stapled peptides with improved potency and specificity that activate p53' ACS CHEMICAL BIOLOGY. vol. 8, no. 3, 2012, pages 506 - 512, XP055139125
- AL-LAZIKANI, B. ET AL.: 'Combinatorial drug therapy for cancer in the post- genomic era' NATURE BIOTECHNOLOGY vol. 30, no. 7, 2012, pages 679 - 692, XP055370881
- LI, F. ET AL.: 'Molecular-targeted agents combination therapy for cancer: Developments and potentials' INTERNATIONAL JOURNAL OF CANCER. vol. 134, no. 6, 01 January 2014, pages 1257 - 1269, XP055517584 DOI: 10.1002/IJC.28261

## Description

### CROSS-REFERENCE

The present invention relates to a peptidomimetic macrocycle and at least one additional pharmaceutically active agent for use in the treatment of cancer. The peptidomimetic macrocycle antagonizes the activity of p53 and/or MDM2 and/or MDMX.

### BACKGROUND OF THE INVENTION

The human transcription factor protein p53 induces cell cycle arrest and apoptosis in response to DNA damage and cellular stress, and thereby plays a critical role in protecting cells from malignant transformation. The E3 ubiquitin ligase MDM2 (also known as HDM2) negatively regulates p53 function through a direct binding interaction that neutralizes the p53 transactivation activity, leads to export from the nucleus of p53 protein, and targets p53 for degradation via the ubiquitylation-proteasomal pathway. Loss of p53 activity, either by deletion, mutation, or MDM2 overexpression, is the most common defect in human cancers. Tumors that express wild type p53 are vulnerable to pharmacologic agents that stabilize or increase the concentration of active p53. In this context, inhibition of the activities of MDM2 has emerged as a validated approach to restore p53 activity and resensitize cancer cells to apoptosis *in vitro* and *in vivo.* MDMX (MDM4) has more recently been identified as a similar negative regulator of p53, and studies have revealed significant structural homology between the p53 binding interfaces of MDM2 and MDMX. The p53-MDM2 and p53-MDMX protein-protein interactions are mediated by the same 15-residue alpha-helical transactivation domain of p53, which inserts into hydrophobic clefts on the surface of MDM2 and MDMX. Three residues within this domain of p53 (F19, W23, and L26) are essential for binding to MDM2 and MDMX. There remains a considerable need for compounds capable of binding to and modulating the activity of p53, MDM2 and/or MDMX. Described herein are p53-based peptidomimetic macrocycles that modulate an activity of p53. Also described herein are p53-based peptidomimetic macrocycles that inhibit the interactions between p53, MDM2 and/or MDMX proteins. Further, described herein are p53-based peptidomimetic macrocycles that can be used for treating diseases including but not limited to cancer and other hyperproliferative diseases. WO 2013/123266 discloses peptidomimetic macrocycles which may be used to treat cancer.

### SUMMARY OF THE INVENTION

the invention consists in the subject-matter of the claims.

Described herein is a method of treating cancer in a subject in need thereof, the method comprising administering to the subject a therapeutically effective amount of a peptidomimetic macrocycle and at least one additional pharmaceutically active agent, wherein the peptidomimetic macrocycle has a Formula: 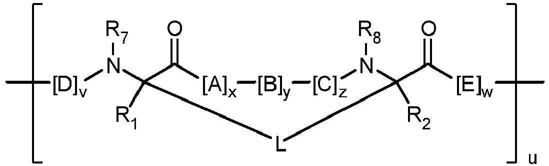 wherein:
- each A, C, D, and E is independently a natural or non-natural amino acid or an amino acid analog, and each terminal D and E independently optionally includes a capping group;
- each B is independently a natural or non-natural amino acid, an amino acid analog, 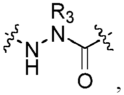 [-NH-L₃-CO-], [-NH-L₃-SO₂-], or [-NH-L₃-];
- each R₁ and R₂ is independently -H, alkyl, alkenyl, alkynyl, arylalkyl, cycloalkyl, cycloalkylalkyl, heteroalkyl, or heterocycloalkyl, unsubstituted or substituted with halo-; or at least one of R₁ and R₂ forms a macrocycle-forming linker L' connected to the alpha position of one of said D or E amino acids;
- each R₃ is independently -H, alkyl, alkenyl, alkynyl, arylalkyl, heteroalkyl, cycloalkyl, heterocycloalkyl, cycloalkylalkyl, aryl, or heteroaryl, optionally substituted with R₅;
- each L or L' is independently a macrocycle-forming linker of the formula -L₁-L₂-;
- each L₁, L₂, and L₃ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- each R₅ is independently halogen, alkyl, -OR₆, -N(R₆)₂, -SR₆, -SOR₆, -SO₂R₆, -CO₂R₆, a fluorescent moiety, a radioisotope or a therapeutic agent;
- each R₆ is independently -H, alkyl, alkenyl, alkynyl, arylalkyl, cycloalkylalkyl, heterocycloalkyl, a fluorescent moiety, a radioisotope or a therapeutic agent;
- each R₇ is independently -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
- each R₈ is independently -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
- each v and w is independently an integer from 1-1000, for example 1-500, 1-200, 1-100, 1-50, 1-30, 1-20, or 1-10;
- u is an integer from 1-10, for example 1-5, 1-3 or 1-2;
- each x, y and z is independently an integer from 0-10, for example the sum of x+y+z is 2, 3, or 6; and
- n is an integer from 1-5.

The present invention provides a peptidomimetic macrocycle or a pharmaceutically acceptable salt thereof and at least one additional pharmaceutically active agent for use in the treatment of cancer in a subject in need thereof, wherein the peptidomimetic macrocycle or the pharmaceutically acceptable salt thereof antagonizes the activity of p53 and/or MDM2 and/or MDMX , and wherein the peptidomimetic macrocycle comprises an amino acid sequence which is at least 60% identical to an amino acid sequence in any of Table 1, Table 1a, Table 1b, and Table 1c, and wherein the peptidomimetic macrocycle has the formula: 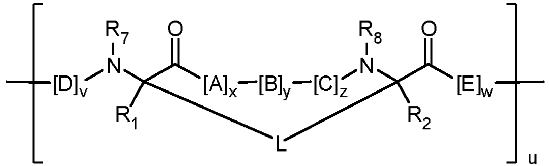 wherein each A, C, D, and E is independently an amino acid; each B is independently an amino acid, 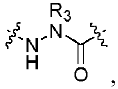 [-NH-L₃-CO-], [-NH-L₃-SO₂-], or [-NH-L₃-]; each R₁ and R₂ is independently hydrogen, alkyl, alkenyl, alkynyl, arylalkyl, cycloalkyl, cycloalkylalkyl, heteroalkyl, or heterocycloalkyl, unsubstituted or substituted with halo-; or forms a macrocycle-forming linker L' connected to the alpha position of one of said D or E amino acids; each R₃ is independently hydrogen, alkyl, alkenyl, alkynyl, arylalkyl, heteroalkyl, cycloalkyl, heterocycloalkyl, cycloalkylalkyl, aryl, or heteroaryl, optionally substituted with R₅; each L and L' is independently a macrocycle-forming linker of the formula -L₁-L₂-; each L₁, L₂, and L₃ is independently alkylene, alkenylene, alkynylene, heteroalkylene, cycloalkylene, heterocycloalkylene, arylene, heteroarylene, or [-R₄-K-R₄-]ₙ, each being optionally substituted with R₅; each R₄ is independently alkylene, alkenylene, alkynylene, heteroalkylene, cycloalkylene, heterocycloalkylene, arylene, or heteroarylene; each K is independently O, S, SO, SOz, CO, COz, or CONR₃; each R₅ is independently halogen, alkyl, -OR₆, -N(R₆)₂, - SR₆, -SOR₆, -SO₂R₆, -CO₂R₆, a fluorescent moiety, a radioisotope or a therapeutic agent; each R₆ is independently hydrogen, alkyl, alkenyl, alkynyl, arylalkyl, cycloalkylalkyl, heterocycloalkyl, a fluorescent moiety, a radioisotope or a therapeutic agent; each R₇ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue; each R₈ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue; each v is independently an integer from 1-1000; each w is independently an integer from 3-1000; u is an integer from 1-10; each x, y and z is independently an integer from 0-10; and each n is independently an integer from 1-5; wherein the at least one additional pharmaceutically active agent is fulvestrant, everolimus, romidepsin, palbociclib, trametinib, rituximab, dabrafenib, vemurafenib, azacitidine, decitabine, midostaurin, vincristine, cyclophosphamide or cytarabine.

In some cases, w>2 and each of the first two amino acid represented by E comprises an uncharged side chain or a negatively charged side chain.

In some cases, the first C-terminal amino acid and/or the second C-terminal amino acid represented by E comprise a hydrophobic side chain. For example, the first C-terminal amino acid and/or the second C-terminal amino acid represented by E comprises a hydrophobic side chain, for example a large hydrophobic side chain.

In some cases, w is between 3 and 1000. For example, the third amino acid represented by E comprises a large hydrophobic side chain.

In other cases, the peptidomimetic macrocycle excludes the sequence of:
Ac-RTQATF$r8NQWAibANle$TNAibTR-NH_{2,} Ac-RTQATF$r8NQWAibANle$TNAibTR-NH₂,
Ac-$r8SQQTFS$LWRLLAibQN-NH₂, Ac-QSQ$r8TFSNLW$LLAibQN-NH₂,
Ac-QS$r5QTFStNLW$LLAibQN-NH₂, or Ac-QSQQ$r8FSNLWR$LAibQN-NH₂.

In other cases, the peptidomimetic macrocycle excludes the sequence of:
Ac-Q$r8QQTFSN$WRLLAibQN-NH₂.

Described herein is a method of treating cancer in a subject in need thereof, the method comprising administering to the subject a therapeutically effective amount of a peptidomimetic macrocycle and at least one additional pharmaceutically active agent, wherein the peptidomimetic macrocycle has a formula: 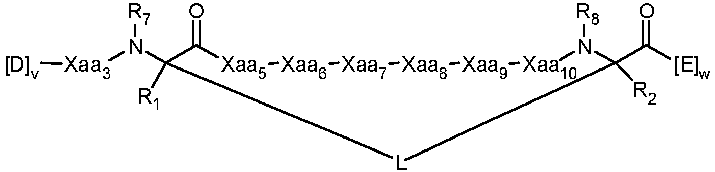
- each of Xaa₃, Xaa₅, Xaa₆, Xaa₇, Xaa₈, Xaa₉, and Xaa₁₀ is individually an amino acid, wherein at least three of Xaa₃, Xaa₅, Xaa₆, Xaa₇, Xaa₈, Xaa₉, and Xaa₁₀ are the same amino acid as the amino acid at the corresponding position of the sequence Phe₃-X₄-His₅-Tyr₆-Trp₇-Ala₈-Gln₉-Leu₁₀-X₁₁-Ser₁₂, wherein each X is an amino acid;
- each D and E is independently an amino acid;
- R₁ and R₂ are independently -H, alkyl, alkenyl, alkynyl, arylalkyl, cycloalkyl, cycloalkylalkyl, heteroalkyl, or heterocycloalkyl, unsubstituted or substituted with halo-; or at least one of R₁ and R₂ forms a macrocycle-forming linker L' connected to the alpha position of one of said D or E amino acids;
- each L and L' is independently a macrocycle-forming linker of the formula -L₁-L₂-;
- each L₁ and L₂ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- each R₅ is independently halogen, alkyl, -OR₆, -N(R₆)₂, -SR₆, -SOR₆, -SO₂R₆, -CO₂R₆, a fluorescent moiety, a radioisotope or a therapeutic agent;
- each R₆ is independently -H, alkyl, alkenyl, alkynyl, arylalkyl, cycloalkylalkyl, heterocycloalkyl, a fluorescent moiety, a radioisotope or a therapeutic agent;
- R₇ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
- R₈ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
- v is an integer from 1-1000, for example 1-500, 1-200, 1-100, 1-50, 1-30, 1-20, or 1-10;
- w is an integer from 3-1000, for example 3-500, 3-200, 3-100, 3-50, 3-30, 3-20, or 3-10; and
- n is an integer from 1-5.

Described herein is a method of treating cancer in a subject in need thereof, the method comprising administering to the subject a therapeutically effective amount of a peptidomimetic macrocycle and at least one additional pharmaceutically active agent, wherein the peptidomimetic macrocycle has a Formula: 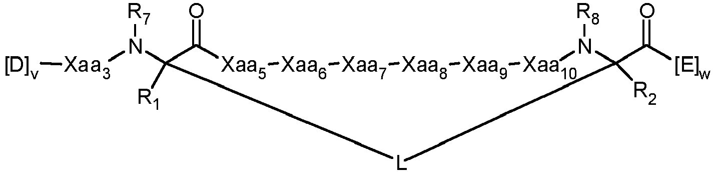 wherein:
- each of Xaa₃, Xaa₅, Xaa₆, Xaa₇, Xaa₈, Xaa₉, and Xaa₁₀ is individually an amino acid, wherein at least three of Xaa₃, Xaa₅, Xaa₆, Xaa₇, Xaa₈, Xaa₉, and Xaa₁₀ are the same amino acid as the amino acid at the corresponding position of the sequence Phe₃-X₄-Glu₅-Tyr₆-Trp₇-Ala₈-Gln₉-Leu₁₀/Cba₁₀-X₁₁-Ala₁₂, wherein each X is an amino acid;
- each D is independently an amino acid;
- each E is independently an amino acid, for example an amino acid selected from Ala (alanine), D-Ala (D-alanine), Aib (α-aminoisobutyric acid), Sar (N-methyl glycine), and Ser (serine);
- R₁ and R₂ are independently -H, alkyl, alkenyl, alkynyl, arylalkyl, cycloalkyl, cycloalkylalkyl, heteroalkyl, or heterocycloalkyl, unsubstituted or substituted with halo-; or at least one of R₁ and R₂ forms a macrocycle-forming linker L' connected to the alpha position of one of said D or E amino acids;
- each L and L' is independently a macrocycle-forming linker of the formula -L₁-L₂-;
- each L₁ and L₂ are independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- each R₅ is independently halogen, alkyl, -OR₆, -N(R₆)₂, -SR₆, -SOR₆, -SO₂R₆, -CO₂R₆, a fluorescent moiety, a radioisotope or a therapeutic agent;
- each R₆ is independently -H, alkyl, alkenyl, alkynyl, arylalkyl, cycloalkylalkyl, heterocycloalkyl, a fluorescent moiety, a radioisotope or a therapeutic agent;
- R₇ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
- R₈ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
- v is an integer from 1-1000, for example 1-500, 1-200, 1-100, 1-50, 1-30, 1-20, or 1-10;
- w is an integer from 3-1000, for example 3-500, 3-200, 3-100, 3-50, 3-30, 3-20, or 3-10; and
- n is an integer from 1-5.

In another case, the disclosure provides a method of treating cancer in a subject in need thereof, the method comprising administering to the subject a therapeutically effective amount of a peptidomimetic macrocycle and at least one additional pharmaceutically active agent, wherein the peptidomimetic macrocycle has a Formula:

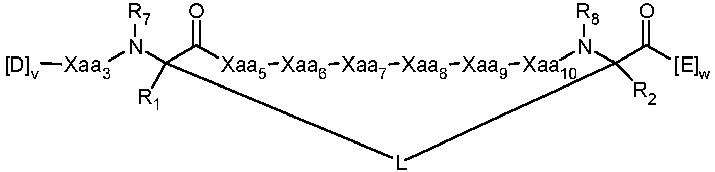

In some cases of any of the Formulas described herein, [D]ᵥ is -Leu₁-Thr₂. In other cases of the Formulas described herein, each E other than the third amino acid represented by E is an amino acid selected from Ala (alanine), D-Ala (D-alanine), Aib (α-aminoisobutyric acid), Sar (N-methyl glycine), and Ser (serine).

In some cases, w is an integer from 3-10, for example 3-6, 3-8, 6-8, or 6-10. In some cases, w is 3. In other cases, w is 6. In some cases, v is an integer from 1-10, for example 2-5. In some cases, v is 2.

In some cases, peptides disclosed herein bind a binding site defined at least in part by the MDMX amino acid side chains of L17, V46, M50, Y96 (forming the rim of the pocket) and L99. Without being bound by theory, binding to such a binding site improves one or more properties such as binding affinity, induction of apoptosis, *in vitro* or *in vivo* anti-tumor efficacy, or reduced ratio of binding affinities to MDMX versus MDM2.

In some cases, the peptidomimetic macrocycle has improved binding affinity to MDM2 or MDMX relative to a corresponding peptidomimetic macrocycle wherein w is 0, 1 or 2. In other instances, the peptidomimetic macrocycle has a reduced ratio of binding affinities to MDMX versus MDM2 relative to a corresponding peptidomimetic macrocycle wherein w is 0, 1 or 2. In still other instances, the peptidomimetic macrocycle has improved *in vitro* anti-tumor efficacy against p53 positive tumor cell lines relative to a corresponding peptidomimetic macrocycle wherein w is 0, 1 or 2. In some cases, the peptidomimetic macrocycle shows improved *in vitro* induction of apoptosis in p53 positive tumor cell lines relative to a corresponding peptidomimetic macrocycle wherein w is 0, 1 or 2. In other instances, the peptidomimetic macrocycle of claim 1, wherein the peptidomimetic macrocycle has an improved *in vitro* anti-tumor efficacy ratio for p53 positive versus p53 negative or mutant tumor cell lines relative to a corresponding peptidomimetic macrocycle wherein w is 0, 1 or 2. In some instances the improved efficacy ratio *in vitro,* is 1-29, ≥30-49, or ≥50. In still other instances, the peptidomimetic macrocycle has improved *in vivo* anti-tumor efficacy against p53 positive tumors relative to a corresponding peptidomimetic macrocycle wherein w is 0, 1 or 2. In some instances the improved efficacy ratio *in vivo* is -29, ≥30-49, or ≥50. In yet other instances, the peptidomimetic macrocycle has improved *in vivo* induction of apoptosis in p53 positive tumors relative to a corresponding peptidomimetic macrocycle wherein w is 0, 1 or 2. In some cases, the peptidomimetic macrocycle has improved cell permeability relative to a corresponding peptidomimetic macrocycle wherein w is 0, 1 or 2. In other cases, the peptidomimetic macrocycle has improved solubility relative to a corresponding peptidomimetic macrocycle wherein w is 0, 1 or 2. Exemplary cell lines include of MCF-7, HCT-116, MV4-11, DOHH2, MEL-HO, MEL-JUSO, SK-MEL-5, HT1080, MES-SA, SR, MDA-MB-134-VI, ZR-75-1, A427, A549, MOLM-13, SJSA-1, U2OS, RKO, A498, Caki-2, 22RV1, MSTO-211H, C3A, AGS, SNU-1, RMG-1, HEC-151, HEC-265, MOLT-3 and A375 cell lines.

In some cases, Xaas is Glu or an amino acid analog thereof. In some cases, Xaas is Glu or an amino acid analog thereof and wherein the peptidomimetic macrocycle has an improved property, such as improved binding affinity, improved solubility, improved cellular efficacy, improved cell permeability, improved *in vivo* or *in vitro* anti-tumor efficacy, or improved induction of apoptosis relative to a corresponding peptidomimetic macrocycle wherein Xaas is Ala.

In some cases, the peptidomimetic macrocycle has improved binding affinity to MDM2 or MDMX relative to a corresponding peptidomimetic macrocycle wherein Xaas is Ala. In other cases, the peptidomimetic macrocycle has a reduced ratio of binding affinities to MDMX vs MDM2 relative to a corresponding peptidomimetic macrocycle wherein Xaas is Ala. In some cases, the peptidomimetic macrocycle has improved solubility relative to a corresponding peptidomimetic macrocycle wherein Xaas is Ala, or the peptidomimetic macrocycle has improved cellular efficacy relative to a corresponding peptidomimetic macrocycle wherein Xaas is Ala.

In some cases, Xaas is Glu or an amino acid analog thereof and wherein the peptidomimetic macrocycle has improved biological activity, such as improved binding affinity, improved solubility, improved cellular efficacy, improved helicity, improved cell permeability, improved *in vivo* or *in vitro* anti-tumor efficacy, or improved induction of apoptosis relative to a corresponding peptidomimetic macrocycle wherein Xaas is Ala.

In some cases, the peptidomimetic macrocycle has an activity against a p53+/+ cell line which is at least 2-fold, 3-fold, 5-fold, 10-fold, 20-fold, 30-fold, 50-fold, 70-fold, or 100-fold greater than its binding affinity against a p53-/- cell line. In some cases, the peptidomimetic macrocycle has an activity against a p53+/+ cell line which is between 1 and 29-fold, between 30 and 49-fold, or ≥50-fold greater than its binding affinity against a p53-/- cell line. Activity can be measured, for example, as an IC50 value. For example, the p53+/+ cell line is SJSA-1, RKO, HCT-116, or MCF-7 and the p53-/- cell line is RKO-E6 or SW-480. In some cases, the peptide has an IC50 against the p53+/+ cell line of less than 1 µM.

In some cases, Xaas is Glu or an amino acid analog thereof and the peptidomimetic macrocycle has an activity against a p53+/+ cell line which is at least 10-fold greater than its binding affinity against a p53-/- cell line.

Also described herein is a method of modulating the activity of p53 and/or MDM2 and/or MDMX in a subject in need thereof comprising administering to the subject a therapeutically-effective amount of a peptidomimetic macrocycle and at least one additional pharmaceutically active agent, wherein the peptidomimetic macrocycle comprises an amino acid sequence which is at least about 60% identical to an amino acid sequence in any of Table 1, Table 1a, Table 1b, and Table 1c, wherein the peptidomimetic macrocycle has the formula: 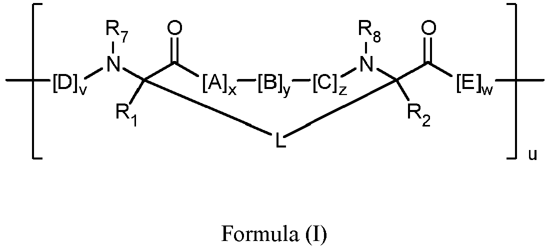 or pharmaceutically acceptable salt thereof, wherein:
- each A, C, D, and E is independently an amino acid;
- each B is independently an amino acid, 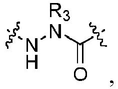 [-NH-L₃-CO-], [-NH-L₃-SO₂-], or [-NH-L₃-];
- each R₁ and R₂ is independently hydrogen, alkyl, alkenyl, alkynyl, arylalkyl, cycloalkyl, cycloalkylalkyl, heteroalkyl, or heterocycloalkyl, unsubstituted or substituted with halo-; or forms a macrocycle-forming linker L' connected to the alpha position of one of said D or E amino acids;
- each R₃ is independently hydrogen, alkyl, alkenyl, alkynyl, arylalkyl, heteroalkyl, cycloalkyl, heterocycloalkyl, cycloalkylalkyl, aryl, or heteroaryl, optionally substituted with R₅;
- each L and L' is independently a macrocycle-forming linker of the formula -L₁-L₂-;
- each L₁ and L₂ and L₃ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- each R₅ is independently halogen, alkyl, -OR₆, -N(R₆)₂, -SR₆, -SOR₆, -SO₂R₆, -CO₂R₆, a fluorescent moiety, a radioisotope or a therapeutic agent;
- each R₆ is independently hydrogen, alkyl, alkenyl, alkynyl, arylalkyl, cycloalkylalkyl, heterocycloalkyl, a fluorescent moiety, a radioisotope or a therapeutic agent;
- each R₇ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
- each R₈ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
- each v is independently an integer from 1-1000;
- each w is independently an integer from 1-1000;
- u is an integer from 1-10;
- each x, y and z is independently an integer from 0-10; and
- each n is independently an integer from 1-5.

Also described herein is a method of antagonizing an interaction between p53 and MDM2 proteins and/or between p53 and MDMX proteins in a subject in need thereof comprising administering to the subject a therapeutically-effective amount of a peptidomimetic macrocycle and at least one additional pharmaceutically active agent, wherein the peptidomimetic macrocycle comprises an amino acid sequence which is at least about 60% identical to an amino acid sequence in any of Table 1, Table 1a, Table 1b, and Table 1c and wherein the peptidomimetic macrocycle has the formula: 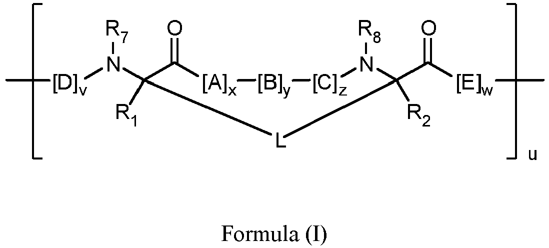 or pharmaceutically acceptable salt thereof,
wherein:
- each A, C, D, and E is independently a natural or non-natural amino acid or an amino acid analog, and each terminal D and E independently optionally includes a capping group;
- each B is independently a natural or non-natural amino acid, an amino acid analog, 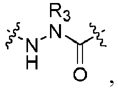 [-NH-L₃-CO-], [-NH-L₃-SO₂-], or [-NH-L₃-];
- each R₁ and R₂ is independently -H, alkyl, alkenyl, alkynyl, arylalkyl, cycloalkyl, cycloalkylalkyl, heteroalkyl, or heterocycloalkyl, unsubstituted or substituted with halo-; or forms a macrocycle-forming linker L' connected to the alpha position of one of said D or E amino acids;
- each R₃ is independently hydrogen, alkyl, alkenyl, alkynyl, arylalkyl, heteroalkyl, cycloalkyl, heterocycloalkyl, cycloalkylalkyl, aryl, or heteroaryl, optionally substituted with R₅;
- each L and L' is independently a macrocycle-forming linker of the formula -L₁-L₂-;
- each L₁, L₂, and L₃ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- each R₅ is independently halogen, alkyl, -OR₆, -N(R₆)₂, -SR₆, -SOR₆, -SO₂R₆, -CO₂R₆, a fluorescent moiety, a radioisotope or a therapeutic agent;
- each R₆ is independently hydrogen, alkyl, alkenyl, alkynyl, arylalkyl, cycloalkylalkyl, heterocycloalkyl, a fluorescent moiety, a radioisotope or a therapeutic agent;
- each R₇ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
- each R₈ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
- each v is independently an integer from 1-1000;
- each w is independently an integer from 1-1000;
- u is an integer from 1-10;
- each x, y, and z is independently an integer from 0-10; and
- each n is independently an integer from 1-5.

In some cases, the cancer is selected from the group consisting of head and neck cancer, melanoma, lung cancer, breast cancer, colon cancer, ovarian cancer, NSCLC, stomach cancer, prostate cancer, leukemia, lymphoma, mesothelioma, renal cancer, non-Hodgkin lymphoma (NHL), and glioma.

In some cases, the at least one additional pharmaceutically active agent is a nucleoside metabolic inhibitor, a microtubule inhibitor, a platinum-based drug, a hypomethylating agent, a protein kinase inhibitor, a bruton's tyrosine kinase inhibitor, a CDK4 and/or CDK6 inhibitor, a B-raf inhibitor, a K-ras inhibitor, a MEK-1 and/or MEK-2 inhibitor, an estrogen receptor antagonist, an HDAC inhibitor, an anti-CD20 monoclonal antibody, an anti-PD-1 monoclonal antibody, a hormonal antagonist, an agent the alleviates CDK2NA deletion, an agent that alleviates CDK9 abnormality, an AMT regulator, an agent that alleviates AKT activation, an agent that alleviates PTEN deletion, an agent that alleviates Wip-1Alpha overexpression, an agent that upregulates BIM, or an aromatase inhibitor.

In some cases, the at least one additional pharmaceutically active agent binds to or modulates B-raf. In some cases, the at least one additional pharmaceutically active agent is a B-raf inhibitor. In some cases, the B-raf inhibitor is vemurafenib, dabrafenib, trametinib, sorafenib, C-1, or NVP-LGX818. In some cases, the B-raf inhibitor is vemurafenib or dabrafenib and the cancer is melanoma.

In some cases, the at least one additional pharmaceutically active agent is a nucleoside metabolic regulator or modulator. In some cases, the at least one additional pharmaceutically active agent is a nucleoside metabolic inhibitor. In some cases, the nucleoside metabolic inhibitor is capecitabine, gemcitabine or cytarabine. In some cases, the nucleoside metabolic inhibitor is capecitabine and the cancer is colon or breast cancer. In some cases, the nucleoside metabolic inhibitor is gemcitabine and the cancer is ovarian, NSCLC, or breast cancer. In some cases, the nucleoside metabolic inhibitor is cytarabine and the cancer is Leukemia or Lymphoma.

In some cases, the at least one additional pharmaceutically active agent is an estrogen receptor antagonist. In some cases, the estrogen receptor antagonist is fulvestrant. In some cases, the cancer is breast cancer. In some cases, the cancer is an estrogen receptor positive breast cancer. In some cases, the cancer is a Her2 negative positive breast cancer.

In some cases, the at least one additional pharmaceutically active agent is a microtubule regulator or modulator. In some cases, the at least one additional pharmaceutically active agent is a microtubule inhibitor. In some cases, the microtubule inhibitor is paclitaxel, abraxane or docetaxel. In some cases, the microtubule inhibitor is paclitaxel and the cancer is ovarian cancer. In some cases, the microtubule inhibitor is abraxane and the cancer is ovarian cancer. In some cases, the microtubule inhibitor is docetaxel and the cancer is NSCLC, breast cancer, prostate cancer or stomach cancer.

In some cases, the at least one additional pharmaceutically active agent is a platinum-based drug. In some cases, the platinum-based drug is carboplatin or cisplatin. In some cases, the platinum-based drug is carboplatin and the cancer is NSCLC or ovarian cancer. In some cases, the platinum-based drug is cisplatin and the cancer is NSCLC, mesothelioma or ovarian cancer.

In some cases, the at least one additional pharmaceutically active agent is a hypomethylating agent. In some cases, the hypomethylating agent is azacitidine or dacogen. In some cases, the hypomethylating agent is azacitidine or dacogen and the cancer is myelodysplastic syndrome.

In some cases, the at least one additional pharmaceutically active agent binds to or modulates a protein kinase. In some cases, the additional pharmaceutically active is a protein kinase inhibitor. In some cases, the protein kinase inhibitor is sorafenib, midostaurin (PKC412), or quizartinib. In some cases, the protein kinase inhibitor is sorafenib and the cancer is kidney or liver cancer.

In some cases, the at least one additional pharmaceutically active agent binds to or modulates a bruton's tyrosine kinase. In some cases, the at least one additional pharmaceutically active agent is a bruton's tyrosine kinase inhibitor. In some cases, the bruton's tyrosine kinase inhibitor is ibrutinib. In some cases, the bruton's tyrosine kinase inhibitor is ibrutinib and the cancer is non-Hodgkin lymphoma (NHL). In some cases, the bruton's tyrosine kinase inhibitor is ibrutinib and the cancer is non-Hodgkin lymphoma (NHL).

In some cases, the at least one additional pharmaceutically active agent binds to or modulates CDK4 and/or CDK6. In some cases, the at least one additional pharmaceutically active agent is a CDK4 and/or CDK6 inhibitor. In some cases, the CDK4 and/or CDK6 inhibitor is palbociclib. In some cases, the CDK4 and/or CDK6 inhibitor is palbociclib and the cancer is breast cancer In some cases, the cancer is breast cancer. In some cases, the cancer is an estrogen receptor positive breast cancer. In some cases, the cancer is a Her2 negative positive breast cancer.

In some cases, the at least one additional pharmaceutically active agent binds to or modulates MEK-1 and/or MEK-2. In some cases, the at least one additional pharmaceutically active agent is a MEK-1 and/or MEK-2 inhibitor. In some cases, the MEK-1 and/or MEK-2 inhibitor is trametinib, pimasertib, or PD0325901. In some cases, the MEK-1 and/or MEK-2 inhibitor is trametinib and the cancer is melanoma. In some cases, the MEK-1 and/or MEK-2 inhibitor is pimasertib. In some cases, the MEK-1 and/or MEK-2 inhibitor is pimasertib and the cancer is NSCLC. In some cases, the MEK-1 and/or MEK-2 inhibitor is PD0325901.

In some cases, the at least one additional pharmaceutically active agent is an anti-CD20 monoclonal antibody. In some cases, the anti-CD20 monoclonal antibody is rituximab or obinutuzumab. In some cases, the cancer is NHL or a B-cell lymphoma.

In some cases, the at least one additional pharmaceutically active agent is an anti-PD-1 monoclonal antibody. In some cases, the anti-PD-1 monoclonal antibody is pembrolizumab or nivolumab. In some cases, the anti-PD-1 monoclonal antibody is pembrolizumab or nivolumab and the cancer is melanoma or NSCLC.

In some cases, the at least one additional pharmaceutically active agent is an aromatase inhibitor. In some cases, the aromatase inhibitor is letrozole or exemestane. In some cases, the aromatase inhibitor is letrozole or exemestane and the cancer is breast cancer.

In some cases, the at least one additional pharmaceutically active agent binds to or modulates topoisomerase I or II. In some cases, the at least one additional pharmaceutically active agent is an inhibitor of topoisomerase I or II. In some cases, the at least one additional pharmaceutically active agent is topotecan, rinotecan, idarubicin, teniposide or epirubicin. In some cases, the at least one additional pharmaceutically active agent is topotecan, rinotecan or epirubicin.

In some cases, the at least one additional pharmaceutically active agent binds to or modulates BCR-ABL kinase or BCR-ABL and Src family tyrosine kinase. In some cases, the at least one additional pharmaceutically active agent is an inhibitor of BCR-ABL kinase or BCR-ABL and Src family tyrosine kinase. In some cases, the at least one additional pharmaceutically active agent is nilotinib, bosutinib, dasatinib or imatinib.

In some cases, the at least one additional pharmaceutically active agent binds to or modulates PI3K. In some cases, the at least one additional pharmaceutically active agent is a PI3K inhibitor. In some cases, the at least one additional pharmaceutically active agent is GDC-0941 or AMG511.

In some cases, the at least one additional pharmaceutically active agent is a hormone antagonist. In some cases, the at least one additional pharmaceutically active agent is letrozole or casodex. In some cases, the at least one additional pharmaceutically active agent is fluoroucil. In some cases, the at least one additional pharmaceutically active agent is a purine analog.

In some cases, the at least one additional pharmaceutically active agent binds to or modulates mTOR. In some cases, the at least one additional pharmaceutically active agent is an mTOR inhibitor. In some cases, the at least one additional pharmaceutically active agent is AD8005. In some cases, the at least one additional pharmaceutically active agent is everolimus. In some cases, the cancer is breast cancer. In some cases, the cancer is an estrogen receptor positive breast cancer. In some cases, the cancer is a Her2 negative positive breast cancer. In some cases, the at least one additional pharmaceutically active agent binds to or modulates both PI3K/mTOR kinase. In some cases, the at least one additional pharmaceutically active agent is a dual PI3K/mTOR kinase inhibitor. In some cases, the at least one additional pharmaceutically active agent is BEZ235.

In some cases, the at least one additional pharmaceutically active agent binds to or modulates both BCL-2 and/or BCL-XL. In some cases, the at least one additional pharmaceutically active agent is BCL-2 and/or BCL-XL inhibitor. In some cases, the at least one additional pharmaceutically active agent is venetoclax (ABT-199) or ABT-263. In some cases, the at least one additional pharmaceutically active agent is a purine analog. In some cases, the at least one additional pharmaceutically active agent is fludarabine.

In some cases, the at least one additional pharmaceutically active agent binds to or modulates wild type or mutant K-ras.

In some cases, the at least one additional pharmaceutically active agent is radiation.

In some cases, the at least one additional pharmaceutically active agent is a multi-targeted tyrosine kinase modulator or binder. In some cases, the at least one additional pharmaceutically active agent is multi-targeted tyrosine kinase inhibitor. In some cases, the at least one additional pharmaceutically active agent is ponatinib.

In some cases, the at least one additional pharmaceutically active agent is a pan-histone deacetylase (HDAC) modulator or binder. In some cases, the at least one additional pharmaceutically active agent is a pan-histone deacetylase (HDAC) inhibitor. In some cases, the at least one additional pharmaceutically active agent is romidepsin. In some cases, the at least one additional pharmaceutically active agent is panobinostat. In some cases, the cancer is adult T cell leukemiallymphoma (ATL), cutaneous T-cell lymphoma (CTCL), or periphieral T-cell lymphoma (PTCL).

In some cases, the at least one additional pharmaceutically active agent binds to or modulates AKT kinase. In some cases, the at least one additional pharmaceutically active agent is an AKT kinase inhibitor. In some cases, the at least one additional pharmaceutically active agent is a MK-2206.

In some cases, the at least one additional pharmaceutically active agent alleviates CDKN2A (cyclin-dependent kinase inhibitor 2A) deletion. In some cases, the at least one additional pharmaceutically active agent alleviates CDK9 (cyclin-dependent kinase 9) abnormality. In some cases, the at least one additional pharmaceutically active agent alleviates ATM deficiency. In some cases, the at least one additional pharmaceutically active agent alleviates AKT activation. In some cases, the at least one additional pharmaceutically active agent alleviates PTEN deletion. In some cases, the at least one additional pharmaceutically active agent alleviates Wip-1Alpha over expression.

In some cases, the at least one additional pharmaceutically active agent upregulates BIM or is a BIM mimetic. In some cases, the at least one additional pharmaceutically active agent is pegylated IFN2a, vinblastine, dexamethasone, or asparaginase. In some cases, the at least one additional pharmaceutically active agent is dexamethasone. In some cases, the cancer is a B-cell lymphoma.

In some cases, the peptidomimetic macrocycle and the additional pharmaceutically active agent are present in a single formulation. In some cases, the peptidomimetic macrocycle and the additional pharmaceutically active agent are present in two different formulations. In some cases, the two different formulations are administered simultaneously. In some cases, the two different formulations are administered sequentially. In some cases, a sub-therapeutic amount of the additional therapeutic agent is administered. In some cases, a therapeutically effective amount of the additional therapeutic agent is administered.

In some cases, the subject comprises cancer cells that overexpress PD-L1. In some cases, the subject comprises cancer cells that overexpress PD-1. In some cases, the subject comprises cancer cells that overexpress miR-34. In some cases, the at least one additional pharmaceutically active agent is a PD-1 antagonist. In some cases, the at least one additional pharmaceutically active agent is a PD-L1 antagonist. In some cases, the at least one additional pharmaceutically active agent is an agent that blocks the binding of PD-L1 to PD-1. In some cases, the at least one additional pharmaceutically active agent specifically binds to PD-1. In some cases, the at least one additional pharmaceutically active agent specifically binds to PD-L1. In some cases, PD-L1 expression is downregulated. In some cases, PD-1 expression is downregulated.

In some cases, the at least one additional pharmaceutically active agent is selected from the group consisting of venetoclax (ABT-199), clofarabine, cyclophosphamide, cytarabine, doxorubicin, imatinib mesylate, methotrexate, prednisone, vincristine, azacitadine, cyclophosphamide, cytarabine, dabrafenib, decitabine, doxorubicin, etoposide, vincristine, doxorubicin, methotrexate, capecitabine, cyclophosphamide, docetaxel, doxorubicin, eribulin mesylate, everolimus, exemestane, fluorouracil, fluorouracil, fulvestrant, gemcitabine, goserelin acetate, letrozole, megestrol acetate, methotrexate, paclitaxel, palbociclib, pertuzumab, tamoxifen citrate, trastuzumab, capecitabine, cetuximab, fluorouracil, irinotecan, ramucirumab, carboplatin, cisplatin, doxorubicin, megestrol acetate, paclitaxel, docetaxel, doxorubicin, fluorouracil, ramucirumab, trastuzumab, axitinib, everolimus, pazopanib, sorafenib tosylate, sorafenib tosylate, dacarbazine, paclitaxel, trametinib, vemurafenib, cisplatin, pemetrexed, bendamustine, bortezomib, brentuximab vedotin, chlorambucil, cyclophosphamide, dexamethasone, doxorubicin, ibrutinib, lenalidomide, methotrexate, prednisone, rituximab, vincristine, afatinib dimaleate, carboplatin, cisplatin, crizotinib, docetaxel, erlotinib, gemcitabine, methotrexate, paclitaxel, pemetrexed, ramucirumab, carboplatin, cisplatin, cyclophosphamide, gemcitabine, olaparib, paclitaxel, topotecan, abiraterone, cabazitaxel, docetaxel, enzalutamide, goserelin acetate, prednisone, doxorubicin, imatinib mesylate, romidepsin, obinutuzumab, pazopanib, and combinations thereof.

In some cases, the at least one additional pharmaceutically active agent inhibits S-phase. In some cases, the at least one additional pharmaceutically active agent inhibits M-phase.

In some cases, the peptidomimetic macrocycle antagonizes an interaction between p53 and MDM2 proteins. In some cases, the peptidomimetic macrocycle antagonizes an interaction between p53 and MDMX proteins. In some cases, the peptidomimetic macrocycle antagonizes an interaction between p53 and MDM2 proteins and p53 and MDMX proteins. In some cases, the peptidomimetic macrocycle antagonizes an interaction between p53 and MDM2 proteins and p53 and MDMX proteins.

In one aspect, described herein is a method of selecting a peptidomimetic macrocycle that reduces PD-L1 expression, comprising: contacting a cancer cell line expressing a first level of PD-L1 with a peptidomimetic macrocycle comprising a polypeptide with a crosslinker connecting a first amino acid and a second amino acid; incubating the cancer cell line for an incubation period; measuring a second level of PD-L1 expression after the incubation period; selecting the peptidomimetic macrocycle as a peptidomimetic macrocycle that reduces PD-L1 expression when the second level of PD-L1 expression is at least 1.1, 1.5, 2, 3, 4, 5, 6, 7, 8, 9, 10, 25, 50, or 100 fold lower than the first level of PD-L1 expression.

In some cases, the measuring comprises flow cytometry. In some cases, the cancer cell line is selected from the group consisting of MCF-7, HCT-116, MV4-11, DOHH2, and A375. In some cases, the method further comprises measuring a level of p53 expression before (a), after (b), or both. In some cases, the method further comprises measuring a level of p21 expression before (a), after (b), or both. In some cases, the method further comprises measuring a level of miR-34 expression before (a), after (b), or both. In some cases, the miR-34 is miR-34a, miR-34b, miR-34c, or a combination thereof. In some cases, the first level of PD-L1 expression in the cancer cell line is high. In some cases, the first level of PD-L1 expression in the cancer cell line is low. In some cases, the cancer cell line is p53 wild-type. In some cases, the incubation period is about 24, 48, or 72 hours after the contacting. In some cases, the incubation period is at least 6, 12, 24, 36, 48, 60, or 72 hours after the contacting. In some cases, the method further comprises measuring a level apoptosis after the incubation period.

### BRIEF DESCRIPTION OF THE DRAWINGS

The inventionis set forth in the claims. A better understanding of the features and advantages of the present invention will be obtained by reference to the following detailed description that sets forth illustrative cases, in which the principles of the invention are utilized, and the accompanying drawings of which:
**Figure 1A** depicts western blots demonstrating Aileron peptide 1 activates the p53-pathway in AML cell lines treated with increasing amounts of Aileron peptide 1.
**Figure 1B** depicts a western blot demonstrating Aileron peptide 1 activates the p53-pathway in AML cell lines treated with the indicated amounts of Aileron peptide 1.
**Figure 1C** depicts a western blot demonstrating Aileron peptide 1 activates the p53-pathway in primary AML cells lines treated with the indicated amounts of Aileron peptide 1.
**Figure 2** depicts graphs of relative mRNA expression normalized to GAPDH in AML cell lines treated with increasing amounts of Aileron peptide 1.
**Figure 3A** depicts a western blot demonstrating that p53 is stabilized in response to Aileron peptide 1 treatment in a dose-dependent manner.
**Figure 3B** depicts a western blot demonstrating that p53 is stabilized in response to Aileron peptide 1 treatment in a time-dependent manner.
**Figure 4A** depicts a western blot of immunoprecipitations demonstrating Aileron peptide 1 is an inhibitor of the p53-MDMX interaction.
**Figure 4B** depicts a western blot of immunoprecipitations demonstrating Aileron peptide 1 is a dual inhibitor of the p53-MDM2 and p53-MDMX interaction.
**Figure 4C** depicts a western blot of immunoprecipitations demonstrating Aileron peptide 1 is an inhibitor of the p53-MDM2 interaction.
**Figure 5A** depicts a graph demonstrating inhibition of cellular proliferation of AML cell lines treated with the indicated amount of Aileron peptide 1 (AP1).
**Figure 5B** depicts a graph demonstrating inhibition of cellular proliferation of AML cell lines treated with the indicated amount of AP1.
**Figure 5C** depicts a graph demonstrating inhibition of cellular proliferation of AML cell lines treated with the indicated amount of AP1.
**Figure 5D** depicts a graph demonstrating inhibition of cellular proliferation of AML cell lines treated with the indicated amount of AP1.
**Figure 6** depicts a graph demonstrating inhibition of clonogenic capacity of AML cell lines treated with the indicated amount of AP1.
**Figure 7A** depicts a graph demonstrating inhibition of cellular proliferation of AML cell lines treated with the indicated amount of AP1.
**Figure 7B** depicts a graph demonstrating inhibition of cellular proliferation of AML cell lines treated with the indicated amount of AP1.
**Figure 8A** depicts a graph (left) and corresponding FACS data (right) demonstrating Aileron peptide 1 induces apoptotic cell death in a p53 wild type AML cell line.
**Figure 8B** depicts a graph (left) and corresponding FACS data (right) demonstrating Aileron peptide 1 induces apoptotic cell death in a p53 wild type AML cell line.
**Figure 8C** depicts a graph (left) and corresponding FACS data (right) demonstrating Aileron peptide 1 does not induce apoptotic cell death in a p53 null AML cell line.
**Figure 8D** depicts a graph (left) and corresponding FACS data (right) demonstrating Aileron peptide 1 induces apoptotic cell death in a p53 wild type AML cell line.
**Figure 8E** depicts a graph (left) and corresponding FACS data (right) demonstrating Aileron peptide 1 induces apoptotic cell death in a p53 wild type AML cell line.
**Figure 9A** depicts a graph demonstrating cytarabine (Ara-C) treatment inhibits proliferation of AML cell lines.
**Figure 9B** depicts a graph demonstrating Ara-C synergizes with AP1 to inhibit proliferation of AML cell lines.
**Figure 9C** depicts a graph demonstrating Ara-C synergizes with AP1 to inhibit proliferation of AML cell lines.
**Figure 10A** depicts a graph demonstrating inhibition of cellular proliferation of primary AML cells treated with the indicated amount of AP1.
**Figure 10B** depicts a graph demonstrating inhibition of cellular proliferation of primary AML cells treated with the indicated amount of AP1.
**Figure 10C** depicts a graph demonstrating inhibition of cellular proliferation of primary AML cells treated with the indicated amount of AP1.
**Figure 10D** depicts a graph demonstrating inhibition of cellular proliferation of primary AML cells treated with the indicated amount of AP1.
**Figure 11A** depicts a graph demonstrating inhibition of clonogenic capacity of primary AML cells treated with the indicated amount of AP1.
**Figure 11B** depicts a graph demonstrating inhibition of clonogenic capacity of primary AML cells treated with the indicated amount of AP1.
**Figure 12** depicts a graph (top) and corresponding FACS data (bottom) demonstrating AP1 induces apoptotic cell death in primary AML cells.
**Figure 13** shows a structure of peptidomimetic macrocycle 46 (Table 2b), a p53 peptidomimetic macrocycle, complexed with MDMX (Primary SwissProt accession number Q7ZUW7; Entry MDM4_DANRE).
**Figure 14** shows overlaid structures of p53 peptidomimetic macrocycles 142 (Table 2b) and SP43 bound to MDMX (Primary SwissProt accession number Q7ZUW7; Entry MDM4_DANRE).
**Figure 15** shows the effect of SP154, a peptidomimetic macrocycle, on tumor growth in a mouse MCF-7 xenograft model.
**Figure 16** shows the effect of SP249, a peptidomimetic macrocycle, on tumor growth in a mouse MCF-7 xenograft model.
**Figure 17** shows the effect of SP315, a peptidomimetic macrocycle, on tumor growth in a mouse MCF-7 xenograft model.
**Figure 18** shows the effect of SP252, a point mutation of SP154, on tumor growth in a mouse MCF-7 xenograft model.
**Figure 19** shows a plot of solubility for peptidomimetic macrocycles with varying C-terminal extensions.
**Figure 20** shows that the peptidomimetic macrocycles of the disclosure show synergy with Zelboraf (Vemurafenib, a.k.a. PLX4032) in B-Raf-mutant Melanoma Cell Line A375.
**Figure 21** shows that the peptidomimetic macrocycles of the disclosure show synergy with Zelboraf in B-Raf-mutant melanoma cell line Mel-Ho but not in B-Raf-WT Mel-Juso.
**Figure 22** shows that the peptidomimetic macrocycles of the disclosure can reduce expression levels of PD-L1 in HCT116 p53+/+ cells.
**Figure 23** shows a graph of MCF-7 cell proliferation determined using a WST-1 assay measured at the indicated time points after different numbers of MCF-7 cells were grown at 37 °C for a 24 hour growth period.
**Figure 24A** shows a bar graph of MCF-7 breast cancer cell proliferation when treated with the indicated concentrations of Aileron peptide 1. Cells were evaluated for viability by MTT assay 5 days after beginning treatment. Treatment with Aileron peptide 1 supresses MCF-7 breast cancer cell growth.
**Figure 24B** shows a bar graph of MOLT-3 cell proliferation when treated with the indicated concentrations of Aileron peptide 1. Cells were evaluated for viability by a WST-1 assay 72 hours after beginning treatment. Treatment with Aileron peptide 1 supresses MOLT-3 cell growth.
**Figure 25A** shows a graph of MCF-7 breast cancer cell proliferation when treated with the indicated amounts of Aileron petide 1 (log µM), Aileron peptide 1 + 400 nM everolimus, or Aileron peptide 1 + 10 nM fulvestrant. Cells were evaluated for viability by MTT assay 5 days after beginning treatment. Aileron peptide 1 in combination with fulvestrant and everolimus yields enhanced inhibition of cancer cell proliferation.
**Figure 25B** shows a graph of MCF-7 breast cancer cell proliferation inhibition (fraction of control) when treated with the indicated amounts of AP1 (µM), AP1+ 400 nM everolimus, or AP1+ 10 nM fulvestrant. Cells were evaluated for viability by MTT assay 5 days after beginning treatment. AP1 in combination with fulvestrant and everolimus yields enhanced inhibition of cancer cell proliferation.
**Figure 26** shows a bar graph of MCF-7 cell proliferation when treated with the indicated concentrations of fulvestrant. Cells were evaluated for viability by a WST-1 assay 5 days after beginning treatment. Fulvestrant treatment inhibited MCF-7 breast cancer cell proliferation with limited cell killing as a single agent.
**Figure 27A** shows a graph of MCF-7 breast cancer cell proliferation when treated with the indicated amounts of Aileron petide 1, Aileron peptide 1 + 3 nM fulvestrant, Aileron peptide 1 + 10 nM fulvestrant, or Aileron peptide 1 + 30 nM fulvestrant. Cells were evaluated for viability by MTT assay 5 days after beginning treatment. Combination with fulvestrant enhances Aileron peptide 1 inhibition of cancer cell proliferation and cell killing.
**Figure 27B** shows a graph of MCF-7 breast cancer cell proliferation when treated with the indicated amounts of fulvestrant, fulvestrant + 0.13 µM Aileron peptide 1, fulvestrant + 0.4 µM Aileron peptide 1, or fulvestrant + 1.2 µM Aileron peptide 1. Cells were evaluated for viability by MTT assay 5 days after beginning treatment. Combination with Aileron peptide 1 enhances fulvestrant inhibition of cancer cell proliferation and cell killing.
**Figure 28A** shows a graph of MCF-7 breast cancer cell proliferation when treated with the indicated fixed amounts of Aileron petide 1 (AP1) in combination with the indicated fixed amounts of fulvestrant (FU). Cells were evaluated for viability by MTT assay 5 days after beginning treatment. Combination with Aileron peptide 1 enhances fulvestrant inhibition of cancer cell proliferation and cell killing.
**Figure 28B** shows a graph of MCF-7 breast cancer cell proliferation when treated with 0.1 µM Aileron petide 1, 3 nM fulvestrant, or 0.1 µM Aileron petide 1 and 3 nM fulvestrant. Cells were evaluated for viability by MTT assay 5 days after beginning treatment. Combination with Aileron peptide 1 enhances fulvestrant inhibition of cancer cell proliferation and cell killing.
**Figure 29** shows a bar graph of MCF-7 cell proliferation when treated with the indicated concentrations of everolimus. Cells were evaluated for viability by a WST-1 assay 5 days after beginning treatment. Everolimus treatment inhibited MCF-7 breast cancer cell proliferation with limited cell killing as a single agent.
**Figure 30A** shows a graph of MCF-7 breast cancer cell proliferation when treated with the indicated amounts of Aileron petide 1, Aileron peptide 1 + 1 nM everolimus, Aileron peptide 1 + 3 nM everolimus, Aileron peptide 1 + 10 nM everolimus, or Aileron peptide 1 + 100 nM everolimus. Cells were evaluated for viability by MTT assay 5 days after beginning treatment. Combination with everolimus enhances Aileron peptide 1 inhibition of cancer cell proliferation and cell killing.
**Figure 30B** shows a graph of MCF-7 breast cancer cell proliferation when treated with the indicated amounts of everolimus, everolimus + 0.13 µM Aileron peptide 1, everolimus + 0.4 µM Aileron peptide 1, or everolimus + 1.2 µM Aileron peptide 1. Cells were evaluated for viability by MTT assay 5 days after beginning treatment. Combination with Aileron peptide 1 enhances everolimus inhibition of cancer cell proliferation and cell killing.
**Figure 31A** shows a graph of MCF-7 breast cancer cell proliferation when treated with the indicated fixed amounts of Aileron petide 1 (AP1) in combination with the indicated fixed amounts of everolimus (EV). Cells were evaluated for viability by MTT assay 5 days after beginning treatment. Combination with Aileron peptide 1 enhances everolimus inhibition of cancer cell proliferation and cell killing.
**Figure 31B** shows a graph of MCF-7 breast cancer cell proliferation when treated with 0.1 µM Aileron petide 1, 3 nM everolimus, or 0.1 µM Aileron petide 1 and 3 nM everolimus. Cells were evaluated for viability by MTT assay 5 days after beginning treatment. Combination with Aileron peptide 1 enhances everolimus inhibition of cancer cell proliferation and cell killing.
**Figure 32** shows a bar graph of MOLT-3 cell proliferation when treated with the indicated concentrations of romidepsin. Cells were evaluated for viability by a WST-1 assay 72 hours after beginning treatment. Romidepsin treatment inhibited MOLT-3 cell proliferation.
**Figure 33A** shows a graph of MOLT-3 cell proliferation when treated with the indicated amounts of Aileron petide 1, Aileron peptide 1 + 0.5 nM romidepsin, Aileron peptide 1 + 1.5 nM romidepsin, or Aileron peptide 1 + 3 nM romidepsin. Cells were evaluated for viability by MTT assay 72 hours after beginning treatment. Combination with romidepsin enhances Aileron peptide 1 inhibition of cancer cell proliferation and cell killing.
**Figure 33B** shows a graph of MOLT-3 cell proliferation when treated with the indicated amounts of romidepsin, romidepsin + 0.05 µM Aileron peptide 1, romidepsin + 0.2 µM Aileron peptide 1, or romidepsin + 0.8 µM Aileron peptide 1. Cells were evaluated for viability by a WST-1 assay 72 hours after beginning treatment. MOLT-3 cells were pretreated with Aileron peptide 1 for 2 hours before adding romedepsin. Combination with Aileron peptide 1 enhances romidepsin inhibition of cancer cell proliferation and cell killing.
**Figure 34A** shows a graph of MOLT-3 cell proliferation when treated with the indicated fixed amounts of Aileron petide 1 (AP1) in combination with the indicated fixed amounts of romidepsin (RO). Cells were evaluated for viability by a WST-1 assay 72 hours after beginning treatment. MOLT-3 cells were pretreated with Aileron peptide 1 for 2 hours before adding romedepsin. Aileron peptide 1 and romidepsin suppressed MOLT-3 cell growth. Combination with romidepsin enhances Aileron peptide 1 inhibition of cancer cell proliferation and cell killing.
**Figure 34B** shows a graph of MOLT-3 cell proliferation when treated with the indicated fixed amounts of Aileron petide 1 (AP1) in combination with the indicated fixed amounts of romidepsin (RO). Cells were evaluated for viability by MTT assay 72 hours after beginning treatment. MOLT-3 cells were pretreated with Aileron peptide 1 for 2 hours before adding romedepsin. Aileron peptide 1 and romidepsin suppressed MOLT-3 cell growth. Combination with romidepsin enhances Aileron peptide 1 inhibition of cancer cell proliferation and cell killing.
**Figure 34C** shows a graph of MOLT-3 cell proliferation when treated with 0.1 µM Aileron petide 1, 1.5 nM romidepsin, or 0.1 µM Aileron petide 1 and 1.5 nM romidepsin. Cells were evaluated for viability by MTT assay 72 hours after beginning treatment. MOLT-3 cells were pretreated with Aileron peptide 1 for 2 hours before adding romedepsin. Aileron peptide 1 and romidepsin suppressed MOLT-3 cell growth. Combination with romidepsin enhances Aileron peptide 1 inhibition of cancer cell proliferation and cell killing.
**Figure 35** shows a bar graph of MCF-7 cell proliferation when treated with the indicated concentrations of palbociclib. Cells were evaluated for viability by a WST-1 assay 5 days after beginning treatment. Palbociclib treatment inhibited MCF-7 breast cancer cell proliferation with limited cell killing as a single agent.

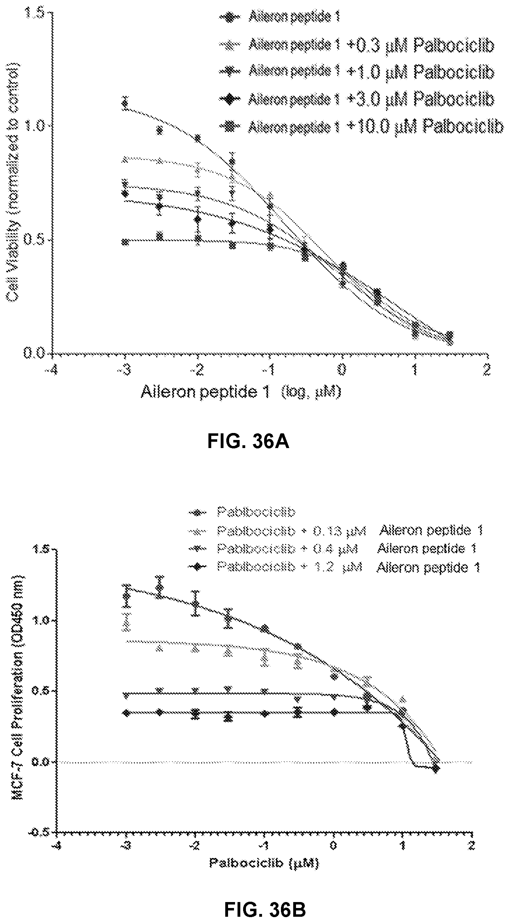

**Figure 36A** shows a graph of MCF-7 breast cancer cell viability when treated with the indicated amounts of Aileron petide 1, Aileron peptide 1 + 0.3 µM palbociclib, Aileron peptide 1 + 1 µM palbociclib, Aileron peptide 1 + 3 µM palbociclib, or Aileron peptide 1 + 10 µM palbociclib. Cells were evaluated for viability by MTT assay 5 days after beginning treatment. Palbociclib has anti-proliferative effects when dosed with Aileron peptide 1. Aileron peptide 1 and palbociclib combination studies show complementary *in vitro* anticancer activity. Combination with palbociclib enhances Aileron peptide 1 inhibition of cancer cell proliferation and cell killing.
**Figure 36B** shows a graph of MCF-7 breast cancer cell proliferation when treated with the indicated amounts of palbociclib, palbociclib + 0.13 µM Aileron peptide 1, palbociclib + 0.4 µM Aileron peptide 1, or palbociclib + 1.2 µM Aileron peptide 1. Cells were evaluated for viability by MTT assay 5 days after beginning treatment. Combination with Aileron peptide 1 enhances palbociclib inhibition of cancer cell proliferation and cell killing.
**Figure 37A** shows a graph of MCF-7 breast cancer cell proliferation when treated with the indicated fixed amounts of Aileron petide 1 (AP1) in combination with the indicated fixed amounts of palbociclib (PO). Cells were evaluated for viability by MTT assay 5 days after beginning treatment.
**Figure 37B** shows a graph of MCF-7 breast cancer cell viability when treated with 0.3 µM palbociclib or 0.3 µM Aileron petide 1 and 0.3 µM palbociclib. Cells were evaluated for viability by MTT assay 5 days after beginning treatment. Aileron peptide 1 kills cancer cells when dosed with palbociclib.
**Figure 38A** shows MV4-11 cell proliferation when treated with the indicated concentrations of Ara-C.
**Figure 38B** shows MV4-1 1 cell viability when treated with varying concentrations of AP1 and Ara-C. Combination with Ara-C enhanced AP1 inhibition of cancer cell proliferation and cell killing.
**Figure 38C** shows a combination index profile of treatment with AP1 and Ara-C.
**Figure 39A** shows MV4-11 cell proliferation when treated with the indicated concentrations of azacitidine.
**Figure 39B** shows MV4-1 1 cell viability when treated with varying concentrations of AP1 and azacitidine. Combination with azacitidine enhanced AP1 inhibition of cancer cell proliferation and cell killing.
**Figure 39C** shows a combination index profile of treatment with AP1 and azacitidine.
**Figure 40A** shows MV4-11 cell proliferation when treated with the indicated concentrations of decitabine.
**Figure 40B** shows MV4-1 1 cell viability when treated with varying concentrations of AP1 and decitabine. Combination with decitabine enhanced AP1 inhibition of cancer cell proliferation and cell killing.
**Figure 40C** shows a combination index profile of treatment with AP1 and decitabine.
**Figure 41A** shows MV4-11 cell proliferation when treated with the indicated concentrations of midostaurin.
**Figure 41B** shows MV4-1 1 cell viability when treated with varying concentrations of AP1 and midostaurin. Combination with midostaurin enhanced AP1 inhibition of cancer cell proliferation and cell killing.
**Figure 41C** shows a combination index profile of treatment with AP1 and midostaurin.
**Figure 42A** shows DOHH-2 cell proliferation when treated with the indicated concentrations of vincristine (VCR).
**Figure 42B** shows DOHH-2 cell viability when treated with varying concentrations of AP1 and VCR. Combination with vincristine enhanced AP1 inhibition of cancer cell proliferation and cell killing.
**Figure 42C** shows a combination index profile of treatment with AP1 and vincristine.
**Figure 43** shows DOHH-2 cell viability when treated with AP1 alone, vincristine alone, and AP1 in combination with vincristine.
**Figure 44A** shows DOHH-2 cell proliferation when treated with the indicated concentrations of AP1.
**Figure 44B** shows DOHH-2 cell viability when treated with varying concentrations of cyclophosphamide (CTX) and AP1. Combination with vincristine enhanced CTX inhibition of cancer cell proliferation and cell killing.
**Figure 44C** shows a combination index profile of treatment with AP1 and CTX.
**Figure 45** shows DOHH-2 cell viability when treated with AP1 alone, CTX alone, and AP1 in combination with CTX.
**Figure 46** shows the order of addition effects on DOHH-2 cell viability using various concentrations of AP1 in combination with VCR.
**Figure 47** shows DOHH-2 cell viability based on the order of addition when treated with varying concentrations of AP1 and VCR after pretreatment with AP1 for 24 hrs.
**Figure 48** shows DOHH-2 cell viability based on the order of addition when treated with varying concentrations of AP1 and VCR after pretreatment with VCR for 24 hrs.
**Figure 49** shows the order of addition effects on DOHH-2 cell viability using various concentrations of AP 1 in combination with CTX.
**Figure 50** shows DOHH-2 cell viability based on the order of addition when treated with varying concentrations of AP1 and CTX after pretreatment with AP1 for 24 hrs.
**Figure 51** shows DOHH-2 cell viability based on the order of addition when treated with varying concentrations of AP1 and CTX after pretreatment with CTX for 24 hrs.
**Figure 52** shows the order of addition effects on MV4-11 cell viability using various concentrations of AP1 and midostaurin.
**Figure 53** shows MV4-11 cell viability based on the order of addition when treated with varying concentrations of AP1 and midostaurin after pretreatment with midostaurin for 24 hrs.
**Figure 54** shows MV4-11 cell viability based on the order of addition when treated with varying concentrations of AP1 and midostaurin after pretreatment with AP 1 for 24 hrs.
**Figure 55** shows the order of addition effects on MV4-11 cell viability using various concentrations of AP1 in combination with decitabine.
**Figure 56** shows MV4-11 cell viability based on the order of addition when treated with varying concentrations of AP1 and decitabine after pretreatment with decitabine for 24 hrs.
**Figure 57** shows MV4-11 cell viability based on the order of addition when treated with varying concentrations of AP1 and decitabine after pretreatment with AP 1 for 24 hrs.
**Figure 58** shows the order of addition effects on MV4-11 cell viability using various concentrations of AP1 in combination with Ara-C.
**Figure 59** shows MV4-11 cell viability based on the order of addition when treated with varying concentrations of AP1 and Ara-C after pretreatment with AP 1 for 24 hrs.
**Figure 60** shows MV4-11 cell viability based on the order of addition when treated with varying concentrations of AP1 and Ara-C after pretreatment with Ara-C for 24 hrs.
**Figure 61** shows the order of addition effects on MV4-11 cell viability using various concentrations of AP1 in combination with azacitidine.
**Figure 62** shows MV4-11 cell viability based on the order of addition when treated with varying concentrations of AP1 and azacitidine after 24 hrs pretreatment with AP 1.
**Figure 63** shows MV4-11 cell viability based on the order of addition when treated with varying concentrations of AP1 and azacitidine after pretreatment with azacitidine for 24 hrs.
**Figure 64A** shows MCF-7 cell proliferation when treated with the indicated concentrations of fulvestrant.
**Figure 64B** shows MCF-7 cell viability when treated with varying concentrations of AP1 and fulvestrant.

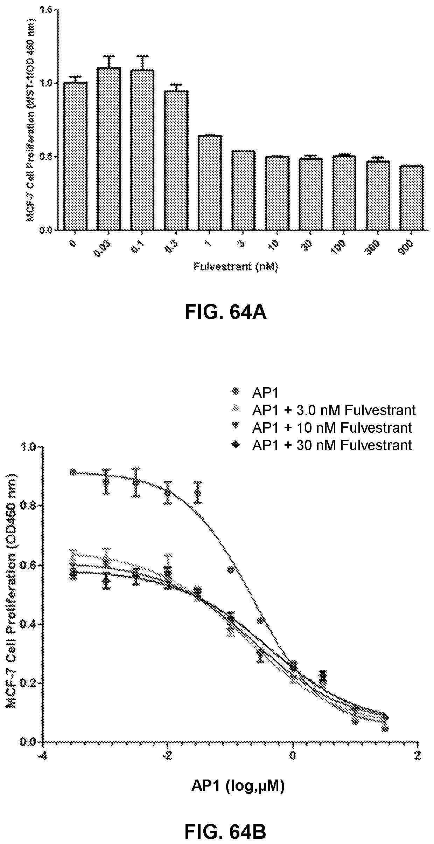

**Figure 64A** shows MCF-7 cell proliferation when treated with varying concentrations of AP1.

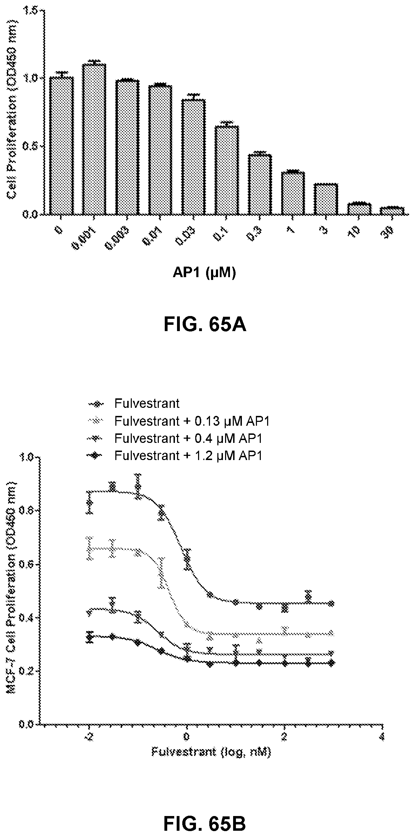

**Figure 65B** shows MCF-7 cell viability when treated with varying concentrations of AP1 and fulvestrant.
**Figure 65C** shows the IC₅₀ values of AP1 alone and AP1 with varying concentrations of fulvestrant (FUL).

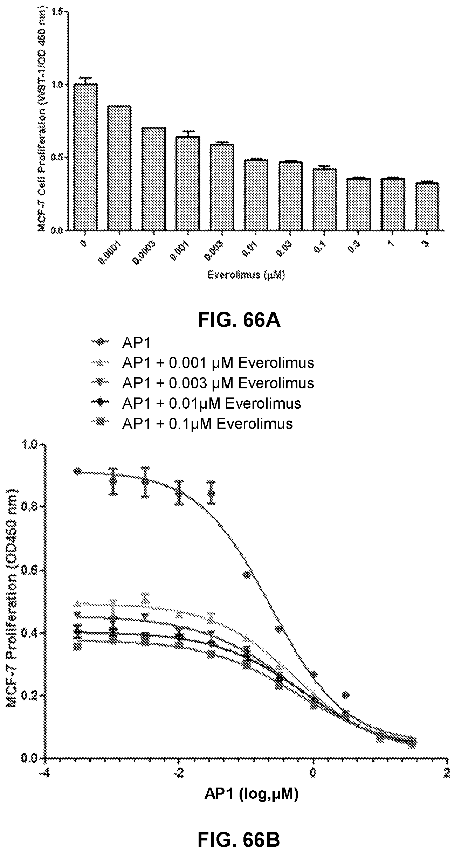

**Figure 66A** shows MCF-7 cell proliferation when treated with varying concentrations of everolimus.
**Figure 66B** shows MCF-7 cell viability when treated with varying concentrations of AP1 and everolimus.

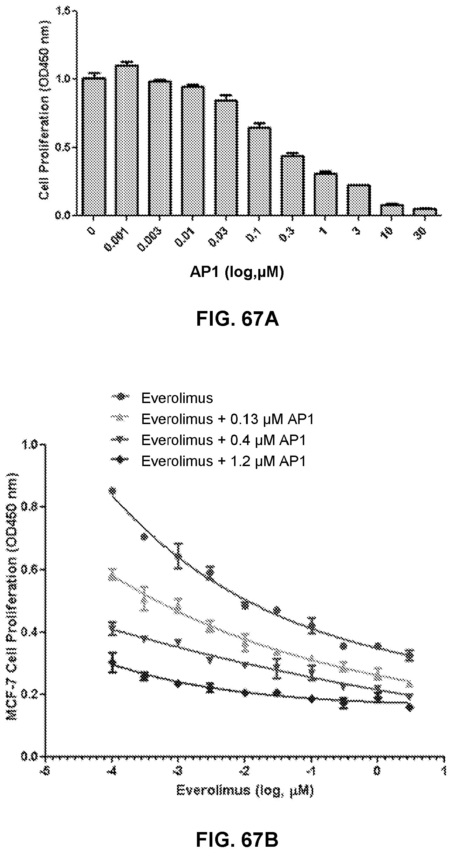

**Figure 67A** shows MCF-7 cell proliferation when treated with varying concentrations of AP1. AP1 treatment suppressed MCF-7 breast cancer cell proliferation.
**Figure 67B** shows a graph of MCF-7 cell viability when treated with varying concentrations of AP1 and everolimus.

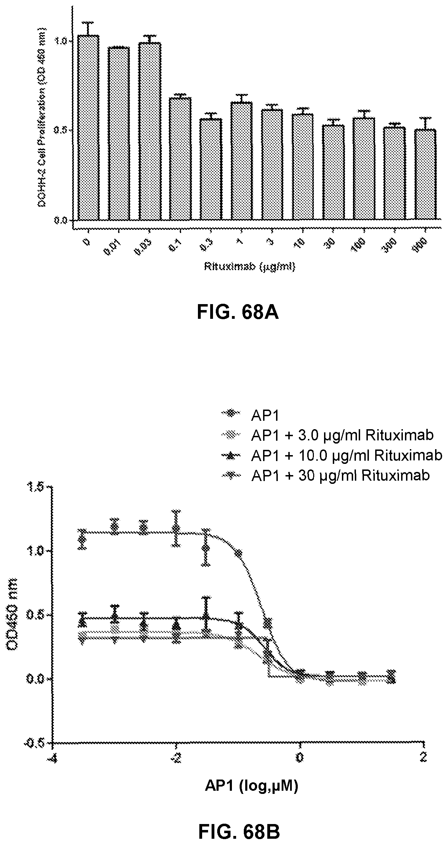

**Figure 68A** shows the effects of rituximab alone on DOHH-2 cell growth.
**Figure 68B** shows DOHH-2 cell viability when treated with varying concentrations of AP1 and rituximab.

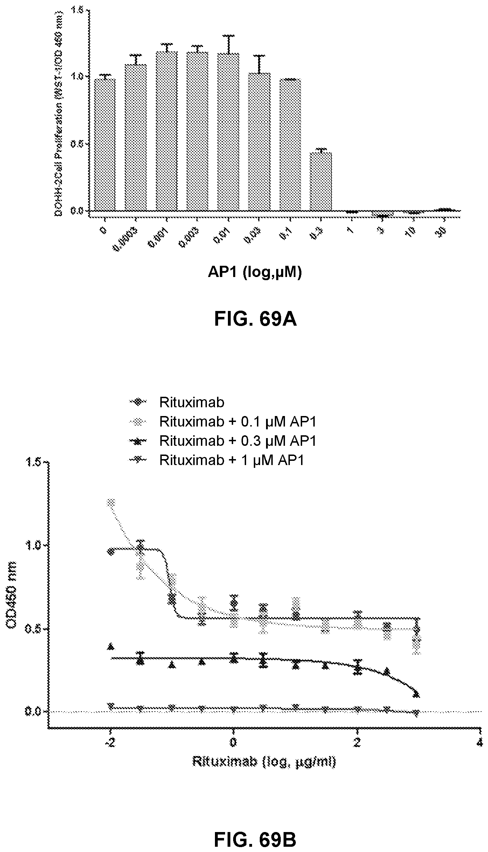

**Figure 69A** shows the effects of AP1 alone on DOHH-2 cell growth.
**Figure 69B** shows DOHH-2 cell viability when treated with varying concentrations of AP1 and rituximab.

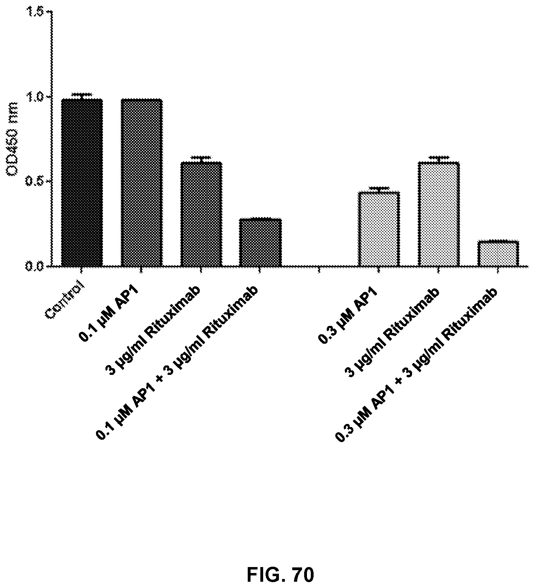

**Figure 70** shows DOHH-2 cell viability when treated with AP1 alone, rituximab alone, and varying concentrations of AP1 in combination with rituximab.

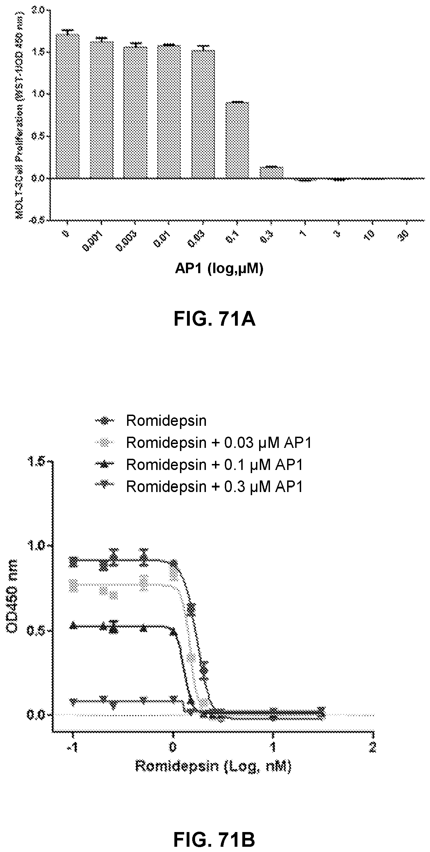

**Figure 71A** shows the effects of AP1 alone on MOLT-3 cell growth.
**Figure 71B** shows MOLT-3 cell viability when treated with varying concentrations of AP1 and romidepsin.

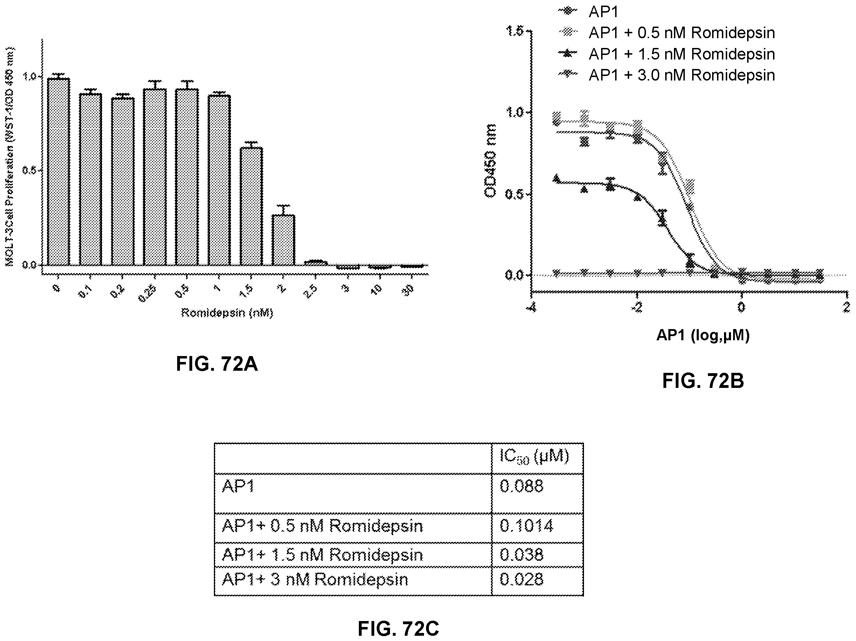

**Figure 72A** shows the effects of romidepsin alone on MOLT-3 cell growth.
**Figure 72B** shows MOLT-3 cell viability when treated with varying concentrations of AP1 and romidepsin.
**Figure 72C** shows the IC₅₀ values of AP1 alone and AP1 with varying concentrations of romidepsin.

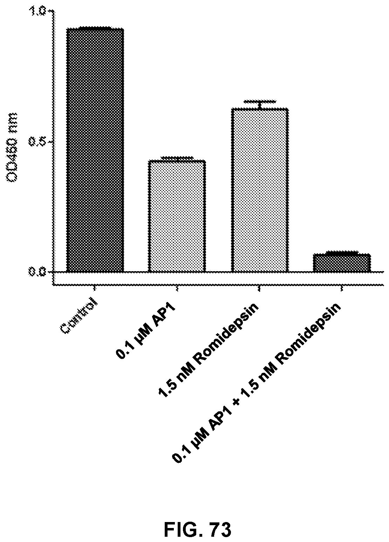

**Figure 73** shows MOLT-3 cell viability when treated with AP1 alone, romidepsin alone, and AP1 in combination with romidepsin.

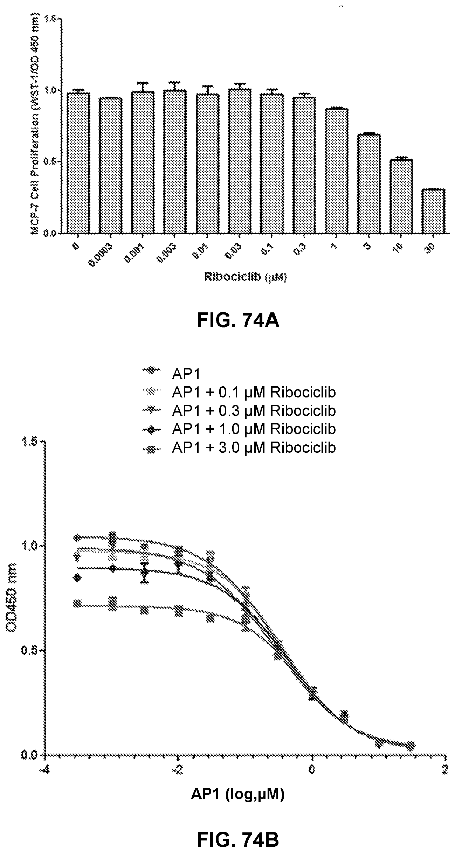

**Figure 74A** shows MCF-7 cell proliferation when treated with varying concentrations of ribociclib.
**Figure 74B** shows MCF-7 cell viability when treated with varying concentrations of AP1 and ribociclib.

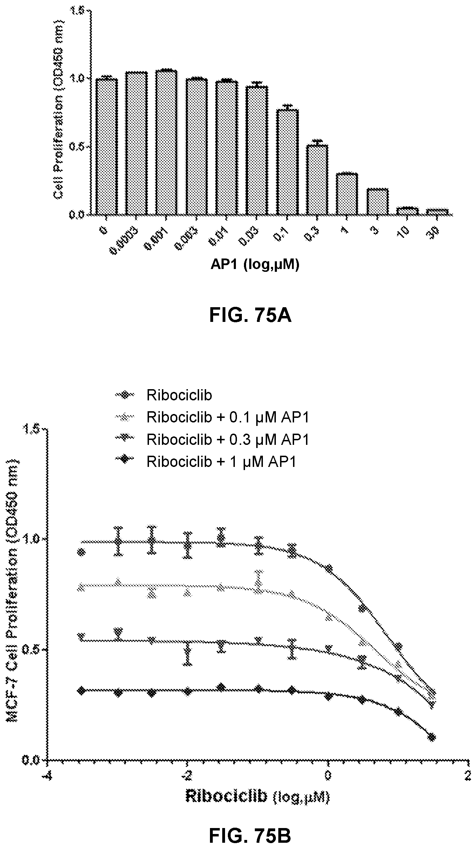

**Figure 75A** shows MCF-7 cell proliferation when treated with varying concentrations of AP1.
**Figure 75B** shows MCF-7 cell viability when treated with varying concentrations of AP1 and ribociclib.

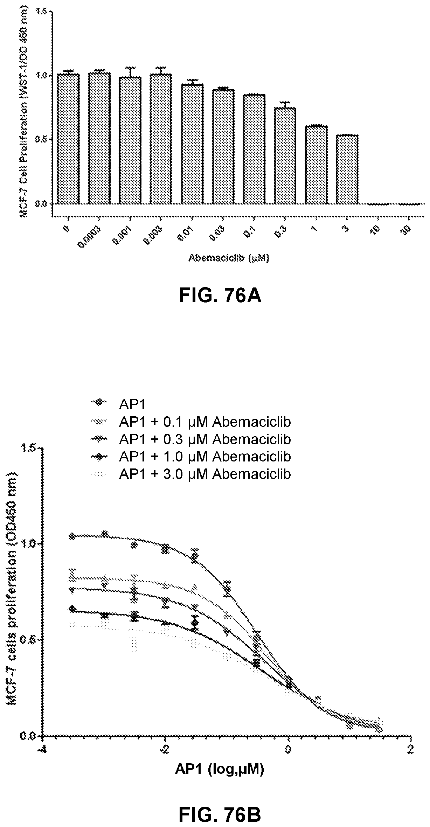

**Figure 76A** shows MCF-7 cell proliferation when treated with varying concentrations of abemaciclib.
**Figure 76B** shows MCF-7 cell viability when treated with varying concentrations of AP1 and abemaciclib.

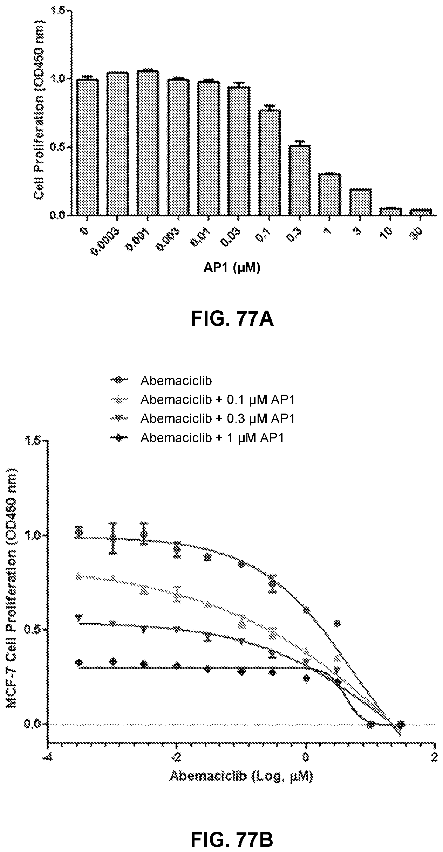

**Figure 77A** shows MCF-7 cell proliferation when treated with varying concentrations of AP1.
**Figure 77B** shows MCF-7 cell viability when treated with varying concentrations of AP1 and abemaciclib.

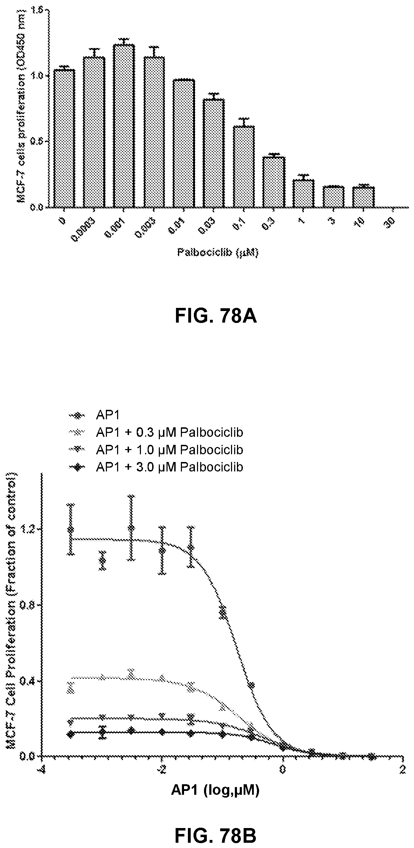

**Figure 78A** shows MCF-7 cell proliferation when treated with varying concentrations of palbociclib.
**Figure 78B** shows MCF-7 cell viability when treated with varying concentrations of AP1 and palbociclib.

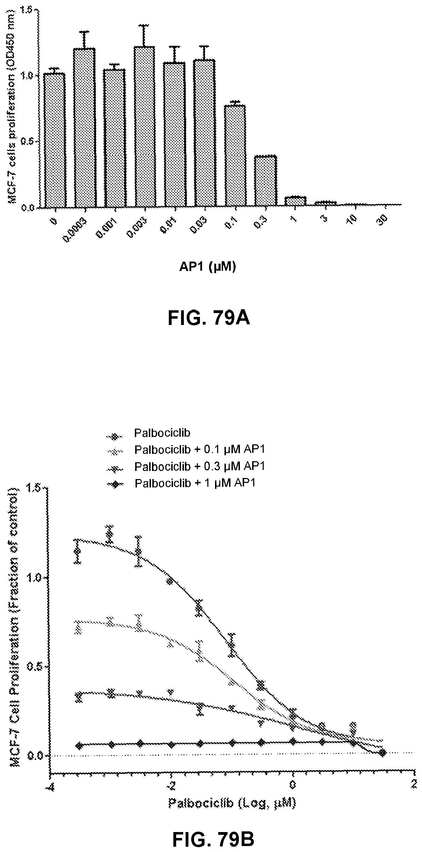

**Figure 79A** shows MCF-7 cell proliferation when treated with varying concentrations of AP1.
**Figure 79B** shows MCF-7 cell viability when treated with varying concentrations of AP1 and palbociclib.

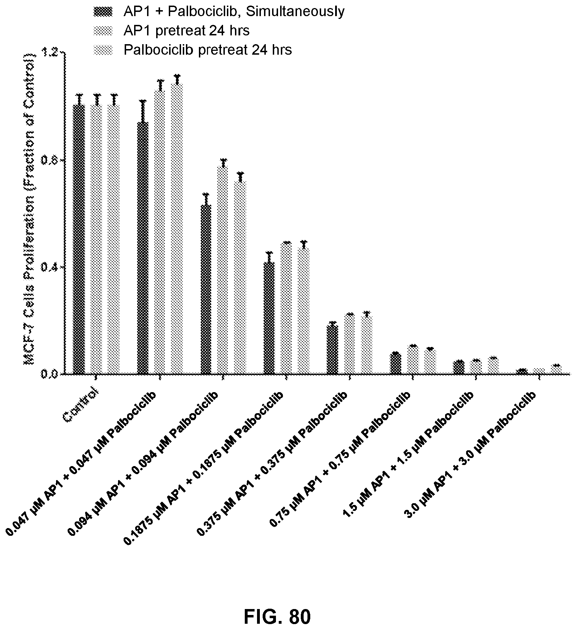

**Figure 80** shows the order of addition effects of AP1 and palbociclib on MCF-7 cell growth.

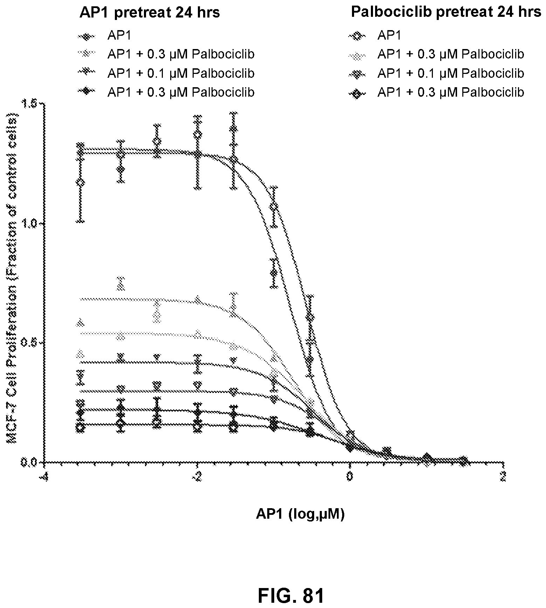

**Figure 81** shows MCF-7 cell viability based on the order of addition when treated with varying concentrations of AP1 and palbociclib after 24 hrs pretreatment with AP1.

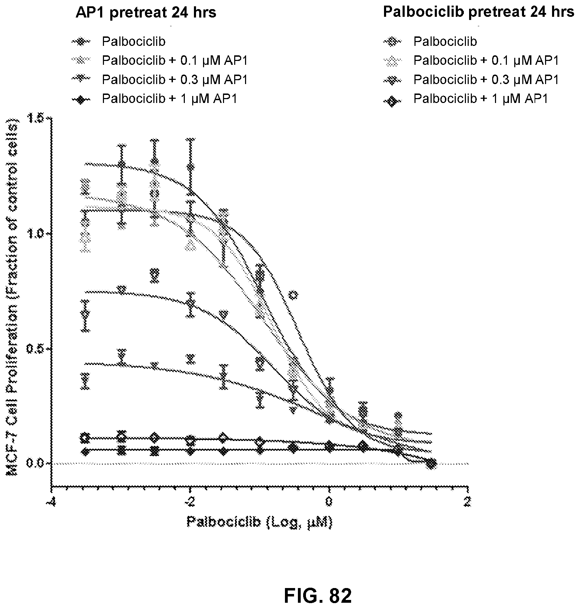

**Figure 82** shows MCF-7 cell viability when treated with varying concentrations of AP1 and palbociclib determined using CyQUANT.

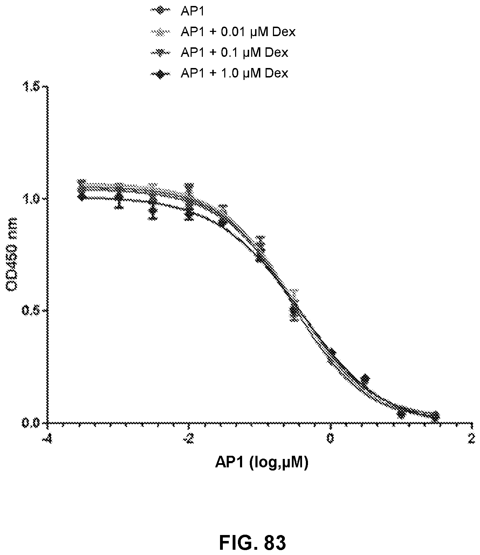

**Figure 83** shows MCF-7 cell viability based on the order of addition when treated with varying concentrations of AP1 and dexamethasone (Dex).

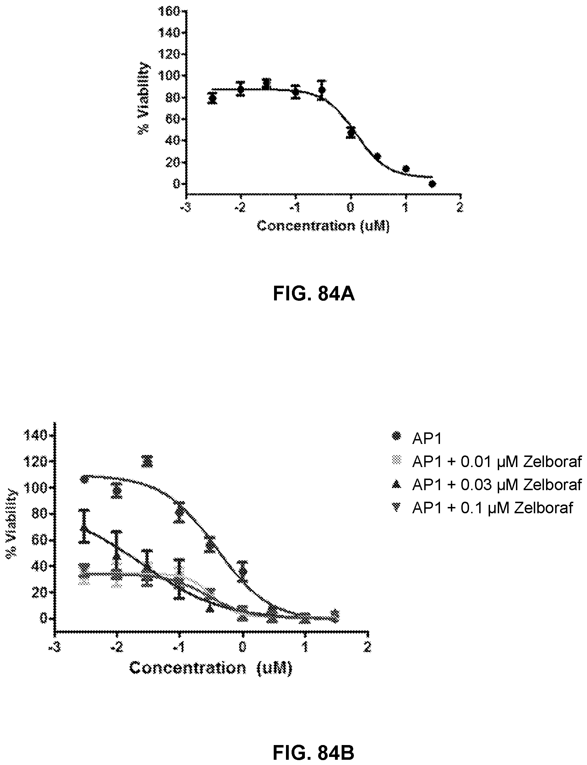

**Figure 84A** shows A375 cell viability when treated with varying concentrations of zelboraf.
**Figure 84B** shows A375 cell viability with treatment with varying concentrations of AP1 and zelboraf.

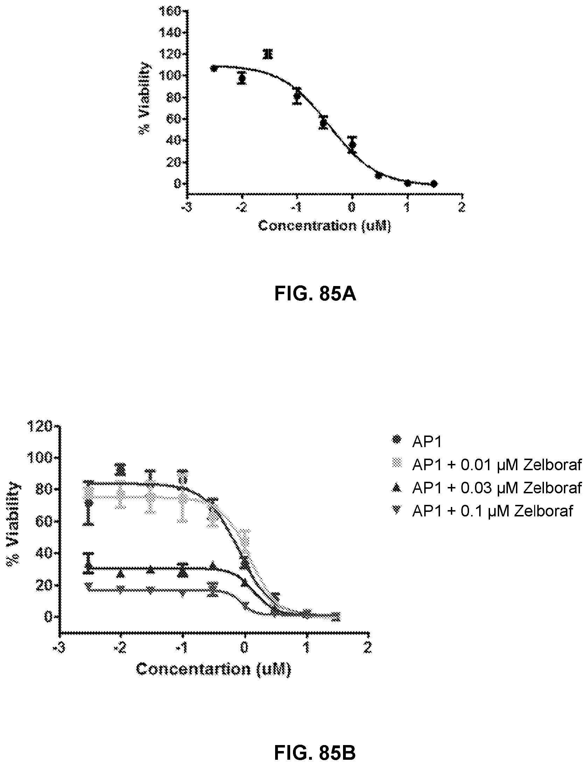

**Figure 85A** shows A375 cell viability when treated with varying concentrations of AP1.
**Figure 85B** shows A375 cell viability with treatment with varying concentrations of zelboraf and AP1.

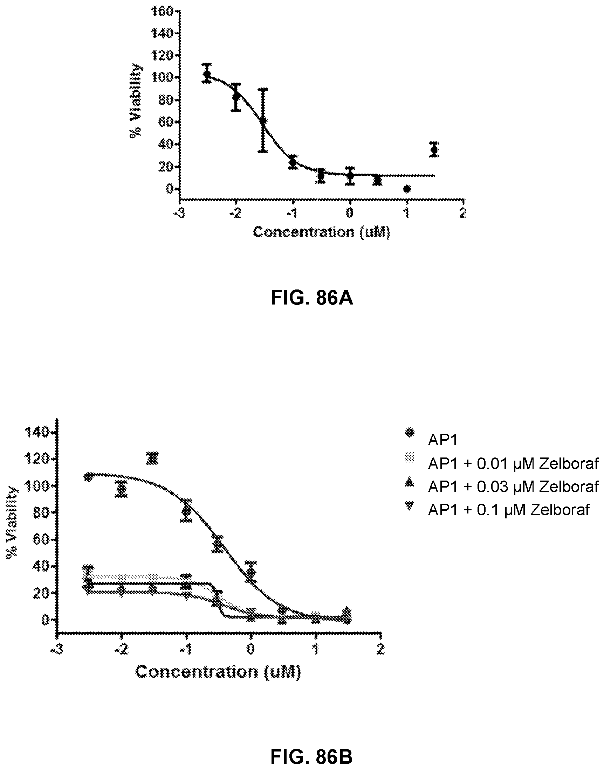

**Figure 86A** shows A375 cell viability when treated with varying concentrations of tafinlar.
**Figure 86B** shows A375 cell viability with treatment with varying concentrations of AP1 and tafinlar.

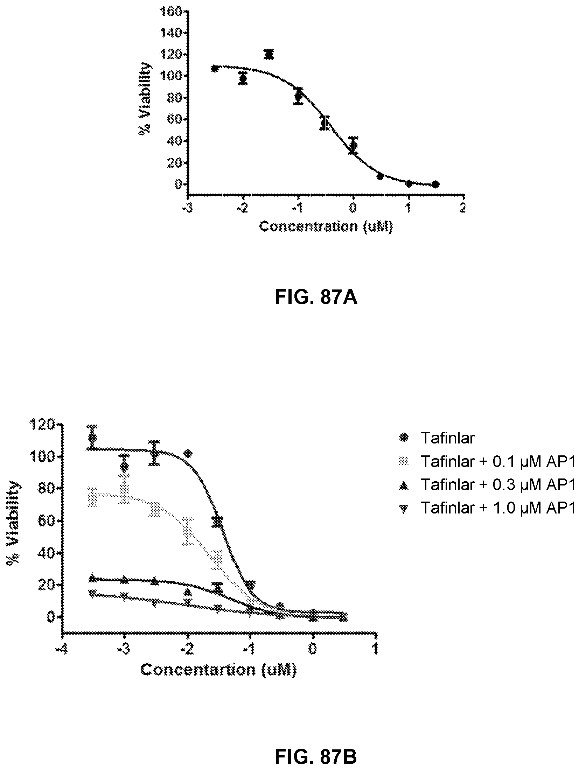

**Figure 87A** shows A375 cell viability when treated with varying concentrations of AP1.
**Figure 87B** shows A375 cell viability with treatment with varying concentrations oftafinlar and AP1.

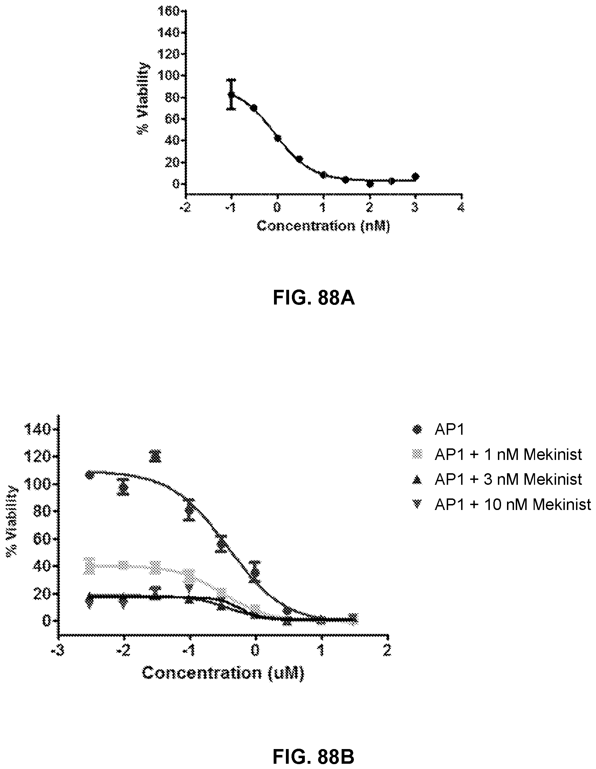

**Figure 88A** shows A375 cell viability when treated with varying concentrations of mekinist.
**Figure 88B** shows cancer cell viability with treatment with varying concentrations of AP1 and mekinist.

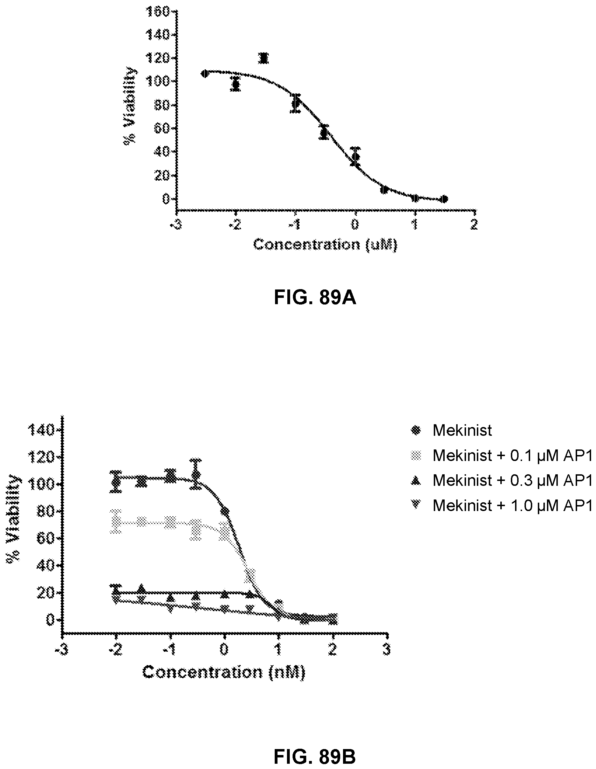

**Figure 89A** shows A375 cell viability when treated with varying concentrations of AP1.
**Figure 89B** shows A375 cell viability with treatment with varying concentrations of mekinist and AP1.

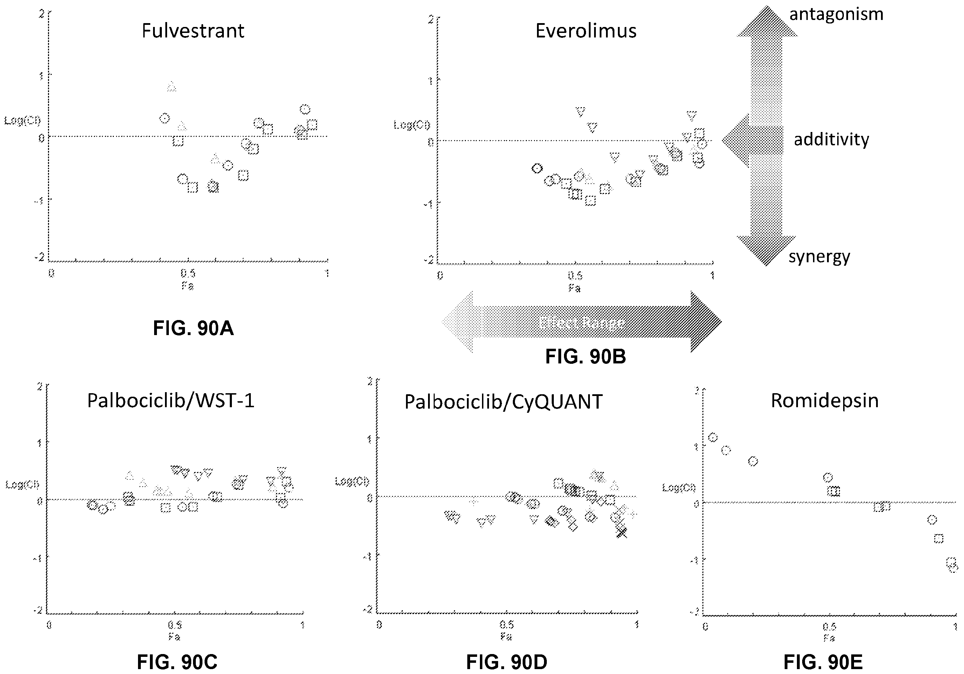

**Figure 90A** shows a combination index plot of fulvestrant in MCF-7 cells.
**Figure 90B** shows a combination index plot of everolimus in MCF-7 cells.
**Figure 90C** shows a combination index plot of palbociclib (WST-1) in MCF-7 cells
**Figure 90D** shows a combination index plot of palbociclib (CyQUANT) in MCF-7 cells.
**Figure 90E** shows a combination index plot of romidepsin in MCF-7 cells.

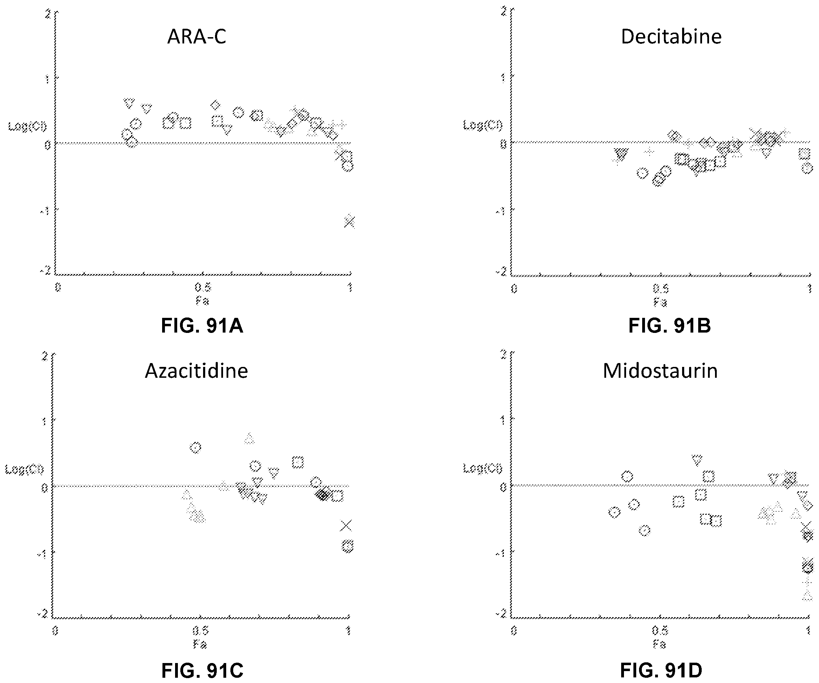

**Figure 91A** shows a combination index plot of Ara-C in MV4-11 cells.
**Figure 91B** shows a combination index plot of decitabine in MV4-11 cells.
**Figure 91C** shows a combination index plot of azacitidine in MV4-11 cells.
**Figure 91D** shows a combination index plot of midostuarin in MV4-11 cells.

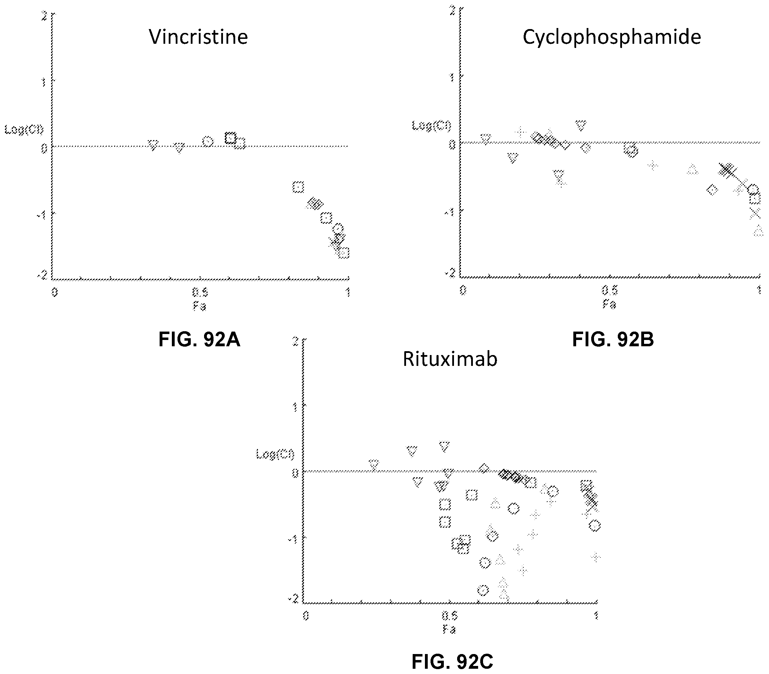

**Figure 92A** shows a combination index plot of vincristine in DOHH-2 cells.
**Figure 92B** shows a combination index plot of cyclophosphamide in DOHH-2 cells.
**Figure 92C** shows a combination index plot ofrituximab in DOHH-2 cells.

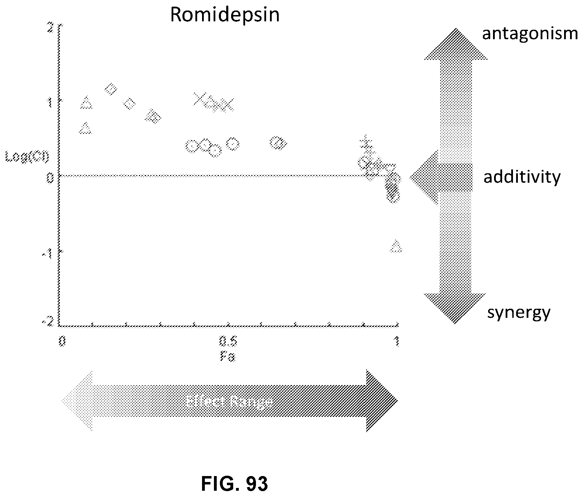

**Figure 93** shows a combination index plot of romidepsin in MOLT-3 cells.

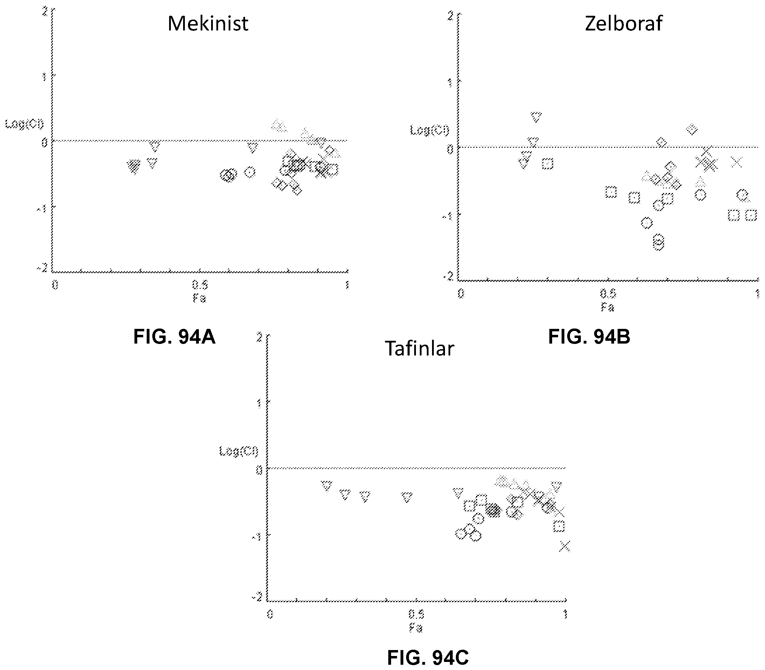

**Figure 94A** shows a combination index plot of mekinist in A375 cells
**Figure 94B** shows a combination index plot of zelboraf in A375 cells.
**Figure 94C** shows a combination index plot of tafinlar in A375 cells.

### DETAILED DESCRIPTION OF THE INVENTION

the invention consists in the subject-matter of the claims.

As used herein, the term "macrocycle" refers to a molecule having a chemical structure including a ring or cycle formed by at least 9 covalently bonded atoms.

As used herein, the term "peptidomimetic macrocycle" or "crosslinked polypeptide" refers to a compound comprising a plurality of amino acid residues joined by a plurality of peptide bonds and at least one macrocycle-forming linker which forms a macrocycle between a first naturally-occurring or non-naturally-occurring amino acid residue (or analog) and a second naturally-occurring or non-naturally-occurring amino acid residue (or analog) within the same molecule. Peptidomimetic macrocycle include cases where the macrocycle-forming linker connects the α-carbon of the first amino acid residue (or analog) to the α-carbon of the second amino acid residue (or analog). The peptidomimetic macrocycles optionally include one or more non-peptide bonds between one or more amino acid residues and/or amino acid analog residues, and optionally include one or more non-naturally-occurring amino acid residues or amino acid analog residues in addition to any which form the macrocycle. A "corresponding uncrosslinked polypeptide" when referred to in the context of a peptidomimetic macrocycle is understood to relate to a polypeptide of the same length as the macrocycle and comprising the equivalent natural amino acids of the wild-type sequence corresponding to the macrocycle.

Aileron peptide 1 is an alpha helical hydrocarbon cross-linked polypeptide macrocycle, with an amino acid sequence less than 20 amino acids long that is derived from the transactivation domain of wild type human p53 protein and that contains a phenylalanine, a tryptophan and a leucine amino acid in the same positions relative to each other as in the transactivation domain of wild type human p53 protein. Aileron peptide 1 has a single cross link spanning amino acids in the i to the i+7 position of the amino acid sequence and has more than three amino acids between the i+7 position and the carboxyl terminus. Aileron peptide 1 binds to human MDM2 and MDM4 and has an observed mass of 950-975 m/e as measured by electrospray ionization-mass spectrometry.

As used herein, the term "stability" refers to the maintenance of a defined secondary structure in solution by a peptidomimetic macrocycle as measured by circular dichroism, NMR or another biophysical measure, or resistance to proteolytic degradation *in vitro* or *in vivo.* Non-limiting examples of secondary structures contemplated herein are α-helices, 3₁₀ helices, β-turns, and β-pleated sheets.

As used herein, the term "helical stability" refers to the maintenance of α helical structure by a peptidomimetic macrocycle as measured by circular dichroism or NMR. For example, in some cases, a peptidomimetic macrocycle exhibits at least a 1.25, 1.5, 1.75 or 2-fold increase in α-helicity as determined by circular dichroism compared to a corresponding uncrosslinked macrocycle.

The term "amino acid" refers to a molecule containing both an amino group and a carboxyl group. Suitable amino acids include, without limitation, both the D-and L-isomers of the naturally-occurring amino acids, as well as non-naturally occurring amino acids prepared by organic synthesis or other metabolic routes. The term amino acid, as used herein, includes, without limitation, α-amino acids, natural amino acids, non-natural amino acids, and amino acid analogs.

The term "α-amino acid" refers to a molecule containing both an amino group and a carboxyl group bound to a carbon which is designated the α-carbon.

The term "β-amino acid" refers to a molecule containing both an amino group and a carboxyl group in a β configuration.

The term "naturally occurring amino acid" refers to any one of the twenty amino acids commonly found in peptides synthesized in nature, and known by the one letter abbreviations A, R, N, C, D, Q, E, G, H, I, L, K, M, F, P, S, T, W, Y and V.

The following table shows a summary of the properties of natural amino acids:

| Amino Acid | 3-Letter Code | 1-Letter Code | Side-chain Polarity | Side-chain charge (pH 7.4) | Hydropathy Index |
|---|---|---|---|---|---|
| Alanine | Ala | A | nonpolar | neutral | 1.8 |
| Arginine | Arg | R | polar | positive | -4.5 |
| Asparagine | Asn | N | polar | neutral | -3.5 |
| Aspartic acid | Asp | D | polar | negative | -3.5 |
| Cysteine | Cys | C | polar | neutral | 2.5 |
| Glutamic acid | Glu | E | polar | negative | -3.5 |
| Glutamine | Gln | Q | polar | neutral | -3.5 |
| Glycine | Gly | G | nonpolar | neutral | -0.4 |
| Histidine | His | H | polar | positive(10%) neutral(90%) | -3.2 |
| Isoleucine | Ile | I | nonpolar | neutral | 4.5 |
| Leucine | Leu | L | nonpolar | neutral | 3.8 |
| Lysine | Lys | K | polar | positive | -3.9 |
| Methionine | Met | M | nonpolar | neutral | 1.9 |
| Phenylalanine | Phe | F | nonpolar | neutral | 2.8 |
| Proline | Pro | P | nonpolar | neutral | -1.6 |
| Serine | Ser | S | polar | neutral | -0.8 |
| Threonine | Thr | T | polar | neutral | -0.7 |
| Tryptophan | Trp | w | nonpolar | neutral | -0.9 |
| Tyrosine | Tyr | Y | polar | neutral | -1.3 |
| Valine | Val | V | nonpolar | neutral | 4.2 |

"Hydrophobic amino acids" include small hydrophobic amino acids and large hydrophobic amino acids. "Small hydrophobic amino acids" are glycine, alanine, proline, and analogs thereof. "Large hydrophobic amino acids" are valine, leucine, isoleucine, phenylalanine, methionine, tryptophan, and analogs thereof. "Polar amino acids" are serine, threonine, asparagine, glutamine, cysteine, tyrosine, and analogs thereof. "Charged amino acids" are lysine, arginine, histidine, aspartate, glutamate, and analogs thereof.

The term "amino acid analog" refers to a molecule which is structurally similar to an amino acid and which can be substituted for an amino acid in the formation of a peptidomimetic macrocycle. Amino acid analogs include, without limitation, β-amino acids and amino acids wherein the amino or carboxy group is substituted by a similarly reactive group (e.g., substitution of the primary amine with a secondary or tertiary amine, or substitution of the carboxy group with an ester).

The term "non-natural amino acid" refers to an amino acid which is not one of the twenty amino acids commonly found in peptides synthesized in nature, and known by the one letter abbreviations A, R, N, C, D, Q, E, G, H, I, L, K, M, F, P, S, T, W, Y and V. Non-natural amino acids or amino acid analogs include, without limitation, structures according to the following:

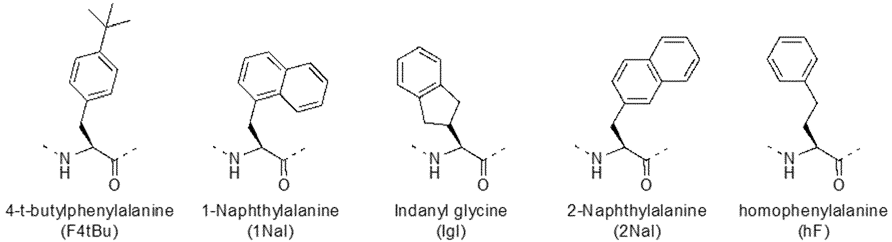

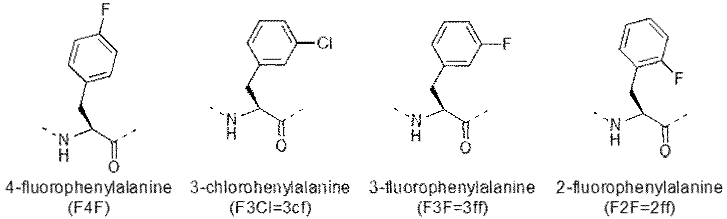

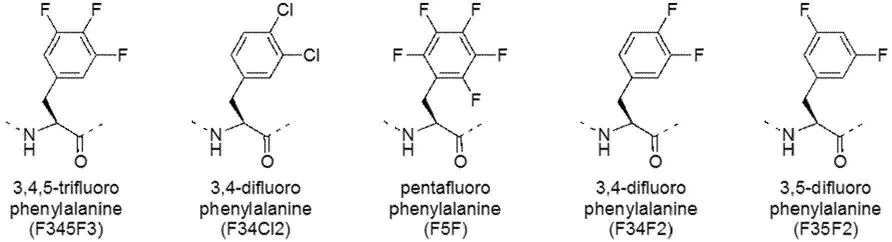

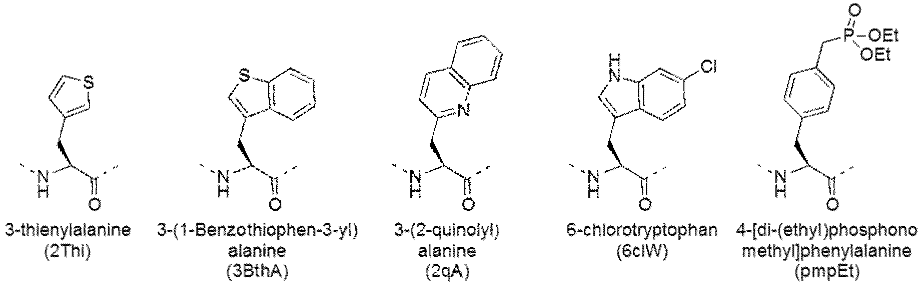

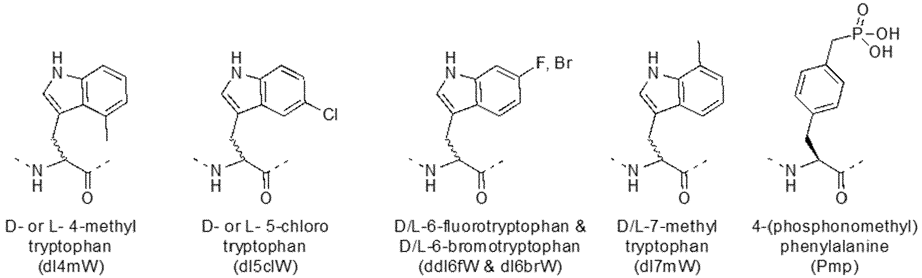

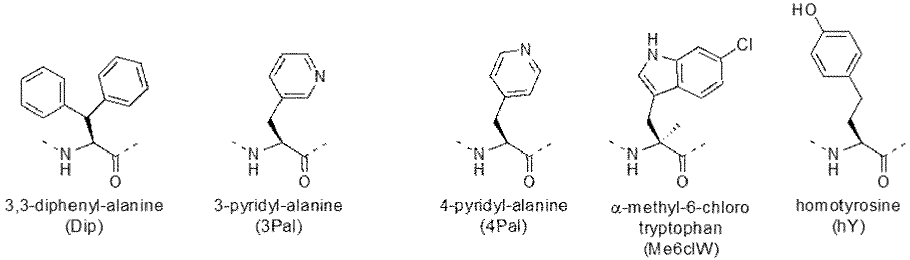

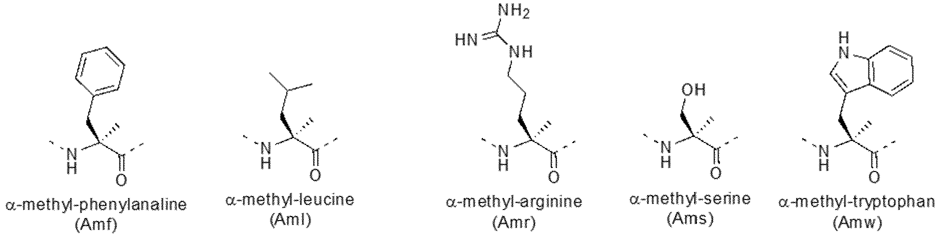

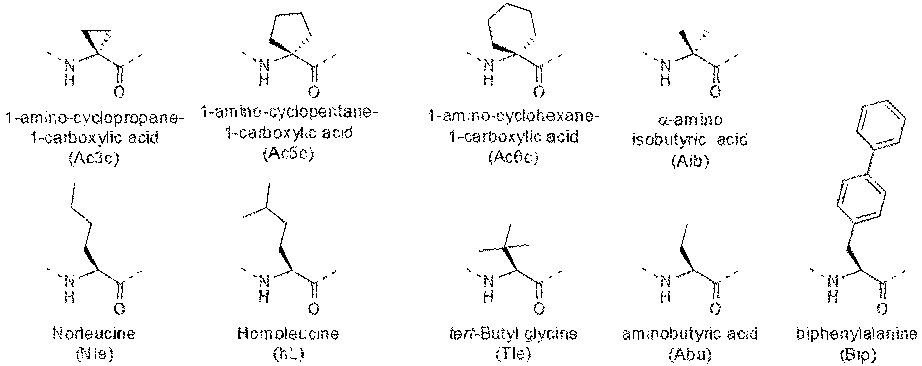

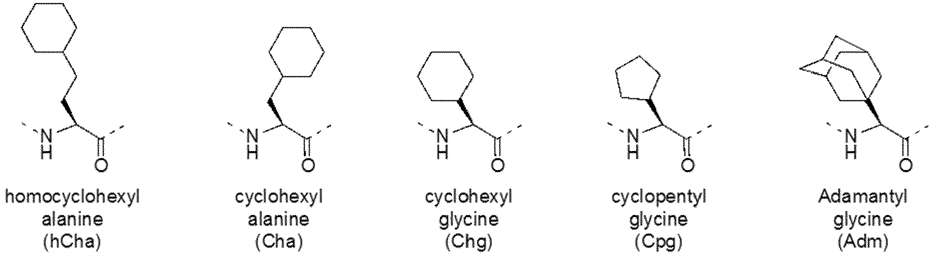

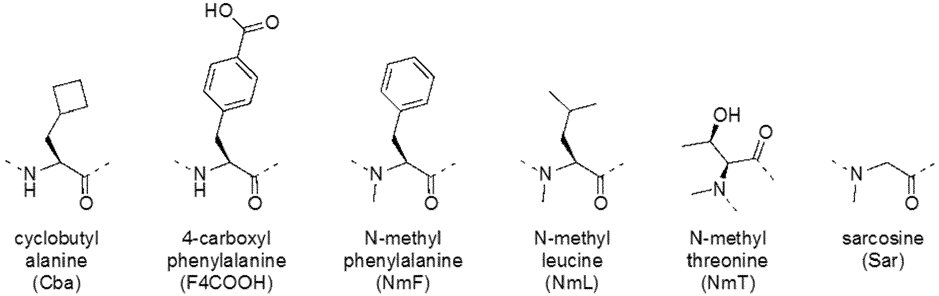

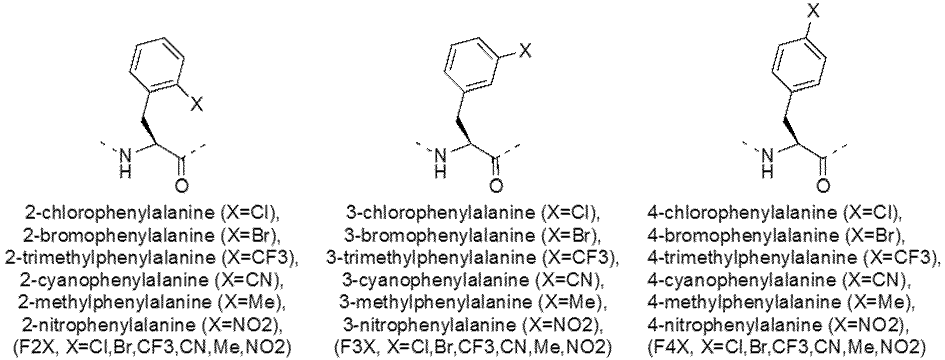

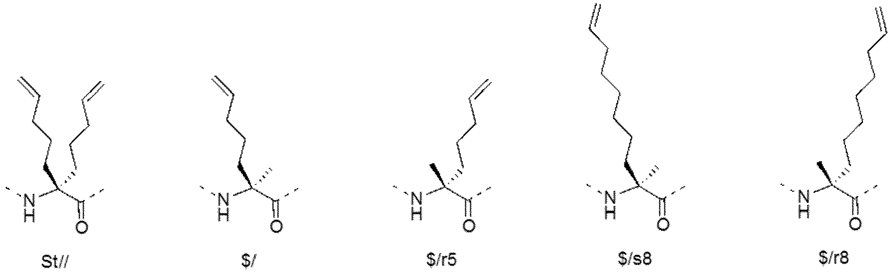

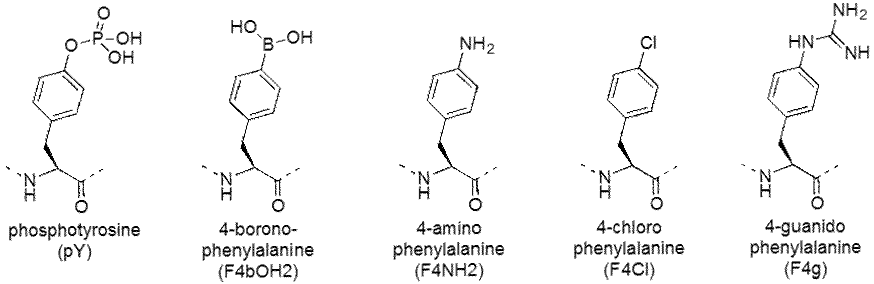

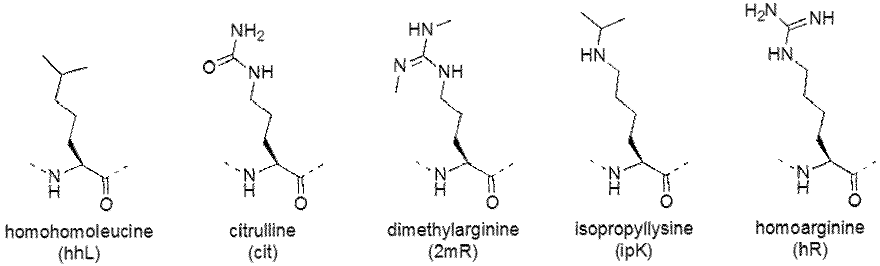

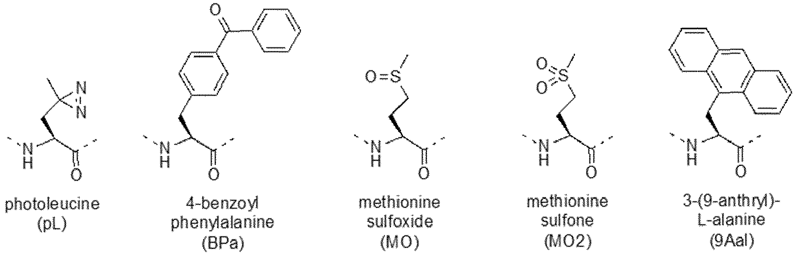

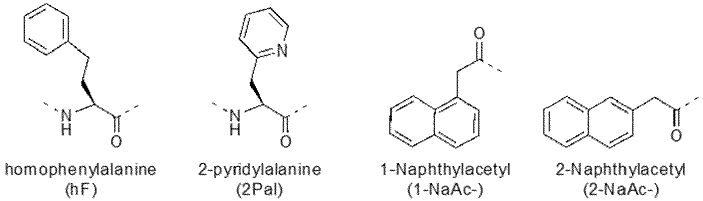

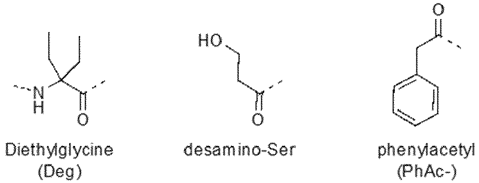

Amino acid analogs include β-amino acid analogs. Examples of β-amino acid analogs include, but are not limited to, the following: cyclic β-amino acid analogs; β-alanine; (R)-β-phenylalanine; (R)-1,2,3,4-tetrahydro-isoquinoline-3-acetic acid; (R)-3-amino-4-(1-naphthyl)-butyric acid; (R)-3-amino-4-(2,4-dichlorophenyl)butyric acid; (R)-3-amino-4-(2-chlorophenyl)-butyric acid; (R)-3-amino-4-(2-cyanophenyl)-butyric acid; (R)-3-amino-4-(2-fluorophenyl)-butyric acid; (R)-3-amino-4-(2-furyl)-butyric acid; (R)-3-amino-4-(2-methylphenyl)-butyric acid; (R)-3-amino-4-(2-naphthyl)-butyric acid; (R)-3-amino-4-(2-thienyl)-butyric acid; (R)-3-amino-4-(2-trifluoromethylphenyl)-butyric acid; (R)-3-amino-4-(3,4-dichlorophenyl)butyric acid; (R)-3-amino-4-(3,4-difluorophenyl)butyric acid; (R)-3-amino-4-(3-benzothienyl)-butyric acid; (R)-3-amino-4-(3-chlorophenyl)-butyric acid; (R)-3-amino-4-(3-cyanophenyl)-butyric acid; (R)-3-amino-4-(3-fluorophenyl)-butyric acid; (R)-3-amino-4-(3-methylphenyl)-butyric acid; (R)-3-amino-4-(3-pyridyl)-butyric acid; (R)-3-amino-4-(3-thienyl)-butyric acid; (R)-3-amino-4-(3-trifluoromethylphenyl)-butyric acid; (R)-3-amino-4-(4-bromophenyl)-butyric acid; (R)-3-amino-4-(4-chlorophenyl)-butyric acid; (R)-3-amino-4-(4-cyanophenyl)-butyric acid; (R)-3-amino-4-(4-fluorophenyl)-butyric acid; (R)-3-amino-4-(4-iodophenyl)-butyric acid; (R)-3-amino-4-(4-methylphenyl)-butyric acid; (R)-3-amino-4-(4-nitrophenyl)-butyric acid; (R)-3-amino-4-(4-pyridyl)-butyric acid; (R)-3-amino-4-(4-trifluoromethylphenyl)-butyric acid; (R)-3-amino-4-pentafluoro-phenylbutyric acid; (R)-3-amino-5-hexenoic acid; (R)-3-amino-5-hexynoic acid; (R)-3-amino-5-phenylpentanoic acid; (R)-3-amino-6-phenyl-5-hexenoic acid; (S)-1,2,3,4-tetrahydro-isoquinoline-3-acetic acid; (S)-3-amino-4-(1-naphthyl)-butyric acid; (S)-3-amino-4-(2,4-dichlorophenyl)butyric acid; (S)-3-amino-4-(2-chlorophenyl)-butyric acid; (S)-3-amino-4-(2-cyanophenyl)-butyric acid; (S)-3-amino-4-(2-fluorophenyl)-butyric acid; (S)-3-amino-4-(2-furyl)-butyric acid; (S)-3-amino-4-(2-methylphenyl)-butyric acid; (S)-3-amino-4-(2-naphthyl)-butyric acid; (S)-3-amino-4-(2-thienyl)-butyric acid; (S)-3-amino-4-(2-trifluoromethylphenyl)-butyric acid; (S)-3-amino-4-(3,4-dichlorophenyl)butyric acid; (S)-3-amino-4-(3,4-difluorophenyl)butyric acid; (S)-3-amino-4-(3-benzothienyl)-butyric acid; (S)-3-amino-4-(3-chlorophenyl)-butyric acid; (S)-3-amino-4-(3-cyanophenyl)-butyric acid; (S)-3-amino-4-(3-fluorophenyl)-butyric acid; (S)-3-amino-4-(3-methylphenyl)-butyric acid; (S)-3-amino-4-(3-pyridyl)-butyric acid; (S)-3-amino-4-(3-thienyl)-butyric acid; (S)-3-amino-4-(3-trifluoromethylphenyl)-butyric acid; (S)-3-amino-4-(4-bromophenyl)-butyric acid; (S)-3-amino-4-(4-chlorophenyl)-butyric acid; (S)-3-amino-4-(4-cyanophenyl)-butyric acid; (S)-3-amino-4-(4-fluorophenyl)-butyric acid; (S)-3-amino-4-(4-iodophenyl)-butyric acid; (S)-3-amino-4-(4-methylphenyl)-butyric acid; (S)-3-amino-4-(4-nitrophenyl)-butyric acid; (S)-3-amino-4-(4-pyridyl)-butyric acid; (S)-3-amino-4-(4-trifluoromethylphenyl)-butyric acid; (S)-3-amino-4-pentafluoro-phenylbutyric acid; (S)-3-amino-5-hexenoic acid; (S)-3-amino-5-hexynoic acid; (S)-3-amino-5-phenylpentanoic acid; (S)-3-amino-6-phenyl-5-hexenoic acid; 1,2,5,6-tetrahydropyridine-3-carboxylic acid; 1,2,5,6-tetrahydropyridine-4-carboxylic acid; 3-amino-3-(2-chlorophenyl)-propionic acid; 3-amino-3-(2-thienyl)-propionic acid; 3-amino-3-(3-bromophenyl)-propionic acid; 3-amino-3-(4-chlorophenyl)-propionic acid; 3-amino-3-(4-methoxyphenyl)-propionic acid; 3-amino-4,4,4-trifluoro-butyric acid; 3-aminoadipic acid; D- β-phenylalanine; β-leucine; L-β-homoalanine; L-β-homoaspartic acid γ-benzyl ester; L-β-homoglutamic acid δ-benzyl ester; L-β-homoisoleucine; L-β-homoleucine; L-β-homomethionine; L-β-homophenylalanine; L-β-homoproline; L-β-homotryptophan; L-β-homovaline; L-Nω-benzyloxycarbonyl-β-homolysine; Nω-L-β-homoarginine; O-benzyl-L-β-homohydroxyproline; O-benzyl-L-β-homoserine; O-benzyl-L-β-homothreonine; O-benzyl-L-β-homotyrosine; γ-trityl-L-β-homoasparagine; (R)-β-phenylalanine; L-β-homoaspartic acid γ-t-butyl ester; L-β-homoglutamic acid δ-t-butyl ester; L-Nω-β-homolysine; Nδ-trityl-L-β-homoglutamine; Nω-2,2,4,6,7-pentamethyl-dihydrobenzofuran-5-sulfonyl-L-β-homoarginine; O-t-butyl-L-β-homohydroxy-proline; O-t-butyl-L-β-homoserine; O-t-butyl-L-β-homothreonine; O-t-butyl-L-β-homotyrosine; 2- aminocyclopentane carboxylic acid; and 2-aminocyclohexane carboxylic acid.

Amino acid analogs include analogs of alanine, valine, glycine or leucine. Examples of amino acid analogs of alanine, valine, glycine, and leucine include, but are not limited to, the following: α-methoxyglycine; α-allyl-L-alanine; α-aminoisobutyric acid; α-methyl-leucine; β-(1-naphthyl)-D-alanine; β-(1-naphthyl)-L-alanine; β-(2-naphthyl)-D-alanine; β-(2-naphthyl)-L-alanine; β-(2-pyridyl)-D-alanine; β-(2-pyridyl)-L-alanine; β-(2-thienyl)-D-alanine; β-(2-thienyl)-L-alanine; β-(3-benzothienyl)-D-alanine; β-(3-benzothienyl)-L-alanine; β-(3-pyridyl)-D-alanine; β-(3-pyridyl)-L-alanine; β-(4-pyridyl)-D-alanine; β-(4-pyridyl)-L-alanine; β-chloro-L-alanine; β-cyano-L-alanine; β-cyclohexyl-D-alanine; β-cyclohexyl-L-alanine; β-cyclopenten-1-yl-alanine; β-cyclopentyl-alanine; β-cyclopropyl-L-Ala-OH • dicyclohexylammonium salt; β-t-butyl-D-alanine; β-t-butyl-L-alanine; γ-aminobutyric acid; L-α,β-diaminopropionic acid; 2,4-dinitro-phenylglycine; 2,5-dihydro-D-phenylglycine; 2-amino-4,4,4-trifluorobutyric acid; 2-fluoro-phenylglycine; 3-amino-4,4,4-trifluoro-butyric acid; 3-fluoro-valine; 4,4,4-trifluoro-valine; 4,5-dehydro-L-leu-OH • dicyclohexylammonium salt; 4-fluoro-D-phenylglycine; 4-fluoro-L-phenylglycine; 4-hydroxy-D-phenylglycine; 5,5,5-trifluoro-leucine; 6-aminohexanoic acid; cyclopentyl-D-Gly-OH • dicyclohexylammonium salt; cyclopentyl-Gly-OH • dicyclohexylammonium salt; D-α,β-diaminopropionic acid; D-α-aminobutyric acid; D-α-t-butylglycine; D-(2-thienyl)glycine; D-(3-thienyl)glycine; D-2-aminocaproic acid; D-2-indanylglycine; D-allylglycine•dicyclohexylammonium salt; D-cyclohexylglycine; D-norvaline; D-phenylglycine; β-aminobutyric acid; β-aminoisobutyric acid; (2-bromophenyl)glycine; (2-methoxyphenyl)glycine; (2-methylphenyl)glycine; (2-thiazoyl)glycine; (2-thienyl)glycine; 2-amino-3-(dimethylamino)-propionic acid; L-α,β-diaminopropionic acid; L-α-aminobutyric acid; L-α-t-butylglycine; L-(3-thienyl)glycine; L-2-amino-3-(dimethylamino)-propionic acid; L-2-aminocaproic acid dicyclohexylammonium salt; L-2-indanylglycine; L-allylglycine•dicyclohexyl ammonium salt; L-cyclohexylglycine; L-phenylglycine; L-propargylglycine; L-norvaline; N-α-aminomethyl-L-alanine; D-α,γ-diaminobutyric acid; L-α,γ-diaminobutyric acid; β-cyclopropyl-L-alanine; (N-β-(2,4-dinitrophenyl))-L-α,β-diaminopropionic acid; (N-β-1-(4,4-dimethyl-2,6-dioxocyclohex-1-ylidene)ethyl)-D-α,β-diaminopropionic acid; (N-β-1-(4,4-dimethyl-2,6-dioxocyclohex-1-ylidene)ethyl)-L-α,β-diaminopropionic acid; (N-β-4-methyltrityl)-L-α,β-diaminopropionic acid; (N-β-allyloxycarbonyl)-L-α,β-diaminopropionic acid; (N-γ-1-(4,4-dimethyl-2,6-dioxocyclohex-1-ylidene)ethyl)-D-α,γ-diaminobutyric acid; (N-γ-1-(4,4-dimethyl-2,6-dioxocyclohex-1-ylidene)ethyl)-L-α,γ-diaminobutyric acid; (N-γ-4-methyltrityl)-D-α,γ-diaminobutyric acid; (N-γ-4-methyltrityl)-L-α,γ-diaminobutyric acid; (N-γ-allyloxycarbonyl)-L-α,γ-diaminobutyric acid; D-α,γ-diaminobutyric acid; 4,5-dehydro-L-leucine; cyclopentyl-D-Gly-OH; cyclopentyl-Gly-OH; D-allylglycine; D-homocyclohexylalanine; L-1-pyrenylalanine; L-2-aminocaproic acid; L-allylglycine; L-homocyclohexylalanine; and N-(2-hydroxy-4-methoxy-Bzl)-Gly-OH.

Amino acid analogs include analogs of arginine or lysine. Examples of amino acid analogs of arginine and lysine include, but are not limited to, the following: citrulline; L-2-amino-3-guanidinopropionic acid; L-2-amino-3-ureidopropionic acid; L-citrulline; Lys(Me)₂-OH; Lys(N₃)-OH; Nδ-benzyloxycarbonyl-L-ornithine; Nω-nitro-D-arginine; Nω-nitro-L-arginine; α-methyl-ornithine; 2,6-diaminoheptanedioic acid; L-ornithine; (Nδ-1-(4,4-dimethyl-2,6-dioxo-cyclohex-1-ylidene)ethyl)-D-ornithine; (Nδ-1-(4,4-dimethyl-2,6-dioxo-cyclohex-1-ylidene)ethyl)-L-ornithine; (Nδ-4-methyltrityl)-D-ornithine; (Nδ-4-methyltrityl)-L-ornithine; D-omithine; L-omithine; Arg(Me)(Pbf)-OH; Arg(Me)₂-OH (asymmetrical); Arg(Me)₂-OH (symmetrical); Lys(ivDde)-OH; Lys(Me)₂-OH • HCl; Lys(Me₃)-OH chloride; Nω-nitro-D-arginine; and Nω-nitro-L-arginine.

Amino acid analogs include analogs of aspartic or glutamic acids. Examples of amino acid analogs of aspartic and glutamic acids include, but are not limited to, the following: α-methyl-D-aspartic acid; α-methyl-glutamic acid; α-methyl-L-aspartic acid; γ-methylene-glutamic acid; (N-γ-ethyl)-L-glutamine; [N-α-(4-aminobenzoyl)]-L-glutamic acid; 2,6-diaminopimelic acid; L-α-aminosuberic acid; D-2-aminoadipic acid; D-α-aminosuberic acid; α-aminopimelic acid; iminodiacetic acid; L-2-aminoadipic acid; threo-β-methyl-aspartic acid; γ-carboxy-D-glutamic acid γ,γ-di-t-butyl ester; γ-carboxy-L-glutamic acid γ,γ-di-t-butyl ester; Glu(OAll)-OH; L-Asu(OtBu)-OH; and pyroglutamic acid.

Amino acid analogs include analogs of cysteine and methionine. Examples of amino acid analogs of cysteine and methionine include, but are not limited to, Cys(farnesyl)-OH, Cys(farnesyl)-OMe, α-methyl-methionine, Cys(2-hydroxyethyl)-OH, Cys(3-aminopropyl)-OH, 2-amino-4-(ethylthio)butyric acid, buthionine, buthioninesulfoximine, ethionine, methionine methylsulfonium chloride, selenomethionine, cysteic acid, [2-(4-pyridyl)ethyl]-DL-penicillamine, [2-(4-pyridyl)ethyl]-L-cysteine, 4-methoxybenzyl-D-penicillamine, 4-methoxybenzyl-L-penicillamine, 4-methylbenzyl-D-penicillamine, 4-methylbenzyl-L-penicillamine, benzyl-D-cysteine, benzyl-L-cysteine, benzyl-DL-homocysteine, carbamoyl-L-cysteine, carboxyethyl-L-cysteine, carboxymethyl-L-cysteine, diphenylmethyl-L-cysteine, ethyl-L-cysteine, methyl-L-cysteine, t-butyl-D-cysteine, trityl-L- homocysteine, trityl-D-penicillamine, cystathionine, homocystine, L-homocystine, (2-aminoethyl)-L-cysteine, seleno-L-cystine, cystathionine, Cys(StBu)-OH, and acetamidomethyl-D-penicillamine.

Amino acid analogs include analogs of phenylalanine and tyrosine. Examples of amino acid analogs of phenylalanine and tyrosine include β-methyl-phenylalanine, β-hydroxyphenylalanine, α-methyl-3-methoxy-DL-phenylalanine, α-methyl-D-phenylalanine, α-methyl-L-phenylalanine, 1,2,3,4-tetrahydroisoquinoline-3-carboxylic acid, 2,4-dichloro-phenylalanine, 2-(trifluoromethyl)-D -phenylalanine, 2-(trifluoromethyl)-L-phenylalanine, 2-bromo-D-phenylalanine, 2-bromo-L-phenylalanine, 2-chloro-D-phenylalanine, 2-chloro-L-phenylalanine, 2-cyano-D-phenylalanine, 2-cyano-L-phenylalanine, 2-fluoro-D-phenylalanine, 2-fluoro-L-phenylalanine, 2-methyl-D-phenylalanine, 2-methyl-L-phenylalanine, 2-nitro-D-phenylalanine, 2-nitro-L-phenylalanine, 2;4;5-trihydroxy-phenylalanine, 3,4,5-trifluoro-D-phenylalanine, 3,4,5-trifluoro-L-phenylalanine, 3,4-dichloro-D-phenylalanine, 3,4-dichloro-L-phenylalanine, 3,4-difluoro-D-phenylalanine, 3,4-difluoro-L-phenylalanine, 3,4-dihydroxy-L-phenylalanine, 3,4-dimethoxy-L-phenylalanine, 3,5,3'-triiodo-L-thyronine, 3,5-diiodo-D-tyrosine, 3,5-diiodo-L-tyrosine, 3,5-diiodo-L-thyronine, 3-(trifluoromethyl)-D-phenylalanine, 3-(trifluoromethyl)-L-phenylalanine, 3-amino-L-tyrosine, 3-bromo-D-phenylalanine, 3-bromo-L-phenylalanine, 3-chloro-D-phenylalanine, 3-chloro-L-phenylalanine, 3-chloro-L-tyrosine, 3-cyano-D-phenylalanine, 3-cyano-L-phenylalanine, 3-fluoro-D-phenylalanine, 3-fluoro-L-phenylalanine, 3-fluoro-tyrosine, 3-iodo-D-phenylalanine, 3-iodo-L-phenylalanine, 3-iodo-L-tyrosine, 3-methoxy-L-tyrosine, 3-methyl-D-phenylalanine, 3-methyl-L-phenylalanine, 3-nitro-D-phenylalanine, 3-nitro-L-phenylalanine, 3-nitro-L-tyrosine, 4-(trifluoromethyl)-D-phenylalanine, 4-(trifluoromethyl)-L-phenylalanine, 4-amino-D-phenylalanine, 4-amino-L-phenylalanine, 4-benzoyl-D-phenylalanine, 4-benzoyl-L-phenylalanine, 4-bis(2-chloroethyl)amino-L-phenylalanine, 4-bromo-D-phenylalanine, 4-bromo-L-phenylalanine, 4-chloro-D-phenylalanine, 4-chloro-L-phenylalanine, 4-cyano-D-phenylalanine, 4-cyano-L-phenylalanine, 4-fluoro-D-phenylalanine, 4-fluoro-L-phenylalanine, 4-iodo-D-phenylalanine, 4-iodo-L-phenylalanine, homophenylalanine, thyroxine, 3,3-diphenylalanine, thyronine, ethyl-tyrosine, and methyl-tyrosine.

Amino acid analogs include analogs of proline. Examples of amino acid analogs of proline include, but are not limited to, 3,4-dehydro-proline, 4-fluoro-proline, cis-4-hydroxy-proline, thiazolidine-2-carboxylic acid, and trans-4-fluoro-proline.

Amino acid analogs include analogs of serine and threonine. Examples of amino acid analogs of serine and threonine include, but are not limited to, 3-amino-2-hydroxy-5-methylhexanoic acid, 2-amino-3-hydroxy-4-methylpentanoic acid, 2-amino-3-ethoxybutanoic acid, 2-amino-3-methoxybutanoic acid, 4-amino-3-hydroxy-6-methylheptanoic acid, 2-amino-3-benzyloxypropionic acid, 2-amino-3-benzyloxypropionic acid, 2-amino-3-ethoxypropionic acid, 4-amino-3-hydroxybutanoic acid, and α-methylserine.

Amino acid analogs include analogs of tryptophan. Examples of amino acid analogs of tryptophan include, but are not limited to, the following: α-methyl-tryptophan; β-(3-benzothienyl)-D-alanine; β-(3-benzothienyl)-L-alanine; 1-methyl-tryptophan; 4-methyl-tryptophan; 5-benzyloxy-tryptophan; 5-bromo-tryptophan; 5-chloro-tryptophan; 5-fluoro-tryptophan; 5-hydroxy-tryptophan; 5-hydroxy-L-tryptophan; 5-methoxy-tryptophan; 5-methoxy-L-tryptophan; 5-methyl-tryptophan; 6-bromo-tryptophan; 6-chloro-D-tryptophan; 6-chloro-tryptophan; 6-fluoro-tryptophan; 6-methyl-tryptophan; 7-benzyloxy-tryptophan; 7-bromo-tryptophan; 7-methyl-- tryptophan; D-1,2,3,4-tetrahydro-norharman-3-carboxylic acid; 6-methoxy-1,2,3,4-tetrahydronorharman-1-carboxylic acid; 7-azatryptophan; L-1,2,3,4-tetrahydro-norharman-3-carboxylic acid; 5-methoxy-2-methyl-tryptophan; and 6-chloro-L-tryptophan.

In some cases, amino acid analogs are racemic. In some cases, the D isomer of the amino acid analog is used. In some cases, the L isomer of the amino acid analog is used. In other cases, the amino acid analog comprises chiral centers that are in the R or S configuration. In still other cases, the amino group(s) of a β-amino acid analog is substituted with a protecting group, e.g., tert-butyloxycarbonyl (BOC group), 9-fluorenylmethyloxycarbonyl (FMOC), tosyl, and the like. In yet other cases, the carboxylic acid functional group of a β-amino acid analog is protected, e.g., as its ester derivative. In some cases the salt of the amino acid analog is used.

A "non-essential" amino acid residue is a residue that can be altered from the wild-type sequence of a polypeptide without abolishing or substantially abolishing its essential biological or biochemical activity (e.g., receptor binding or activation). An "essential" amino acid residue is a residue that, when altered from the wild-type sequence of the polypeptide, results in abolishing or substantially abolishing the polypeptide's essential biological or biochemical activity.

A "conservative amino acid substitution" is one in which the amino acid residue is replaced with an amino acid residue having a similar side chain. Families of amino acid residues having similar side chains have been defined in the art. These families include amino acids with basic side chains (e.g., K, R, H), acidic side chains (e.g., D, E), uncharged polar side chains (e.g., G, N, Q, S, T, Y, C), nonpolar side chains (e.g., A, V, L, I, P, F, M, W), beta-branched side chains (e.g., T, V, I) and aromatic side chains (e.g., Y, F, W, H). Thus, a predicted nonessential amino acid residue in a polypeptide, e.g., is replaced with another amino acid residue from the same side chain family. Other examples of acceptable substitutions are substitutions based on isosteric considerations (e.g., norleucine for methionine) or other properties (e.g., 2-thienylalanine for phenylalanine, or 6-Cl-tryptophan for tryptophan).

The term "capping group" refers to the chemical moiety occurring at either the carboxy or amino terminus of the polypeptide chain of the subject peptidomimetic macrocycle. The capping group of a carboxy terminus includes an unmodified carboxylic acid (i.e. -COOH) or a carboxylic acid with a substituent. For example, the carboxy terminus can be substituted with an amino group to yield a carboxamide at the C-terminus. Various substituents include but are not limited to primary, secondary, and tertiary amines, including pegylated secondary amines. Representative secondary amine capping groups for the C-terminus include:

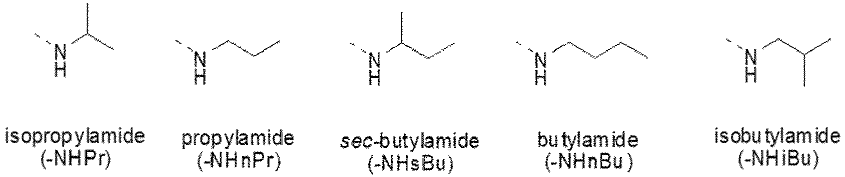

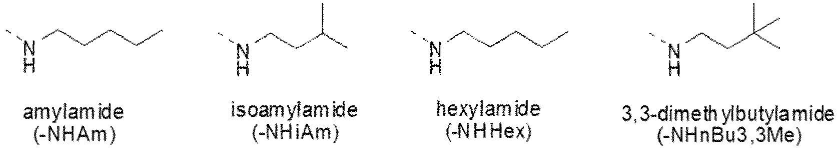

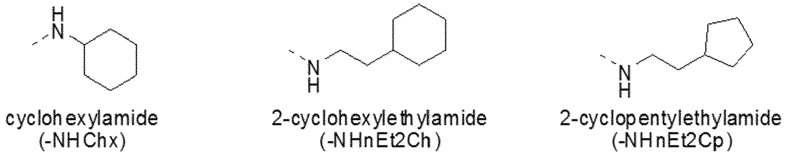

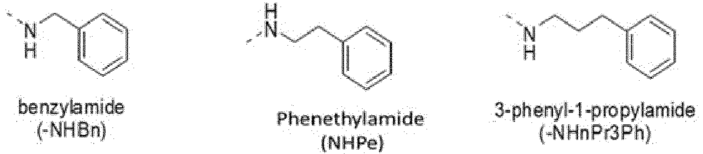

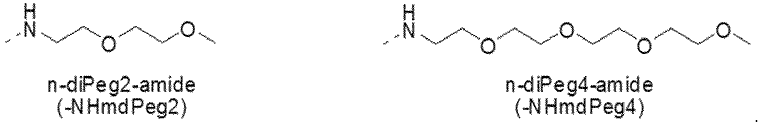

The capping group of an amino terminus includes an unmodified amine (*i. e.* -NH₂) or an amine with a substituent. For example, the amino terminus can be substituted with an acyl group to yield a carboxamide at the N-terminus. Various substituents include but are not limited to substituted acyl groups, including C₁-C₆ carbonyls, C₇-C₃₀ carbonyls, and pegylated carbamates. Representative capping groups for the N-terminus include, but are not limited to, 4-FBzl (4-fluoro-benzyl) and the following:

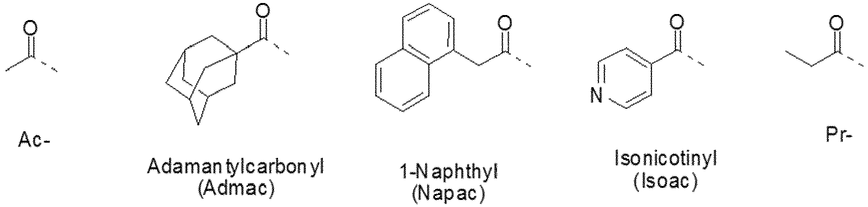

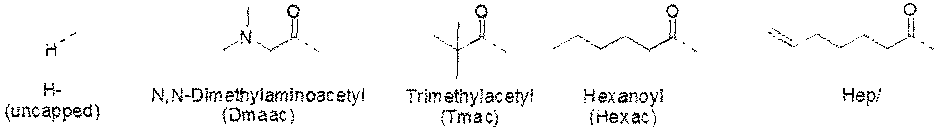

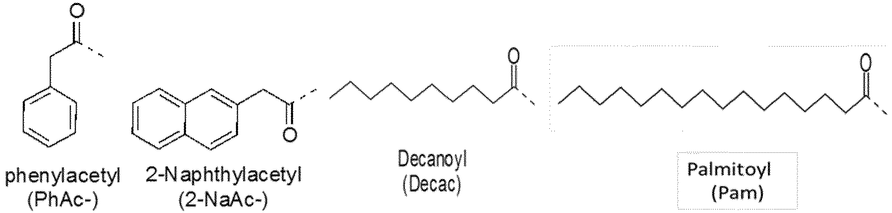

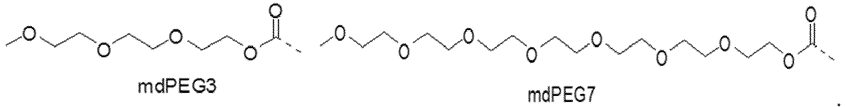

The term "member" as used herein in conjunction with macrocycles or macrocycle-forming linkers refers to the atoms that form or can form the macrocycle, and excludes substituent or side chain atoms. By analogy, cyclodecane, 1,2-difluoro-decane and 1,3-dimethyl cyclodecane are all considered ten-membered macrocycles as the hydrogen or fluoro substituents or methyl side chains do not participate in forming the macrocycle.

The symbol " 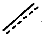 " when used as part of a molecular structure refers to a single bond or a *trans* or *cis* double bond.

The term "amino acid side chain" refers to a moiety attached to the α-carbon (or another backbone atom) in an amino acid. For example, the amino acid side chain for alanine is methyl, the amino acid side chain for phenylalanine is phenylmethyl, the amino acid side chain for cysteine is thiomethyl, the amino acid side chain for aspartate is carboxymethyl, the amino acid side chain for tyrosine is 4-hydroxyphenylmethyl, etc. Other non-naturally occurring amino acid side chains are also included, for example, those that occur in nature (*e.g.,* an amino acid metabolite) or those that are made synthetically *(e.g.,* an α,α di-substituted amino acid).

The term "α,α di-substituted amino" acid refers to a molecule or moiety containing both an amino group and a carboxyl group bound to a carbon (the α-carbon) that is attached to two natural or non-natural amino acid side chains.

The term "polypeptide" encompasses two or more naturally or non-naturally-occurring amino acids joined by a covalent bond *(e.g.,* an amide bond). Polypeptides as described herein include full length proteins *(e.g.,* fully processed proteins) as well as shorter amino acid sequences *(e.g.,* fragments of naturally-occurring proteins or synthetic polypeptide fragments).

The term "first C-terminal amino acid" refers to the amino acid which is closest to the C-terminus. The term "second C-terminal amino acid" refers to the amino acid attached at the N-terminus of the first C-terminal amino acid.

The term "macrocyclization reagent" or "macrocycle-forming reagent" as used herein refers to any reagent which can be used to prepare a peptidomimetic macrocycle by mediating the reaction between two reactive groups. Reactive groups can be, for example, an azide and alkyne, in which case macrocyclization reagents include, without limitation, Cu reagents such as reagents which provide a reactive Cu(I) species, such as CuBr, CuI or CuOTf, as well as Cu(II) salts such as Cu(CO₂CH₃)₂, CuSO₄, and CuCl₂ that can be converted in situ to an active Cu(I) reagent by the addition of a reducing agent such as ascorbic acid or sodium ascorbate. Macrocyclization reagents can additionally include, for example, Ru reagents known in the art such as Cp^{∗}RuCl(PPh₃)₂, [Cp^{∗}RuCl]₄ or other Ru reagents which can provide a reactive Ru(II) species. In other cases, the reactive groups are terminal olefins. In such cases, the macrocyclization reagents or macrocycle-forming reagents are metathesis catalysts including, but not limited to, stabilized, late transition metal carbene complex catalysts such as Group VIII transition metal carbene catalysts. For example, such catalysts are Ru and Os metal centers having a +2 oxidation state, an electron count of 16 and pentacoordinated. In other examples, catalysts have W or Mo centers. Various catalysts are disclosed in Grubbs et al., Acc. Chem. Res. 1995, 28, 446-452, U.S. Pat. No. 5,811,515; U.S. Pat. No. 7,932,397; U.S. Application No. 2011/0065915; U.S. Application No. 2011/0245477; Yu et al., Nature 2011, 479, 88; and Peryshkov et al., J. Am. Chem. Soc. 2011, 133, 20754. In yet other cases, the reactive groups are thiol groups. In such cases, the macrocyclization reagent is, for example, a linker functionalized with two thiol-reactive groups such as halogen groups.

The term "halo" or "halogen" refers to fluorine, chlorine, bromine or iodine or a radical thereof.

The term "alkyl" refers to a hydrocarbon chain that is a straight chain or branched chain, containing the indicated number of carbon atoms. For example, C₁-C₁₀ indicates that the group has from 1 to 10 (inclusive) carbon atoms in it. In the absence of any numerical designation, "alkyl" is a chain (straight or branched) having 1 to 20 (inclusive) carbon atoms in it.

The term "alkylene" refers to a divalent alkyl (*i.e.,* -R-).

The term "alkenyl" refers to a hydrocarbon chain that is a straight chain or branched chain having one or more carbon-carbon double bonds. The alkenyl moiety contains the indicated number of carbon atoms. For example, C₂-C₁₀ indicates that the group has from 2 to 10 (inclusive) carbon atoms in it. The term "lower alkenyl" refers to a C₂-C₆ alkenyl chain. In the absence of any numerical designation, "alkenyl" is a chain (straight or branched) having 2 to 20 (inclusive) carbon atoms in it.

The term "alkynyl" refers to a hydrocarbon chain that is a straight chain or branched chain having one or more carbon-carbon triple bonds. The alkynyl moiety contains the indicated number of carbon atoms. For example, C₂-C₁₀ indicates that the group has from 2 to 10 (inclusive) carbon atoms in it. The term "lower alkynyl" refers to a C₂-C₆ alkynyl chain. In the absence of any numerical designation, "alkynyl" is a chain (straight or branched) having 2 to 20 (inclusive) carbon atoms in it.

The term "aryl" refers to a 6-carbon monocyclic or 10-carbon bicyclic aromatic ring system wherein 0, 1, 2, 3, or 4 atoms of each ring are substituted by a substituent. Examples of aryl groups include phenyl, naphthyl and the like. The term "arylalkoxy" refers to an alkoxy substituted with aryl.

"Arylalkyl" refers to an aryl group, as defined above, wherein one of the aryl group's hydrogen atoms has been replaced with a C₁-C₅ alkyl group, as defined above. Representative examples of an arylalkyl group include, but are not limited to, 2-methylphenyl, 3-methylphenyl, 4-methylphenyl, 2-ethylphenyl, 3-ethylphenyl, 4-ethylphenyl, 2-propylphenyl, 3-propylphenyl, 4-propylphenyl, 2-butylphenyl, 3-butylphenyl, 4-butylphenyl, 2-pentylphenyl, 3-pentylphenyl, 4-pentylphenyl, 2-isopropylphenyl, 3-isopropylphenyl, 4-isopropylphenyl, 2-isobutylphenyl, 3-isobutylphenyl, 4-isobutylphenyl, 2-sec-butylphenyl, 3-sec-butylphenyl, 4-sec-butylphenyl, 2-t-butylphenyl, 3-t-butylphenyl and 4-t-butylphenyl.

"Arylamido" refers to an aryl group, as defined above, wherein one of the aryl group's hydrogen atoms has been replaced with one or more -C(O)NHz groups. Representative examples of an arylamido group include 2-C(O)NH₂-phenyl, 3-C(O)NH₂-phenyl, 4-C(O)NH₂-phenyl, 2-C(O)NH₂-pyridyl, 3-C(O)NH₂-pyridyl, and 4-C(O)NH₂-pyridyl,

"Alkylheterocycle" refers to a C₁-C₅ alkyl group, as defined above, wherein one of the C₁-C₅ alkyl group's hydrogen atoms has been replaced with a heterocycle. Representative examples of an alkylheterocycle group include, but are not limited to, -CHzCHz-morpholine, -CHzCHz-piperidine, -CH₂CH₂CH₂-morpholine, and -CH₂CH₂CH₂-imidazole.

"Alkylamido" refers to a C₁-C₅ alkyl group, as defined above, wherein one of the C₁-C₅ alkyl group's hydrogen atoms has been replaced with a -C(O)NHz group. Representative examples of an alkylamido group include, but are not limited to, -CH₂-C(O)NH₂, -CH₂CH₂-C(O)NH₂, -CH₂CH₂CH₂C(O)NH₂, -CH₂CH₂CH₂CH₂C(O)NH₂, -CH₂CH₂CH₂CH₂CH₂C(O)NH₂, -CH₂CH(C(O)NH₂)CH₃, -CH₂CH(C(O)NH₂)CH₂CH₃, -CH(C(O)NH₂)CH₂CH₃, -C(CH₃)₂CH₂C(O)NH₂, -CH₂-CH₂-NH-C(O)-CH₃, -CH₂-CH₂-NH-C(O)-CH₃-CH3, and -CH₂-CH₂-NH-C(O)-CH=CH₂.

"Alkanol" refers to a C₁-C₅ alkyl group, as defined above, wherein one of the C₁-C₅ alkyl group's hydrogen atoms has been replaced with a hydroxyl group. Representative examples of an alkanol group include, but are not limited to, -CH₂OH, -CH₂CH₂OH, -CH₂CH₂CH₂OH, -CH₂CH₂CH₂CH₂OH, -CH₂CH₂CH₂ CH₂CH₂OH, -CH₂CH(OH)CH₃, -CH₂CH(OH)CH₂CH₃, -CH(OH)CH₃ and -C(CH₃)₂CH₂OH.

"Alkylcarboxy" refers to a C₁-C₅ alkyl group, as defined above, wherein one of the C₁-C₅ alkyl group's hydrogen atoms has been replaced with a --COOH group. Representative examples of an alkylcarboxy group include, but are not limited to, -CH₂COOH, -CH₂CH₂COOH, -CH₂CH₂CH₂COOH, -CH₂CH₂CH₂CH₂COOH, -CH₂CH(COOH)CH₃, -CH₂CH₂CH₂CH₂CH₂COOH, -CH₂CH(COOH)CH₂CH₃, -CH(COOH)CH₂CH₃ and -C(CH₃)₂CH₂COOH.

The term "cycloalkyl" as employed herein includes saturated and partially unsaturated cyclic hydrocarbon groups having 3 to 12 carbons, preferably 3 to 8 carbons, and more preferably 3 to 6 carbons, wherein the cycloalkyl group additionally is optionally substituted. Some cycloalkyl groups include, without limitation, cyclopropyl, cyclobutyl, cyclopentyl, cyclopentenyl, cyclohexyl, cyclohexenyl, cycloheptyl, and cyclooctyl.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of O, N, or S if monocyclic, bicyclic, or tricyclic, respectively), wherein 0, 1, 2, 3, or 4 atoms of each ring are substituted by a substituent. Examples of heteroaryl groups include pyridyl, furyl or furanyl, imidazolyl, benzimidazolyl, pyrimidinyl, thiophenyl or thienyl, quinolinyl, indolyl, thiazolyl, and the like.

The term "heteroarylalkyl" or the term "heteroaralkyl" refers to an alkyl substituted with a heteroaryl. The term "heteroarylalkoxy" refers to an alkoxy substituted with heteroaryl.

The term "heteroarylalkyl" or the term "heteroaralkyl" refers to an alkyl substituted with a heteroaryl. The term "heteroarylalkoxy" refers to an alkoxy substituted with heteroaryl.

The term "heterocyclyl" refers to a nonaromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (*e*.*g*., carbon atoms and 1-3, 1-6, or 1-9 heteroatoms of O, N, or S if monocyclic, bicyclic, or tricyclic, respectively), wherein 0, 1, 2 or 3 atoms of each ring are substituted by a substituent. Examples of heterocyclyl groups include piperazinyl, pyrrolidinyl, dioxanyl, morpholinyl, tetrahydrofuranyl, and the like.

The term "substituent" refers to a group replacing a second atom or group such as a hydrogen atom on any molecule, compound or moiety. Suitable substituents include, without limitation, halo, hydroxy, mercapto, oxo, nitro, haloalkyl, alkyl, alkaryl, aryl, aralkyl, alkoxy, thioalkoxy, aryloxy, amino, alkoxycarbonyl, amido, carboxy, alkanesulfonyl, alkylcarbonyl, and cyano groups.

In some cases, the compounds disclosed herein contain one or more asymmetric centers and thus occur as racemates and racemic mixtures, single enantiomers, individual diastereomers and diastereomeric mixtures. All such isomeric forms of these compounds are included unless expressly described otherwise. In some cases, the compounds disclosed herein are also represented in multiple tautomeric forms, in such instances, the compounds include all tautomeric forms of the compounds described herein *(e.g.,* if alkylation of a ring system results in alkylation at multiple sites, the invention includes all such reaction products). All such isomeric forms of such compounds are included unless expressly described otherwise. All crystal forms of the compounds described herein are included unless expressly described otherwise.

As used herein, the terms "increase" and "decrease" mean, respectively, to cause a statistically significantly (*i*.*e*., p < 0.1) increase or decrease of at least 5%.

As used herein, the recitation of a numerical range for a variable is intended to convey that the variable is equal to any of the values within that range. Thus, for a variable which is inherently discrete, the variable is equal to any integer value within the numerical range, including the end-points of the range. Similarly, for a variable which is inherently continuous, the variable is equal to any real value within the numerical range, including the end-points of the range. As an example, and without limitation, a variable which is described as having values between 0 and 2 takes the values 0, 1 or 2 if the variable is inherently discrete, and takes the values 0.0, 0.1, 0.01, 0.001, or any other real values ≥ 0 and ≤ 2 if the variable is inherently continuous.

As used herein, unless specifically indicated otherwise, the word "or" is used in the inclusive sense of "and/or" and not the exclusive sense of "either/or."

The term "on average" represents the mean value derived from performing at least three independent replicates for each data point.

The term "biological activity" encompasses structural and functional properties of a macrocycle. Biological activity is, for example, structural stability, alpha-helicity, affinity for a target, resistance to proteolytic degradation, cell penetrability, intracellular stability, *in vivo* stability, or any combination thereof.

The term "binding affinity" refers to the strength of a binding interaction, for example between a peptidomimetic macrocycle and a target. Binding affinity can be expressed, for example, as an equilibrium dissociation constant ("K_{D}"), which is expressed in units which are a measure of concentration (e.g. M, mM, µM, nM etc). Numerically, binding affinity and K_{D} values vary inversely, such that a lower binding affinity corresponds to a higher K_{D} value, while a higher binding affinity corresponds to a lower K_{D} value. Where high binding affinity is desirable, "improved" binding affinity refers to higher binding affinity and therefore lower K_{D} values.

The term *"in vitro* efficacy" refers to the extent to which a test compound, such as a peptidomimetic macrocycle, produces a beneficial result in an *in vitro* test system or assay. *In vitro* efficacy can be measured, for example, as an "IC₅₀" or "EC₅₀" value, which represents the concentration of the test compound which produces 50% of the maximal effect in the test system.

The term "ratio *of in vitro* efficacies" or *"in vitro* efficacy ratio" refers to the ratio of IC₅₀ or EC₅₀ values from a first assay (the numerator) versus a second assay (the denominator). Consequently, an improved *in vitro* efficacy ratio for Assay 1 versus Assay 2 refers to a lower value for the ratio expressed as ICso(Assay 1)/ICso(Assay 2) or alternatively as EC₅₀(Assay 1)/ECso(Assay 2). This concept can also be characterized as "improved selectivity" in Assay 1 versus Assay 2, which can be due either to a decrease in the IC₅₀ or EC₅₀ value for Target 1 or an increase in the value for the IC₅₀ or EC₅₀ value for Target 2.

Solid tumor cancers that can be treated with the peptidomimetic macrocycle and at least one additional pharmaceutically active agent as described herein include sarcomas, carcinomas and lymphomas. The term "solid tumor" or "solid cancer" as used herein refers to tumors that usually do not contain cysts or liquid areas. In various cases, leukemia (cancer of blood) is not solid tumor.

Solid tumor cancers that can be treated by the methods described herein include, but are not limited to, sarcomas, carcinomas, and lymphomas. In specific cases, solid tumors that can be treated in accordance with the methods described include, but are not limited to, cancer of the breast, liver, neuroblastoma, head, neck, eye, mouth, throat, esophagus, esophagus, chest, bone, lung, kidney, colon, rectum or other gastrointestinal tract organs, stomach, spleen, skeletal muscle, subcutaneous tissue, prostate, breast, ovaries, testicles or other reproductive organs, skin, thyroid, blood, lymph nodes, kidney, liver, pancreas, and brain or central nervous system. Solid tumors that can be treated by the instant methods include tumors and/or metastasis (wherever located) other than lymphatic cancer, for example brain and other central nervous system tumors (including but not limited to tumors of the meninges, brain, spinal cord, cranial nerves and other parts of central nervous system, e.g. glioblastomas or medulla blastemas); head and/or neck cancer; breast tumors; circulatory system tumors (including but not limited to heart, mediastinum and pleura, and other intrathoracic organs, vascular tumors and tumor-associated vascular tissue); excretory system tumors (including but not limited to tumors of kidney, renal pelvis, ureter, bladder, other and unspecified urinary organs); gastrointestinal tract tumors (including but not limited to tumors of oesophagus, stomach, small intestine, colon, colorectal, rectosigmoid junction, rectum, anus and anal canal, tumors involving the liver and intrahepatic bile ducts, gall bladder, other and unspecified parts of biliary tract, pancreas, other and digestive organs); oral cavity tumors (including but not limited to tumors of lip, tongue, gum, floor of mouth, palate, and other parts of mouth, parotid gland, and other parts of the salivary glands, tonsil, oropharynx, nasopharynx, pyriform sinus, hypopharynx, and other sites in the lip, oral cavity and pharynx); reproductive system tumors (including but not limited to tumors of vulva, vagina, Cervix uteri, Corpus uteri, uterus, ovary, and other sites associated with female genital organs, placenta, penis, prostate, testis, and other sites associated with male genital organs); respiratory tract tumors (including but not limited to tumors of nasal cavity and middle ear, accessory sinuses, larynx, trachea, bronchus and lung, e.g. small cell lung cancer or non-small cell lung cancer); skeletal system tumors (including but not limited to tumors of bone and articular cartilage of limbs, bone articular cartilage and other sites); skin tumors (including but not limited to malignant melanoma of the skin, non-melanoma skin cancer, basal cell carcinoma of skin, squamous cell carcinoma of skin, mesothelioma, Kaposi's sarcoma); and tumors involving other tissues including peripheral nerves and autonomic nervous system, connective and soft tissue, retroperitoneum and peritoneum, eye and adnexa, thyroid, adrenal gland and other endocrine glands and related structures, secondary and unspecified malignant neoplasm of lymph nodes, secondary malignant neoplasm of respiratory and digestive systems and secondary malignant neoplasm of other sites.

In some examples, the solid tumor treated by the methods of the instant disclosure is pancreatic cancer, bladder cancer, colon cancer, liver cancer, colorectal cancer (colon cancer or rectal cancer), breast cancer, prostate cancer, renal cancer, hepatocellular cancer, lung cancer, ovarian cancer, cervical cancer, gastric cancer, esophageal cancer, head and neck cancer, melanoma, neuroendocrine cancers, CNS cancers, brain tumors, bone cancer, skin cancer, ocular tumor, choriocarcinoma (tumor of the placenta), sarcoma or soft tissue cancer.

In some examples, the solid tumor to be treated by the methods of the instant disclosure is selected bladder cancer, bone cancer, breast cancer, cervical cancer, CNS cancer, colon cancer, ocular tumor, renal cancer, liver cancer, lung cancer, pancreatic cancer, choriocarcinoma (tumor of the placenta), prostate cancer, sarcoma, skin cancer, soft tissue cancer or gastric cancer.

In some examples, the solid tumor treated by the methods of the instant disclosure is breast cancer. Non limiting examples of breast cancer that can be treated by the instant methods include ductal carcinoma in situ (DCIS or intraductal carcinoma), lobular carcinoma in situ (LCIS), invasive (or infiltrating) ductal carcinoma, invasive (or infiltrating) lobular carcinoma, inflammatory breast cancer, triple-negative breast cancer, paget disease of the nipple, phyllodes tumor (phylloides tumor or cystosarcoma phyllodes), angiosarcoma, adenoid cystic (or adenocystic) carcinoma, low-grade adenosquamous carcinoma, medullary carcinoma, papillary carcinoma, tubular carcinoma, metaplastic carcinoma, micropapillary carcinoma, and mixed carcinoma.

In some examples, the solid tumor treated by the methods of the instant disclosure is bone cancer. Non limiting examples of bone cancer that can be treated by the instant methods include osteosarcoma, chondrosarcoma, the Ewing Sarcoma Family of Tumors (ESFTs).

In some examples, the solid tumor treated by the methods of the instant disclosure is skin cancer. Non limiting examples of skin cancer that can be treated by the instant methods include melanoma, basal cell skin cancer, and squamous cell skin cancer.

In some examples, the solid tumor treated by the methods of the instant disclosure is ocular tumor. Non limiting examples of ocular tumor that can be treated by the methods of the instant disclosure include ocular tumor is choroidal nevus, choroidal melanoma, choroidal metastasis, choroidal hemangioma, choroidal osteoma, iris melanoma, uveal melanoma, intraocular lymphoma, melanocytoma, metastasis retinal capillary hemangiomas, congenital hypertrophy of the RPE, RPE adenoma or retinoblastoma.

In some cases solid tumors treated by the methods disclosed herein exclude cancers that are known to be associated with HPV (Human papillomavirus). The excluded group includes HPV positive cervical cancer, HPV positive anal cancer, and HPV head and neck cancers, such as oropharyngeal cancers.

The term "liquid cancer" as used herein refers to cancer cells that are present in body fluids, such as blood, lymph and bone marrow. Liquid cancers include leukemia, myeloma and liquid lymphomas. Liquid lymphomas include lymphomas that contain cysts or liquid areas. Liquid cancers as used herein do not include solid tumors, such as sarcomas and carcinomas or solid lymphomas that do not contain contain cysts or liquid areas.

Liquid cancers that can be treated with the describedpeptidomimetic macrocycle and at least one additional pharmaceutically active agent as described herein include, but are not limited to, leukemias, myelomas, and liquid lymphomas. In specific cases, liquid cancers that can be treated in accordance with the methods described include, but are not limited to, liquid lymphomas, lekemias, and myelomas. Exemplary liquid lymphomas and leukemias that can be treated in accordance with the methods described include, but are not limited to, chronic lymphocytic leukemialsmall lymphocytic lymphoma, B-cell prolymphocytic leukemia, lymphoplasmacytic lymphoma (such as waldenström macroglobulinemia), splenic marginal zone lymphoma, plasma cell myeloma, plasmacytoma, monoclonal immunoglobulin deposition diseases, heavy chain diseases, extranodal marginal zone B cell lymphoma, also called malt lymphoma, nodal marginal zone B cell lymphoma (nmzl), follicular lymphoma, mantle cell lymphoma, diffuse large B cell lymphoma, mediastinal (thymic) large B cell lymphoma, intravascular large B cell lymphoma, primary effusion lymphoma, burkitt lymphomalleukemia, T cell prolymphocytic leukemia, T cell large granular lymphocytic leukemia, aggressive NK cell leukemia, adult T cell leukemia/lymphoma, extranodal NK/T cell lymphoma, nasal type, enteropathy-type T cell lymphoma, hepatosplenic T cell lymphoma, blastic NK cell lymphoma, mycosis fungoides / sezary syndrome, primary cutaneous CD30-positive T cell lymphoproliferative disorders, primary cutaneous anaplastic large cell lymphoma, lymphomatoid papulosis, angioimmunoblastic T cell lymphoma, peripheral T cell lymphoma, unspecified, anaplastic large cell lymphoma, classical Hodgkin lymphomas (nodular sclerosis, mixed cellularity, lymphocyte-rich, lymphocyte depleted or not depleted), and nodular lymphocyte-predominant Hodgkin lymphoma.

Examples of liquid cancers include cancers involving hyperplastic/neoplastic cells of hematopoietic origin, e.g., arising from myeloid, lymphoid or erythroid lineages, or precursor cells thereof. Exemplary disorders include: acute leukemias, e.g., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L. (1991) Crit Rev. in Oncol./Hemotol. 11:267-97); lymphoid malignancies include, but are not limited to acute lymphoblastic leukemia (ALL) which includes B- lineage ALL and T-lineage ALL, chronic lymphocytic leukemia (CLL), prolymphocytic leukemia (PLL), multiple mylenoma, hairy cell leukemia (HLL) and Waldenstrom's macroglobulinemia (WM). Additional forms of malignant liquid lymphomas include, but are not limited to non-Hodgkin lymphoma and variants thereof, adult T cell leukemiallymphoma (ATL), cutaneous T-cell lymphoma (CTCL), periphieral T-cell lymphoma (PTCL), large granular lymphocytic leukemia (LGF), Hodgkin's disease and Reed-Sternberg disease. For example, liquid cancers include, but are not limited to, acute lymphocytic leukemia (ALL); T-cell acute lymphocytic leukemia (T-ALL); anaplastic large cell lymphoma (ALCL); chronic myelogenous leukemia (CML); acute myeloid leukemia (AML); chronic lymphocytic leukemia (CLL); B-cell chronic lymphocytic leukemia (B-CLL); diffuse large B-cell lymphomas (DLBCL); hyper eosinophilia / chronic eosinophilia; and Burkitt's lymphoma.

In cases, the cancer comprises an acute lymphoblastic leukemia; acute myeloid leukemia; AIDS-related cancers; AIDS-related lymphoma; chronic lymphocytic leukemia; chronic myelogenous leukemia; chronic myeloproliferative disorders; adult T cell leukemia/lymphoma (ATL), cutaneous T-cell lymphoma (CTCL), periphieral T-cell lymphoma (PTCL); Hodgkin lymphoma; multiple myeloma; multiple myelomalplasma cell neoplasm; Non-Hodgkin lymphoma; or primary central nervous system (CNS) lymphoma. In various cases, the liquid cancer can be B-cell chronic lymphocytic leukemia, B-cell lymphoma-DLBCL, B-cell lymphoma-DLBCL-germinal center-like, B-cell lymphoma-DLBCL-activated B-cell-like, or Burkitt's lymphoma.

In some cases, a subject treated with the peptidomimetic macrocycle and at least one additional pharmaceutically active agent as described herein is a human who has or is diagnosed with cancer lacking p53 deactivating mutation and/or expressing wild type p53. In some cases, a subject treated for cancer in accordance with the methods described herein is a human predisposed or susceptible to cancer lacking p53 deactivating mutation and/or expressing wild type p53. In some cases, a subject treated for cancer in accordance with the methods described herein is a human at risk of developing cancer lacking p53 deactivating mutation and/or expressing wild type p53. A p53 deactivating mutation in some example can be a mutation in DNA-binding domain of the p53 protein. In some examples the p53 deactivating mutation can be a missense mutation. In various examples, the cancer can be determined to lack one or more p53 deactivating mutations selected from mutations at one or more of residues R175, G245, R248, R249, R273, and R282. The lack of p53 deactivating mutation and/or the presence of wild type p53 in the cancer can be determined by any suitable method known in art, for example by sequencing, array based testing, RNA analysis and amplifications methods like PCR.

In certain cases, the human subject is refractory and/or intolerant to one or more other standard treatment of the cancer known in art. In some cases, the human subject has had at least one unsuccessful prior treatment and/or therapy of the cancer.

In some cases, a subject treated with the peptidomimetic macrocycle and at least one additional pharmaceutically active agent as described herein is a human, who has or is diagnosed with a tumor. In other cases, a subject treated for tumor in accordance with the methods described herein is a human, predisposed or susceptible to a tumor. In some cases, a subject treated for tumor in accordance with the methods described herein is a human, at risk of developing a tumor.

In some cases, a subject treated with the peptidomimetic macrocycle and at least one additional pharmaceutically active agent as describeddescribed herein is a human, who has or is diagnosed with a tumor, determined to lack a p53 deactivating mutation and/or expressing wild type p53. In other cases, a subject treated for tumor in accordance with the methods described herein is a human, predisposed or susceptible to a tumor, determined to lack a p53 deactivating mutation and/or expressing wild type p53. In some cases, a subject treated for tumor in accordance with the methods described herein is a human, at risk of developing a tumor, determined to lack a p53 deactivating mutation and/or expressing wild type p53. A p53 deactivating mutation, as used herein is any mutation that leads to loss of (or a decrease in) the *in vitro* apoptotic activity of p53.

In some cases, the subject treated with the peptidomimetic macrocycle and at least one additional pharmaceutically active agent as described herein is a human, who has or is diagnosed with a tumor, determined to have a p53 gain of function mutation. In other cases, a subject treated for tumor in accordance with the methods described herein is a human, predisposed or susceptible to a tumor, determined to have a p53 gain of function mutation. In some cases, a subject treated for tumor in accordance with the methods described herein is a human, at risk of developing a tumor, determined to have a p53 gain of function mutation. A p53 gain of function mutation, as used herein is any mutation such that the mutant p53 exerts oncogenic functions beyond their negative domination over the wild-type p53 tumor suppressor functions. The p53 gain of function mutant protein mat exhibit new activities that can contribute actively to various stages of tumor progression and to increased resistance to anticancer treatments. Accordingly, in some cases, a subject with a tumor in accordance with the composition as described herein is a human who has or is diagnosed with a tumor that is determined to have a p53 gain of function mutation.

In some cases, the subject treated with the peptidomimetic macrocycle and at least one additional pharmaceutically active agent as describedherein is a human, who has or is diagnosed with a tumor that is not p53 negative. In other cases, a subject treated for tumor in accordance with the methods described herein is a human, predisposed or susceptible to a tumor that is not p53 negative. In some cases, a subject treated for tumor in accordance with the methods described herein is a human, at risk of developing a tumor that is not p53 negative.

In some cases, the subject treated with the peptidomimetic macrocycle and at least one additional pharmaceutically active agent as describedd herein is a human, who has or is diagnosed with a tumor that expresses p53 with partial loss of function mutation. In other cases, a subject treated for tumor in accordance with the methods described herein is a human, predisposed or susceptible to a tumor that expresses p53 with partial loss of function mutation. In some cases, a subject treated for tumor in accordance with the methods described herein is a human, at risk of developing a tumor that expresses p53 with partial loss of function mutation. As used herein "a partial loss of p53 function" mutation means that the mutant p53 exhibits some level of function of normal p53, but to a lesser or slower extent. For example, a partial loss of p53 function can mean that the cells become arrested in cell division to a lesser or slower extent.

In some cases, the subject treated with the peptidomimetic macrocycle and at least one additional pharmaceutically active agent as described herein is a human, who has or is diagnosed with a tumor that expresses p53 with a copy loss mutation and a deactivating mutation. In other cases, a subject treated for tumor in accordance with the methods described herein is a human, predisposed or susceptible to a tumor that expresses p53 with a copy loss mutation and a deactivating mutation. In some cases, a subject treated for tumor in accordance with the methods described herein is a human, at risk of developing a tumor that expresses p53 with a copy loss mutation and a deactivating mutation. [

In some cases, the subject treated with the peptidomimetic macrocycle and at least one additional pharmaceutically active agent as described herein is a human, who has or is diagnosed with a tumor that expresses p53 with a copy loss mutation. In other cases, a subject treated for tumor in accordance with the methods described herein is a human, predisposed or susceptible to a tumor that expresses p53 with a copy loss mutation. In some cases, a subject treated for tumor in accordance with the methods described herein is a human, at risk of developing a tumor that expresses p53 with a copy loss mutation.

In some cases, the subject treated with the peptidomimetic macrocycle and at least one additional pharmaceutically active agent as described herein is a human, who has or is diagnosed with a tumor that expresses p53 with one or more silent mutations. In other cases, a subject treated for tumor in accordance with the methods described herein is a human, predisposed or susceptible to a tumor that expresses p53 with one or more silent mutations. In some cases, a subject treated for tumor in accordance with the methods described herein is a human, at risk of developing a tumor that expresses p53 with one or more silent mutations. Silent mutations as used herein are mutations which cause no change in the encoded p53 amino acid sequence.

In some cases, a subject treated with the peptidomimetic macrocycle and at least one additional pharmaceutically active agent as described herein is a human, who has or is diagnosed with a tumor, determined to lack a dominant p53 deactivating mutation. Dominant p53 deactivating mutation or dominant negative mutation, as used herein, is a mutation wherein the mutated p53 inhibits or disrupt the activity of the wild-type p53 gene.

The details of one or more particular cases of the invention are set forth in the accompanying drawings and the description below. Other features, objects, and advantages of the invention will be apparent from the description and drawings, and from the claims. Pharmaceutically-acceptable salts

The invention provides the use of pharmaceutically-acceptable salts of any therapeutic compound described in the claims Pharmaceutically-acceptable salts include, for example, acid-addition salts and base-addition salts. The acid that is added to the compound to form an acid-addition salt can be an organic acid or an inorganic acid. A base that is added to the compound to form a base-addition salt can be an organic base or an inorganic base. In some cases, a pharmaceutically-acceptable salt is a metal salt. In some cases, a pharmaceutically-acceptable salt is an ammonium salt.

Metal salts can arise from the addition of an inorganic base to a compound of the invention. The inorganic base consists of a metal cation paired with a basic counterion, such as, for example, hydroxide, carbonate, bicarbonate, or phosphate. The metal can be an alkali metal, alkaline earth metal, transition metal, or main group metal. In some cases, the metal is lithium, sodium, potassium, cesium, cerium, magnesium, manganese, iron, calcium, strontium, cobalt, titanium, aluminum, copper, cadmium, or zinc.

In some cases, a metal salt is a lithium salt, a sodium salt, a potassium salt, a cesium salt, a cerium salt, a magnesium salt, a manganese salt, an iron salt, a calcium salt, a strontium salt, a cobalt salt, a titanium salt, an aluminum salt, a copper salt, a cadmium salt, or a zinc salt.

Ammonium salts can arise from the addition of ammonia or an organic amine to a compound of the invention. In some cases, the organic amine is triethyl amine, diisopropyl amine, ethanol amine, diethanol amine, triethanol amine, morpholine, N-methylmorpholine, piperidine, N-methylpiperidine, N-ethylpiperidine, dibenzylamine, piperazine, pyridine, pyrrazole, pipyrrazole, imidazole, pyrazine, or pipyrazine.

In some cases, an ammonium salt is a triethyl amine salt, a diisopropyl amine salt, an ethanol amine salt, a diethanol amine salt, a triethanol amine salt, a morpholine salt, an N-methylmorpholine salt, a piperidine salt, an N-methylpiperidine salt, an N-ethylpiperidine salt, a dibenzylamine salt, a piperazine salt, a pyridine salt, a pyrrazole salt, a pipyrrazole salt, an imidazole salt, a pyrazine salt, or a pipyrazine salt.

Acid addition salts can arise from the addition of an acid to a compound of the invention. In some cases, the acid is organic. In some cases, the acid is inorganic. In some cases, the acid is hydrochloric acid, hydrobromic acid, hydroiodic acid, nitric acid, nitrous acid, sulfuric acid, sulfurous acid, a phosphoric acid, isonicotinic acid, lactic acid, salicylic acid, tartaric acid, ascorbic acid, gentisinic acid, gluconic acid, glucaronic acid, saccaric acid, formic acid, benzoic acid, glutamic acid, pantothenic acid, acetic acid, propionic acid, butyric acid, fumaric acid, succinic acid, methanesulfonic acid, ethanesulfonic acid, benzenesulfonic acid, p-toluenesulfonic acid, citric acid, oxalic acid, or maleic acid.

In some cases, the salt is a hydrochloride salt, a hydrobromide salt, a hydroiodide salt, a nitrate salt, a nitrite salt, a sulfate salt, a sulfite salt, a phosphate salt, isonicotinate salt, a lactate salt, a salicylate salt, a tartrate salt, an ascorbate salt, a gentisinate salt, a gluconate salt, a glucaronate salt, a saccarate salt, a formate salt, a benzoate salt, a glutamate salt, a pantothenate salt, an acetate salt, a propionate salt, a butyrate salt, a fumarate salt, a succinate salt, a methanesulfonate (mesylate) salt, an ethanesulfonate salt, a benzenesulfonate salt, a p-toluenesulfonate salt, a citrate salt, an oxalate salt, or a maleate salt.

### Purity of Compounds of the Invention

Any compound herein can be purified. A compound herein can be least 1% pure, at least 2% pure, at least 3% pure, at least 4% pure, at least 5% pure, at least 6% pure, at least 7% pure, at least 8% pure, at least 9% pure, at least 10% pure, at least 11% pure, at least 12% pure, at least 13% pure, at least 14% pure, at least 15% pure, at least 16% pure, at least 17% pure, at least 18% pure, at least 19% pure, at least 20% pure, at least 21% pure, at least 22% pure, at least 23% pure, at least 24% pure, at least 25% pure, at least 26% pure, at least 27% pure, at least 28% pure, at least 29% pure, at least 30% pure, at least 31% pure, at least 32% pure, at least 33% pure, at least 34% pure, at least 35% pure, at least 36% pure, at least 37% pure, at least 38% pure, at least 39% pure, at least 40% pure, at least 41% pure, at least 42% pure, at least 43% pure, at least 44% pure, at least 45% pure, at least 46% pure, at least 47% pure, at least 48% pure, at least 49% pure, at least 50% pure, at least 51% pure, at least 52% pure, at least 53% pure, at least 54% pure, at least 55% pure, at least 56% pure, at least 57% pure, at least 58% pure, at least 59% pure, at least 60% pure, at least 61% pure, at least 62% pure, at least 63% pure, at least 64% pure, at least 65% pure, at least 66% pure, at least 67% pure, at least 68% pure, at least 69% pure, at least 70% pure, at least 71% pure, at least 72% pure, at least 73% pure, at least 74% pure, at least 75% pure, at least 76% pure, at least 77% pure, at least 78% pure, at least 79% pure, at least 80% pure, at least 81% pure, at least 82% pure, at least 83% pure, at least 84% pure, at least 85% pure, at least 86% pure, at least 87% pure, at least 88% pure, at least 89% pure, at least 90% pure, at least 91% pure, at least 92% pure, at least 93% pure, at least 94% pure, at least 95% pure, at least 96% pure, at least 97% pure, at least 98% pure, at least 99% pure, at least 99.1% pure, at least 99.2% pure, at least 99.3% pure, at least 99.4% pure, at least 99.5% pure, at least 99.6% pure, at least 99.7% pure, at least 99.8% pure, or at least 99.9% pure.

### Formulation and Administration

### Mode of Administration

An effective amount of a peptidomimetic macrocycles of the disclosure can be administered in either single or multiple doses by any of the accepted modes of administration. In some cases, the peptidomimetic macrocycles of the disclosure are administered parenterally, for example, by subcutaneous, intramuscular, intrathecal, intravenous or epidural injection. For example, the peptidomimetic macrocycle is administered intravenously, intraarterially, subcutaneously or by infusion. In some examples, the peptidomimetic macrocycle is administered intravenously. In some examples, the peptidomimetic macrocycle is administered intraarterially.

Regardless of the route of administration selected, the peptidomimetic macrocycles of the present disclosure, and/or the pharmaceutical compositions of the present disclosure, are formulated into pharmaceutically-acceptable dosage forms. The peptidomimetic macrocycles according to the disclosure can be formulated for administration in any convenient way for use in human or veterinary medicine, by analogy with other pharmaceuticals.

In one aspect, the disclosure provides pharmaceutical formulation comprising a therapeutically-effective amount of one or more of the peptidomimetic macrocycles described above, formulated together with one or more pharmaceutically acceptable carriers (additives) and/or diluents. In one case, one or more of the peptidomimetic macrocycles described herein are formulated for parenteral administration for parenteral administration, one or more peptidomimetic macrocycles disclosed herein can be formulated as aqueous or nonaqueous solutions, dispersions, suspensionsor emulsions or sterile powders which can be reconstituted into sterile injectable solutions or dispersions just prior to use. Such formulations can comprise sugars, alcohols, antioxidants, buffers, bacteriostats, solutes which render the formulation isotonic with the blood of the intended recipient or suspending or thickening agents. These compositions can also contain adjuvants such as preservatives, wetting agents, emulsifying agents and dispersing agents. Prevention of the action of microorganisms upon the subject compounds can be ensured by the inclusion of various antibacterial and antifungal agents, for example, paraben, chlorobutanol, phenol sorbic acid, and the like. It can also be desirable to include isotonic agents, such as sugars, sodium chloride, and the like into the compositions. In addition, prolonged absorption of the injectable pharmaceutical form can be brought about by the inclusion of agents which delay absorption such as aluminum monostearate and gelatin. If desired the formulation can be diluted prior to use with, for example, an isotonic saline solution or a dextrose solution. In some examples, the peptidomimetic macrocycle is formulated as an aqueous solution and is administered intravenously.

### Amount and frequency of administration

Dosing can be determined using various techniques. The selected dosage level can depend upon a variety of factors including the activity of the particular peptidomimetic macrocycle employed, the route of administration, the time of administration, the rate of excretion or metabolism of the particular peptidomimetic macrocycle being employed, the duration of the treatment, other drugs, compounds and/or materials used in combination with the particular peptidomimetic macrocycle employed, the age, sex, weight, condition, general health and prior medical history of the patient being treated, and like factors well known in the medical arts. The dosage values can also vary with the severity of the condition to be alleviated. For any particular subject, specific dosage regimens can be adjusted over time according to the individual need and the professional judgment of the person administering or supervising the administration of the compositions.

A physician or veterinarian can prescribe the effective amount of the pharmaceutical composition required. For example, the physician or veterinarian could start doses of the compounds of the disclosure employed in the pharmaceutical composition at levels lower than that required in order to achieve the desired therapeutic effect and gradually increase the dosage until the desired effect is achieved.

In some cases, a suitable daily dose of a peptidomimetic macrocycle of the disclosure can be that amount of the peptidomimetic macrocycle which is the lowest dose effective to produce a therapeutic effect. Such an effective dose will generally depend upon the factors described above. The precise time of administration and amount of any particular peptidomimetic macrocycle that will yield the most effective treatment in a given patient will depend upon the activity, pharmacokinetics, and bioavailability of a particular peptidomimetic macrocycle, physiological condition of the patient (including age, sex, disease type and stage, general physical condition, responsiveness to a given dosage and type of medication), route of administration, and the like.

Dosage can be based on the amount of the peptidomimetic macrocycle per kg body weight of the patient. Alternatively, the dosage of the subject disclosure can be determined by reference to the plasma concentrations of the peptidomimetic macrocycle. For example, the maximum plasma concentration (Cmax) and the area under the plasma concentration-time curve from time 0 to infinity (AUC) can be used.

In some cases, the subject is a human subject and the amount of the peptidomimetic macrocycle administered is 0.01-100 mg per kilogram body weight of the human subject. For example, in various examples, the amount of the peptidomimetic macrocycle administered is about .01-50 mg/kg, about 0.01-20 mg/kg, about 0.01-10 mg/kg, about 0.1-100 mg/kg, about 0.1-50 mg/kg, about 0.1-20 mg/kg, about 0.1-10 mg/kg, about 0.5-100 mg/kg, about 0.5-50 mg/kg, about 0.5-20 mg/kg, about 0.5-10 mg/kg, about 1-100 mg/kg, about 1-50 mg/kg, about 1-20 mg/kg, about 1-10 mg/kg body weight of the human subject. In one case, about 0.5 mg-10 mg of the peptidomimetic macrocycle per kilogram body weight of the human subject is administered. In some examples the amount of the peptidomimetic macrocycle administered is about 0.16 mg, about 0.32 mg, about 0.64 mg, about 1.28 mg, about 3.56 mg, about 7.12 mg, about 14.24 mg, or about 20 mg per kilogram body weight of the human subject. In some examples the amount of the peptidomimetic macrocycle administered is about 0.16 mg, about 0.32 mg, about 0.64 mg, about 1.28 mg, about 3.56 mg, about 7.12 mg, or about 14.24 mg per kilogram body weight of the human subject. In some examples the amount of the peptidomimetic macrocycle administered is about 0.16 mg per kilogram body weight of the human subject. In some examples the amount of the peptidomimetic macrocycle administered is about 0.32 mg per kilogram body weight of the human subject. In some examples the amount of the peptidomimetic macrocycle administered is about 0.64 mg per kilogram body weight of the human subject. In some examples the amount of the peptidomimetic macrocycle administered is about 1.28 mg per kilogram body weight of the human subject. In some examples the amount of the peptidomimetic macrocycle administered is about 3.56 mg per kilogram body weight of the human subject. In some examples the amount of the peptidomimetic macrocycle administered is about 7.12 mg per kilogram body weight of the human subject. In some examples the amount of the peptidomimetic macrocycle administered is about 14.24 mg per kilogram body weight of the human subject.

In some cases about 0.5- about 20 mg or about 0.5- about 10 mg of the peptidomimetic macrocycle per kilogram body weight of the human subject is administered two times a week. For example about 0.5-about 1 mg, about 0.5- about 5 mg, about 0.5- about 10 mg, about 0.5- about 15 mg, about 1- about 5 mg, about 1- about 10 mg, about 1- about 15 mg, about 1- about 20 mg, about 5- about 10 mg, about 1- about 15 mg, about 5- about 20 mg, about 10- about 15 mg, about 10- about 20 mg, or about 15- about 20 mg of the peptidomimetic macrocycle per kilogram body weight of the human subject is administered about twice a week. In some examples about 1 mg, about 1.25 mg, about 1.5 mg, about 1.75 mg, about 2 mg, about 2.25 mg, about 2.5 mg, about 2.75 mg, about 3 mg, about 3.25 mg, about 3.5 mg, about 3.75 mg, about 4 mg, about 4.25 mg, about 4.5 mg, about 4.75 mg, about 5 mg, about 5.25 mg, about 5.5 mg, about 5.75 mg, about 6 mg, about 6.25 mg, about 6.5 mg, about 6.75 mg, about 7 mg, about 7.25 mg, about 7.5 mg, about 7.75 mg, about 8 mg, about 8.25 mg, about 8.5 mg, about 8.75 mg, about 9 mg, about 9.25 mg, about 9.5 mg, about 9.75 mg, about 10 mg, about 10.25 mg, about 10.5 mg, about 10.75 mg, about 11 mg, about 11.25 mg, about 11.5 mg, about 11.75 mg, about 12 mg, about 12.25 mg, about 12.5 mg, about 12.75 mg, about 13 mg, about 13.25 mg, about 13.5 mg, about 13.75 mg, about 14 mg, about 14.25 mg, about 14.5 mg, about 14.75 mg, about 15 mg, about 15.25 mg, about 15.5 mg, about 15.75 mg, about 16 mg, about 16.5 mg, about 17 mg, about 17.5 mg, about 18 mg, about 18.5 mg, about 19 mg, about 19.5 mg, or about 20 mg of the peptidomimetic macrocycle per kilogram body weight of the human subject is administered two times a week. In some examples, the amount of the peptidomimetic macrocycle administered is about 1.25 mg, about 2.5 mg, about 5 mg, about 10 mg, or about 20 mg per kilogram body weight of the human subject and the peptidomimetic macrocycle is administered two times a week. In some examples, the amount of the peptidomimetic macrocycle administered is about 1.25 mg, about 2.5 mg, about 5 mg or about 10 mg per kilogram body weight of the human subject and the peptidomimetic macrocycle is administered two times a week.

In some cases about 0.5- about 20 mg or about 0.5- about 10 mg of the peptidomimetic macrocycle per kilogram body weight of the human subject is administered once a week. For example about 0.5- about 1 mg, about 0.5- about 5 mg, about 0.5- about 10 mg, about 0.5- about 15 mg, about 1- about 5 mg, about 1-about 10 mg, about 1- about 15 mg, about 1- about 20 mg, about 5- about 10 mg, about 1- about 15 mg, about 5- about 20 mg, about 10- about 15 mg, about 10- about 20 mg, or about 15- about 20 mg of the peptidomimetic macrocycle per kilogram body weight of the human subject is administered once a week. In some examples about 1 mg, about 1.25 mg, about 1.5 mg, about 1.75 mg, about 2 mg, about 2.25 mg, about 2.5 mg, about 2.75 mg, about 3 mg, about 3.25 mg, about 3.5 mg, about 3.75 mg, about 4 mg, about 4.25 mg, about 4.5 mg, about 4.75 mg, about 5 mg, about 5.25 mg, about 5.5 mg, about 5.75 mg, about 6 mg, about 6.25 mg, about 6.5 mg, about 6.75 mg, about 7 mg, about 7.25 mg, about 7.5 mg, about 7.75 mg, about 8 mg, about 8.25 mg, about 8.5 mg, about 8.75 mg, about 9 mg, about 9.25 mg, about 9.5 mg, about 9.75 mg, about 10 mg, about 10.25 mg, about 10.5 mg, about 10.75 mg, about 11 mg, about 11.25 mg, about 11.5 mg, about 11.75 mg, about 12 mg, about 12.25 mg, about 12.5 mg, about 12.75 mg, about 13 mg, about 13.25 mg, about 13.5 mg, about 13.75 mg, about 14 mg, about 14.25 mg, about 14.5 mg, about 14.75 mg, about 15 mg, about 15.25 mg, about 15.5 mg, about 15.75 mg, about 16 mg, about 16.5 mg, about 17 mg, about 17.5 mg, about 18 mg, about 18.5 mg, about 19 mg, about 19.5 mg, or about 20 mg of the peptidomimetic macrocycle per kilogram body weight of the human subject is administered once a week. In some examples, the amount of the peptidomimetic macrocycle administered is about 1.25 mg, about 2.5 mg, about 5 mg, about 10 mg, or about 20 mg per kilogram body weight of the human subject and the peptidomimetic macrocycle is administered once a week. In some examples, the amount of the peptidomimetic macrocycle administered is about 1.25 mg, about 2.5 mg, about 5 mg or about 10 mg per kilogram body weight of the human subject and the peptidomimetic macrocycle is administered once a week.

In some cases about 0.5- about 20 mg or about 0.5- about 10 mg of the peptidomimetic macrocycle per kilogram body weight of the human subject is administered 3, 4, 5, 6, or 7 times a week. For example, about 0.5- about 1 mg, about 0.5- about 5 mg, about 0.5- about 10 mg, about 0.5- about 15 mg, about 1- about 5 mg, about 1- about 10 mg, about 1- about 15 mg, about 1- about 20 mg, about 5- about 10 mg, about 1-about 15 mg, about 5- about 20 mg, about 10- about 15 mg, about 10- about 20 mg, or about 15- about 20 mg of the peptidomimetic macrocycle per kilogram body weight of the human subject is administered 3, 4, 5, 6, or 7 times a week. In some examples about 1 mg, about 1.25 mg, about 1.5 mg, about 1.75 mg, about 2 mg, about 2.25 mg, about 2.5 mg, about 2.75 mg, about 3 mg, about 3.25 mg, about 3.5 mg, about 3.75 mg, about 4 mg, about 4.25 mg, about 4.5 mg, about 4.75 mg, about 5 mg, about 5.25 mg, about 5.5 mg, about 5.75 mg, about 6 mg, about 6.25 mg, about 6.5 mg, about 6.75 mg, about 7 mg, about 7.25 mg, about 7.5 mg, about 7.75 mg, about 8 mg, about 8.25 mg, about 8.5 mg, about 8.75 mg, about 9 mg, about 9.25 mg, about 9.5 mg, about 9.75 mg, about 10 mg, about 10.25 mg, about 10.5 mg, about 10.75 mg, about 11 mg, about 11.25 mg, about 11.5 mg, about 11.75 mg, about 12 mg, about 12.25 mg, about 12.5 mg, about 12.75 mg, about 13 mg, about 13.25 mg, about 13.5 mg, about 13.75 mg, about 14 mg, about 14.25 mg, about 14.5 mg, about 14.75 mg, about 15 mg, about 15.25 mg, about 15.5 mg, about 15.75 mg, about 16 mg, about 16.5 mg, about 17 mg, about 17.5 mg, about 18 mg, about 18.5 mg, about 19 mg, about 19.5 mg, or about 20 mg of the peptidomimetic macrocycle per kilogram body weight of the human subject is administered 3, 4, 5, 6, or 7 times a week. In some examples, the amount of the peptidomimetic macrocycle administered is about 1.25 mg, about 2.5 mg, about 5 mg, about 10 mg, or about 20 mg per kilogram body weight of the human subject and the peptidomimetic macrocycle is administered 3, 4, 5, 6, or 7 times a week. In some examples, the amount of the peptidomimetic macrocycle administered is about 1.25 mg, about 2.5 mg, about 5 mg, or about 10 mg per kilogram body weight of the human subject and the peptidomimetic macrocycle is administered 3, 4, 5, 6, or 7 times a week.

In some cases, about 0.5- about 20 mg or about 0.5- about 10 mg of the peptidomimetic macrocycle per kilogram body weight of the human subject is administered once every 2, 3, or 4 weeks. For example, about 0.5- about 1 mg, about 0.5- about 5 mg, about 0.5- about 10 mg, about 0.5- about 15 mg, about 1- about 5 mg, about 1- about 10 mg, about 1- about 15 mg, about 1- about 20 mg, about 5- about 10 mg, about 1-about 15 mg, about 5- about 20 mg, about 10- about 15 mg, about 10- about 20 mg, or about 15- about 20 mg of the peptidomimetic macrocycle per kilogram body weight of the human subject is administrated 3, 4, 5, 6, or 7 once every 2 or 3 week. In some examples about 1 mg, about 1.25 mg, about 1.5 mg, about 1.75 mg, about 2 mg, about 2.25 mg, about 2.5 mg, about 2.75 mg, about 3 mg, about 3.25 mg, about 3.5 mg, about 3.75 mg, about 4 mg, about 4.25 mg, about 4.5 mg, about 4.75 mg, about 5 mg, about 5.25 mg, about 5.5 mg, about 5.75 mg, about 6 mg, about 6.25 mg, about 6.5 mg, about 6.75 mg, about 7 mg, about 7.25 mg, about 7.5 mg, about 7.75 mg, about 8 mg, about 8.25 mg, about 8.5 mg, about 8.75 mg, about 9 mg, about 9.25 mg, about 9.5 mg, about 9.75 mg, about 10 mg, about 10.25 mg, about 10.5 mg, about 10.75 mg, about 11 mg, about 11.25 mg, about 11.5 mg, about 11.75 mg, about 12 mg, about 12.25 mg, about 12.5 mg, about 12.75 mg, about 13 mg, about 13.25 mg, about 13.5 mg, about 13.75 mg, about 14 mg, about 14.25 mg, about 14.5 mg, about 14.75 mg, about 15 mg, about 15.25 mg, about 15.5 mg, about 15.75 mg, about 16 mg, about 16.5 mg, about 17 mg, about 17.5 mg, about 18 mg, about 18.5 mg, about 19 mg, about 19.5 mg, or about 20 mg of the peptidomimetic macrocycle per kilogram body weight of the human subject is administered once every 2 or 3 weeks. In some examples, the amount of the peptidomimetic macrocycle administered is about 1.25 mg, about 2.5 mg, about 5 mg, about 10 mg, or about 20 mg per kilogram body weight of the human subject and the peptidomimetic macrocycle is administered once every 2 weeks. In some examples, the amount of the peptidomimetic macrocycle administered is about 1.25 mg, about 2.5 mg, about 5 mg or about 10 mg per kilogram body weight of the human subject and the peptidomimetic macrocycle is administered once every 2 weeks. In some examples, the amount of the peptidomimetic macrocycle administered is about 1.25 mg, about 2.5 mg, about 5 mg, about 10 mg, or about 20 mg per kilogram body weight of the human subject and the peptidomimetic macrocycle is administered once every 3 weeks. In some examples, the amount of the peptidomimetic macrocycle administered is about 1.25 mg, about 2.5 mg, about 5 mg, or about 10 mg per kilogram body weight of the human subject and the peptidomimetic macrocycle is administered once every 3 weeks.

In some cases, the peptidomimetic macrocycle is administered gradually over a period of time. A desired amount of peptidomimetic macrocycle can, for example can be administered gradually over a period of from about 0.1 h -24 h. In some cases a desired amount of peptidomimetic macrocycle is administered gradually over a period of 0.1 h, 0.5 h, 1 h, 1.5 h, 2 h, 2.5 h, 3 h, 3.5 h, 4 h, 4.5 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h, 23 h, or 24 h. In some examples, a desired amount of peptidomimetic macrocycle is administered gradually over a period of 0.25 -12 h, for example over a period of 0.25-1 h, 0.25-2 h, 0.25-3 h, 0.25-4 h, 0.25-6 h, 0.25-8 h, 0.25-10 h. In some examples, a desired amount of peptidomimetic macrocycle is administered gradually over a period of 0.25-2 h. In some examples, a desired amount of peptidomimetic macrocycle is administered gradually over a period of 0.25-1 h. In some examples, a desired amount of peptidomimetic macrocycle is administered gradually over a period of 0.25 h, 0.3 h, 0.4 h, 0.5 h, 0.6 h, 0.7 h, 0.8 h, 0.9 h, 1.0 h, 1.1 h, 1.2 h, 1.3 h, 1.4 h, 1.5 h, 1.6 h, 1.7 h, 1.8 h, 1.9 h, or 2.0 h. In some examples, a desired amount of peptidomimetic macrocycle is administered gradually over a period of 1 h. In some examples, a desired amount of peptidomimetic macrocycle is administered gradually over a period of 2 h.

Administration of the peptidomimetic macrocycles can continue as long as necessary. In some cases, one or more peptidomimetic macrocycle of the disclosure is administered for more than 1 day, more than 1 week, more than 1 month, more than 2 months, more than 3 months, more than 4 months, more than 5 months, more than 6 months, more than 7 months, more than 8 months, more than 9 months, more than 10 months, more than 11 months, more than 12 months, more than 13 months, more than 14 months, more than 15 months, more than 16 months, more than 17 months, more than 18 months, more than 19 months, more than 20 months, more than 21 months, more than 22 months, more than 23 months, or more than 24 months. In some cases, one or more peptidomimetic macrocycle of the disclosure is administered for less than 1 week, less than 1 month, less than 2 months, less than 3 months, less than 4 months, less than 5 months, less than 6 months, less than 7 months, less than 8 months, less than 9 months, less than 10 months, less than 11 months, less than 12 months, less than 13 months, less than 14 months, less than 15 months, less than 16 months, less than 17 months, less than 18 months, less than 19 months, less than 20 months, less than 21 months, less than 22 months, less than 23 months, or less than 24 months.

In some cases, the peptidomimetic macrocycle is administered on day 1, 8, 15 and 28 of a 28 day cycle. In some cases, the peptidomimetic macrocycle is administered on day 1, 8, 15 and 28 of a 28 day cycle and administration is continued for two cycles. In some cases, the peptidomimetic macrocycle is administered on day 1, 8, 15 and 28 of a 28 day cycle and administration is continued for three cycles. In some cases, the peptidomimetic macrocycle is administered on day 1, 8, 15 and 28 of a 28 day cycle and administration is continued for 4, 5, 6, 7, 8, 9, 10, or more cycles.

In some cases, the peptidomimetic macrocycle is administered on day 1, 8, 11 and 21 of a 21-day cycle. In some cases, the peptidomimetic macrocycle is administered on day 1, 8, 11 and 21 of a 21-day cycle and administration is continued for two cycles. In some cases, the peptidomimetic macrocycle is administered on day 1, 8, 11 and 21 of a 21-day cycle and administration is continued for three cycles. In some cases, the peptidomimetic macrocycle is administered on day 1, 8, 11 and 21 of a 21-day cycle and administration is continued for 4, 5, 6, 7, 8, 9, 10, or more cycles.

In some cases, one or more peptidomimetic macrocycle of the disclosure is administered chronically on an ongoing basis. In some cases administration of one or more peptidomimetic macrocycle of the disclosure is continued until documentation of disease progression, unacceptable toxicity, or patient or physician decision to discontinue administration.

In some cases, the peptidomimetic macrocycle and at least one additional pharmaceutically active agent as described hereincan be used to treat one condition. In some cases, the peptidomimetic macrocycle and at least one additional pharmaceutically active agent as described hereincan be used to treat two conditions. In some cases, the peptidomimetic macrocycle and at least one additional pharmaceutically active agent as described hereincan be used to treat three conditions. In some cases, the peptidomimetic macrocycle and at least one additional pharmaceutically active agent as described hereincan be used to treat four conditions. In some cases, the peptidomimetic macrocycle and at least one additional pharmaceutically active agent as described hereincan be used to treat five conditions.

### Sequence Homology

Two or more peptides can share a degree of homology. A pair of peptides can have, for example, up to about 20% pairwise homology, up to about 25% pairwise homology, up to about 30% pairwise homology, up to about 35% pairwise homology, up to about 40% pairwise homology, up to about 45% pairwise homology, up to about 50% pairwise homology, up to about 55% pairwise homology, up to about 60% pairwise homology, up to about 65% pairwise homology, up to about 70% pairwise homology, up to about 75% pairwise homology, up to about 80% pairwise homology, up to about 85% pairwise homology, up to about 90% pairwise homology, up to about 95% pairwise homology, up to about 96% pairwise homology, up to about 97% pairwise homology, up to about 98% pairwise homology, up to about 99% pairwise homology, up to about 99.5% pairwise homology, or up to about 99.9% pairwise homology. A pair of peptides can have, for example, at least about 20% pairwise homology, at least about 25% pairwise homology, at least about 30% pairwise homology, at least about 35% pairwise homology, at least about 40% pairwise homology, at least about 45% pairwise homology, at least about 50% pairwise homology, at least about 55% pairwise homology, at least about 60% pairwise homology, at least about 65% pairwise homology, at least about 70% pairwise homology, at least about 75% pairwise homology, at least about 80% pairwise homology, at least about 85% pairwise homology, at least about 90% pairwise homology, at least about 95% pairwise homology, at least about 96% pairwise homology, at least about 97% pairwise homology, at least about 98% pairwise homology, at least about 99% pairwise homology, at least about 99.5% pairwise homology, at least about 99.9% pairwise homology.

Various methods and software programs can be used to determine the homology between two or more peptides, such as NCBI BLAST, Clustal W, MAFFT, Clustal Omega, AlignMe, Praline, or another suitable method or algorithm.

### Peptidomimetic Macrocycles

In some cases, the peptidomimetic macrocycle employed with at least one pharmaceutically active agent comprises an amino acid sequence which is at least least 60% identical to an amino acid sequence in any of Table 1, Table 1a, Table 1b, and Table 1c, and wherein the peptidomimetic macrocycle has the formula: 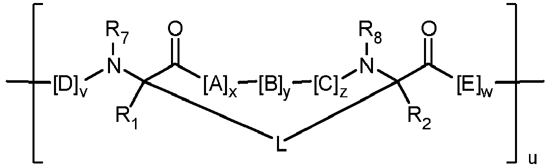 wherein: each A, C, D, and E is independently an amino acid; each B is independently an amino acid, 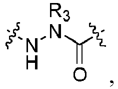 [-NH-L₃-CO-], [-NH-L₃-SO₂-], or [-NH-L₃-]; each R₁ and R₂ is independently hydrogen, alkyl, alkenyl, alkynyl, arylalkyl, cycloalkyl, cycloalkylalkyl, heteroalkyl, or heterocycloalkyl, unsubstituted or substituted with halo-; or forms a macrocycle-forming linker L' connected to the alpha position of one of said D or E amino acids; each R₃ is independently hydrogen, alkyl, alkenyl, alkynyl, arylalkyl, heteroalkyl, cycloalkyl, heterocycloalkyl, cycloalkylalkyl, aryl, or heteroaryl, optionally substituted with R₅; each L and L' is independently a macrocycle-forming linker of the formula -L₁-L₂-; each L₁, L₂, and L₃ is independently alkylene, alkenylene, alkynylene, heteroalkylene, cycloalkylene, heterocycloalkylene, arylene, heteroarylene, or [-R₄-K-R₄-]ₙ, each being optionally substituted with R₅; each R₄ is independently alkylene, alkenylene, alkynylene, heteroalkylene, cycloalkylene, heterocycloalkylene, arylene, or heteroarylene; each K is independently O, S, SO, SOz, CO, COz, or CONR₃; each R₅ is independently halogen, alkyl, -OR₆, -N(R₆)₂,-SR₆, -SOR₆, -SO₂R₅, -CO₂R₅, a fluorescent moiety, a radioisotope or a therapeutic agent; each R₆ is independently hydrogen, alkyl, alkenyl, alkynyl, arylalkyl, cycloalkylalkyl, heterocycloalkyl, a fluorescent moiety, a radioisotope or a therapeutic agent; each R₇ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue; each R₈ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue; each v is independently an integer from 1-1000; each w is independently an integer from 3-1000; u is an integer from 1-10; each x, y and z is independently an integer from 0-10; and each n is independently an integer from 1-5; wherein the at least one additional pharmaceutically active agent is fulvestrant, everolimus, romidepsin, palbociclib, trametinib, rituximab, dabrafenib, vemurafenib, azacitidine, decitabine, midostaurin, vincristine, cyclophosphamide or cytarabine.

In some cases, v and w are integers from 1-30. In some cases, w is an integer from 3-1000, for example 3-500, 3-200, 3-100, 3-50, 3-30, 3-20, or 3-10. In some cases, the sum of x+y+z is 3 or 6. In some cases, the sum of x+y+z is 3. In other cases, the sum of x+y+z is 6.

In some cases, w is an integer from 3-10, for example 3-6, 3-8, 6-8, or 6-10. In some cases, w is 3. In other cases, w is 6. In some cases, v is an integer from 1-1000, for example 1-500, 1-200, 1-100, 1-50, 1-30, 1-20, or 1-10. In some cases, v is 2.

In an case of any of the Formulas described herein, L₁ and Lz, either alone or in combination, do not form a triazole or a thioether.

In one example, at least one of R₁ and R₂ is alkyl that is unsubstituted or substituted with halo-. In another example, both R₁ and R₂ are independently alkyl that is unsubstituted or substituted with halo-. In some cases, at least one of R₁ and R₂ is methyl. In other cases, R₁ and R₂ are methyl.

In some cases, x+y+z is at least 3. In other cases, x+y+z is 1, 2, 3, 4, 5, 6, 7, 8, 9 or 10. In some cases, the sum of x+y+z is 3 or 6. In some cases, the sum of x+y+z is 3. In other cases, the sum of x+y+z is 6. Each occurrence of A, B, C, D or E in a macrocycle or macrocycle precursor is independently selected. For example, a sequence represented by the formula [A]ₓ, when x is 3, encompasses cases wherein the amino acids are not identical, e.g. Gln-Asp-Ala as well as cases wherein the amino acids are identical, e.g. Gln-Gln-Gln. This applies for any value of x, y, or z in the indicated ranges. Similarly, when u is greater than 1, each compound can encompass peptidomimetic macrocycles which are the same or different. For example, a compound can comprise peptidomimetic macrocycles comprising different linker lengths or chemical compositions.

In some cases, the peptidomimetic macrocycle comprises a secondary structure which is an α-helix and R₈ is -H, allowing for intrahelical hydrogen bonding. In some cases, at least one of A, B, C, D or E is an α,α-disubstituted amino acid. In one example, B is an α,α-disubstituted amino acid. For instance, at least one of A, B, C, D or E is 2-aminoisobutyric acid. In other cases, at least one of A, B, C, D or E is 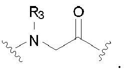

In other cases, the length of the macrocycle-forming linker L as measured from a first Cα to a second Cα is selected to stabilize a desired secondary peptide structure, such as an α-helix formed by residues of the peptidomimetic macrocycle including, but not necessarily limited to, those between the first Cα to a second Cα.

In some cases, peptidomimetic macrocycles are also described of the formula: 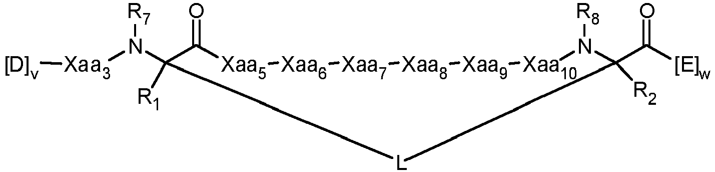 wherein:
- each of Xaa₃, Xaa₅, Xaa₆, Xaa₇, Xaa₈, Xaa₉, and Xaa₁₀ is individually an amino acid, wherein at least three of Xaa₃, Xaa₅, Xaa₆, Xaa₇, Xaa₈, Xaa₉, and Xaa₁₀ are the same amino acid as the amino acid at the corresponding position of the sequence Phe₃-X₄-His₅-Tyr₆-Trp₇-Ala₈-Gln₉-Leu₁₀-X₁₁-Ser₁₂, wherein each X is an amino acid;
- each D and E is independently a natural or non-natural amino acid or an amino acid analog;
- R₁ and R₂ are independently -H, alkyl, alkenyl, alkynyl, arylalkyl, cycloalkyl, cycloalkylalkyl, heteroalkyl, or heterocycloalkyl, unsubstituted or substituted with halo-; or at least one of R₁ and R₂ forms a macrocycle-forming linker L' connected to the alpha position of one of said D or E amino acids;
- each L and L' is independently a macrocycle-forming linker of the formula -L₁-L₂-;
- each L₁ and L₂ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- each R₅ is independently halogen, alkyl, -OR₆, -N(R₆)₂, -SR₆, -SOR₆, -SO₂R₆, -CO₂R₅, a fluorescent moiety, a radioisotope or a therapeutic agent;
- each R₆ is independently -H, alkyl, alkenyl, alkynyl, arylalkyl, cycloalkylalkyl, heterocycloalkyl, a fluorescent moiety, a radioisotope or a therapeutic agent;
- R₇ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
- R₈ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
- v is an integer from 1-1000, for example 1-500, 1-200, 1-100, 1-50, 1-30, 1-20 or 1-10;
- w is an integer from 3-1000, for example 3-500, 3-200, 3-100, 3-50, 3-30, 3-20, or 3-10; and
- n is an integer from 1-5.

In some cases, v and w are integers from 1-30. In some cases, w is an integer from 3-1000, for example 3-500, 3-200, 3-100, 3-50, 3-30, 3-20, or 3-10. In some cases, the sum of x+y+z is 3 or 6. In some cases, the sum of x+y+z is 3. In other cases, the sum of x+y+z is 6.

In some cases of any of the Formulas described herein, at least three of Xaa₃, Xaa₅, Xaa₆, Xaa₇, Xaa₈, Xaa₉, and Xaa₁₀ are the same amino acid as the amino acid at the corresponding position of the sequence Phe₃-X₄-His₅-Tyr₆-Trp₇-Ala₈-Gln₉-Leu₁₀-X₁₁-Ser₁₂. In other cases, at least four of Xaa₃, Xaa₅, Xaa₆, Xaa₇, Xaa₅, Xaa₉, and Xaa₁₀ are the same amino acid as the amino acid at the corresponding position of the sequence Phe₃-X₄-His₅-Tyr₆-Trp₇-Ala₈-Gln₉-Leu₁₀-X₁₁-Ser₁₂. In other cases, at least five of Xaa₃, Xaa₅, Xaa₆, Xaa₇, Xaa₈, Xaa₉, and Xaa₁₀ are the same amino acid as the amino acid at the corresponding position of the sequence Phe₃-X₄-His₅-Tyr₆-Trp₇-Ala₈-Gln₉-Leu₁₀-X₁₁-Ser₁₂. In other cases, at least six of Xaa₃, Xaa₅, Xaa₆, Xaa₇, Xaa₈, Xaa₉, and Xaa₁₀ are the same amino acid as the amino acid at the corresponding position of the sequence Phe₃-X₄-His₅-Tyr₆-Trp₇-Ala₈-Gln₉-Leu₁₀-X₁₁-Ser₁₂. In other cases, at least seven of Xaa₃, Xaa₅, Xaa₆, Xaa₇, Xaa₈, Xaa₉, and Xaa₁₀ are the same amino acid as the amino acid at the corresponding position of the sequence Phe₃-X₄-His₅-Tyr₆-Trp₇-Ala₈-Gln₉-Leu₁₀-X₁₁-Ser₁₂.

In some cases, a peptidomimetic macrocycle has the Formula: 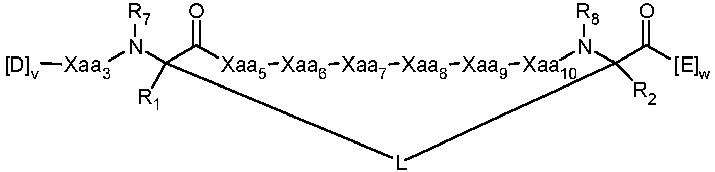 wherein:
- each of Xaa₃, Xaa₅, Xaa₆, Xaa₇, Xaa₈, Xaa₉, and Xaa₁₀ is individually an amino acid, wherein at least three of Xaa₃, Xaa₅, Xaa₆, Xaa₇, Xaa₈, Xaa₉, and Xaa₁₀ are the same amino acid as the amino acid at the corresponding position of the sequence Phe₃-X₄-Glu₅-Tyr₆-Trp₇-Ala₈-Gln₉-Leu₁₀/Cba₁₀-X₁₁-Ala₁₂, wherein each X is an amino acid;
- each D is independently a natural or non-natural amino acid or an amino acid analog;
- each E is independently a natural or non-natural amino acid or an amino acid analog, for example an amino acid selected from Ala (alanine), D-Ala (D-alanine), Aib (α-aminoisobutyric acid), Sar (N-methyl glycine), and Ser (serine);
- R₁ and R₂ are independently -H, alkyl, alkenyl, alkynyl, arylalkyl, cycloalkyl, cycloalkylalkyl, heteroalkyl, or heterocycloalkyl, unsubstituted or substituted with halo-; or at least one of R₁ and R₂ forms a macrocycle-forming linker L' connected to the alpha position of one of said D or E amino acids;
- each L and L' is independently a macrocycle-forming linker of the formula -L₁-L₂-;
- each L₁ and L₂ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- each R₅ is independently halogen, alkyl, -OR₆, -N(R₆)₂, -SR₆, -SOR₆, -SO₂R₅, -CO₂R₅, a fluorescent moiety, a radioisotope or a therapeutic agent;
- each R₆ is independently -H, alkyl, alkenyl, alkynyl, arylalkyl, cycloalkylalkyl, heterocycloalkyl, a fluorescent moiety, a radioisotope or a therapeutic agent;
- R₇ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
- R₈ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
- v is an integer from 1-1000, for example 1-500, 1-200, 1-100, 1-50, 1-30, 1-20, or 1-10;
- w is an integer from 3-1000, for example 3-500, 3-200, 3-100, 3-50, 3-30, 3-20, or 3-10; and
- n is an integer from 1-5.

In some cases of the above Formula, at least three of Xaa₃, Xaa₅, Xaa₆, Xaa₇, Xaa₈, Xaa₉, and Xaa₁₀ are the same amino acid as the amino acid at the corresponding position of the sequence Phe₃-X₄-Glu₅-Tyr₆-Trp₇-Ala₈-Gln₉-Leu₁₀/Cba₁₀-X₁₁-Ala₁₂. In other cases of the above Formula, at least four of Xaa₃, Xaa₅, Xaa₆, Xaa₇, Xaa₈, Xaa₉, and Xaa₁₀ are the same amino acid as the amino acid at the corresponding position of the sequence Phe₃-X₄-Glu₅-Tyr₆-Trp₇-Ala₈-Gln₉-Leu₁₀/Cba₁₀-X₁₁-Ala₁₂. In other cases of the above Formula, at least five of Xaa₃, Xaa₅, Xaa₆, Xaa₇, Xaa₈, Xaa₉, and Xaa₁₀ are the same amino acid as the amino acid at the corresponding position of the sequence Phe₃-X₄-Glu₅-Tyr₆-Trp₇-Ala₈-Gln₉-Leu₁₀/Cba₁₀-X₁₁-Ala₁₂. In other cases of the above Formula, at least six of Xaa₃, Xaa₅, Xaa₆, Xaa₇, Xaa₈, Xaa₉, and Xaa₁₀ are the same amino acid as the amino acid at the corresponding position of the sequence Phe₃-X₄-Glu₅-Tyr₆-Trp₇-Ala₈-Gln₉-Leu₁₀/Cba₁₀-X₁₁-Ala₁₂. In other cases of the above Formula, at least seven of Xaa₃, Xaa₅, Xaa₆, Xaa₇, Xaa₈, Xaa₉, and Xaa₁₀ are the same amino acid as the amino acid at the corresponding position of the sequence Phe₃-X₄-Glu₅-Tyr₆-Trp₇-Ala₈-Gln₉-Leu₁₀/Cba₁₀-X₁₁-Ala_{12.}

In some cases, w is an integer from 3-10, for example 3-6, 3-8, 6-8, or 6-10. In some cases, w is 3. In other cases, w is 6. In some cases, v is an integer from 1-10, for example 2-5. In some cases, v is 2.

In an case of any of the Formulas described herein, L₁ and Lz, either alone or in combination, do not form a triazole or a thioether.

In one example, at least one of R₁ and R₂ is alkyl, unsubstituted or substituted with halo-. In another example, both R₁ and R₂ are independently alkyl, unsubstituted or substituted with halo-. In some cases, at least one of R₁ and R₂ is methyl. In other cases, R₁ and R₂ are methyl.

In some cases, x+y+z is at least 3. In other cases, x+y+z is 1, 2, 3, 4, 5, 6, 7, 8, 9 or 10. In some cases, the sum of x+y+z is 3 or 6. In some cases, the sum of x+y+z is 3. In other cases, the sum of x+y+z is 6. Each occurrence of A, B, C, D or E in a macrocycle or macrocycle precursor is independently selected. For example, a sequence represented by the formula [A]ₓ, when x is 3, encompasses cases wherein the amino acids are not identical, e.g. Gln-Asp-Ala as well as cases wherein the amino acids are identical, e.g. Gln-Gln-Gln. This applies for any value of x, y, or z in the indicated ranges. Similarly, when u is greater than 1, each compound can encompass peptidomimetic macrocycles which are the same or different. For example, a compound can comprise peptidomimetic macrocycles comprising different linker lengths or chemical compositions.

In some cases, the peptidomimetic macrocycle comprises a secondary structure which is an α-helix and R₈ is -H, allowing intrahelical hydrogen bonding. In some cases, at least one of A, B, C, D or E is an α,α-disubstituted amino acid. In one example, B is an α,α-disubstituted amino acid. For instance, at least one of A, B, C, D or E is 2-aminoisobutyric acid. In other cases, at least one of A, B, C, D or E is

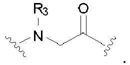

In other cases, the length of the macrocycle-forming linker L as measured from a first Cα to a second Cα is selected to stabilize a desired secondary peptide structure, such as an α-helix formed by residues of the peptidomimetic macrocycle including, but not necessarily limited to, those between the first Cα to a second Cα.

In some cases, a peptidomimetic macrocycle of Formula (I) has Formula (Ia): 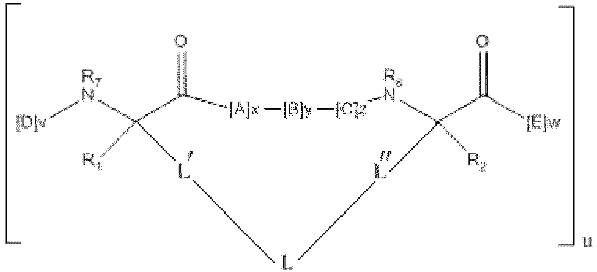 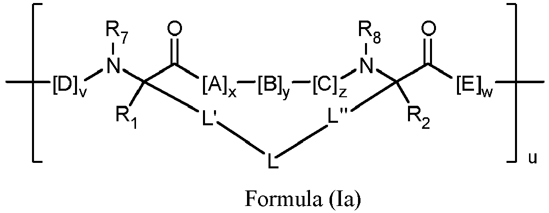 wherein:
- each A, C, D, and E is independently a natural or non-natural amino acid or an amino acid analog;
- each B is independently a natural or non-natural amino acid, amino acid analog, 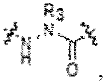 [-NH-L₃-CO-], [-NH-L₃-SO₂-], or [-NH-L₃-];
- each L is independently a macrocycle-forming linker;
- each L' is independently alkylene, alkenylene, alkynylene, heteroalkylene, cycloalkylene, heterocycloalkylene, arylene, or heteroarylene, each being optionally substituted with R₅, or a bond, or together with R₁ and the atom to which both R₁ and L' are bound forms a ring;
- each L" is independently alkylene, alkenylene, alkynylene, heteroalkylene, cycloalkylene, heterocycloalkylene, arylene, or heteroarylene, each being optionally substituted with R₅, or a bond, or together with R₂ and the atom to which both R₂ and L" are bound forms a ring;
- each R₁ is independently -H, alkyl, alkenyl, alkynyl, arylalkyl, cycloalkyl, cycloalkylalkyl, heteroalkyl, or heterocycloalkyl, unsubstituted or substituted with halo-, or together with L' and the atom to which both R₁ and L' are bound forms a ring;
- each R₂ is independently -H, alkyl, alkenyl, alkynyl, arylalkyl, cycloalkyl, cycloalkylalkyl, heteroalkyl, or heterocycloalkyl, unsubstituted or substituted with halo-, or together with L" and the atom to which both R₂ and L" are bound forms a ring;
- each R₃ is independently hydrogen, alkyl, alkenyl, alkynyl, arylalkyl, heteroalkyl, cycloalkyl, heterocycloalkyl, cycloalkylalkyl, aryl, or heteroaryl, optionally substituted with R₅;
- each L₃ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- n is an integer from 1-5;
- each R₅ is independently halogen, alkyl, -OR₆, -N(R₆)₂, -SR₆, -SOR₆, -SO₂R₅, -CO₂R₆, a fluorescent moiety, a radioisotope or a therapeutic agent;
- each R₆ is independently -H, alkyl, alkenyl, alkynyl, arylalkyl, cycloalkylalkyl, heterocycloalkyl, a fluorescent moiety, a radioisotope or a therapeutic agent;
- each R₇ is independently -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
- each R₈ is independently -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
- each v and w is independently an integer from 1-1000, for example 1-500, 1-200, 1-100, 1-50, 1-40, 1-25, 1-20, 1-15, or 1-10; and- each x, y and z is independently an integer from 0-10, for example x+y+z is 2, 3, or 6; and
- u is an integer from 1-10, for example 1-5, 1-3, or 1-2.

In some cases, L is a macrocycle-forming linker of the formula -L₁-L₂-. In some cases, each L₁ and L₂ is independently alkylene, alkenylene, alkynylene, heteroalkylene, cycloalkylene, heterocycloalkylene, arylene, heteroarylene, or [-R₄-K-R₄-]ₙ, each being optionally substituted with R₅; each R₄ is independently alkylene, alkenylene, alkynylene, heteroalkylene, cycloalkylene, heterocycloalkylene, arylene, or heteroarylene; each K is independently O, S, SO, SOz, CO, COz, or CONR₃; and n is an integer from 1-5.

In one example, at least one of R₁ and R₂ is alkyl, unsubstituted or substituted with halo-. In another example, both R₁ and R₂ are independently alkyl, unsubstituted or substituted with halo-. In some cases, at least one of R₁ and R₂ is methyl. In other cases, R₁ and R₂ are methyl.

In some cases, x+y+z is at least 2. In other cases, x+y+z is 1, 2, 3, 4, 5, 6, 7, 8, 9 or 10. Each occurrence of A, B, C, D or E in a macrocycle or macrocycle precursor is independently selected. For example, a sequence represented by the formula [A]ₓ, when x is 3, encompasses cases where the amino acids are not identical, e.g. Gln-Asp-Ala as well as cases wherein the amino acids are identical, e.g. Gln-Gln-Gln. This applies for any value of x, y, or z in the indicated ranges. Similarly, when u is greater than 1, each compound may encompass peptidomimetic macrocycles which are the same or different. For example, a compound may comprise peptidomimetic macrocycles comprising different linker lengths or chemical compositions.

In some cases, the peptidomimetic macrocycle comprises a secondary structure which is a helix and R₈ is -H, allowing intrahelical hydrogen bonding. In some cases, at least one of A, B, C, D or E is an α,α-disubstituted amino acid. In one example, B is an α,α-disubstituted amino acid. For instance, at least one of A, B, C, D or E is 2-aminoisobutyric acid. In other cases, at least one of A, B, C, D or E is

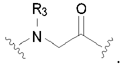

In other cases, the length of the macrocycle-forming linker L as measured from a first Cα to a second Cα is selected to stabilize a desired secondary peptide structure, such as a helix formed by residues of the peptidomimetic macrocycle including, but not necessarily limited to, those between the first Cα to a second Cα.

In one case, the peptidomimetic macrocycle of Formula (I) is: 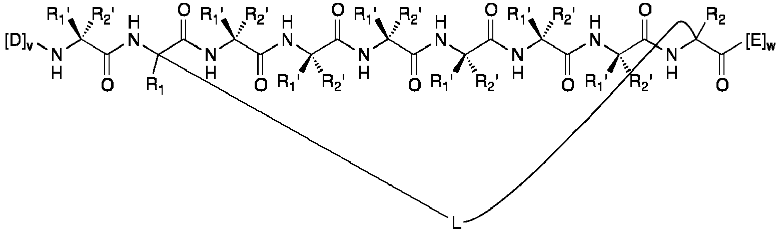 wherein each R₁ and R₂ is independently -H, alkyl, alkenyl, alkynyl, arylalkyl, cycloalkyl, cycloalkylalkyl, heteroalkyl, or heterocycloalkyl, unsubstituted or substituted with halo-.

In related cases, the peptidomimetic macrocycle of Formula (I) is: 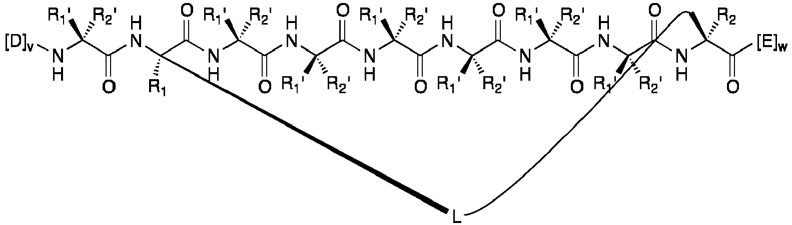 wherein each Ri' and R₂' is independently an amino acid.

In other cases, the peptidomimetic macrocycle of Formula (I) is a compound of any of the formulas shown below: 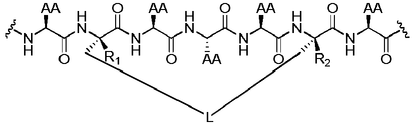 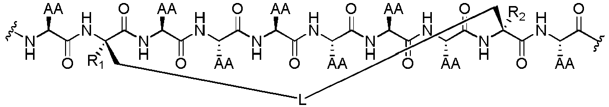 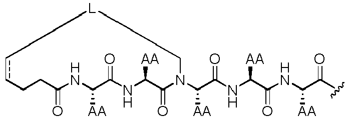 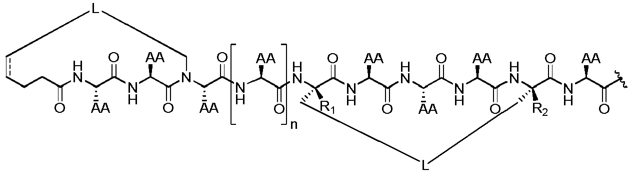 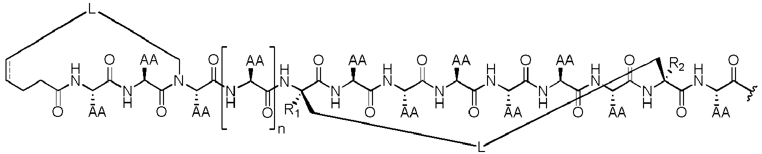 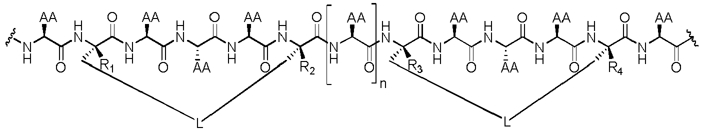 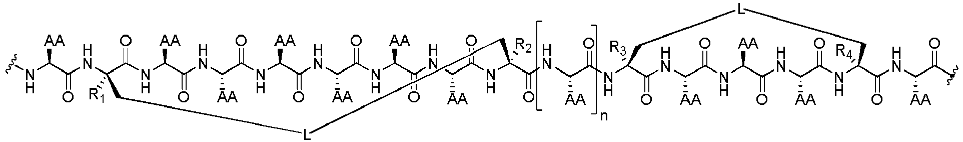 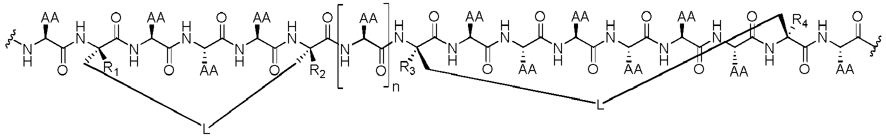 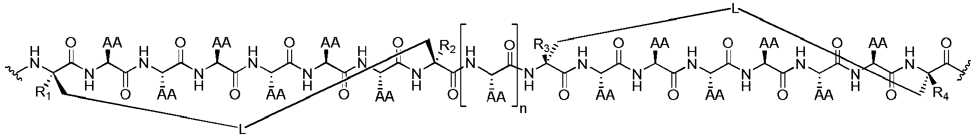 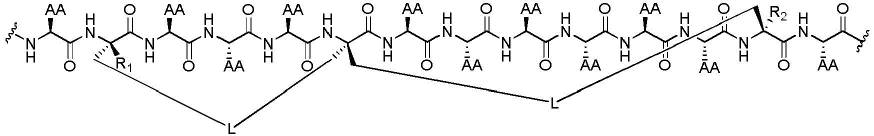 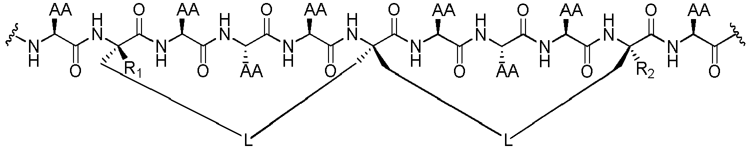 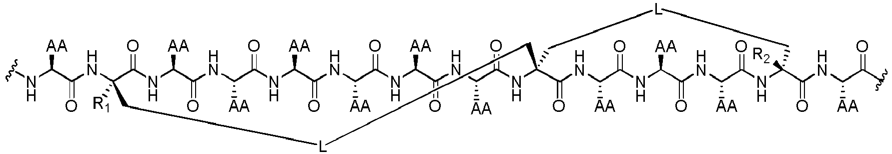 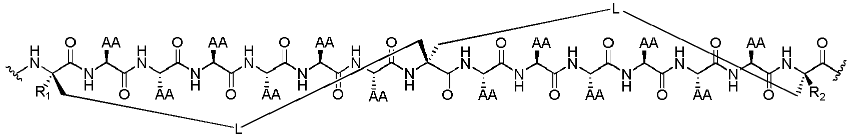 wherein "AA" represents any natural or non-natural amino acid side chain and " 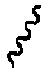 " is [D]ᵥ, [E]_{w} as defined above, and n is an integer between 0 and 20, 50, 100, 200, 300, 400 or 500. In some cases, n is 0. In other cases, n is less than 50.

Exemplary cases of the macrocycle-forming linker L are shown below.

| | |
|---|---|
| 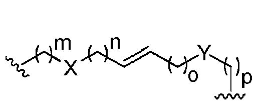 | 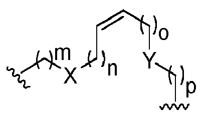 |
| where X, Y = -CH₂-, O, S, or NH | where X, Y = -CH₂-, O, S, or NH |
| m, n, o, p = 0-10 | m, n, o, p = 0-10 |
| 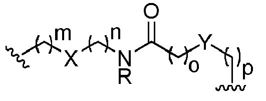 | 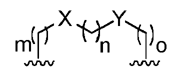 |
| where X, Y = -CH₂-, O, S, or NH | where X, Y = -CH₂-, O, S, or NH |
| m, n, o, p = 0-10 | m, n, o = 0-10 |
| R = H, alkyl, other substituent | |

In other cases, D and/or E in the compound of Formula I are further modified to facilitate cellular uptake. In some cases, lipidating or PEGylating a peptidomimetic macrocycle facilitates cellular uptake, increases bioavailability, increases blood circulation, alters pharmacokinetics, decreases immunogenicity and/or decreases the needed frequency of administration.

In other cases, at least one of [D] and [E] in the compound of Formula I represents a moiety comprising an additional macrocycle-forming linker such that the peptidomimetic macrocycle comprises at least two macrocycle-forming linkers. In a specific case, a peptidomimetic macrocycle comprises two macrocycle-forming linkers. In an case, u is 2.

In some cases, the peptidomimetic macrocycles have the Formula (I): 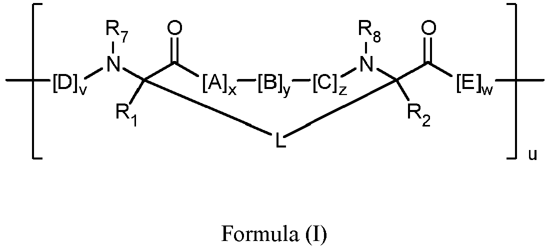 wherein:
- each A, C, D, and E is independently a natural or non-natural amino acid or an amino acid analog;
- each B is independently a natural or non-natural amino acid, amino acid analog, 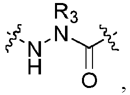 [-NH-L₃-CO-], [-NH-L₃-SO₂-], or [-NH-L₃-];
- each R₁ and R₂ is independently -H, alkyl, alkenyl, alkynyl, arylalkyl, cycloalkyl, cycloalkylalkyl, heteroalkyl, or heterocycloalkyl, unsubstituted or substituted with halo-, or at least one of R₁ and R₂ forms a macrocycle-forming linker L' connected to the alpha position of one of said D or E amino acids;
- each R₃ is independently hydrogen, alkyl, alkenyl, alkynyl, arylalkyl, heteroalkyl, cycloalkyl, heterocycloalkyl, cycloalkylalkyl, aryl, or heteroaryl, optionally substituted with R₅;
- each L and L' is independently macrocycle-forming linker of the formula 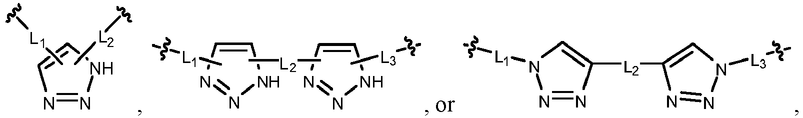 wherein each L₁, L₂ and L₃ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- each R₅ is independently halogen, alkyl, -OR₆, -N(R₆)₂, -SR₆, -SOR₆, -SO₂R₅, -CO₂R₅, a fluorescent moiety, a radioisotope or a therapeutic agent;
- each R₆ is independently -H, alkyl, alkenyl, alkynyl, arylalkyl, cycloalkylalkyl, heterocycloalkyl, a fluorescent moiety, a radioisotope or a therapeutic agent;
- each R₇ is independently -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
- each R₈ is independently -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
- each v and w is independently an integer from 1-1000;
- each x, y and z is independently an integer from 0-10;
- us ia an integer from 1-10; and
- n is an integer from 1-5.

In one example, at least one of R₁ and R₂ is alkyl that is unsubstituted or substituted with halo-. In another example, both R₁ and R₂ are independently alkyl that are unsubstituted or substituted with halo-. In some cases, at least one of R₁ and R₂ is methyl. In other cases, R₁ and R₂ are methyl.

In some cases, x+y+z is at least 2. In other cases, x+y+z is 1, 2, 3, 4, 5, 6, 7, 8, 9 or 10. Each occurrence of A, B, C, D or E in a macrocycle or macrocycle precursor is independently selected. For example, a sequence represented by the formula [A]ₓ, when x is 3, encompasses cases where the amino acids are not identical, e.g. Gln-Asp-Ala as well as cases wherein the amino acids are identical, e.g. Gln-Gln-Gln. This applies for any value of x, y, or z in the indicated ranges.

In some cases, each of the first two amino acid represented by E comprises an uncharged side chain or a negatively charged side chain. In some cases, each of the first three amino acid represented by E comprises an uncharged side chain or a negatively charged side chain. In some cases, each of the first four amino acid represented by E comprises an uncharged side chain or a negatively charged side chain. In some cases, one or more or each of the amino acid that is *i*+1, *i*+2, *i*+3, *i*+4, *i*+5, and/or *i*+6 with respect to Xaa₁₃ represented by E comprises an uncharged side chain or a negatively charged side chain.

In some cases, the first C-terminal amino acid and/or the second C-terminal amino acid represented by E comprise a hydrophobic side chain. For example, the first C-terminal amino acid and/or the second C-terminal amino acid represented by E comprises a hydrophobic side chain, for example a small hydrophobic side chain. In some cases, the first C-terminal amino acid, the second C-terminal amino acid, and/or the third C-terminal amino acid represented by E comprise a hydrophobic side chain. For example, the first C-terminal amino acid, the second C-terminal amino acid, and/or the third C-terminal amino acid represented by E comprises a hydrophobic side chain, for example a small hydrophobic side chain. In some cases, one or more or each of the amino acid that is *i*+1, *i*+2, *i*+3, *i*+4, *i*+5, and/or *i*+6 with respect to Xaa₁₃ represented by E comprises an uncharged side chain or a negatively charged side chain

In some cases, w is between 1 and 1000. For example, the first amino acid represented by E comprises a small hydrophobic side chain. In some cases, w is between 2 and 1000. For example, the second amino acid represented by E comprises a small hydrophobic side chain. In some cases, w is between 3 and 1000. For example, the third amino acid represented by E comprises a small hydrophobic side chain. For example, the third amino acid represented by E comprises a small hydrophobic side chain. In some cases, w is between 4 and 1000. In some cases, w is between 5 and 1000. In some cases, w is between 6 and 1000. In some cases, w is between 7 and 1000. In some cases, w is between 8 and 1000.

In some cases, the peptidomimetic macrocycle comprises a secondary structure which is a helix and R₈ is -H, allowing intrahelical hydrogen bonding. In some cases, at least one of A, B, C, D or E is an α,α-disubstituted amino acid. In one example, B is an α,α-disubstituted amino acid. For instance, at least one of A, B, C, D or E is 2-aminoisobutyric acid. In other cases, at least one of A, B, C, D or E is

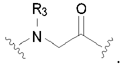

In other cases, the length of the macrocycle-forming linker L as measured from a first Cα to a second Cα is selected to stabilize a desired secondary peptide structure, such as a helix formed by residues of the peptidomimetic macrocycle including, but not necessarily limited to, those between the first Cα to a second Cα.

In some cases, L is a macrocycle-forming linker of the formula

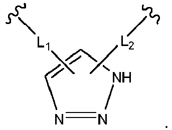

In some cases, L is a macrocycle-forming linker of the formula 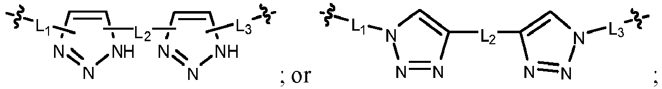 or a tautomer thereof.

Exemplary cases of the macrocycle-forming linker L are shown below.

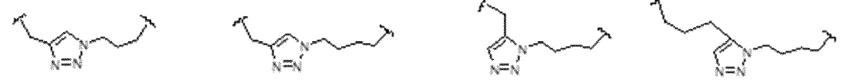

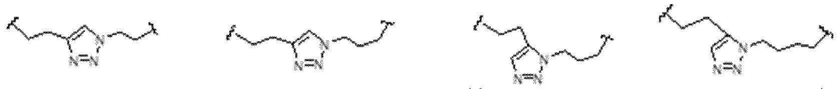

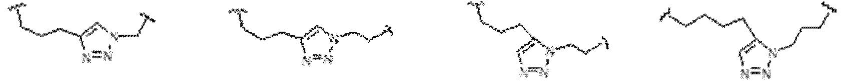

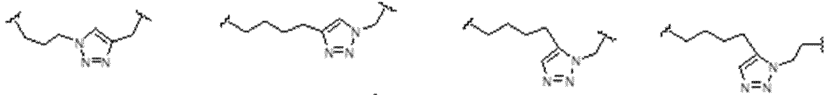

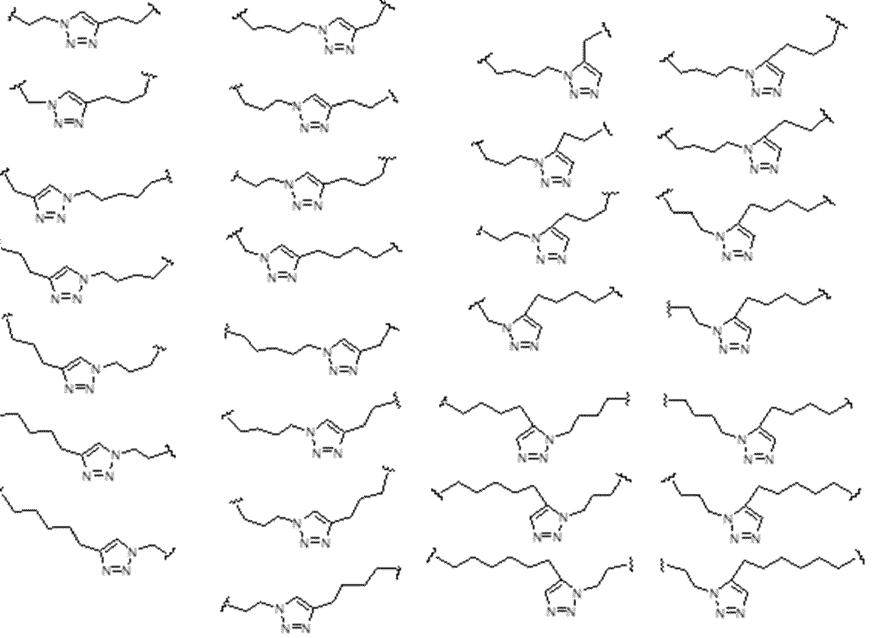

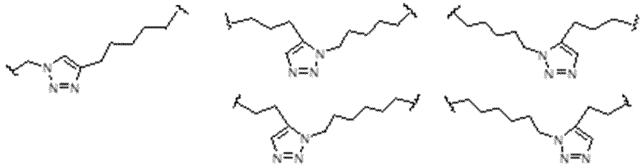

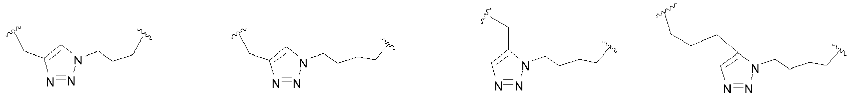

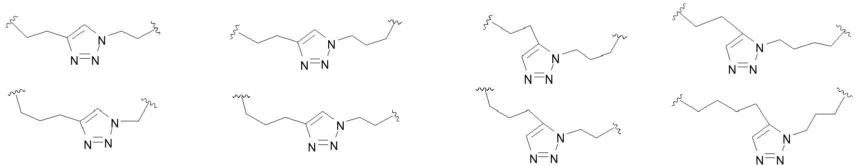

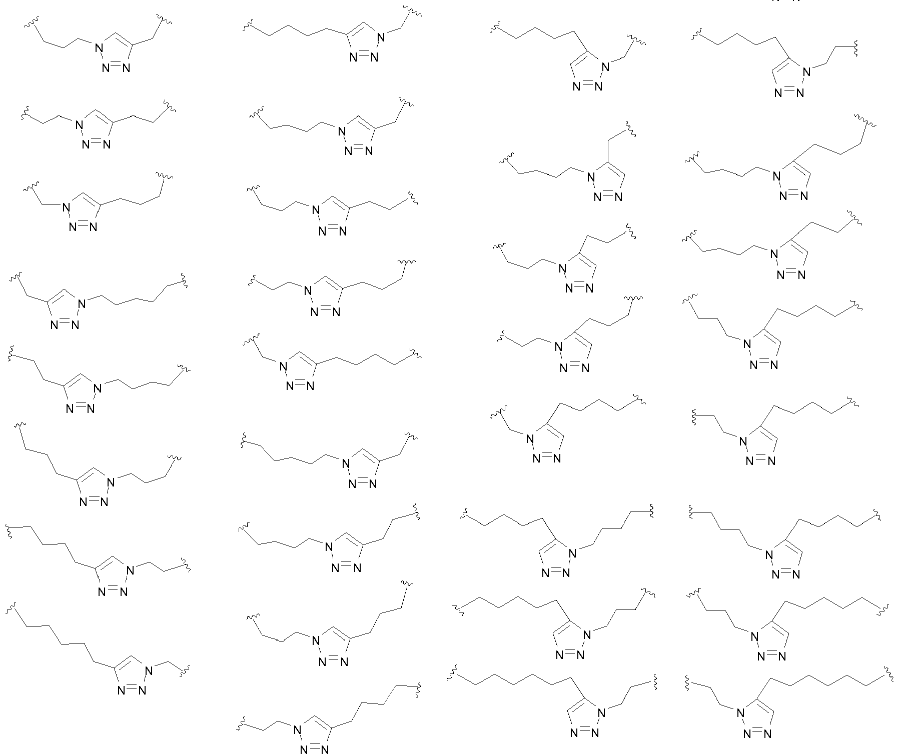

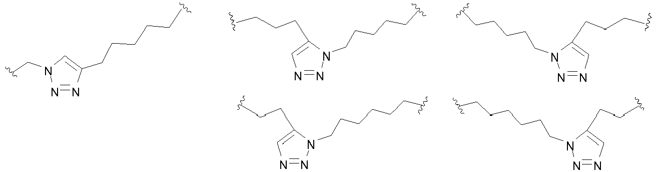

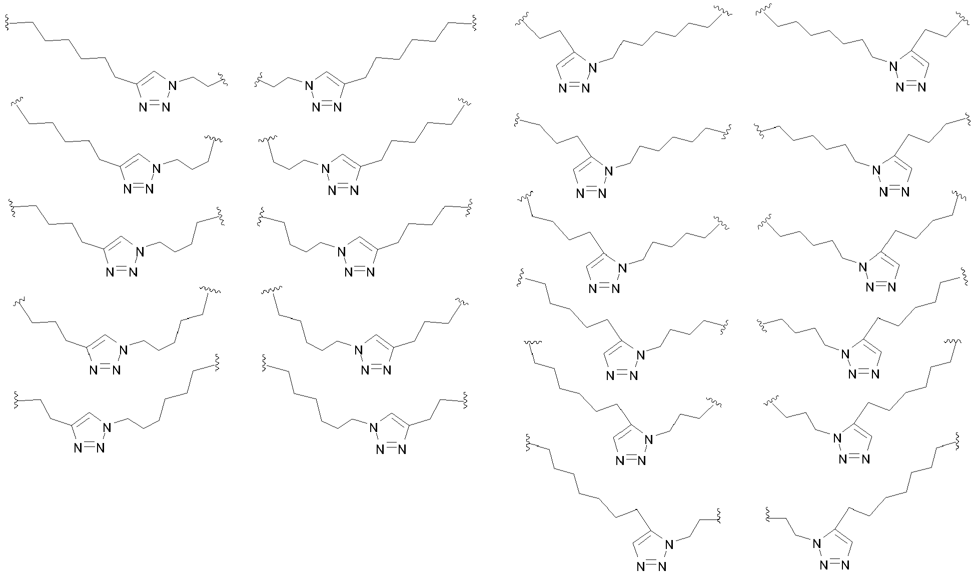

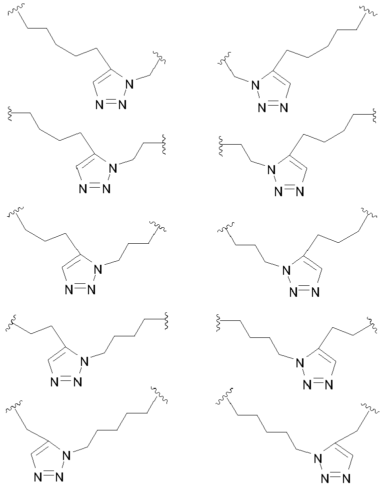

Amino acids which are used in the formation of triazole crosslinkers are represented according to the legend indicated below. Stereochemistry at the alpha position of each amino acid is S unless otherwise indicated. For azide amino acids, the number of carbon atoms indicated refers to the number of methylene units between the alpha carbon and the terminal azide. For alkyne amino acids, the number of carbon atoms indicated is the number of methylene units between the alpha position and the triazole moiety plus the two carbon atoms within the triazole group derived from the alkyne.

| | |
|---|---|
| $5a5 | Alpha-Me alkyne 1,5 triazole (5 carbon) |
| $5n3 | Alpha-Me azide 1,5 triazole (3 carbon) |
| $4rn6 | Alpha-Me R-azide 1,4 triazole (6 carbon) |
| $4a5 | Alpha-Me alkyne 1,4 triazole (5 carbon) |

In some cases, any of the macrocycle-forming linkers described herein can be used in any combination with any of the sequences shown in Table 1, Table 1a, Table 1b, or Table 1c and also with any of the R- substituents indicated herein.

In some cases, the peptidomimetic macrocycle comprises at least one α-helix motif. For example, A, B and/or C in the compound of Formula I include one or more α-helices. As a general matter, α-helices include between 3 and 4 amino acid residues per turn. In some cases, the α-helix of the peptidomimetic macrocycle includes 1 to 5 turns and, therefore, 3 to 20 amino acid residues. In specific cases, the α-helix includes 1 turn, 2 turns, 3 turns, 4 turns, or 5 turns. In some cases, the macrocycle-forming linker stabilizes an α-helix motif included within the peptidomimetic macrocycle. Thus, in some cases, the length of the macrocycle-forming linker L from a first Cα to a second Cα is selected to increase the stability of an α-helix. In some cases, the macrocycle-forming linker spans from 1 turn to 5 turns of the α-helix. In some cases, the macrocycle-forming linker spans approximately 1 turn, 2 turns, 3 turns, 4 turns, or 5 turns of the α-helix. In some cases, the length of the macrocycle-forming linker is approximately 5 Å to 9 Å per turn of the α-helix, or approximately 6 Å to 8 Å per turn of the α-helix. Where the macrocycle-forming linker spans approximately 1 turn of an α-helix, the length is equal to approximately 5 carbon-carbon bonds to 13 carbon-carbon bonds, approximately 7 carbon-carbon bonds to 11 carbon-carbon bonds, or approximately 9 carbon-carbon bonds. Where the macrocycle-forming linker spans approximately 2 turns of an α-helix, the length is equal to approximately 8 carbon-carbon bonds to 16 carbon-carbon bonds, approximately 10 carbon-carbon bonds to 14 carbon-carbon bonds, or approximately 12 carbon-carbon bonds. Where the macrocycle-forming linker spans approximately 3 turns of an α-helix, the length is equal to approximately 14 carbon-carbon bonds to 22 carbon-carbon bonds, approximately 16 carbon-carbon bonds to 20 carbon-carbon bonds, or approximately 18 carbon-carbon bonds. Where the macrocycle-forming linker spans approximately 4 turns of an α-helix, the length is equal to approximately 20 carbon-carbon bonds to 28 carbon-carbon bonds, approximately 22 carbon-carbon bonds to 26 carbon-carbon bonds, or approximately 24 carbon-carbon bonds. Where the macrocycle-forming linker spans approximately 5 turns of an α-helix, the length is equal to approximately 26 carbon-carbon bonds to 34 carbon-carbon bonds, approximately 28 carbon-carbon bonds to 32 carbon-carbon bonds, or approximately 30 carbon-carbon bonds. Where the macrocycle-forming linker spans approximately 1 turn of an α-helix, the linkage contains approximately 4 atoms to 12 atoms, approximately 6 atoms to 10 atoms, or approximately 8 atoms. Where the macrocycle-forming linker spans approximately 2 turns of the α-helix, the linkage contains approximately 7 atoms to 15 atoms, approximately 9 atoms to 13 atoms, or approximately 11 atoms. Where the macrocycle-forming linker spans approximately 3 turns of the α-helix, the linkage contains approximately 13 atoms to 21 atoms, approximately 15 atoms to 19 atoms, or approximately 17 atoms. Where the macrocycle-forming linker spans approximately 4 turns of the α-helix, the linkage contains approximately 19 atoms to 27 atoms, approximately 21 atoms to 25 atoms, or approximately 23 atoms. Where the macrocycle-forming linker spans approximately 5 turns of the α-helix, the linkage contains approximately 25 atoms to 33 atoms, approximately 27 atoms to 31 atoms, or approximately 29 atoms. Where the macrocycle-forming linker spans approximately 1 turn of the α-helix, the resulting macrocycle forms a ring containing approximately 17 members to 25 members, approximately 19 members to 23 members, or approximately 21 members. Where the macrocycle-forming linker spans approximately 2 turns of the α-helix, the resulting macrocycle forms a ring containing approximately 29 members to 37 members, approximately 31 members to 35 members, or approximately 33 members. Where the macrocycle-forming linker spans approximately 3 turns of the α-helix, the resulting macrocycle forms a ring containing approximately 44 members to 52 members, approximately 46 members to 50 members, or approximately 48 members. Where the macrocycle-forming linker spans approximately 4 turns of the α-helix, the resulting macrocycle forms a ring containing approximately 59 members to 67 members, approximately 61 members to 65 members, or approximately 63 members. Where the macrocycle-forming linker spans approximately 5 turns of the α-helix, the resulting macrocycle forms a ring containing approximately 74 members to 82 members, approximately 76 members to 80 members, or approximately 78 members.

In other cases, described are peptidomimetic macrocycles of Formula (II) or (IIa): 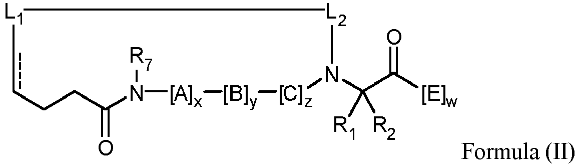 wherein: 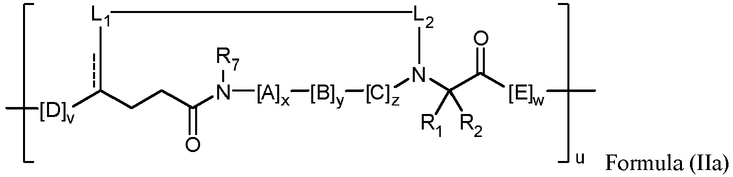
- each A, C, D, and E is independently a natural or non-natural amino acid or an amino acid analog, and the terminal D and E independently optionally include a capping group;
- each B is independently a natural or non-natural amino acid, amino acid analog, 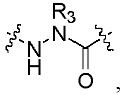 [-NH-L₃-CO-], [-NH-L₃-SO₂-], or [-NH-L₃-];
- each R₁ and R₂ is independently -H, alkyl, alkenyl, alkynyl, arylalkyl, cycloalkyl, cycloalkylalkyl, heteroalkyl, or heterocycloalkyl, unsubstituted or substituted with halo-; or at least one of R₁ and R₂ forms a macrocycle-forming linker L' connected to the alpha position of one of said D or E amino acids;
- each R₃ is independently hydrogen, alkyl, alkenyl, alkynyl, arylalkyl, heteroalkyl, cycloalkyl, heterocycloalkyl, cycloalkylalkyl, aryl, or heteroaryl, optionally substituted with R₅;
- each L and L' is a macrocycle-forming linker of the formula -L₁-L₂-;
- each L₁, L₂, and L₃ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
- each R₄ is independently alkylene, alkenylene, alkynylene, heteroalkylene, cycloalkylene, heterocycloalkylene, arylene, or heteroarylene;
- each K is independently O, S, SO, SOz, CO, COz, or CONR₃;
- each R₅ is independently halogen, alkyl, -OR₆, -N(R₆)₂, -SR₆, -SOR₆, -SO₂R₅, -CO₂R₅, a fluorescent moiety, a radioisotope or a therapeutic agent;
- each R₆ is independently -H, alkyl, alkenyl, alkynyl, arylalkyl, cycloalkylalkyl, heterocycloalkyl, a fluorescent moiety, a radioisotope or a therapeutic agent;
- each R₇ is independently -H, alkyl, alkenyl, alkynyl, arylalkyl, cycloalkyl, heteroalkyl, cycloalkylalkyl, heterocycloalkyl, aryl, or heteroaryl, optionally substituted with R₅;
- each v and w is independently an integer from 1-1000;
- u is an integer from 1-10;
- each x, y, and z is independently integers from 0-10; and
n is an integer from 1-5.

In one example, L₁ and Lz, either alone or in combination, do not form a triazole or a thioether.

In one example, at least one of R₁ and R₂ is alkyl, unsubstituted or substituted with halo-. In another example, both R₁ and R₂ are independently alkyl, unsubstituted or substituted with halo-. In some cases, at least one of R₁ and R₂ is methyl. In other cases, R₁ and R₂ are methyl.

In some cases, x+y+z is at least 1. In other cases, x+y+z is at least 2. In other cases, x+y+z is 1, 2, 3, 4, 5, 6, 7, 8, 9 or 10. Each occurrence of A, B, C, D or E in a macrocycle or macrocycle precursor is independently selected. For example, a sequence represented by the formula [A]ₓ, when x is 3, encompasses cases wherein the amino acids are not identical, e.g. Gln-Asp-Ala as well as cases wherein the amino acids are identical, e.g. Gln-Gln-Gln. This applies for any value of x, y, or z in the indicated ranges.

In some cases, the peptidomimetic macrocycle comprises a secondary structure which is an α-helix and R₈ is -H, allowing intrahelical hydrogen bonding. In some cases, at least one of A, B, C, D or E is an α,α-disubstituted amino acid. In one example, B is an α,α-disubstituted amino acid. For example, at least one of A, B, C, D or E is 2-aminoisobutyric acid. In other cases, at least one of A, B, C, D or E is

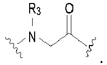

In other cases, the length of the macrocycle-forming linker L as measured from a first Cα to a second Cα is selected to stabilize a desired secondary peptide structure, such as an α-helix formed by residues of the peptidomimetic macrocycle including, but not necessarily limited to, those between the first Cα to a second Cα.

Exemplary cases of the macrocycle-forming linker -L₁-L₂- are shown below.

| | |
|---|---|
| 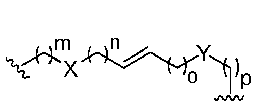 | 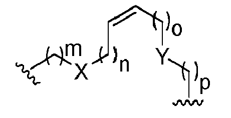 |
| where X, Y = -CH₂-, O, S, or NH | where X, Y = -CH₂-, O, S, or NH |
| m, n, o, p = 0-10 | m, n, o, p = 0-10 |
| 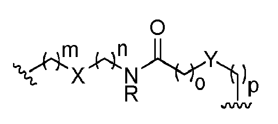 | 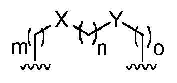 |
| where X, Y = -CH₂-, O, S, or NH | where X, Y = -CH₂-, O, S, or NH |
| m, n, o, p = 0-10 | m, n, o = 0-10 |
| R = H, alkyl, other substituent | |

In some cases, the peptidomimetic macrocycle has the Formula (III) or Formula (IIIa): 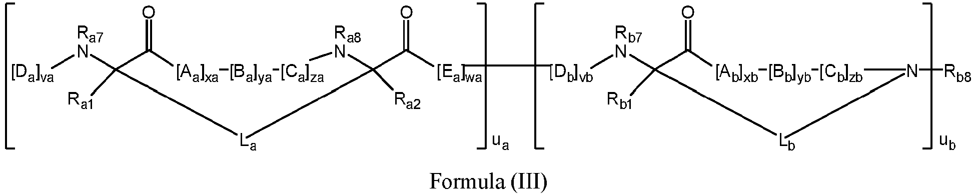 or 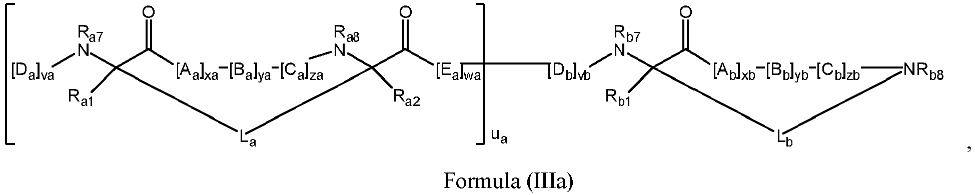 wherein:
- each Aₐ, Cₐ, Dₐ, Eₐ, A_{b}, C_{b}, and D_{b} is independently a natural or non-natural amino acid or an amino acid analog;
- each Bₐ and B_{b} is independently a natural or non-natural amino acid, amino acid analog, 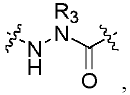 [-NH-L₄-CO-], [-NH-L₄-SO₂-], or [-NH-L₄-];
- each Rₐ₁ is independently alkyl, alkenyl, alkynyl, arylalkyl, cycloalkyl, cycloalkylalkyl, heteroalkyl, or heterocycloalkyl, any of which is unsubstituted or substituted; or H; or Rₐ₁ forms a macrocycle-forming linker L' connected to the alpha position of one of the Dₐ or Eₐ amino acids; or together with Lₐ forms a ring that is unsubstituted or substituted;
- each Rₐ₂ is independently alkyl, alkenyl, alkynyl, arylalkyl, cycloalkyl, cycloalkylalkyl, heteroalkyl, or heterocycloalkyl, any of which is unsubstituted or substituted; or H; or Rₐ₂ forms a macrocycle-forming linker L' connected to the alpha position of one of the Dₐ or Eₐ amino acids; or together with Lₐ forms a ring that is unsubstituted or substituted;
- each R_{b1} is independently alkyl, alkenyl, alkynyl, arylalkyl, cycloalkyl, cycloalkylalkyl, heteroalkyl, or heterocycloalkyl, any of which is unsubstituted or substituted; or H; or R_{b1} forms a macrocycle-forming linker L' connected to the alpha position of one of the D_{b} amino acids; or together with L_{b} forms a ring that is unsubstituted or substituted;
- each R₃ is independently alkyl, alkenyl, alkynyl, arylalkyl, heteroalkyl, cycloalkyl, heterocycloalkyl, cycloalkylalkyl, cycloaryl, or heterocycloaryl, any of which is unsubstituted or substituted, or H;
- each Lₐ is independently a macrocycle-forming linker, and optionally forms a ring with Rₐ₁ or Rₐ₂ that is unsubstituted or substituted;
- each L_{b} is independently a macrocycle-forming linker, and optionally forms a ring with R_{b1} that is unsubstituted or substituted;
- each L' is independently a macrocycle-forming linker;
- each L₄ is independently alkylene, alkenylene, alkynylene, heteroalkylene, cycloalkylene, heterocycloalkylene, cycloarylene, heterocycloarylene, or [-R₄-K-R₄-]ₙ, any of which is unsubstituted or substituted;
- each R₄ is independently alkylene, alkenylene, alkynylene, heteroalkylene, cycloalkylene, heterocycloalkylene, arylene, or heteroarylene, any of which is unsubstituted or substituted;
- each K is independently O, S, SO, SO₂, CO, CO₂, OCO₂, NR₃, CONR₃, OCONR₃, OSO₂NR₃, NR_{3q}, CONR_{3q}, OCONR_{3q}, or OSO₂NR_{3q}, wherein each R_{3q} is independently a point of attachment to Rₐ₁, Rₐ₂, or Rbi;
- Rₐ₇ is alkyl, alkenyl, alkynyl, arylalkyl, cycloalkyl, heteroalkyl, cycloalkylalkyl, heterocycloalkyl, cycloaryl, or heterocycloaryl, any of which is unsubstituted or substituted; or H; or part of a cyclic structure with a Dₐ amino acid;
- R_{b7} is alkyl, alkenyl, alkynyl, arylalkyl, cycloalkyl, heteroalkyl, cycloalkylalkyl, heterocycloalkyl, cycloaryl, or heterocycloaryl, any of which is unsubstituted or substituted; or H; or part of a cyclic structure with a D_{b} amino acid;
- Rₐ₈ is alkyl, alkenyl, alkynyl, arylalkyl, cycloalkyl, heteroalkyl, cycloalkylalkyl, heterocycloalkyl, cycloaryl, or heterocycloaryl, any of which is unsubstituted or substituted; or H; or part of a cyclic structure with an Eₐ amino acid;
- R_{b8} is alkyl, alkenyl, alkynyl, arylalkyl, cycloalkyl, heteroalkyl, cycloalkylalkyl, heterocycloalkyl, cycloaryl, or heterocycloaryl, any of which is unsubstituted or substituted; or H; or an amino acid sequence of 1-1000 amino acid residues;
- each va and vb is independently an integer from 0-1000;
- each wa and wb is independently an integer from 0-1000;
- each ua and ub is independently 0, 1, 2, 3, 4, 5, 6, 7, 8, 9, or 10, wherein ua+ub is at least 1;
- each xa and xb is independently 0, 1, 2, 3, 4, 5, 6, 7, 8, 9, or 10;
- each ya and yb is independently 0, 1, 2, 3, 4, 5, 6, 7, 8, 9, or 10;
- each za and zb is independently 0, 1, 2, 3, 4, 5, 6, 7, 8, 9, or 10; and
- each n is independently 1, 2, 3, 4, or 5,
or a pharmaceutically-acceptable salt thereof.

In some cases, the peptidomimetic macrocycle has the Formula (III) or Formula (IIIa): 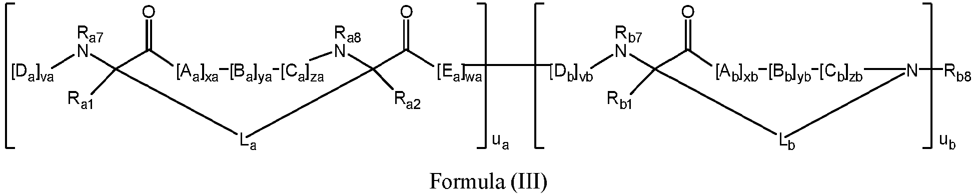 or 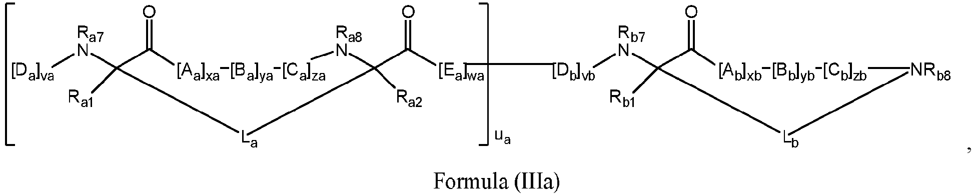 wherein:
- each Aₐ, Cₐ, Dₐ, Eₐ, A_{b}, C_{b}, and D_{b} is independently a natural or non-natural amino acid or an amino acid analgo;
- each Bₐ and B_{b} is independently a natural or non-natural amino acid, amino acid analog, 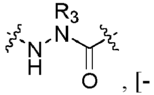 NH-L₄-CO-], [-NH-L₄-SO₂-], or [-NH-L₄-];
- each Rₐ₁ is independently alkyl, alkenyl, alkynyl, arylalkyl, cycloalkyl, cycloalkylalkyl, heteroalkyl, or heterocycloalkyl, any of which is unsubstituted or substituted; or H; or Rₐ₁ forms a macrocycle-forming linker L' connected to the alpha position of one of the Dₐ or Eₐ amino acids; or together with Lₐ forms a ring that is unsubstituted or substituted;
- each Rₐ₂ is independently alkyl, alkenyl, alkynyl, arylalkyl, cycloalkyl, cycloalkylalkyl, heteroalkyl, or heterocycloalkyl, any of which is unsubstituted or substituted; or H; or Rₐ₂ forms a macrocycle-forming linker L' connected to the alpha position of one of the Dₐ or Eₐ amino acids; or together with Lₐ forms a ring that is unsubstituted or substituted;
- each R_{b1} is independently alkyl, alkenyl, alkynyl, arylalkyl, cycloalkyl, cycloalkylalkyl, heteroalkyl, or heterocycloalkyl, any of which is unsubstituted or substituted; or H; or R_{b1} forms a macrocycle-forming linker L' connected to the alpha position of one of the D_{b} amino acids; or together with L_{b} forms a ring that is unsubstituted or substituted;
- each R₃ is independently alkyl, alkenyl, alkynyl, arylalkyl, heteroalkyl, cycloalkyl, heterocycloalkyl, cycloalkylalkyl, cycloaryl, or heterocycloaryl, any of which is unsubstituted or substituted with R₅, or H;
- each Lₐ is independently a macrocycle-forming linker, and optionally forms a ring with Rₐ₁ or Rₐ₂ that is unsubstituted or substituted;
- each L_{b} is independently a macrocycle-forming linker, and optionally forms a ring with R_{b1} that is unsubstituted or substituted;
- each L' is independently a macrocycle-forming linker;
- each L₄ is independently alkylene, alkenylene, alkynylene, heteroalkylene, cycloalkylene, heterocycloalkylene, cycloarylene, heterocycloarylene, or [-R₄-K-R₄-]ₙ, any of which is unsubstituted or substituted with R₅;
- each R₄ is independently alkylene, alkenylene, alkynylene, heteroalkylene, cycloalkylene, heterocycloalkylene, arylene, or heteroarylene, any of which is unsubstituted or substituted with R₅;
- each K is independently O, S, SO, SO₂, CO, CO₂, OCO₂, NR₃, CONR₃, OCONR₃, OSO₂NR₃, NR_{3q}, CONR_{3q}, OCONR_{3q}, or OSO₂NR_{3q}, wherein each R_{3q} is independently a point of attachment to Rₐ₁, Rₐ₂, or Rbi;
- each R₅ is independently halogen, alkyl, -OR₅, -N(R₆)₂, -SR₆, -SOR₆, -SO₂R₅, -CO₂R₅, a fluorescent moiety, a radioisotope, or a therapeutic agent;
- each R₆ is independently H, alkyl, alkenyl, alkynyl, arylalkyl, cycloalkylalkyl, heterocycloalkyl, a fluorescent moiety, a radioisotope or a therapeutic agent;
- each Rₐ₇ is independently alkyl, alkenyl, alkynyl, arylalkyl, cycloalkyl, heteroalkyl, cycloalkylalkyl, heterocycloalkyl, cycloaryl, or heterocycloaryl, any of which is unsubstituted or substituted with R₅; or H; or part of a cyclic structure with a Dₐ amino acid;
- R_{b7} is alkyl, alkenyl, alkynyl, arylalkyl, cycloalkyl, heteroalkyl, cycloalkylalkyl, heterocycloalkyl, cycloaryl, or heterocycloaryl, any of which is unsubstituted or substituted with R₅; or H; or part of a cyclic structure with a D_{b} amino acid;
- each Rₐ₈ is independently alkyl, alkenyl, alkynyl, arylalkyl, cycloalkyl, heteroalkyl, cycloalkylalkyl, heterocycloalkyl, cycloaryl, or heterocycloaryl, any of which is unsubstituted or substituted with R₅; or H; or part of a cyclic structure with an Eₐ amino acid;
- R_{b8} is alkyl, alkenyl, alkynyl, arylalkyl, cycloalkyl, heteroalkyl, cycloalkylalkyl, heterocycloalkyl, cycloaryl, or heterocycloaryl, any of which is unsubstituted or substituted with R₅; or H; or an amino acid sequence of 1-1000 amino acid residues;
- each va and vb is independently an integer from 0-1000;
- each wa and wb is independently an integer from 0-1000;
- each ua and ub is independently 0, 1, 2, 3, 4, 5, 6, 7, 8, 9, or 10, wherein ua+ub is at least 1;
- each xa and xb is independently 0, 1, 2, 3, 4, 5, 6, 7, 8, 9, or 10;
- each ya and yb is independently 0, 1, 2, 3, 4, 5, 6, 7, 8, 9, or 10;
- each za and zb is independently 0, 1, 2, 3, 4, 5, 6, 7, 8, 9, or 10; and
- each n is independently 1, 2, 3, 4, or 5,
or a pharmaceutically-acceptable salt thereof.

In some cases, the peptidomimetic macrocycle of the invention has the formula defined above, wherein:
- each Lₐ is independently a macrocycle-forming linker of the formula -L₁-L₂-, and optionally forms a ring with Rₐ₁ or Rₐ₂ that is unsubstituted or substituted;
- each L_{b} is independently a macrocycle-forming linker of the formula -L₁-L₂-, and optionally forms a ring with R_{b1} that is unsubstituted or substituted;
- each L' is independently a macrocycle-forming linker of the formula -L₁-L₂-;
- each L₁ and L₂ is independently alkylene, alkenylene, alkynylene, heteroalkylene, cycloalkylene, heterocycloalkylene, cycloarylene, heterocycloarylene, or [-R₄-K-R₄-]ₙ, any of which is unsubstituted or substituted with R₅;
- each R₄ is independently alkylene, alkenylene, alkynylene, heteroalkylene, cycloalkylene, heterocycloalkylene, arylene, or heteroarylene, any of which is unsubstituted or substituted with R₅;
- each K is independently O, S, SO, SO₂, CO, CO₂, OCO₂, NR₃, CONR₃, OCONR₃, OSO₂NR₃, NR_{3q}, CONR_{3q}, OCONR_{3q}, or OSO₂NR_{3q}, wherein each R_{3q} is independently a point of attachment to Rₐ₁, Rₐ₂, or R_{b1};
- each R₅ is independently halogen, alkyl, -OR₅, -N(R₆)₂, -SR₆, -SOR₆, -SO₂R₆, -CO₂R₅, a fluorescent moiety, a radioisotope, or a therapeutic agent; and
- each R₆ is independently H, alkyl, alkenyl, alkynyl, arylalkyl, cycloalkylalkyl, heterocycloalkyl, a fluorescent moiety, a radioisotope or a therapeutic agent,
or a pharmaceutically-acceptable salt thereof.

In some cases, the peptidomimetic macrocycle has the formula defined above wherein one of Lₐ and L_{b} is a bis-thioether-containing macrocycle-forming linker. In some cases, one of Lₐ and L_{b} is a macrocycle-forming linker of the formula -L₁-S-L₂-S-L₃-.

In some cases, the peptidomimetic macrocycle has the formula defined above wherein one of Lₐ and L_{b} is a bis-sulfone-containing macrocycle-forming linker. In some cases, one of Lₐ and L_{b} is a macrocycle-forming linker of the formula -L₁-SO₂-L₂-SO₂-L₃-.

In some cases, the peptidomimetic macrocycle has the formula defined above wherein one of Lₐ and L_{b} is a bis-sulfoxide-containing macrocycle-forming linker. In some cases, one of Lₐ and L_{b} is a macrocycle-forming linker of the formula -L₁-S(O)-L₂-S(O)-L₃-.

In some cases, a peptidomimetic macrocycle of the invention comprises one or more secondary structures. In some cases, the peptidomimetic macrocycle comprises a secondary structure that is an α-helix. In some cases, the peptidomimetic macrocycle comprises a secondary structure that is a β-hairpin turn.

In some cases, uₐ is 0. In some cases, uₐ is 0, and L_{b} is a macrocycle-forming linker that crosslinks an α-helical secondary structure. In some cases, uₐ is 0, and L_{b} is a macrocycle-forming linker that crosslinks a β-hairpin secondary structure. In some cases, uₐ is 0, and L_{b} is a hydrocarbon-containing macrocycle-forming linker that crosslinks an α-helical secondary structure. In some cases, uₐ is 0, and L_{b} is a hydrocarbon-containing macrocycle-forming linker that crosslinks a β-hairpin secondary structure.

In some cases, u_{b} is 0. In some cases, u_{b} is 0, and Lₐ is a macrocycle-forming linker that crosslinks an α-helical secondary structure. In some cases, u_{b} is 0, and Lₐ is a macrocycle-forming linker that crosslinks a β-hairpin secondary structure. In some cases, u_{b} is 0, and Lₐ is a hydrocarbon-containing macrocycle-forming linker that crosslinks an α-helical secondary structure. In some cases, u_{b} is 0, and Lₐ is a hydrocarbon-containing macrocycle-forming linker that crosslinks a β-hairpin secondary structure.

In some cases, the peptidomimetic macrocycle comprises only α-helical secondary structures. In other cases, the peptidomimetic macrocycle comprises only β-hairpin secondary structures.

In other cases, the peptidomimetic macrocycle comprises a combination of secondary structures, wherein the secondary structures are α-helical and β-hairpin structures. In some cases, Lₐ and L_{b} are a combination of hydrocarbon-, triazole, or sulfur-containing macrocycle-forming linkers. In some cases, the peptidomimetic macrocycle comprises Lₐ and L_{b}, wherein Lₐ is a hydrocarbon-containing macrocycle-forming linker that crosslinks a β-hairpin structure, and L_{b} is a triazole-containing macrocycle-forming linker that crosslinks an α-helical structure. In some cases, the peptidomimetic macrocycle comprises Lₐ and L_{b}, wherein Lₐ is a hydrocarbon-containing macrocycle-forming linker that crosslinks an α-helical structure, and L_{b} is a triazole-containing macrocycle-forming linker that crosslinks a β-hairpin structure. In some cases, the peptidomimetic macrocycle comprises Lₐ and L_{b}, wherein Lₐ is a triazole-containing macrocycle-forming linker that crosslinks an α-helical structure, and L_{b} is a hydrocarbon-containing macrocycle-forming linker that crosslinks a β-hairpin structure. In some cases, the peptidomimetic macrocycle comprises Lₐ and L_{b}, wherein Lₐ is a triazole-containing macrocycle-forming linker that crosslinks a β-hairpin structure, and L_{b} is a hydrocarbon-containing macrocycle-forming linker that crosslinks an α-helical structure.

In some cases, uₐ+u_{b} is at least 1. In some cases, uₐ+u_{b} = 2.

In some cases, uₐ is 1, u_{b} is 1, Lₐ is a triazole-containing macrocycle-forming linker that crosslinks an α-helical secondary structure, and L_{b} is a hydrocarbon-containing macrocycle-forming linker that crosslinks an α-helical structure. In some cases, uₐ is 1, u_{b} is 1, Lₐ is a triazole-containing macrocycle-forming linker that crosslinks an α-helical secondary structure, and L_{b} is a hydrocarbon-containing macrocycle-forming linker that crosslinks a β-hairpin structure. In some cases, uₐ is 1, u_{b} is 1, Lₐ is a triazole-containing macrocycle-forming linker that crosslinks a β-hairpin secondary structure, and L_{b} is a hydrocarbon-containing macrocycle-forming linker that crosslinks an α-helical structure. In some cases, uₐ is 1, u_{b} is 1, Lₐ is a triazole-containing macrocycle-forming linker that crosslinks a β-hairpin secondary structure, and L_{b} is a hydrocarbon-containing macrocycle-forming linker that crosslinks a β-hairpin structure.

In some cases, uₐ is 1, u_{b} is 1, Lₐ is a hydrocarbon-containing macrocycle-forming linker that crosslinks an α-helical secondary structure, and L_{b} is a triazole-containing macrocycle-forming linker that crosslinks an α-helical secondary structure. In some cases, uₐ is 1, u_{b} is 1, Lₐ is a hydrocarbon-containing macrocycle-forming linker that crosslinks an α-helical secondary structure, and L_{b} is a triazole-containing macrocycle-forming linker that crosslinks a β-hairpin secondary structure. In some cases, uₐ is 1, u_{b} is 1, Lₐ is a hydrocarbon-containing macrocycle-forming linker that crosslinks a β-hairpin secondary structure, and L_{b} is a triazole-containing macrocycle-forming linker that crosslinks an α-helical secondary structure. In some cases, uₐ is 1, u_{b} is 1, Lₐ is a hydrocarbon-containing macrocycle-forming linker that crosslinks a β-hairpin secondary structure, and L_{b} is a triazole-containing macrocycle-forming linker that crosslinks a β-hairpin secondary structure.

In some cases, uₐ is 1, u_{b} is 1, Lₐ is a hydrocarbon-containing macrocycle-forming linker with an α-helical secondary structure, and L_{b} is a sulfur-containing macrocycle-forming linker. In some cases, uₐ is 1, u_{b} is 1, Lₐ is a hydrocarbon-containing macrocycle-forming linker with a β-hairpin secondary structure, and L_{b} is a sulfur-containing macrocycle-forming linker.

In some cases, uₐ is 1, u_{b} is 1, Lₐ is a sulfur-containing macrocycle-forming linker, and L_{b} is a hydrocarbon-containing macrocycle-forming linker with an α-helical secondary structure. In some cases, uₐ is 1, u_{b} is 1, Lₐ is a sulfur-containing macrocycle-forming linker, and L_{b} is a hydrocarbon-containing macrocycle-forming linker with a β-hairpin secondary structure.

In some cases, uₐ is 1, u_{b} is 1, Lₐ is a hydrocarbon-containing macrocycle-forming linker that crosslinks an α-helical structure, and L_{b} is a hydrocarbon-containing macrocycle-forming linker that crosslinks an α-helical structure. In some cases, uₐ is 1, u_{b} is 1, Lₐ is a hydrocarbon-containing macrocycle-forming linker that crosslinks an α-helical structure, and L_{b} is a hydrocarbon-containing macrocycle-forming linker that crosslinks a β-hairpin structure. In some cases, uₐ is 1, u_{b} is 1, Lₐ is a hydrocarbon-containing macrocycle-forming linker that crosslinks a β-hairpin structure, and L_{b} is a hydrocarbon-containing macrocycle-forming linker that crosslinks an α-helical structure. In some cases, uₐ is 1, u_{b} is 1, Lₐ is a hydrocarbon-containing macrocycle-forming linker that crosslinks a β-hairpin structure, and L_{b} is a hydrocarbon-containing macrocycle-forming linker that crosslinks a β-hairpin structure.

In some cases, R_{b1} is H.

Unless otherwise stated, any compounds (including peptidomimetic macrocycles, macrocycle precursors, and other compositions) are also meant to encompass compounds which differ only in the presence of one or more isotopically enriched atoms. For example, compounds having the described structures except for the replacement of a hydrogen atom by deuterium or tritium, or the replacement of a carbon atom by ¹³Cor ¹⁴C are contemplated.

Unless otherwise stated, any compounds (including peptidomimetic macrocycles, macrocycle precursors, and other compositions) are also meant to encompass compounds which differ only in the presence of one or more isotopically enriched atoms. For example, compounds having the described structures except for the replacement of a hydrogen by a deuterium or tritium, or the replacement of a carbon by ¹³C- or ¹⁴C-enriched carbon are within the scope of this invention.

In some cases, the compounds disclosed herein can contain unnatural proportions of atomic isotopes at one or more of atoms that constitute such compounds. For example, the compounds can be radiolabeled with radioactive isotopes, such as for example tritium (³H), iodine-125 (¹²⁵I) or carbon-14 (¹⁴C). In other cases, one or more carbon atoms is replaced with a silicon atom. All isotopic variations of the compounds disclosed herein, whether radioactive or not, are contemplated herein.

### Preparation of Peptidomimetic Macrocycles

Peptidomimetic macrocycles can be prepared by any of a variety of methods known in the art. For example, any of the residues indicated by "$" or "$r8" in Table 1, Table 1a, Table 1b, or Table 1c can be substituted with a residue capable of forming a crosslinker with a second residue in the same molecule or a precursor of such a residue.

Various methods to effect formation of peptidomimetic macrocycles are known in the art. For example, the preparation of peptidomimetic macrocycles of Formula I is described in Schafmeister et al., J. Am. Chem. Soc. 122:5891-5892 (2000); Schafmeister & Verdine, J. Am. Chem. Soc. 122:5891 (2005); Walensky et al., Science 305: 1466-1470 (2004); US Patent No. 7,192,713 and PCT application WO 2008/121767. The α,α-disubstituted amino acids and amino acid precursors disclosed in the cited references can be employed in synthesis of the peptidomimetic macrocycle precursor polypeptides. For example, the "SS-olefin amino acid" is (S)-α-(2'-pentenyl) alanine and the "R8 olefin amino acid" is (R)-α-(2'-octenyl) alanine. Following incorporation of such amino acids into precursor polypeptides, the terminal olefins are reacted with a metathesis catalyst, leading to the formation of the peptidomimetic macrocycle. In various cases, the following amino acids can be employed in the synthesis of the peptidomimetic macrocycle:

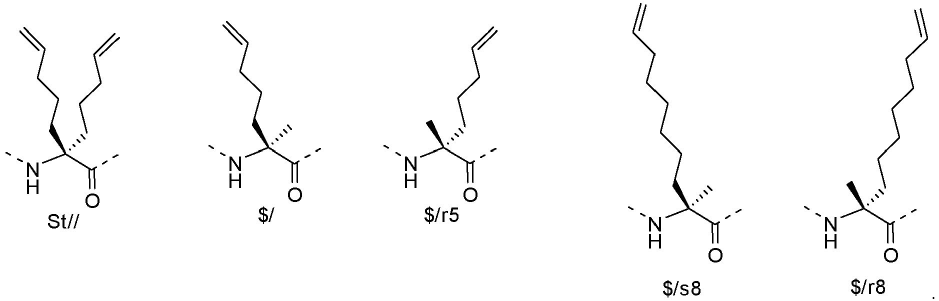

In other cases, the peptidomimetic macrocycles are of Formula IV or IVa. Methods for the preparation of such macrocycles are described, for example, in US Patent No. 7,202,332.

Additional methods of forming peptidomimetic macrocycles which are envisioned as suitable include those disclosed by Mustapa et al., J. Org. Chem. (2003), 68, pp. 8193-8198; Yang et al. Bioorg. Med. Chem. Lett. (2004), 14, pp. 1403-1406; U.S. Patent No. 5,364,851; U.S. Patent No. 5,446,128; U.S. Patent No. 5,824,483; U.S. Patent No. 6,713,280; and U.S. Patent No. 7,202,332. In such cases, amino acid precursors are used containing an additional substituent R- at the alpha position. Such amino acids are incorporated into the macrocycle precursor at the desired positions, which can be at the positions where the crosslinker is substituted or, alternatively, elsewhere in the sequence of the macrocycle precursor. Cyclization of the precursor is then effected according to the indicated method.

### Assays

The properties of peptidomimetic macrocycles are assayed, for example, by using the methods described below. In some cases, a peptidomimetic macrocycle has improved biological properties relative to a corresponding polypeptide lacking the substituents described herein.

### Biological Samples

As used in the present application, "biological sample" means any fluid or other material derived from the body of a normal or diseased subject, such as blood, serum, plasma, lymph, urine, saliva, tears, cerebrospinal fluid, milk, amniotic fluid, bile, ascites fluid, pus, and the like. Also included within the meaning of the term "biological sample" is an organ or tissue extract and culture fluid in which any cells or tissue preparation from a subject has been incubated. The biological samples can be any samples from which genetic material can be obtained. Biological samples can also include solid or liquid cancer cell samples or specimens. The cancer cell sample can be a cancer cell tissue sample. In some cases, the cancer cell tissue sample can obtained from surgically excised tissue. Exemplary sources of biological samples include fine needle aspiration, core needle biopsy, vacuum assisted biopsy, incisional biopsy, excisional biopsy, punch biopsy, shave biopsy or skin biopsy. In some cases, the biological samples comprise fine needle aspiration samples. In some cases, the biological samples comprise tissue samples, including, for example, excisional biopsy, incisional biopsy, or other biopsy. The biological samples can comprise a mixture of two or more sources; for example, fine needle aspirates and tissue samples. Tissue samples and cellular samples can also be obtained without invasive surgery, for example by punctuating the chest wall or the abdominal wall or from masses of breast, thyroid or other sites with a fine needle and withdrawing cellular material (fine needle aspiration biopsy). In some cases, a biological sample is a bone marrow aspirate sample. A biological sample can be obtained by methods known in the art such as the biopsy methods described herein, swabbing, scraping, phlebotomy, or any other suitable method.

### Methods of detecting wild type p53 and/or p53 mutations

In some cases, a subject lacking p53-deactivating mutations is a candidate for cancer treatment with a compound of the invention. Cancer cells from patient groups should be assayed in order to determine p53-deactivating mutations and/or expression of wild type p53 prior to treatment with a compound of the invention.

The activity of the p53 pathway can be determined by the mutational status of genes involved in the p53 pathways, including, for example, AKT1, AKT2, AKT3, ALK, BRAF, CDK4, CDKN2A, DDR2, EGFR, ERBB2 (HER2), FGFR1, FGFR3, GNA11, GNQ, GNAS, KDR, KIT, KRAS, MAP2K1 (MEK1), MET, HRAS, NOTCH1, NRAS, NTRK2, PIK3CA, NF1, PTEN, RAC1, RB1, NTRK3, STK11, PIK3R1, TSC1, TSC2, RET, TP53, and VHL. Genes that modulate the activity of p53 can also be assessed, including, for example, kinases: ABL1, JAK1, JAAK2, JAK3; receptor tyrosine kinases: FLT3 and KIT; receptors: CSF3R, IL7R, MPL, and NOTCH1; transcription factors: BCOR, CEBPA, CREBBP, ETV6, GATA1, GATA2. MLL, KZF1, PAX5, RUNX1, STAT3, WT1, and TP53; epigenetic factors: ASXL1, DNMT3A, EZH2, KDM6A (UTX), SUZ12, TET2, PTPN11, SF3B1, SRSF2, U2AF35, ZRSR2; RAS proteins: HRAS, KRAS, and NRAS; adaptors CBL and CBL-B; FBXW7, IDH1, IDH2, and NPM1.

Cancer cell samples can be obtained, for example, from solid or liquid tumors via primary or metastatic tumor resection (e.g. pneumonectomy, lobetomy, wedge resection, and craniotomy) primary or metastatic disease biopsy (e.g. transbronchial or needle core), pleural or ascites fluid (e.g. FFPE cell pellet), bone marrow aspirate, bone marrow clot, and bone marrow biopsy, or macro-dissection of tumor rich areas (solid tumors).

To detect the p53 wild type gene and/or lack of p53 deactivation mutation in a tissue, cancerous tissue can be isolated from surrounding normal tissues. For example, the tissue can be isolated from paraffin or cryostat sections. Cancer cells can also be separated from normal cells by flow cytometry. If the cancer cells tissue is highly contaminated with normal cells, detection of mutations can be more difficult.

Various methods and assays for analyzing wild type p53 and/or p53 mutations are suitable for use in the invention. Non-limiting examples of assays include polymerase chain reaction (PCR), restriction fragment length polymorphism (RFLP), microarray, Southern Blot, Northern Blot, Western Blot, Eastern Blot, H&E staining, microscopic assessment of tumors, next-generation DNA sequencing (NGS) (e.g. extraction, purification, quantification, and amplification of DNA, library preparation) immunohistochemistry, and fluorescent *in situ* hybridization (FISH).

A microarray allows a researcher to investigate multiple DNA sequences attached to a surface, for example, a DNA chip made of glass or silicon, or a polymeric bead or resin. The DNA sequences are hybridized with fluorescent or luminescent probes. The microarray can indicate the presence of oligonucleotide sequences in a sample based on hybridization of sample sequences to the probes, followed by washing and subsequent detection of the probes. Quantification of the fluorescent or luminescent signal indicates the presence of known oligonucleotide sequences in the sample.

PCR allows amplification of DNA oligomers rapidly, and can be used to identify an oligonucleotide sequence in a sample. PCR experiments involve contacting an oligonucleotide sample with a PCR mixture containing primers complementary to a target sequence, one or more DNA polymerase enzymes, deoxnucleotide triphosphate (dNTP) building blocks, including dATP, dGTP, dTTP, and dCTP, and suitable buffers, salts, and additives. If a sample contains an oligonucleotide sequence complementary to a pair of primers, the experiment amplifies the sample sequence, which can be collected and identified.

In some cases, an assay comprises amplifying a biomolecule from the cancer sample. The biomolecule can be a nucleic acid molecule, such as DNA or RNA. In some cases, the assay comprises circularization of a nucleic acid molecule, followed by digestion of the circularized nucleic acid molecule.

In some cases, the assay comprises contacting an organism, or a biochemical sample collected from an organism, such as a nucleic acid sample, with a library of oligonucleotides, such as PCR primers. The library can contain any number of oligonucleotide molecules. The oligonucleotide molecules can bind individual DNA or RNA motifs, or any combination of motifs described herein. The motifs can be any distance apart, and the distance can be known or unknown. In some cases, two or more oligonucleotides in the same library bind motifs a known distance apart in a parent nucleic acid sequence. Binding of the primers to the parent sequence can take place based on the complementarity of the primers to the parent sequence. Binding can take place, for example, under annealing, or under stringent conditions.

In some cases, the results of an assay are used to design a new oligonucleotide sequence for future use. In some cases, the results of an assay are used to design a new oligonucleotide library for future use. In some cases, the results of an assay are used to revise, refine, or update an existing oligonucleotide library for future use. For example, an assay can reveal that a previously-undocumented nucleic acid sequence is associated with the presence of a target material. This information can be used to design or redesign nucleic acid molecules and libraries.

In some cases, one or more nucleic acid molecules in a library comprise a barcode tag. In some cases, one or more of the nucleic acid molecules in a library comprise type I or type II restriction sites suitable for circularization and cutting an amplified sample nucleic acid sequence. Such primers can be used to circularize a PCR product and cut the PCR product to provide a product nucleic acid sequence with a sequence that is organized differently from the nucleic acid sequence native to the sample organism.

After a PCR experiment, the presence of an amplified sequence can be verified. Non-limiting examples of methods for finding an amplified sequence include DNA sequencing, whole transcriptome shotgun sequencing (WTSS, or RNA-seq), mass spectrometry (MS), microarray, pyrosequencing, column purification analysis, polyacrylamide gel electrophoresis, and index tag sequencing of a PCR product generated from an index-tagged primer.

In some cases, more than one nucleic acid sequence in the sample organism is amplified. Non-limiting examples of methods of separating different nucleic acid sequences in a PCR product mixture include column purification, high performance liquid chromatography (HPLC), HPLC/MS, polyacrylamide gel electrophoresis, size exclusion chromatography.

The amplified nucleic acid molecules can be identified by sequencing. Nucleic acid sequencing can be done on automated instrumentation. Sequencing experiments can be done in parallel to analyze tens, hundreds, or thousands of sequences simultaneously. Non-limiting examples of sequencing techniques follow.

In pyrosequencing, DNA is amplified within a water droplet containing a single DNA template bound to a primer-coated bead in an oil solution. Nucleotides are added to a growing sequence, and the addition of each base is evidenced by visual light.

Ion semiconductor sequencing detects the addition of a nucleic acid residue as an electrical signal associated with a hydrogen ion liberated during synthesis. A reaction well containing a template is flooded with the four types of nucleotide building blocks, one at a time. The timing of the electrical signal identifies which building block was added, and identifies the corresponding residue in the template.

DNA nanoball uses rolling circle replication to amplify DNA into nanoballs. Unchained sequencing by ligation of the nanoballs reveals the DNA sequence.

In a reversible dyes approach, nucleic acid molecules are annealed to primers on a slide and amplified. Four types of fluorescent dye residues, each complementary to a native nucleobase, are added, the residue complementary to the next base in the nucleic acid sequence is added, and unincorporated dyes are rinsed from the slide. Four types of reversible terminator bases (RT-bases) are added, and non-incorporated nucleotides are washed away. Fluorescence indicates the addition of a dye residue, thus identifying the complementary base in the template sequence. The dye residue is chemically removed, and the cycle repeats.

Detection of point mutations can be accomplished by molecular cloning of the p53 allele(s) present in the cancer cell tissue and sequencing that allele(s). Alternatively, the polymerase chain reaction can be used to amplify p53 gene sequences directly from a genomic DNA preparation from the cancer cell tissue. The DNA sequence of the amplified sequences can then be determined. See e.g., Saiki et al., Science, Vol. 239, p. 487, 1988; U.S. Pat. No. 4,683,202; and U.S. Pat. No. 4,683,195. Specific deletions of p53 genes can also be detected. For example, restriction fragment length polymorphism (RFLP) probes for the p53 gene or surrounding marker genes can be used to score loss of a p53 allele.

Loss of wild type p53 genes can also be detected on the basis of the loss of a wild type expression product of the p53 gene. Such expression products include both the mRNA as well as the p53 protein product itself. Point mutations can be detected by sequencing the mRNA directly or via molecular cloning of cDNA made from the mRNA. The sequence of the cloned cDNA can be determined using DNA sequencing techniques. The cDNA can also be sequenced via the polymerase chain reaction (PCR).

Alternatively, mismatch detection can be used to detect point mutations in the p53 gene or the mRNA product. The method can involve the use of a labeled riboprobe that is complementary to the human wild type p53 gene. The riboprobe and either mRNA or DNA isolated from the cancer cell tissue are annealed (hybridized) together and subsequently digested with the enzyme RNase A which is able to detect some mismatches in a duplex RNA structure. If a mismatch is detected by RNase A, the enzyme cleaves at the site of the mismatch. Thus, when the annealed RNA preparation is separated on an electrophoretic gel matrix, if a mismatch has been detected and cleaved by RNase A, an RNA product is seen that is smaller than the full-length duplex RNA for the riboprobe and the p53 mRNA or DNA. The riboprobe need not be the full length of the p53 mRNA or gene but can be a segment of either. If the riboprobe comprises only a segment of the p53 mRNA or gene it will be desirable to use a number of these probes to screen the whole mRNA sequence for mismatches.

In similar fashion, DNA probes can be used to detect mismatches, through enzymatic or chemical cleavage. See, e.g., Cotton et al., Proc. Natl. Acad. Sci. USA, vol. 85, 4397, 1988; and Shenk et al., Proc. Natl. Acad. Sci. USA, vol. 72, p. 989, 1975. Alternatively, mismatches can be detected by shifts in the electrophoretic mobility of mismatched duplexes relative to matched duplexes. See, e.g., Cariello, Human Genetics, vol. 42, p. 726, 1988. With either riboprobes or DNA probes, the cellular mRNA or DNA which might contain a mutation can be amplified using PCR (see below) before hybridization.

DNA sequences of the p53 gene from the cancer cell tissue which have been amplified by use of polymerase chain reaction can also be screened using allele-specific probes. These probes are nucleic acid oligomers, each of which contains a region of the p53 gene sequence harboring a known mutation. For example, one oligomer can be about 30 nucleotides in length, corresponding to a portion of the p53 gene sequence. At the position coding for the 175th codon of p53 gene the oligomer encodes an alanine, rather than the wild type codon valine. By use of a battery of such allele-specific probes, the PCR amplification products can be screened to identify the presence of a previously identified mutation in the p53 gene. Hybridization of allele-specific probes with amplified p53 sequences can be performed, for example, on a nylon filter. Hybridization to a particular probe indicates the presence of the same mutation in the cancer cell tissue as in the allele-specific probe.

The identification of p53 gene structural changes in cancer cells can be facilitated through the application of a diverse series of high resolution, high throughput microarray platforms. Essentially two types of array include those that carry PCR products from cloned nucleic acids (e.g. cDNA, BACs, cosmids) and those that use oligonucleotides. The methods can provide a way to survey genome wide DNA copy number abnormalities and expression levels to allow correlations between losses, gains and amplifications in cancer cells with genes that are over- and under- expressed in the same samples. The gene expression arrays that provide estimates of mRNA levels in cancer cells have given rise to exon-specific arrays that can identify both gene expression levels, alternative splicing events and mRNA processing alterations.

Oligonucleotide arrays can be used to interrogate single nucleotide polymorphisms (SNPs) throughout the genome for linkage and association studies and these have been adapted to quantify copy number abnormalities and loss of heterozygosity events. DNA sequencing arrays can allow resequencing of chromosome regions, exomes, and whole genomes.

SNP-based arrays or other gene arrays or chips can determine the presence of wild type p53 allele and the structure of mutations. A single nucleotide polymorphism (SNP), a variation at a single site in DNA, is the most frequent type of variation in the genome. For example, there are an estimated 5-10 million SNPs in the human genome. SNPs can be synonymous or nonsynonymous substitutions. Synonymous SNP substitutions do not result in a change of amino acid in the protein due to the degeneracy of the genetic code, but can affect function in other ways. For example, a seemingly silent mutation in a gene that codes for a membrane transport protein can slow down translation, allowing the peptide chain to misfold, and produce a less functional mutant membrane transport protein. Nonsynonymous SNP substitutions can be missense substitutions or nonsense substitutions. Missense substitutions occur when a single base change results in change in amino acid sequence of the protein and malfunction thereof leads to disease. Nonsense substitutions occur when a point mutation results in a premature stop codon, or a nonsense codon in the transcribed mRNA, which results in a truncated and usually, nonfunctional, protein product. As SNPs are highly conserved throughout evolution and within a population, the map of SNPs serves as an excellent genotypic marker for research. SNP array is a useful tool to study the whole genome.

In addition, SNP array can be used for studying the Loss Of Heterozygosity (LOH). LOH is a form of allelic imbalance that can result from the complete loss of an allele or from an increase in copy number of one allele relative to the other. While other chip-based methods (e.g., comparative genomic hybridization can detect only genomic gains or deletions), SNP array has the additional advantage of detecting copy number neutral LOH due to uniparental disomy (UPD). In UPD, one allele or whole chromosome from one parent are missing leading to reduplication of the other parental allele (uni-parental = from one parent, disomy = duplicated). In a disease setting this occurrence can be pathologic when the wild type allele (e.g., from the mother) is missing and instead two copies of the heterozygous allele (e.g., from the father) are present. This usage of SNP array has a huge potential in cancer diagnostics as LOH is a prominent characteristic of most human cancers. SNP array technology have shown that cancers (e.g. gastric cancer, liver cancer, etc.) and hematologic malignancies (ALL, MDS, CML etc) have a high rate of LOH due to genomic deletions or UPD and genomic gains. In the present disclosure, using high density SNP array to detect LOH allows identification of pattern of allelic imbalance to determine the presence of wild type p53 allele (Lips *et al.,* 2005; Lai *et al.,* 2007).

Examples of p53 gene sequence and single nucleotide polymorphism arrays include p53 Gene Chip (Affymetrix, Santa Clara, CA), Roche p53 Ampli-Chip (Roche Molecular Systems, Pleasanton, CA), GeneChip Mapping arrays (Affymetrix, Santa Clara, CA), SNP Array 6.0 (Affymetrix, Santa Clara, CA), BeadArrays (Illumina, San Diego, CA), etc.

Mutations of wild type p53 genes can also be detected on the basis of the mutation of a wild type expression product of the p53 gene. Such expression products include both the mRNA as well as the p53 protein product itself. Point mutations can be detected by sequencing the mRNA directly or via molecular cloning of cDNA made from the mRNA. The sequence of the cloned cDNA can be determined using DNA sequencing techniques. The cDNA can also be sequenced via the polymerase chain reaction (PCR). A panel of monoclonal antibodies could be used in which each of the epitopes involved in p53 functions are represented by a monoclonal antibody. Loss or perturbation of binding of a monoclonal antibody in the panel can indicate mutational alteration of the p53 protein and thus of the p53 gene itself. Mutant p53 genes or gene products can also be detected in body samples, including, for example, serum, stool, urine, and sputum. The same techniques discussed above for detection of mutant p53 genes or gene products in tissues can be applied to other body samples.
Loss of wild type p53 genes can also be detected by screening for loss of wild type p53 protein function. Although all of the functions which the p53 protein undoubtedly possesses have yet to be elucidated, at least two specific functions are known. Protein p53 binds to the SV40 large T antigen as well as to the adenovirus E1B antigen. Loss of the ability of the p53 protein to bind to either or both of these antigens indicates a mutational alteration in the protein which reflects a mutational alteration of the gene itself. Alternatively, a panel of monoclonal antibodies could be used in which each of the epitopes involved in p53 functions are represented by a monoclonal antibody. Loss or perturbation of binding of a monoclonal antibody in the panel would indicate mutational alteration of the p53 protein and thus of the p53 gene itself. Any method for detecting an altered p53 protein can be used to detect loss of wild type p53 genes.

### Assay to Determine α-helicity

In solution, the secondary structure of polypeptides with α-helical domains will reach a dynamic equilibrium between random coil structures and α-helical structures, often expressed as a "percent helicity". Thus, for example, alpha-helical domains are predominantly random coils in solution, with α-helical content usually under 25%. Peptidomimetic macrocycles with optimized linkers, on the other hand, possess, for example, an alpha-helicity that is at least two-fold greater than that of a corresponding uncrosslinked polypeptide. In some cases, macrocycles will possess an alpha-helicity of greater than 50%. To assay the helicity of peptidomimetic macrocycles, the compounds are dissolved in an aqueous solution (e.g. 50 mM potassium phosphate solution at pH 7, or distilled HzO, to concentrations of 25-50 µM). Circular dichroism (CD) spectra are obtained on a spectropolarimeter (e.g., Jasco J-710) using standard measurement parameters (e.g. temperature, 20°C; wavelength, 190-260 nm; step resolution, 0.5 nm; speed, 20 nm/sec; accumulations, 10; response, 1 sec; bandwidth, 1 nm; path length, 0.1 cm). The α-helical content of each peptide is calculated by dividing the mean residue ellipticity (e.g. [Φ]222obs) by the reported value for a model helical decapeptide (Yang et al. (1986), Methods Enzymol. 130:208)).

### Assay to Determine Melting Temperature (Tm).

A peptidomimetic macrocycle comprising a secondary structure such as an α-helix exhibits, for example, a higher melting temperature than a corresponding uncrosslinked polypeptide. Typically peptidomimetic macrocycles exhibit Tm of > 60°C representing a highly stable structure in aqueous solutions. To assay the effect of macrocycle formation on melting temperature, peptidomimetic macrocycles or unmodified peptides are dissolved in distilled H₂O (*e.g.* at a final concentration of 50 µM) and the Tm is determined by measuring the change in ellipticity over a temperature range (e.g. 4 to 95 °C) on a spectropolarimeter (e.g., Jasco J-710) using standard parameters (e.g. wavelength 222nm; step resolution, 0.5 nm; speed, 20 nm/sec; accumulations, 10; response, 1 sec; bandwidth, 1 nm; temperature increase rate: 1°C/min; path length, 0.1 cm).

### Protease Resistance Assay.

The amide bond of the peptide backbone is susceptible to hydrolysis by proteases, thereby rendering peptidic compounds vulnerable to rapid degradation *in vivo.* Peptide helix formation, however, typically buries the amide backbone and therefore can shield it from proteolytic cleavage. The peptidomimetic macrocycles can be subjected to *in vitro* trypsin proteolysis to assess for any change in degradation rate compared to a corresponding uncrosslinked polypeptide. For example, the peptidomimetic macrocycle and a corresponding uncrosslinked polypeptide are incubated with trypsin agarose and the reactions quenched at various time points by centrifugation and subsequent HPLC injection to quantitate the residual substrate by ultraviolet absorption at 280 nm. Briefly, the peptidomimetic macrocycle and peptidomimetic precursor (5 mcg) are incubated with trypsin agarose (Pierce) (S/E ~125) for 0, 10, 20, 90, and 180 minutes. Reactions are quenched by tabletop centrifugation at high speed; remaining substrate in the isolated supernatant is quantified by HPLC-based peak detection at 280 nm. The proteolytic reaction displays first order kinetics and the rate constant, k, is determined from a plot of ln[S] versus time (k=-1Xslope).

### Ex Vivo Stability Assay.

Peptidomimetic macrocycles with optimized linkers possess, for example, an *ex vivo* half-life that is at least two-fold greater than that of a corresponding uncrosslinked polypeptide, and possess an *ex vivo* half-life of 12 hours or more. For *ex vivo* serum stability studies, a variety of assays can be used. For example, a peptidomimetic macrocycle and a corresponding uncrosslinked polypeptide (2 mcg) are incubated with fresh mouse, rat and/or human serum (2 mL) at 37°C for 0, 1, 2, 4, 8, and 24 hours. To determine the level of intact compound, the following procedure can be used: The samples are extracted by transferring 100 µL of sera to 2 ml centrifuge tubes followed by the addition of 10 µL of 50 % formic acid and 500µL acetonitrile and centrifugation at 14,000 RPM for 10 min at 4 ± 2°C. The supernatants are then transferred to fresh 2 ml tubes and evaporated on Turbovap under N₂ < 10 psi, 37°C. The samples are reconstituted in 100µL of 50:50 acetonitrile:water and submitted to LC-MS/MS analysis.

### In vitro Binding Assays.

To assess the binding and affinity of peptidomimetic macrocycles and peptidomimetic precursors to acceptor proteins, a fluorescence polarization assay (FPA) is used, for example. The FPA technique measures the molecular orientation and mobility using polarized light and fluorescent tracer. When excited with polarized light, fluorescent tracers (e.g., FITC) attached to molecules with high apparent molecular weights (e.g. FITC-labeled peptides bound to a large protein) emit higher levels of polarized fluorescence due to their slower rates of rotation as compared to fluorescent tracers attached to smaller molecules (*e*.*g*. FITC- labeled peptides that are free in solution).

For example, fluoresceinated peptidomimetic macrocycles (25 nM) are incubated with the acceptor protein (25- 1000nM) in binding buffer (140mM NaCl, 50 mM Tris-HCL, pH 7.4) for 30 minutes at room temperature. Binding activity is measured, for example, by fluorescence polarization on a luminescence spectrophotometer (*e*.*g*. Perkin-Elmer LS50B). Kd values can be determined by nonlinear regression analysis using, for example, GraphPad Prism software (GraphPad Software, Inc., San Diego, CA). A peptidomimetic macrocycle shows, In some cases, similar or lower Kd than a corresponding uncrosslinked polypeptide.

### In vitro Displacement Assays To Characterize Antagonists of Peptide-Protein Interactions.

To assess the binding and affinity of compounds that antagonize the interaction between a peptide and an acceptor protein, a fluorescence polarization assay (FPA) utilizing a fluoresceinated peptidomimetic macrocycle derived from a peptidomimetic precursor sequence is used, for example. The FPA technique measures the molecular orientation and mobility using polarized light and fluorescent tracer. When excited with polarized light, fluorescent tracers (*e*.*g*., FITC) attached to molecules with high apparent molecular weights (*e*.*g*. FITC-labeled peptides bound to a large protein) emit higher levels of polarized fluorescence due to their slower rates of rotation as compared to fluorescent tracers attached to smaller molecules (*e*.*g*. FITC-labeled peptides that are free in solution). A compound that antagonizes the interaction between the fluoresceinated peptidomimetic macrocycle and an acceptor protein will be detected in a competitive binding FPA experiment.

For example, putative antagonist compounds (1 nM to 1 mM) and a fluoresceinated peptidomimetic macrocycle (25 nM) are incubated with the acceptor protein (50 nM) in binding buffer (140mM NaCl, 50 mM Tris-HCL, pH 7.4) for 30 minutes at room temperature. Antagonist binding activity is measured, for example, by fluorescence polarization on a luminescence spectrophotometer (*e*.*g*. Perkin-Elmer LS50B). Kd values can be determined by nonlinear regression analysis using, for example, Graphpad Prism software (GraphPad Software, Inc., San Diego, CA).

Any class of molecule, such as small organic molecules, peptides, oligonucleotides or proteins can be examined as putative antagonists in this assay.

### Assay for Protein-ligand binding by Affinity Selection-Mass Spectrometry

To assess the binding and affinity of test compounds for proteins, an affinity-selection mass spectrometry assay is used, for example. Protein-ligand binding experiments are conducted according to the following representative procedure outlined for a system-wide control experiment using 1 µM peptidomimetic macrocycle plus 5 µM hMDM2. A 1 µL DMSO aliquot of a 40 µM stock solution of peptidomimetic macrocycle is dissolved in 19 µL of PBS (Phosphate-buffered saline: 50 mM, pH 7.5 Phosphate buffer containing 150 mM NaCl). The resulting solution is mixed by repeated pipetting and clarified by centrifugation at 10 000g for 10 min. To a 4 µL aliquot of the resulting supernatant is added 4 µL of 10 µM hMDM2 in PBS. Each 8.0 µL experimental sample thus contains 40 pmol (1.5 µg) of protein at 5.0 µM concentration in PBS plus 1 µM peptidomimetic macrocycle and 2.5% DMSO. Duplicate samples thus prepared for each concentration point are incubated for 60 min at room temperature, and then chilled to 4 °C prior to size-exclusion chromatography-LC-MS analysis of 5.0 µL injections. Samples containing a target protein, protein-ligand complexes, and unbound compounds are injected onto an SEC column, where the complexes are separated from non-binding component by a rapid SEC step. The SEC column eluate is monitored using UV detectors to confirm that the early-eluting protein fraction, which elutes in the void volume of the SEC column, is well resolved from unbound components that are retained on the column. After the peak containing the protein and protein-ligand complexes elutes from the primary UV detector, it enters a sample loop where it is excised from the flow stream of the SEC stage and transferred directly to the LC-MS via a valving mechanism. The (M + 3H)³⁺ ion of the peptidomimetic macrocycle is observed by ESI-MS at the expected m/z, confirming the detection of the protein-ligand complex.

### Assay for Protein-ligand Kd Titration Experiments.

To assess the binding and affinity of test compounds for proteins, a protein-ligand Kd titration experiment is performed, for example. Protein-ligand *K*_{d} titrations experiments are conducted as follows: 2 µL DMSO aliquots of a serially diluted stock solution of titrant peptidomimetic macrocycle (5, 2.5,..., 0.098 mM) are prepared then dissolved in 38 µL of PBS. The resulting solutions are mixed by repeated pipetting and clarified by centrifugation at 10 000g for 10 min. To 4.0 µL aliquots of the resulting supernatants is added 4.0 µL of 10 µM hMDM2 in PBS. Each 8.0 µL experimental sample thus contains 40 pmol (1.5 µg) of protein at 5.0 µM concentration in PBS, varying concentrations (125, 62.5,..., 0.24 µM) of the titrant peptide, and 2.5% DMSO. Duplicate samples thus prepared for each concentration point are incubated at room temperature for 30 min, then chilled to 4 °C prior to SEC-LC-MS analysis of 2.0 µL injections. The (M + H)¹⁺, (M + 2H)²⁺, (M + 3H)³⁺, and/or (M + Na)¹⁺ ion is observed by ESI-MS; extracted ion chromatograms are quantified, then fit to equations to derive the binding affinity *K*_{d} as described in Annis, D. A.; Nazef, N.; Chuang, C. C.; Scott, M. P.; Nash, H. M. J. Am. Chem. Soc. 2004, 126, 15495-15503; also in D. A. Annis, C.-C. Chuang, and N. Nazef. In Mass Spectrometry in Medicinal Chemistry. Edited by Wanner K, Höfner G: Wiley-VCH; 2007: 121-184. Mannhold R, Kubinyi H, Folkers G (Series Editors): Methods and Principles in Medicinal Chemistry.

### Assay for Competitive Binding Experiments by Affinity Selection-Mass Spectrometry

To determine the ability of test compounds to bind competitively to proteins, an affinity selection mass spectrometry assay is performed, for example. A mixture of ligands at 40 µM per component is prepared by combining 2 µL aliquots of 400 µM stocks of each of the three compounds with 14 µL of DMSO. Then, 1 µL aliquots of this 40 µM per component mixture are combined with 1 µL DMSO aliquots of a serially diluted stock solution of titrant peptidomimetic macrocycle (10, 5, 2.5,..., 0.078 mM). These 2 µL samples are dissolved in 38 µL of PBS. The resulting solutions were mixed by repeated pipetting and clarified by centrifugation at 10 000g for 10 min. To 4.0 µL aliquots of the resulting supernatants is added 4.0 µL of 10 µM hMDM2 protein in PBS. Each 8.0 µL experimental sample thus contains 40 pmol (1.5 µg) of protein at 5.0 µM concentration in PBS plus 0.5 µM ligand, 2.5% DMSO, and varying concentrations (125, 62.5,..., 0.98 µM) of the titrant peptidomimetic macrocycle. Duplicate samples thus prepared for each concentration point are incubated at room temperature for 60 min, then chilled to 4 °C prior to SEC-LC-MS analysis of 2.0 µL injections. Additional details on these and other methods are described in *"*A General Technique to Rank Protein-Ligand Binding Affinities and Determine Allosteric vs. Direct Binding Site Competition in Compound Mixtures." Annis, D. A.; Nazef, N.; Chuang, C. C.; Scott, M. P.; Nash, H. M. J. Am. Chem. Soc. 2004, 126, 15495-15503; also in *"*ALIS: An Affinity Selection-Mass Spectrometry System for the Discovery and Characterization of Protein-Ligand Interactions" D. A. Annis, C.-C. Chuang, and N. Nazef. In Mass Spectrometry in Medicinal Chemistry. Edited by Wanner K, Höfner G: Wiley-VCH; 2007:121-184. Mannhold R, Kubinyi H, Folkers G (Series Editors): Methods and Principles in Medicinal Chemistry.

### Binding Assays in Intact Cells.

It is possible to measure binding of peptides or peptidomimetic macrocycles to their natural acceptors in intact cells by immunoprecipitation experiments. For example, intact cells are incubated with fluoresceinated (FITC-labeled) compounds for 4 hrs in the absence of serum, followed by serum replacement and further incubation that ranges from 4-18 hrs. Cells are then pelleted and incubated in lysis buffer (50 mM Tris [pH 7.6], 150 mM NaCl, 1% CHAPS and protease inhibitor cocktail) for 10 minutes at 4 °C. Extracts are centrifuged at 14,000 rpm for 15 minutes and supernatants collected and incubated with 10 µL goat anti-FITC antibody for 2 hrs, rotating at 4 °C followed by further 2 hrs incubation at 4 °C with protein A/G Sepharose (50 µL of 50% bead slurry). After quick centrifugation, the pellets are washed in lysis buffer containing increasing salt concentration (*e.g.*, 150, 300, 500 mM). The beads are then re-equilibrated at 150 mM NaCl before addition of SDS-containing sample buffer and boiling. After centrifugation, the supernatants are optionally electrophoresed using 4%-12% gradient Bis-Tris gels followed by transfer into Immobilon-P membranes. After blocking, blots are optionally incubated with an antibody that detects FITC and also with one or more antibodies that detect proteins that bind to the peptidomimetic macrocycle.

### Cellular Penetrability Assays.

A peptidomimetic macrocycle is, for example, more cell penetrable compared to a corresponding uncrosslinked macrocycle. Peptidomimetic macrocycles with optimized linkers possess, for example, cell penetrability that is at least two-fold greater than a corresponding uncrosslinked macrocycle, and often 20% or more of the applied peptidomimetic macrocycle will be observed to have penetrated the cell after 4 hours. To measure the cell penetrability of peptidomimetic macrocycles and corresponding uncrosslinked macrocycle, intact cells are incubated with fluorescently-labeled (e.g. fluoresceinated) peptidomimetic macrocycles or corresponding uncrosslinked macrocycle (10 µM) for 4 hrs in serum free media at 37°C, washed twice with media and incubated with trypsin (0.25%) for 10 min at 37°C. The cells are washed again and resuspended in PBS. Cellular fluorescence is analyzed, for example, by using either a FACSCalibur flow cytometer or Cellomics' KineticScan ^{®} HCS Reader.

### Cellular Efficacy Assays.

The efficacy of certain peptidomimetic macrocycles is determined, for example, in cell-based killing assays using a variety of tumorigenic and non-tumorigenic cell lines and primary cells derived from human or mouse cell populations. Cell viability is monitored, for example, over 24-96 hrs of incubation with peptidomimetic macrocycles (0.5 to 50 µM) to identify those that kill at EC₅₀<10 µM. Several standard assays that measure cell viability are commercially available and are optionally used to assess the efficacy of the peptidomimetic macrocycles. In addition, assays that measure Annexin V and caspase activation are optionally used to assess whether the peptidomimetic macrocycles kill cells by activating the apoptotic machinery. For example, the Cell Titer-glo assay is used which determines cell viability as a function of intracellular ATP concentration.

### In Vivo Stability Assay.

To investigate the *in vivo* stability of the peptidomimetic macrocycles, the compounds are, for example, administered to mice and/or rats by IV, IP, PO or inhalation routes at concentrations ranging from 0.1 to 50 mg/kg and blood specimens withdrawn at 0', 5', 15', 30', 1 hr, 4 hrs, 8 hrs and 24 hours post-injection. Levels of intact compound in 25 µL of fresh serum are then measured by LC-MS/MS as above.

### In vivo Efficacy in Animal Models.

To determine the anti-oncogenic activity of peptidomimetic macrocycles *in vivo,* the compounds are, for example, given alone (IP, IV, PO, by inhalation or nasal routes) or in combination with sub-optimal doses of relevant chemotherapy (*e*.*g*., cyclophosphamide, doxorubicin, etoposide). In one example, 5 × 10⁶ RS4;11 cells (established from the bone marrow of a patient with acute lymphoblastic leukemia) that stably express luciferase are injected by tail vein in NOD-SCID mice 3 hrs after they have been subjected to total body irradiation. If left untreated, this form of leukemia is fatal in 3 weeks in this model. The leukemia is readily monitored, for example, by injecting the mice with D-luciferin (60 mg/kg) and imaging the anesthetized animals *(e.g.,* Xenogen *In Vivo* Imaging System, Caliper Life Sciences, Hopkinton, MA). Total body bioluminescence is quantified by integration of photonic flux (photons/sec) by Living Image Software (Caliper Life Sciences, Hopkinton, MA). Peptidomimetic macrocycles alone or in combination with sub-optimal doses of relevant chemotherapeutics agents are, for example, administered to leukemic mice (10 days after injection/day 1 of experiment, in bioluminescence range of 14-16) by tail vein or IP routes at doses ranging from 0.1mg/kg to 50 mg/kg for 7 to 21 days. Optionally, the mice are imaged throughout the experiment every other day and survival monitored daily for the duration of the experiment. Expired mice are optionally subjected to necropsy at the end of the experiment. Another animal model is implantation into NOD-SCID mice of DoHH2, a cell line derived from human follicular lymphoma that stably expresses luciferase. These *in vivo* tests optionally generate preliminary pharmacokinetic, pharmacodynamic and toxicology data.

### Clinical Trials

To determine the suitability of the peptidomimetic macrocycles for treatment of humans, clinical trials are performed. For example, patients diagnosed with cancer and in need of treatment can be selected and separated in treatment and one or more control groups, wherein the treatment group is administered a peptidomimetic macrocycle, while the control groups receive a placebo or a known anti-cancer drug. The treatment safety and efficacy of the peptidomimetic macrocycles can thus be evaluated by performing comparisons of the patient groups with respect to factors such as survival and quality-of-life. In this example, the patient group treated with a peptidomimetic macrocycle can show improved long-term survival compared to a patient control group treated with a placebo.

### Pharmaceutical Compositions and Routes of Administration

Pharmaceutical compositions disclosed herein include peptidomimetic macrocycles and pharmaceutically acceptable salts thereof.

In some cases, peptidomimetic macrocycles are modified by covalently or non-covalently joining appropriate functional groups to enhance selective biological properties. Such modifications include those which increase biological penetration into a given biological compartment (*e*.*g*., blood, lymphatic system, central nervous system), increase oral availability, increase solubility to allow administration by injection, alter metabolism, and alter rate of excretion.

Pharmaceutically acceptable salts of the compounds disclosed herein include those derived from pharmaceutically acceptable inorganic and organic acids and bases. Examples of suitable acid salts include acetate, adipate, benzoate, benzenesulfonate, butyrate, citrate, digluconate, dodecylsulfate, formate, fumarate, glycolate, hemisulfate, heptanoate, hexanoate, hydrochloride, hydrobromide, hydroiodide, lactate, maleate, malonate, methanesulfonate, 2-naphthalenesulfonate, nicotinate, nitrate, palmoate, phosphate, picrate, pivalate, propionate, salicylate, succinate, sulfate, tartrate, tosylate and undecanoate. Salts derived from appropriate bases include alkali metal *(e.g.,* sodium), alkaline earth metal *(e.g.,* magnesium), ammonium and N-(alkyl)₄⁺ salts.

For preparing pharmaceutical compositions from the compounds disclosed herein, pharmaceutically acceptable carriers include either solid or liquid carriers. Solid form preparations include powders, tablets, pills, capsules, cachets, suppositories, and dispersible granules. A solid carrier can be one or more substances, which also acts as diluents, flavoring agents, binders, preservatives, tablet disintegrating agents, or an encapsulating material. Details on techniques for formulation and administration are well described in the scientific and patent literature, see, *e.g.,* the latest edition of Remington's Pharmaceutical Sciences, Maack Publishing Co, Easton PA.

In powders, the carrier is a finely divided solid, which is in a mixture with the finely divided active component. In tablets, the active component is mixed with the carrier having the necessary binding properties in suitable proportions and compacted in the shape and size desired.

Suitable solid excipients are carbohydrate or protein fillers include, but are not limited to sugars, including lactose, sucrose, mannitol, or sorbitol; starch from corn, wheat, rice, potato, or other plants; cellulose such as methyl cellulose, hydroxypropylmethyl-cellulose, or sodium carboxymethylcellulose; and gums including arabic and tragacanth; as well as proteins such as gelatin and collagen. If desired, disintegrating or solubilizing agents are added, such as the cross-linked polyvinyl pyrrolidone, agar, alginic acid, or a salt thereof, such as sodium alginate.

Liquid form preparations include solutions, suspensions, and emulsions, for example, water or water/propylene glycol solutions. For parenteral injection, liquid preparations can be formulated in solution in aqueous polyethylene glycol solution.

The pharmaceutical preparation can be in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.
When one or more compositions disclosed herein comprise a combination of a peptidomimetic macrocycle and one or more additional therapeutic or prophylactic agents, both the compound and the additional agent should be present at dosage levels of between about 1 to 100%, and more preferably between about 5 to 95% of the dosage normally administered in a monotherapy regimen. In some cases, the additional agents are administered separately, as part of a multiple dose regimen, from one or more compounds disclosed herein. Alternatively, those agents are part of a single dosage form, mixed together with the compounds disclosed herein in a single composition.

### Methods of Use

Described herein are novel peptidomimetic macrocycles that are useful in competitive binding assays to identify agents which bind to the natural ligand(s) of the proteins or peptides upon which the peptidomimetic macrocycles are modeled. For example, in the p53/MDMX system, labeled peptidomimetic macrocycles based on p53 can be used in a MDMX binding assay along with small molecules that competitively bind to MDMX. Competitive binding studies allow for rapid *in vitro* evaluation and determination of drug candidates specific for the p53/MDMX system. Such binding studies can be performed with any of the peptidomimetic macrocycles disclosed herein and their binding partners. Further described are methods for the generation of antibodies against the peptidomimetic macrocycles. In some cases, these antibodies specifically bind both the peptidomimetic macrocycle and the precursor peptides, such as p53, to which the peptidomimetic macrocycles are related. Such antibodies, for example, disrupt the native protein-protein interaction, for example, binding between p53 and MDMX.

Also described herein are both prophylactic and therapeutic methods of treating a subject at risk of (or susceptible to) a disorder or having a disorder associated with aberrant (*e*.*g*., insufficient or excessive) expression or activity of the molecules including p53, MDM2 or MDMX.

In another case, a disorder is caused, at least in part, by an abnormal level of p53 or MDM2 or MDMX, (*e*.*g*., over or under expression), or by the presence of p53 or MDM2 or MDMX exhibiting abnormal activity. As such, the reduction in the level and/or activity of p53 or MDM2 or MDMX, or the enhancement of the level and/or activity of p53 or MDM2 or MDMX, by peptidomimetic macrocycles derived from p53, is used, for example, to ameliorate or reduce the adverse symptoms of the disorder.

Also described herein are methods for treating or preventing a disease including hyperproliferative disease and inflammatory disorder by interfering with the interaction or binding between binding partners, for example, between p53 and MDM2 or p53 and MDMX. These methods comprise administering an effective amount of a compound to a warm blooded animal, including a human. In some cases, the administration of one or more compounds disclosed herein induces cell growth arrest or apoptosis.

As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease.

In some cases, the peptidomimetic macrocycles can be used to treat, prevent, and/or diagnose cancers and neoplastic conditions. As used herein, the terms "cancer", "hyperproliferative" and "neoplastic" refer to cells having the capacity for autonomous growth, *i.e.,* an abnormal state or condition characterized by rapidly proliferating cell growth. Hyperproliferative and neoplastic disease states can be categorized as pathologic, *i.e.*, characterizing or constituting a disease state, or can be categorized as non-pathologic, *i.e*., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. A metastatic tumor can arise from a multitude of primary tumor types, including but not limited to those of breast, lung, liver, colon and ovarian origin. "Pathologic hyperproliferative" cells occur in disease states characterized by malignant tumor growth. Examples of non-pathologic hyperproliferative cells include proliferation of cells associated with wound repair. Examples of cellular proliferative and/or differentiation disorders include cancer, e.g., carcinoma, sarcoma, or metastatic disorders. In some cases, the peptidomimetic macrocycles are novel therapeutic agents for controlling breast cancer, ovarian cancer, colon cancer, lung cancer, metastasis of such cancers and the like.

Examples of cancers or neoplastic conditions include, but are not limited to, a fibrosarcoma, myosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, gastric cancer, esophageal cancer, rectal cancer, pancreatic cancer, ovarian cancer, prostate cancer, uterine cancer, cancer of the head and neck, skin cancer, brain cancer, squamous cell carcinoma, sebaceous gland carcinoma, papillary carcinoma, papillary adenocarcinoma, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular cancer, small cell lung carcinoma, non-small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, lymphoma, or Kaposi sarcoma.

In some cases, the cancer is head and neck cancer, melanoma, lung cancer, breast cancer, or glioma.

Examples of proliferative disorders include hematopoietic neoplastic disorders. As used herein, the term "hematopoietic neoplastic disorders" includes diseases involving hyperplastic/neoplastic cells of hematopoietic origin, *e.g.,* arising from myeloid, lymphoid or erythroid lineages, or precursor cells thereof. The diseases can arise from poorly differentiated acute leukemias, *e*.*g*., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus (1991), Crit Rev. Oncol./Hemotol. 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemiallymphoma (ATL), cutaneous T-cell lymphoma (CTCL), periphieral T-cell lymphoma (PTCL), large granular lymphocytic leukemia (LGF), Hodgkin's disease and Reed-Stemberg disease.

Examples of cellular proliferative and/or differentiation disorders of the breast include, but are not limited to, proliferative breast disease including, *e*.*g*., epithelial hyperplasia, sclerosing adenosis, and small duct papillomas; tumors, *e*.*g*., stromal tumors such as fibroadenoma, phyllodes tumor, and sarcomas, and epithelial tumors such as large duct papilloma; carcinoma of the breast including in situ (noninvasive) carcinoma that includes ductal carcinoma in situ (including Paget's disease) and lobular carcinoma in situ, and invasive (infiltrating) carcinoma including, but not limited to, invasive ductal carcinoma, invasive lobular carcinoma, medullary carcinoma, colloid (mucinous) carcinoma, tubular carcinoma, and invasive papillary carcinoma, and miscellaneous malignant neoplasms. Disorders in the male breast include, but are not limited to, gynecomastia and carcinoma.

Examples of cellular proliferative and/or differentiative disorders of the skin include, but are not limited to proliferative skin disease such as melanomas, including mucosal melanoma, superficial spreading melanoma, nodular melanoma, lentigo (*e.g.* lentigo maligna, lentigo maligna melanoma, or acral lentiginous melanoma), amelanotic melanoma, desmoplastic melanoma, melanoma with features of a Spitz nevus, melanoma with small nevus-like cells, polypoid melanoma, and soft-tissue melanoma; basal cell carcinomas including micronodular basal cell carcinoma, superficial basal cell carcinoma, nodular basal cell carcinoma (rodent ulcer), cystic basal cell carcinoma, cicatricial basal cell carcinoma, pigmented basal cell carcinoma, aberrant basal cell carcinoma, infiltrative basal cell carcinoma, nevoid basal cell carcinoma syndrome, polypoid basal cell carcinoma, pore-like basal cell carcinoma, and fibroepithelioma of Pinkus; squamus cell carcinomas including acanthoma (large cell acanthoma), adenoid squamous cell carcinoma, basaloid squamous cell carcinoma, clear cell squamous cell carcinoma, signet-ring cell squamous cell carcinoma, spindle cell squamous cell carcinoma, Marjolin's ulcer, erythroplasia of Queyrat, and Bowen's disease; or other skin or subcutaneous tumors.

Examples of cellular proliferative and/or differentiation disorders of the lung include, but are not limited to, bronchogenic carcinoma, including paraneoplastic syndromes, bronchioloalveolar carcinoma, neuroendocrine tumors, such as bronchial carcinoid, miscellaneous tumors, and metastatic tumors; pathologies of the pleura, including inflammatory pleural effusions, noninflammatory pleural effusions, pneumothorax, and pleural tumors, including solitary fibrous tumors (pleural fibroma) and malignant mesothelioma.

Examples of cellular proliferative and/or differentiative disorders of the colon include, but are not limited to, non-neoplastic polyps, adenomas, familial syndromes, colorectal carcinogenesis, colorectal carcinoma, and carcinoid tumors.

Examples of cellular proliferative and/or differentiative disorders of the liver include, but are not limited to, nodular hyperplasias, adenomas, and malignant tumors, including primary carcinoma of the liver and metastatic tumors.

Examples of cellular proliferative and/or differentiative disorders of the ovary include, but are not limited to, ovarian tumors such as, tumors of coelomic epithelium, serous tumors, mucinous tumors, endometrioid tumors, clear cell adenocarcinoma, cystadenofibroma, Brenner tumor, surface epithelial tumors; germ cell tumors such as mature (benign) teratomas, monodermal teratomas, immature malignant teratomas, dysgerminoma, endodermal sinus tumor, choriocarcinoma; sex cord-stomal tumors such as, granulosa-theca cell tumors, thecomafibromas, androblastomas, hill cell tumors, and gonadoblastoma; and metastatic tumors such as Krukenberg tumors.

### Combination Treatment

The terms "combination therapy" or "combined treatment" or in "combination" as used herein denotes any form of concurrent or parallel treatment with at least two distinct therapeutic agents.

The combination therapy described herein can be particularly advantageous, since not only the therapeutic (for e.g. anti-cancerous) effect may be enhanced compared to the effect of each compound alone, the dosage of each agent in a combination therapy may also be reduced as compared to monotherapy with each agent, while still achieving an overall therapeutic (e.g. anti-tumor) effect. In addition, in some cases, the peptidomimetic macrocycles of the disclosure can exhibit synergistic effect with the additional pharmaceutical agents. In such cases, due to the synergistic effect, the total amount of drugs administered to a patient can advantageously be reduced, which may result in decreased side effects.

Also described herein are combination therapies in which the peptidomimetic macrocycles of the disclosure are used in combination with at least one additional pharmaceutically active agent. In various cases, the at least one additional pharmaceutically active agent may be capable of modulating the same or a different target as the peptidomimetic macrocycles of the disclosure. In some cases, the at least one additional pharmaceutically active agent may modulate the same target as the peptidomimetic macrocycles of the disclosure, or other components of the same pathway, or even overlapping sets of target enzymes. In some cases, the at least one additional pharmaceutically active agent may modulate a different target as the peptidomimetic macrocycles of the disclosure.

Combination therapy includes but is not limited to the combination of peptidomimetic macrocycles of this disclosure with chemotherapeutic agents, therapeutic antibodies, and radiation treatment, to provide a synergistic therapeutic effect.

Also described herein is a method for treating cancer, the method comprising administering to a subject in need thereof (a) an effective amount of a peptidomimetic macrocycle of the disclosure and (b) an effective amount of at least one additional pharmaceutically active agent to provide a combination therapy. In some cases, the combination therapy may have an enhanced therapeutic effect compared to the effect of the peptidomimetic macrocycle and the at least one additional pharmaceutically active agent each administered alone. According to certain exemplary cases, the combination therapy has a synergistic therapeutic effect. According to this case, the combination therapy produces a significantly better therapeutic result (e.g., anti-cancer, cell growth arrest, apoptosis, induction of differentiation, cell death, etc.) than the additive effects achieved by each individual constituent when administered alone at a therapeutic dose.

In some cases, the peptidomimetic macrocycles of the disclosure are used in combination with one or more anti-cancer (antineoplastic or cytotoxic) chemotherapy drug. Suitable chemotherapeutic agents for use in the combinations of the present disclosure include, but are not limited to, alkylating agents, antibiotic agents, antimetabolic agents, hormonal agents, plant-derived agents, anti-angiogenic agents, differentiation inducing agents, cell growth arrest inducing agents, apoptosis inducing agents, cytotoxic agents, agents affecting cell bioenergetics, biologic agents, *e*.*g*., monoclonal antibodies, kinase inhibitors and inhibitors of growth factors and their receptors, gene therapy agents, cell therapy, *e*.*g*., stem cells, or any combination thereof.

In some cases, the peptidomimetic macrocycles of the disclosure are used in combination with an estrogen receptor antagonist. In one example, the peptidomimetic macrocycles of the disclosure are used in combination with the estrogen receptor antagonist fulvestrant (FASLODEX). Fulvestrant is a selective estrogen receptor degrader (SERD) and is indicated for the treatment of hormone receptor positive metastatic breast cancer in postmenopausal women with disease progression following anti-estrogen therapy. Fulvestrant is a complete estrogen receptor antagonist with little to no agonist effects and accelerates the proteasomal degradation of the estrogen receptor. Fulvestrant has poor oral bioavailability and is typically administered via intramuscular injection. Fulvestrant-induced expression of ErbB3 and ErbB4 receptors sensitizes oestrogen receptor-positive breast cancer cells to heregulin beta1 (*see,* e.g., Hutcheson et al., Breast cancer Research (2011) 13:R29).

In some cases, the peptidomimetic macrocycles of the disclosure are used in combination with an aromatase inhibitor. In one example, the peptidomimetic macrocycles of the disclosure are used in combination with exemestane.

In some cases, the peptidomimetic macrocycles of the disclosure are used in combination with a mTOR inhibitor. In one example, the peptidomimetic macrocycles of the disclosure are used in combination with everolimus (AFINITOR). Everolimus affects the mTORC1 protein complex and can lead to hyper-activation of the kinase AKT, which can lead to longer survival in some cell types. Everolimus binds to FKBP12, a protein receptor which directly interacts with mTORC1 and inhibits downstream signaling. mRNAs that codify proteins implicated in the cell cycle and in the glycolysis process are impaired or altered as a result, inhibiting tumor growth and proliferation. In some cases, the peptidomimetic macrocycles of the disclosure are used in combination with a mTOR inhibitor and an aromatase inhibitor. For example, the peptidomimetic macrocyclyes can be used in combination with everolimus and exemestane. Everolimus shows clinical efficacy in combination with tamoxifen, letrozole, or exemestane for the treatment of estrogen receptor-positive breast cancer (*see, e.g.,* Chen et al., Mol. Cancer Res. 11(10); 1269-78 (2013).

In some examples, the peptidomimetic macrocycles of the disclosure are used in combination with one or more antimetabolites, for example in combination with Capecitabine (XELODA), Gemcitabine (GEMZAR) and Cytarabine (cytosine arabinoside also known as Ara-C (arabinofuranosyl cytidine; Cytosar-U)).

In some cases, the peptidomimetic macrocycles of the disclosure are used in combination with taxanes. Exemplary non-limiting taxanes that may be used in combination with the instant peptidomimetic macrocycles include paclitaxel (ABRAXANE or TAXOL) and docetaxel (TAXOTERE). In some cases the peptidomimetic macrocycles of the instant disclosure are used in combination with paclitaxel. In some cases the peptidomimetic macrocycles of the instant disclosure are used in combination with docetaxel.

In some cases, the peptidomimetic macrocycles of the disclosure are used in combination with therapeutic antibodies. Examples of therapeutic antibodies that can be combined with compounds of this disclosure include but are not limited to anti CD20 antibodies, for example rituximab (MABTHERA/ RITUXAN) or obinutuzumab (GAZYVA). Other antibodies that can be used in combination with the peptidomimetic macrocycles of the disclosure include antibodies against the programed cell death (PD-1) receptor, for example pembrolizumab (KEYTRUDA) or nivolumba (OPDIVO).

PD-1 antagonists useful in the any of the treatment method, medicaments and uses of the present invention include a monoclonal antibody (mAb), or antigen binding fragment thereof, which specifically binds to PD-1 or PD-L1, and preferably specifically binds to human PD-1 or human PD-L1. A PD-1 antagonist can be any chemical compound or biological molecule that blocks binding of PD-L1 expressed on a cancer cell to PD-1 expressed on an immune cell (T cell, B cell or NKT cell) and may also block binding of PD-L2 expressed on a cancer cell to the immune-cell expressed PD-1. Alternative names or synonyms for PD-1 and its ligands include: PDCD1, PD1, CD279 and SLEB2 for PD-1; PDCD1L1, PDL1, B7H1, B7-4, CD274 and B7-H for PD-Ll; and PDCD1L2, PDL2, B7-DC, Btdc and CD273 for PD-L2. In any of the treatment method, medicaments and uses of the present invention in which a human individual is being treated, the PD-1 antagonist can block binding of human PD-L1 to human PD-1, and may block binding of both human PD-L1 and PD-L2 to human PD- 1.

Examples of mAbs that bind to human PD-1, and useful in the treatment method, medicaments and uses of the present invention, are described in US7521051, US8008449, and US8354509. Specific anti-human PD-1 mAbs useful as the PD-1 antagonist in the treatment method, medicaments and uses of the present invention include: MK-3475, a humanized IgG4 mAb with the structure described in WHO Drug Information, Vol. 27, No. 2, pages 161-162 (2013), nivolumab (BMS-936558), a human IgG4 mAb with the structure described in WHO Drug Information, Vol. 27, No. 1, pages 68-69 (2013); the humanized antibodies h409A11, h409A16 and h409A17, which are described in WO2008/156712, and AMP-514.

Other PD-1 antagonists useful in the any of the treatment method, medicaments and uses of the present invention include an immunoadhesin that specifically binds to PD-1 or PD-L1. Examples of immunoadhesion molecules that specifically bind to PD-1 are described in WO2010/027827 and WO2011/066342. Specific fusion proteins useful as thePD-1 antagonist in the treatment method, medicaments and uses of the present invention include AMP-224 (also known as B7-DCIg), which is a PD-L2-FC fusion protein and binds to human PD-1.

Other antibodies that can be used in combination with the peptidomimetic macrocycles of the disclosure include antibodies against human PD-L1. Examples of antibodies that bind to human PD-L1 and useful in the treatment method, medicaments and uses of the present invention, are described in WO2013/019906, WO2010/077634 A1 and US8383796. Specific anti-human PD-L1 mAbs useful as the PD-1 antagonist in the treatment method, medicaments and uses of the present invention include MPDL3280A, BMS-936559, MEDI4736, MSB0010718C and an antibody which comprises the heavy chain and light chain variable regions of SEQ ID NO:24 and SEQ ID NO:21, respectively, of WO2013/019906. Exemplary useful antibodies targeting PD-1 receptors include Pidilizumab, BMS 936559, and MPDL328OA. An exemplary anti-PD-Ll antibody is human monoclonal antibody MDX-1105 which binds PD-L1 and blocks its binding to and activation of its receptor PD-1, which may enhance the T cell-mediated immune response to neoplasms and reverse T-cell inactivation in chronic infections disease. An exemplary anti-PD-1 antibody is human monoclonal antibody MDX-1106 which binds and blocks the activation of PD-1 by its ligands PD-L1 and PD-L2, resulting in the activation of T cells and cell-mediated immune responses against tumor cell

Several approaches have been described for quantifying PD-L1 protein expression in IHC assays of tumor tissue sections. See, e.g., Thompson, R. H., et al, PNAS 101 (49); 17174-17179 (2004); Thompson, R. H. et al, Cancer Res. 66:3381-3385 (2006); Gadiot, J., et al, Cancer 117:2192-2201 (2011); Taube, J. M. et al, Sci Transl Med 4, 127ra37 (2012); and Toplian, S. L. et al, New Eng. J Med. 366 (26): 2443-2454 (2012). One approach employs a simple binary end-point of positive or negative for PD- LI expression, with a positive result defined in terms of the percentage of tumor cells that exhibit histologic evidence of cell-surface membrane staining. A tumor tissue section is counted as positive for PD-L1 expression is at least 1%, and preferably 5% of total tumor cells. In another approach, PD-L1 expression in the tumor tissue section is quantified in the tumor cells as well as in infiltrating immune cells, which predominantly comprise lymphocytes. The percentage of tumor cells and infiltrating immune cells that exhibit membrane staining are separately quantified as < 5%, 5 to 9%, and then in 10% increments up to 100%. For tumor cells, PD-L1 expression is counted as negative if the score is < 5% score and positive if the score is > 5%. PD-L1 expression in the immune infiltrate is reported as a semi-quantitative measurement called the adjusted inflammation score (AIS), which is determined by multiplying the percent of membrane staining cells by the intensity of the infiltrate, which is graded as none (0), mild (score of 1, rare lymphocytes), moderate (score of 2, focal infiltration of tumor by lymphohistiocytic aggregates), or severe (score of 3, diffuse infiltration). A tumor tissue section is counted as positive for PD-L1 expression by immune infiltrates if the AIS is > 5. A tissue section from a tumor that has been stained by IHC with a diagnostic PD- LI antibody may also be scored for PD-L1 protein expression by assessing PD-L1 expression in both the tumor cells and infiltrating immune cells in the tissue section. This PD-L1 scoring process can comprise examining each tumor nest in the tissue section for staining, and assigning to the tissue section one or both of a modified H score (MHS) and a modified proportion score (MPS). To assign the MHS, four separate percentages are estimated across all of the viable tumor cells and stained mononuclear inflammatory cells in all of the examined tumor nests: (a) cells that have no staining (intensity = 0), (b) weak staining (intensity =1+), (c) moderate staining (intensity =2+) and (d) strong staining (intensity =3+). A cell must have at least partial membrane staining to be included in the weak, moderate or strong staining percentages. The estimated percentages, the sum of which is 100%, are then input into the formula of 1 x (percent of weak staining cells) + 2 x (percent of moderate staining cells) + 3 x (percent of strong staining cells), and the result is assigned to the tissue section as the MHS. The MPS is assigned by estimating, across all of the viable tumor cells and stained mononuclear inflammatory cells in all of the examined tumor nests, the percentage of cells that have at least partial membrane staining of any intensity, and the resulting percentage is assigned to the tissue section as the MPS. In some cases, the tumor is designated as positive for PD-L1 expression if the MHS or the MPS is positive. The level of PD-L mRNA expression may be compared to the mRNA expression levels of one or more reference genes that are frequently used in quantitative RT-PCR, such as ubiquitin C. In some cases, a level of PD-L1 expression (protein and/or mRNA) by malignant cells and/or by infiltrating immune cells within a tumor is determined to be "overexpressed" or "elevated" based on comparison with the level of PD-L1 expression (protein and/ or mRNA) by an appropriate control. For example, a control PD-L1 protein or mRNA expression level may be the level quantified in nonmalignant cells of the same type or in a section from a matched normal tissue. In some preferred cases, PD-L1 expression in a tumor sample is determined to be elevated if PD-L1 protein (and/or PD-L1 mRNA) in the sample is at least 10%, 20%, or 30% greater than in the control.

In some cases, the peptidomimetic macrocycles of the disclosure are used in combination with antihormone therapy. Exemplary hormone antagonists that may be used in combination with the peptidomimetic macrocycles of the instant disclosure include letrozole (FEMARA) and casodex.

In some cases, the peptidomimetic macrocycles of the disclosure are used in in combination with hypomethylating agents or demethylating agents. Examples of such agents that may be used in combination with the peptidomimetic macrocycles of the disclosure include azacitidine (VIDAZA, AZADINE) and decitabine (Dacogen).

In some cases, the peptidomimetic macrocycles of the disclosure are used in in combination with an anti-inflammatory agent. In some cases, the peptidomimetic macrocycles of the disclosure are used in in combination with a corticosteroid. In some cases, the peptidomimetic macrocycles of the disclosure are used in in combination with a glucocorticosteroid. In one example, the peptidomimetic macrocycles of the disclosure are used in combination with dexamethasone.

In some cases, the peptidomimetic macrocycles of the disclosure are used in in combination with a histone deacetylase (HDAC) inhibitor. In some cases, the peptidomimetic macrocycles of the disclosure are used in in combination with a depsipeptide. In one example, the peptidomimetic macrocycles of the disclosure are used in combination with romidepsin (ISTODAX). Exemplary cancers for treatment with the peptidomimetic macrocycles of the disclosure and HDAC inhibitors, such as romidepsin, include T-cell lymphomas, for example, adult T cell leukemiallymphoma (ATL), cutaneous T-cell lymphoma (CTCL), or periphieral T-cell lymphoma (PTCL). HDAC inhibitors may interact synergistically with MDM2 inhibitors by mediating hyperacetylation of p53. Acetylation may be required for p53 activation. HDAC inhibitors may enhance the antitumor action of MDM2 inhibitors by diminishing MDM2 inhibitor-induced MDM2 expression. MDM2 is upregulated by p53 activation in a feedback loop that negatively controls p53 activity. MDM2 inhibitors may elicit cancer cell death by downregulating MDM4 expression. MDM4 is the second main negative regulator of p53, which is structurally homologues, but functionally not redundant to MDM2. Nutlin-3 and vorinostat cooperate in affecting cell viability and in inducing cell death and Δψm loss in A549 cells and cooperate in inducing cell death, Δψm loss and caspase-3 activity in A2780 cells (*see, e.g.,* J. Sonnemann et al., Invest New Drugs (2012) 30:25-36).

In some cases, the peptidomimetic macrocycles of the disclosure are used in in combination with platinum-based antineoplastic drugs (platinum drugs or platins). Examples of the platins that may be used in combination with the peptidomimetic macrocycles of the disclosure include cisplatin (also known as cisplatinum, platamin, neoplatin, cismaplat, cis-diamminedichloroplatinum(II), or CDDP; tradename PLATINOL) and carboplatin (also known as cis-diammine(1,1-cyclobutanedicarboxylato)platinum(II); tradenames PARAPLATIN and PARAPLATIN-AQ).

In some cases, the peptidomimetic macrocycles of the disclosure are used in in combination with a kinase inhibitor drug. The compounds described herein can be used in combination with MEK inhibitors. The compounds described herein can be used in combination with MEK1 inhibitors. The compounds described herein can be used in combination with MEK2 inhibitors. The compounds described herein can be used in combination with inhibitors of MEK1 and MEK2. In one example, he peptidomimetic macrocycles of the disclosure are used in in combination with trametinib (MEKINIST). The compounds described herein can be used in combination with BRAF inhibitors. The BRAF inhibitors used in combination with the peptidomimetic macrocycles of the disclosure may be inhibitor of either wild type or mutated BRAF. In some examples, the peptidomimetic macrocycles of the disclosure are used in combination with at least one additional pharmaceutically active agent that is an inhibitor of wild type BRAF. In some examples, the peptidomimetic macrocycles of the disclosure are used in combination with at least one additional pharmaceutically active agent that is an inhibitor of mutated BRAF. In some examples, the peptidomimetic macrocycles of the disclosure are used in combination with at least one additional pharmaceutically active agent that is an inhibitor of a V600E mutated BRAF. In some cases the compounds described herein can be used in combination with one or more BRAF inhibitors selected from vemurafenib (ZELBORAF a.k.a. PLX4032), dabrafenib (TAFINLAR), C-1, NVP-LGX818 and sorafenib (NEXAVAR). In some cases the compounds described herein can synergize with one or more BRAF inhibitors. In some cases one or more of the compounds described herein can synergize with all BRAF inhibitors.

The compounds described herein can be used in combination with KRAS inhibitors. The KRAS inhibitors used in combination with the peptidomimetic macrocycles of the disclosure may be inhibitor of either wild type or mutated KRAS. In some examples, the peptidomimetic macrocycles of the disclosure are used in combination with at least one additional pharmaceutically active agent that is an inhibitor of wild type KRAS. In some examples, the peptidomimetic macrocycles of the disclosure are used in combination with at least one additional pharmaceutically active agent that is an inhibitor of mutated KRAS. In some cases the compounds described herein can synergize with one or more KRAS inhibitors. In some cases one or more of the compounds described herein can synergize with all KRAS inhibitors.

The peptidomimetic macrocycles of the disclosure may also be used in combination with Bruton's tyrosine kinase (BTK) inhibitor, for example in combination with ibrutinib (IMBRUVICA). In some cases the compounds described herein can synergize with one or more BTK inhibitor. In some cases one or more of the compounds described herein can synergize with all BTK inhibitors.

In some examples, the peptidomimetic macrocycles of the disclosure may also be used in combination with inhibitors of the cyclin-dependent kinases, for example with an inhibitor of CDK4 and/or CDK6. An example of such inhibitor that may be used in combination with the instant peptidomimetic macrocycle is palbociclib (IBRANCE) (*see, e.g.,* Clin. Cancer Res.; 2015, 21(13); 2905-10). In some examples, the peptidomimetic macrocycles of the disclosure may be used in combination with an inhibitor of CDK4 and/or CDK6 and with an agent that reinforces the cytostatic activity of CDK4/6 inhibitors and/or with an agent that converts reversible cytostasis into irreversible growth arrest or cell death. Exemplary cancer subtypes include NSCLC, melanoma, neuroblastoma, glioblastoma, liposarcoma, and mantle cell lymphoma.

In some cases, a method of treating cancer in a subject in need thereof can comprise administering to the subject a therapeutically effective amount of a p53 agent that inhibits the interaction between p53 and MDM2 and/or p53 and MDMX, and/or modulates the activity of p53 and/or MDM2 and/or MDMX; and at least one additional pharmaceutically active agent, wherein the at least one additional pharmaceutically active agent modulates the activity of CDK4 and/or CDK6, and/or inhibits CDK4 and/or CDK6. In some examples, the p53 agent antagonizes an interaction between p53 and MDM2 proteins and/or between p53 and MDMX proteins. In some examples, the at least one additional pharmaceutically active agent binds to CDK4 and/or CDK6. In some examples, the p53 agent is selected from the group consisting of a small organic or inorganic molecule; a saccharine; an oligosaccharide; a polysaccharide; a peptide, a protein, a peptide analog, a peptide derivative; an antibody, an antibody fragment, a peptidomimetic; a peptidomimetic macrocycle of any one of claims 1-56; a nucleic acid; a nucleic acid analog, a nucleic acid derivative; an extract made from biological materials; a naturally occurring or synthetic composition; and any combination thereof. In some examples, the p53 agent is selected from the group consisting of RG7388 (RO5503781, idasanutlin); RG7112 (RO5045337); nutlin3a; nutlin3b; nutlin3; nutlin2; spirooxindole containing small molecules; 1,4-diazepines; 1,4-benzodiazepine-2,5-dione compounds; WK23; WK298; SJ172550; RO2443; RO5963; RO5353; RO2468; MK8242 (SCH900242); MI888; MI773 (SAR405838); NVPCGM097; DS3032b; AM8553; AMG232; NSC207895 (XI006); JNJ26854165 (serdemetan); RITA (NSC652287); YH239EE; and any combination thereof. In some examples, the at least one additional pharmaceutically active agent is selected from the group consisting of a small organic or inorganic molecule; a saccharine; an oligosaccharide; a polysaccharide; a peptide, a protein, a peptide analog, a peptide derivative; an antibody, an antibody fragment, a peptidomimetic; a peptidomimetic macrocycle of any one of claims 1-56; a nucleic acid; a nucleic acid analog, a nucleic acid derivative; an extract made from biological materials; a naturally occurring or synthetic composition; and any combination thereof. In some examples, the at least one additional pharmaceutically active agent is selected from the group consisting of palbociclib (PD0332991); abemaciclib (LY2835219); ribociclib (LEE 011); voruciclib (P1446A-05); fascaplysin; arcyriaflavin; 2-bromo-12,13-dihydro-5H-indolo[2,3-a]pyrrolo[3,4-c]carbazole-5,7(6H)-dione; 3-amino thioacridone (3-ATA), trans-4-((6-(ethylamino)-2-((1-(phenylmethyl)-1H-indol-5-yl)amino)-4-pyrimidinyl)amino)-cyclohexano (CINK4); 1,4-dimethoxyacridine-9(10H)-thione (NSC 625987); 2-methyl-5-(p-tolylamino)benzo[d]thiazole-4,7-dione (ryuvidine); and flavopiridol (alvocidib); and any combination thereof.

In some examples, the peptidomimetic macrocycles of the disclosure may also be used in combination with inhibitors of the cyclin-dependent kinases and an estrogen receptor antagonist. An example of such inhibitors that may be used in combination with the instant peptidomimetic macrocycle is palbociclib and fulvestrant. In some examples, the peptidomimetic macrocycles of the disclosure may also be used in combination with at least one additional pharmaceutically active agent that alleviates CDKN2A (cyclin-dependent kinase inhibitor 2A) deletion. In some example, the peptidomimetic macrocycles of the disclosure may also be used in combination with at least one additional pharmaceutically active agent that alleviates CDK9 (cyclin-dependent kinase 9) abnormality.

The peptidomimetic macrocycles of the disclosure may also be used in combination with one or more pharmaceutically active agent that regulates the ATM (upregulate or downregulate). In some cases the compounds described herein can synergize with one or more ATM regulators. In some cases one or more of the compounds described herein can synergize with all ATM regulators.

In some cases, the peptidomimetic macrocycles of the disclosure may be used in combination with one or more pharmaceutically active agent that inhibits the AKT (protein kinase B (PKB)). In some cases the compounds described herein can synergize with one or more AKT inhibitors.

In some examples, the peptidomimetic macrocycles of the disclosure may also be used in combination with at least one additional pharmaceutically active agent that alleviates PTEN (phosphatase and tensin homolog) deletion.

In some examples, the peptidomimetic macrocycles of the disclosure may also be used in combination with at least one additional pharmaceutically active agent that alleviates Wip-1Alpha over expression.

In some examples, the peptidomimetic macrocycles of the disclosure may be used in combination with at least one additional pharmaceutically active agent that is a Nucleoside metabolic inhibitor. Exemplary nucleoside metabolic inhibitors that may be used include capecitabine, gemcitabine and cytarabine (Arac).

The table below lists various suitable additional pharmaceutically active agents for use with the methods described herein.

| **Cancer Type** | **Drug name** | **Brand name** | **Drug works predominately in S or M phase** |
|---|---|---|---|
| ALL | ABT-199 | none | No |
| ALL | clofarabine | Clofarex | Yes; S phase |
| ALL | cyclophosphamide | Clafen, Cytoxan, Neosar | Yes: S phase |
| ALL | cytarabine | Cytosar-U, Tarabine PFS | Yes: S phase |
| ALL | doxorubicin | Adriamycin | Yes: S phase |
| ALL | imatinib mesylate | Gleevec | No |
| ALL | methotrexate | Abitrexate, Mexate, Folex | Yes: S phase |
| ALL | prednisone | Deltasone, Medicorten | No |
| ALL | romidepsin | Istodax | |
| ALL | vincristine | Vincasar | Yes: M phase |
| AML | ABT-199 | none | No |
| AML | azacitadine | Vidaza | No |
| AML | cyclophosphamide | Clafen, Cytoxan, Neosar | Yes: S phase |
| AML | cytarabine | Cytosar-U, Tarabine PFS | Yes: S phase |
| AML | decitabine | Dacogen | No |
| AML | doxorubicin | Adriamycin | Yes: S phase |
| AML | etoposide | Etopophos, Vepesid | Yes: S and M phases |
| AML | vincristine | Vincasar | Yes: M phase |
| bone | doxorubicin | Adriamycin | Yes: S phase |
| bone | methotrexate | Abitrexate, Mexate, Folex | Yes: S phase |
| breast | capecitabine | Xeloda | Yes: S phase |
| breast | cyclophosphamide | Clafen, Cytoxan, Neosar | Yes: S phase |
| breast | docetaxel | Taxotere | Yes: M phase |
| breast | doxorubicin | Adriamycin | Yes: S phase |
| breast | eribulin mesylate | Haliben | Yes: M phase |
| breast | everolimus | Afinitor | No |
| breast | exemestane | Aromasin | No |
| breast | fluorouracil | Adrucil, Efudex | Yes: S phase |
| breast | fulvestrant | Faslofex | |
| breast | gemcitabine | Gemzar | Yes: S phase |
| breast | goserelin acetate | Zoladex | No |
| breast | letrozole | Femara | No |
| breast | megestrol acetate | Megace | No |
| breast | methotrexate | Abitrexate, Mexate, Folex | Yes: S phase |
| breast | paclitaxel | Abraxane, Taxol | Yes: M phase |
| breast | palbociclib | Ibrance | Might cause G1 arrest |
| breast | pertuzumab | Perjeta | No |
| breast | tamoxifen citrate | Nolvadex | No |
| breast | trastuzumab | Herceptin, Kadcyla | No |
| colon | capecitabine | Xeloda | Yes: S phase |
| colon | cetuximab | Erbitux | No |
| colon | fluorouracil | Adrucil, Efudex | Yes: S phase |
| colon | irinotecan | camptosar | Yes: S and M phases |
| colon | ramucirumab | Cyramza | No |
| endometrial | carboplatin | Paraplatin, Paraplat | Yes: S phase |
| endometrial | cisplatin | Platinol | Yes: S phase |
| endometrial | doxorubicin | Adriamycin | Yes: S phase |
| endometrial | megestrol acetate | Megace | No |
| endometrial | paclitaxel | Abraxane, Taxol | Yes: M phase |
| gastric | docetaxel | Taxotere | Yes: M phase |
| gastric | doxorubicin | Adriamycin | Yes: S phase |
| gastric | fluorouracil | Adrucil, Efudex | Yes: S phase |
| gastric | ramucirumab | Cyramza | No |
| gastric | trastuzumab | Herceptin | No |
| kidney | axitinib | Inlyta | No |
| kidney | everolimus | Afinitor | No |
| kidney | pazopanib | Votrient | No |
| kidney | sorafenib tosylate | Nexavar | No |
| liver | sorafenib tosylate | Nexavar | No |
| melanoma | dacarbazine | DTIC, DTIC-Dome | Yes: S phase |
| melanoma | paclitaxel | Abraxane, Taxol | Yes: M phase |
| melanoma | trametinib | Mekinist | No |
| melanoma | vemurafenib | Zelboraf | No |
| melanoma | dabrafenib | Taflinar | |
| mesothelioma | cisplatin | Platinol | Yes: S phase |
| mesothelioma | pemetrexed | Alimta | Yes: S phase |
| NHL | ABT-199 | none | No |
| NHL | bendamustine | Treanda | Causes DNA crosslinking, but is also toxic to resting cells |
| NHL | bortezomib | Velcade | No |
| NHL | brentuximab vedotin | Adcetris | Yes: M phase |
| NHL | chlorambucil | Ambochlorin, Leukeran, Linfolizin | Yes: S phase |
| NHL | cyclophosphamide | Clafen, Cytoxan, Neosar | Yes: S phase |
| NHL | dexamethasone | Decadrone, Dexasone | No |
| NHL | doxorubicin | Adriamycin | Yes: S phase |
| NHL | Ibrutinib | Imbruvica | No |
| NHL | lenalidomide | Revlimid | No |
| NHL | methotrexate | Abitrexate, Mexate, Folex | Yes: S phase |
| NHL | obinutuzumab | Gazyva | No |
| NHL | prednisone | Deltasone, Medicorten | No |
| NHL | romidepsin | Istodax | |
| NHL | rituximab | Rituxan | No |
| NHL | vincristine | Vincasar | Yes: M phase |
| NSCLC | afatinib Dimaleate | Gilotrif | No |
| NSCLC | carboplatin | Paraplatin, Paraplat | Yes: S phase |
| NSCLC | cisplatin | Platinol | Yes: S phase |
| NSCLC | crizotinib | Xalkori | No |
| NSCLC | docetaxel | Taxotere | Yes: M phase |
| NSCLC | erlotinib | Tarceva | No |
| NSCLC | gemcitabine | Gemzar | Yes: S phase |
| NSCLC | methotrexate | Abitrexate, Mexate, Folex | Yes: S phase |
| NSCLC | paclitaxel | Abraxane, Taxol | Yes: M phase |
| NSCLC | palbociclib | Ibrance | Might cause G1 arrest |
| NSCLC | pemetrexed | Alimta | Yes: S phase |
| NSCLC | ramucirumab | Cyramza | No |
| ovarian | carboplatin | Paraplatin, Paraplat | Yes: S phase |
| ovarian | cisplatin | Platinol | Yes; S phase |
| ovarian | cyclophosphamide | Clafen, Cytoxan, Neosar | Yes: S phase |
| ovarian | gemcitabine | Gemzar | Yes: S phase |
| ovarian | olaparib | Lynparza | Yes: G2/M phase arrest |
| ovarian | paclitaxel | Abraxane, Taxol | Yes: M phase |
| ovarian | topotecan | Hycamtin | Yes: S phase |
| prostate | abiraterone | Zytiga | No |
| prostate | cabazitaxel | Jevtana | Yes: M phase |
| prostate | docetaxel | Taxotere | Yes: M phase |
| prostate | enzalutamide | Xtandi | No |
| prostate | goserelin acetate | Zoladex | No |
| prostate | prednisone | Deltasone, Medicorten | No |
| soft tissue sarcoma | doxorubicin | Adriamycin | Yes: S phase |
| soft tissue sarcoma | imatinib mesylate | Gleevec | No |
| soft tissue sarcoma | pazopanib | Votrient | No |
| T-cell lymphoma | romidepsin | Istodax | |

The peptidomimetic macrocycles or a composition comprising same and the at least one additional pharmaceutically active agent or a composition comprising same can be administered simultaneously (*i. e.,* simultaneous administration) and/or sequentially (*i*.*e*., sequential administration).

According to certain cases, the peptidomimetic macrocycles and the at least one additional pharmaceutically active agent are administered simultaneously, either in the same composition or in separate compositions. The term "simultaneous administration," as used herein, means that the peptidomimetic macrocycle and the at least one additional pharmaceutically active agent are administered with a time separation of no more than about 15 minutes, such as no more than about any of 10, 5, or 1 minutes. When the drugs are administered simultaneously, the peptidomimetic macrocycle and the at least one additional pharmaceutically active agent may be contained in the same composition *(e.g.,* a composition comprising both the peptidomimetic macrocycle and the at least additional pharmaceutically active agent) or in separate compositions (*e*.*g*., the peptidomimetic macrocycle is contained in one composition and the at least additional pharmaceutically active agent is contained in another composition).

According to other cases, the peptidomimetic macrocycles and the at least one additional pharmaceutically active agent are administered sequentially, *i.e.,* the peptidomimetic macrocycle is administered either prior to or after the administration of the additional pharmaceutically active agent. The term "sequential administration" as used herein means that the peptidomimetic macrocycle and the additional pharmaceutically active agent are administered with a time separation of more than about 15 minutes, such as more than about any of 20, 30, 40, 50, 60 or more minutes. Either the peptidomimetic macrocycle or the pharmaceutically active agent may be administered first. The peptidomimetic macrocycle and the additional pharmaceutically active agent are contained in separate compositions, which may be contained in the same or different packages.

In some cases, the administration of the peptidomimetic macrocycles and the additional pharmaceutically active agent are concurrent, *i.e.,* the administration period of the peptidomimetic macrocycles and that of the agent overlap with each other. In some cases, the administration of the peptidomimetic macrocycles and the additional pharmaceutically active agent are non-concurrent. For example, in some cases, the administration of the peptidomimetic macrocycles is terminated before the additional pharmaceutically active agent is administered. In some cases, the administration of the additional pharmaceutically active agent is terminated before the peptidomimetic macrocycle is administered. The time period between these two non-concurrent administrations can range from being days apart to being weeks apart.

The dosing frequency of the peptidomimetic macrocycle and the at least one additional pharmaceutically active agent may be adjusted over the course of the treatment, based on the judgment of the administering physician. When administered separately, the peptidomimetic macrocycle and the at least one additional pharmaceutically active agent can be administered at different dosing frequency or intervals. For example, the peptidomimetic macrocycle can be administered weekly, while the at least one additional pharmaceutically active agent can be administered more or less frequently. Or, the peptidomimetic macrocycle can be administered twice weekly, while the at least one additional pharmaceutically active agent can be administered more or less frequently. In addition, the peptidomimetic macrocycle and the at least one additional pharmaceutically active agent can be administered using the same route of administration or using different routes of administration.

According to certain cases, the peptidomimetic macrocycles and the additional pharmaceutically active agent are administered within a single pharmaceutical composition. According to some cases, the pharmaceutical composition further comprises pharmaceutically acceptable diluents or carrier. According to certain cases, the peptidomimetic macrocycles and the additional pharmaceutically active agent are administered within different pharmaceutical composition.

According to certain cases, peptidomimetic macrocycles is administered in an amount of from 0 mg/kg body weight to 100 mg/kg body weight. According to other cases, the peptidomimetic macrocycle is administered at an amount of from 0.5 mg/kg body weight to 20 mg/kg body weight. According to additional cases, the peptidomimetic macrocycle is administered at an amount of from 1.0 mg/kg body weight to 10 mg/kg body weight. The at least one additional pharmaceutical agent is administered at the therapeutic amount known to be used for treating the specific type of cancer. According to other cases, the at least one additional pharmaceutical agent is administered in an amount lower than the therapeutic amount known to be used for treating the disease, *i.e.* a sub-therapeutic amount of the at least one additional pharmaceutical agent is administered.

### Examples

### Example 1: Synthesis of 6-chlorotryptophan Fmoc amino acids

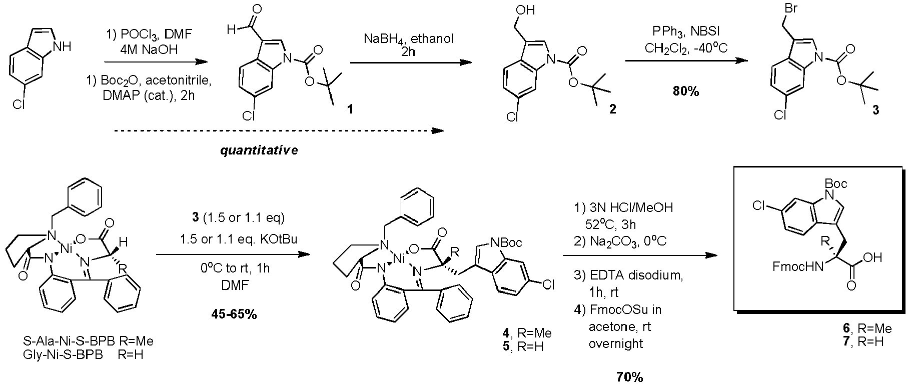

Tert-butyl 6-chloro-3-formyl-1H-indole-1-carboxylate, 1. To a stirred solution of dry DMF (12 mL) was added dropwise POCl₃ (3.92 mL, 43 mmol, 1.3 equiv) at 0 °C under Argon. The solution was stirred at the same temperature for 20 min before a solution of 6-chloroindole (5.0 g, 33 mmol, 1 eq.) in dry DMF (30 mL) was added dropwise. The resulting mixture was allowed to warm to room temperature and stirred for an additional 2.5h. Water (50 mL) was added and the solution was neutralized with 4M aqueous NaOH (pH ~ 8). The resulting solid was filtered off, washed with water and dried under vacuum. This material was directly used in the next step without additional purification. To a stirred solution of the crude formyl indole (33 mmol, 1 eq.) in THF (150 mL) was added successively Boc₂O (7.91 g, 36.3 mmol, 1.1 equiv) and DMAP (0.4 g, 3.3 mmol, 0.1 equiv) at room temperature under N₂. The resulting mixture was stirred at room temperature for 1.5h and the solvent was evaporated under reduced pressure. The residue was taken up in EtOAc and washed with 1N HCl, dried and concentrated to give the formyl indole 1 (9 g, 98 % over 2 steps) as a white solid. ¹H NMR (CDCl₃) δ: 1.70 (s, Boc, 9H); 7.35 (dd, 1H); 8.21 (m, 3H); 10.07 (s, 1H).

Tert-butyl 6-chloro-3-(hydroxymethyl)-1H-indole-1-carboxylate, 2. To a solution of compound 1 (8.86g, 32 mmol, 1 eq.) in ethanol (150 mL) was added NaBH₄ (2.4g, 63 mmol, 2 eq.). The reaction was stirred for 3 h at room temperature. The reaction mixture was concentrated and the residue was poured into diethyl ether and water. The organic layer was separated, dried over magnesium sulfate and concentrated to give a white solid (8.7g, 98%). This material was directly used in the next step without additional purification. ¹H NMR (CDCl₃) δ: 1.65 (s, Boc, 9H); 4.80 (s, 2H, CH₂); 7.21 (dd, 1H); 7.53 (m, 2H); 8.16 (bs, 1H).

Tert-butyl 3-(bromomethyl)-6-chloro-1H-indole-1-carboxylate, 3. To a solution of compound 2 (4.1g, 14.6 mmol, 1 eq.) in dichloromethane (50 mL) under argon was added a solution of triphenylphosphine (4.59g, 17.5 mmol, 1.2 eq.) in dichloromethane (50 mL) at -40°C. The reaction solution was stirred an additional 30 min at 40°C. Then NBS (3.38g, 19 mmol, 1.3 eq.) was added. The resulting mixture was allowed to warm to room temperature and stirred overnight. Dichloromethane was evaporated, Carbon Tetrachloride (100 mL) was added and the mixture was stirred for 1h and filtrated. The filtrate was concentrated, loaded in a silica plug and quickly eluted with 25% EtOAc in Hexanes. The solution was concentrated to give a white foam (3.84g, 77%). ¹H NMR (CDCl₃) δ: 1.66 (s, Boc, 9H); 4.63 (s, 2H, CH₂); 7.28 (dd, 1H); 7.57 (d, 1H); 7.64 (bs, 1H); 8.18 (bs, 1H).

αMe-6Cl-Trp(Boc)-Ni-S-BPB, **4.** To **S-Ala-Ni-S-BPB** (2.66g, 5.2 mmol, 1 eq.) and KO-tBu (0.87g, 7.8 mmol, 1.5 eq.) was added 50 mL of DMF under argon. The bromide derivative compound **3** (2.68g, 7.8 mmol, 1.5 eq.) in solution of DMF (5.0 mL) was added via syringe. The reaction mixture was stirred at ambient temperature for 1h. The solution was then quenched with 5 % aqueous acetic acid and diluted with water. The desired product was extracted in dichloromethane, dried and concentrated. The oily product **4** was purified by flash chromatography (solid loading) on normal phase using EtOAc and Hexanes as eluents to give a red solid (1.78g, 45% yield). αMe-6Cl-Trp(Boc)-Ni-S-BPB, **4:** M+H calc. 775.21, M+H obs. 775.26; ¹H NMR (CDCl₃) δ: 1.23 (s, 3H, aMe); 1.56 (m, 11H, Boc + CH₂); 1.82-2.20 (m, 4H, 2CH₂); 3.03 (m, 1H, CH_{α}); 3.24 (m, 2H, CH₂); 3.57 and 4.29 (AB system, 2H, CH₂ (benzyl), J= 12.8Hz); 6.62 (d, 2H); 6.98 (d, 1H); 7.14 (m, 2H); 7.23 (m, 1H); 7.32-7.36 (m, 5H); 7.50 (m, 2H); 7.67 (bs, 1H); 7.98 (d, 2H); 8.27 (m, 2H).

Fmoc-αMe-6Cl-Trp(Boc)-OH, 6. To a solution of 3N HCl/MeOH (1/3, 15 mL) at 50°C was added a solution of compound **4** (1.75g, 2.3 mmol, 1 eq.) in MeOH (5 ml) dropwise. The starting material disappeared within 3-4 h. The acidic solution was then cooled to 0°C with an ice bath and quenched with an aqueous solution of Na₂CO₃ (1.21g, 11.5 mmol, 5 eq.). Methanol was removed and 8 more equivalents of Na₂CO₃ (1.95g, 18.4 mmol) were added to the suspension. The Nickel scavenging EDTA disodium salt dihydrate (1.68g, 4.5 mmol, 2 eq.) was then added and the suspension was stirred for 2h. A solution of Fmoc-OSu (0.84g, 2.5 mmol, 1.1 eq.) in acetone (50 mL) was added and the reaction was stirred overnight. Afterwards, the reaction was diluted with diethyl ether and 1N HCl. The organic layer was then dried over magnesium sulfate and concentrated in vacuo. The desired product 6 was purified on normal phase using acetone and dichloromethane as eluents to give a white foam (0.9g, 70% yield). Fmoc-αMe-6Cl-Trp(Boc)-OH, 6: M+H calc. 575.19, M+H obs. 575.37; ¹H NMR (CDCl₃) 1.59 (s, 9H, Boc); 1.68 (s, 3H, Me); 3.48 (bs, 2H, CH₂); 4.22 (m, 1H, CH); 4.39 (bs, 2H, CH₂); 5.47 (s, 1H, NH); 7.10 (m, 1H); 7.18 (m, 2H); 7.27 (m, 2H); 7.39 (m, 2H); 7.50 (m, 2H); 7.75 (d, 2H); 8.12 (bs, 1H).

6Cl-Trp(Boc)-Ni-S-BPB, **5.** To **Gly-Ni-S-BPB** (4.6g, 9.2 mmol, 1 eq.) and KO-tBu (1.14g, 10.1 mmol, 1.1 eq.) was added 95 mL of DMF under argon. The bromide derivative compound 3 (3.5g, 4.6 mmol, 1.1 eq.) in solution of DMF (10 mL) was added via syringe. The reaction mixture was stirred at ambient temperature for 1h. The solution was then quenched with 5 % aqueous acetic acid and diluted with water. The desired product was extracted in dichloromethane, dried and concentrated. The oily product **5** was purified by flash chromatography (solid loading) on normal phase using EtOAc and Hexanes as eluents to give a red solid (5g, 71% yield). 6Cl-Trp(Boc)-Ni-S-BPB, 5: M+H calc. 761.20, M+H obs. 761.34; ¹H NMR (CDCl₃) δ: 1.58 (m, 11H, Boc + CH₂); 1.84 (m, 1H); 1.96 (m, 1H); 2.24 (m, 2H, CH₂); 3.00 (m, 1H, CH_{α}); 3.22 (m, 2H, CH₂); 3.45 and 4.25 (AB system, 2H, CH₂ (benzyl), J= 12.8Hz); 4.27 (m, 1H, CH_{α}); 6.65 (d, 2H); 6.88 (d, 1H); 7.07 (m, 2H); 7.14 (m, 2H); 7.28 (m, 3H); 7.35-7.39 (m, 2H); 7.52 (m, 2H); 7.96 (d, 2H); 8.28 (m, 2H).

Fmoc-6Cl-Trp(Boc)-OH, **7.** To a solution of 3N HCl/MeOH (1/3, 44 mL) at 50°C was added a solution of compound **5** (5g, 6.6 mmol, 1 eq.) in MeOH (10 ml) dropwise. The starting material disappeared within 3-4 h. The acidic solution was then cooled to 0°C with an ice bath and quenched with an aqueous solution of Na₂CO₃ (3.48g, 33 mmol, 5 eq.). Methanol was removed and 8 more equivalents of Na₂CO₃ (5.57g, 52 mmol) were added to the suspension. The Nickel scavenging EDTA disodium salt dihydrate (4.89g, 13.1 mmol, 2 eq.) and the suspension was stirred for 2h. A solution of Fmoc-OSu (2.21g, 6.55 mmol, 1.1 eq.) in acetone (100 mL) was added and the reaction was stirred overnight. Afterwards, the reaction was diluted with diethyl ether and 1N HCl. The organic layer was then dried over magnesium sulfate and concentrated in vacuo. The desired product **7** was purified on normal phase using acetone and dichloromethane as eluents to give a white foam (2.6g, 69% yield). Fmoc-6Cl-Trp(Boc)-OH, 7: M+H calc. 561.17, M+H obs. 561.37; ¹H NMR (CDCl₃) 1.63 (s, 9H, Boc); 3.26 (m, 2H, CH₂); 4.19 (m, 1H, CH); 4.39 (m, 2H, CH₂); 4.76 (m, 1H); 5.35 (d, 1H, NH); 7.18 (m, 2H); 7.28 (m, 2H); 7.39 (m, 3H); 7.50 (m, 2H); 7.75 (d, 2H); 8.14 (bs, 1H).

### Example 2: Peptidomimetic macrocycles

Peptidomimetic macrocycles were synthesized, purified and analyzed as previously described and as described below (Schafmeister et al., J. Am. Chem. Soc. 122:5891-5892 (2000); Schafineister & Verdine, J. Am. Chem. Soc. 122:5891 (2005); Walensky et al., Science 305: 1466-1470 (2004); and US Patent No. 7,192,713). Peptidomimetic macrocycles were designed by replacing two or more naturally occurring amino acids with the corresponding synthetic amino acids. Substitutions were made at i and i+4, and i and i+7 positions. Peptide synthesis was performed either manually or on an automated peptide synthesizer (Applied Biosystems, model 433A), using solid phase conditions, rink amide AM resin (Novabiochem), and Fmoc main-chain protecting group chemistry. For the coupling of natural Fmoc-protected amino acids (Novabiochem), 10 equivalents of amino acid and a 1:1:2 molar ratio of coupling reagents HBTU/HOBt (Novabiochem)/DIEA were employed. Non-natural amino acids (4 equiv) were coupled with a 1:1:2 molar ratio of HATU (Applied Biosystems)/HOBt/DIEA. The N-termini of the synthetic peptides were acetylated, while the C-termini were amidated.

Purification of cross-linked compounds was achieved by high performance liquid chromatography (HPLC) (Varian ProStar) on a reverse phase C18 column (Varian) to yield the pure compounds. Chemical composition of the pure products was confirmed by LC/MS mass spectrometry (Micromass LCT interfaced with Agilent 1100 HPLC system) and amino acid analysis (Applied Biosystems, model 420A).

The following protocol was used in the synthesis of dialkyne-crosslinked peptidomimetic macrocycles, including SP662, SP663 and SP664. Fully protected resin-bound peptides were synthesized on a PEG-PS resin (loading 0.45 mmol/g) on a 0.2 mmol scale. Deprotection of the temporary Fmoc group was achieved by 3 × 10 min treatments of the resin bound peptide with 20% (v/v) piperidine in DMF. After washing with NMP (3x), dichloromethane (3x) and NMP (3x), coupling of each successive amino acid was achieved with 1 × 60 min incubation with the appropriate preactivated Fmoc-amino acid derivative. All protected amino acids (0.4 mmol) were dissolved in NMP and activated with HCTU (0.4 mmol) and DIEA (0.8 mmol) prior to transfer of the coupling solution to the deprotected resin-bound peptide. After coupling was completed, the resin was washed in preparation for the next deprotection/coupling cycle. Acetylation of the amino terminus was carried out in the presence of acetic anhydride/DIEA in NMP. The LC-MS analysis of a cleaved and deprotected sample obtained from an aliquot of the fully assembled resin-bound peptide was accomplished in order to verifying the completion of each coupling. In a typical example, tetrahydrofuran (4ml) and triethylamine (2ml) were added to the peptide resin (0.2 mmol) in a 40ml glass vial and shaken for 10 minutes. Pd(PPh₃)₂Cl₂ (0.014g, 0.02 mmol) and copper iodide (0.008g, 0.04 mmol) were then added and the resulting reaction mixture was mechanically shaken 16 hours while open to atmosphere. The diyne-cyclized resin-bound peptides were deprotected and cleaved from the solid support by treatment with TFA/H₂O/TIS (95/5/5 v/v) for 2.5 h at room temperature. After filtration of the resin the TFA solution was precipitated in cold diethyl ether and centrifuged to yield the desired product as a solid. The crude product was purified by preparative HPLC.

The following protocol was used in the synthesis of single alkyne-crosslinked peptidomimetic macrocycles, including SP665. Fully protected resin-bound peptides were synthesized on a Rink amide MBHA resin (loading 0.62 mmol/g) on a 0.1 mmol scale. Deprotection of the temporary Fmoc group was achieved by 2 × 20 min treatments of the resin bound peptide with 25% (v/v) piperidine in NMP. After extensive flow washing with NMP and dichloromethane, coupling of each successive amino acid was achieved with 1 × 60 min incubation with the appropriate preactivated Fmoc-amino acid derivative. All protected amino acids (1 mmol) were dissolved in NMP and activated with HCTU (1 mmol) and DIEA (1 mmol) prior to transfer of the coupling solution to the deprotected resin-bound peptide. After coupling was completed, the resin was extensively flow washed in preparation for the next deprotection/coupling cycle. Acetylation of the amino terminus was carried out in the presence of acetic anhydride/DIEA in NMP/NMM. The LC-MS analysis of a cleaved and deprotected sample obtained from an aliquot of the fully assembled resin-bound peptide was accomplished in order to verifying the completion of each coupling. In a typical example, the peptide resin (0.1 mmol) was washed with DCM. Resin was loaded into a microwave vial. The vessel was evacuated and purged with nitrogen. Molybdenumhexacarbonyl (0.01 eq, Sigma Aldrich 199959) was added. Anhydrous chlorobenzene was added to the reaction vessel. Then 2-fluorophenol (1eq, Sigma Aldrich F12804) was added. The reaction was then loaded into the microwave and held at 130°C for 10 minutes. Reaction may need to be pushed a subsequent time for completion. The alkyne metathesized resin-bound peptides were deprotected and cleaved from the solid support by treatment with TFA/H₂O/TIS (94/3/3 v/v) for 3 h at room temperature. After filtration of the resin the TFA solution was precipitated in cold diethyl ether and centrifuged to yield the desired product as a solid. The crude product was purified by preparative HPLC.

Table 1 shows a list of peptidomimetic macrocycles prepared.

**Table 1**

| **SP** | **Sequence** | **Isomer** | **Exact Mass** | **Found Mass** | **Calc (M+1)/1** | **Calc (M+2)/2** | **Calc (M+3)/3** |
|---|---|---|---|---|---|---|---|
| SP1 | Ac-F$r8AYWEAc3cL$AAA-NH₂ | | 1456.78 | 729.44 | 1457.79 | 729.4 | 486.6 |
| SP2 | Ac-F$r8AYWEAc3cL$AAibA-NH₂ | | 1470.79 | 736.4 | 1471.8 | 736.4 | 491.27 |
| SP3 | Ac-LTF$r8AYWAQL$SANle-NH₂ | | 1715.97 | 859.02 | 1716.98 | 858.99 | 573 |
| SP4 | Ac-LTF$r8AYWAQL$SAL-NH₂ | | 1715.97 | 859.02 | 1716.98 | 858.99 | 573 |
| SP5 | Ac-LTF$r8AYWAQL$SAM-NH₂ | | 1733.92 | 868.48 | 1734.93 | 867.97 | 578.98 |
| SP6 | Ac-LTF$r8AYWAQL$SAhL-NH₂ | | 1729.98 | 865.98 | 1730.99 | 866 | 577.67 |
| SP7 | Ac-LTF$r8AYWAQL$SAF-NH₂ | | 1749.95 | 876.36 | 1750.96 | 875.98 | 584.32 |
| SP8 | Ac-LTF$r8AYWAQL$SAI-NH₂ | | 1715.97 | 859.02 | 1716.98 | 858.99 | 573 |
| SP9 | Ac-LTF$r8AYWAQL$SAChg-NH₂ | | 1741.98 | 871.98 | 1742.99 | 872 | 581.67 |
| SP10 | Ac-LTF$r8AYWAQL$SAAib-NH₂ | | 1687.93 | 845.36 | 1688.94 | 844.97 | 563.65 |
| SP11 | Ac-LTF$r8AYWAQL$SAA-NH₂ | | 1673.92 | 838.01 | 1674.93 | 837.97 | 558.98 |
| SP12 | Ac-LTF$r8AYWA$L$S$Nle-NH₂ | | 1767.04 | 884.77 | 1768.05 | 884.53 | 590.02 |
| SP13 | Ac-LTF$r8AYWA$L$S$A-NH₂ | | 1724.99 | 864.23 | 1726 | 863.5 | 576 |
| SP14 | Ac-F$r8AYWEAc3cL$AANle-NH₂ | | 1498.82 | 750.46 | 1499.83 | 750.42 | 500.61 |
| SP15 | Ac-F$r8AYWEAc3cL$AAL-NH₂ | | 1498.82 | 750.46 | 1499.83 | 750.42 | 500.61 |
| SP16 | Ac-F$r8AYWEAc3cL$AAM-NH₂ | | 1516.78 | 759.41 | 1517.79 | 759.4 | 506.6 |
| SP17 | Ac-F$r8AYWEAc3cL$AAhL-NH₂ | | 1512.84 | 757.49 | 1513.85 | 757.43 | 505.29 |
| SP18 | Ac-F$r8AYWEAc3cL$AAF-NH₂ | | 1532.81 | 767.48 | 1533.82 | 767.41 | 511.94 |
| SP19 | Ac-F$r8AYWEAc3cL$AAI-NH₂ | | 1498.82 | 750.39 | 1499.83 | 750.42 | 500.61 |
| SP20 | Ac-F$r8AYWEAc3cL$AAChg-NH₂ | | 1524.84 | 763.48 | 1525.85 | 763.43 | 509.29 |
| SP21 | Ac-F$r8AYWEAc3cL$AACha-NH₂ | | 1538.85 | 770.44 | 1539.86 | 770.43 | 513.96 |
| SP22 | Ac-F$r8AYWEAc3cL$AAAib-NH₂ | | 1470.79 | 736.84 | 1471.8 | 736.4 | 491.27 |
| SP23 | Ac-LTF$r8AYWAQL$AAAibV-NH₂ | | 1771.01 | 885.81 | 1772.02 | 886.51 | 591.34 |
| SP24 | Ac-LTF$r8AYWAQL$AAAibV-NH₂ | iso2 | 1771.01 | 886.26 | 1772.02 | 886.51 | 591.34 |
| SP25 | Ac-LTF$r8AYWAQL$SAibAA-NH₂ | | 1758.97 | 879.89 | 1759.98 | 880.49 | 587.33 |
| SP26 | Ac-LTF$r8AYWAQL$SAibAA-NH₂ | iso2 | 1758.97 | 880.34 | 1759.98 | 880.49 | 587.33 |
| SP27 | Ac-HLTF$r8HHWHQL$AANleNle-NH₂ | | 2056.15 | 1028.86 | 2057.16 | 1029.08 | 686.39 |
| SP28 | Ac-DLTF$r8HHWHQL$RRLV-NH₂ | | 2190.23 | 731.15 | 2191.24 | 1096.12 | 731.08 |
| SP29 | Ac-HHTF$r8HHWHQL$AAML-NH₂ | | 2098.08 | 700.43 | 2099.09 | 1050.05 | 700.37 |
| SP30 | Ac-F$r8HHWHQL$RRDCha-NH₂ | | 1917.06 | 959.96 | 1918.07 | 959.54 | 640.03 |
| SP31 | Ac-F$r8HHWHQL$HRFV-NH₂ | | 1876.02 | 938.65 | 1877.03 | 939.02 | 626.35 |
| SP32 | Ac-HLTF$r8HHWHQL$AAhLA-NH₂ | | 2028.12 | 677.2 | 2029.13 | 1015.07 | 677.05 |
| SP33 | Ac-DLTF$r8HHWHQL$RRChgl-NH₂ | | 2230.26 | 1115.89 | 2231.27 | 1116.14 | 744.43 |
| SP34 | Ac-DLTF$r8HHWHQL$RRChgl-NH₂ | iso2 | 2230.26 | 1115.96 | 2231.27 | 1116.14 | 744.43 |
| SP35 | Ac-HHTF$r8HHWHQL$AAChav-NH₂ | | 2106.14 | 1053.95 | 2107.15 | 1054.08 | 703.05 |
| SP36 | Ac-F$r8HHWHQL$RRDa-NH₂ | | 1834.99 | 918.3 | 1836 | 918.5 | 612.67 |
| SP37 | Ac-F$r8HHWHQL$HRAibG-NH₂ | | 1771.95 | 886.77 | 1772.96 | 886.98 | 591.66 |
| SP38 | Ac-F$r8AYWAQL$HHNleL-NH₂ | | 1730.97 | 866.57 | 1731.98 | 866.49 | 578 |
| SP39 | Ac-F$r8AYWSAL$HQANle-NH₂ | | 1638.89 | 820.54 | 1639.9 | 820.45 | 547.3 |
| SP40 | Ac-F$r8AYWVQL$QHChgl-NH₂ | | 1776.01 | 889.44 | 1777.02 | 889.01 | 593.01 |
| SP41 | Ac-F$r8AYWTAL$QQNlev-NH₂ | | 1671.94 | 836.97 | 1672.95 | 836.98 | 558.32 |
| SP42 | Ac-F$r8AYWYQL$HAibAa-NH₂ | | 1686.89 | 844.52 | 1687.9 | 844.45 | 563.3 |
| SP43 | Ac-LTF$r8AYWAQL$HHLa-NH₂ | | 1903.05 | 952.27 | 1904.06 | 952.53 | 635.36 |
| SP44 | Ac-LTF$r8AYWAQL$HHLa-NH₂ | iso2 | 1903.05 | 952.27 | 1904.06 | 952.53 | 635.36 |
| SP45 | Ac-LTF$r8AYWAQL$HQNlev-NH₂ | | 1922.08 | 962.48 | 1923.09 | 962.05 | 641.7 |
| SP46 | Ac-LTF$r8AYWAQL$HQNlev-NH₂ | iso2 | 1922.08 | 962.4 | 1923.09 | 962.05 | 641.7 |
| SP47 | Ac-LTF$r8AYWAQL$QQMl-NH₂ | | 1945.05 | 973.95 | 1946.06 | 973.53 | 649.36 |
| SP48 | Ac-LTF$r8AYWAQL$QQMl-NH₂ | iso2 | 1945.05 | 973.88 | 1946.06 | 973.53 | 649.36 |
| SP49 | Ac-LTF$r8AYWAQL$HAibhLV-NH₂ | | 1893.09 | 948.31 | 1894.1 | 947.55 | 632.04 |
| SP50 | Ac-LTF$r8AYWAQL$AHFA-NH₂ | | 1871.01 | 937.4 | 1872.02 | 936.51 | 624.68 |
| SP51 | Ac-HLTF$r8HHWHQL$AANlel-NH₂ | | 2056.15 | 1028.79 | 2057.16 | 1029.08 | 686.39 |
| SP52 | Ac-DLTF$r8HHWHQL$RRLa-NH₂ | | 2162.2 | 721.82 | 2163.21 | 1082.11 | 721.74 |
| SP53 | Ac-HHTF$r8HHWHQL$AAMv-NH₂ | | 2084.07 | 1042.92 | 2085.08 | 1043.04 | 695.7 |
| SP54 | Ac-F$r8HHWHQL$RRDA-NH₂ | | 1834.99 | 612.74 | 1836 | 918.5 | 612.67 |
| SP55 | Ac-F$r8HHWHQL$HRFCha-NH₂ | | 1930.06 | 966.47 | 1931.07 | 966.04 | 644.36 |
| SP56 | Ac-F$r8AYWEAL$AA-NHAm | | 1443.82 | 1445.71 | 1444.83 | 722.92 | 482.28 |
| SP57 | Ac-F$r8AYWEAL$AA-NHiAm | | 1443.82 | 723.13 | 1444.83 | 722.92 | 482.28 |
| SP58 | Ac-F$r8AYWEAL$AA-NHnPr3Ph | | 1491.82 | 747.3 | 1492.83 | 746.92 | 498.28 |
| SP59 | Ac-F$r8AYWEAL$AA-NHnBu33Me | | 1457.83 | 1458.94 | 1458.84 | 729.92 | 486.95 |
| SP60 | Ac-F$r8AYWEAL$AA-NHnPr | | 1415.79 | 709.28 | 1416.8 | 708.9 | 472.94 |
| SP61 | Ac-F$r8AYWEAL$AA-NHnEt2Ch | | 1483.85 | 1485.77 | 1484.86 | 742.93 | 495.62 |
| SP62 | Ac-F$r8AYWEAL$AA-NHnEt2Cp | | 1469.83 | 1470.78 | 1470.84 | 735.92 | 490.95 |
| SP63 | Ac-F$r8AYWEAL$AA-NHHex | | 1457.83 | 730.19 | 1458.84 | 729.92 | 486.95 |
| SP64 | Ac-LTF$r8AYWAQL$AAIA-NH₂ | | 1771.01 | 885.81 | 1772.02 | 886.51 | 591.34 |
| SP65 | Ac-LTF$r8AYWAQL$AAIA-NH₂ | iso2 | 1771.01 | 866.8 | 1772.02 | 886.51 | 591.34 |
| SP66 | Ac-LTF$r8AYWAAL$AAMA-NH₂ | | 1731.94 | 867.08 | 1732.95 | 866.98 | 578.32 |
| SP67 | Ac-LTF$r8AYWAAL$AAMA-NH₂ | iso2 | 1731.94 | 867.28 | 1732.95 | 866.98 | 578.32 |
| SP68 | Ac-LTF$r8AYWAQL$AANleA-NH₂ | | 1771.01 | 867.1 | 1772.02 | 886.51 | 591.34 |
| SP69 | Ac-LTF$r8AYWAQL$AANleA-NH₂ | iso2 | 1771.01 | 886.89 | 1772.02 | 886.51 | 591.34 |
| SP70 | Ac-LTF$r8AYWAQL$AAIa-NH₂ | | 1771.01 | 886.8 | 1772.02 | 886.51 | 591.34 |
| SP71 | Ac-LTF$r8AYWAQL$AAIa-NH₂ | iso2 | 1771.01 | 887.09 | 1772.02 | 886.51 | 591.34 |
| SP72 | Ac-LTF$r8AYWAAL$AAMa-NH₂ | | 1731.94 | 867.17 | 1732.95 | 866.98 | 578.32 |
| SP73 | Ac-LTF$r8AYWAAL$AAMa-NH₂ | iso2 | 1731.94 | 867.37 | 1732.95 | 866.98 | 578.32 |
| SP74 | Ac-LTF$r8AYWAQL$AANlea-NH₂ | | 1771.01 | 887.08 | 1772.02 | 886.51 | 591.34 |
| SP75 | Ac-LTF$r8AYWAQL$AANlea-NH₂ | iso2 | 1771.01 | 887.08 | 1772.02 | 886.51 | 591.34 |
| SP76 | Ac-LTF$r8AYWAAL$AAIv-NH₂ | | 1742.02 | 872.37 | 1743.03 | 872.02 | 581.68 |
| SP77 | Ac-LTF$r8AYWAAL$AAIv-NH₂ | iso2 | 1742.02 | 872.74 | 1743.03 | 872.02 | 581.68 |
| SP78 | Ac-LTF$r8AYWAQL$AAMv-NH₂ | | 1817 | 910.02 | 1818.01 | 909.51 | 606.67 |
| SP79 | Ac-LTF$r8AYWAAL$AANlev-NH₂ | | 1742.02 | 872.37 | 1743.03 | 872.02 | 581.68 |
| SP80 | Ac-LTF$r8AYWAAL$AANlev-NH₂ | iso2 | 1742.02 | 872.28 | 1743.03 | 872.02 | 581.68 |
| SP81 | Ac-LTF$r8AYWAQL$AAIl-NH₂ | | 1813.05 | 907.81 | 1814.06 | 907.53 | 605.36 |
| SP82 | Ac-LTF$r8AYWAQL$AAIl-NH₂ | iso2 | 1813.05 | 907.81 | 1814.06 | 907.53 | 605.36 |
| SP83 | Ac-LTF$r8AYWAAL$AAMl-NH₂ | | 1773.99 | 887.37 | 1775 | 888 | 592.34 |
| SP84 | Ac-LTF$r8AYWAQL$AANlel-NH₂ | | 1813.05 | 907.61 | 1814.06 | 907.53 | 605.36 |
| SP85 | Ac-LTF$r8AYWAQL$AANlel-NH₂ | iso2 | 1813.05 | 907.71 | 1814.06 | 907.53 | 605.36 |
| SP86 | Ac-F$r8AYWEAL$AAMA-NH₂ | | 1575.82 | 789.02 | 1576.83 | 788.92 | 526.28 |
| SP87 | Ac-F$r8AYWEAL$AANleA-NH₂ | | 1557.86 | 780.14 | 1558.87 | 779.94 | 520.29 |
| SP88 | Ac-F$r8AYWEAL$AAIa-NH₂ | | 1557.86 | 780.33 | 1558.87 | 779.94 | 520.29 |
| SP89 | Ac-F$r8AYWEAL$AAMa-NH₂ | | 1575.82 | 789.3 | 1576.83 | 788.92 | 526.28 |
| SP90 | Ac-F$r8AYWEAL$AANlea-NH₂ | | 1557.86 | 779.4 | 1558.87 | 779.94 | 520.29 |
| SP91 | Ac-F$r8AYWEAL$AAIv-NH₂ | | 1585.89 | 794.29 | 1586.9 | 793.95 | 529.64 |
| SP92 | Ac-F$r8AYWEAL$AAMv-NH₂ | | 1603.85 | 803.08 | 1604.86 | 802.93 | 535.62 |
| SP93 | Ac-F$r8AYWEAL$AANlev-NH₂ | | 1585.89 | 793.46 | 1586.9 | 793.95 | 529.64 |
| SP94 | Ac-F$r8AYWEAL$AAIl-NH₂ | | 1599.91 | 800.49 | 1600.92 | 800.96 | 534.31 |
| SP95 | Ac-F$r8AYWEAL$AAMl-NH₂ | | 1617.86 | 809.44 | 1618.87 | 809.94 | 540.29 |
| SP96 | Ac-F$r8AYWEAL$AANlel-NH₂ | | 1599.91 | 801.7 | 1600.92 | 800.96 | 534.31 |
| SP97 | Ac-F$r8AYWEAL$AANlel-NH₂ | iso2 | 1599.91 | 801.42 | 1600.92 | 800.96 | 534.31 |
| SP98 | Ac-LTF$r8AY6clWAQL$SAA-NH₂ | | 1707.88 | 855.72 | 1708.89 | 854.95 | 570.3 |
| SP99 | Ac-LTF$r8AY6clWAQL$SAA-NH₂ | iso2 | 1707.88 | 855.35 | 1708.89 | 854.95 | 570.3 |
| SP100 | Ac-WTF$r8FYWSQL$AVAa-NH₂ | | 1922.01 | 962.21 | 1923.02 | 962.01 | 641.68 |
| SP101 | Ac-WTF$r8FYWSQL$AVAa-NH₂ | iso2 | 1922.01 | 962.49 | 1923.02 | 962.01 | 641.68 |
| SP 102 | Ac-WTF$r8VYWSQL$AVA-NH₂ | | 1802.98 | 902.72 | 1803.99 | 902.5 | 602 |
| SP103 | Ac-WTF$r8VYWSQL$AVA-NH₂ | iso2 | 1802.98 | 903 | 1803.99 | 902.5 | 602 |
| SP104 | Ac-WTF$r8FYWSQL$SAAa-NH₂ | | 1909.98 | 956.47 | 1910.99 | 956 | 637.67 |
| SP105 | Ac-WTF$r8FYWSQL$SAAa-NH₂ | iso2 | 1909.98 | 956.47 | 1910.99 | 956 | 637.67 |
| SP 106 | Ac-WTF$r8VYWSQL$AVAaa-NH₂ | | 1945.05 | 974.15 | 1946.06 | 973.53 | 649.36 |
| SP107 | Ac-WTF$r8VYWSQL$AVAaa-NH₂ | iso2 | 1945.05 | 973.78 | 1946.06 | 973.53 | 649.36 |
| SP108 | Ac-LTF$r8AYWAQL$AVG-NH₂ | | 1671.94 | 837.52 | 1672.95 | 836.98 | 558.32 |
| SP 109 | Ac-LTF$r8AYWAQL$AVG-NH₂ | iso2 | 1671.94 | 837.21 | 1672.95 | 836.98 | 558.32 |
| SP110 | Ac-LTF$r8AYWAQL$AVQ-NH₂ | | 1742.98 | 872.74 | 1743.99 | 872.5 | 582 |
| SP111 | Ac-LTF$r8AYWAQL$AVQ-NH₂ | iso2 | 1742.98 | 872.74 | 1743.99 | 872.5 | 582 |
| SP112 | Ac-LTF$r8AYWAQL$SAa-NH₂ | | 1673.92 | 838.23 | 1674.93 | 837.97 | 558.98 |
| SP113 | Ac-LTF$r8AYWAQL$SAa-NH₂ | iso2 | 1673.92 | 838.32 | 1674.93 | 837.97 | 558.98 |
| SP114 | Ac-LTF$r8AYWAQhL$SAA-NH₂ | | 1687.93 | 844.37 | 1688.94 | 844.97 | 563.65 |
| SP115 | Ac-LTF$r8AYWAQhL$SAA-NH₂ | iso2 | 1687.93 | 844.81 | 1688.94 | 844.97 | 563.65 |
| SP116 | Ac-LTF$r8AYWEQLStSA$-NH₂ | | 1826 | 905.27 | 1827.01 | 914.01 | 609.67 |
| SP117 | Ac-LTF$r8AYWAQL$SLA-NH₂ | | 1715.97 | 858.48 | 1716.98 | 858.99 | 573 |
| SP118 | Ac-LTF$r8AYWAQL$SLA-NH₂ | iso2 | 1715.97 | 858.87 | 1716.98 | 858.99 | 573 |
| SP119 | Ac-LTF$r8AYWAQL$SWA-NH₂ | | 1788.96 | 895.21 | 1789.97 | 895.49 | 597.33 |
| SP120 | Ac-LTF$r8AYWAQL$SWA-NH₂ | iso2 | 1788.96 | 895.28 | 1789.97 | 895.49 | 597.33 |
| SP121 | Ac-LTF$r8AYWAQL$SVS-NH₂ | | 1717.94 | 859.84 | 1718.95 | 859.98 | 573.65 |
| SP122 | Ac-LTF$r8AYWAQL$SAS-NH₂ | | 1689.91 | 845.85 | 1690.92 | 845.96 | 564.31 |
| SP123 | Ac-LTF$r8AYWAQL$SVG-NH₂ | | 1687.93 | 844.81 | 1688.94 | 844.97 | 563.65 |
| SP124 | Ac-ETF$r8VYWAQL$SAa-NH₂ | | 1717.91 | 859.76 | 1718.92 | 859.96 | 573.64 |
| SP125 | Ac-ETF$r8VYWAQL$SAA-NH₂ | | 1717.91 | 859.84 | 1718.92 | 859.96 | 573.64 |
| SP126 | Ac-ETF$r8VYWAQL$SVA-NH₂ | | 1745.94 | 873.82 | 1746.95 | 873.98 | 582.99 |
| SP 127 | Ac-ETF$r8VYWAQL$SLA-NH₂ | | 1759.96 | 880.85 | 1760.97 | 880.99 | 587.66 |
| SP128 | Ac-ETF$r8VYWAQL$SWA-NH₂ | | 1832.95 | 917.34 | 1833.96 | 917.48 | 611.99 |
| SP129 | Ac-ETF$r8KYWAQL$SWA-NH₂ | | 1861.98 | 931.92 | 1862.99 | 932 | 621.67 |
| SP130 | Ac-ETF$r8VYWAQL$SVS-NH₂ | | 1761.93 | 881.89 | 1762.94 | 881.97 | 588.32 |
| SP131 | Ac-ETF$r8VYWAQL$SAS-NH₂ | | 1733.9 | 867.83 | 1734.91 | 867.96 | 578.97 |
| SP132 | Ac-ETF$r8VYWAQL$SVG-NH₂ | | 1731.92 | 866.87 | 1732.93 | 866.97 | 578.31 |
| SP133 | Ac-LTF$r8VYWAQL$SSa-NH₂ | | 1717.94 | 859.47 | 1718.95 | 859.98 | 573.65 |
| SP134 | Ac-ETF$r8VYWAQL$SSa-NH₂ | | 1733.9 | 867.83 | 1734.91 | 867.96 | 578.97 |
| SP135 | Ac-LTF$r8VYWAQL$SNa-NH₂ | | 1744.96 | 873.38 | 1745.97 | 873.49 | 582.66 |
| SP136 | Ac-ETF$r8VYWAQL$SNa-NH₂ | | 1760.91 | 881.3 | 1761.92 | 881.46 | 587.98 |
| SP137 | Ac-LTF$r8VYWAQL$SAa-NH₂ | | 1701.95 | 851.84 | 1702.96 | 851.98 | 568.32 |
| SP138 | Ac-LTF$r8VYWAQL$SVA-NH₂ | | 1729.98 | 865.53 | 1730.99 | 866 | 577.67 |
| SP139 | Ac-LTF$r8VYWAQL$SVA-NH₂ | iso2 | 1729.98 | 865.9 | 1730.99 | 866 | 577.67 |
| SP140 | Ac-LTF$r8VYWAQL$SWA-NH₂ | | 1816.99 | 909.42 | 1818 | 909.5 | 606.67 |
| SP141 | Ac-LTF$r8VYWAQL$SVS-NH₂ | | 1745.98 | 873.9 | 1746.99 | 874 | 583 |
| SP142 | Ac-LTF$r8VYWAQL$SVS-NH₂ | iso2 | 1745.98 | 873.9 | 1746.99 | 874 | 583 |
| **S**P143 | Ac-LTF$r8VYWAQL$SAS-NH₂ | | 1717.94 | 859.84 | 1718.95 | 859.98 | 573.65 |
| SP144 | Ac-LTF$r8VYWAQL$SAS-NH₂ | iso2 | 1717.94 | 859.91 | 1718.95 | 859.98 | 573.65 |
| SP145 | Ac-LTF$r8VYWAQL$SVG-NH₂ | | 1715.97 | 858.87 | 1716.98 | 858.99 | 573 |
| SP146 | Ac-LTF$r8VYWAQL$SVG-NH₂ | iso2 | 1715.97 | 858.87 | 1716.98 | 858.99 | 573 |
| SP147 | Ac-LTF$r8EYWAQCha$SAA-NH₂ | | 1771.96 | 886.85 | 1772.97 | 886.99 | 591.66 |
| SP148 | Ac-LTF$r8EYWAQCha$SAA-NH₂ | iso2 | 1771.96 | 886.85 | 1772.97 | 886.99 | 591.66 |
| SP149 | Ac-LTF$r8EYWAQCpg$SAA-NH₂ | | 1743.92 | 872.86 | 1744.93 | 872.97 | 582.31 |
| SP150 | Ac-LTF$r8EYWAQCpg$SAA-NH₂ | iso2 | 1743.92 | 872.86 | 1744.93 | 872.97 | 582.31 |
| SP151 | Ac-LTF$r8EYWAQF$SAA-NH₂ | | 1765.91 | 883.44 | 1766.92 | 883.96 | 589.64 |
| SP152 | Ac-LTF$r8EYWAQF$SAA-NH₂ | iso2 | 1765.91 | 883.89 | 1766.92 | 883.96 | 589.64 |
| SP153 | Ac-LTF$r8EYWAQCba$SAA-NH₂ | | 1743.92 | 872.42 | 1744.93 | 872.97 | 582.31 |
| SP154 | Ac-LTF$r8EYWAQCba$SAA-NH₂ | iso2 | 1743.92 | 873.39 | 1744.93 | 872.97 | 582.31 |
| SP155 | Ac-LTF3Cl$r8EYWAQL$SAA-NH₂ | | 1765.89 | 883.89 | 1766.9 | 883.95 | 589.64 |
| SP156 | Ac-LTF3Cl$r8EYWAQL$SAA-NH₂ | iso2 | 1765.89 | 883.96 | 1766.9 | 883.95 | 589.64 |
| SP157 | Ac-LTF34F2$r8EYWAQL$SAA-NH₂ | | 1767.91 | 884.48 | 1768.92 | 884.96 | 590.31 |
| SP158 | Ac-LTF34F2$r8EYWAQL$SAA-NH₂ | iso2 | 1767.91 | 884.48 | 1768.92 | 884.96 | 590.31 |
| SP159 | Ac-LTF34F2$r8EYWAQhL$SAA-NH₂ | | 1781.92 | 891.44 | 1782.93 | 891.97 | 594.98 |
| SP160 | Ac-LTF34F2$r8EYWAQhL$SAA-NH₂ | iso2 | 1781.92 | 891.88 | 1782.93 | 891.97 | 594.98 |
| SP161 | Ac-ETF$r8EYWAQL$SAA-NH₂ | | 1747.88 | 874.34 | 1748.89 | 874.95 | 583.63 |
| **S**P162 | Ac-LTF$r8AYWVQL$SAA-NH₂ | | 1701.95 | 851.4 | 1702.96 | 851.98 | 568.32 |
| SP163 | Ac-LTF$r8AHWAQL$SAA-NH₂ | | 1647.91 | 824.83 | 1648.92 | 824.96 | 550.31 |
| SP164 | Ac-LTF$r8AEWAQL$SAA-NH₂ | | 1639.9 | 820.39 | 1640.91 | 820.96 | 547.64 |
| SP165 | Ac-LTF$r8ASWAQL$SAA-NH₂ | | 1597.89 | 799.38 | 1598.9 | 799.95 | 533.64 |
| SP166 | Ac-LTF$r8AEWAQL$SAA-NH₂ | iso2 | 1639.9 | 820.39 | 1640.91 | 820.96 | 547.64 |
| SP167 | Ac-LTF$r8ASWAQL$SAA-NH₂ | iso2 | 1597.89 | 800.31 | 1598.9 | 799.95 | 533.64 |
| SP168 | Ac-LTF$r8AF4coohWAQL$SAA-NH₂ | | 1701.91 | 851.4 | 1702.92 | 851.96 | 568.31 |
| **S**P169 | Ac-LTF$r8AF4coohWAQL$SAA-NH₂ | iso2 | 1701.91 | 851.4 | 1702.92 | 851.96 | 568.31 |
| SP170 | Ac-LTF$r8AHWAQL$AAIa-NH₂ | | 1745 | 874.13 | 1746.01 | 873.51 | 582.67 |
| SP171 | Ac-ITF$r8FYWAQL$AAIa-NH₂ | | 1847.04 | 923.92 | 1848.05 | 924.53 | 616.69 |
| SP172 | Ac-ITF$r8EHWAQL$AAIa-NH₂ | | 1803.01 | 903.17 | 1804.02 | 902.51 | 602.01 |
| SP173 | Ac-ITF$r8EHWAQL$AAIa-NH₂ | iso2 | 1803.01 | 903.17 | 1804.02 | 902.51 | 602.01 |
| SP174 | Ac-ETF$r8EHWAQL$AAIa-NH₂ | | 1818.97 | 910.76 | 1819.98 | 910.49 | 607.33 |
| SP175 | Ac-ETF$r8EHWAQL$AAIa-NH₂ | iso2 | 1818.97 | 910.85 | 1819.98 | 910.49 | 607.33 |
| SP176 | Ac-LTF$r8AHWVQL$AAIa-NH₂ | | 1773.03 | 888.09 | 1774.04 | 887.52 | 592.02 |
| SP177 | Ac-ITF$r8FYWVQL$AAIa-NH₂ | | 1875.07 | 939.16 | 1876.08 | 938.54 | 626.03 |
| SP178 | Ac-ITF$r8EYWVQL$AAIa-NH₂ | | 1857.04 | 929.83 | 1858.05 | 929.53 | 620.02 |
| SP179 | Ac-ITF$r8EHWVQL$AAIa-NH₂ | | 1831.04 | 916.86 | 1832.05 | 916.53 | 611.35 |
| SP180 | Ac-LTF$r8AEWAQL$AAIa-NH₂ | | 1736.99 | 869.87 | 1738 | 869.5 | 580 |
| SP181 | Ac-LTF$r8AF4coohWAQL$AAIa-NH₂ | | 1799 | 900.17 | 1800.01 | 900.51 | 600.67 |
| SP182 | Ac-LTF$r8AF4coohWAQL$AAIa-NH₂ | iso2 | 1799 | 900.24 | 1800.01 | 900.51 | 600.67 |
| SP183 | Ac-LTF$r8AHWAQL$AHFA-NH₂ | | 1845.01 | 923.89 | 1846.02 | 923.51 | 616.01 |
| SP184 | Ac-ITF$r8FYWAQL$AHFA-NH₂ | | 1947.05 | 975.05 | 1948.06 | 974.53 | 650.02 |
| SP185 | Ac-ITF$r8FYWAQL$AHFA-NH₂ | iso2 | 1947.05 | 976.07 | 1948.06 | 974.53 | 650.02 |
| SP186 | Ac-ITF$r8FHWAQL$AEFA-NH₂ | | 1913.02 | 958.12 | 1914.03 | 957.52 | 638.68 |
| SP187 | Ac-ITF$r8FHWAQL$AEFA-NH₂ | iso2 | 1913.02 | 957.86 | 1914.03 | 957.52 | 638.68 |
| SP188 | Ac-ITF$r8EHWAQL$AHFA-NH₂ | | 1903.01 | 952.94 | 1904.02 | 952.51 | 635.34 |
| SP189 | Ac-ITF$r8EHWAQL$AHFA-NH₂ | iso2 | 1903.01 | 953.87 | 1904.02 | 952.51 | 635.34 |
| SP190 | Ac-LTF$r8AHWVQL$AHFA-NH₂ | | 1873.04 | 937.86 | 1874.05 | 937.53 | 625.35 |
| SP191 | Ac-ITF$r8FYWVQL$AHFA-NH₂ | | 1975.08 | 988.83 | 1976.09 | 988.55 | 659.37 |
| SP192 | Ac-ITF$r8EYWVQL$AHFA-NH₂ | | 1957.05 | 979.35 | 1958.06 | 979.53 | 653.36 |
| SP193 | Ac-ITF$r8EHWVQL$AHFA-NH₂ | | 1931.05 | 967 | 1932.06 | 966.53 | 644.69 |
| SP194 | Ac-ITF$r8EHWVQL$AHFA-NH₂ | iso2 | 1931.05 | 967.93 | 1932.06 | 966.53 | 644.69 |
| SP195 | Ac-ETF$r8EYWAAL$SAA-NH₂ | | 1690.86 | 845.85 | 1691.87 | 846.44 | 564.63 |
| SP196 | Ac-LTF$r8AYWVAL$SAA-NH₂ | | 1644.93 | 824.08 | 1645.94 | 823.47 | 549.32 |
| SP197 | Ac-LTF$r8AHWAAL$SAA-NH₂ | | 1590.89 | 796.88 | 1591.9 | 796.45 | 531.3 |
| SP198 | Ac-LTF$r8AEWAAL$SAA-NH₂ | | 1582.88 | 791.9 | 1583.89 | 792.45 | 528.63 |
| SP199 | Ac-LTF$r8AEWAAL$SAA-NH₂ | iso2 | 1582.88 | 791.9 | 1583.89 | 792.45 | 528.63 |
| SP200 | Ac-LTF$r8ASWAAL$SAA-NH₂ | | 1540.87 | 770.74 | 1541.88 | 771.44 | 514.63 |
| SP201 | Ac-LTF$r8ASWAAL$SAA-NH₂ | iso2 | 1540.87 | 770.88 | 1541.88 | 771.44 | 514.63 |
| SP202 | Ac-LTF$r8AYWAAL$AAIa-NH₂ | | 1713.99 | 857.39 | 1715 | 858 | 572.34 |
| SP203 | Ac-LTF$r8AYWAAL$AAIa-NH₂ | iso2 | 1713.99 | 857.84 | 1715 | 858 | 572.34 |
| SP204 | Ac-LTF$r8AYWAAL$AHFA-NH₂ | | 1813.99 | 907.86 | 1815 | 908 | 605.67 |
| SP205 | Ac-LTF$r8EHWAQL$AHIa-NH₂ | | 1869.03 | 936.1 | 1870.04 | 935.52 | 624.02 |
| SP206 | Ac-LTF$r8EHWAQL$AHIa-NH₂ | iso2 | 1869.03 | 937.03 | 1870.04 | 935.52 | 624.02 |
| SP207 | Ac-LTF$r8AHWAQL$AHIa-NH₂ | | 1811.03 | 906.87 | 1812.04 | 906.52 | 604.68 |
| SP208 | Ac-LTF$r8EYWAQL$AHIa-NH₂ | | 1895.04 | 949.15 | 1896.05 | 948.53 | 632.69 |
| SP209 | Ac-LTF$r8AYWAQL$AAFa-NH₂ | | 1804.99 | 903.2 | 1806 | 903.5 | 602.67 |
| SP210 | Ac-LTF$r8AYWAQL$AAFa-NH₂ | iso2 | 1804.99 | 903.28 | 1806 | 903.5 | 602.67 |
| SP211 | Ac-LTF$r8AYWAQL$AAWa-NH₂ | | 1844 | 922.81 | 1845.01 | 923.01 | 615.67 |
| SP212 | Ac-LTF$r8AYWAQL$AAVa-NH₂ | | 1756.99 | 878.86 | 1758 | 879.5 | 586.67 |
| SP213 | Ac-LTF$r8AYWAQL$AAVa-NH₂ | iso2 | 1756.99 | 879.3 | 1758 | 879.5 | 586.67 |
| SP214 | Ac-LTF$r8AYWAQL$AALa-NH₂ | | 1771.01 | 886.26 | 1772.02 | 886.51 | 591.34 |
| SP215 | Ac-LTF$r8AYWAQL$AALa-NH₂ | iso2 | 1771.01 | 886.33 | 1772.02 | 886.51 | 591.34 |
| SP216 | Ac-LTF$r8EYWAQL$AAIa-NH₂ | | 1829.01 | 914.89 | 1830.02 | 915.51 | 610.68 |
| SP217 | Ac-LTF$r8EYWAQL$AAIa-NH₂ | iso2 | 1829.01 | 915.34 | 1830.02 | 915.51 | 610.68 |
| SP218 | Ac-LTF$r8EYWAQL$AAFa-NH₂ | | 1863 | 932.87 | 1864.01 | 932.51 | 622.01 |
| SP219 | Ac-LTF$r8EYWAQL$AAFa-NH₂ | iso2 | 1863 | 932.87 | 1864.01 | 932.51 | 622.01 |
| SP220 | Ac-LTF$r8EYWAQL$AAVa-NH₂ | | 1815 | 908.23 | 1816.01 | 908.51 | 606.01 |
| SP221 | Ac-LTF$r8EYWAQL$AAVa-NH₂ | iso2 | 1815 | 908.31 | 1816.01 | 908.51 | 606.01 |
| SP222 | Ac-LTF$r8EHWAQL$AAIa-NH₂ | | 1803.01 | 903.17 | 1804.02 | 902.51 | 602.01 |
| SP223 | Ac-LTF$r8EHWAQL$AAIa-NH₂ | iso2 | 1803.01 | 902.8 | 1804.02 | 902.51 | 602.01 |
| SP224 | Ac-LTF$r8EHWAQL$AAWa-NH₂ | | 1876 | 939.34 | 1877.01 | 939.01 | 626.34 |
| SP225 | Ac-LTF$r8EHWAQL$AAWa-NH₂ | iso2 | 1876 | 939.62 | 1877.01 | 939.01 | 626.34 |
| SP226 | Ac-LTF$r8EHWAQL$AALa-NH₂ | | 1803.01 | 902.8 | 1804.02 | 902.51 | 602.01 |
| SP227 | Ac-LTF$r8EHWAQL$AALa-NH₂ | iso2 | 1803.01 | 902.9 | 1804.02 | 902.51 | 602.01 |
| SP228 | Ac-ETF$r8EHWVQL$AALa-NH₂ | | 1847 | 924.82 | 1848.01 | 924.51 | 616.67 |
| SP229 | Ac-LTF$r8AYWAQL$AAAa-NH₂ | | 1728.96 | 865.89 | 1729.97 | 865.49 | 577.33 |
| SP230 | Ac-LTF$r8AYWAQL$AAAa-NH₂ | iso2 | 1728.96 | 865.89 | 1729.97 | 865.49 | 577.33 |
| SP231 | Ac-LTF$r8AYWAQL$AAAibA-NH₂ | | 1742.98 | 872.83 | 1743.99 | 872.5 | 582 |
| SP232 | Ac-LTF$r8AYWAQL$AAAibA-NH₂ | iso2 | 1742.98 | 872.92 | 1743.99 | 872.5 | 582 |
| SP233 | Ac-LTF$r8AYWAQL$AAAAa-NH₂ | | 1800 | 901.42 | 1801.01 | 901.01 | 601.01 |
| SP234 | Ac-LTF$r5AYWAQL$s8AAIa-NH₂ | | 1771.01 | 887.17 | 1772.02 | 886.51 | 591.34 |
| SP235 | Ac-LTF$r5AYWAQL$s8SAA-NH₂ | | 1673.92 | 838.33 | 1674.93 | 837.97 | 558.98 |
| SP236 | Ac-LTF$r8AYWAQCba$AANleA-NH₂ | | 1783.01 | 892.64 | 1784.02 | 892.51 | 595.34 |
| SP237 | Ac-ETF$r8AYWAQCba$AANleA-NH₂ | | 1798.97 | 900.59 | 1799.98 | 900.49 | 600.66 |
| SP238 | Ac-LTF$r8EYWAQCba$AANleA-NH₂ | | 1841.01 | 922.05 | 1842.02 | 921.51 | 614.68 |
| SP239 | Ac-LTF$r8AYWAQCba$AWNleA-NH₂ | | 1898.05 | 950.46 | 1899.06 | 950.03 | 633.69 |
| SP240 | Ac-ETF$r8AYWAQCba$AWNleA-NH₂ | | 1914.01 | 958.11 | 1915.02 | 958.01 | 639.01 |
| SP241 | Ac-LTF$r8EYWAQCba$AWNleA-NH₂ | | 1956.06 | 950.62 | 1957.07 | 979.04 | 653.03 |
| SP242 | Ac-LTF$r8EYWAQCba$SAFA-NH₂ | | 1890.99 | 946.55 | 1892 | 946.5 | 631.34 |
| SP243 | Ac-LTF34F2$r8EYWAQCba$SANleA-NH₂ | | 1892.99 | 947.57 | 1894 | 947.5 | 632 |
| SP244 | Ac-LTF$r8EF4coohWAQCba$SANleA-NH₂ | | 1885 | 943.59 | 1886.01 | 943.51 | 629.34 |
| SP245 | Ac-LTF$r8EYWSQCba$SANleA-NH₂ | | 1873 | 937.58 | 1874.01 | 937.51 | 625.34 |
| SP246 | Ac-LTF$r8EYWWQCba$SANleA-NH₂ | | 1972.05 | 987.61 | 1973.06 | 987.03 | 658.36 |
| SP247 | Ac-LTF$r8EYWAQCba$AAIa-NH₂ | | 1841.01 | 922.05 | 1842.02 | 921.51 | 614.68 |
| SP248 | Ac-LTF34F2$r8EYWAQCba$AAIa-NH₂ | | 1876.99 | 939.99 | 1878 | 939.5 | 626.67 |
| SP249 | Ac-LTF$r8EF4coohWAQCba$AAIa-NH₂ | | 1869.01 | 935.64 | 1870.02 | 935.51 | 624.01 |
| SP250 | Pam-ETF$r8EYWAQCba$SAA-NH₂ | | 1956.1 | 979.57 | 1957.11 | 979.06 | 653.04 |
| SP251 | Ac-LThF$r8EFWAQCba$SAA-NH₂ | | 1741.94 | 872.11 | 1742.95 | 871.98 | 581.65 |
| SP252 | Ac-LTA$r8EYWAQCba$SAA-NH₂ | | 1667.89 | 835.4 | 1668.9 | 834.95 | 556.97 |
| SP253 | Ac-LTF$r8EYAAQCba$SAA-NH₂ | | 1628.88 | 815.61 | 1629.89 | 815.45 | 543.97 |
| SP254 | Ac-LTF$r8EY2NalAQCba$SAA-NH₂ | | 1754.93 | 879.04 | 1755.94 | 878.47 | 585.98 |
| SP255 | Ac-LTF$r8AYWAQCba$SAA-NH₂ | | 1685.92 | 844.71 | 1686.93 | 843.97 | 562.98 |
| SP256 | Ac-LTF$r8EYWAQCba$SAF-NH₂ | | 1819.96 | 911.41 | 1820.97 | 910.99 | 607.66 |
| SP257 | Ac-LTF$r8EYWAQCba$SAFa-NH₂ | | 1890.99 | 947.41 | 1892 | 946.5 | 631.34 |
| SP258 | Ac-LTF$r8AYWAQCba$SAF-NH₂ | | 1761.95 | 882.73 | 1762.96 | 881.98 | 588.32 |
| SP259 | Ac-LTF34F2$r8AYWAQCba$SAF-NH₂ | | 1797.93 | 900.87 | 1798.94 | 899.97 | 600.32 |
| SP260 | Ac-LTF$r8AF4coohWAQCba$SAF-NH₂ | | 1789.94 | 896.43 | 1790.95 | 895.98 | 597.65 |
| SP261 | Ac-LTF$r8EY6clWAQCba$SAF-NH₂ | | 1853.92 | 929.27 | 1854.93 | 927.97 | 618.98 |
| SP262 | Ac-LTF$r8AYWSQCba$SAF-NH₂ | | 1777.94 | 890.87 | 1778.95 | 889.98 | 593.65 |
| SP263 | Ac-LTF$r8AYWWQCba$SAF-NH₂ | | 1876.99 | 939.91 | 1878 | 939.5 | 626.67 |
| SP264 | Ac-LTF$r8AYWAQCba$AAIa-NH₂ | | 1783.01 | 893.19 | 1784.02 | 892.51 | 595.34 |
| SP265 | Ac-LTF34F2$r8AYWAQCba$AAIa-NH₂ | | 1818.99 | 911.23 | 1820 | 910.5 | 607.34 |
| SP266 | Ac-LTF$r8AY6clWAQCba$AAIa-NH₂ | | 1816.97 | 909.84 | 1817.98 | 909.49 | 606.66 |
| SP267 | Ac-LTF$r8AF4coohWAQCba$AAIa-NH₂ | | 1811 | 906.88 | 1812.01 | 906.51 | 604.67 |
| SP268 | Ac-LTF$r8EYWAQCba$AAFa-NH₂ | | 1875 | 938.6 | 1876.01 | 938.51 | 626.01 |
| SP269 | Ac-LTF$r8EYWAQCba$AAFa-NH₂ | iso2 | 1875 | 938.6 | 1876.01 | 938.51 | 626.01 |
| SP270 | Ac-ETF$r8AYWAQCba$AWNlea-NH₂ | | 1914.01 | 958.42 | 1915.02 | 958.01 | 639.01 |
| SP271 | Ac-LTF$r8EYWAQCba$AWNlea-NH₂ | | 1956.06 | 979.42 | 1957.07 | 979.04 | 653.03 |
| SP272 | Ac-ETF$r8EYWAQCba$AWNlea-NH₂ | | 1972.01 | 987.06 | 1973.02 | 987.01 | 658.34 |
| SP273 | Ac-ETF$r8EYWAQCba$AWNlea-NH₂ | iso2 | 1972.01 | 987.06 | 1973.02 | 987.01 | 658.34 |
| SP274 | Ac-LTF$r8AYWAQCba$SAFa-NH₂ | | 1832.99 | 917.89 | 1834 | 917.5 | 612 |
| SP275 | Ac-LTF$r8AYWAQCba$SAFa-NH₂ | iso2 | 1832.99 | 918.07 | 1834 | 917.5 | 612 |
| SP276 | Ac-ETF$r8AYWAQL$AWNlea-NH₂ | | 1902.01 | 952.22 | 1903.02 | 952.01 | 635.01 |
| SP277 | Ac-LTF$r8EYWAQL$AWNlea-NH₂ | | 1944.06 | 973.5 | 1945.07 | 973.04 | 649.03 |
| SP278 | Ac-ETF$r8EYWAQL$AWNlea-NH₂ | | 1960.01 | 981.46 | 1961.02 | 981.01 | 654.34 |
| SP279 | Dmaac-LTF$r8EYWAQhL$SAA-NH₂ | | 1788.98 | 896.06 | 1789.99 | 895.5 | 597.33 |
| SP280 | Hexac-LTF$r8EYWAQhL$SAA-NH₂ | | 1802 | 902.9 | 1803.01 | 902.01 | 601.67 |
| SP281 | Napac-LTF$r8EYWAQhL$SAA-NH₂ | | 1871.99 | 937.58 | 1873 | 937 | 625 |
| SP282 | Decac-LTF$r8EYWAQhL$SAA-NH₂ | | 1858.06 | 930.55 | 1859.07 | 930.04 | 620.36 |
| SP283 | Admac-LTF$r8EYWAQhL$SAA-NH₂ | | 1866.03 | 934.07 | 1867.04 | 934.02 | 623.02 |
| SP284 | Tmac-LTF$r8EYWAQhL$SAA-NH₂ | | 1787.99 | 895.41 | 1789 | 895 | 597 |
| SP285 | Pam-LTF$r8EYWAQhL$SAA-NH₂ | | 1942.16 | 972.08 | 1943.17 | 972.09 | 648.39 |
| SP286 | Ac-LTF$r8AYWAQCba$AANleA-NH₂ | iso2 | 1783.01 | 892.64 | 1784.02 | 892.51 | 595.34 |
| SP287 | Ac-LTF34F2$r8EYWAQCba$AAIa-NH₂ | iso2 | 1876.99 | 939.62 | 1878 | 939.5 | 626.67 |
| SP288 | Ac-LTF34F2$r8EYWAQCba$SAA-NH₂ | | 1779.91 | 892.07 | 1780.92 | 890.96 | 594.31 |
| SP289 | Ac-LTF34F2$r8EYWAQCba$SAA-NH₂ | iso2 | 1779.91 | 891.61 | 1780.92 | 890.96 | 594.31 |
| SP290 | Ac-LTF$r8EF4coohWAQCba$SAA-NH₂ | | 1771.92 | 887.54 | 1772.93 | 886.97 | 591.65 |
| SP291 | Ac-LTF$r8EF4coohWAQCba$SAA-NH₂ | iso2 | 1771.92 | 887.63 | 1772.93 | 886.97 | 591.65 |
| SP292 | Ac-LTF$r8EYWSQCba$SAA-NH₂ | | 1759.92 | 881.9 | 1760.93 | 880.97 | 587.65 |
| SP293 | Ac-LTF$r8EYWSQCba$SAA-NH₂ | iso2 | 1759.92 | 881.9 | 1760.93 | 880.97 | 587.65 |
| SP294 | Ac-LTF$r8EYWAQhL$SAA-NH₂ | | 1745.94 | 875.05 | 1746.95 | 873.98 | 582.99 |
| SP295 | Ac-LTF$r8AYWAQhL$SAF-NH₂ | | 1763.97 | 884.02 | 1764.98 | 882.99 | 589 |
| SP296 | Ac-LTF$r8AYWAQhL$SAF-NH₂ | iso2 | 1763.97 | 883.56 | 1764.98 | 882.99 | 589 |
| SP297 | Ac-LTF34F2$r8AYWAQhL$SAA-NH₂ | | 1723.92 | 863.67 | 1724.93 | 862.97 | 575.65 |
| SP298 | Ac-LTF34F2$r8AYWAQhL$SAA-NH₂ | iso2 | 1723.92 | 864.04 | 1724.93 | 862.97 | 575.65 |
| SP299 | Ac-LTF$r8AF4coohWAQhL$SAA-NH₂ | | 1715.93 | 859.44 | 1716.94 | 858.97 | 572.98 |
| SP300 | Ac-LTF$r8AF4coohWAQhL$SAA-NH₂ | iso2 | 1715.93 | 859.6 | 1716.94 | 858.97 | 572.98 |
| SP301 | Ac-LTF$r8AYWSQhL$SAA-NH₂ | | 1703.93 | 853.96 | 1704.94 | 852.97 | 568.98 |
| SP302 | Ac-LTF$r8AYWSQhL$SAA-NH₂ | iso2 | 1703.93 | 853.59 | 1704.94 | 852.97 | 568.98 |
| SP303 | Ac-LTF$r8EYWAQL$AANleA-NH₂ | | 1829.01 | 915.45 | 1830.02 | 915.51 | 610.68 |
| SP304 | Ac-LTF34F2$r8AYWAQL$AANleA-NH₂ | | 1806.99 | 904.58 | 1808 | 904.5 | 603.34 |
| SP305 | Ac-LTF$r8AF4coohWAQL$AANleA-NH₂ | | 1799 | 901.6 | 1800.01 | 900.51 | 600.67 |
| SP306 | Ac-LTF$r8AYWSQL$AANleA-NH₂ | | 1787 | 894.75 | 1788.01 | 894.51 | 596.67 |
| SP307 | Ac-LTF34F2$r8AYWAQhL$AANleA-NH₂ | | 1821 | 911.79 | 1822.01 | 911.51 | 608.01 |
| SP308 | Ac-LTF34F2$r8AYWAQhL$AANleA-NH₂ | iso2 | 1821 | 912.61 | 1822.01 | 911.51 | 608.01 |
| SP309 | Ac-LTF$r8AF4coohWAQhL$AANleA-NH₂ | | 1813.02 | 907.95 | 1814.03 | 907.52 | 605.35 |
| SP310 | Ac-LTF$r8AF4coohWAQhL$AANleA-NH₂ | iso2 | 1813.02 | 908.54 | 1814.03 | 907.52 | 605.35 |
| SP311 | Ac-LTF$r8AYWSQhL$AANleA-NH₂ | | 1801.02 | 901.84 | 1802.03 | 901.52 | 601.35 |
| SP312 | Ac-LTF$r8AYWSQhL$AANleA-NH₂ | iso2 | 1801.02 | 902.62 | 1802.03 | 901.52 | 601.35 |
| SP313 | Ac-LTF$r8AYWAQhL$AAAAa-NH₂ | | 1814.01 | 908.63 | 1815.02 | 908.01 | 605.68 |
| SP314 | Ac-LTF$r8AYWAQhL$AAAAa-NH₂ | iso2 | 1814.01 | 908.34 | 1815.02 | 908.01 | 605.68 |
| SP315 | Ac-LTF$r8AYWAQL$AAAAAa-NH₂ | | 1871.04 | 936.94 | 1872.05 | 936.53 | 624.69 |
| SP316 | Ac-LTF$r8AYWAQL$AAAAAAa-NH₂ | iso2 | 1942.07 | 972.5 | 1943.08 | 972.04 | 648.37 |
| SP317 | Ac-LTF$r8AYWAQL$AAAAAAa-NH₂ | iso1 | 1942.07 | 972.5 | 1943.08 | 972.04 | 648.37 |
| SP318 | Ac-LTF$r8EYWAQhL$AANleA-NH₂ | | 1843.03 | 922.54 | 1844.04 | 922.52 | 615.35 |
| SP319 | Ac-AATF$r8AYWAQL$AANleA-NH₂ | | 1800 | 901.39 | 1801.01 | 901.01 | 601.01 |
| SP320 | Ac-LTF$r8AYWAQL$AANleAA-NH₂ | | 1842.04 | 922.45 | 1843.05 | 922.03 | 615.02 |
| SP321 | Ac-ALTF$r8AYWAQL$AANleAA-NH₂ | | 1913.08 | 957.94 | 1914.09 | 957.55 | 638.7 |
| SP322 | Ac-LTF$r8AYWAQCba$AANleAA-NH₂ | | 1854.04 | 928.43 | 1855.05 | 928.03 | 619.02 |
| SP323 | Ac-LTF$r8AYWAQhL$AANleAA-NH₂ | | 1856.06 | 929.4 | 1857.07 | 929.04 | 619.69 |
| SP324 | Ac-LTF$r8EYWAQCba$SAAA-NH₂ | | 1814.96 | 909.37 | 1815.97 | 908.49 | 605.99 |
| SP325 | Ac-LTF$r8EYWAQCba$SAAA-NH₂ | iso2 | 1814.96 | 909.37 | 1815.97 | 908.49 | 605.99 |
| SP326 | Ac-LTF$r8EYWAQCba$SAAAA-NH₂ | | 1886 | 944.61 | 1887.01 | 944.01 | 629.67 |
| SP327 | Ac-LTF$r8EYWAQCba$SAAAA-NH₂ | iso2 | 1886 | 944.61 | 1887.01 | 944.01 | 629.67 |
| SP328 | Ac-ALTF$r8EYWAQCba$SAA-NH₂ | | 1814.96 | 909.09 | 1815.97 | 908.49 | 605.99 |
| SP329 | Ac-ALTF$r8EYWAQCba$SAAA-NH₂ | | 1886 | 944.61 | 1887.01 | 944.01 | 629.67 |
| SP330 | Ac-ALTF$r8EYWAQCba$SAA-NH₂ | iso2 | 1814.96 | 909.09 | 1815.97 | 908.49 | 605.99 |
| SP331 | Ac-LTF$r8EYWAQL$AAAAAa-NH₂ | iso2 | 1929.04 | 966.08 | 1930.05 | 965.53 | 644.02 |
| SP332 | Ac-LTF$r8EY6clWAQCba$SAA-NH₂ | | 1777.89 | 890.78 | 1778.9 | 889.95 | 593.64 |
| SP333 | Ac-LTF$r8EF4cooh6clWAQCba$SANleA-NH₂ | | 1918.96 | 961.27 | 1919.97 | 960.49 | 640.66 |
| SP334 | Ac-LTF$r8EF4cooh6clWAQCba$SANleA-NH₂ | iso2 | 1918.96 | 961.27 | 1919.97 | 960.49 | 640.66 |
| SP335 | Ac-LTF$r8EF4cooh6clWAQCba$AAIa-NH₂ | | 1902.97 | 953.03 | 1903.98 | 952.49 | 635.33 |
| SP336 | Ac-LTF$r8EF4cooh6clWAQCba$AAIa-NH₂ | iso2 | 1902.97 | 953.13 | 1903.98 | 952.49 | 635.33 |
| SP337 | Ac-LTF$r8AY6clWAQL$AAAAAa-NH₂ | | 1905 | 954.61 | 1906.01 | 953.51 | 636.01 |
| SP338 | Ac-LTF$r8AY6clWAQL$AAAAAa-NH₂ | iso2 | 1905 | 954.9 | 1906.01 | 953.51 | 636.01 |
| SP339 | Ac-F$r8AY6clWEAL$AAAAAAa-NH₂ | | 1762.89 | 883.01 | 1763.9 | 882.45 | 588.64 |
| SP340 | Ac-ETF$r8EYWAQL$AAAAAa-NH₂ | | 1945 | 974.31 | 1946.01 | 973.51 | 649.34 |
| SP341 | Ac-ETF$r8EYWAQL$AAAAAa-NH₂ | iso2 | 1945 | 974.49 | 1946.01 | 973.51 | 649.34 |
| SP342 | Ac-LTF$r8EYWAQL$AAAAAAa-NH₂ | | 2000.08 | 1001.6 | 2001.09 | 1001.05 | 667.7 |
| SP343 | Ac-LTF$r8EYWAQL$AAAAAAa-NH₂ | iso2 | 2000.08 | 1001.6 | 2001.09 | 1001.05 | 667.7 |
| SP344 | Ac-LTF$r8AYWAQL$AANleAAa-NH₂ | | 1913.08 | 958.58 | 1914.09 | 957.55 | 638.7 |
| SP345 | Ac-LTF$r8AYWAQL$AANleAAa-NH₂ | iso2 | 1913.08 | 958.58 | 1914.09 | 957.55 | 638.7 |
| SP346 | Ac-LTF$r8EYWAQCba$AAAAAa-NH₂ | | 1941.04 | 972.55 | 1942.05 | 971.53 | 648.02 |
| SP347 | Ac-LTF$r8EYWAQCba$AAAAAa-NH₂ | iso2 | 1941.04 | 972.55 | 1942.05 | 971.53 | 648.02 |
| SP348 | Ac-LTF$r8EF4coohWAQCba$AAAAAa-NH₂ | | 1969.04 | 986.33 | 1970.05 | 985.53 | 657.35 |
| SP349 | Ac-LTF$r8EF4coohWAQCba$AAAAAa-NH₂ | iso2 | 1969.04 | 986.06 | 1970.05 | 985.53 | 657.35 |
| SP350 | Ac-LTF$r8EYWSQCba$AAAAAa-NH₂ | | 1957.04 | 980.04 | 1958.05 | 979.53 | 653.35 |
| SP351 | Ac-LTF$r8EYWSQCba$AAAAAa-NH₂ | iso2 | 1957.04 | 980.04 | 1958.05 | 979.53 | 653.35 |
| SP352 | Ac-LTF$r8EYWAQCba$SAAa-NH₂ | | 1814.96 | 909 | 1815.97 | 908.49 | 605.99 |
| SP353 | Ac-LTF$r8EYWAQCba$SAAa-NH₂ | iso2 | 1814.96 | 909 | 1815.97 | 908.49 | 605.99 |
| SP354 | Ac-ALTF$r8EYWAQCba$SAAa-NH₂ | | 1886 | 944.52 | 1887.01 | 944.01 | 629.67 |
| SP355 | Ac-ALTF$r8EYWAQCba$SAAa-NH₂ | iso2 | 1886 | 944.98 | 1887.01 | 944.01 | 629.67 |
| SP356 | Ac-ALTF$r8EYWAQCba$SAAAa-NH₂ | | 1957.04 | 980.04 | 1958.05 | 979.53 | 653.35 |
| SP357 | Ac-ALTF$r8EYWAQCba$SAAAa-NH₂ | iso2 | 1957.04 | 980.04 | 1958.05 | 979.53 | 653.35 |
| SP358 | Ac-AALTF$r8EYWAQCba$SAAAa-NH₂ | | 2028.07 | 1016.1 | 2029.08 | 1015.04 | 677.03 |
| SP359 | Ac-AALTF$r8EYWAQCba$SAAAa-NH₂ | iso2 | 2028.07 | 1015.57 | 2029.08 | 1015.04 | 677.03 |
| SP360 | Ac-RTF$r8EYWAQCba$SAA-NH₂ | | 1786.94 | 895.03 | 1787.95 | 894.48 | 596.65 |
| SP361 | Ac-LRF$r8EYWAQCba$SAA-NH₂ | | 1798.98 | 901.51 | 1799.99 | 900.5 | 600.67 |
| SP362 | Ac-LTF$r8EYWRQCba$SAA-NH₂ | | 1828.99 | 916.4 | 1830 | 915.5 | 610.67 |
| SP363 | Ac-LTF$r8EYWARCba$SAA-NH₂ | | 1771.97 | 887.63 | 1772.98 | 886.99 | 591.66 |
| SP364 | Ac-LTF$r8EYWAQCba$RAA-NH₂ | | 1812.99 | 908.08 | 1814 | 907.5 | 605.34 |
| SP365 | Ac-LTF$r8EYWAQCba$SRA-NH₂ | | 1828.99 | 916.12 | 1830 | 915.5 | 610.67 |
| SP366 | Ac-LTF$r8EYWAQCba$SAR-NH₂ | | 1828.99 | 916.12 | 1830 | 915.5 | 610.67 |
| SP367 | 5-FAM-BaLTF$r8EYWAQCba$SAA-NH₂ | | 2131 | 1067.09 | 2132.01 | 1066.51 | 711.34 |
| SP368 | 5-FAM-BaLTF$r8AYWAQL$AANleA-NH₂ | | 2158.08 | 1080.6 | 2159.09 | 1080.05 | 720.37 |
| SP369 | Ac-LAF$r8EYWAQL$AANleA-NH₂ | | 1799 | 901.05 | 1800.01 | 900.51 | 600.67 |
| SP370 | Ac-ATF$r8EYWAQL$AANleA-NH₂ | | 1786.97 | 895.03 | 1787.98 | 894.49 | 596.66 |
| SP371 | Ac-AAF$r8EYWAQL$AANleA-NH₂ | | 1756.96 | 880.05 | 1757.97 | 879.49 | 586.66 |
| SP372 | Ac-AAAF$r8EYWAQL$AANleA-NH₂ | | 1827.99 | 915.57 | 1829 | 915 | 610.34 |
| SP373 | Ac-AAAAF$r8EYWAQL$AANleA-NH₂ | | 1899.03 | 951.09 | 1900.04 | 950.52 | 634.02 |
| SP374 | Ac-AATF$r8EYWAQL$AANleA-NH₂ | | 1858 | 930.92 | 1859.01 | 930.01 | 620.34 |
| SP375 | Ac-AALTF$r8EYWAQL$AANleA-NH₂ | | 1971.09 | 987.17 | 1972.1 | 986.55 | 658.04 |
| SP376 | Ac-AAALTF$r8EYWAQL$AANleA-NH₂ | | 2042.12 | 1023.15 | 2043.13 | 1022.07 | 681.71 |
| SP377 | Ac-LTF$r8EYWAQL$AANleAA-NH₂ | | 1900.05 | 952.02 | 1901.06 | 951.03 | 634.36 |
| SP378 | Ac-ALTF$r8EYWAQL$AANleAA-NH₂ | | 1971.09 | 987.63 | 1972.1 | 986.55 | 658.04 |
| SP379 | Ac-AALTF$r8EYWAQL$AANleAA-NH₂ | | 2042.12 | 1022.69 | 2043.13 | 1022.07 | 681.71 |
| SP380 | Ac-LTF$r8EYWAQCba$AANleAA-NH₂ | | 1912.05 | 958.03 | 1913.06 | 957.03 | 638.36 |
| SP381 | Ac-LTF$r8EYWAQhL$AANleAA-NH₂ | | 1914.07 | 958.68 | 1915.08 | 958.04 | 639.03 |
| SP382 | Ac-ALTF$r8EYWAQhL$AANleAA-NH₂ | | 1985.1 | 994.1 | 1986.11 | 993.56 | 662.71 |
| SP383 | Ac-LTF$r8ANmYWAQL$AANleA-NH₂ | | 1785.02 | 894.11 | 1786.03 | 893.52 | 596.01 |
| SP384 | Ac-LTF$r8ANmYWAQL$AANleA-NH₂ | iso2 | 1785.02 | 894.11 | 1786.03 | 893.52 | 596.01 |
| SP385 | Ac-LTF$r8AYNmWAQL$AANleA-NH₂ | | 1785.02 | 894.11 | 1786.03 | 893.52 | 596.01 |
| SP386 | Ac-LTF$r8AYNmWAQL$AANleA-NH₂ | iso2 | 1785.02 | 894.11 | 1786.03 | 893.52 | 596.01 |
| SP387 | Ac-LTF$r8AYAmwAQL$AANleA-NH₂ | | 1785.02 | 894.01 | 1786.03 | 893.52 | 596.01 |
| SP388 | Ac-LTF$r8AYAmwAQL$AANleA-NH₂ | iso2 | 1785.02 | 894.01 | 1786.03 | 893.52 | 596.01 |
| SP389 | Ac-LTF$r8AYWAibQL$AANleA-NH₂ | | 1785.02 | 894.01 | 1786.03 | 893.52 | 596.01 |
| SP390 | Ac-LTF$r8AYWAibQL$AANleA-NH₂ | iso2 | 1785.02 | 894.01 | 1786.03 | 893.52 | 596.01 |
| SP391 | Ac-LTF$r8AYWAQL$AAibNleA-NH₂ | | 1785.02 | 894.38 | 1786.03 | 893.52 | 596.01 |
| SP392 | Ac-LTF$r8AYWAQL$AAibNleA-NH₂ | iso2 | 1785.02 | 894.38 | 1786.03 | 893.52 | 596.01 |
| SP393 | Ac-LTF$r8AYWAQL$AaNleA-NH₂ | | 1771.01 | 887.54 | 1772.02 | 886.51 | 591.34 |
| SP394 | Ac-LTF$r8AYWAQL$AaNleA-NH₂ | iso2 | 1771.01 | 887.54 | 1772.02 | 886.51 | 591.34 |
| SP395 | Ac-LTF$r8AYWAQL$ASarNleA-NH₂ | | 1771.01 | 887.35 | 1772.02 | 886.51 | 591.34 |
| SP396 | Ac-LTF$r8AYWAQL$ASarNleA-NH₂ | iso2 | 1771.01 | 887.35 | 1772.02 | 886.51 | 591.34 |
| SP397 | Ac-LTF$r8AYWAQL$AANleAib-NH₂ | | 1785.02 | 894.75 | 1786.03 | 893.52 | 596.01 |
| SP398 | Ac-LTF$r8AYWAQL$AANleAib-NH₂ | iso2 | 1785.02 | 894.75 | 1786.03 | 893.52 | 596.01 |
| SP399 | Ac-LTF$r8AYWAQL$AANleNmA-NH₂ | | 1785.02 | 894.6 | 1786.03 | 893.52 | 596.01 |
| SP400 | Ac-LTF$r8AYWAQL$AANleNmA-NH₂ | iso2 | 1785.02 | 894.6 | 1786.03 | 893.52 | 596.01 |
| SP401 | Ac-LTF$r8AYWAQL$AANleSar-NH₂ | | 1771.01 | 886.98 | 1772.02 | 886.51 | 591.34 |
| SP402 | Ac-LTF$r8AYWAQL$AANleSar-NH₂ | iso2 | 1771.01 | 886.98 | 1772.02 | 886.51 | 591.34 |
| SP403 | Ac-LTF$r8AYWAQL$AANleAAib-NH₂ | | 1856.06 | | 1857.07 | 929.04 | 619.69 |
| SP404 | Ac-LTF$r8AYWAQL$AANleAAib-NH₂ | iso2 | 1856.06 | | 1857.07 | 929.04 | 619.69 |
| SP405 | Ac-LTF$r8AYWAQL$AANleANmA-NH₂ | | 1856.06 | 930.37 | 1857.07 | 929.04 | 619.69 |
| SP406 | Ac-LTF$r8AYWAQL$AANleANmA-NH₂ | iso2 | 1856.06 | 930.37 | 1857.07 | 929.04 | 619.69 |
| SP407 | Ac-LTF$r8AYWAQL$AANleAa-NH₂ | | 1842.04 | 922.69 | 1843.05 | 922.03 | 615.02 |
| SP408 | Ac-LTF$r8AYWAQL$AANleAa-NH₂ | iso2 | 1842.04 | 922.69 | 1843.05 | 922.03 | 615.02 |
| SP409 | Ac-LTF$r8AYWAQL$AANleASar-NH₂ | | 1842.04 | 922.6 | 1843.05 | 922.03 | 615.02 |
| SP410 | Ac-LTF$r8AYWAQL$AANleASar-NH₂ | iso2 | 1842.04 | 922.6 | 1843.05 | 922.03 | 615.02 |
| SP411 | Ac-LTF$/r8AYWAQL$/AANleA-NH₂ | | 1799.04 | 901.14 | 1800.05 | 900.53 | 600.69 |
| SP412 | Ac-LTFAibAYWAQLAibAANleA-NH₂ | | 1648.9 | 826.02 | 1649.91 | 825.46 | 550.64 |
| SP413 | Ac-LTF$r8Cou4YWAQL$AANleA-NH₂ | | 1975.05 | 989.11 | 1976.06 | 988.53 | 659.36 |
| SP414 | Ac-LTF$r8Cou4YWAQL$AANleA-NH₂ | iso2 | 1975.05 | 989.11 | 1976.06 | 988.53 | 659.36 |
| SP415 | Ac-LTF$r8AYWCou4QL$AANleA-NH₂ | | 1975.05 | 989.11 | 1976.06 | 988.53 | 659.36 |
| SP416 | Ac-LTF$r8AYWAQL$Cou4ANleA-NH₂ | | 1975.05 | 989.57 | 1976.06 | 988.53 | 659.36 |
| SP417 | Ac-LTF$r8AYWAQL$Cou4ANleA-NH₂ | iso2 | 1975.05 | 989.57 | 1976.06 | 988.53 | 659.36 |
| SP418 | Ac-LTF$r8AYWAQL$ACou4NleA-NH₂ | | 1975.05 | 989.57 | 1976.06 | 988.53 | 659.36 |
| SP419 | Ac-LTF$r8AYWAQL$ACou4NleA-NH₂ | iso2 | 1975.05 | 989.57 | 1976.06 | 988.53 | 659.36 |
| SP420 | Ac-LTF$r8AYWAQL$AANleA-OH | | 1771.99 | 887.63 | 1773 | 887 | 591.67 |
| SP421 | Ac-LTF$r8AYWAQL$AANleA-OH | iso2 | 1771.99 | 887.63 | 1773 | 887 | 591.67 |
| SP422 | Ac-LTF$r8AYWAQL$AANleA-NHnPr | | 1813.05 | 908.08 | 1814.06 | 907.53 | 605.36 |
| SP423 | Ac-LTF$r8AYWAQL$AANleA-NHnPr | iso2 | 1813.05 | 908.08 | 1814.06 | 907.53 | 605.36 |
| SP424 | Ac-LTF$r8AYWAQL$AANleA-NHnBu33Me | | 1855.1 | 929.17 | 1856.11 | 928.56 | 619.37 |
| SP425 | Ac-LTF$r8AYWAQL$AANleA-NHnBu33Me | iso2 | 1855.1 | 929.17 | 1856.11 | 928.56 | 619.37 |
| SP426 | Ac-LTF$r8AYWAQL$AANleA-NHHex | | 1855.1 | 929.17 | 1856.11 | 928.56 | 619.37 |
| SP427 | Ac-LTF$r8AYWAQL$AANleA-NHHex | iso2 | 1855.1 | 929.17 | 1856.11 | 928.56 | 619.37 |
| SP428 | Ac-LTA$r8AYWAQL$AANleA-NH₂ | | 1694.98 | 849.33 | 1695.99 | 848.5 | 566 |
| SP429 | Ac-LThL$r8AYWAQL$AANleA-NH₂ | | 1751.04 | 877.09 | 1752.05 | 876.53 | 584.69 |
| SP430 | Ac-LTF$r8AYAAQL$AANleA-NH₂ | | 1655.97 | 829.54 | 1656.98 | 828.99 | 553 |
| SP431 | Ac-LTF$r8AY2NalAQL$AANleA-NH₂ | | 1782.01 | 892.63 | 1783.02 | 892.01 | 595.01 |
| SP432 | Ac-LTF$r8EYWCou4QCba$SAA-NH₂ | | 1947.97 | 975.8 | 1948.98 | 974.99 | 650.33 |
| SP433 | Ac-LTF$r8EYWCou7QCba$SAA-NH₂ | | 16.03 | 974.9 | 17.04 | 9.02 | 6.35 |
| SP434 | Ac-LTF%r8EYWAQCba%SAA-NH₂ | | 1745.94 | 874.8 | 1746.95 | 873.98 | 582.99 |
| SP435 | Dmaac-LTF$r8EYWAQCba$SAA-NH₂ | | 1786.97 | 894.8 | 1787.98 | 894.49 | 596.66 |
| SP436 | Dmaac-LTF$r8AYWAQL$AAAAAa-NH₂ | | 1914.08 | 958.2 | 1915.09 | 958.05 | 639.03 |
| SP437 | Dmaac-LTF$r8AYWAQL$AAAAAa-NH₂ | iso2 | 1914.08 | 958.2 | 1915.09 | 958.05 | 639.03 |
| SP438 | Dmaac-LTF$r8EYWAQL$AAAAAa-NH₂ | | 1972.08 | 987.3 | 1973.09 | 987.05 | 658.37 |
| SP439 | Dmaac-LTF$r8EYWAQL$AAAAAa-NH₂ | iso2 | 1972.08 | 987.3 | 1973.09 | 987.05 | 658.37 |
| SP440 | Dmaac-LTF$r8EF4coohWAQCba$AAIa-NH₂ | | 1912.05 | 957.4 | 1913.06 | 957.03 | 638.36 |
| SP441 | Dmaac-LTF$r8EF4coohWAQCba$AAIa-NH₂ | iso2 | 1912.05 | 957.4 | 1913.06 | 957.03 | 638.36 |
| SP442 | Dmaac-LTF$r8AYWAQL$AANleA-NH₂ | | 1814.05 | 908.3 | 1815.06 | 908.03 | 605.69 |
| SP443 | Dmaac-LTF$r8AYWAQL$AANleA-NH₂ | iso2 | 1814.05 | 908.3 | 1815.06 | 908.03 | 605.69 |
| SP444 | Ac-LTF%r8AYWAQL%AANleA-NH₂ | | 1773.02 | 888.37 | 1774.03 | 887.52 | 592.01 |
| SP445 | Ac-LTF%r8EYWAQL%AAAAAa-NH₂ | | 1931.06 | 966.4 | 1932.07 | 966.54 | 644.69 |
| SP446 | Cou6BaLTF$r8EYWAQhL$SAA-NH₂ | | 2018.05 | 1009.9 | 2019.06 | 1010.03 | 673.69 |
| SP447 | Cou8BaLTF$r8EYWAQhL$SAA-NH₂ | | 1962.96 | 982.34 | 1963.97 | 982.49 | 655.32 |
| SP448 | Ac-LTF4I$r8EYWAQL$AAAAAa-NH₂ | | 2054.93 | 1028.68 | 2055.94 | 1028.47 | 685.98 |
| SP449 | Ac-LTF$r8EYWAQL$AAAAAa-NH₂ | | 1929.04 | 966.17 | 1930.05 | 965.53 | 644.02 |
| SP550 | Ac-LTF$r8EYWAQL$AAAAAa-OH | | 1930.02 | 966.54 | 1931.03 | 966.02 | 644.35 |
| SP551 | Ac-LTF$r8EYWAQL$AAAAAa-OH | iso2 | 1930.02 | 965.89 | 1931.03 | 966.02 | 644.35 |
| SP552 | Ac-LTF$r8EYWAEL$AAAAAa-NH₂ | | 1930.02 | 966.82 | 1931.03 | 966.02 | 644.35 |
| SP553 | Ac-LTF$r8EYWAEL$AAAAAa-NH₂ | iso2 | 1930.02 | 966.91 | 1931.03 | 966.02 | 644.35 |
| SP554 | Ac-LTF$r8EYWAEL$AAAAAa-OH | | 1931.01 | 967.28 | 1932.02 | 966.51 | 644.68 |
| SP555 | Ac-LTF$r8EY6clWAQL$AAAAAa-NH₂ | | 1963 | 983.28 | 1964.01 | 982.51 | 655.34 |
| SP556 | Ac-LTF$r8EF4bOH2WAQL$AAAAAa-NH₂ | | 1957.05 | 980.04 | 1958.06 | 979.53 | 653.36 |
| SP557 | Ac-AAALTF$r8EYWAQL$AAAAAa-NH₂ | | 2142.15 | 1072.83 | 2143.16 | 1072.08 | 715.06 |
| SP558 | Ac-LTF34F2$r8EYWAQL$AAAAAa-NH₂ | | 1965.02 | 984.3 | 1966.03 | 983.52 | 656.01 |
| SP559 | Ac-RTF$r8EYWAQL$AAAAAa-NH₂ | | 1972.06 | 987.81 | 1973.07 | 987.04 | 658.36 |
| SP560 | Ac-LTA$r8EYWAQL$AAAAAa-NH₂ | | 1853.01 | 928.33 | 1854.02 | 927.51 | 618.68 |
| SP561 | Ac-LTF$r8EYWAibQL$AAAAAa-NH₂ | | 1943.06 | 973.48 | 1944.07 | 972.54 | 648.69 |
| SP562 | Ac-LTF$r8EYWAQL$AAibAAAa-NH₂ | | 1943.06 | 973.11 | 1944.07 | 972.54 | 648.69 |
| SP563 | Ac-LTF$r8EYWAQL$AAAibAAa-NH₂ | | 1943.06 | 973.48 | 1944.07 | 972.54 | 648.69 |
| SP564 | Ac-LTF$r8EYWAQL$AAAAibAa-NH₂ | | 1943.06 | 973.48 | 1944.07 | 972.54 | 648.69 |
| SP565 | Ac-LTF$r8EYWAQL$AAAAAiba-NH₂ | | 1943.06 | 973.38 | 1944.07 | 972.54 | 648.69 |
| SP566 | Ac-LTF$r8EYWAQL$AAAAAiba-NH₂ | iso2 | 1943.06 | 973.38 | 1944.07 | 972.54 | 648.69 |
| SP567 | Ac-LTF$r8EYWAQL$AAAAAAib-NH₂ | | 1943.06 | 973.01 | 1944.07 | 972.54 | 648.69 |
| SP568 | Ac-LTF$r8EYWAQL$AaAAAa-NH₂ | | 1929.04 | 966.54 | 1930.05 | 965.53 | 644.02 |
| SP569 | Ac-LTF$r8EYWAQL$AAaAAa-NH₂ | | 1929.04 | 966.35 | 1930.05 | 965.53 | 644.02 |
| SP570 | Ac-LTF$r8EYWAQL$AAAaAa-NH₂ | | 1929.04 | 966.54 | 1930.05 | 965.53 | 644.02 |
| SP571 | Ac-LTF$r8EYWAQL$AAAaAa-NH₂ | iso2 | 1929.04 | 966.35 | 1930.05 | 965.53 | 644.02 |
| SP572 | Ac-LTF$r8EYWAQL$AAAAaa-NH₂ | | 1929.04 | 966.35 | 1930.05 | 965.53 | 644.02 |
| SP573 | Ac-LTF$r8EYWAQL$AAAAAA-NH₂ | | 1929.04 | 966.35 | 1930.05 | 965.53 | 644.02 |
| SP574 | Ac-LTF$r8EYWAQL$ASarAAAa-NH₂ | | 1929.04 | 966.54 | 1930.05 | 965.53 | 644.02 |
| SP575 | Ac-LTF$r8EYWAQL$AASarAAa-NH₂ | | 1929.04 | 966.35 | 1930.05 | 965.53 | 644.02 |
| SP576 | Ac-LTF$r8EYWAQL$AAASarAa-NH₂ | | 1929.04 | 966.35 | 1930.05 | 965.53 | 644.02 |
| SP577 | Ac-LTF$r8EYWAQL$AAAASara-NH₂ | | 1929.04 | 966.35 | 1930.05 | 965.53 | 644.02 |
| SP578 | Ac-LTF$r8EYWAQL$AAAAASar-NH₂ | | 1929.04 | 966.08 | 1930.05 | 965.53 | 644.02 |
| SP579 | Ac-7LTF$r8EYWAQL$AAAAAa-NH₂ | | 1918.07 | 951.99 | 1919.08 | 960.04 | 640.37 |
| SP581 | Ac-TF$r8EYWAQL$AAAAAa-NH₂ | | 1815.96 | 929.85 | 1816.97 | 908.99 | 606.33 |
| SP582 | Ac-F$r8EYWAQL$AAAAAa-NH₂ | | 1714.91 | 930.92 | 1715.92 | 858.46 | 572.64 |
| SP583 | Ac-LVF$r8EYWAQL$AAAAAa-NH₂ | | 1927.06 | 895.12 | 1928.07 | 964.54 | 643.36 |
| SP584 | Ac-AAF$r8EYWAQL$AAAAAa-NH₂ | | 1856.98 | 859.51 | 1857.99 | 929.5 | 620 |
| SP585 | Ac-LTF$r8EYWAQL$AAAAa-NH₂ | | 1858 | 824.08 | 1859.01 | 930.01 | 620.34 |
| SP586 | Ac-LTF$r8EYWAQL$AAAa-NH₂ | | 1786.97 | 788.56 | 1787.98 | 894.49 | 596.66 |
| SP587 | Ac-LTF$r8EYWAQL$AAa-NH₂ | | 1715.93 | 1138.57 | 1716.94 | 858.97 | 572.98 |
| SP588 | Ac-LTF$r8EYWAQL$Aa-NH₂ | | 1644.89 | 1144.98 | 1645.9 | 823.45 | 549.3 |
| SP589 | Ac-LTF$r8EYWAQL$a-NH₂ | | 1573.85 | 1113.71 | 1574.86 | 787.93 | 525.62 |
| SP590 | Ac-LTF$r8EYWAQL$AAA-OH | | 1716.91 | 859.55 | 1717.92 | 859.46 | 573.31 |
| SP591 | Ac-LTF$r8EYWAQL$A-OH | | 1574.84 | 975.14 | 1575.85 | 788.43 | 525.95 |
| SP592 | Ac-LTF$r8EYWAQL$AAA-NH₂ | | 1715.93 | 904.75 | 1716.94 | 858.97 | 572.98 |
| SP593 | Ac-LTF$r8EYWAQCba$SAA-OH | | 1744.91 | 802.49 | 1745.92 | 873.46 | 582.64 |
| SP594 | Ac-LTF$r8EYWAQCba$S-OH | | 1602.83 | 913.53 | 1603.84 | 802.42 | 535.28 |
| SP595 | Ac-LTF$r8EYWAQCba$S-NH₂ | | 1601.85 | 979.58 | 1602.86 | 801.93 | 534.96 |
| SP596 | 4-FBzl-LTF$r8EYWAQL$AAAAAa-NH₂ | | 2009.05 | 970.52 | 2010.06 | 1005.53 | 670.69 |
| SP597 | 4-FBzl-LTF$r8EYWAQCba$SAA-NH₂ | | 1823.93 | 965.8 | 1824.94 | 912.97 | 608.98 |
| SP598 | Ac-LTF$r8RYWAQL$AAAAAa-NH₂ | | 1956.1 | 988.28 | 1957.11 | 979.06 | 653.04 |
| SP599 | Ac-LTF$r8HYWAQL$AAAAAa-NH₂ | | 1937.06 | 1003.54 | 1938.07 | 969.54 | 646.69 |
| SP600 | Ac-LTF$r8QYWAQL$AAAAAa-NH₂ | | 1928.06 | 993.92 | 1929.07 | 965.04 | 643.69 |
| SP601 | Ac-LTF$r8CitYWAQL$AAAAAa-NH₂ | | 1957.08 | 987 | 1958.09 | 979.55 | 653.37 |
| SP602 | Ac-LTF$r8GlaYWAQL$AAAAAa-NH₂ | | 1973.03 | 983 | 1974.04 | 987.52 | 658.68 |
| SP603 | Ac-LTF$r8F4gYWAQL$AAAAAa-NH₂ | | 2004.1 | 937.86 | 2005.11 | 1003.06 | 669.04 |
| SP604 | Ac-LTF$r82mRYWAQL$AAAAAa-NH₂ | | 1984.13 | 958.58 | 1985.14 | 993.07 | 662.38 |
| SP605 | Ac-LTF$r8ipKYWAQL$AAAAAa-NH₂ | | 1970.14 | 944.52 | 1971.15 | 986.08 | 657.72 |
| SP606 | Ac-LTF$r8F4NH₂YWAQL$AAAAAa-NH₂ | | 1962.08 | 946 | 1963.09 | 982.05 | 655.03 |
| SP607 | Ac-LTF$r8EYWAAL$AAAAAa-NH₂ | | 1872.02 | 959.32 | 1873.03 | 937.02 | 625.01 |
| SP608 | Ac-LTF$r8EYWALL$AAAAAa-NH₂ | | 1914.07 | 980.88 | 1915.08 | 958.04 | 639.03 |
| SP609 | Ac-LTF$r8EYWAAibL$AAAAAa-NH₂ | | 1886.03 | 970.61 | 1887.04 | 944.02 | 629.68 |
| SP610 | Ac-LTF$r8EYWASL$AAAAAa-NH₂ | | 1888.01 | 980.51 | 1889.02 | 945.01 | 630.34 |
| SP611 | Ac-LTF$r8EYWANL$AAAAAa-NH₂ | | 1915.02 | 1006.41 | 1916.03 | 958.52 | 639.35 |
| SP612 | Ac-LTF$r8EYWACitL$AAAAAa-NH₂ | | 1958.07 | | 1959.08 | 980.04 | 653.7 |
| SP613 | Ac-LTF$r8EYWAHL$AAAAAa-NH₂ | | 1938.04 | 966.24 | 1939.05 | 970.03 | 647.02 |
| SP614 | Ac-LTF$r8EYWARL$AAAAAa-NH₂ | | 1957.08 | | 1958.09 | 979.55 | 653.37 |
| SP615 | Ac-LTF$r8EpYWAQL$AAAAAa-NH₂ | | 2009.01 | | 2010.02 | 1005.51 | 670.68 |
| SP616 | Cbm-LTF$r8EYWAQCba$SAA-NH₂ | | 1590.85 | | 1591.86 | 796.43 | 531.29 |
| SP617 | Cbm-LTF$r8EYWAQL$AAAAAa-NH₂ | | 1930.04 | | 1931.05 | 966.03 | 644.35 |
| SP618 | Ac-LTF$r8EYWAQL$SAAAAa-NH₂ | | 1945.04 | 1005.11 | 1946.05 | 973.53 | 649.35 |
| SP619 | Ac-LTF$r8EYWAQL$AAAASa-NH₂ | | 1945.04 | 986.52 | 1946.05 | 973.53 | 649.35 |
| SP620 | Ac-LTF$r8EYWAQL$SAAASa-NH₂ | | 1961.03 | 993.27 | 1962.04 | 981.52 | 654.68 |
| SP621 | Ac-LTF$r8EYWAQTba$AAAAAa-NH₂ | | 1943.06 | 983.1 | 1944.07 | 972.54 | 648.69 |
| SP622 | Ac-LTF$r8EYWAQAdm$AAAAAa-NH₂ | | 2007.09 | 990.31 | 2008.1 | 1004.55 | 670.04 |
| SP623 | Ac-LTF$r8EYWAQCha$AAAAAa-NH₂ | | 1969.07 | 987.17 | 1970.08 | 985.54 | 657.36 |
| SP624 | Ac-LTF$r8EYWAQhCha$AAAAAa-NH₂ | | 1983.09 | 1026.11 | 1984.1 | 992.55 | 662.04 |
| SP625 | Ac-LTF$r8EYWAQF$AAAAAa-NH₂ | | 1963.02 | 957.01 | 1964.03 | 982.52 | 655.35 |
| SP626 | Ac-LTF$r8EYWAQhF$AAAAAa-NH₂ | | 1977.04 | 1087.81 | 1978.05 | 989.53 | 660.02 |
| SP627 | Ac-LTF$r8EYWAQL$AANleAAa-NH₂ | | 1971.09 | 933.45 | 1972.1 | 986.55 | 658.04 |
| SP628 | Ac-LTF$r8EYWAQAdm$AANleAAa-NH₂ | | 2049.13 | 1017.97 | 2050.14 | 1025.57 | 684.05 |
| SP629 | 4-FBz-BaLTF$r8EYWAQL$AAAAAa-NH₂ | | 2080.08 | | 2081.09 | 1041.05 | 694.37 |
| SP630 | 4-FBz-BaLTF$r8EYWAQCba$SAA-NH₂ | | 1894.97 | | 1895.98 | 948.49 | 632.66 |
| SP631 | Ac-LTF$r5EYWAQL$s8AAAAAa-NH₂ | | 1929.04 | 1072.68 | 1930.05 | 965.53 | 644.02 |
| SP632 | Ac-LTF$r5EYWAQCba$s8SAA-NH₂ | | 1743.92 | 1107.79 | 1744.93 | 872.97 | 582.31 |
| SP633 | Ac-LTF$r8EYWAQL$AAhhLAAa-NH₂ | | 1999.12 | | 2000.13 | 1000.57 | 667.38 |
| SP634 | Ac-LTF$r8EYWAQL$AAAAAAAa-NH₂ | | 2071.11 | | 2072.12 | 1036.56 | 691.38 |
| SP635 | Ac-LTF$r8EYWAQL$AAAAAAAAa-NH₂ | | 2142.15 | 778.1 | 2143.16 | 1072.08 | 715.06 |
| SP636 | Ac-LTF$r8EYWAQL$AAAAAAAAAa-NH₂ | | 2213.19 | 870.53 | 2214.2 | 1107.6 | 738.74 |
| SP637 | Ac-LTA$r8EYAAQCba$SAA-NH₂ | | 1552.85 | | 1553.86 | 777.43 | 518.62 |
| SP638 | Ac-LTA$r8EYAAQL$AAAAAa-NH₂ | | 1737.97 | 779.45 | 1738.98 | 869.99 | 580.33 |
| SP639 | Ac-LTF$r8EPmpWAQL$AAAAAa-NH₂ | | 2007.03 | 779.54 | 2008.04 | 1004.52 | 670.02 |
| SP640 | Ac-LTF$r8EPmpWAQCba$SAA-NH₂ | | 1821.91 | 838.04 | 1822.92 | 911.96 | 608.31 |
| SP641 | Ac-ATF$r8HYWAQL$S-NH₂ | | 1555.82 | 867.83 | 1556.83 | 778.92 | 519.61 |
| SP642 | Ac-LTF$r8HAWAQL$S-NH₂ | | 1505.84 | 877.91 | 1506.85 | 753.93 | 502.95 |
| SP643 | Ac-LTF$r8HYWAQA$S-NH₂ | | 1555.82 | 852.52 | 1556.83 | 778.92 | 519.61 |
| SP644 | Ac-LTF$r8EYWAQCba$SA-NH₂ | | 1672.89 | 887.18 | 1673.9 | 837.45 | 558.64 |
| SP645 | Ac-LTF$r8EYWAQL$SAA-NH₂ | | 1731.92 | 873.32 | 1732.93 | 866.97 | 578.31 |
| SP646 | Ac-LTF$r8HYWAQCba$SAA-NH₂ | | 1751.94 | 873.05 | 1752.95 | 876.98 | 584.99 |
| SP647 | Ac-LTF$r8SYWAQCba$SAA-NH₂ | | 1701.91 | 844.88 | 1702.92 | 851.96 | 568.31 |
| SP648 | Ac-LTF$r8RYWAQCba$SAA-NH₂ | | 1770.98 | 865.58 | 1771.99 | 886.5 | 591.33 |
| SP649 | Ac-LTF$r8KYWAQCba$SAA-NH₂ | | 1742.98 | 936.57 | 1743.99 | 872.5 | 582 |
| SP650 | Ac-LTF$r8QYWAQCba$SAA-NH₂ | | 1742.94 | 930.93 | 1743.95 | 872.48 | 581.99 |
| SP651 | Ac-LTF$r8EYWAACba$SAA-NH₂ | | 1686.9 | 1032.45 | 1687.91 | 844.46 | 563.31 |
| SP652 | Ac-LTF$r8EYWAQCba$AAA-NH₂ | | 1727.93 | 895.46 | 1728.94 | 864.97 | 576.98 |
| SP653 | Ac-LTF$r8EYWAQL$AAAAA-OH | | 1858.99 | 824.54 | 1860 | 930.5 | 620.67 |
| SP654 | Ac-LTF$r8EYWAQL$AAAA-OH | | 1787.95 | 894.48 | 1788.96 | 894.98 | 596.99 |
| SP655 | Ac-LTF$r8EYWAQL$AA-OH | | 1645.88 | 856 | 1646.89 | 823.95 | 549.63 |
| SP656 | Ac-LTF$r8AF4bOH2WAQL$AAAAAa-NH₂ | | | | | | |
| SP657 | Ac-LTF$r8AF4bOH2WAAL$AAAAAa-NH₂ | | | | | | |
| SP658 | Ac-LTF$r8EF4bOH2WAQCba$SAA-NH₂ | | | | | | |
| SP659 | Ac-LTF$r8ApYWAQL$AAAAAa-NH₂ | | | | | | |
| SP660 | Ac-LTF$r8ApYWAAL$AAAAAa-NH₂ | | | | | | |
| SP661 | Ac-LTF$r8EpYWAQCba$SAA-NH₂ | | | | | | |
| SP662 | Ac-LTF$rda6AYWAQL$da5AAAAAa-NH₂ | | 1974.06 | 934.44 | | | |
| SP663 | Ac-LTF$rda6EYWAQCba$da5SAA-NH₂ | | 1846.95 | 870.52 | | 869.94 | |
| SP664 | Ac-LTF$rda6EYWAQL$da5AAAAAa-NH₂ | | | | | | |
| SP665 | Ac-LTF$ra9EYWAQL$a6AAAAAa-NH₂ | | | 936.57 | | 935.51 | |
| SP666 | Ac-LTF$ra9EYWAQL$a6AAAAAa-NH₂ | | | | | | |
| SP667 | Ac-LTF$ra9EYWAQCba$a6SAA-NH₂ | | | | | | |
| SP668 | Ac-LTA$ra9EYWAQCba$a6SAA-NH₂ | | | | | | |
| SP669 | 5-FAM-BaLTF$ra9EYWAQCba$a6SAA-NH₂ | | | | | | |
| SP670 | 5-FAM-BaLTF$r8EYWAQL$AAAAAa-NH₂ | | 2316.11 | | | | |
| SP671 | 5-FAM-BaLTF$/r8EYWAQL$/AAAAAa-NH₂ | | 2344.15 | | | | |
| SP672 | 5-FAM-BaLTA$r8EYWAQL$AAAAAa-NH₂ | | 2240.08 | | | | |
| SP673 | 5-FAM-BaLTF$r8AYWAQL$AAAAAa-NH₂ | | 2258.11 | | | | |
| SP674 | 5-FAM-BaATF$r8EYWAQL$AAAAAa-NH₂ | | 2274.07 | | | | |
| SP675 | 5-FAM-BaLAF$r8EYWAQL$AAAAAa-NH₂ | | 2286.1 | | | | |
| SP676 | 5-FAM-BaLTF$r8EAWAQL$AAAAAa-NH₂ | | 2224.09 | | | | |
| SP677 | 5-FAM-BaLTF$r8EYAAQL$AAAAAa-NH₂ | | 2201.07 | | | | |
| SP678 | 5-FAM-BaLTA$r8EYAAQL$AAAAAa-NH₂ | | 2125.04 | | | | |
| SP679 | 5-FAM-BaLTF$r8EYWAAL$AAAAAa-NH₂ | | 2259.09 | | | | |
| SP680 | 5-FAM-BaLTF$r8EYWAQA$AAAAAa-NH₂ | | 2274.07 | | | | |
| SP681 | 5-FAM-BaLTF$/r8EYWAQCba$/SAA-NH₂ | | 2159.03 | | | | |
| SP682 | 5-FAM-BaLTA$r8EYWAQCba$SAA-NH₂ | | 2054.97 | | | | |
| SP683 | 5-FAM-BaLTF$r8EYAAQCba$SAA-NH₂ | | 2015.96 | | | | |
| SP684 | 5-FAM-BaLTA$r8EYAAQCba$SAA-NH₂ | | 1939.92 | | | | |
| SP685 | 5-FAM-BaQSQQTF$r8NLWRLL$QN-NH₂ | | 2495.23 | | | | |
| SP686 | 5-TAMRA-BaLTF$r8EYWAQCba$SAA-NH₂ | | 2186.1 | | | | |
| SP687 | 5-TAMRA-BaLTA$r8EYWAQCba$SAA-NH₂ | | 2110.07 | | | | |
| SP688 | 5-TAMRA-BaLTF$r8EYAAQCba$SAA-NH₂ | | 2071.06 | | | | |
| SP689 | 5-TAMRA-BaLTA$r8EYAAQCba$SAA-NH₂ | | 1995.03 | | | | |
| SP690 | 5-TAMRA-BaLTF$/r8EYWAQCba$/SAA-NH₂ | | 2214.13 | | | | |
| SP691 | 5-TAMRA-BaLTF$r8EYWAQL$AAAAAa-NH₂ | | 2371.22 | | | | |
| SP692 | 5-TAMRA-BaLTA$r8EYWAQL$AAAAAa-NH₂ | | 2295.19 | | | | |
| SP693 | 5-TAMRA-BaLTF$/r8EYWAQL$/AAAAAa-NH₂ | | 2399.25 | | | | |
| SP694 | Ac-LTF$r8EYWCou7QCba$SAA-OH | | 1947.93 | | | | |
| SP695 | Ac-LTF$r8EYWCou7QCba$S-OH | | 1805.86 | | | | |
| SP696 | Ac-LTA$r8EYWCou7QCba$SAA-NH₂ | | 1870.91 | | | | |
| SP697 | Ac-LTF$r8EYACou7QCba$SAA-NH₂ | | 1831.9 | | | | |
| SP698 | Ac-LTA$r8EYACou7QCba$SAA-NH₂ | | 1755.87 | | | | |
| SP699 | Ac-LTF$/r8EYWCou7QCba$/SAA-NH₂ | | 1974.98 | | | | |
| SP700 | Ac-LTF$r8EYWCou7QL$AAAAAa-NH₂ | | 2132.06 | | | | |
| SP701 | Ac-LTF$/r8EYWCou7QL$/AAAAAa-NH₂ | | 2160.09 | | | | |
| SP702 | Ac-LTF$r8EYWCou7QL$AAAAA-OH | | 2062.01 | | | | |
| SP703 | Ac-LTF$r8EYWCou7QL$AAAA-OH | | 1990.97 | | | | |
| SP704 | Ac-LTF$r8EYWCou7QL$AAA-OH | | 1919.94 | | | | |
| SP705 | Ac-LTF$r8EYWCou7QL$AA-OH | | 1848.9 | | | | |
| SP706 | Ac-LTF$r8EYWCou7QL$A-OH | | 1777.86 | | | | |
| SP707 | Ac-LTF$r8EYWAQL$AAAASa-NH₂ | iso2 | | 974.4 | | 973.53 | |
| SP708 | Ac-LTF$r8AYWAAL$AAAAAa-NH₂ | iso2 | 1814.01 | 908.82 | 1815.02 | 908.01 | 605.68 |
| SP709 | Biotin-BaLTF$r8EYWAQL$AAAAAa-NH₂ | | 2184.14 | 1093.64 | 2185.15 | 1093.08 | 729.05 |
| SP710 | Ac-LTF$r8HAWAQL$S-NH₂ | iso2 | 1505.84 | 754.43 | 1506.85 | 753.93 | 502.95 |
| SP711 | Ac-LTF$r8EYWAQCba$SA-NH₂ | iso2 | 1672.89 | 838.05 | 1673.9 | 837.45 | 558.64 |
| SP712 | Ac-LTF$r8HYWAQCba$SAA-NH₂ | iso2 | 1751.94 | 877.55 | 1752.95 | 876.98 | 584.99 |
| SP713 | Ac-LTF$r8SYWAQCba$SAA-NH₂ | iso2 | 1701.91 | 852.48 | 1702.92 | 851.96 | 568.31 |
| SP714 | Ac-LTF$r8RYWAQCba$SAA-NH₂ | iso2 | 1770.98 | 887.45 | 1771.99 | 886.5 | 591.33 |
| SP715 | Ac-LTF$r8KYWAQCba$SAA-NH₂ | iso2 | 1742.98 | 872.92 | 1743.99 | 872.5 | 582 |
| SP716 | Ac-LTF$r8EYWAQCba$AAA-NH₂ | iso2 | 1727.93 | 865.71 | 1728.94 | 864.97 | 576.98 |
| SP717 | Ac-LTF$r8EYWAQL$AAAAAaBaC-NH₂ | | 2103.09 | 1053.12 | 2104.1 | 1052.55 | 702.04 |
| SP718 | Ac-LTF$r8EYWAQL$AAAAAadPeg4C-NH₂ | | 2279.19 | 1141.46 | 2280.2 | 1140.6 | 760.74 |
| SP719 | Ac-LTA$r8AYWAAL$AAAAAa-NH₂ | | 1737.98 | 870.43 | 1738.99 | 870 | 580.33 |
| SP720 | Ac-LTF$r8AYAAAL$AAAAAa-NH₂ | | 1698.97 | 851 | 1699.98 | 850.49 | 567.33 |
| SP721 | 5-FAM-BaLTF$r8AYWAAL$AAAAAa-NH₂ | | 2201.09 | 1101.87 | 2202.1 | 1101.55 | 734.7 |
| SP722 | Ac-LTA$r8AYWAQL$AAAAAa-NH₂ | | 1795 | 898.92 | 1796.01 | 898.51 | 599.34 |
| SP723 | Ac-LTF$r8AYAAQL$AAAAAa-NH₂ | | 1755.99 | 879.49 | 1757 | 879 | 586.34 |
| SP724 | Ac-LTF$rda6AYWAAL$da5AAAAAa-NH₂ | | 1807.97 | | 1808.98 | 904.99 | 603.66 |
| SP725 | FITC-BaLTF$r8EYWAQL$AAAAAa-NH₂ | | 2347.1 | 1174.49 | 2348.11 | 1174.56 | 783.37 |
| SP726 | FITC-BaLTF$r8EYWAQCba$SAA-NH₂ | | 2161.99 | 1082.35 | 2163 | 1082 | 721.67 |
| SP733 | Ac-LTF$r8EYWAQL$EAAAAa-NH₂ | | 1987.05 | 995.03 | 1988.06 | 994.53 | 663.36 |
| SP734 | Ac-LTF$r8AYWAQL$EAAAAa-NH₂ | | 1929.04 | 966.35 | 1930.05 | 965.53 | 644.02 |
| SP735 | Ac-LTF$r8EYWAQL$AAAAAaBaKbio-NH₂ | | 2354.25 | 1178.47 | 2355.26 | 1178.13 | 785.76 |
| SP736 | Ac-LTF$r8AYWAAL$AAAAAa-NH₂ | | 1814.01 | 908.45 | 1815.02 | 908.01 | 605.68 |
| SP737 | Ac-LTF$r8AYAAAL$AAAAAa-NH₂ | iso2 | 1698.97 | 850.91 | 1699.98 | 850.49 | 567.33 |
| SP738 | Ac-LTF$r8AYAAQL$AAAAAa-NH₂ | iso2 | 1755.99 | 879.4 | 1757 | 879 | 586.34 |
| SP739 | Ac-LTF$r8EYWAQL$EAAAAa-NH₂ | iso2 | 1987.05 | 995.21 | 1988.06 | 994.53 | 663.36 |
| SP740 | Ac-LTF$r8AYWAQL$EAAAAa-NH₂ | iso2 | 1929.04 | 966.08 | 1930.05 | 965.53 | 644.02 |
| SP741 | Ac-LTF$r8EYWAQCba$SAAAAa-NH₂ | | 1957.04 | 980.04 | 1958.05 | 979.53 | 653.35 |
| SP742 | Ac-LTF$r8EYWAQLStAAA$r5AA-NH₂ | | 2023.12 | 1012.83 | 2024.13 | 1012.57 | 675.38 |
| SP743 | Ac-LTF$r8EYWAQL$A$AAA$A-NH₂ | | 2108.17 | 1055.44 | 2109.18 | 1055.09 | 703.73 |
| SP744 | Ac-LTF$r8EYWAQL$AA$AAA$A-NH₂ | | 2179.21 | 1090.77 | 2180.22 | 1090.61 | 727.41 |
| SP745 | Ac-LTF$r8EYWAQL$AAA$AAA$A-NH₂ | | 2250.25 | 1126.69 | 2251.26 | 1126.13 | 751.09 |
| SP746 | Ac-AAALTF$r8EYWAQL$AAA-OH | | 1930.02 | | 1931.03 | 966.02 | 644.35 |
| SP747 | Ac-AAALTF$r8EYWAQL$AAA-NH₂ | | 1929.04 | 965.85 | 1930.05 | 965.53 | 644.02 |
| SP748 | Ac-AAAALTF$r8EYWAQL$AAA-NH₂ | | 2000.08 | 1001.4 | 2001.09 | 1001.05 | 667.7 |
| SP749 | Ac-AAAAALTF$r8EYWAQL$AAA-NH₂ | | 2071.11 | 1037.13 | 2072.12 | 1036.56 | 691.38 |
| SP750 | Ac-AAAAAALTF$r8EYWAQL$AAA-NH₂ | | 2142.15 | | 2143.16 | 1072.08 | 715.06 |
| SP751 | Ac-LTF$rda6EYWAQCba$da6SAA-NH₂ | iso2 | 1751.89 | 877.36 | 1752.9 | 876.95 | 584.97 |
| SP752 | Ac-t$r5wya$r5f4CF3ekllr-NH₂ | | | 844.25 | | | |
| SP753 | Ac-tawy$r5nf4CF3e$r5llr-NH₂ | | | 837.03 | | | |
| SP754 | Ac-tawya$r5f4CF3ek$r51r-NH₂ | | | 822.97 | | | |
| SP755 | Ac-tawyanf4CF3e$r5llr$r5a-NH₂ | | | 908.35 | | | |
| SP756 | Ac-t$s8wyanf4CF3e$r5llr-NH₂ | | | 858.03 | | | |
| SP757 | Ac-tawy$s8nf4CF3ekll$r5a-NH₂ | | | 879.86 | | | |
| SP758 | Ac-tawya$s8f4CF3ekllr$r5a-NH₂ | | | 936.38 | | | |
| SP759 | Ac-tawy$s8naekll$r5a-NH₂ | | | 844.25 | | | |
| SP760 | 5-FAM-Batawy$s8nf4CF3ekll$r5a-NH₂ | | | | | | |
| SP761 | 5-FAM-Batawy$s8naekll$r5a-NH₂ | | | | | | |
| SP762 | Ac-tawy$s8nf4CF3eall$r5a-NH₂ | | | | | | |
| SP763 | Ac-tawy$s8nf4CF3ekll$r5aaaaa-NH₂ | | | | | | |
| SP764 | Ac-tawy$s8nf4CF3eall$r5aaaaa-NH₂ | | | | | | |

Table 1a shows a selection of peptidomimetic macrocycles.

**Table 1a**

| **SP** | **Sequence** | **Isomer** | **Exact Mass** | **Found Mass** | **Calc (M+1)/1** | **Calc (M+2)/2** | **Calc (M+3)/3** |
|---|---|---|---|---|---|---|---|
| SP244 | Ac-LTF$r8EF4coohWAQCba$SANleA-NH₂ | | 1885 | 943.59 | 1886.01 | 943.51 | 629.34 |
| SP331 | Ac-LTF$r8EYWAQL$AAAAAa-NH₂ | iso2 | 1929.04 | 966.08 | 1930.05 | 965.53 | 644.02 |
| SP555 | Ac-LTF$r8EY6clWAQL$AAAAAa-NH₂ | | 1963 | 983.28 | 1964.01 | 982.51 | 655.34 |
| SP557 | Ac-AAALTF$r8EYWAQL$AAAAAa-NH₂ | | 2142.15 | 1072.83 | 2143.16 | 1072.08 | 715.06 |
| SP558 | Ac-LTF34F2$r8EYWAQL$AAAAAa-NH₂ | | 1965.02 | 984.3 | 1966.03 | 983.52 | 656.01 |
| SP562 | Ac-LTF$r8EYWAQL$AAibAAAa-NH₂ | | 1943.06 | 973.11 | 1944.07 | 972.54 | 648.69 |
| SP564 | Ac-LTF$r8EYWAQL$AAAAibAa-NH₂ | | 1943.06 | 973.48 | 1944.07 | 972.54 | 648.69 |
| SP566 | Ac-LTF$r8EYWAQL$AAAAAiba-NH₂ | iso2 | 1943.06 | 973.38 | 1944.07 | 972.54 | 648.69 |
| SP567 | Ac-LTF$r8EYWAQL$AAAAAAib-NH₂ | | 1943.06 | 973.01 | 1944.07 | 972.54 | 648.69 |
| SP572 | Ac-LTF$r8EYWAQL$AAAAaa-NH₂ | | 1929.04 | 966.35 | 1930.05 | 965.53 | 644.02 |
| SP573 | Ac-LTF$r8EYWAQL$AAAAAA-NH₂ | | 1929.04 | 966.35 | 1930.05 | 965.53 | 644.02 |
| SP578 | Ac-LTF$r8EYWAQL$AAAAASar-NH₂ | | 1929.04 | 966.08 | 1930.05 | 965.53 | 644.02 |
| SP551 | Ac-LTF$r8EYWAQL$AAAAAa-OH | iso2 | 1930.02 | 965.89 | 1931.03 | 966.02 | 644.35 |
| SP662 | Ac-LTF$rda6AYWAQL$da5AAAAAa-NH₂ | | 1974.06 | 934.44 | | 933.49 | |
| SP367 | 5-FAM-BaLTF$r8EYWAQCba$SAA-NH₂ | | 2131 | 1067.09 | 2132.01 | 1066.51 | 711.34 |
| SP349 | Ac-LTF$r8EF4coohWAQCba$AAAAAa-NH₂ | iso2 | 1969.04 | 986.06 | 1970.05 | 985.53 | 657.35 |
| SP347 | Ac-LTF$r8EYWAQCba$AAAAAa-NH₂ | iso2 | 1941.04 | 972.55 | 1942.05 | 971.53 | 648.02 |

Table 1b shows a further selection of peptidomimetic macrocycles.

**Table 1b**

| **SP** | **Sequence** | **Isomer** | **Exact Mass** | **Found Mass** | **Calc (M+1)/1** | **Calc (M+2)/2** | **Calc (M+3)/3** |
|---|---|---|---|---|---|---|---|
| SP581 | Ac-TF$r8EYWAQL$AAAAAa-NH₂ | | 1815.96 | 929.85 | 1816.97 | 908.99 | 606.33 |
| SP582 | Ac-F$r8EYWAQL$AAAAAa-NH₂ | | 1714.91 | 930.92 | 1715.92 | 858.46 | 572.64 |
| SP583 | Ac-LVF$r8EYWAQL$AAAAAa-NH₂ | | 1927.06 | 895.12 | 1928.07 | 964.54 | 643.36 |
| SP584 | Ac-AAF$r8EYWAQL$AAAAAa-NH₂ | | 1856.98 | 859.51 | 1857.99 | 929.5 | 620 |
| SP585 | Ac-LTF$r8EYWAQL$AAAAa-NH₂ | | 1858 | 824.08 | 1859.01 | 930.01 | 620.34 |
| SP586 | Ac-LTF$r8EYWAQL$AAAa-NH₂ | | 1786.97 | 788.56 | 1787.98 | 894.49 | 596.66 |
| SP587 | Ac-LTF$r8EYWAQL$AAa-NH₂ | | 1715.93 | 1138.57 | 1716.94 | 858.97 | 572.98 |
| SP588 | Ac-LTF$r8EYWAQL$Aa-NH₂ | | 1644.89 | 1144.98 | 1645.9 | 823.45 | 549.3 |
| SP589 | Ac-LTF$r8EYWAQL$a-NH₂ | | 1573.85 | 1113.71 | 1574.86 | 787.93 | 525.62 |

In the sequences shown above and elsewhere, the following abbreviations are used: "Nle" represents norleucine, "Aib" represents 2-aminoisobutyric acid, "Ac" represents acetyl, and "Pr" represents propionyl. Amino acids represented as "$" are alpha-Me S5-pentenyl-alanine olefin amino acids connected by an all-carbon crosslinker comprising one double bond. Amino acids represented as "$r5" are alpha-Me R5-pentenyl-alanine olefin amino acids connected by an all-carbon comprising one double bond. Amino acids represented as "$s8" are alpha-Me S8-octenyl-alanine olefin amino acids connected by an all-carbon crosslinker comprising one double bond. Amino acids represented as "$r8" are alpha-Me R8-octenyl-alanine olefin amino acids connected by an all-carbon crosslinker comprising one double bond. "Ahx" represents an aminocyclohexyl linker. The crosslinkers are linear all-carbon crosslinker comprising eight or eleven carbon atoms between the alpha carbons of each amino acid. Amino acids represented as "$/" are alpha-Me S5-pentenyl-alanine olefin amino acids that are not connected by any crosslinker. Amino acids represented as "$/r5" are alpha-Me R5-pentenyl-alanine olefin amino acids that are not connected by any crosslinker. Amino acids represented as "$/s8" are alpha-Me S8-octenyl-alanine olefin amino acids that are not connected by any crosslinker. Amino acids represented as "$/r8" are alpha-Me R8-octenyl-alanine olefin amino acids that are not connected by any crosslinker. Amino acids represented as "Amw" are alpha-Me tryptophan amino acids. Amino acids represented as "Ami" are alpha-Me leucine amino acids. Amino acids represented as "Amf" are alpha-Me phenylalanine amino acids. Amino acids represented as "2ff" are 2-fluoro-phenylalanine amino acids. Amino acids represented as "3ff' are 3-fluoro-phenylalanine amino acids. Amino acids represented as "St" are amino acids comprising two pentenyl-alanine olefin side chains, each of which is crosslinked to another amino acid as indicated. Amino acids represented as "St//" are amino acids comprising two pentenyl-alanine olefin side chains that are not crosslinked. Amino acids represented as "%St" are amino acids comprising two pentenyl-alanine olefin side chains, each of which is crosslinked to another amino acid as indicated via fully saturated hydrocarbon crosslinks. Amino acids represented as "Ba" are beta-alanine. The lower-case character "e" or "z" within the designation of a crosslinked amino acid (e.g. "$er8" or "$zr8") represents the configuration of the double bond (E or Z, respectively). In other contexts, lower-case letters such as "a" or "f" represent D amino acids (e.g. D-alanine, or D-phenylalanine, respectively). Amino acids designated as "NmW" represent N-methyltryptophan. Amino acids designated as "NmY" represent N-methyltyrosine. Amino acids designated as "NmA" represent N-methylalanine. "Kbio" represents a biotin group attached to the side chain amino group of a lysine residue. Amino acids designated as "Sar" represent sarcosine. Amino acids designated as "Cha" represent cyclohexyl alanine. Amino acids designated as "Cpg" represent cyclopentyl glycine. Amino acids designated as "Chg" represent cyclohexyl glycine. Amino acids designated as "Cba" represent cyclobutyl alanine. Amino acids designated as "F4I" represent 4-iodo phenylalanine. "7L" represents N15 isotopic leucine. Amino acids designated as "F3Cl" represent 3-chloro phenylalanine. Amino acids designated as "F4cooh" represent 4-carboxy phenylalanine. Amino acids designated as "F34F2" represent 3,4-difluoro phenylalanine. Amino acids designated as "6clW" represent 6-chloro tryptophan. Amino acids designated as "$rda6" represent alpha-Me R6-hexynyl-alanine alkynyl amino acids, crosslinked via a dialkyne bond to a second alkynyl amino acid. Amino acids designated as "$da5" represent alpha-Me S5-pentynyl-alanine alkynyl amino acids, wherein the alkyne forms one half of a dialkyne bond with a second alkynyl amino acid. Amino acids designated as "$ra9" represent alpha-Me R9-nonynyl-alanine alkynyl amino acids, crosslinked via an alkyne metathesis reaction with a second alkynyl amino acid. Amino acids designated as "$a6" represent alpha-Me S6-hexynyl-alanine alkynyl amino acids, crosslinked via an alkyne metathesis reaction with a second alkynyl amino acid. The designation "iso1" or "iso2" indicates that the peptidomimetic macrocycle is a single isomer.

Amino acids designated as "Cit" represent citrulline. Amino acids designated as "Cou4", "Cou6", "Cou7" and "Cou8", respectively, represent the following structures:

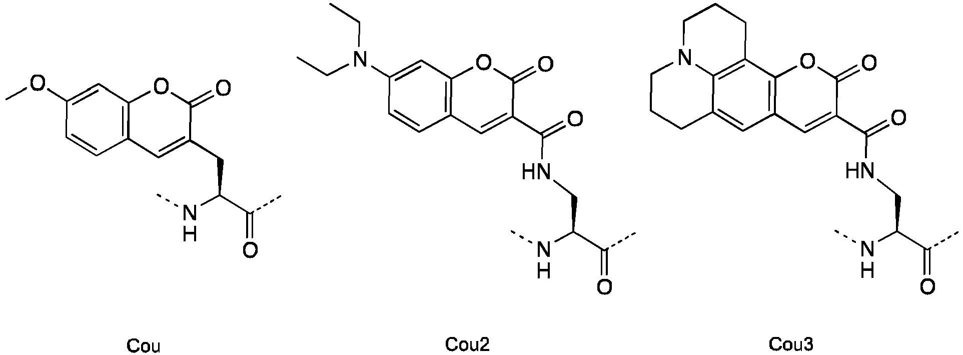

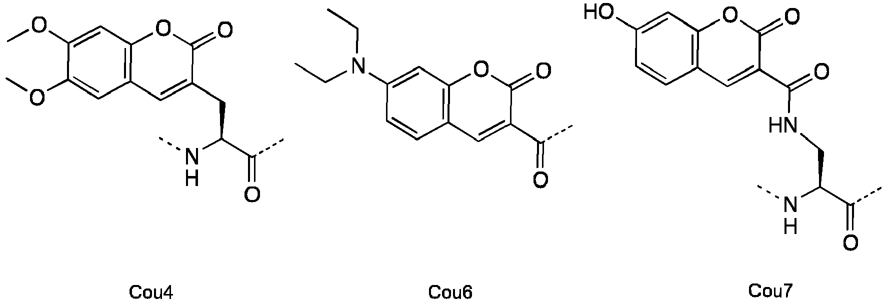

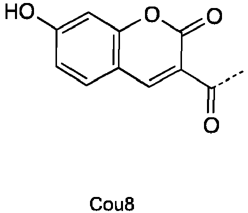

In some cases, a peptidomimetic macrocycle is obtained in more than one isomer, for example due to the configuration of a double bond within the structure of the crosslinker (*E* vs *Z*). Such isomers can or cannot be separable by conventional chromatographic methods. In some cases, one isomer has improved biological properties relative to the other isomer. In one case, an *E* crosslinker olefin isomer of a peptidomimetic macrocycle has better solubility, better target affinity, better *in vivo* or *in vitro* efficacy, higher helicity, or improved cell permeability relative to its *Z* counterpart. In another case, a *Z* crosslinker olefin isomer of a peptidomimetic macrocycle has better solubility, better target affinity, better *in vivo* or *in vitro* efficacy, higher helicity, or improved cell permeability relative to its *E* counterpart.

Table 1c shows exemplary peptidomimetic macrocycles:

**Table 1c**

| SP# | **Structure** |
|---|---|
| SP154 | 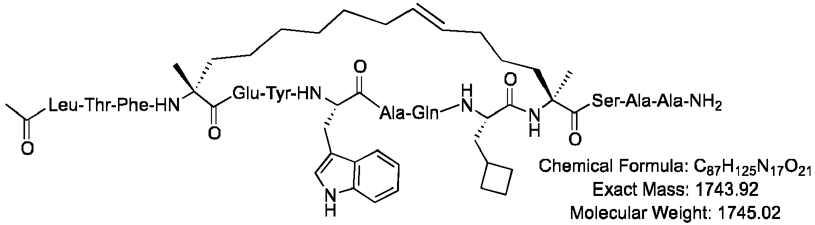 |
| | Ac-L T F$er8EYWAQCba$eSAA-NH2 |
| SP115 | 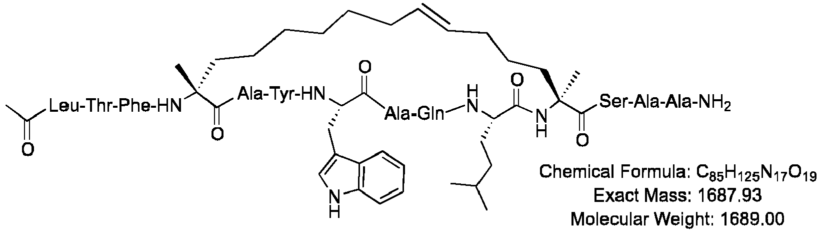 |
| | Ac-L T F$er8AYWAQhL$eSAA-NH2 |
| SP114 | 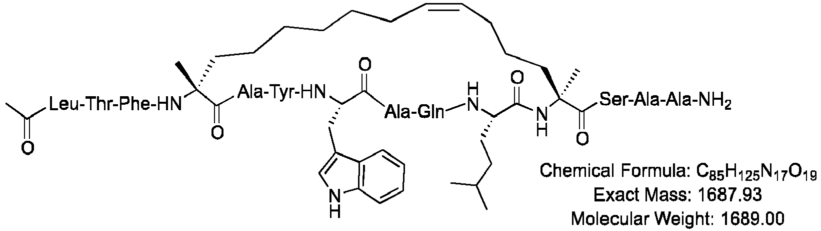 |
| | Ac-L T F$zr8AYWAQhL$zSAA-NH2 |
| SP99 | 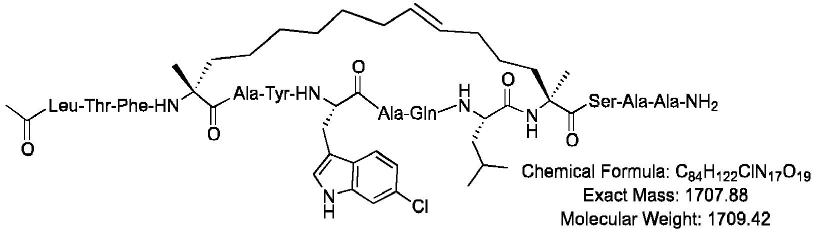 |
| | Ac-L T F$er8AY6clWAQL$eSAA -NH2 |
| SP388 | 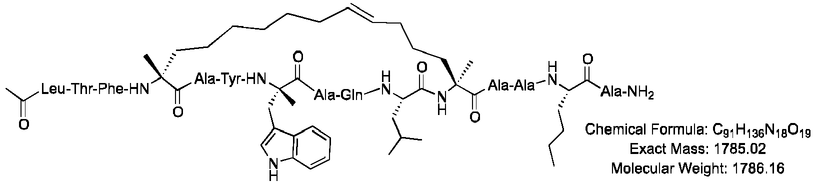 |
| | Ac-L T F$er8AY**Amw**AQL$eAANIeA-NH2 |
| SP331 | 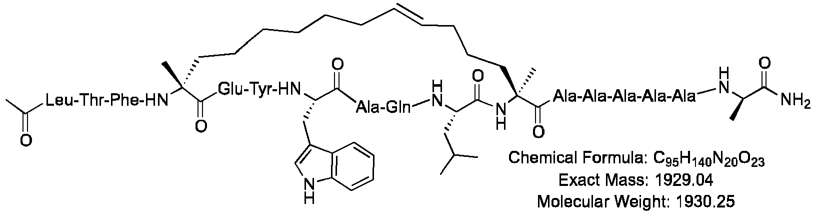 |
| | Ac-L T F$erBEYWAQL$eAAAAAa-NH2 |
| SP445 | 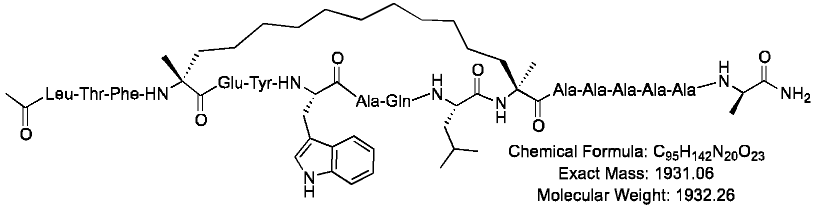 |
| | Ac-L T F%rBEYWAQL%AAAAAa-NH2 |
| SP351 | 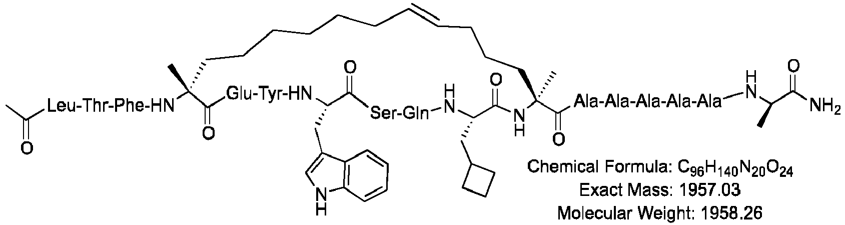 |
| | Ac-L T F$er8EYWSQCba$eAAAAAa-NH2 |
| SP71 | 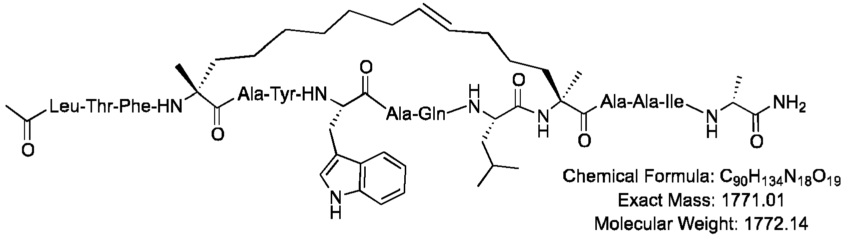 |
| | Ac-L T F$er8AYWAQL$eAA I a-NH2 |
| SP69 | 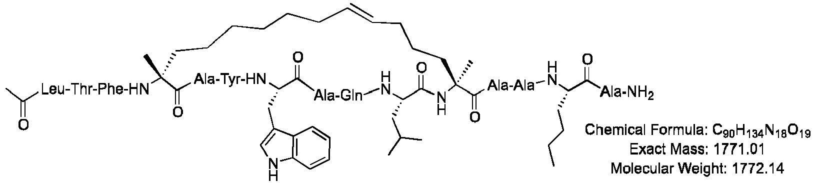 |
| | Ac-L T F$er8AYWAQL$eAANleA-NH2 |
| SP7 | 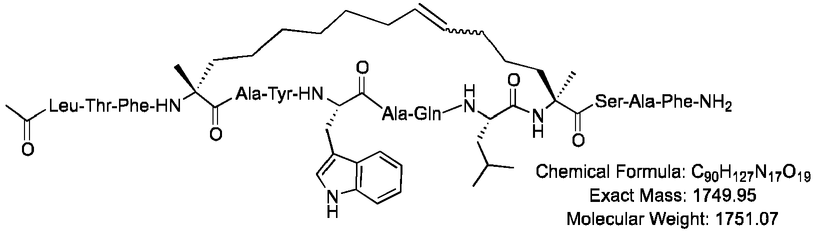 |
| | Ac-L T F$r8AYWAQL$SA F-NH2 |
| SP160 | 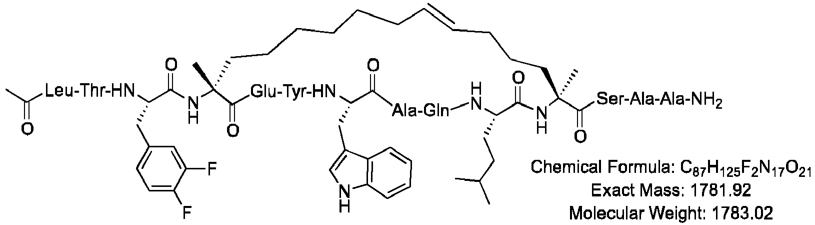 |
| | Ac-L T F34F2$er8EYWAQhL$eSAA-NH2 |
| SP315 | 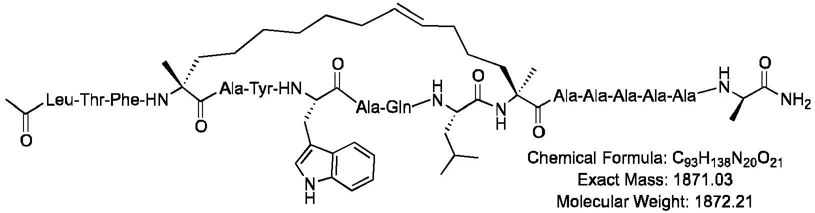 |
| | Ac-L T FSer8AYWAQL$eAAAAAa-NH2 |
| SP249 | 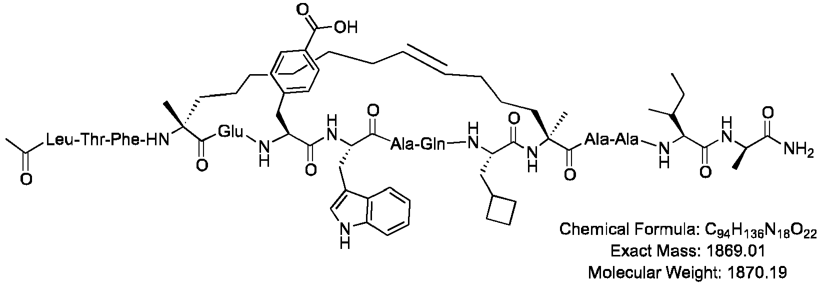 |
| | Ac-L T F$er8EF4coohWAQCba$e AA-I-a -NH2 |
| SP437 | 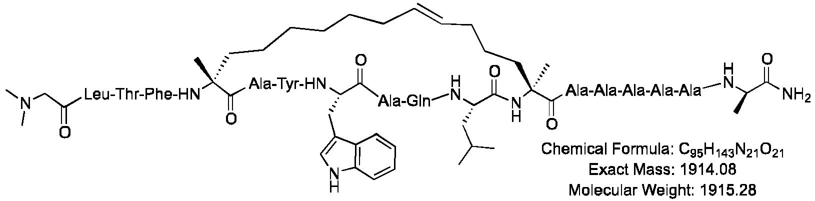 |
| | Dmaac- L T F$er8AYWAQL$eAAAAAa-NH2 |
| SP349 | 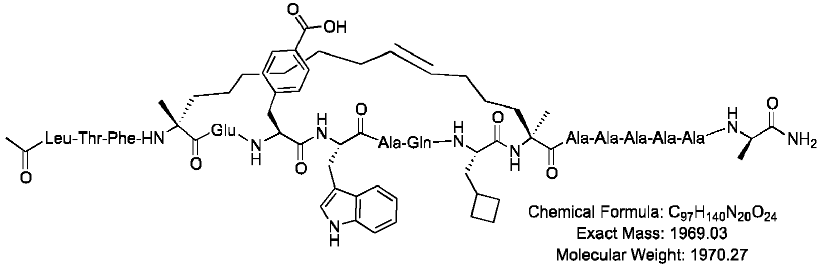 |
| | Ac-L T F$er8EF4coohWAQCba$eAAAAAa-NH2 |
| SP555 | 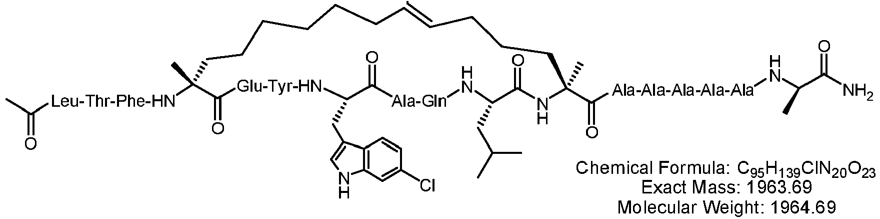 |
| | Ac-LTF$er8EY6clWAQL$eAAAAAa-NH2 |
| SP557 | 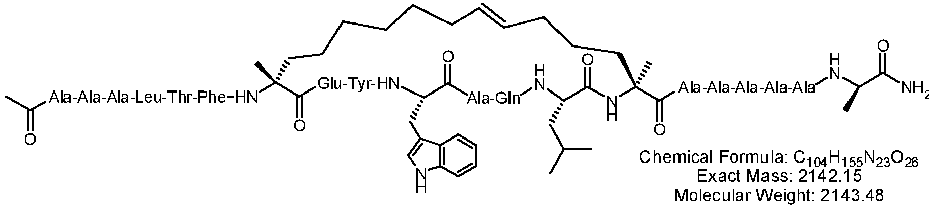 |
| | Ac-AAALTF$er8EYWAQL$eAAAAAa-NH2 |
| SP558 | 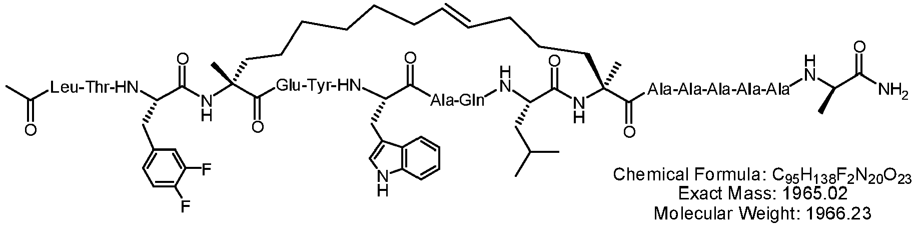 |
| | Ac-LTF34F2$er8EYWAQL$eAAAAAa-NH2 |
| SP367 | 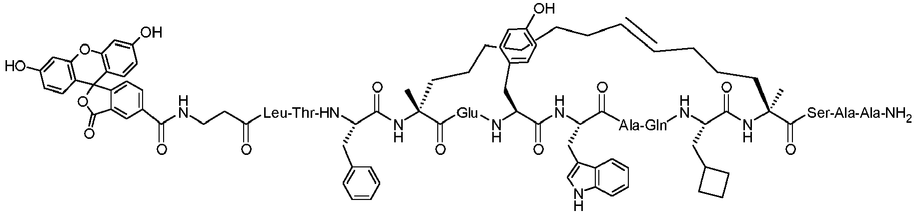 |
| | 5-FAM-BaLTF$er8EYWAQCba$eSAA-NH2 |
| SP562 | 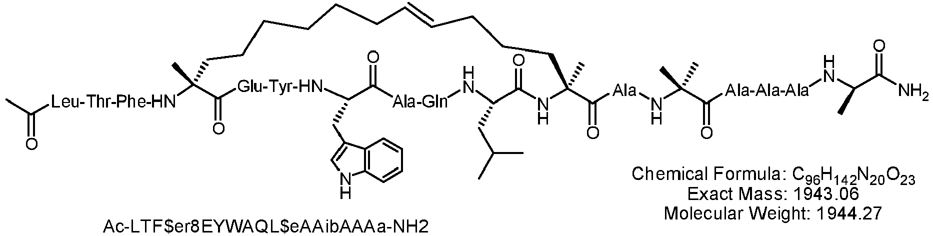 |
| SP564 | 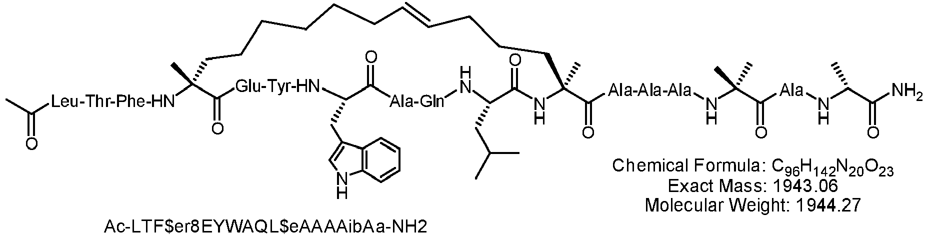 |
| SP566 | 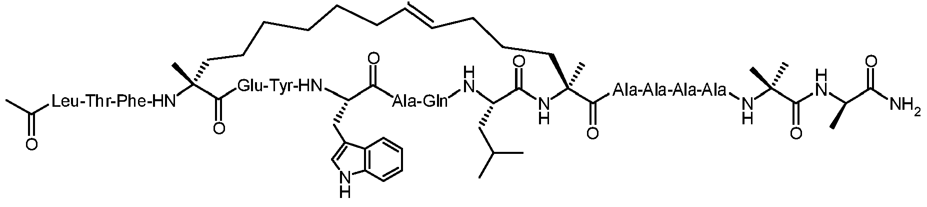 |
| SP567 | 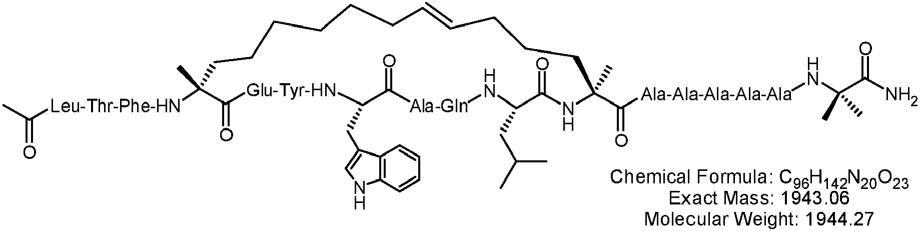 |
| | Ac-LTF$er8EYWAQL$eAAAAAAib-NH2 |
| SP572 | 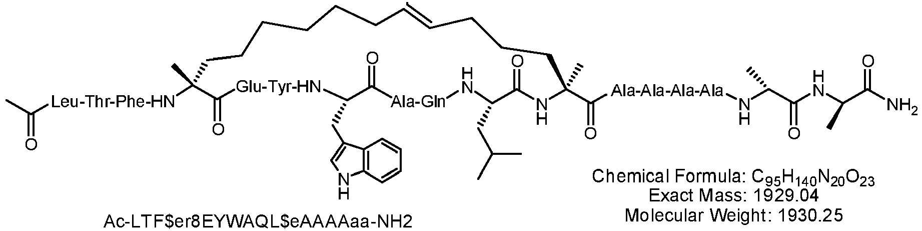 |
| SP573 | 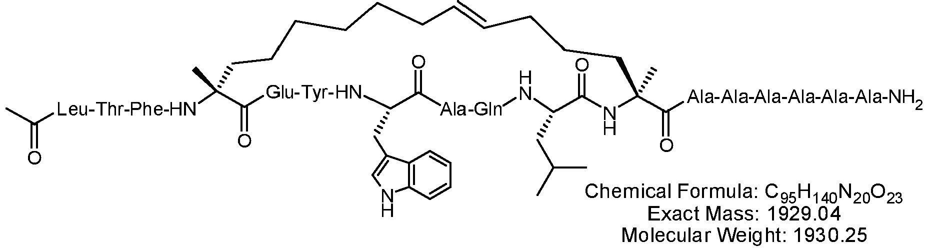 |
| | Ac-LTF$er8EYWAQL$eAAAAAA-NH2 |
| SP578 | 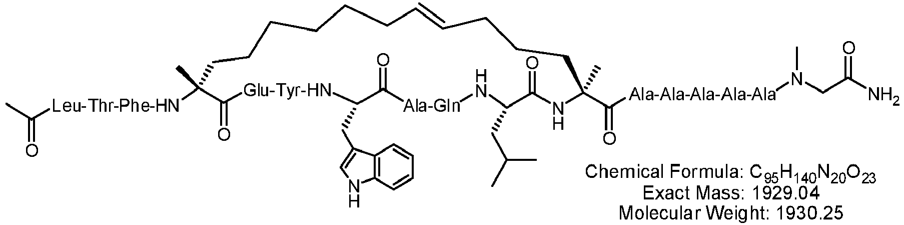 |
| | Ac-LTF$er8EYWAQL$eAAAAASar-NH2 |
| SP664 | 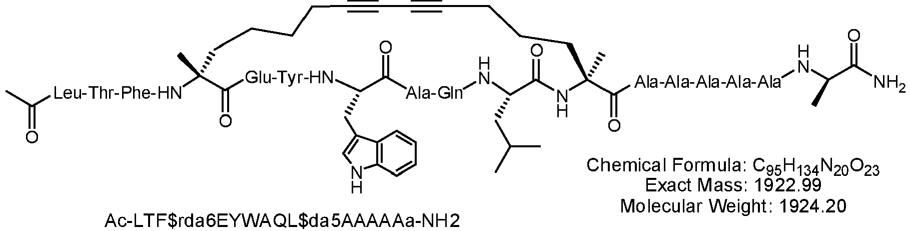 |
| SP662 | 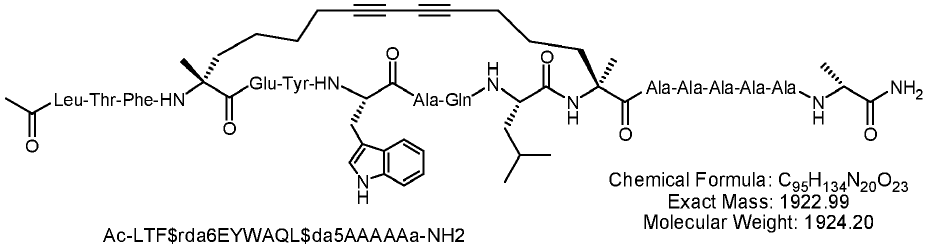 |
| | 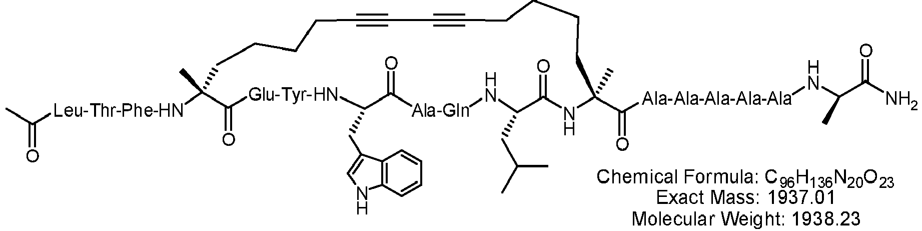 |

In some cases, peptidomimetic macrocycles exclude peptidomimetic macrocycles shown in Table 2a:

**Table 2a**

| Sequence |
|---|
| L$r5QETFSD$s8WKLLPEN |
| LSQ$r5TFSDLW$s8LLPEN |
| LSQE$r5FSDLWK$s8LPEN |
| LSQET$r5SDLWKL$s8PEN |
| LSQETF$r5DLWKLL$s8EN |
| LXQETFS$r5LWKLLP$s8N |
| LSQETFSD$r5WKLLPE$s8 |
| LSQQTF$r5DLWKLL$s8EN |
| LSQETF$r5DLWKLL$s8QN |
| LSQQTF$r5DLWKLL$s8QN |
| LSQETF$r5NLWKLL$s8QN |
| LSQQTF$r5NLWKLL$s8QN |
| LSQQTF$r5NLWRLL$s8QN |
| QSQQTF$r5NLWKLL$s8QN |
| QSQQTF$r5NLWRLL$s8QN |
| QSQQTA$r5NLWRLL$s8QN |
| L$r8QETFSD$WKLLPEN |
| LSQ$r8TFSDLW$LLPEN |
| LSQE$r8FSDLWK$LPEN |
| LSQET$r8SDLWKL$PEN |
| LSQETF$r8DLWKLL$EN |
| LXQETFS$r8LWKLLP$N |
| LSQETFSD$r8WKLLPE$ |
| LSQQTF$r8DLWKLL$EN |
| LSQETF$r8DLWKLL$QN |
| LSQQTF$r8DLWKLL$QN |
| LSQETF$r8NLWKLL$QN |
| LSQQTF$r8NLWKLL$QN |
| LSQQTF$r8NLWRLL$QN |
| QSQQTF$r8NLWKLL$QN |
| QSQQTF$r8NLWRLL$QN |
| QSQQTA$ r8NLWRLL$QN |
| QSQQTF$r8NLWRKK$QN |
| QQTF$r8DLWRLL$EN |
| QQTF$r8DLWRLL$ |
| LSQQTF$DLW$LL |
| QQTF$DLW$LL |
| QQTA$r8DLWRLL$EN |
| QSQQTF$r5NLWRLL$s8QN (dihydroxylated olefin) |
| QSQQTA$r5NLWRLL$s8QN (dihydroxylated olefin) |
| QSQQTF$r8DLWRLL$QN |
| QTF$r8NLWRLL$ |
| QSQQTF$NLW$LLPQN |
| QS$QTF$NLWRLLPQN |
| $TFS$LWKLL |
| ETF$DLW$LL |
| QTF$NLW$LL |
| $SQE$FSNLWKLL |

In Table 2a, X represents S or any amino acid. Peptides shown can comprise an N-terminal capping group such as acetyl or an additional linker such as beta-alanine between the capping group and the start of the peptide sequence.

In some cases, peptidomimetic macrocycles do not comprise a peptidomimetic macrocycle structure as shown in Table 2a.

In some cases, peptidomimetic macrocycles exclude those shown in Table 2b:

**Table 2b**

| **Number** | **Sequence** | **Exact Mass** | **M+2** | **Observed mass (m/e)** |
|---|---|---|---|---|
| 1 | Ac-LSQETF$r8DLWKLL$EN-NH₂ | 2068.13 | 1035.07 | 1035.36 |
| 2 | Ac-LSQETF$r8NLWKLL$QN-NH₂ | 2066.16 | 1034.08 | 1034.31 |
| 3 | Ac-LSQQTF$r8NLWRLL$QN-NH₂ | 2093.18 | 1047.59 | 1047.73 |
| 4 | Ac-QSQQTF$r8NLWKLL$QN-NH₂ | 2080.15 | 1041.08 | 1041.31 |
| 5 | Ac-QSQQTF$r8NLWRLL$Q.N-NH₂ | 2108.15 | 1055.08 | 1055.32 |
| 6 | Ac-QSQQTA$r8NLWRLL$QN-NH₂ | 2032.12 | 1017.06 | 1017.24 |
| 7 | Ac-QAibQQTF$r8NLWRLL$QN-NH₂ | 2106.17 | 1054.09 | 1054.34 |
| 8 | Ac-QSQQTFSNLWRLLPQN-NH₂ | 2000.02 | 1001.01 | 1001.26 |
| 9 | Ac-QSQQTF$/r8NLWRLL$/QN-NH₂ | 2136.18 | 1069.09 | 1069.37 |
| 10 | Ac-QSQAibTF$r8NLWRLL$QN-NH₂ | 2065.15 | 1033.58 | 1033.71 |
| 11 | Ac-QSQQTF$r8NLWRLL$AN-NH₂ | 2051.13 | 1026.57 | 1026.70 |
| 12 | Ac-ASQQTF$r8NLWRLL$QN-NH₂ | 2051.13 | 1026.57 | 1026.90 |
| 13 | Ac-QSQQTF$r8ALWRLL$QN-NH₂ | 2065.15 | 1033.58 | 1033.41 |
| 14 | Ac-QSQETF$r8NLWRLL$QN-NH₂ | 2109.14 | 1055.57 | 1055.70 |
| 15 | Ac-RSQQTF$r8NLWRLL$QN-NH₂ | 2136.20 | 1069.10 | 1069.17 |
| 16 | Ac-RSQQTF$r8NLWRLL$EN-NH₂ | 2137.18 | 1069.59 | 1069.75 |
| 17 | Ac-LSQETFSDLWKLLPEN-NH₂ | 1959.99 | 981.00 | 981.24 |
| 18 | Ac-QSQ$TFS$LWRLLPQN-NH₂ | 2008.09 | 1005.05 | 1004.97 |
| 19 | Ac-QSQQ$FSN$WRLLPQN-NH₂ | 2036.06 | 1019.03 | 1018.86 |
| 20 | Ac-QSQQT$SNL$RLLPQN-NH₂ | 1917.04 | 959.52 | 959.32 |
| 21 | Ac-QSQQTF$NLW$LLPQN-NH₂ | 2007.06 | 1004.53 | 1004.97 |
| 22 | Ac-RTQATF$r8NQWAibANle$TNAibTR-NH₂ | 2310.26 | 1156.13 | 1156.52 |
| 23 | Ac-QSQQTF$r8NLWRLL$RN-NH₂ | 2136.20 | 1069.10 | 1068.94 |
| 24 | Ac-QSQRTF$r8NLWRLL$QN-NH₂ | 2136.20 | 1069.10 | 1068.94 |
| 25 | Ac-QSQQTF$r8NNIeWRLL$QN-NH₂ | 2108.15 | 1055.08 | 1055.44 |
| 26 | Ac-QSQQTF$r8NLWRNIeL$QN-NH₂ | 2108.15 | 1055.08 | 1055.84 |
| 27 | Ac-QSQQTF$r8NLWRLNIe$QN-NH₂ | 2108.15 | 1055.08 | 1055.12 |
| 28 | Ac-QSQQTY$r8NLWRLL$QN-NH₂ | 2124.15 | 1063.08 | 1062.92 |
| 29 | Ac-RAibQQTF$r8NLWRLL$QN-NH₂ | 2134.22 | 1068.11 | 1068.65 |
| 30 | Ac-MPRFMDYWEGLN-NH₂ | 1598.70 | 800.35 | 800.45 |
| 31 | Ac-RSQQRF$r8NLWRLL$QN-NH₂ | 2191.25 | 1096.63 | 1096.83 |
| 32 | Ac-QSQQRF$r8NLWRLL$QN-NH₂ | 2163.21 | 1082.61 | 1082.87 |
| 33 | Ac-RAibQQRF$r8NLWRLL$QN-NH₂ | 2189.27 | 1095.64 | 1096.37 |
| 34 | Ac-RSQQRF$r8NFWRLL$QN-NH₂ | 2225.23 | 1113.62 | 1114.37 |
| 35 | Ac-RSQQRF$r8NYWRLL$QN-NH₂ | 2241.23 | 1121.62 | 1122.37 |
| 36 | Ac-RSQQTF$r8NLWQLL$QN-NH₂ | 2108.15 | 1055.08 | 1055.29 |
| 37 | Ac-QSQQTF$r8NLWQAmIL$QN-NH₂ | 2094.13 | 1048.07 | 1048.32 |
| 38 | Ac-QSQQTF$r8NAmIWRLL$QN-NH₂ | 2122.17 | 1062.09 | 1062.35 |
| 39 | Ac-NIePRF$r8DYWEGL$QN-NH₂ | 1869.98 | 935.99 | 936.20 |
| 40 | Ac-NIePRF$r8NYWRLL$QN-NH₂ | 1952.12 | 977.06 | 977.35 |
| 41 | Ac-RF$r8NLWRLL$Q-NH₂ | 1577.96 | 789.98 | 790.18 |
| 42 | Ac-QSQQTF$r8N2ffWRLL$QN-NH₂ | 2160.13 | 1081.07 | 1081.40 |
| 43 | Ac-QSQQTF$r8N3ffWRLL$QN-NH₂ | 2160.13 | 1081.07 | 1081.34 |
| 44 | Ac-QSQQTF#r8NLWRLL#QN-NH₂ | 2080.12 | 1041.06 | 1041.34 |
| 45 | Ac-RSQQTA$r8NLWRLL$QN-NH₂ | 2060.16 | 1031.08 | 1031.38 |
| 46 | Ac-QSQQTF%r8NLWRLL%QN-NH₂ | 2110.17 | 1056.09 | 1056.55 |
| 47 | HepQSQ$TFSNLWRLLPQN-NH₂ | 2051.10 | 1026.55 | 1026.82 |
| 48 | HepQSQ$TF$r8NLWRLL$QN-NH₂ | 2159.23 | 1080.62 | 1080.89 |
| 49 | Ac-QSQQTF$r8NL6cIWRLL$QN-NH₂ | 2142.11 | 1072.06 | 1072.35 |
| 50 | Ac-QSQQTF$r8NLMe6cIwRLL$QN-NH₂ | 2156.13 | 1079.07 | 1079.27 |
| 51 | Ac-LTFEHYWAQLTS-NH₂ | 1535.74 | 768.87 | 768.91 |
| 52 | Ac-LTF$HYW$QLTS-NH₂ | 1585.83 | 793.92 | 794.17 |
| 53 | Ac-LTFE$YWA$LTS-NH₂ | 1520.79 | 761.40 | 761.67 |
| 54 | Ac-LTF$zr8HYWAQL$zS-NH₂ | 1597.87 | 799.94 | 800.06 |
| 55 | Ac-LTF$r8HYWRQL$S-NH₂ | 1682.93 | 842.47 | 842.72 |
| 56 | Ac-QS$QTFStNLWRLL$s8QN-NH₂ | 2145.21 | 1073.61 | 1073.90 |
| 57 | Ac-QSQQTASNLWRLLPQN-NH₂ | 1923.99 | 963.00 | 963.26 |
| 58 | Ac-QSQQTA$/r8NLWRLL$/QN-NH₂ | 2060.15 | 1031.08 | 1031.24 |
| 59 | Ac-ASQQTF$/r8NLWRLL$/QN-NH₂ | 2079.16 | 1040.58 | 1040.89 |
| 60 | Ac-$SQQ$FSNLWRLLAibQN-NH₂ | 2009.09 | 1005.55 | 1005.86 |
| 61 | Ac-QS$QTF$NLWRLLAibQN-NH₂ | 2023.10 | 1012.55 | 1012.79 |
| 62 | Ac-QSQQ$FSN$WRLLAibQN-NH₂ | 2024.06 | 1013.03 | 1013.31 |
| 63 | Ac-QSQQTF$NLW$LLAibQN-NH₂ | 1995.06 | 998.53 | 998.87 |
| 64 | Ac-QSQQTFS$LWR$LAibQN-NH₂ | 2011.06 | 1006.53 | 1006.83 |
| 65 | Ac-QSQQTFSNLW$LLA$N-NH₂ | 1940.02 | 971.01 | 971.29 |
| 66 | Ac-$/SQQ$/FSNLWRLLAibQN-NH₂ | 2037.12 | 1019.56 | 1019.78 |
| 67 | Ac-QS$/QTF$/NLWRLLAibQN-NH₂ | 2051.13 | 1026.57 | 1026.90 |
| 68 | Ac-QSQQ$/FSN$/WRLLAibQN-NH₂ | 2052.09 | 1027.05 | 1027.36 |
| 69 | Ac-QSQQTF$/NLW$/LLAibQN-NH₂ | 2023.09 | 1012.55 | 1013.82 |
| 70 | Ac-QSQ$TFS$LWRLLAibQN-NH₂ | 1996.09 | 999.05 | 999.39 |
| 71 | Ac-QSQ$/TFS$/LWRLLAibQN-NH₂ | 2024.12 | 1013.06 | 1013.37 |
| 72 | Ac-QS$/QTFSt//NLWRLL$/s8QN-NH₂ | 2201.27 | 1101.64 | 1102.00 |
| 73 | Ac-$r8SQQTFS$LWRLLAibQN-NH₂ | 2038.14 | 1020.07 | 1020.23 |
| 74 | Ac-QSQ$r8TFSNLW$LLAibQN-NH₂ | 1996.08 | 999.04 | 999.32 |
| 75 | Ac-QSQQTFS$r8LWRLLA$N-NH₂ | 2024.12 | 1013.06 | 1013.37 |
| 76 | Ac-QS$r5QTFStNLW$LLAibQN-NH₂ | 2032.12 | 1017.06 | 1017.39 |
| 77 | Ac-$/r8SQQTFS$/LWRLLAibQN-NH₂ | 2066.17 | 1034.09 | 1034.80 |
| 78 | Ac-QSQ$/r8TFSNLW$/LLAibQN-NH₂ | 2024.11 | 1013.06 | 1014.34 |
| 79 | Ac-QSQQTFS$/r8LWRLLA$/N-NH₂ | 2052.15 | 1027.08 | 1027.16 |
| 80 | Ac-QS$/r5QTFSt//NLW$/LLAibQN-NH₂ | 2088.18 | 1045.09 | 1047.10 |
| 81 | Ac-QSQQTFSNLWRLLAibQN-NH₂ | 1988.02 | 995.01 | 995.31 |
| 82 | Hep/QSQ$/TF$/r8NLWRLL$/QN-NH₂ | 2215.29 | 1108.65 | 1108.93 |
| 83 | Ac-ASQQTF$r8NLRWLL$QN-NH₂ | 2051.13 | 1026.57 | 1026.90 |
| 84 | Ac-QSQQTF$/r8NLWRLL$/Q-NH₂ | 2022.14 | 1012.07 | 1012.66 |
| 85 | Ac-QSQQTF$r8NLWRLL$Q-NH₂ | 1994.11 | 998.06 | 998.42 |
| 86 | Ac-AAARAA$r8AAARAA$AA-N H₂ | 1515.90 | 758.95 | 759.21 |
| 87 | Ac-LTFEHYWAQLTSA-NH₂ | 1606.78 | 804.39 | 804.59 |
| 88 | Ac-LTF$r8HYWAQL$SA-NH₂ | 1668.90 | 835.45 | 835.67 |
| 89 | Ac-ASQQTFSNLWRLLPQN-NH₂ | 1943.00 | 972.50 | 973.27 |
| 90 | Ac-QS$QTFStNLW$r5LLAibQN-NH₂ | 2032.12 | 1017.06 | 1017.30 |
| 91 | Ac-QSQQTFAibNLWRLLAibQN-NH₂ | 1986.04 | 994.02 | 994.19 |
| 92 | Ac-QSQQTFNeNLWRLLNeQN-NH₂ | 2042.11 | 1022.06 | 1022.23 |
| 93 | Ac-QSQQTF$/r8NLWRLLAibQN-NH₂ | 2082.14 | 1042.07 | 1042.23 |
| 94 | Ac-QSQQTF$/r8NLWRLLNIeQN-NH₂ | 2110.17 | 1056.09 | 1056.29 |
| 95 | Ac-QSQQTFAibNLWRLL$/QN-NH₂ | 2040.09 | 1021.05 | 1021.25 |
| 96 | Ac-QSQQTFNIeNLWRLL$/QN-NH₂ | 2068.12 | 1035.06 | 1035.31 |
| 97 | Ac-QSQQTF%r8NL6cIWRNIeL%QN-NH₂ | 2144.13 | 1073.07 | 1073.32 |
| 98 | Ac-QSQQTF%r8NLMe6clWRLL%QN-NH₂ | 2158.15 | 1080.08 | 1080.31 |
| 101 | Ac-FNle$YWE$L-NH₂ | 1160.63 | - | 1161.70 |
| 102 | Ac-F$r8AYWELL$A-NH₂ | 1344.75 | - | 1345.90 |
| 103 | Ac-F$r8AYWQLL$A-NH₂ | 1343.76 | - | 1344.83 |
| 104 | Ac-NlePRF$r8NYWELL$QN-NH₂ | 1925.06 | 963.53 | 963.69 |
| 105 | Ac-NlePRF$r8DYWRLL$QN-NH₂ | 1953.10 | 977.55 | 977.68 |
| 106 | Ac-NlePRF$r8NYWRLL$Q-NH₂ | 1838.07 | 920.04 | 920.18 |
| 107 | Ac-NlePRF$r8NYWRLL$-NH₂ | 1710.01 | 856.01 | 856.13 |
| 108 | Ac-QSQQTF$r8DLWRLL$QN-NH₂ | 2109.14 | 1055.57 | 1055.64 |
| 109 | Ac-QSQQTF$r8NLWRLL$EN-NH₂ | 2109.14 | 1055.57 | 1055.70 |
| 110 | Ac-QSQQTF$r8NLWRLL$QD-NH₂ | 2109.14 | 1055.57 | 1055.64 |
| 111 | Ac-QSQQTF$r8NLWRLL$S-NH₂ | 1953.08 | 977.54 | 977.60 |
| 112 | Ac-ESQQTF$r8NLWRLL$QN-NH₂ | 2109.14 | 1055.57 | 1055.70 |
| 113 | Ac-LTF$r8NLWRNIeL$Q-NH₂ | 1635.99 | 819.00 | 819.10 |
| 114 | Ac-LRF$r8NLWRNIeL$Q-NH₂ | 1691.04 | 846.52 | 846.68 |
| 115 | Ac-QSQQTF$r8NWWRNIeL$QN-NH₂ | 2181.15 | 1091.58 | 1091.64 |
| 116 | Ac-QSQQTF$r8NLWRNIeL$Q-NH₂ | 1994.11 | 998.06 | 998.07 |
| 117 | Ac-QTF$r8NLWRNIeL$QN-NH₂ | 1765.00 | 883.50 | 883.59 |
| 118 | Ac-NIePRF$r8NWWRLL$QN-NH₂ | 1975.13 | 988.57 | 988.75 |
| 119 | Ac-NIePRF$r8NWWRLL$A-NH₂ | 1804.07 | 903.04 | 903.08 |
| 120 | Ac-TSFAEYWNLLNH₂ | 1467.70 | 734.85 | 734.90 |
| 121 | Ac-QTF$r8HWWSQL$S-NH₂ | 1651.85 | 826.93 | 827.12 |
| 122 | Ac-FM$YWE$L-NH₂ | 1178.58 | - | 1179.64 |
| 123 | Ac-QTFEHWWSQLLS-NH₂ | 1601.76 | 801.88 | 801.94 |
| 124 | Ac-QSQQTF$r8NLAmwRLNIe$QN-NH₂ | 2122.17 | 1062.09 | 1062.24 |
| 125 | Ac-FMAibY6cIWEAc3cL-NH₂ | 1130.47 | - | 1131.53 |
| 126 | Ac-FNIe$Y6cIWE$L-NH₂ | 1194.59 | - | 1195.64 |
| 127 | Ac-F$zr8AY6cIWEAc3cL$z-NH₂ | 1277.63 | 639.82 | 1278.71 |
| 128 | Ac-F$r8AY6cIWEAc3cL$A-NH₂ | 1348.66 | - | 1350.72 |
| 129 | Ac-NIePRF$r8NY6cIWRLL$QN-NH₂ | 1986.08 | 994.04 | 994.64 |
| 130 | Ac-AF$r8AAWALA$A-NH₂ | 1223.71 | - | 1224.71 |
| 131 | Ac-TF$r8AAWRLA$Q-NH₂ | 1395.80 | 698.90 | 399.04 |
| 132 | Pr-TF$r8AAWRLA$Q-NH₂ | 1409.82 | 705.91 | 706.04 |
| 133 | Ac-QSQQTF%r8NLWRNIeL%QN-NH₂ | 2110.17 | 1056.09 | 1056.22 |
| 134 | Ac-LTF%r8HYWAQL%SA-NH₂ | 1670.92 | 836.46 | 836.58 |
| 135 | Ac-NIePRF%r8NYWRLL%QN-NH₂ | 1954.13 | 978.07 | 978.19 |
| 136 | Ac-NIePRF%r8NY6cIWRLL%QN-NH₂ | 1988.09 | 995.05 | 995.68 |
| 137 | Ac-LTF%r8HY6cIWAQL%S-NH₂ | 1633.84 | 817.92 | 817.93 |
| 138 | Ac-QS%QTF%StNLWRLL%s8QN-NH₂ | 2149.24 | 1075.62 | 1075.65 |
| 139 | Ac-LTF%r8HY6cIWRQL%S-NH₂ | 1718.91 | 860.46 | 860.54 |
| 140 | Ac-QSQQTF%r8NL6cIWRLL%QN-NH₂ | 2144.13 | 1073.07 | 1073.64 |
| 141 | Ac-%r8SQQTFS%LWRLLAibQN-NH₂ | 2040.15 | 1021.08 | 1021.13 |
| 142 | Ac-LTF%r8HYWAQL%S-NH₂ | 1599.88 | 800.94 | 801.09 |
| 143 | Ac-TSF%r8QYWNLL%P-NH₂ | 1602.88 | 802.44 | 802.58 |
| 147 | Ac-LTFEHYWAQLTS-NH₂ | 1535.74 | 768.87 | 769.5 |
| 152 | Ac-F$er8AY6cIWEAc3cL$e-NH₂ | 1277.63 | 639.82 | 1278.71 |
| 153 | Ac-AF$r8AAWALA$A-NH₂ | 1277.63 | 639.82 | 1277.84 |
| 154 | Ac-TF$r8AAWRLA$Q-NH₂ | 1395.80 | 698.90 | 699.04 |
| 155 | Pr-TF$r8AAWRLA$Q-NH₂ | 1409.82 | 705.91 | 706.04 |
| 156 | Ac-LTF$er8HYWAQL$eS-NH₂ | 1597.87 | 799.94 | 800.44 |
| 159 | Ac-CCPGCCBQSQQTF$r8NLWRLL$QN-NH₂ | 2745.30 | 1373.65 | 1372.99 |
| 160 | Ac-CCPGCCBaQSQQTA$r8NLWRLL$QN-NH₂ | 2669.27 | 1335.64 | 1336.09 |
| 161 | Ac-CCPGCCBaNIePRF$r8NYWRLL$QN-NH₂ | 2589.26 | 1295.63 | 1296.2 |
| 162 | Ac-LTF$/r8HYWAQL$/S-NH₂ | 1625.90 | 813.95 | 814.18 |
| 163 | Ac-F%r8HY6cIWRAc3cL%-NH₂ | 1372.72 | 687.36 | 687.59 |
| 164 | Ac-QTF%r8HWWSQL%S-NH₂ | 1653.87 | 827.94 | 827.94 |
| 165 | Ac-LTA$r8HYWRQL$S-NH₂ | 1606.90 | 804.45 | 804.66 |
| 166 | Ac-Q$r8QQTFSN$WRLLAibQN-NH₂ | 2080.12 | 1041.06 | 1041.61 |
| 167 | Ac-QSQQ$r8FSNLWR$LAibQN-NH₂ | 2066.11 | 1034.06 | 1034.58 |
| 168 | Ac-F$r8AYWEAc3cL$A-NH₂ | 1314.70 | 658.35 | 1315.88 |
| 169 | Ac-F$r8AYWEAc3cL$S-NH₂ | 1330.70 | 666.35 | 1331.87 |
| 170 | Ac-F$r8AYWEAc3cL$Q-NH₂ | 1371.72 | 686.86 | 1372.72 |
| 171 | Ac-F$r8AYWEAibL$S-NH₂ | 1332.71 | 667.36 | 1334.83 |
| 172 | Ac-F$r8AYWEAL$S-NH₂ | 1318.70 | 660.35 | 1319.73 |
| 173 | Ac-F$r8AYWEQL$S-NH₂ | 1375.72 | 688.86 | 1377.53 |
| 174 | Ac-F$r8HYWEQL$S-NH₂ | 1441.74 | 721.87 | 1443.48 |
| 175 | Ac-F$r8HYWAQL$S-NH₂ | 1383.73 | 692.87 | 1385.38 |
| 176 | Ac-F$r8HYWAAc3cL$S-NH₂ | 1338.71 | 670.36 | 1340.82 |
| 177 | Ac-F$r8HYWRAc3cL$S-NH₂ | 1423.78 | 712.89 | 713.04 |
| 178 | Ac-F$r8AYWEAc3cL#A-NH₂ | 1300.69 | 651.35 | 1302.78 |
| 179 | Ac-NIePTF%r8NYWRLL%QN-NH₂ | 1899.08 | 950.54 | 950.56 |
| 180 | Ac-TF$r8AAWRAL$Q-NH₂ | 1395.80 | 698.90 | 699.13 |
| 181 | Ac-TSF%r8HYWAQL%S-NH₂ | 1573.83 | 787.92 | 787.98 |
| 184 | Ac-F%r8AY6clWEAc3cL%A-NH₂ | 1350.68 | 676.34 | 676.91 |
| 185 | Ac-LTF$r8HYWAQI$S-NH₂ | 1597.87 | 799.94 | 800.07 |
| 186 | Ac-LTF$r8HYWAQNIe$S-NH₂ | 1597.87 | 799.94 | 800.07 |
| 187 | Ac-LTF$r8HYWAQL$A-NH₂ | 1581.87 | 791.94 | 792.45 |
| 188 | Ac-LTF$r8HYWAQL$Abu-NH₂ | 1595.89 | 798.95 | 799.03 |
| 189 | Ac-LTF$r8HYWAbuQL$S-NH₂ | 1611.88 | 806.94 | 807.47 |
| 190 | Ac-LTF$er8AYWAQL$eS-NH₂ | 1531.84 | 766.92 | 766.96 |
| 191 | Ac-LAF$r8HYWAQL$S-NH₂ | 1567.86 | 784.93 | 785.49 |
| 192 | Ac-LAF$r8AYWAQL$S-NH₂ | 1501.83 | 751.92 | 752.01 |
| 193 | Ac-LTF$er8AYWAQL$eA-NH₂ | 1515.85 | 758.93 | 758.97 |
| 194 | Ac-LAF$r8AYWAQL$A-NH₂ | 1485.84 | 743.92 | 744.05 |
| 195 | Ac-LTF$r8NLWANIeL$Q-NH₂ | 1550.92 | 776.46 | 776.61 |
| 196 | Ac-LTF$r8NLWANIeL$A-NH₂ | 1493.90 | 747.95 | 1495.6 |
| 197 | Ac-LTF$r8ALWANIeL$Q-NH₂ | 1507.92 | 754.96 | 755 |
| 198 | Ac-LAF$r8NLWANIeL$Q-NH₂ | 1520.91 | 761.46 | 761.96 |
| 199 | Ac-LAF$r8ALWANIeL$A-NH₂ | 1420.89 | 711.45 | 1421.74 |
| 200 | Ac-A$r8AYWEAc3cL$A-NH₂ | 1238.67 | 620.34 | 1239.65 |
| 201 | Ac-F$r8AYWEAc3cL$AA-NH₂ | 1385.74 | 693.87 | 1386.64 |
| 202 | Ac-F$r8AYWEAc3cL$Abu-NH₂ | 1328.72 | 665.36 | 1330.17 |
| 203 | Ac-F$r8AYWEAc3cL$NIe-NH₂ | 1356.75 | 679.38 | 1358.22 |
| 204 | Ac-F$r5AYWEAc3cL$s8A-NH₂ | 1314.70 | 658.35 | 1315.51 |
| 205 | Ac-F$AYWEAc3cL$r8A-NH₂ | 1314.70 | 658.35 | 1315.66 |
| 206 | Ac-F$r8AYWEAc3cI$A-NH₂ | 1314.70 | 658.35 | 1316.18 |
| 207 | Ac-F$r8AYWEAc3cNIe$A-NH₂ | 1314.70 | 658.35 | 1315.66 |
| 208 | Ac-F$r8AYWEAmIL$A-NH₂ | 1358.76 | 680.38 | 1360.21 |
| 209 | Ac-F$r8AYWENIeL$A-NH₂ | 1344.75 | 673.38 | 1345.71 |
| 210 | Ac-F$r8AYWQAc3cL$A-NH₂ | 1313.72 | 657.86 | 1314.7 |
| 211 | Ac-F$r8AYWAAc3cL$A-NH₂ | 1256.70 | 629.35 | 1257.56 |
| 212 | Ac-F$r8AYWAbuAc3cL$A-NH₂ | 1270.71 | 636.36 | 1272.14 |
| 213 | Ac-F$r8AYWNIeAc3cL$A-NH₂ | 1298.74 | 650.37 | 1299.67 |
| 214 | Ac-F$r8AbuYWEAc3cL$A-NH₂ | 1328.72 | 665.36 | 1329.65 |
| 215 | Ac-F$r8NIeYWEAc3cL$A-NH₂ | 1356.75 | 679.38 | 1358.66 |
| 216 | 5-FAM-BaLTFEHYWAQLTS-NH₂ | 1922.82 | 962.41 | 962.87 |
| 217 | 5-FAM-BaLTF%r8HYWAQL%S-NH₂ | 1986.96 | 994.48 | 994.97 |
| 218 | Ac-LTF$r8HYWAQhL$S-NH₂ | 1611.88 | 806.94 | 807 |
| 219 | Ac-LTF$r8HYWAQTIe$S-NH₂ | 1597.87 | 799.94 | 799.97 |
| 220 | Ac-LTF$r8HYWAQAdm$S-NH₂ | 1675.91 | 838.96 | 839.09 |
| 221 | Ac-LTF$r8HYWAQhCha$S-NH₂ | 1651.91 | 826.96 | 826.98 |
| 222 | Ac-LTF$r8HYWAQCha$S-NH₂ | 1637.90 | 819.95 | 820.02 |
| 223 | Ac-LTF$r8HYWAc6cQL$S-NH₂ | 1651.91 | 826.96 | 826.98 |
| 224 | Ac-LTF$r8HYWAc5cQL$S-NH₂ | 1637.90 | 819.95 | 820.02 |
| 225 | Ac-LThF$r8HYWAQL$S-NH₂ | 1611.88 | 806.94 | 807 |
| 226 | Ac-LTIgI$r8HYWAQL$S-NH₂ | 1625.90 | 813.95 | 812.99 |
| 227 | Ac-LTF$r8HYWAQChg$S-NH₂ | 1623.88 | 812.94 | 812.99 |
| 228 | Ac-LTF$r8HYWAQF$S-NH₂ | 1631.85 | 816.93 | 816.99 |
| 229 | Ac-LTF$r8HYWAQIgI$S-NH₂ | 1659.88 | 830.94 | 829.94 |
| 230 | Ac-LTF$r8HYWAQCba$S-NH₂ | 1609.87 | 805.94 | 805.96 |
| 231 | Ac-LTF$r8HYWAQCpg$S-NH₂ | 1609.87 | 805.94 | 805.96 |
| 232 | Ac-LTF$r8HhYWAQL$S-NH₂ | 1611.88 | 806.94 | 807 |
| 233 | Ac-F$r8AYWEAc3chL$A-NH₂ | 1328.72 | 665.36 | 665.43 |
| 234 | Ac-F$r8AYWEAc3cTIe$A-NH₂ | 1314.70 | 658.35 | 1315.62 |
| 235 | Ac-F$r8AYWEAc3cAdm$A-NH₂ | 1392.75 | 697.38 | 697.47 |
| 236 | Ac-F$r8AYWEAc3chCha$A-NH₂ | 1368.75 | 685.38 | 685.34 |
| 237 | Ac-F$r8AYWEAc3cCha$A-NH₂ | 1354.73 | 678.37 | 678.38 |
| 238 | Ac-F$r8AYWEAc6cL$A-NH₂ | 1356.75 | 679.38 | 679.42 |
| 239 | Ac-F$r8AYWEAc5cL$A-NH₂ | 1342.73 | 672.37 | 672.46 |
| 240 | Ac-hF$r8AYWEAc3cL$A-NH₂ | 1328.72 | 665.36 | 665.43 |
| 241 | Ac-IgI$r8AYWEAc3cL$A-NH₂ | 1342.73 | 672.37 | 671.5 |
| 243 | Ac-F$r8AYWEAc3cF$A-NH₂ | 1348.69 | 675.35 | 675.35 |
| 244 | Ac-F$r8AYWEAc3cIgI$A-NH₂ | 1376.72 | 689.36 | 688.37 |
| 245 | Ac-F$r8AYWEAc3cCba$A-NH₂ | 1326.70 | 664.35 | 664.47 |
| 246 | Ac-F$r8AYWEAc3cCpg$A-NH₂ | 1326.70 | 664.35 | 664.39 |
| 247 | Ac-F$r8AhYWEAc3cL$A-NH₂ | 1328.72 | 665.36 | 665.43 |
| 248 | Ac-F$r8AYWEAc3cL$Q-NH₂ | 1371.72 | 686.86 | 1372.87 |
| 249 | Ac-F$r8AYWEAibL$A-NH₂ | 1316.72 | 659.36 | 1318.18 |
| 250 | Ac-F$r8AYWEAL$A-NH₂ | 1302.70 | 652.35 | 1303.75 |
| 251 | Ac-LAF$r8AYWAAL$A-NH₂ | 1428.82 | 715.41 | 715.49 |
| 252 | Ac-LTF$r8HYWAAc3cL$S-NH₂ | 1552.84 | 777.42 | 777.5 |
| 253 | Ac-NIeTF$r8HYWAQL$S-NH₂ | 1597.87 | 799.94 | 800.04 |
| 254 | Ac-VTF$r8HYWAQL$S-NH₂ | 1583.85 | 792.93 | 793.04 |
| 255 | Ac-FTF$r8HYWAQL$S-NH₂ | 1631.85 | 816.93 | 817.02 |
| 256 | Ac-WTF$r8HYWAQL$S-NH₂ | 1670.86 | 836.43 | 836.85 |
| 257 | Ac-RTF$r8HYWAQL$S-NH₂ | 1640.88 | 821.44 | 821.9 |
| 258 | Ac-KTF$r8HYWAQL$S-NH₂ | 1612.88 | 807.44 | 807.91 |
| 259 | Ac-LNIeF$r8HYWAQL$S-NH₂ | 1609.90 | 805.95 | 806.43 |
| 260 | Ac-LVF$r8HYWAQL$S-NH₂ | 1595.89 | 798.95 | 798.93 |
| 261 | Ac-LFF$r8HYWAQL$S-NH₂ | 1643.89 | 822.95 | 823.38 |
| 262 | Ac-LWF$r8HYWAQL$S-NH₂ | 1682.90 | 842.45 | 842.55 |
| 263 | Ac-LRF$r8HYWAQL$S-NH₂ | 1652.92 | 827.46 | 827.52 |
| 264 | Ac-LKF$r8HYWAQL$S-NH₂ | 1624.91 | 813.46 | 813.51 |
| 265 | Ac-LTF$r8NIeYWAQL$S-NH₂ | 1573.89 | 787.95 | 788.05 |
| 266 | Ac-LTF$r8VYWAQL$S-NH₂ | 1559.88 | 780.94 | 780.98 |
| 267 | Ac-LTF$r8FYWAQL$S-NH₂ | 1607.88 | 804.94 | 805.32 |
| 268 | Ac-LTF$r8WYWAQL$S-NH₂ | 1646.89 | 824.45 | 824.86 |
| 269 | Ac-LTF$r8RYWAQL$S-NH₂ | 1616.91 | 809.46 | 809.51 |
| 270 | Ac-LTF$r8KYWAQL$S-NH₂ | 1588.90 | 795.45 | 795.48 |
| 271 | Ac-LTF$r8HNIeWAQL$S-NH₂ | 1547.89 | 774.95 | 774.98 |
| 272 | Ac-LTF$r8HVWAQL$S-NH₂ | 1533.87 | 767.94 | 767.95 |
| 273 | Ac-LTF$r8HFWAQL$S-NH₂ | 1581.87 | 791.94 | 792.3 |
| 274 | Ac-LTF$r8HWWAQL$S-NH₂ | 1620.88 | 811.44 | 811.54 |
| 275 | Ac-LTF$r8HRWAQL$S-NH₂ | 1590.90 | 796.45 | 796.52 |
| 276 | Ac-LTF$r8HKWAQL$S-NH₂ | 1562.90 | 782.45 | 782.53 |
| 277 | Ac-LTF$r8HYWNIeQL$S-NH₂ | 1639.91 | 820.96 | 820.98 |
| 278 | Ac-LTF$r8HYWVQL$S-NH₂ | 1625.90 | 813.95 | 814.03 |
| 279 | Ac-LTF$r8HYWFQL$S-NH₂ | 1673.90 | 837.95 | 838.03 |
| 280 | Ac-LTF$r8HYWWQL$S-NH₂ | 1712.91 | 857.46 | 857.5 |
| 281 | Ac-LTF$r8HYWKQL$S-NH₂ | 1654.92 | 828.46 | 828.49 |
| 282 | Ac-LTF$r8HYWANIeL$S-NH₂ | 1582.89 | 792.45 | 792.52 |
| 283 | Ac-LTF$r8HYWAVL$S-NH₂ | 1568.88 | 785.44 | 785.49 |
| 284 | Ac-LTF$r8HYWAFL$S-NH₂ | 1616.88 | 809.44 | 809.47 |
| 285 | Ac-LTF$r8HYWAWL$S-NH₂ | 1655.89 | 828.95 | 829 |
| 286 | Ac-LTF$r8HYWARL$S-NH₂ | 1625.91 | 813.96 | 813.98 |
| 287 | Ac-LTF$r8HYWAQL$NIe-NH₂ | 1623.92 | 812.96 | 813.39 |
| 288 | Ac-LTF$r8HYWAQL$V-NH₂ | 1609.90 | 805.95 | 805.99 |
| 289 | Ac-LTF$r8HYWAQL$F-NH₂ | 1657.90 | 829.95 | 830.26 |
| 290 | Ac-LTF$r8HYWAQL$W-NH₂ | 1696.91 | 849.46 | 849.5 |
| 291 | Ac-LTF$r8HYWAQL$R-NH₂ | 1666.94 | 834.47 | 834.56 |
| 292 | Ac-LTF$r8HYWAQL$K-NH₂ | 1638.93 | 820.47 | 820.49 |
| 293 | Ac-Q$r8QQTFSN$WRLLAibQN-NH₂ | 2080.12 | 1041.06 | 1041.54 |
| 294 | Ac-QSQQ$r8FSNLWR$LAibQN-NH₂ | 2066.11 | 1034.06 | 1034.58 |
| 295 | Ac-LT2PaI$r8HYWAQL$S-NH₂ | 1598.86 | 800.43 | 800.49 |
| 296 | Ac-LT3PaI$r8HYWAQL$S-NH₂ | 1598.86 | 800.43 | 800.49 |
| 297 | Ac-LT4PaI$r8HYWAQL$S-NH₂ | 1598.86 | 800.43 | 800.49 |
| 298 | Ac-LTF2CF3$r8HYWAQL$S-NH₂ | 1665.85 | 833.93 | 834.01 |
| 299 | Ac-LTF2CN$r8HYWAQL$S-NH₂ | 1622.86 | 812.43 | 812.47 |
| 300 | Ac-LTF2Me$r8HYWAQL$S-NH₂ | 1611.88 | 806.94 | 807 |
| 301 | Ac-LTF3CI$r8HYWAQL$S-NH₂ | 1631.83 | 816.92 | 816.99 |
| 302 | Ac-LTF4CF3$r8HYWAQL$S-NH₂ | 1665.85 | 833.93 | 833.94 |
| 303 | Ac-LTF4tBu$r8HYWAQL$S-NH₂ | 1653.93 | 827.97 | 828.02 |
| 304 | Ac-LTF5F$r8HYWAQL$S-NH₂ | 1687.82 | 844.91 | 844.96 |
| 305 | Ac-LTF$r8HY3BthAAQL$S-NH₂ | 1614.83 | 808.42 | 808.48 |
| 306 | Ac-LTF2Br$r8HYWAQL$S-NH₂ | 1675.78 | 838.89 | 838.97 |
| 307 | Ac-LTF4Br$r8HYWAQL$S-NH₂ | 1675.78 | 838.89 | 839.86 |
| 308 | Ac-LTF2CI$r8HYWAQL$S-NH₂ | 1631.83 | 816.92 | 816.99 |
| 309 | Ac-LTF4CI$r8HYWAQL$S-NH₂ | 1631.83 | 816.92 | 817.36 |
| 310 | Ac-LTF3CN$r8HYWAQL$S-NH₂ | 1622.86 | 812.43 | 812.47 |
| 311 | Ac-LTF4CN$r8HYWAQL$S-NH₂ | 1622.86 | 812.43 | 812.47 |
| 312 | Ac-LTF34CI2$r8HYWAQL$S-NH₂ | 1665.79 | 833.90 | 833.94 |
| 313 | Ac-LTF34F2$r8HYWAQL$S-NH₂ | 1633.85 | 817.93 | 817.95 |
| 314 | Ac-LTF35F2$r8HYWAQL$S-NH₂ | 1633.85 | 817.93 | 817.95 |
| 315 | Ac-LTDip$r8HYWAQL$S-NH₂ | 1673.90 | 837.95 | 838.01 |
| 316 | Ac-LTF2F$r8HYWAQL$S-NH₂ | 1615.86 | 808.93 | 809 |
| 317 | Ac-LTF3F$r8HYWAQL$S-NH₂ | 1615.86 | 808.93 | 809 |
| 318 | Ac-LTF4F$r8HYWAQL$S-NH₂ | 1615.86 | 808.93 | 809 |
| 319 | Ac-LTF41$r8HYWAQL$S-NH₂ | 1723.76 | 862.88 | 862.94 |
| 320 | Ac-LTF3Me$r8HYWAQL$S-NH₂ | 1611.88 | 806.94 | 807.07 |
| 321 | Ac-LTF4Me$r8HYWAQL$S-NH₂ | 1611.88 | 806.94 | 807 |
| 322 | Ac-LT1NaI$r8HYWAQL$S-NH₂ | 1647.88 | 824.94 | 824.98 |
| 323 | Ac-LT2NaI$r8HYWAQL$S-NH₂ | 1647.88 | 824.94 | 825.06 |
| 324 | Ac-LTF3CF3$r8HYWAQL$S-NH₂ | 1665.85 | 833.93 | 834.01 |
| 325 | Ac-LTF4NO2$r8HYWAQL$S-NH₂ | 1642.85 | 822.43 | 822.46 |
| 326 | Ac-LTF3NO2$r8HYWAQL$S-NH₂ | 1642.85 | 822.43 | 822.46 |
| 327 | Ac-LTF$r82ThiYWAQL$S-NH₂ | 1613.83 | 807.92 | 807.96 |
| 328 | Ac-LTF$r8HBipWAQL$S-NH₂ | 1657.90 | 829.95 | 830.01 |
| 329 | Ac-LTF$r8HF4tBuWAQL$S-NH₂ | 1637.93 | 819.97 | 820.02 |
| 330 | Ac-LTF$r8HF4CF3WAQ.L$S-NH₂ | 1649.86 | 825.93 | 826.02 |
| 331 | Ac-LTF$r8HF4CIWAQL$S-NH₂ | 1615.83 | 808.92 | 809.37 |
| 332 | Ac-LTF$r8HF4MeWAQL$S-NH₂ | 1595.89 | 798.95 | 799.01 |
| 333 | Ac-LTF$r8HF4BrWAQL$S-NH₂ | 1659.78 | 830.89 | 830.98 |
| 334 | Ac-LTF$r8HF4CNWAQL$S-NH₂ | 1606.87 | 804.44 | 804.56 |
| 335 | Ac-LTF$r8HF4NO2WAQ.L$S-NH₂ | 1626.86 | 814.43 | 814.55 |
| 336 | Ac-LTF$r8H1NaIWAQL$S-NH₂ | 1631.89 | 816.95 | 817.06 |
| 337 | Ac-LTF$r8H2NalWAQ.L$S-NH₂ | 1631.89 | 816.95 | 816.99 |
| 338 | Ac-LTF$r8HWAQL$S-NH₂ | 1434.80 | 718.40 | 718.49 |
| 339 | Ac-LTF$r8HY1NaIAQL$S-NH₂ | 1608.87 | 805.44 | 805.52 |
| 340 | Ac-LTF$r8HY2NaIAQL$S-NH₂ | 1608.87 | 805.44 | 805.52 |
| 341 | Ac-LTF$r8HYWAQI$S-NH₂ | 1597.87 | 799.94 | 800.07 |
| 342 | Ac-LTF$r8HYWAQNIe$S-NH₂ | 1597.87 | 799.94 | 800.44 |
| 343 | Ac-LTF$er8HYWAQL$eA-NH₂ | 1581.87 | 791.94 | 791.98 |
| 344 | Ac-LTF$r8HYWAQL$Abu-NH₂ | 1595.89 | 798.95 | 799.03 |
| 345 | Ac-LTF$r8HYWAbuQL$S-NH₂ | 1611.88 | 806.94 | 804.47 |
| 346 | Ac-LAF$r8HYWAQL$S-NH₂ | 1567.86 | 784.93 | 785.49 |
| 347 | Ac-LTF$r8NLWANIeL$Q-NH₂ | 1550.92 | 776.46 | 777.5 |
| 348 | Ac-LTF$r8ALWANIeL$Q-NH₂ | 1507.92 | 754.96 | 755.52 |
| 349 | Ac-LAF$r8NLWANIeL$Q-NH₂ | 1520.91 | 761.46 | 762.48 |
| 350 | Ac-F$r8AYWAAc3cL$A-NH₂ | 1256.70 | 629.35 | 1257.56 |
| 351 | Ac-LTF$r8AYWAAL$S-NH₂ | 1474.82 | 738.41 | 738.55 |
| 352 | Ac-LVF$r8AYWAQL$S-NH₂ | 1529.87 | 765.94 | 766 |
| 353 | Ac-LTF$r8AYWAbuQL$S-NH₂ | 1545.86 | 773.93 | 773.92 |
| 354 | Ac-LTF$r8AYWNIeQL$S-NH₂ | 1573.89 | 787.95 | 788.17 |
| 355 | Ac-LTF$r8AbuYWAQL$S-NH₂ | 1545.86 | 773.93 | 773.99 |
| 356 | Ac-LTF$r8AYWHQL$S-NH₂ | 1597.87 | 799.94 | 799.97 |
| 357 | Ac-LTF$r8AYWKQL$S-NH₂ | 1588.90 | 795.45 | 795.53 |
| 358 | Ac-LTF$r8AYWOQL$S-NH₂ | 1574.89 | 788.45 | 788.5 |
| 359 | Ac-LTF$r8AYWRQL$S-NH₂ | 1616.91 | 809.46 | 809.51 |
| 360 | Ac-LTF$r8AYWSQL$S-NH₂ | 1547.84 | 774.92 | 774.96 |
| 361 | Ac-LTF$r8AYWRAL$S-NH₂ | 1559.89 | 780.95 | 780.95 |
| 362 | Ac-LTF$r8AYWRQL$A-NH₂ | 1600.91 | 801.46 | 801.52 |
| 363 | Ac-LTF$r8AYWRAL$A-NH₂ | 1543.89 | 772.95 | 773.03 |
| 364 | Ac-LTF$r5HYWAQL$s8S-NH₂ | 1597.87 | 799.94 | 799.97 |
| 365 | Ac-LTF$HYWAQL$r8S-NH₂ | 1597.87 | 799.94 | 799.97 |
| 366 | Ac-LTF$r8HYWAAL$S-NH₂ | 1540.84 | 771.42 | 771.48 |
| 367 | Ac-LTF$r8HYWAAbuL$S-NH₂ | 1554.86 | 778.43 | 778.51 |
| 368 | Ac-LTF$r8HYWALL$S-NH₂ | 1582.89 | 792.45 | 792.49 |
| 369 | Ac-F$r8AYWHAL$A-NH₂ | 1310.72 | 656.36 | 656.4 |
| 370 | Ac-F$r8AYWAAL$A-NH₂ | 1244.70 | 623.35 | 1245.61 |
| 371 | Ac-F$r8AYWSAL$A-NH₂ | 1260.69 | 631.35 | 1261.6 |
| 372 | Ac-F$r8AYWRAL$A-NH₂ | 1329.76 | 665.88 | 1330.72 |
| 373 | Ac-F$r8AYWKAL$A-NH₂ | 1301.75 | 651.88 | 1302.67 |
| 374 | Ac-F$r8AYWOAL$A-NH₂ | 1287.74 | 644.87 | 1289.13 |
| 375 | Ac-F$r8VYWEAc3cL$A-NH₂ | 1342.73 | 672.37 | 1343.67 |
| 376 | Ac-F$r8FYWEAc3cL$A-NH₂ | 1390.73 | 696.37 | 1392.14 |
| 377 | Ac-F$r8WYWEAc3cL$A-NH₂ | 1429.74 | 715.87 | 1431.44 |
| 378 | Ac-F$r8RYWEAc3cL$A-NH₂ | 1399.77 | 700.89 | 700.95 |
| 379 | Ac-F$r8KYWEAc3cL$A-NH₂ | 1371.76 | 686.88 | 686.97 |
| 380 | Ac-F$r8ANIeWEAc3cL$A-NH₂ | 1264.72 | 633.36 | 1265.59 |
| 381 | Ac-F$r8AVWEAc3cL$A-NH₂ | 1250.71 | 626.36 | 1252.2 |
| 382 | Ac-F$r8AFWEAc3cL$A-NH₂ | 1298.71 | 650.36 | 1299.64 |
| 383 | Ac-F$r8AWWEAc3cL$A-NH₂ | 1337.72 | 669.86 | 1338.64 |
| 384 | Ac-F$r8ARWEAc3cL$A-NH₂ | 1307.74 | 654.87 | 655 |
| 385 | Ac-F$r8AKWEAc3cL$A-NH₂ | 1279.73 | 640.87 | 641.01 |
| 386 | Ac-F$r8AYWVAc3cL$A-NH₂ | 1284.73 | 643.37 | 643.38 |
| 387 | Ac-F$r8AYWFAc3cL$A-NH₂ | 1332.73 | 667.37 | 667.43 |
| 388 | Ac-F$r8AYWWAc3cL$A-NH₂ | 1371.74 | 686.87 | 686.97 |
| 389 | Ac-F$r8AYWRAc3cL$A-NH₂ | 1341.76 | 671.88 | 671.94 |
| 390 | Ac-F$r8AYWKAc3cL$A-NH₂ | 1313.75 | 657.88 | 657.88 |
| 391 | Ac-F$r8AYWEVL$A-NH₂ | 1330.73 | 666.37 | 666.47 |
| 392 | Ac-F$r8AYWEFL$A-NH₂ | 1378.73 | 690.37 | 690.44 |
| 393 | Ac-F$r8AYWEWL$A-NH₂ | 1417.74 | 709.87 | 709.91 |
| 394 | Ac-F$r8AYWERL$A-NH₂ | 1387.77 | 694.89 | 1388.66 |
| 395 | Ac-F$r8AYWEKL$A-NH₂ | 1359.76 | 680.88 | 1361.21 |
| 396 | Ac-F$r8AYWEAc3cL$V-NH₂ | 1342.73 | 672.37 | 1343.59 |
| 397 | Ac-F$r8AYWEAc3cL$F-NH₂ | 1390.73 | 696.37 | 1392.58 |
| 398 | Ac-F$r8AYWEAc3cL$W-NH₂ | 1429.74 | 715.87 | 1431.29 |
| 399 | Ac-F$r8AYWEAc3cL$R-NH₂ | 1399.77 | 700.89 | 700.95 |
| 400 | Ac-F$r8AYWEAc3cL$K-NH₂ | 1371.76 | 686.88 | 686.97 |
| 401 | Ac-F$r8AYWEAc3cL$AV-NH₂ | 1413.77 | 707.89 | 707.91 |
| 402 | Ac-F$r8AYWEAc3cL$AF-NH₂ | 1461.77 | 731.89 | 731.96 |
| 403 | Ac-F$r8AYWEAc3cL$AW-NH₂ | 1500.78 | 751.39 | 751.5 |
| 404 | Ac-F$r8AYWEAc3cL$AR-NH₂ | 1470.80 | 736.40 | 736.47 |
| 405 | Ac-F$r8AYWEAc3cL$AK-NH₂ | 1442.80 | 722.40 | 722.41 |
| 406 | Ac-F$r8AYWEAc3cL$AH-NH₂ | 1451.76 | 726.88 | 726.93 |
| 407 | Ac-LTF2NO2$r8HYWAQL$S-NH₂ | 1642.85 | 822.43 | 822.54 |
| 408 | Ac-LTA$r8HYAAQL$S-NH₂ | 1406.79 | 704.40 | 704.5 |
| 409 | Ac-LTF$r8HYAAQL$S-NH₂ | 1482.82 | 742.41 | 742.47 |
| 410 | Ac-QSQQTF$r8NLWALL$AN-NH₂ | 1966.07 | 984.04 | 984.38 |
| 411 | Ac-QAibQQTF$r8NLWALL$AN-NH₂ | 1964.09 | 983.05 | 983.42 |
| 412 | Ac-QAibQQTF$r8ALWALL$AN-NH₂ | 1921.08 | 961.54 | 961.59 |
| 413 | Ac-AAAATF$r8AAWAAL$AA-NH₂ | 1608.90 | 805.45 | 805.52 |
| 414 | Ac-F$r8AAWRAL$Q-NH₂ | 1294.76 | 648.38 | 648.48 |
| 415 | Ac-TF$r8AAWAAL$Q-NH₂ | 1310.74 | 656.37 | 1311.62 |
| 416 | Ac-TF$r8AAWRAL$A-NH₂ | 1338.78 | 670.39 | 670.46 |
| 417 | Ac-VF$r8AAWRAL$Q-NH₂ | 1393.82 | 697.91 | 697.99 |
| 418 | Ac-AF$r8AAWAAL$A-NH₂ | 1223.71 | 612.86 | 1224.67 |
| 420 | Ac-TF$r8AAWKAL$Q-NH₂ | 1367.80 | 684.90 | 684.97 |
| 421 | Ac-TF$r8AAWOAL$Q-NH₂ | 1353.78 | 677.89 | 678.01 |
| 422 | Ac-TF$r8AAWSAL$Q-NH₂ | 1326.73 | 664.37 | 664.47 |
| 423 | Ac-LTF$r8AAW RAL$Q-N H₂ | 1508.89 | 755.45 | 755.49 |
| 424 | Ac-F$r8AYWAQL$A-NH₂ | 1301.72 | 651.86 | 651.96 |
| 425 | Ac-F$r8AWWAAL$A-NH₂ | 1267.71 | 634.86 | 634.87 |
| 426 | Ac-F$r8AWWAQL$A-NH₂ | 1324.73 | 663.37 | 663.43 |
| 427 | Ac-F$r8AYWEAL$-NH₂ | 1231.66 | 616.83 | 1232.93 |
| 428 | Ac-F$r8AYWAAL$-NH₂ | 1173.66 | 587.83 | 1175.09 |
| 429 | Ac-F$r8AYWKAL$-NH₂ | 1230.72 | 616.36 | 616.44 |
| 430 | Ac-F$r8AYWOAL$-NH₂ | 1216.70 | 609.35 | 609.48 |
| 431 | Ac-F$r8AYWQAL$-NH₂ | 1230.68 | 616.34 | 616.44 |
| 432 | Ac-F$r8AYWAQL$-NH₂ | 1230.68 | 616.34 | 616.37 |
| 433 | Ac-F$r8HYWDQL$S-NH₂ | 1427.72 | 714.86 | 714.86 |
| 434 | Ac-F$r8HFWEQL$S-NH₂ | 1425.74 | 713.87 | 713.98 |
| 435 | Ac-F$r8AYWHQL$S-NH₂ | 1383.73 | 692.87 | 692.96 |
| 436 | Ac-F$r8AYWKQL$S-NH₂ | 1374.77 | 688.39 | 688.45 |
| 437 | Ac-F$r8AYWOQL$S-NH₂ | 1360.75 | 681.38 | 681.49 |
| 438 | Ac-F$r8HYWSQL$S-NH₂ | 1399.73 | 700.87 | 700.95 |
| 439 | Ac-F$r8HWWEQL$S-NH₂ | 1464.76 | 733.38 | 733.44 |
| 440 | Ac-F$r8HWWAQL$S-NH₂ | 1406.75 | 704.38 | 704.43 |
| 441 | Ac-F$r8AWWHQL$S-NH₂ | 1406.75 | 704.38 | 704.43 |
| 442 | Ac-F$r8AWWKQL$S-NH₂ | 1397.79 | 699.90 | 699.92 |
| 443 | Ac-F$r8AWWOQL$S-NH₂ | 1383.77 | 692.89 | 692.96 |
| 444 | Ac-F$r8HWWSQL$S-NH₂ | 1422.75 | 712.38 | 712.42 |
| 445 | Ac-LTF$r8NYWANIeL$Q-NH₂ | 1600.90 | 801.45 | 801.52 |
| 446 | Ac-LTF$r8NLWAQL$Q-NH₂ | 1565.90 | 783.95 | 784.06 |
| 447 | Ac-LTF$r8NYWANIeL$A-NH₂ | 1543.88 | 772.94 | 773.03 |
| 448 | Ac-LTF$r8NLWAQL$A-NH₂ | 1508.88 | 755.44 | 755.49 |
| 449 | Ac-LTF$r8AYWANIeL$Q-NH₂ | 1557.90 | 779.95 | 780.06 |
| 450 | Ac-LTF$r8ALWAQL$Q-NH₂ | 1522.89 | 762.45 | 762.45 |
| 451 | Ac-LAF$r8NYWANIeL$Q-NH₂ | 1570.89 | 786.45 | 786.5 |
| 452 | Ac-LAF$r8NLWAQL$Q-NH₂ | 1535.89 | 768.95 | 769.03 |
| 453 | Ac-LAF$r8AYWANIeL$A-NH₂ | 1470.86 | 736.43 | 736.47 |
| 454 | Ac-LAF$r8ALWAQL$A-NH₂ | 1435.86 | 718.93 | 719.01 |
| 455 | Ac-LAF$r8AYWAAL$A-NH₂ | 1428.82 | 715.41 | 715.41 |
| 456 | Ac-F$r8AYWEAc3cL$AAib-NH₂ | 1399.75 | 700.88 | 700.95 |
| 457 | Ac-F$r8AYWAQL$AA-NH₂ | 1372.75 | 687.38 | 687.78 |
| 458 | Ac-F$r8AYWAAc3cL$AA-NH₂ | 1327.73 | 664.87 | 664.84 |
| 459 | Ac-F$r8AYWSAc3cL$AA-NH₂ | 1343.73 | 672.87 | 672.9 |
| 460 | Ac-F$r8AYWEAc3cL$AS-NH₂ | 1401.73 | 701.87 | 701.84 |
| 461 | Ac-F$r8AYWEAc3cL$AT-NH₂ | 1415.75 | 708.88 | 708.87 |
| 462 | Ac-F$r8AYWEAc3cL$AL-NH₂ | 1427.79 | 714.90 | 714.94 |
| 463 | Ac-F$r8AYWEAc3cL$AQ-NH₂ | 1442.76 | 722.38 | 722.41 |
| 464 | Ac-F$r8AFWEAc3cL$AA-NH₂ | 1369.74 | 685.87 | 685.93 |
| 465 | Ac-F$r8AWWEAc3cL$AA-NH₂ | 1408.75 | 705.38 | 705.39 |
| 466 | Ac-F$r8AYWEAc3cL$SA-NH₂ | 1401.73 | 701.87 | 701.99 |
| 467 | Ac-F$r8AYWEAL$AA-NH₂ | 1373.74 | 687.87 | 687.93 |
| 468 | Ac-F$r8AYWENIeL$AA-NH₂ | 1415.79 | 708.90 | 708.94 |
| 469 | Ac-F$r8AYWEAc3cL$AbuA-NH₂ | 1399.75 | 700.88 | 700.95 |
| 470 | Ac-F$r8AYWEAc3cL$NleA-NH₂ | 1427.79 | 714.90 | 714.86 |
| 471 | Ac-F$r8AYWEAibL$NleA-NH₂ | 1429.80 | 715.90 | 715.97 |
| 472 | Ac-F$r8AYWEAL$NIeA-NH₂ | 1415.79 | 708.90 | 708.94 |
| 473 | Ac-F$r8AYWENIeL$NIeA-NH₂ | 1457.83 | 729.92 | 729.96 |
| 474 | Ac-F$r8AYWEAibL$Abu-NH₂ | 1330.73 | 666.37 | 666.39 |
| 475 | Ac-F$r8AYWENIeL$Abu-NH₂ | 1358.76 | 680.38 | 680.39 |
| 476 | Ac-F$r8AYWEAL$Abu-NH₂ | 1316.72 | 659.36 | 659.36 |
| 477 | Ac-LTF$r8AFWAQL$S-NH₂ | 1515.85 | 758.93 | 759.12 |
| 478 | Ac-LTF$r8AWWAQL$S-NH₂ | 1554.86 | 778.43 | 778.51 |
| 479 | Ac-LTF$r8AYWAQI$S-NH₂ | 1531.84 | 766.92 | 766.96 |
| 480 | Ac-LTF$r8AYWAQNIe$S-NH₂ | 1531.84 | 766.92 | 766.96 |
| 481 | Ac-LTF$r8AYWAQL$SA-NH₂ | 1602.88 | 802.44 | 802.48 |
| 482 | Ac-LTF$r8AWWAQL$A-NH₂ | 1538.87 | 770.44 | 770.89 |
| 483 | Ac-LTF$r8AYWAQI$A-NH₂ | 1515.85 | 758.93 | 759.42 |
| 484 | Ac-LTF$r8AYWAQNIe$A-NH₂ | 1515.85 | 758.93 | 759.42 |
| 485 | Ac-LTF$r8AYWAQL$AA-NH₂ | 1586.89 | 794.45 | 794.94 |
| 486 | Ac-LTF$r8HWWAQL$S-NH₂ | 1620.88 | 811.44 | 811.47 |
| 487 | Ac-LTF$r8HRWAQL$S-NH₂ | 1590.90 | 796.45 | 796.52 |
| 488 | Ac-LTF$r8HKWAQL$S-NH₂ | 1562.90 | 782.45 | 782.53 |
| 489 | Ac-LTF$r8HYWAQL$W-NH₂ | 1696.91 | 849.46 | 849.5 |
| 491 | Ac-F$r8AYWAbuAL$A-NH₂ | 1258.71 | 630.36 | 630.5 |
| 492 | Ac-F$r8AbuYWEAL$A-NH₂ | 1316.72 | 659.36 | 659.51 |
| 493 | Ac-NIePRF%r8NYWRLL%QN-NH₂ | 1954.13 | 978.07 | 978.54 |
| 494 | Ac-TSF%r8HYWAQL%S-NH₂ | 1573.83 | 787.92 | 787.98 |
| 495 | Ac-LTF%r8AYWAQL%S-NH₂ | 1533.86 | 767.93 | 768 |
| 496 | Ac-HTF$r8HYWAQL$S-NH₂ | 1621.84 | 811.92 | 811.96 |
| 497 | Ac-LHF$r8HYWAQL$S-NH₂ | 1633.88 | 817.94 | 818.02 |
| 498 | Ac-LTF$r8HHWAQL$S-NH₂ | 1571.86 | 786.93 | 786.94 |
| 499 | Ac-LTF$r8HYWHQL$S-NH₂ | 1663.89 | 832.95 | 832.38 |
| 500 | Ac-LTF$r8HYWAHL$S-NH₂ | 1606.87 | 804.44 | 804.48 |
| 501 | Ac-LTF$r8HYWAQL$H-NH₂ | 1647.89 | 824.95 | 824.98 |
| 502 | Ac-LTF$r8HYWAQL$S-NHPr | 1639.91 | 820.96 | 820.98 |
| 503 | Ac-LTF$r8HYWAQL$S-NHsBu | 1653.93 | 827.97 | 828.02 |
| 504 | Ac-LTF$r8HYWAQL$S-NHiBu | 1653.93 | 827.97 | 828.02 |
| 505 | Ac-LTF$r8HYWAQL$S-NHBn | 1687.91 | 844.96 | 844.44 |
| 506 | Ac-LTF$r8HYWAQL$S-NHPe | 1700.92 | 851.46 | 851.99 |
| 507 | Ac-LTF$r8HYWAQL$S-NHChx | 1679.94 | 840.97 | 841.04 |
| 508 | Ac-ETF$r8AYWAQL$S-NH₂ | 1547.80 | 774.90 | 774.96 |
| 509 | Ac-STF$r8AYWAQL$S-NH₂ | 1505.79 | 753.90 | 753.94 |
| 510 | Ac-LEF$r8AYWAQL$S-NH₂ | 1559.84 | 780.92 | 781.25 |
| 511 | Ac-LSF$r8AYWAQL$S-NH₂ | 1517.83 | 759.92 | 759.93 |
| 512 | Ac-LTF$r8EYWAQL$S-NH₂ | 1589.85 | 795.93 | 795.97 |
| 513 | Ac-LTF$r8SYWAQL$S-NH₂ | 1547.84 | 774.92 | 774.96 |
| 514 | Ac-LTF$r8AYWEQL$S-NH₂ | 1589.85 | 795.93 | 795.9 |
| 515 | Ac-LTF$r8AYWAEL$S-NH₂ | 1532.83 | 767.42 | 766.96 |
| 516 | Ac-LTF$r8AYWASL$S-NH₂ | 1490.82 | 746.41 | 746.46 |
| 517 | Ac-LTF$r8AYWAQL$E-NH₂ | 1573.85 | 787.93 | 787.98 |
| 518 | Ac-LTF2CN$r8HYWAQL$S-NH₂ | 1622.86 | 812.43 | 812.47 |
| 519 | Ac-LTF3CI$r8HYWAQL$S-NH₂ | 1631.83 | 816.92 | 816.99 |
| 520 | Ac-LTDip$r8HYWAQL$S-NH₂ | 1673.90 | 837.95 | 838.01 |
| 521 | Ac-LTF$r8HYWAQTIe$S-NH₂ | 1597.87 | 799.94 | 800.04 |
| 522 | Ac-F$r8AY6clWEAL$A-NH₂ | 1336.66 | 669.33 | 1338.56 |
| 523 | Ac-F$r8AYdI6brWEAL$A-NH₂ | 1380.61 | 691.31 | 692.2 |
| 524 | Ac-F$r8AYdI6fWEAL$A-NH₂ | 1320.69 | 661.35 | 1321.61 |
| 525 | Ac-F$r8AYdI4mWEAL$A-NH₂ | 1316.72 | 659.36 | 659.36 |
| 526 | Ac-F$r8AYdI5cIWEAL$A-NH₂ | 1336.66 | 669.33 | 669.35 |
| 527 | Ac-F$r8AYdI7mWEAL$A-NH₂ | 1316.72 | 659.36 | 659.36 |
| 528 | Ac-LTF%r8HYWAQL%A-NH₂ | 1583.89 | 792.95 | 793.01 |
| 529 | Ac-LTF$r8HCouWAQL$S-NH₂ | 1679.87 | 840.94 | 841.38 |
| 530 | Ac-LTFEHCouWAQLTS-NH₂ | 1617.75 | 809.88 | 809.96 |
| 531 | Ac-LTA$r8HCouWAQL$S-NH₂ | 1603.84 | 802.92 | 803.36 |
| 532 | Ac-F$r8AYWEAL$AbuA-NH₂ | 1387.75 | 694.88 | 694.88 |
| 533 | Ac-F$r8AYWEAI$AA-NH₂ | 1373.74 | 687.87 | 687.93 |
| 534 | Ac-F$r8AYWEANIe$AA-NH₂ | 1373.74 | 687.87 | 687.93 |
| 535 | Ac-F$r8AYWEAmIL$AA-NH₂ | 1429.80 | 715.90 | 715.97 |
| 536 | Ac-F$r8AYWQAL$AA-NH₂ | 1372.75 | 687.38 | 687.48 |
| 537 | Ac-F$r8AYWAAL$AA-NH₂ | 1315.73 | 658.87 | 658.92 |
| 538 | Ac-F$r8AYWAbuAL$AA-NH₂ | 1329.75 | 665.88 | 665.95 |
| 539 | Ac-F$r8AYWNIeAL$AA-NH₂ | 1357.78 | 679.89 | 679.94 |
| 540 | Ac-F$r8AbuYWEAL$AA-NH₂ | 1387.75 | 694.88 | 694.96 |
| 541 | Ac-F$r8NIeYWEAL$AA-NH₂ | 1415.79 | 708.90 | 708.94 |
| 542 | Ac-F$r8FYWEAL$AA-NH₂ | 1449.77 | 725.89 | 725.97 |
| 543 | Ac-LTF$r8HYWAQhL$S-NH₂ | 1611.88 | 806.94 | 807 |
| 544 | Ac-LTF$r8HYWAQAdm$S-NH₂ | 1675.91 | 838.96 | 839.04 |
| 545 | Ac-LTF$r8HYWAQIgI$S-NHz | 1659.88 | 830.94 | 829.94 |
| 546 | Ac-F$r8AYWAQL$AA-NH₂ | 1372.75 | 687.38 | 687.48 |
| 547 | Ac-LTF$r8ALWAQL$Q-NH₂ | 1522.89 | 762.45 | 762.52 |
| 548 | Ac-F$r8AYWEAL$AA-NH₂ | 1373.74 | 687.87 | 687.93 |
| 549 | Ac-F$r8AYWENIeL$AA-NH₂ | 1415.79 | 708.90 | 708.94 |
| 550 | Ac-F$r8AYWEAibL$Abu-NH₂ | 1330.73 | 666.37 | 666.39 |
| 551 | Ac-F$r8AYWENIeL$Abu-NH₂ | 1358.76 | 680.38 | 680.38 |
| 552 | Ac-F$r8AYWEAL$Abu-NH₂ | 1316.72 | 659.36 | 659.36 |
| 553 | Ac-F$r8AYWEAc3cL$AbuA-NH₂ | 1399.75 | 700.88 | 700.95 |
| 554 | Ac-F$r8AYWEAc3cL$NIeA-NH₂ | 1427.79 | 714.90 | 715.01 |
| 555 | H-LTF$r8AYWAQL$S-NH₂ | 1489.83 | 745.92 | 745.95 |
| 556 | mdPEG3-LTF$r8AYWAQL$S-NH₂ | 1679.92 | 840.96 | 840.97 |
| 557 | mdPEG7-LTF$r8AYWAQL$S-NH₂ | 1856.02 | 929.01 | 929.03 |
| 558 | Ac-F$r8ApmpEt6cIWEAL$A-NH₂ | 1470.71 | 736.36 | 788.17 |
| 559 | Ac-LTF3CI$r8AYWAQL$S-NH₂ | 1565.81 | 783.91 | 809.18 |
| 560 | Ac-LTF3CI$r8HYWAQL$A-NH₂ | 1615.83 | 808.92 | 875.24 |
| 561 | Ac-LTF3CI$r8HYWWQL$S-NH₂ | 1746.87 | 874.44 | 841.65 |
| 562 | Ac-LTF3CI$r8AYWWQL$S-NH₂ | 1680.85 | 841.43 | 824.63 |
| 563 | Ac-LTF$r8AYWWQL$S-NH₂ | 1646.89 | 824.45 | 849.98 |
| 564 | Ac-LTF$r8HYWWQL$A-NH₂ | 1696.91 | 849.46 | 816.67 |
| 565 | Ac-LTF$r8AYWWQL$A-NH₂ | 1630.89 | 816.45 | 776.15 |
| 566 | Ac-LTF4F$r8AYWAQL$S-NH₂ | 1549.83 | 775.92 | 776.15 |
| 567 | Ac-LTF2F$r8AYWAQL$S-NH₂ | 1549.83 | 775.92 | 776.15 |
| 568 | Ac-LTF3F$r8AYWAQL$S-NH₂ | 1549.83 | 775.92 | 785.12 |
| 569 | Ac-LTF34F2$r8AYWAQL$S-NH₂ | 1567.83 | 784.92 | 785.12 |
| 570 | Ac-LTF35F2$r8AYWAQL$S-NH₂ | 1567.83 | 784.92 | 1338.74 |
| 571 | Ac-F3CI$r8AYWEAL$A-NH₂ | 1336.66 | 669.33 | 705.28 |
| 572 | Ac-F3CI$r8AYWEAL$AA-NH₂ | 1407.70 | 704.85 | 680.11 |
| 573 | Ac-F$r8AY6cIWEAL$AA-NH₂ | 1407.70 | 704.85 | 736.83 |
| 574 | Ac-F$r8AY6cIWEAL$-NH₂ | 1265.63 | 633.82 | 784.1 |
| 575 | Ac-LTF$r8HYWAQLSt/S-NH₂ | 16.03 | 9.02 | 826.98 |
| 576 | Ac-LTF$r8HYWAQL$S-NHsBu | 1653.93 | 827.97 | 828.02 |
| 577 | Ac-STF$r8AYWAQL$S-NH₂ | 1505.79 | 753.90 | 753.94 |
| 578 | Ac-LTF$r8AYWAEL$S-NH₂ | 1532.83 | 767.42 | 767.41 |
| 579 | Ac-LTF$r8AYWAQL$E-NH₂ | 1573.85 | 787.93 | 787.98 |
| 580 | mdPEG3-LTF$r8AYWAQL$S-NH₂ | 1679.92 | 840.96 | 840.97 |
| 581 | Ac-LTF$r8AYWAQhL$S-NH₂ | 1545.86 | 773.93 | 774.31 |
| 583 | Ac-LTF$r8AYWAQCha$S-NH₂ | 1571.88 | 786.94 | 787.3 |
| 584 | Ac-LTF$r8AYWAQChg$S-NH₂ | 1557.86 | 779.93 | 780.4 |
| 585 | Ac-LTF$r8AYWAQCba$S-NH₂ | 1543.84 | 772.92 | 780.13 |
| 586 | Ac-LTF$r8AYWAQF$S-NH₂ | 1565.83 | 783.92 | 784.2 |
| 587 | Ac-LTF4F$r8HYWAQhL$S-NH₂ | 1629.87 | 815.94 | 815.36 |
| 588 | Ac-LTF4F$r8HYWAQCha$S-NH₂ | 1655.89 | 828.95 | 828.39 |
| 589 | Ac-LTF4F$r8HYWAQChg$S-NH₂ | 1641.87 | 821.94 | 821.35 |
| 590 | Ac-LTF4F$r8HYWAQCba$S-NH₂ | 1627.86 | 814.93 | 814.32 |
| 591 | Ac-LTF4F$r8AYWAQhL$S-NH₂ | 1563.85 | 782.93 | 782.36 |
| 592 | Ac-LTF4F$r8AYWAQCha$S-NH₂ | 1589.87 | 795.94 | 795.38 |
| 593 | Ac-LTF4F$r8AYWAQChg$S-NH₂ | 1575.85 | 788.93 | 788.35 |
| 594 | Ac-LTF4F$r8AYWAQCba$S-NH₂ | 1561.83 | 781.92 | 781.39 |
| 595 | Ac-LTF3CI$r8AYWAQhL$S-NH₂ | 1579.82 | 790.91 | 790.35 |
| 596 | Ac-LTF3CI$r8AYWAQCha$S-NH₂ | 1605.84 | 803.92 | 803.67 |
| 597 | Ac-LTF3CI$r8AYWAQChg$S-NH₂ | 1591.82 | 796.91 | 796.34 |
| 598 | Ac-LTF3CI$r8AYWAQCba$S-NH₂ | 1577.81 | 789.91 | 789.39 |
| 599 | Ac-LTF$r8AYWAQhF$S-NH₂ | 1579.84 | 790.92 | 791.14 |
| 600 | Ac-LTF$r8AYWAQF3CF3$S-NH₂ | 1633.82 | 817.91 | 818.15 |
| 601 | Ac-LTF$r8AYWAQF3Me$S-NH₂ | 1581.86 | 791.93 | 791.32 |
| 602 | Ac-LTF$r8AYWAQ1NaI$S-NH₂ | 1615.84 | 808.92 | 809.18 |
| 603 | Ac-LTF$r8AYWAQBip$S-NH₂ | 1641.86 | 821.93 | 822.13 |
| 604 | Ac-LTF$r8FYWAQL$A-NH₂ | 1591.88 | 796.94 | 797.33 |
| 605 | Ac-LTF$r8HYWAQL$S-NHAm | 1667.94 | 834.97 | 835.92 |
| 606 | Ac-LTF$r8HYWAQL$S-NHiAm | 1667.94 | 834.97 | 835.55 |
| 607 | Ac-LTF$r8HYWAQL$S-NHnPr3Ph | 1715.94 | 858.97 | 859.79 |
| 608 | Ac-LTF$r8HYWAQL$S-NHnBu3,3Me | 1681.96 | 841.98 | 842.49 |
| 610 | Ac-LTF$r8HYWAQL$S-NHnPr | 1639.91 | 820.96 | 821.58 |
| 611 | Ac-LTF$r8HYWAQL$S-NHnEt2Ch | 1707.98 | 854.99 | 855.35 |
| 612 | Ac-LTF$r8HYWAQL$S-NHHex | 1681.96 | 841.98 | 842.4 |
| 613 | Ac-LTF$r8AYWAQL$S-N Hmd Peg2 | 1633.91 | 817.96 | 818.35 |
| 614 | Ac-LTF$r8AYWAQL$A-N Hmd Peg2 | 1617.92 | 809.96 | 810.3 |
| 615 | Ac-LTF$r8AYWAQL$A-N Hmd Peg4 | 1705.97 | 853.99 | 854.33 |
| 616 | Ac-F$r8AYdI4mWEAL$A-NH₂ | 1316.72 | 659.36 | 659.44 |
| 617 | Ac-F$r8AYdI5cIWEAL$A-NH₂ | 1336.66 | 669.33 | 669.43 |
| 618 | Ac-LThF$r8AYWAQL$S-NH₂ | 1545.86 | 773.93 | 774.11 |
| 619 | Ac-LT2NaI$r8AYWAQL$S-NH₂ | 1581.86 | 791.93 | 792.43 |
| 620 | Ac-LTA$r8AYWAQL$S-NH₂ | 1455.81 | 728.91 | 729.15 |
| 621 | Ac-LTF$r8AYWVQL$S-NH₂ | 1559.88 | 780.94 | 781.24 |
| 622 | Ac-LTF$r8HYWAAL$A-NH₂ | 1524.85 | 763.43 | 763.86 |
| 623 | Ac-LTF$r8VYWAQL$A-NH₂ | 1543.88 | 772.94 | 773.37 |
| 624 | Ac-LTF$r8IYWAQL$S-NH₂ | 1573.89 | 787.95 | 788.17 |
| 625 | Ac-FTF$r8VYWSQL$S-NH₂ | 1609.85 | 805.93 | 806.22 |
| 626 | Ac-ITF$r8FYWAQL$S-N H₂ | 1607.88 | 804.94 | 805.2 |
| 627 | Ac-2NalTF$r8VYWSQL$S-NH₂ | 1659.87 | 830.94 | 831.2 |
| 628 | Ac-ITF$r8LYWSQL$S-NH₂ | 1589.89 | 795.95 | 796.13 |
| 629 | Ac-FTF$r8FYWAQL$S-NH₂ | 1641.86 | 821.93 | 822.13 |
| 630 | Ac-WTF$r8VYWAQL$S-NH₂ | 1632.87 | 817.44 | 817.69 |
| 631 | Ac-WTF$r8WYWAQL$S-NH₂ | 1719.88 | 860.94 | 861.36 |
| 632 | Ac-VTF$r8AYWSQL$S-NH₂ | 1533.82 | 767.91 | 768.19 |
| 633 | Ac-WTF$r8FYWSQL$S-N H₂ | 1696.87 | 849.44 | 849.7 |
| 634 | Ac-FTF$r8IYWAQL$S-NH₂ | 1607.88 | 804.94 | 805.2 |
| 635 | Ac-WTF$r8VYWSQL$S-N H₂ | 1648.87 | 825.44 | 824.8 |
| 636 | Ac-FTF$r8LYWSQL$S-NH₂ | 1623.87 | 812.94 | 812.8 |
| 637 | Ac-YTF$r8FYWSQL$S-NH₂ | 1673.85 | 837.93 | 837.8 |
| 638 | Ac-LTF$r8AY6clWEAL$A-NH₂ | 1550.79 | 776.40 | 776.14 |
| 639 | Ac-LTF$r8AY6clWSQL$S-NH₂ | 1581.80 | 791.90 | 791.68 |
| 640 | Ac-F$r8AY6clWSAL$A-N H₂ | 1294.65 | 648.33 | 647.67 |
| 641 | Ac-F$r8AY6clWQAL$AA-NH₂ | 1406.72 | 704.36 | 703.84 |
| 642 | Ac-LHF$r8AYWAQL$S-NH₂ | 1567.86 | 784.93 | 785.21 |
| 643 | Ac-LTF$r8AYWAQL$S-NH₂ | 1531.84 | 766.92 | 767.17 |
| 644 | Ac-LTF$r8AHWAQL$S-NH₂ | 1505.84 | 753.92 | 754.13 |
| 645 | Ac-LTF$r8AYWAHL$S-NH₂ | 1540.84 | 771.42 | 771.61 |
| 646 | Ac-LTF$r8AYWAQL$H-NH₂ | 1581.87 | 791.94 | 792.15 |
| 647 | H-LTF$r8AYWAQL$A-NH₂ | 1473.84 | 737.92 | 737.29 |
| 648 | Ac-HHF$r8AYWAQL$S-NH₂ | 1591.83 | 796.92 | 797.35 |
| 649 | Ac-aAi bWTF$r8VYWSQL$S-N H₂ | 1804.96 | 903.48 | 903.64 |
| 650 | Ac-AibWTF$r8HYWAQL$S-NH₂ | 1755.91 | 878.96 | 879.4 |
| 651 | Ac-AibAWTF$r8HYWAQL$S-NH₂ | 1826.95 | 914.48 | 914.7 |
| 652 | Ac-fWTF$r8HYWAQL$S-N H₂ | 1817.93 | 909.97 | 910.1 |
| 653 | Ac-AibWWTF$r8HYWAQL$S-NH₂ | 1941.99 | 972.00 | 972.2 |
| 654 | Ac-WTF$r8LYWSQL$S-N H₂ | 1662.88 | 832.44 | 832.8 |
| 655 | Ac-WTF$r8NleYWSQL$S-NH₂ | 1662.88 | 832.44 | 832.6 |
| 656 | Ac-LTF$r8AYWSQL$a-NH₂ | 1531.84 | 766.92 | 767.2 |
| 657 | Ac-LTF$r8EYWARL$A-NH₂ | 1601.90 | 801.95 | 802.1 |
| 658 | Ac-LTF$r8EYWAHL$A-NH₂ | 1582.86 | 792.43 | 792.6 |
| 659 | Ac-aTF$r8AYWAQL$S-N H₂ | 1489.80 | 745.90 | 746.08 |
| 660 | Ac-AibTF$r8AYWAQL$S-NH₂ | 1503.81 | 752.91 | 753.11 |
| 661 | Ac-AmfTF$r8AYWAQL$S-NH₂ | 1579.84 | 790.92 | 791.14 |
| 662 | Ac-AmwTF$r8AYWAQL$S-NH₂ | 1618.86 | 810.43 | 810.66 |
| 663 | Ac-NmLTF$r8AYWAQL$S-NH₂ | 1545.86 | 773.93 | 774.11 |
| 664 | Ac-LNmTF$r8AYWAQL$S-NH₂ | 1545.86 | 773.93 | 774.11 |
| 665 | Ac-LSarF$r8AYWAQL$S-NH₂ | 1501.83 | 751.92 | 752.18 |
| 667 | Ac-LGF$r8AYWAQL$S-NH₂ | 1487.82 | 744.91 | 745.15 |
| 668 | Ac-LTNmF$r8AYWAQL$S-NH₂ | 1545.86 | 773.93 | 774.2 |
| 669 | Ac-TF$r8AYWAQL$S-NH₂ | 1418.76 | 710.38 | 710.64 |
| 670 | Ac-ETF$r8AYWAQL$A-NH₂ | 1531.81 | 766.91 | 767.2 |
| 671 | Ac-LTF$r8EYWAQL$A-NH₂ | 1573.85 | 787.93 | 788.1 |
| 672 | Ac-LT2Nal$r8AYWSQL$S-NH₂ | 1597.85 | 799.93 | 800.4 |
| 673 | Ac-LTF$r8AYWAAL$S-NH₂ | 1474.82 | 738.41 | 738.68 |
| 674 | Ac-LTF$r8AYWAQhCha$S-NH₂ | 1585.89 | 793.95 | 794.19 |
| 675 | Ac-LTF$r8AYWAQChg$S-NH₂ | 1557.86 | 779.93 | 780.97 |
| 676 | Ac-LTF$r8AYWAQCba$S-N H₂ | 1543.84 | 772.92 | 773.19 |
| 677 | Ac-LTF$r8AYWAQF3CF3$S-NH₂ | 1633.82 | 817.91 | 818.15 |
| 678 | Ac-LTF$r8AYWAQ1Nal$S-NH₂ | 1615.84 | 808.92 | 809.18 |
| 679 | Ac-LTF$r8AYWAQBip$S-NH₂ | 1641.86 | 821.93 | 822.32 |
| 680 | Ac-LT2Nal$r8AYWAQL$S-NH₂ | 1581.86 | 791.93 | 792.15 |
| 681 | Ac-LTF$r8AYWVQL$S-NH₂ | 1559.88 | 780.94 | 781.62 |
| 682 | Ac-LTF$r8AWWAQL$S-NH₂ | 1554.86 | 778.43 | 778.65 |
| 683 | Ac-FTF$r8VYWSQL$S-NH₂ | 1609.85 | 805.93 | 806.12 |
| 684 | Ac-ITF$r8FYWAQL$S-NH₂ | 1607.88 | 804.94 | 805.2 |
| 685 | Ac-ITF$r8LYWSQL$S-NH₂ | 1589.89 | 795.95 | 796.22 |
| 686 | Ac-FTF$r8FYWAQL$S-NH₂ | 1641.86 | 821.93 | 822.41 |
| 687 | Ac-VTF$r8AYWSQL$S-N H₂ | 1533.82 | 767.91 | 768.19 |
| 688 | Ac-LTF$r8AHWAQL$S-NH₂ | 1505.84 | 753.92 | 754.31 |
| 689 | Ac-LTF$r8AYWAQL$H-NH₂ | 1581.87 | 791.94 | 791.94 |
| 690 | Ac-LTF$r8AYWAHL$S-NH₂ | 1540.84 | 771.42 | 771.61 |
| 691 | Ac-aAi bWTF$r8VYWSQL$S-N H₂ | 1804.96 | 903.48 | 903.9 |
| 692 | Ac-AibWTF$r8HYWAQL$S-NH₂ | 1755.91 | 878.96 | 879.5 |
| 693 | Ac-AibAWTF$r8HYWAQL$S-NH₂ | 1826.95 | 914.48 | 914.7 |
| 694 | Ac-fWTF$r8HYWAQL$S-NH₂ | 1817.93 | 909.97 | 910.2 |
| 695 | Ac-AibWWTF$r8HYWAQL$S-NH₂ | 1941.99 | 972.00 | 972.7 |
| 696 | Ac-WTF$r8LYWSQL$S-N H₂ | 1662.88 | 832.44 | 832.7 |
| 697 | Ac-WTF$r8NleYWSQL$S-NH₂ | 1662.88 | 832.44 | 832.7 |
| 698 | Ac-LTF$r8AYWSQL$a-NH₂ | 1531.84 | 766.92 | 767.2 |
| 699 | Ac-LTF$r8EYWARL$A-NH₂ | 1601.90 | 801.95 | 802.2 |
| 700 | Ac-LTF$r8EYWAHL$A-NH₂ | 1582.86 | 792.43 | 792.6 |
| 701 | Ac-aTF$r8AYWAQL$S-NH₂ | 1489.80 | 745.90 | 746.1 |
| 702 | Ac-AibTF$r8AYWAQL$S-NH₂ | 1503.81 | 752.91 | 753.2 |
| 703 | Ac-AmfTF$r8AYWAQL$S-NH₂ | 1579.84 | 790.92 | 791.2 |
| 704 | Ac-AmwTF$r8AYWAQL$S-NH₂ | 1618.86 | 810.43 | 810.7 |
| 705 | Ac-NmLTF$r8AYWAQL$S-NH₂ | 1545.86 | 773.93 | 774.1 |
| 706 | Ac-LNmTF$r8AYWAQL$S-NH₂ | 1545.86 | 773.93 | 774.4 |
| 707 | Ac-LSarF$r8AYWAQL$S-NH₂ | 1501.83 | 751.92 | 752.1 |
| 708 | Ac-TF$r8AYWAQL$S-NH₂ | 1418.76 | 710.38 | 710.8 |
| 709 | Ac-ETF$r8AYWAQL$A-NH₂ | 1531.81 | 766.91 | 767.4 |
| 710 | Ac-LTF$r8EYWAQL$A-NH₂ | 1573.85 | 787.93 | 788.2 |
| 711 | Ac-WTF$r8VYWSQL$S-NH₂ | 1648.87 | 825.44 | 825.2 |
| 713 | Ac-YTF$r8FYWSQL$S-NH₂ | 1673.85 | 837.93 | 837.3 |
| 714 | Ac-F$r8AY6clWSAL$A-N H₂ | 1294.65 | 648.33 | 647.74 |
| 715 | Ac-ETF$r8EYWVQL$S-NH₂ | 1633.84 | 817.92 | 817.36 |
| 716 | Ac-ETF$r8EHWAQL$A-NH₂ | 1563.81 | 782.91 | 782.36 |
| 717 | Ac-ITF$r8EYWAQL$S-NH₂ | 1589.85 | 795.93 | 795.38 |
| 718 | Ac-ITF$r8EHWVQL$A-NH₂ | 1575.88 | 788.94 | 788.42 |
| 719 | Ac-ITF$r8EHWAQL$S-NH₂ | 1563.85 | 782.93 | 782.43 |
| 720 | Ac-LTF4F$r8AYWAQCba$S-NH₂ | 1561.83 | 781.92 | 781.32 |
| 721 | Ac-LTF3CI$r8AYWAQhL$S-NH₂ | 1579.82 | 790.91 | 790.64 |
| 722 | Ac-LTF3CI$r8AYWAQCha$S-NH₂ | 1605.84 | 803.92 | 803.37 |
| 723 | Ac-LTF3CI$r8AYWAQChg$S-NH₂ | 1591.82 | 796.91 | 796.27 |
| 724 | Ac-LTF3CI$r8AYWAQCba$S-NH₂ | 1577.81 | 789.91 | 789.83 |
| 725 | Ac-LTF$r8AY6clWSQL$S-N H₂ | 1581.80 | 791.90 | 791.75 |
| 726 | Ac-LTF4F$r8HYWAQhL$S-NH₂ | 1629.87 | 815.94 | 815.36 |
| 727 | Ac-LTF4F$r8HYWAQCba$S-NH₂ | 1627.86 | 814.93 | 814.32 |
| 728 | Ac-LTF4F$r8AYWAQhL$S-NH₂ | 1563.85 | 782.93 | 782.36 |
| 729 | Ac-LTF4F$r8AYWAQChg$S-NH₂ | 1575.85 | 788.93 | 788.35 |
| 730 | Ac-ETF$r8EYWVAL$S-NH₂ | 1576.82 | 789.41 | 788.79 |
| 731 | Ac-ETF$r8EHWAAL$A-NH₂ | 1506.79 | 754.40 | 754.8 |
| 732 | Ac-ITF$r8EYWAAL$S-NH₂ | 1532.83 | 767.42 | 767.75 |
| 733 | Ac-ITF$r8EHWVAL$A-NH₂ | 1518.86 | 760.43 | 760.81 |
| 734 | Ac-ITF$r8EHWAAL$S-NH₂ | 1506.82 | 754.41 | 754.8 |
| 735 | Pam-LTF$r8EYWAQL$S-NH₂ | 1786.07 | 894.04 | 894.48 |
| 736 | Pam-ETF$r8EYWAQL$S-NH₂ | 1802.03 | 902.02 | 902.34 |
| 737 | Ac-LTF$r8AYWLQL$S-NH₂ | 1573.89 | 787.95 | 787.39 |
| 738 | Ac-LTF$r8EYWLQL$S-NH₂ | 1631.90 | 816.95 | 817.33 |
| 739 | Ac-LTF$r8EHWLQL$S-NH₂ | 1605.89 | 803.95 | 804.29 |
| 740 | Ac-LTF$r8VYWAQL$S-NH₂ | 1559.88 | 780.94 | 781.34 |
| 741 | Ac-LTF$r8AYWSQL$S-NH₂ | 1547.84 | 774.92 | 775.33 |
| 742 | Ac-ETF$r8AYWAQL$S-NH₂ | 1547.80 | 774.90 | 775.7 |
| 743 | Ac-LTF$r8EYWAQL$S-NH₂ | 1589.85 | 795.93 | 796.33 |
| 744 | Ac-LTF$r8HYWAQL$S-N HAm | 1667.94 | 834.97 | 835.37 |
| 745 | Ac-LTF$r8HYWAQL$S-NHiAm | 1667.94 | 834.97 | 835.27 |
| 746 | Ac-LTF$r8HYWAQL$S-NHnPr3Ph | 1715.94 | 858.97 | 859.42 |
| 747 | Ac-LTF$r8HYWAQL$S-NHnBu3,3Me | 1681.96 | 841.98 | 842.67 |
| 748 | Ac-LTF$r8HYWAQL$S-N H n Bu | 1653.93 | 827.97 | 828.24 |
| 749 | Ac-LTF$r8HYWAQL$S-N H n Pr | 1639.91 | 820.96 | 821.31 |
| 750 | Ac-LTF$r8HYWAQL$S-N H n Et2Ch | 1707.98 | 854.99 | 855.35 |
| 751 | Ac-LTF$r8HYWAQL$S-NHHex | 1681.96 | 841.98 | 842.4 |
| 752 | Ac-LTF$r8AYWAQL$S-NHmdPeg2 | 1633.91 | 817.96 | 855.35 |
| 753 | Ac-LTF$r8AYWAQL$A-NHmdPeg2 | 1617.92 | 809.96 | 810.58 |
| 754 | Ac-LTF$r5AYWAAL$s8S-NH₂ | 1474.82 | 738.41 | 738.79 |
| 755 | Ac-LTF$r8AYWCouQL$S-NH₂ | 1705.88 | 853.94 | 854.61 |
| 756 | Ac-LTF$r8CouYWAQL$S-NH₂ | 1705.88 | 853.94 | 854.7 |
| 757 | Ac-CouTF$r8AYWAQL$S-NH₂ | 1663.83 | 832.92 | 833.33 |
| 758 | H-LTF$r8AYWAQL$A-NH₂ | 1473.84 | 737.92 | 737.29 |
| 759 | Ac-HHF$r8AYWAQL$S-NH₂ | 1591.83 | 796.92 | 797.72 |
| 760 | Ac-LT2Nal$r8AYWSQL$S-NH₂ | 1597.85 | 799.93 | 800.68 |
| 761 | Ac-LTF$r8HCouWAQL$S-NH₂ | 1679.87 | 840.94 | 841.38 |
| 762 | Ac-LTF$r8AYWCou2QL$S-NH₂ | 1789.94 | 895.97 | 896.51 |
| 763 | Ac-LTF$r8Cou2YWAQL$S-NH₂ | 1789.94 | 895.97 | 896.5 |
| 764 | Ac-Cou2TF$r8AYWAQL$S-NH₂ | 1747.90 | 874.95 | 875.42 |
| 765 | Ac-LTF$r8ACou2WAQL$S-NH₂ | 1697.92 | 849.96 | 850.82 |
| 766 | Dmaac-LTF$r8AYWAQL$S-NH₂ | 1574.89 | 788.45 | 788.82 |
| 767 | Hexac-LTF$r8AYWAQL$S-NH₂ | 1587.91 | 794.96 | 795.11 |
| 768 | Napac-LTF$r8AYWAQL$S-NH₂ | 1657.89 | 829.95 | 830.36 |
| 769 | Pam-LTF$r8AYWAQL$S-NH₂ | 1728.06 | 865.03 | 865.45 |
| 770 | Ac-LT2Nal$r8HYAAQL$S-NH₂ | 1532.84 | 767.42 | 767.61 |
| 771 | Ac-LT2Nal$/r8HYWAQL$/S-NH₂ | 1675.91 | 838.96 | 839.1 |
| 772 | Ac-LT2Nal$r8HYFAQL$S-NH₂ | 1608.87 | 805.44 | 805.9 |
| 773 | Ac-LT2Nal$r8HWAAQL$S-NH₂ | 1555.86 | 778.93 | 779.08 |
| 774 | Ac-LT2Nal$r8HYAWQL$S-NH₂ | 1647.88 | 824.94 | 825.04 |
| 775 | Ac-LT2Nal$r8HYAAQW$S-NH₂ | 1605.83 | 803.92 | 804.05 |
| 776 | Ac-LTW$r8HYWAQL$S-NH₂ | 1636.88 | 819.44 | 819.95 |
| 777 | Ac-LT1Nal$r8HYWAQL$S-N H₂ | 1647.88 | 824.94 | 825.41 |

In some cases, a peptidomimetic macrocycles disclosed herein does not comprise a peptidomimetic macrocycle structure as shown in Table 2b.

Table 2c shows examples of non-crosslinked polypeptides comprising D-amino acids.

**Table 2c**

| **SP** | **Sequence** | **Isomer** | **Exact Mass** | **Found Mass** | **Calc (M+1)/1** | **Calc (M+2)/2** | **Calc (M+3)/3** |
|---|---|---|---|---|---|---|---|
| SP765 | Ac-tawyanfekllr-NH₂ | | | 777.46 | | | |
| SP766 | Ac-tawyanf4CF3ekllr-NH₂ | | | 811.41 | | | |

### Example 3: X-ray co-crystallography of peptidomimetic macrocycles in complex with MDMX

For co-crystallization with peptide 46 (Table 2b), a stoichiometric amount of compound from a 100 mM stock solution in DMSO was added to the zebrafish MDMX protein solution and allowed to sit overnight at 4°C before setting up crystallization experiments. Procedures were similar to those described by Popowicz et al. with some variations, as noted below. Protein (residues 15-129, L46V/V95L) was obtained from an E. *coli* BL21(DE3) expression system using the pET15b vector. Cells were grown at 37°C and induced with 1 mM IPTG at an OD₆₀₀ of 0.7. Cells were allowed to grow an additional 18 hr at 23°C. Protein was purified using Ni-NT Agarose followed by Superdex 75 buffered with 50 mM NaPO₄, pH 8.0, 150 mM NaCl, 2 mM TCEP and then concentrated to 24 mg/ml. The buffer was exchanged to 20 mM Tris, pH 8.0, 50 mM NaCl, 2 mM DTT for crystallization experiments. Initial crystals were obtained with the Nextal (Qiagen) AMS screen #94 and the final optimized reservoir was 2.6 M AMS, 75 mM Hepes, pH 7.5. Crystals grew routinely as thin plates at 4°C and were cryo-protected by pulling them through a solution containing concentrated (3.4 M) malonate followed by flash cooling, storage, and shipment in liquid nitrogen.

Data collection was performed at the APS at beamline 31-ID (SGX-CAT) at 100°K and wavelength 0.97929Å. The beamline was equipped with a Rayonix 225-HE detector. For data collection, crystals were rotated through 180° in 1° increments using 0.8 second exposure times. Data were processed and reduced using Mosflm/scala (CCP4; see The CCP4 Suite: Programs for Protein Crystallography. Acta Crystallogr. D50, 760-763 (1994); P.R. Evans. Joint CCP4 and ESF-EACBM Newsletter 33, 22-24 (1997)) in space group C2 (unit cell: a = 109.2786, b = 81.0836, c = 30.9058Å, α = 90, β = 89.8577, γ = 90°). Molecular replacement with program Molrep (CCP4; see A.Vagin & A. Teplyakov. J. Appl. Cryst. 30, 1022-1025 (1997)) was performed with the MDMX component of the structure determined by Popowicz et al. (2Z5S; see G.M. Popowicz, A. Czama, U. Rothweiler, A. Szwagierczak, M. Krajewski, L. Weber & T.A. Holak. Cell Cycle 6, 2386-2392 (2007)) and identified two molecules in the asymmetric unit. Initial refinement of just the two molecules of the zebrafish MDMX with program Refmac (CCP4; see G.N. Murshudov, A.A. Vagin & E.J. Dodson. Acta Crystallogr. D53, 240-255 (1997)) resulted in an R-factor of 0.3424 (R_{free} = 0.3712) and rmsd values for bonds (0.018 Å) and angles (1.698°). The electron density for the stapled peptide components, starting with Gln¹⁹ and including all of the aliphatic staple, was very clear. Further refinement with CNX (Accelrys) using data to 2.3 Å resolution resulted in a model (comprised of 1448 atoms from MDMX, 272 atoms from the stapled peptides and 46 water molecules) that is well refined (R_{f} = 0.2601, R_{free} = 0.3162, rmsd bonds = 0.007 Å and rmsd angles = 0.916°).

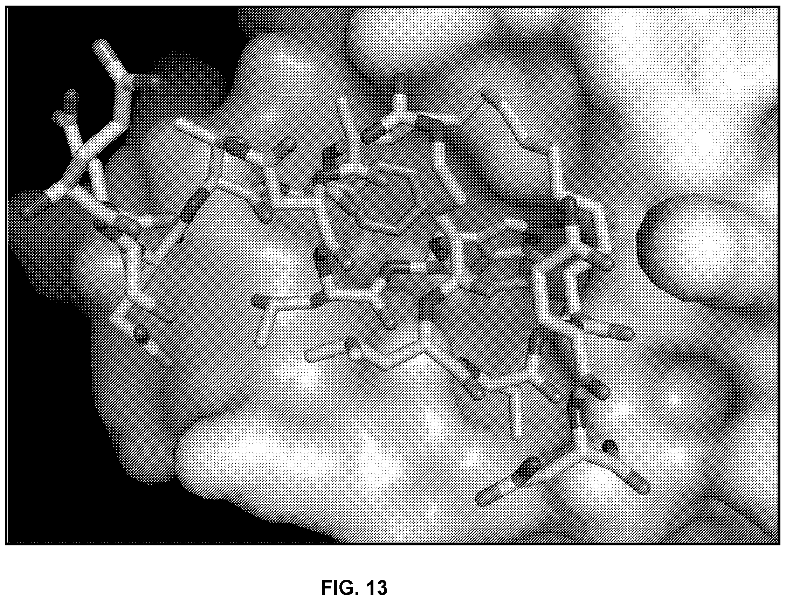

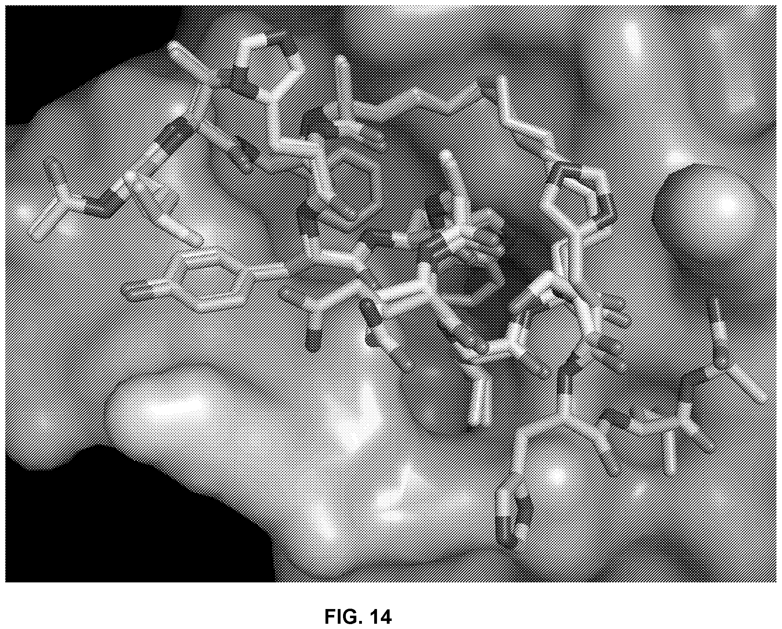

Results from this Example are shown in Figures 13 and 14.

### Example 4: Circular Dichroism (CD) analysis of alpha-helicity

Peptide solutions were analyzed by CD spectroscopy using a Jasco J-815 spectropolarimeter (Jasco Inc., Easton, MD) with the Jasco Spectra Manager Ver.2 system software. A Peltier temperature controller was used to maintain temperature control of the optical cell. Results are expressed as mean molar ellipticity [0] (deg cm² dmol⁻¹) as calculated from the equation [θ]=θobs·MRW/10^{∗}1^{∗}c where θobs is the observed ellipticity in millidegrees, MRW is the mean residue weight of the peptide (peptide molecular weight/number of residues), 1 is the optical path length of the cell in centimeters, and c is the peptide concentration in mg/ml. Peptide concentrations were determined by amino acid analysis. Stock solutions of peptides were prepared in benign CD buffer (20 mM phosphoric acid, pH 2). The stocks were used to prepare peptide solutions of 0.05 mg/ml in either benign CD buffer or CD buffer with 50% trifluoroethanol (TFE) for analyses in a 10 mm pathlength cell. Variable wavelength measurements of peptide solutions were scanned at 4 °C from 195 to 250 nm, in 0.2 nm increments, and a scan rate 50 nm per minute. The average of six scans was reported.

Table 3 shows circular dichroism data for selected peptidomimetic macrocycles:

**Table 3**

| **SP#** | **Molar Ellipticity Benign (222 in 0%TFE)** | **Molar Ellipticity 50%TFE (222 in 50%TFE)** | **Molar Ellipticity TFE - Molar Ellipticity Benign** | **% Helix 50% TFE compared to 50%TFE parent (CD)** | **% Helix benign compared to 50%TFE parent (CD)** |
|---|---|---|---|---|---|
| 7 | 124 | -19921.4 | -20045.4 | 137.3 | -0.9 |
| 11 | -398.2 | -16623.4 | 16225.2 | 106.1 | 2.5 |
| 41 | -909 | -21319.4 | 20410.4 | 136 | 5.8 |
| 43 | -15334.5 | -18247.4 | 2912.9 | 116.4 | 97.8 |
| 69 | -102.6 | -21509.7 | -21407.1 | 148.2 | 0.7 |
| 71 | -121.2 | -17957 | -17835.9 | 123.7 | 0.8 |
| 154 | -916.2 | -30965.1 | -30048.9 | 213.4 | 6.3 |
| 230 | -213.2 | -17974 | -17760.8 | 123.9 | 1.5 |
| 233 | -477.9 | -19032.6 | -18554.7 | 131.2 | 3.3 |

### Example 5: Direct binding assay MDM2 with Fluorescence polarization (FP)

The assay was performed according to the following general protocol:
1. Dilute MDM2 (In-house, 41kD) into FP buffer (High salt buffer-200 mM Nacl,5 mM CHAPS, pH 7.5) to make 10 µM working stock solution.
2. Add 30 µl of 10 µM of protein stock solution into A1 and B1 well of 96-well black HE microplate (Molecular Devices).
3. Fill in 30 µl of FP buffer into column A2 to A12, B2 to B12, C1 to C12, and D1 to D 12.
4. 2 or 3 fold series dilution of protein stock from A1, B1 into A2, B2; A2, B2 to A3, B3; ... to reach the single digit nM concentration at the last dilution point.
5. Dilute 1mM (in 100% DMSO) of FAM labeled linear peptide with DMSO to 100 µM (dilution 1: 10). Then, dilute from 100 µM to 10 µM with water (dilution 1:10) and then dilute with FP buffer from 10 µM to 40 nM (dilution 1:250). This is the working solution which will be a 10 nM concentration in well (dilution 1:4). Keep the diluted FAM labeled peptide in the dark until use.
6. Add 10 µl of 10 nM of FAM labeled peptide into each well and incubate, and read at different time points. K_{D} with 5-FAM-BaLTFEHYWAQLTS-NH₂ is -13.38 nM.

### Example 6: Competitive Fluorescence polarization assay for MDM2

The assay was performed according to the following general protocol:
1. Dilute MDM2 (In-house, 41kD) into FP buffer (High salt buffer-200 mM Nacl,5 mM CHAPS, pH 7.5) to make 84 nM (2X) working stock solution.
2. Add 20µl of 84 nM (2X) of protein stock solution into each well of 96-well black HE microplate (Molecular Devices)
3. Dilute 1 mM (in 100% DMSO) of FAM labeled linear peptide with DMSO to 100 µM (dilution 1: 10). Then, dilute from 100 µM to 10 µM with water (dilution 1:10) and then dilute with FP buffer from 10 µM to 40 nM (dilution 1:250). This is the working solution which will be a 10 nM concentration in well (dilution 1:4). Keep the diluted FAM labeled peptide in the dark until use.
4. Make unlabeled peptide dose plate with FP buffer starting with 1 µM (final) of peptide and making 5 fold serial dilutions for 6 points using following dilution scheme. Dilute 10mM (in 100% DMSO) with DMSO to 5mM (dilution 1: 2). Then, dilute from 5mM to 500 µM with H₂O (dilution 1:10) and then dilute with FP buffer from 500 µM to 20 µM (dilution 1:25). Making 5 fold serial dilutions from 4 µM (4X) for 6 points.
5. Transfer 10 µl of serial diluted unlabeled peptides to each well which is filled with 20µl of 84nM of protein.
6. Add 10 µl of 10 nM (4X) of FAM labeled peptide into each well and incubate for 3hr to read.

### Example 7: Direct binding assay MDMX with Fluorescence polarization (FP)

The assay was performed according to the following general protocol:
1. Dilute MDMX (In-house, 40kD) into FP buffer (High salt buffer-200 mM Nacl,5 mM CHAPS, pH 7.5) to make 10 µM working stock solution.
2. Add 30 µl of 10 µM of protein stock solution into A1 and B1 well of 96-well black HE microplate (Molecular Devices).
3. Fill in 30 µl of FP buffer into column A2 to A12, B2 to B12, C1 to C12, and D1 to D 12.
4. 2 or 3 fold series dilution of protein stock from A1, B1 into A2, B2; A2, B2 to A3, B3; ... to reach the single digit nM concentration at the last dilution point.
5. Dilute 1mM (in 100% DMSO) of FAM labeled linear peptide with DMSO to 100 µM (dilution 1: 10). Then, dilute from 100 µM to 10 µM with water (dilution 1:10) and then dilute with FP buffer from 10 µM to 40 nM (dilution 1:250). This is the working solution which will be a 10nM concentration in well (dilution 1:4). Keep the diluted FAM labeled peptide in the dark until use.
6. Add 10 µl of 10 nM of FAM labeled peptide into each well and incubate, and read at different time points. K_{D} with 5-FAM-BaLTFEHYWAQLTS-NH₂ is ~51 nM.

### Example 8: Competitive Fluorescence polarization assay for MDMX

The assay was performed according to the following general protocol:
1. Dilute MDMX (In-house, 40kD) into FP buffer (High salt buffer-200 mM NaCl, 5 mM CHAPS, pH 7.5.) to make 300 nM (2X) working stock solution.
2. Add 20 µl of 300 nM (2X) of protein stock solution into each well of 96-well black HE microplate (Molecular Devices)
3. Dilute 1mM (in 100% DMSO) of FAM labeled linear peptide with DMSO to 100 µM (dilution 1: 10). Then, dilute from 100 µM to 10 µM with water (dilution 1:10) and then dilute with FP buffer from 10 µM to 40 nM (dilution 1:250). This is the working solution which will be a 10 nM concentration in well (dilution 1:4). Keep the diluted FAM labeled peptide in the dark until use.
4. Make unlabeled peptide dose plate with FP buffer starting with 5 µM (final) of peptide and making 5 fold serial dilutions for 6 points using following dilution scheme.
5. Dilute 10 mM (in 100% DMSO) with DMSO to 5 mM (dilution 1: 2). Then, dilute from 5 mM to 500 µM with H₂O (dilution 1:10) and then dilute with FP buffer from 500 µM to 20 µM (dilution 1:25). Making 5 fold serial dilutions from 20 µM (4X) for 6 points.
6. Transfer 10 µl of serial diluted unlabeled peptides to each well which is filled with 20 µl of 300 nM of protein.
7. Add 10µl of 10nM (4X) of FAM labeled peptide into each well and incubate for 3hr to read.

Results from Examples 5-8 are shown in Table 4. The following scale is used: "+" represents a value greater than 1000 nM, "++" represents a value greater than 100 and less than or equal to 1000 nM, "+++" represents a value greater than 10 nM and less than or equal to 100 nM, and "++++" represents a value of less than or equal to 10 nM.

**Table 4**

| SP# | IC₅₀ (MDM2) | IC₅₀ (MDMX) | Ki (MDM2) | Ki (MDMX) |
|---|---|---|---|---|
| 3 | ++ | ++ | +++ | +++ |
| 4 | +++ | ++ | ++++ | +++ |
| 5 | +++ | ++ | ++++ | +++ |
| 6 | ++ | ++ | +++ | +++ |
| 7 | +++ | +++ | ++++ | +++ |
| 8 | ++ | ++ | +++ | +++ |
| 9 | ++ | ++ | +++ | +++ |
| 10 | ++ | ++ | +++ | +++ |
| 11 | +++ | ++ | ++++ | +++ |
| 12 | + | + | +++ | ++ |
| 13 | ++ | ++ | +++ | ++ |
| 14 | +++ | +++ | ++++ | ++++ |
| 15 | +++ | ++ | +++ | +++ |
| 16 | +++ | +++ | ++++ | +++ |
| 17 | +++ | +++ | ++++ | +++ |
| 18 | +++ | +++ | ++++ | ++++ |
| 19 | ++ | +++ | +++ | +++ |
| 20 | ++ | ++ | +++ | +++ |
| 21 | ++ | +++ | +++ | +++ |
| 22 | +++ | +++ | ++++ | +++ |
| 23 | ++ | ++ | +++ | +++ |
| 24 | +++ | ++ | ++++ | +++ |
| 26 | +++ | ++ | ++++ | +++ |
| 28 | +++ | +++ | ++++ | +++ |
| 30 | ++ | ++ | +++ | +++ |
| 32 | +++ | ++ | ++++ | +++ |
| 38 | + | ++ | ++ | +++ |
| 39 | + | ++ | ++ | ++ |
| 40 | ++ | ++ | ++ | +++ |
| 41 | ++ | +++ | +++ | +++ |
| 42 | ++ | ++ | +++ | ++ |
| 43 | +++ | +++ | ++++ | +++ |
| 45 | +++ | +++ | ++++ | ++++ |
| 46 | +++ | +++ | ++++ | +++ |
| 47 | ++ | ++ | +++ | +++ |
| 48 | ++ | ++ | +++ | +++ |
| 49 | ++ | ++ | +++ | +++ |
| 50 | +++ | ++ | ++++ | +++ |
| 52 | +++ | +++ | ++++ | ++++ |
| 54 | ++ | ++ | +++ | +++ |
| 55 | + | + | ++ | ++ |
| 65 | +++ | ++ | ++++ | +++ |
| 68 | ++ | ++ | +++ | +++ |
| 69 | +++ | ++ | ++++ | +++ |
| 70 | ++ | ++ | ++++ | +++ |
| 71 | +++ | ++ | ++++ | +++ |
| 75 | +++ | ++ | ++++ | +++ |
| 77 | +++ | ++ | ++++ | +++ |
| 80 | +++ | ++ | ++++ | +++ |
| 81 | ++ | ++ | +++ | +++ |
| 82 | ++ | ++ | +++ | +++ |
| 85 | +++ | ++ | ++++ | +++ |
| 99 | ++++ | ++ | ++++ | +++ |
| 100 | ++ | ++ | ++++ | +++ |
| 101 | +++ | ++ | ++++ | +++ |
| 102 | ++ | ++ | ++++ | +++ |
| 103 | ++ | ++ | ++++ | +++ |
| 104 | +++ | ++ | ++++ | +++ |
| 105 | +++ | ++ | ++++ | +++ |
| 106 | ++ | ++ | +++ | +++ |
| 107 | ++ | ++ | +++ | +++ |
| 108 | +++ | ++ | ++++ | +++ |
| 109 | +++ | ++ | ++++ | +++ |
| 110 | ++ | ++ | ++++ | +++ |
| 111 | ++ | ++ | ++++ | +++ |
| 112 | ++ | ++ | +++ | +++ |
| 113 | ++ | ++ | +++ | +++ |
| 114 | +++ | ++ | ++++ | +++ |
| 115 | ++++ | ++ | ++++ | +++ |
| 116 | + | + | ++ | ++ |
| 118 | ++++ | ++ | ++++ | +++ |
| 120 | +++ | ++ | ++++ | +++ |
| 121 | ++++ | ++ | ++++ | +++ |
| 122 | ++++ | ++ | ++++ | +++ |
| 123 | ++++ | ++ | ++++ | +++ |
| 124 | ++++ | ++ | ++++ | +++ |
| 125 | ++++ | ++ | ++++ | +++ |
| 126 | ++++ | ++ | ++++ | +++ |
| 127 | ++++ | ++ | ++++ | +++ |
| 128 | ++++ | ++ | ++++ | +++ |
| 129 | ++++ | ++ | ++++ | +++ |
| 130 | ++++ | ++ | ++++ | +++ |
| 133 | ++++ | ++ | ++++ | +++ |
| 134 | ++++ | ++ | ++++ | +++ |
| 135 | ++++ | ++ | ++++ | +++ |
| 136 | ++++ | ++ | ++++ | +++ |
| 137 | ++++ | ++ | ++++ | +++ |
| 139 | ++++ | ++ | ++++ | +++ |
| 142 | ++++ | +++ | ++++ | +++ |
| 144 | ++++ | ++ | ++++ | +++ |
| 146 | ++++ | ++ | ++++ | +++ |
| 148 | ++++ | ++ | ++++ | +++ |
| 150 | ++++ | ++ | ++++ | +++ |
| 153 | ++++ | +++ | ++++ | +++ |
| 154 | ++++ | +++ | ++++ | ++++ |
| 156 | ++++ | ++ | ++++ | +++ |
| 158 | ++++ | ++ | ++++ | +++ |
| 160 | ++++ | ++ | ++++ | +++ |
| 161 | ++++ | ++ | ++++ | +++ |
| 166 | ++++ | ++ | ++++ | +++ |
| 167 | +++ | ++ | ++++ | ++ |
| 169 | ++++ | +++ | ++++ | +++ |
| 170 | ++++ | ++ | ++++ | +++ |
| 173 | ++++ | ++ | ++++ | +++ |
| 175 | ++++ | ++ | ++++ | +++ |
| 177 | +++ | ++ | ++++ | +++ |
| 180 | +++ | ++ | ++++ | +++ |
| 182 | ++++ | ++ | ++++ | +++ |
| 185 | +++ | + | ++++ | ++ |
| 186 | +++ | ++ | ++++ | +++ |
| 189 | +++ | ++ | ++++ | +++ |
| 192 | +++ | ++ | ++++ | +++ |
| 194 | +++ | ++ | ++++ | ++ |
| 196 | +++ | ++ | ++++ | +++ |
| 197 | ++++ | ++ | ++++ | +++ |
| 199 | +++ | ++ | ++++ | ++ |
| 201 | +++ | ++ | ++++ | ++ |
| 203 | +++ | ++ | ++++ | +++ |
| 204 | +++ | ++ | ++++ | +++ |
| 206 | +++ | ++ | ++++ | +++ |
| 207 | ++++ | ++ | ++++ | +++ |
| 210 | ++++ | ++ | ++++ | +++ |
| 211 | ++++ | ++ | ++++ | +++ |
| 213 | ++++ | ++ | ++++ | +++ |
| 215 | +++ | ++ | ++++ | +++ |
| 217 | ++++ | ++ | ++++ | +++ |
| 218 | ++++ | ++ | ++++ | +++ |
| 221 | ++++ | +++ | ++++ | +++ |
| 227 | ++++ | ++ | ++++ | +++ |
| 230 | ++++ | +++ | ++++ | ++++ |
| 232 | ++++ | ++ | ++++ | +++ |
| 233 | ++++ | +++ | ++++ | +++ |
| 236 | +++ | ++ | ++++ | +++ |
| 237 | +++ | ++ | ++++ | +++ |
| 238 | +++ | +++ | ++++ | +++ |
| 239 | +++ | ++ | +++ | +++ |
| 240 | +++ | ++ | ++++ | +++ |
| 241 | +++ | ++ | ++++ | +++ |
| 242 | +++ | ++ | ++++ | +++ |
| 243 | +++ | +++ | ++++ | +++ |
| 244 | +++ | +++ | ++++ | ++++ |
| 245 | +++ | +++ | ++++ | +++ |
| 246 | +++ | ++ | ++++ | +++ |
| 247 | +++ | +++ | ++++ | +++ |
| 248 | +++ | +++ | ++++ | +++ |
| 249 | +++ | +++ | ++++ | ++++ |
| 250 | ++ | + | ++ | + |
| 252 | ++ | + | ++ | + |
| 254 | +++ | ++ | ++++ | +++ |
| 255 | +++ | +++ | ++++ | +++ |
| 256 | +++ | +++ | ++++ | +++ |
| 257 | +++ | +++ | ++++ | +++ |
| 258 | +++ | ++ | ++++ | +++ |
| 259 | +++ | +++ | ++++ | +++ |
| 260 | +++ | +++ | ++++ | +++ |
| 261 | +++ | ++ | ++++ | +++ |
| 262 | +++ | ++ | ++++ | +++ |
| 263 | +++ | ++ | ++++ | +++ |
| 264 | +++ | +++ | ++++ | +++ |
| 266 | +++ | ++ | ++++ | +++ |
| 267 | +++ | +++ | ++++ | ++++ |
| 270 | ++++ | +++ | ++++ | +++ |
| 271 | ++++ | +++ | ++++ | ++++ |
| 272 | ++++ | +++ | ++++ | ++++ |
| 276 | +++ | +++ | ++++ | ++++ |
| 277 | +++ | +++ | ++++ | ++++ |
| 278 | +++ | +++ | ++++ | ++++ |
| 279 | ++++ | +++ | ++++ | +++ |
| 280 | +++ | ++ | ++++ | +++ |
| 281 | +++ | + | +++ | ++ |
| 282 | ++ | + | +++ | + |
| 283 | +++ | ++ | +++ | ++ |
| 284 | +++ | ++ | ++++ | +++ |
| 289 | +++ | +++ | ++++ | +++ |
| 291 | +++ | +++ | ++++ | ++++ |
| 293 | ++++ | +++ | ++++ | +++ |
| 306 | ++++ | ++ | ++++ | +++ |
| 308 | ++ | ++ | +++ | +++ |
| 310 | +++ | +++ | ++++ | +++ |
| 312 | +++ | ++ | +++ | +++ |
| 313 | ++++ | ++ | ++++ | +++ |
| 314 | ++++ | +++ | ++++ | ++++ |
| 315 | +++ | +++ | ++++ | +++ |
| 316 | ++++ | ++ | ++++ | +++ |
| 317 | +++ | ++ | +++ | +++ |
| 318 | +++ | ++ | +++ | +++ |
| 319 | +++ | ++ | +++ | ++ |
| 320 | +++ | ++ | +++ | ++ |
| 321 | +++ | ++ | ++++ | +++ |
| 322 | +++ | ++ | +++ | ++ |
| 323 | +++ | + | +++ | ++ |
| 328 | +++ | +++ | ++++ | +++ |
| 329 | +++ | +++ | ++++ | +++ |
| 331 | ++++ | +++ | ++++ | ++++ |
| 332 | ++++ | +++ | ++++ | ++++ |
| 334 | ++++ | +++ | ++++ | ++++ |
| 336 | ++++ | +++ | ++++ | ++++ |
| 339 | ++++ | ++ | ++++ | +++ |
| 341 | +++ | +++ | ++++ | ++++ |
| 343 | +++ | +++ | ++++ | ++++ |
| 347 | +++ | +++ | ++++ | +++ |
| 349 | ++++ | +++ | ++++ | ++++ |
| 351 | ++++ | +++ | ++++ | ++++ |
| 353 | ++++ | +++ | ++++ | ++++ |
| 355 | ++++ | +++ | ++++ | ++++ |
| 357 | ++++ | +++ | ++++ | ++++ |
| 359 | ++++ | +++ | ++++ | +++ |
| 360 | ++++ | ++++ | ++++ | ++++ |
| 363 | +++ | +++ | ++++ | ++++ |
| 364 | +++ | +++ | ++++ | ++++ |
| 365 | +++ | +++ | ++++ | ++++ |
| 366 | +++ | +++ | ++++ | +++ |
| 369 | ++ | ++ | +++ | +++ |
| 370 | +++ | +++ | ++++ | +++ |
| 371 | ++ | ++ | +++ | +++ |
| 372 | ++ | ++ | +++ | +++ |
| 373 | +++ | +++ | +++ | +++ |
| 374 | +++ | +++ | ++++ | ++++ |
| 375 | +++ | +++ | ++++ | ++++ |
| 376 | +++ | +++ | ++++ | ++++ |
| 377 | +++ | +++ | ++++ | +++ |
| 378 | +++ | +++ | ++++ | +++ |
| 379 | +++ | +++ | ++++ | +++ |
| 380 | +++ | +++ | ++++ | +++ |
| 381 | +++ | +++ | ++++ | +++ |
| 382 | +++ | +++ | ++++ | ++++ |
| 384 | ++ | + | ++ | + |
| 386 | ++ | + | ++ | + |
| 388 | ++ | +++ | +++ | ++++ |
| 390 | +++ | +++ | ++++ | +++ |
| 392 | +++ | +++ | ++++ | ++++ |
| 394 | ++++ | +++ | ++++ | ++++ |
| 396 | ++++ | ++++ | ++++ | ++++ |
| 398 | +++ | +++ | ++++ | +++ |
| 402 | ++++ | ++++ | ++++ | ++++ |
| 404 | +++ | +++ | ++++ | ++++ |
| 408 | +++ | +++ | ++++ | +++ |
| 410 | ++++ | ++++ | ++++ | ++++ |
| 411 | ++ | + | ++ | + |
| 412 | ++++ | +++ | ++++ | ++++ |
| 415 | ++++ | ++++ | ++++ | ++++ |
| 416 | +++ | +++ | ++++ | +++ |
| 417 | +++ | +++ | ++++ | +++ |
| 418 | ++++ | +++ | ++++ | ++++ |
| 419 | +++ | +++ | +++ | ++++ |
| 421 | ++++ | ++++ | ++++ | ++++ |
| 423 | +++ | +++ | ++++ | +++ |
| 425 | +++ | +++ | +++ | +++ |
| 427 | ++ | ++ | +++ | +++ |
| 432 | ++++ | +++ | ++++ | ++++ |
| 434 | +++ | +++ | ++++ | +++ |
| 435 | ++++ | +++ | ++++ | ++++ |
| 437 | +++ | +++ | ++++ | +++ |
| 439 | ++++ | +++ | ++++ | ++++ |
| 441 | ++++ | ++++ | ++++ | ++++ |
| 443 | +++ | +++ | ++++ | +++ |
| 445 | +++ | ++ | ++++ | +++ |
| 446 | +++ | + | ++++ | + |
| 447 | ++ | + | ++ | + |
| 551 | N/A | N/A | ++++ | +++ |
| 555 | N/A | N/A | ++++ | +++ |
| 556 | N/A | N/A | ++++ | +++ |
| 557 | N/A | N/A | +++ | +++ |
| 558 | N/A | N/A | +++ | +++ |
| 559 | N/A | N/A | +++ | +++ |
| 560 | N/A | N/A | + | + |
| 561 | N/A | N/A | ++++ | +++ |
| 562 | N/A | N/A | +++ | +++ |
| 563 | N/A | N/A | +++ | +++ |
| 564 | N/A | N/A | ++++ | +++ |
| 565 | N/A | N/A | +++ | +++ |
| 566 | N/A | N/A | ++++ | +++ |
| 567 | N/A | N/A | ++++ | +++ |
| 568 | N/A | N/A | ++++ | ++++ |
| 569 | N/A | N/A | ++++ | +++ |
| 570 | N/A | N/A | ++++ | +++ |
| 571 | N/A | N/A | ++++ | +++ |
| 572 | N/A | N/A | +++ | +++ |
| 573 | N/A | N/A | +++ | +++ |
| 574 | N/A | N/A | ++++ | +++ |
| 575 | N/A | N/A | ++++ | +++ |
| 576 | N/A | N/A | ++++ | +++ |
| 577 | N/A | N/A | ++++ | +++ |
| 578 | N/A | N/A | ++++ | +++ |
| 585 | N/A | N/A | +++ | +++ |
| 586 | N/A | N/A | ++++ | +++ |
| 587 | N/A | N/A | ++++ | ++++ |
| 589 | N/A | N/A | ++++ | |
| 594 | N/A | N/A | ++++ | ++++ |
| 596 | N/A | N/A | ++++ | +++ |
| 597 | N/A | N/A | ++++ | +++ |
| 598 | N/A | N/A | ++++ | +++ |
| 600 | N/A | N/A | ++++ | ++++ |
| 602 | N/A | N/A | ++++ | ++++ |
| 603 | N/A | N/A | ++++ | ++++ |
| 604 | N/A | N/A | +++ | +++ |
| 608 | N/A | N/A | ++++ | +++ |
| 609 | N/A | N/A | ++++ | +++ |
| 610 | N/A | N/A | ++++ | +++ |
| 611 | N/A | N/A | ++++ | +++ |
| 612 | N/A | N/A | ++++ | +++ |
| 613 | N/A | N/A | ++++ | +++ |
| 615 | N/A | N/A | ++++ | ++++ |
| 433 | N/A | N/A | ++++ | +++ |
| 686 | N/A | N/A | ++++ | +++ |
| 687 | N/A | N/A | ++ | ++ |
| 595 | N/A | N/A | + | N/A |
| 665 | N/A | N/A | +++ | N/A |
| 708 | N/A | N/A | +++ | +++ |
| 710 | N/A | N/A | +++ | +++ |
| 711 | N/A | N/A | +++ | ++ |
| 712 | N/A | N/A | ++++ | ++++ |
| 713 | N/A | N/A | ++++ | ++++ |
| 716 | N/A | N/A | ++++ | ++++ |
| 765 | + | + | | |
| 766 | +++ | + | | |
| 752 | ++ | + | | |
| 753 | +++ | + | | |
| 754 | ++ | + | | |
| 755 | ++++ | + | | |
| 756 | +++ | + | | |
| 757 | ++++ | + | | |
| 758 | +++ | + | | |

### Example 9: Competition Binding ELISA (MDM2 & MDMX)

p53-His6 protein (30 nM/well) is coated overnight at room temperature in the wells of a 96-well Immulon plates. On the day of the experiment, plates are washed with 1X PBS-Tween 20 (0.05%) using an automated ELISA plate washer, blocked with ELISA Micro well Blocking for 30 minutes at room temperature; excess blocking agent is washed off by washing plates with 1X PBS-Tween 20 (0.05%). Peptides are diluted from 10 mM DMSO stocks to 500 µM working stocks in sterile water, further dilutions made in 0.5% DMSO to keep the concentration of DMSO constant across the samples. The peptides are added to wells at 2X desired concentrations in 50 µL volumes, followed by addition of diluted GST-MDM2 or GST-HMDX protein (final concentration: 10nM). Samples are incubated at room temperature for 2h, plates are washed with PBS-Tween 20 (0.05%) prior to adding 100 µL of HRP-conjugated anti-GST antibody [Hypromatrix, INC] diluted to 0.5 µg/ml in HRP-stabilizing buffer. Post 30 min incubation with detection antibody, plates are washed and incubated with 100 µL per well of TMB-E Substrate solution up to 30 minutes; reactions are stopped using 1M HCL and absorbance measured at 450 nm on micro plate reader. Data is analyzed using Graph Pad PRISM software.

### Example 10: Cell Viability assay

The assay was performed according to the following general protocol:
Cell Plating: Trypsinize, count and seed cells at the pre-determined densities in 96-well plates a day prior to assay. Following cell densities are used for each cell line in use:
- SJSA-1: 7500 cells/ well
- RKO: 5000 cells/well
- RKO-E6: 5000 cells/well
- HCT-116: 5000 cells/well
- SW-480: 2000 cells/well
- MCF-7: 5000 cells/well

On the day of study, replace media with fresh media with 11% FBS (assay media) at room temperature. Add 180µL of the assay media per well. Control wells with no cells, receive 200 µL media.

Peptide dilution: all dilutions are made at room temperature and added to cells at room temperature.
- Prepare 10 mM stocks of the peptides in DMSO. Serially dilute the stock using 1:3 dilution scheme to get 10, 3.3, 1.1, 0.33, 0.11, 0.03, 0.01mM solutions using DMSO as diluents. Dilute the serially DMSO-diluted peptides 33.3 times using sterile water. This gives range of 10X working stocks. Also prepare DMSO/sterile water (3% DMSO) mix for control wells.
- Thus the working stocks concentration range µM will be 300, 100, 30, 10, 3, 1, 0.3 and 0 µM. Mix well at each dilution step using multichannel.
- Row H has controls. H1- H3 will receive 20 µL of assay media. H4-H9 will receive 20 µL of 3% DMSO-water vehicle. H10-H12 will have media alone control with no cells.
- Positive control: MDM2 small molecule inhibitor,Nutlin-3a (10 mM) is used as positive control.

Nutlin was diluted using the same dilution scheme as peptides.

Addition of working stocks to cells:
- Add 20 µL of 10X desired concentration to appropriate well to achieve the final concentrations in total 200 µL volume in well. (20 µL of 300 µM peptide + 180 µL of cells in media = 30 µM final concentration in 200 µL volume in wells). Mix gently a few times using pipette. Thus final concentration range used will be 30, 10, 3, 1, 0.3, 0.1, 0.03 & 0 µM (for potent peptides further dilutions are included).
- Controls include wells that get no peptides but contain the same concentration of DMSO as the wells containing the peptides, and wells containing NO CELLS.
- Incubate for 72 hours at 37°C in humidified 5% CO₂ atmosphere.
- The viability of cells is determined using MTT reagent from Promega. Viability of SJSA-1, RKO, RKO-E6, HCT-116 cells is determined on day 3, MCF-7 cells on day 5 and SW-480 cells on day 6. At the end of designated incubation time, allow the plates to come to room temperature. Remove 80 µL of assay media from each well. Add 15 µL of thawed MTT reagent to each well.
- Allow plate to incubate for 2h at 37°C in humidified 5% CO₂ atmosphere and add 100 µL solubilization reagent as per manufacturer's protocol. Incubate with agitation for 1h at room temperature and read on Synergy Biotek multiplate reader for absorbance at 570nM.
- Analyze the cell viability against the DMSO controls using GraphPad PRISM analysis tools.

### Reagents:

- Invitrogen cell culture Media
- Falcon 96-well clear cell culture treated plates (Nunc 353072)
- DMSO ( Sigma D 2650)
- RPMI 1640 (Invitrogen 72400)
- MTT (Promega G4000)

### Instruments: Multiplate Reader for Absorbance readout (Synergy 2).

Results from cell viability assays are shown in Tables 5 and 6. The following scale is used: "+" represents a value greater than 30 µM, "++" represents a value greater than 15 µM and less than or equal to 30 µM, "+++" represents a value greater than 5 µM and less than or equal to 15 µM, and "++++" represents a value of less than or equal to 5 µM. "IC50 ratio" represents the ratio of average IC50 in p53+/+ cells relative to average IC50 in p53-/- cells.

**Table 5**

| **SP#** | **SJSA-1 EC50 (72h)** | **SP#** | **SJSA-1 EC50 (72h)** | **SP#** | **SJSA-1 EC50 (72h)** | **SP#** | **SJSA-1 EC50 (72h)** |
|---|---|---|---|---|---|---|---|
| 3 | +++ | 170 | ++++ | 295 | +++ | 443 | ++++ |
| 4 | +++ | 171 | ++ | 296 | ++++ | 444 | +++ |
| 5 | ++++ | 173 | +++ | 297 | +++ | 445 | ++++ |
| 6 | ++ | 174 | ++++ | 298 | ++++ | 449 | ++++ |
| 7 | ++++ | 175 | +++ | 300 | ++++ | 551 | ++++ |
| 8 | +++ | 176 | +++ | 301 | ++++ | 552 | ++++ |
| 9 | +++ | 177 | ++++ | 302 | ++++ | 554 | + |
| 10 | +++ | 179 | +++ | 303 | ++++ | 555 | ++++ |
| 11 | ++++ | 180 | +++ | 304 | ++++ | 586 | ++++ |
| 12 | ++ | 181 | +++ | 305 | ++++ | 587 | ++++ |
| 13 | +++ | 182 | ++++ | 306 | ++++ | 588 | ++++ |
| 14 | + | 183 | ++++ | 307 | +++ | 589 | +++ |
| 15 | ++ | 184 | +++ | 308 | ++++ | 432 | ++++ |
| 16 | + | 185 | +++ | 309 | +++ | 672 | + |
| 17 | + | 186 | ++ | 310 | ++++ | 673 | ++ |
| 18 | + | 188 | ++ | 312 | ++++ | 682 | + |
| 19 | ++ | 190 | ++++ | 313 | ++++ | 686 | + |
| 20 | + | 192 | +++ | 314 | ++++ | 557 | ++++ |
| 21 | + | 193 | ++ | 315 | ++++ | 558 | ++++ |
| 22 | + | 194 | + | 316 | ++++ | 560 | + |
| 24 | +++ | 195 | ++++ | 317 | ++++ | 561 | ++++ |
| 26 | ++++ | 196 | ++++ | 318 | ++++ | 562 | ++++ |
| 28 | + | 197 | ++++ | 319 | ++++ | 563 | ++++ |
| 29 | + | 198 | ++ | 320 | ++++ | 564 | ++++ |
| 30 | + | 199 | +++ | 321 | ++++ | 566 | ++++ |
| 32 | ++ | 200 | +++ | 322 | ++++ | 567 | ++++ |
| 38 | + | 201 | ++++ | 323 | ++++ | 568 | +++ |
| 39 | + | 202 | +++ | 324 | ++++ | 569 | ++++ |
| 40 | + | 203 | ++++ | 326 | ++++ | 571 | ++++ |
| 41 | + | 204 | ++++ | 327 | ++++ | 572 | ++++ |
| 42 | + | 205 | ++ | 328 | ++++ | 573 | ++++ |
| 43 | ++ | 206 | ++ | 329 | ++++ | 574 | ++++ |
| 45 | + | 207 | +++ | 330 | ++++ | 575 | ++++ |
| 46 | + | 208 | +++ | 331 | ++++ | 576 | ++++ |
| 47 | + | 209 | ++++ | 332 | ++++ | 577 | ++++ |
| 48 | + | 210 | +++ | 333 | ++ | 578 | ++++ |
| 49 | +++ | 211 | ++++ | 334 | +++ | 585 | ++++ |
| 50 | ++++ | 213 | ++++ | 335 | ++++ | 687 | + |
| 52 | + | 214 | ++++ | 336 | ++++ | 662 | ++++ |
| 54 | + | 215 | ++++ | 337 | ++++ | 663 | ++++ |
| 55 | + | 216 | ++++ | 338 | ++++ | 553 | +++ |
| 65 | ++++ | 217 | ++++ | 339 | ++++ | 559 | ++++ |
| 68 | ++++ | 218 | ++++ | 340 | ++++ | 579 | ++++ |
| 69 | ++++ | 219 | ++++ | 341 | ++++ | 581 | ++++ |
| 70 | ++++ | 220 | +++ | 342 | ++++ | 582 | ++ |
| 71 | ++++ | 221 | ++++ | 343 | ++++ | 582 | ++++ |
| 72 | ++++ | 222 | +++ | 344 | ++++ | 584 | +++ |
| 74 | ++++ | 223 | ++++ | 345 | ++++ | 675 | ++++ |
| 75 | ++++ | 224 | ++ | 346 | ++++ | 676 | ++++ |
| 77 | ++++ | 225 | +++ | 347 | ++++ | 677 | + |
| 78 | ++ | 226 | ++ | 348 | ++++ | 679 | ++++ |
| 80 | ++++ | 227 | +++ | 349 | ++++ | 700 | +++ |
| 81 | +++ | 228 | ++++ | 350 | ++++ | 704 | +++ |
| 82 | +++ | 229 | ++++ | 351 | ++++ | 591 | + |
| 83 | +++ | 230 | ++++ | 352 | ++++ | 706 | ++ |
| 84 | + | 231 | ++++ | 353 | ++++ | 695 | ++ |
| 85 | +++ | 232 | ++++ | 355 | ++++ | 595 | ++++ |
| 99 | ++++ | 233 | ++++ | 357 | ++++ | 596 | ++++ |
| 102 | +++ | 234 | ++++ | 358 | ++++ | 597 | +++ |
| 103 | +++ | 235 | ++++ | 359 | ++++ | 598 | +++ |
| 104 | +++ | 236 | ++++ | 360 | ++++ | 599 | ++++ |
| 105 | +++ | 237 | ++++ | 361 | +++ | 600 | ++++ |
| 108 | +++ | 238 | ++++ | 362 | ++++ | 601 | +++ |
| 109 | +++ | 239 | +++ | 363 | ++++ | 602 | +++ |
| 110 | +++ | 240 | ++ | 364 | ++++ | 603 | +++ |
| 111 | ++ | 241 | +++ | 365 | +++ | 604 | +++ |
| 114 | ++++ | 242 | ++++ | 366 | ++++ | 606 | ++++ |
| 115 | ++++ | 243 | ++++ | 367 | ++++ | 607 | ++++ |
| 118 | ++++ | 244 | ++++ | 368 | + | 608 | ++++ |
| 120 | ++++ | 245 | ++++ | 369 | ++++ | 610 | ++++ |
| 121 | ++++ | 246 | +++ | 370 | ++++ | 611 | ++++ |
| 122 | ++++ | 247 | ++++ | 371 | ++++ | 612 | ++++ |
| 123 | ++++ | 248 | ++++ | 372 | +++ | 613 | +++ |
| 124 | +++ | 249 | ++++ | 373 | +++ | 614 | +++ |
| 125 | ++++ | 250 | ++ | 374 | ++++ | 615 | ++++ |
| 126 | ++++ | 251 | + | 375 | ++++ | 618 | ++++ |
| 127 | ++++ | 252 | + | 376 | ++++ | 619 | ++++ |
| 128 | +++ | 253 | + | 377 | ++++ | 707 | ++++ |
| 129 | ++ | 254 | +++ | 378 | ++++ | 620 | ++++ |
| 130 | ++++ | 255 | +++ | 379 | ++++ | 621 | ++++ |
| 131 | +++ | 256 | ++ | 380 | ++++ | 622 | ++++ |
| 132 | ++++ | 257 | +++ | 381 | ++++ | 623 | ++++ |
| 133 | +++ | 258 | +++ | 382 | ++++ | 624 | ++++ |
| 134 | +++ | 259 | ++ | 386 | +++ | 625 | ++++ |
| 135 | +++ | 260 | ++ | 388 | ++ | 626 | +++ |
| 136 | ++ | 261 | ++ | 390 | ++++ | 631 | ++++ |
| 137 | +++ | 262 | +++ | 392 | +++ | 633 | ++++ |
| 139 | ++++ | 263 | ++ | 394 | +++ | 634 | ++++ |
| 142 | +++ | 264 | ++++ | 396 | +++ | 635 | +++ |
| 144 | ++++ | 266 | +++ | 398 | +++ | 636 | +++ |
| 147 | ++++ | 267 | ++++ | 402 | +++ | 638 | + |
| 148 | ++++ | 270 | ++ | 404 | +++ | 641 | +++ |
| 149 | ++++ | 271 | ++ | 408 | ++++ | 665 | ++++ |
| 150 | ++++ | 272 | ++ | 410 | +++ | 708 | ++++ |
| 152 | +++ | 276 | ++ | 411 | +++ | 709 | +++ |
| 153 | ++++ | 277 | ++ | 412 | + | 710 | + |
| 154 | ++++ | 278 | ++ | 421 | +++ | 711 | ++++ |
| 155 | ++ | 279 | ++++ | 423 | ++++ | 712 | ++++ |
| 156 | +++ | 280 | +++ | 425 | ++++ | 713 | ++++ |
| 157 | +++ | 281 | ++ | 427 | ++++ | 714 | +++ |
| 158 | +++ | 282 | ++ | 434 | +++ | 715 | +++ |
| 160 | ++++ | 283 | ++ | 435 | ++++ | 716 | ++++ |
| 161 | ++++ | 284 | ++++ | 436 | ++++ | 765 | + |
| 162 | +++ | 289 | ++++ | 437 | ++++ | 753 | + |
| 163 | +++ | 290 | +++ | 438 | ++++ | 754 | + |
| 166 | ++ | 291 | ++++ | 439 | ++++ | 755 | + |
| 167 | +++ | 292 | ++++ | 440 | ++++ | 756 | + |
| 168 | ++ | 293 | ++++ | 441 | ++++ | 757 | ++++ |
| 169 | ++++ | 294 | ++++ | 442 | ++++ | 758 | +++ |

**Table 6**

| **SP#** | **HCT-116 EC50 (72h)** | **RKO EC₅₀ (72h)** | **RKO-E6 EC₅₀ (72h)** | **SW480 EC50 (6days)** | **IC₅₀ Ratio** |
|---|---|---|---|---|---|
| 4 | ++++ | ++++ | +++ | ++++ | |
| 5 | ++++ | ++++ | +++ | ++++ | |
| 7 | ++++ | ++++ | +++ | ++++ | |
| 10 | ++++ | +++ | +++ | +++ | |
| 11 | ++++ | ++++ | ++ | +++ | |
| 50 | ++++ | ++++ | ++ | +++ | |
| 65 | +++ | +++ | +++ | +++ | |
| 69 | ++++ | ++++ | + | ++++ | |
| 70 | ++++ | ++++ | ++ | +++ | |
| 71 | ++++ | ++++ | +++ | +++ | |
| 81 | +++ | +++ | +++ | +++ | |
| 99 | ++++ | ++++ | +++ | ++++ | |
| 109 | ++++ | ++++ | ++ | +++ | |
| 114 | | +++ | + | +++ | |
| 115 | | +++ | + | +++ | 1-29 |
| 118 | +++ | ++++ | + | ++++ | |
| 120 | ++++ | ++++ | + | ++++ | |
| 121 | ++++ | ++++ | + | ++++ | |
| 122 | | +++ | + | +++ | 1-29 |
| 125 | +++ | +++ | + | + | |
| 126 | + | + | + | + | |
| 148 | | ++ | + | + | |
| 150 | | ++ | + | + | |
| 153 | +++ | | + | | |
| 154 | +++ | +++ | + | + | 30-49 |
| 158 | + | + | + | + | |
| 160 | +++ | + | + | + | 1-29 |
| 161 | +++ | + | + | + | |
| 175 | + | + | + | + | |
| 196 | ++++ | ++++ | +++ | ++++ | |
| 219 | ++++ | +++ | + | + | 1-29 |
| 233 | ++++ | | | | |
| 237 | ++++ | | + | + | |
| 238 | ++++ | | + | + | |
| 243 | ++++ | | + | + | |
| 244 | ++++ | | + | + | ≥50 |
| 245 | ++++ | | + | + | |
| 247 | ++++ | | + | + | |
| 249 | ++++ | ++++ | + | + | ≥50 |
| 255 | ++++ | | + | | |
| 291 | | | + | | |
| 293 | +++ | | + | | |
| 303 | +++ | | + | | 1-29 |
| 305 | | | + | | |
| 306 | ++++ | | + | | |
| 310 | ++++ | | + | | |
| 312 | ++++ | | | | |
| 313 | ++++ | | ++ | | |
| 314 | | | + | | |
| 315 | ++++ | ++++ | ++ | ++++ | ≥50 |
| 316 | ++++ | ++++ | + | +++ | ≥50 |
| 317 | +++ | | + | ++ | |
| 321 | ++++ | | + | | |
| 324 | +++ | | + | | |
| 325 | +++ | | | | |
| 326 | +++ | | + | | |
| 327 | +++ | | + | | |
| 328 | +++ | | ++ | | |
| 329 | ++++ | | + | | |
| 330 | | | + | | |
| 331 | ++++ | ++++ | + | + | ≥50 |
| 338 | ++++ | ++++ | ++ | +++ | |
| 341 | +++ | ++ | + | + | |
| 343 | +++ | | + | + | |
| 346 | ++++ | | + | + | |
| 347 | +++ | | + | + | |
| 349 | ++++ | +++ | + | + | 30-49 |
| 350 | ++++ | | + | + | |
| 351 | ++++ | +++ | + | + | 30-49 |
| 353 | ++ | ++ | + | + | |
| 355 | ++++ | ++ | + | + | 1-29 |
| 357 | ++++ | ++++ | + | + | |
| 358 | ++++ | ++ | + | + | |
| 359 | ++++ | ++ | + | + | |
| 367 | ++++ | | + | + | 30-49 |
| 386 | ++++ | ++++ | ++++ | ++++ | |
| 388 | ++ | ++ | + | +++ | 1-29 |
| 390 | ++++ | ++++ | +++ | ++++ | |
| 435 | +++ | ++ | + | | |
| 436 | ++++ | ++++ | ++ | | |
| 437 | ++++ | ++++ | ++ | ++++ | 30-49 |
| 440 | ++ | ++ | + | | |
| 442 | ++++ | ++++ | ++ | | |
| 444 | ++++ | ++++ | +++ | | |
| 445 | ++++ | +++ | + | + | ≥50 |
| 555 | | | | | ≥50 |
| 557 | | | | | ≥50 |
| 558 | | | | | 30-49 |
| 562 | | | | | 30-49 |
| 564 | | | | | 30-49 |
| 566 | | | | | 30-49 |
| 567 | | | | | ≥50 |
| 572 | | | | | ≥50 |
| 573 | | | | | 30-49 |
| 578 | | | | | 30-49 |
| 662 | | | | | ≥50 |
| 379 | | | | | 1-29 |
| 375 | | | | | 1-29 |
| 559 | | | | | ≥50 |
| 561 | | | | | 1-29 |
| 563 | | | | | 1-29 |
| 568 | | | | | 1-29 |
| 569 | | | | | 1-29 |
| 571 | | | | | 1-29 |
| 574 | | | | | 1-29 |
| 575 | | | | | 1-29 |
| 576 | | | | | 1-29 |
| 577 | | | | | 30-49 |
| 433 | | | | | 1-29 |
| 551 | | | | | 30-49 |
| 553 | | | | | 1-29 |
| 710 | | | | + | |
| 711 | | | | + | |
| 712 | | | | ++ | |
| 713 | | | | ++ | |
| 714 | | | | +++ | |
| 715 | | | | +++ | |
| 716 | | | | + | |

### Example 11: p21 ELISA assay

### The assay was performed according to the following general protocol:

### Cell Plating:

- Trypsinize, count and seed SJSA1 cells at the density of 7500 cells/ 100 µL/well in 96-well plates a day prior to assay.
- On the day of study, replace media with fresh RPMI-11% FBS (assay media). Add 90µL of the assay media per well. Control wells with no cells, receive 100 µL media.

### Peptide dilution:

- Prepare 10 mM stocks of the peptides in DMSO. Serially dilute the stock using 1:3 dilution scheme to get 10, 3.3, 1.1, 0.33, 0.11, 0.03, 0.01mM solutions using DMSO as diluents. Dilute the serially DMSO-diluted peptides 33.3 times using sterile water This gives range of 10X working stocks. Also prepare DMSO/sterile water (3% DMSO) mix for control wells.
- Thus the working stocks concentration range µM will be 300, 100, 30, 10, 3, 1, 0.3 and 0 µM. Mix well at each dilution step using multichannel.
- Row H has controls. H1- H3 will receive 10 µL of assay media. H4-H9 will receive 10 µL of 3% DMSO-water vehicle. H10-H12 will have media alone control with no cells.
- Positive control: MDM2 small molecule inhibitor,Nutlin-3a (10 mM) is used as positive control. Nutlin was diluted using the same dilution scheme as peptides.

Addition of working stocks to cells:
- Add 10 µL of 10X desired concentration to appropriate well to achieve the final concentrations in total 100 µL volume in well. (10 µL of 300 µM peptide + 90 µL of cells in media = 30 µM final concentration in 100 µL volume in wells). Thus final concentration range used will be 30, 10, 3, 1, 0.3& 0 µM.
- Controls will include wells that get no peptides but contain the same concentration of DMSO as the wells containing the peptides, and wells containing NO CELLS.
- 20h-post incubation, aspirate the media; wash cells with 1X PBS (without Ca⁺⁺/Mg⁺⁺) and lyse in 60 µL of 1X Cell lysis buffer (Cell Signaling technologies 10X buffer diluted to 1X and supplemented with protease inhibitors and Phosphatase inhibitors) on ice for 30 min.
- Centrifuge plates in at 5000 rpm speed in at 4⁰C for 8 min; collect clear supernatants and freeze at -80 ⁰C till further use.

Protein Estimation:
- Total protein content of the lysates is measured using BCA protein detection kit and BSA standards from Thermofisher. Typically about 6-7 µg protein is expected per well.
- Use 50 µL of the lysate per well to set up p21 ELISA.

**Human Total p21 ELISA:** The ELISA assay protocol is followed as per the manufacturer's instructions. 50 µL lysate is used for each well, and each well is set up in triplicate.

### Reagents:

- -Cell-Based Assay (-)-Nutlin-3 (10 mM): Cayman Chemicals, catalog # 600034
- OptiMEM, Invitrogen catalog # 51985
- -Cell Signaling Lysis Buffer (10X), Cell signaling technology, Catalog # 9803
- -Protease inhibitor Cocktail tablets(mini), Roche Chemicals, catalog # 04693124001
- -Phosphatase inhibitor Cocktail tablet, Roche Chemicals, catalog # 04906837001
- -Human total p21 ELISA kit, R&D Systems, DYC1047-5
- -STOP Solution (1M HCL), Cell Signaling Technologies, Catalog # 7002

### Instruments: Micro centrifuge- Eppendorf 5415D and Multiplate Reader for Absorbance readout (Synergy 2).

### Example 12: Caspase 3 Detection assay:

The assay was performed according to the following general protocol:
Cell Plating: Trypsinize, count and seed SJSA1 cells at the density of 7500 cells/ 100 µL/well in 96-well plates a day prior to assay. On the day of study, replace media with fresh RPMI-11% FBS (assay media). Add 180µL of the assay media per well. Control wells with no cells, receive 200 µL media.

### Peptide dilution:

- Prepare 10 mM stocks of the peptides in DMSO. Serially dilute the stock using 1:3 dilution scheme to get 10, 3.3, 1.1, 0.33, 0.11, 0.03, 0.01mM solutions using DMSO as diluents. Dilute the serially DMSO-diluted peptides 33.3 times using sterile water This gives range of 10X working stocks. Also prepare DMSO/sterile water (3% DMSO) mix for control wells.
- Thus the working stocks concentration range µM will be 300, 100, 30, 10, 3, 1, 0.3 and 0 µM. Mix well at each dilution step using multichannel. Add 20 µL of 10X working stocks to appropriate wells.
- Row H has controls. H1- H3 will receive 20 µL of assay media. H4-H9 will receive 20 µL of 3% DMSO-water vehicle. H10-H12 will have media alone control with no cells.
- Positive control: MDM2 small molecule inhibitor,Nutlin-3a (10 mM) is used as positive control. Nutlin was diluted using the same dilution scheme as peptides.

Addition of working stocks to cells:
- Add 10 µL of 10X desired concentration to appropriate well to achieve the final concentrations in total 100 µL volume in well. (10 µL of 300 µM peptide + 90 µL of cells in media = 30 µM final concentration in 100 µL volume in wells). Thus final concentration range used will be 30, 10, 3, 1, 0.3& 0 µM.
- Controls will include wells that get no peptides but contain the same concentration of DMSO as the wells containing the peptides, and wells containing NO CELLS.
- 48 h-post incubation, aspirate 80 µL media from each well; add 100 µL Caspase3/7Glo assay reagent (Promega Caspase 3/7 glo assay system, G8092)per well, incubate with gentle shaking for 1h at room temperature.
- read on Synergy Biotek multiplate reader for luminescence.
- Data is analyzed as Caspase 3 activation over DMSO-treated cells.

Results from Examples 11 and 12 are shown in Table 7:

**Table 7**

| SP# | caspase 0.3µM | caspase 1 µM | caspase 3µM | caspase 10µM | caspase 30µM | p21 0.3µM | p21 1µM | p21 3µM | p21 10µM | p21 30µM |
|---|---|---|---|---|---|---|---|---|---|---|
| 4 | | | 9 | 37 | 35 | | | 317 | 3049 | 3257 |
| 7 | 0.93 | 1.4 | 5.08 | 21.7 | 23.96 | | 18 | 368 | 1687 | 2306 |
| 8 | | | 1 | 19 | 25 | | | 34 | 972 | 2857 |
| 10 | 1 | | 1 | 17 | 32 | | 10 | 89 | 970 | 2250 |
| 11 | 1 | | 5 | 23 | 33.5 | | 140 | 350 | 2075.5 | 3154 |
| 26 | 1 | | 1 | 3 | 14 | | | | | |
| 50 | | | 8 | 29 | 29 | | 44 | 646 | 1923 | 1818 |
| 65 | 1 | | 6 | 28 | 34 | -69 | -24 | 122 | 843 | 1472 |
| 69 | 4.34 | 9.51 | 16.39 | 26.59 | 26.11 | 272 | 458.72 | 1281.39 | 2138.88 | 1447.22 |
| 70 | | | 1 | 9 | 26 | | -19 | 68 | 828 | 1871 |
| 71 | 0.95 | 1.02 | 3.68 | 14.72 | 23.52 | | 95 | 101 | 1204 | 2075 |
| 72 | 1 | | 1 | 4 | 10 | -19 | 57 | 282 | 772 | 1045 |
| 77 | 1 | | 2 | 19 | 23 | | | | | |
| 80 | 1 | | 2 | 13 | 20 | | | | | |
| 81 | 1 | | 1 | 6 | 21 | | 0 | 0 | 417 | 1649 |
| 99 | 1 | | 7 | 31 | 33 | -19 | 117 | 370 | 996 | 1398 |
| 109 | | | 4 | 16 | 25 | | 161 | 445 | 1221 | 1680 |
| 114 | 1 | | 6 | 28 | 34 | -21 | 11 | 116 | 742 | 910 |
| 115 | 1 | | 10 | 26 | 32 | -10 | 36 | 315 | 832 | 1020 |
| 118 | 1 | | 2 | 18 | 27 | -76 | -62 | -11 | 581 | 1270 |
| 120 | 2 | | 11 | 20 | 30 | -4 | 30 | 164 | 756 | 1349 |
| 121 | 1 | | 5 | 19 | 30 | 9 | 33 | 81 | 626 | 1251 |
| 122 | 1 | | 2 | 15 | 30 | -39 | -18 | 59 | 554 | 1289 |
| 123 | 1 | | 1 | 6 | 14 | | | | | |
| 125 | 1 | | 3 | 9 | 29 | 50 | 104 | 196 | 353 | 1222 |
| 126 | 1 | | 1 | 6 | 30 | -47 | -10 | 90 | 397 | 1443 |
| 127 | 1 | | 1 | 4 | 13 | | | | | |
| 130 | 1 | | 2 | 6 | 17 | | | | | |
| 139 | 1 | | 2 | 9 | 18 | | | | | |
| 142 | 1 | | 2 | 15 | 20 | | | | | |
| 144 | 1 | | 4 | 10 | 16 | | | | | |
| 148 | 1 | | 11 | 23 | 31 | -23 | 55 | 295 | 666 | 820 |
| 149 | 1 | | 2 | 4 | 10 | 35 | 331 | 601 | 1164 | 1540 |
| 150 | 2 | | 11 | 19 | 35 | -37 | 24 | 294 | 895 | 906 |
| 153 | 2 | | 10 | 15 | 20 | | | | | |
| 154 | 2.68 | 4 | 13.93 | 19.86 | 30.14 | 414.04 | 837.45 | 1622.42 | 2149.51 | 2156.98 |
| 158 | 1 | | 1.67 | 5 | 16.33 | -1.5 | 95 | 209.5 | 654 | 1665.5 |
| 160 | 2 | | 10 | 16 | 31 | -43 | 46 | 373 | 814 | 1334 |
| 161 | 2 | | 8 | 14 | 22 | 13 | 128 | 331 | 619 | 1078 |
| 170 | 1 | | 1 | 16 | 20 | | | | | |
| 175 | 1 | | 5 | 12 | 21 | -65 | 1 | 149 | 543 | 1107 |
| 177 | 1 | | 1 | 8 | 20 | | | | | |
| 183 | 1 | | 1 | 4 | 8 | -132 | -119 | -14 | 1002 | 818 |
| 196 | 1 | | 4 | 33 | 26 | -49 | -1 | 214 | 1715 | 687 |
| 197 | 1 | | 1 | 10 | 20 | | | | | |
| 203 | 1 | | 3 | 12 | 10 | 77 | 329 | 534 | 1805 | 380 |
| 204 | 1 | | 4 | 10 | 10 | 3 | 337 | 928 | 1435 | 269 |
| 218 | 1 | | 2 | 8 | 18 | | | | | |
| 219 | 1 | | 5 | 17 | 34 | 28 | 53 | 289 | 884 | 1435 |
| 221 | 1 | | 3 | 6 | 12 | 127 | 339 | 923 | 1694 | 1701 |
| 223 | 1 | | 1 | 5 | 18 | | | | | |
| 230 | 1 | | 2 | 3 | 11 | 245.5 | 392 | 882 | 1549 | 2086 |
| 233 | 6 | 8 | 17 | 22 | 23 | 2000 | 2489 | 3528 | 3689 | 2481 |
| 237 | 1 | | 5 | 9 | 15 | 0 | 0 | 2 | 284 | 421 |
| 238 | 1 | | 2 | 4 | 21 | 0 | 149 | 128 | 825 | 2066 |
| 242 | 1 | | 4 | 5 | 18 | 0 | 0 | 35 | 577 | 595 |
| 243 | 1 | | 2 | 5 | 23 | 0 | 0 | 0 | 456 | 615 |
| 244 | 1 | | 2 | 7 | 17 | 0 | 178 | 190 | 708 | 1112 |
| 245 | 1 | | 3 | 9 | 16 | 0 | 0 | 0 | 368 | 536 |
| 247 | 1 | | 3 | 11 | 24 | 0 | 0 | 49 | 492 | 699 |
| 248 | | | | | | 0 | 50 | 22 | 174 | 1919 |
| 249 | 2 | | 5 | 11 | 23 | 0 | 0 | 100 | 907 | 1076 |
| 251 | | | | | | 0 | 0 | 0 | 0 | 0 |
| 252 | | | | | | 0 | 0 | 0 | 0 | 0 |
| 253 | | | | | | 0 | 0 | 0 | 0 | 0 |
| 254 | 1 | 3 | 7 | 14 | 22 | 118 | 896 | 1774 | 3042 | 3035 |
| 286 | 1 | 4 | 11 | 20 | 22 | 481 | 1351 | 2882 | 3383 | 2479 |
| 287 | 1 | 1 | 3 | 11 | 23 | 97 | 398 | 986 | 2828 | 3410 |
| 315 | 11 | 14.5 | 25.5 | 32 | 34 | 2110 | 2209 | 2626 | 2965 | 2635 |
| 316 | 6.5 | 10.5 | 21 | 32 | 32.5 | 1319 | 1718 | 2848 | 2918 | 2540 |
| 317 | 3 | 4 | 9 | 26 | 35 | 551 | 624 | 776 | 1367 | 1076 |
| 331 | 4.5 | 8 | 11 | 14.5 | 30.5 | 1510 | 1649 | 2027 | 2319 | 2509 |
| 338 | 1 | 5 | 23 | 20 | 29 | 660.37 | 1625.38 | 3365.87 | 2897.62 | 2727 |
| 341 | 3 | 8 | 11 | 14 | 21 | 1325.62 | 1873 | 2039.75 | 2360.75 | 2574 |
| 343 | 1 | 1 | 2 | 5 | 29 | 262 | 281 | 450 | 570 | 1199 |
| 346 | | | | | | 235.86 | 339.82 | 620.36 | 829.32 | 1695.78 |
| 347 | 2 | 3 | 5 | 8 | 29 | 374 | 622 | 659 | 905 | 1567 |
| 349 | 1 | 8 | 11 | 16 | 24 | 1039.5 | 1598.88 | 1983.75 | 2191.25 | 2576.38 |
| 351 | 3 | 9 | 13 | 15 | 24 | 1350.67 | 1710.67 | 2030.92 | 2190.67 | 2668.54 |
| 353 | 1 | 2 | 5 | 7 | 30 | 390 | 490 | 709 | 931 | 1483 |
| 355 | 1 | 4 | 11 | 13 | 30 | 191 | 688 | 1122 | 1223 | 1519 |
| 357 | 2 | 7 | 11 | 15 | 23 | 539 | 777 | 1080 | 1362 | 1177 |
| 358 | 1 | 2 | 3 | 6 | 24 | 252 | 321 | 434 | 609 | 1192 |
| 359 | 3 | 9 | 11 | 13 | 23 | 1163.29 | 1508.79 | 1780.29 | 2067.67 | 2479.29 |
| 416 | | | | | | 33.74 | 39.82 | 56.57 | 86.78 | 1275.28 |
| 417 | | | | | | 0 | 0 | 101.13 | 639.04 | 2016.58 |
| 419 | | | | | | 58.28 | 97.36 | 221.65 | 1520.69 | 2187.94 |
| 432 | | | | | | 54.86 | 68.86 | 105.11 | 440.28 | 1594.4 |

### Example 13. Cell Lysis by Peptidomimetic Macrocycles

SJSA-1 cells were plated out one day in advance in clear flat-bottom plates (Costar, catalog number 353072) at 7500cells/well with 100ul/well of growth media, leaving row H columns 10-12 empty for media alone. On the day of the assay, media was exchanged with RPMI 1% FBS media, 90uL of media per well.

10 mM stock solutions of the peptidomimetic macrocycles were prepared in 100% DMSO. Peptidomimetic macrocycles were then diluted serially in 100% DMSO, and then further diluted 20-fold in sterile water to prepare working stock solutions in 5% DMSO/water of each peptidomimetic macrocycle at concentrations ranging from 500 µM to 62.5 µM.

10 µL of each compound was added to the 90 uL of SJSA-1 cells to yield final concentrations of 50 µM to 6.25 µM in 0.5% DMSO-containing media. The negative control (non-lytic) sample was 0.5% DMSO alone and positive control (lytic) samples include 10 µM Melittin and 1% Triton X-100.

Cell plates were incubated for 1 hour at 37 °C. After the 1 hour incubation, the morphology of the cells is examined by microscope and then the plates were centrifuged at 1200 rpm for 5 minutes at room temperature. 40 µL of supernatant for each peptidomimetic macrocycle and control sample is transferred to clear assay plates. LDH release is measured using the LDH cytotoxicity assay kit from Caymen, catalog# 1000882. Results are shown in Table 8:

**Table 8**

| SP# | 6.25 µM % Lysed cells (1h LDH) | 12.5 µM % Lysed cells (1h LDH) | 25 µM % Lysed cells (1h LDH) | 50 µM % Lysed cells (1h LDH) |
|---|---|---|---|---|
| 3 | 1 | 0 | 1 | 3 |
| 4 | -2 | 1 | 1 | 2 |
| 6 | 1 | 1 | 1 | 1 |
| 7 | 0 | 0 | 0 | 0 |
| 8 | -1 | 0 | 1 | 1 |
| 9 | -3 | 0 | 0 | 2 |
| 11 | -2 | 1 | 2 | 3 |
| 15 | 1 | 2 | 2 | 5 |
| 18 | 0 | 1 | 2 | 4 |
| 19 | 2 | 2 | 3 | 21 |
| 22 | 0 | -1 | 0 | 0 |
| 26 | 2 | 5 | -1 | 0 |
| 32 | 0 | 0 | 2 | 0 |
| 39 | 0 | -1 | 0 | 3 |
| 43 | 0 | 0 | -1 | -1 |
| 55 | 1 | 5 | 9 | 13 |
| 65 | 0 | 0 | 0 | 2 |
| 69 | 1 | 0.5 | -0.5 | 5 |
| 71 | 0 | 0 | 0 | 0 |
| 72 | 2 | 1 | 0 | 3 |
| 75 | -1 | 3 | 1 | 1 |
| 77 | -2 | -2 | 1 | -1 |
| 80 | 0 | 1 | 1 | 5 |
| 81 | 1 | 1 | 0 | 0 |
| 82 | 0 | 0 | 0 | 1 |
| 99 | 1.5 | 3 | 2 | 3.5 |
| 108 | 0 | 0 | 0 | 1 |
| 114 | 3 | -1 | 4 | 9 |
| 115 | 0 | 1 | -1 | 6 |
| 118 | 4 | 2 | 2 | 4 |
| 120 | 0 | -1 | 0 | 6 |
| 121 | 1 | 0 | 1 | 7 |
| 122 | 1 | 3 | 0 | 6 |
| 123 | -2 | 2 | 5 | 3 |
| 125 | 0 | 1 | 0 | 2 |
| 126 | 1 | 2 | 1 | 1 |
| 130 | 1 | 3 | 0 | -1 |
| 139 | -2 | -3 | -1 | -1 |
| 142 | 1 | 0 | 1 | 3 |
| 144 | 1 | 2 | -1 | 2 |
| 147 | 8 | 9 | 16 | 55 |
| 148 | 0 | 1 | -1 | 0 |
| 149 | 6 | 7 | 7 | 21 |
| 150 | -1 | -2 | 0 | 2 |
| 153 | 4 | 3 | 2 | 3 |
| 154 | -1 | -1.5 | -1 | -1 |
| 158 | 0 | -6 | -2 | |
| 160 | -1 | 0 | -1 | 1 |
| 161 | 1 | 1 | -1 | 0 |
| 169 | 2 | 3 | 3 | 7 |
| 170 | 2 | 2 | 1 | -1 |
| 174 | 5 | 3 | 2 | 5 |
| 175 | 3 | 2 | 1 | 0 |
| 177 | -1 | -1 | 0 | 1 |
| 182 | 0 | 2 | 3 | 6 |
| 183 | 2 | 1 | 0 | 3 |
| 190 | -1 | -1 | 0 | 1 |
| 196 | 0 | -2 | 0 | 3 |
| 197 | 1 | -4 | -1 | -2 |
| 203 | 0 | -1 | 2 | 2 |
| 204 | 4 | 3 | 2 | 0 |
| 211 | 5 | 4 | 3 | 1 |
| 217 | 2 | 1 | 1 | 2 |
| 218 | 0 | -3 | -4 | 1 |
| 219 | 0 | 0 | -1 | 2 |
| 221 | 3 | 3 | 3 | 11 |
| 223 | -2 | -2 | -4 | -1 |
| 230 | 0.5 | -0.5 | 0 | 3 |
| 232 | 6 | 6 | 5 | 5 |
| 233 | 2.5 | 4.5 | 3.5 | 6 |
| 237 | 0 | 3 | 7 | 55 |
| 243 | 4 | 23 | 39 | 64 |
| 244 | 0 | 1 | 0 | 4 |
| 245 | 1 | 14 | 11 | 56 |
| 247 | 0 | 0 | 0 | 4 |
| 249 | 0 | 0 | 0 | 0 |
| 254 | 11 | 34 | 60 | 75 |
| 279 | 6 | 4 | 5 | 6 |
| 280 | 5 | 4 | 6 | 18 |
| 284 | 5 | 4 | 5 | 6 |
| 286 | 0 | 0 | 0 | 0 |
| 287 | 0 | 6 | 11 | 56 |
| 316 | 0 | 1 | 0 | 1 |
| 317 | 0 | 1 | 0 | 0 |
| 331 | 0 | 0 | 0 | 0 |
| 335 | 0 | 0 | 0 | 1 |
| 336 | 0 | 0 | 0 | 0 |
| 338 | 0 | 0 | 0 | 1 |
| 340 | 0 | 2 | 0 | 0 |
| 341 | 0 | 0 | 0 | 0 |
| 343 | 0 | 1 | 0 | 0 |
| 347 | 0 | 0 | 0 | 0 |
| 349 | 0 | 0 | 0 | 0 |
| 351 | 0 | 0 | 0 | 0 |
| 353 | 0 | 0 | 0 | 0 |
| 355 | 0 | 0 | 0 | 0 |
| 357 | 0 | 0 | 0 | 0 |
| 359 | 0 | 0 | 0 | 0 |
| 413 | 5 | 3 | 3 | 3 |
| 414 | 3 | 3 | 2 | 2 |
| 415 | 4 | 4 | 2 | 2 |

### Example 14: p53 GRIP assay

Thermo Scientific* BioImage p53-MDM2 Redistribution Assay monitors the protein interaction with MDM2 and cellular translocation of GFP-tagged p53 in response to drug compounds or other stimuli. Recombinant CHO-hIR cells stably express human p53 (1-312) fused to the C-terminus of enhanced green fluorescent protein (EGFP) and PDE4A4-MDM2 (1-124), a fusion protein between PDE4A4 and MDM2 (1-124). They provide a ready-to-use assay system for measuring the effects of experimental conditions on the interaction of p53 and MDM2. Imaging and analysis is performed with a HCS platform.

CHO-hIR cells are regularly maintained in Ham's F12 media supplemented with 1% Penicillin-Streptomycin, 0.5 mg/ml Geneticin, 1 mg/ml Zeocin and 10% FBS. Cells seeded into 96-well plates at the density of 7000 cells/ 100 µL per well 18-24 hours prior to running the assay using culture media. The next day, media is refreshed and PD-177 is added to cells to the final concentration of 3µM to activate foci formation. Control wells are kept without PD-177 solution. 24h post stimulation with PD-177, cells are washed once with Opti-MEM Media and 50 µL of the Opti-MEM Media supplemented with PD-177 (6 µM) is added to cells. Peptides are diluted from 10 mM DMSO stocks to 500 µM working stocks in sterile water, further dilutions made in 0.5% DMSO to keep the concentration of DMSO constant across the samples. Final highest DMSO concentration is 0.5% and is used as the negative control. Cayman Chemicals Cell-Based Assay (-)-Nutlin-3 (10 mM) is used as positive control. Nutlin was diluted using the same dilution scheme as peptides.50 µL of 2X desired concentrations is added to the appropriate well to achieve the final desired concentrations. Cells are then incubated with peptides for 6 h at 37 °C in humidified 5% CO2 atmosphere. Post-incubation period, cells are fixed by gently aspirating out the media and adding 150 µL of fixing solution per well for 20 minutes at room temperature. Fixed cells are washed 4 times with 200 µL PBS per well each time. At the end of last wash, 100 µL of 1µM Hoechst staining solution is added. Sealed plates incubated for at least 30 min in dark, washed with PBS to remove excess stain and PBS is added to each well. Plates can be stored at 4 °C in dark up to 3 days. The translocation of p53/MDM2 is imaged using Molecular translocation module on Cellomics Arrayscan instrument using 10x objective, XF-100 filter sets for Hoechst and GFP. The output parameters were Mean- CircRINGAveIntenRatio (the ratio of average fluorescence intensities of nucleus and cytoplasm (well average)). The minimally acceptable number of cells per well used for image analysis was set to 500 cells.

### Example 15: MCF-7 Breast Cancer Study using SP315, SP249 and SP154

A xenograft study was performed to test the efficacy of SP315, SP249 and SP 154 in inhibiting tumor growth in athymic mice in the MCF-7 breast cancer xenograft model. A negative control stapled peptide. SP252, a point mutation of SP154 (F to A at position 19) was also tested in one group; this peptide had shown no activity in the SJSA-1 *in vitro* viability assay. Slow release 90 day 0.72 mg 17β-estradiol pellets (Innovative Research, Sarasota, FL) were implanted subcutaneously (sc) on the nape of the neck one day prior to tumor cell implantation (Day -1). On Day 0, MCF-7 tumor cells were implanted sc in the flank of female nude (Crl:NU-Foxnlnu) mice. On Day 18, the resultant sc tumors were measured using calipers to determine their length and width and the mice were weighed. The tumor sizes were calculated using the formula (length × width²)/2 and expressed as cubic millimeters (mm³). Mice with tumors smaller than 85.3 mm³ or larger than 417.4 mm³ were excluded from the subsequent group formation. Thirteen groups of mice, 10 mice per group, were formed by randomization such that the group mean tumor sizes were essentially equivalent (mean of groups ± standard deviation of groups = 180.7 ± 17.5 mm³).

SP315, SP249, SP154 and SP252 dosing solutions were prepared from peptides formulated in a vehicle containing MPEG(2K)-DSPE at 50 mg/mL concentration in a 10 mM Histidine buffered saline at pH 7. This formulation was prepared once for the duration of the study. This vehicle was used as the vehicle control in the subsequent study.

Each group was assigned to a different treatment regimen. Group 1, as the vehicle negative control group, received the vehicle administered at 8 mL/kg body weight intravenously (iv) three times per week from Days 18-39. Groups 2 and 3 received SP154 as an iv injection at 30 mg/kg three times per week or 40 mg/kg twice a week, respectively. Group 4 received 6.7 mg/kg SP249 as an iv injection three times per week. Groups 5, 6, 7 and 8 received SP315 as an iv injection of 26.7 mg/kg three times per week, 20 mg/kg twice per week, 30 mg/kg twice per week, or 40 mg/kg twice per week, respectively. Group 9 received 30 mg/kg SP252 as an iv injection three times per week.

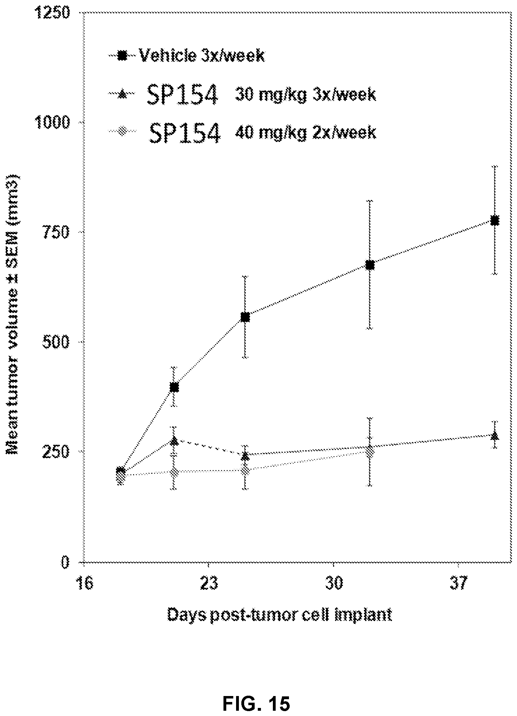

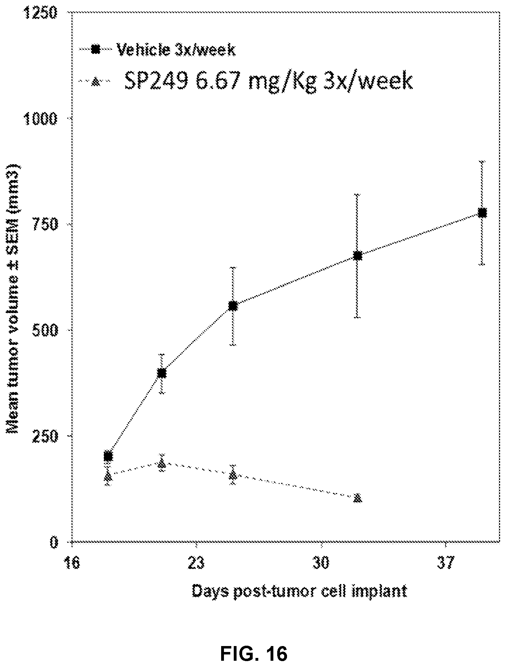

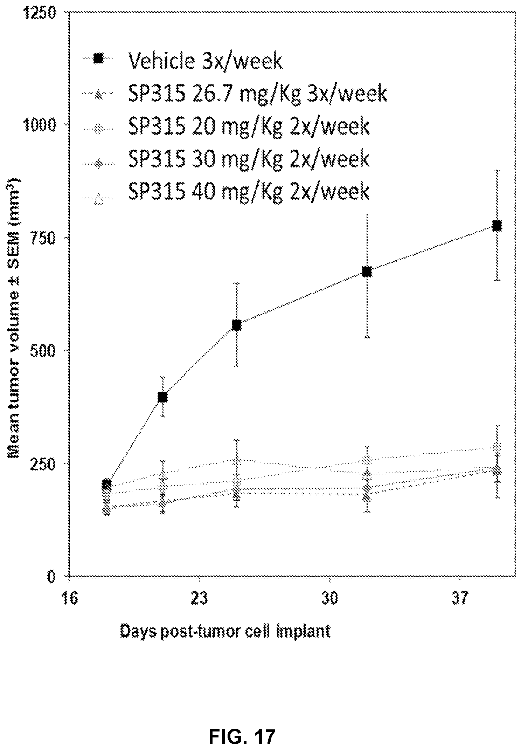

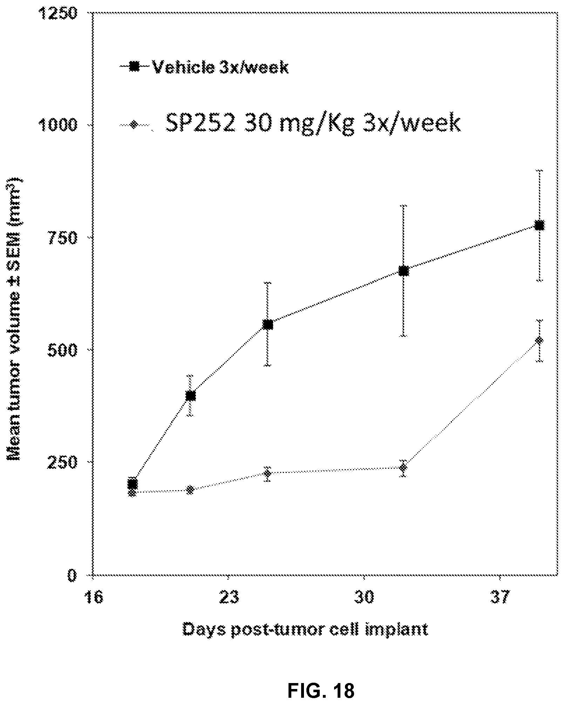

During the dosing period the mice were weighed and tumors measured 1-2 times per week. Results in terms of tumor volume are shown in **Figures 15-18** and tumor growth inhibition compared with the vehicle group, body weight change and number of mice with ≥20% body weight loss or death is shown in Table 9. Tumor growth inhibition (TGI) was calculated as %TGI=100-[(TuVol^{Treated-day x}-TuVol^{Treated-day18})/(TuVol^{Vehicle negative control-day x}-TuVol^{Vehicle negative control - day 18})^{∗} 100, where x= day that effect of treatment is being assessed. Group 1, the vehicle negative control group, showed good tumor growth rate for this tumor model.

For SP154, in the group dosed with 40 mg/kg twice a week 2 mice died during treatment, indicating that this dosing regimen was not tolerable. The dosing regimen of 30 mg/kg of SP154 three times per week was well-tolerated and yielded a TGI of 84%.

For SP249, the group dosed with 6.7 mg/kg three times per week 4 mice died during treatment, indicating that this dosing regimen was not tolerable.

All dosing regimens used for SP315 showed good tolerability, with no body weight loss or deaths noted. Dosing with 40 mg/kg of SP315 twice per week produced the highest TGI (92%). The dosing regimens of SP315 of 26.7 mg/kg three times per week, 20 mg/kg twice per week, 30 mg/kg twice per week produced TGI of 86, 82, and 85%, respectively.

For SP252, the point mutation of SP154 which shows no appreciable activity in *in vitro* assays, dosing with 30 mg/kg three times per week was well-tolerated with no body weight loss or deaths noted. While TGI of 88% was noted by Day 32, that TGI was reduced to 41% by Day 39.

Results from this Example are shown in **Figures 15-18** and are summarized in Table 9.

**Table 9**

| **Group Number** | **Treatment Group** | **% BW Change** | **No. with ≥ 10% BW Loss** | **No. with ≥ 20% BW Loss or death** | **% TGI** |
|---|---|---|---|---|---|
| 1 | Vehicle | +8.6 | 0/10 | 0/10 | - |
| 2 | SP154 30 mg/kg 3x/wk iv | +5.7 | 0/10 | 0/10 | *84 |
| 3 | SP154 40 mg/kg 2x/wk iv | N/A | 0/10 | 2/10 (2 deaths) | Regimen not tolerated |
| 4 | SP249 6.7 mg/kg 3x/wk iv | N/A | 6/10 | 4/10 | Regimen not tolerated |
| 5 | SP315 26.7 mg/kg 3x/wk iv | +3.7 | 0/10 | 0/10 | *86 |
| 6 | SP315 20 mg/kg 2x/wk iv | +3.9 | 0/10 | 0/10 | *82 |
| 7 | SP315 30 mg/kg 2x/wk iv | +8.0 | 0/10 | 0/10 | *85 |
| 8 | SP315 40 mg/kg 2x/wk iv | +2.1 | 0/10 | 0/10 | *92 |
| 9 | SP252 30 mg/kg 3x/wk iv | +3.3 | 0/10 | 0/10 | *41 |

| | | | | | |
|---|---|---|---|---|---|
| ***p ≤ 0.05 Vs Vehicle Control** | | | | | |

### Example 16: Solubility Determination for Peptidomimetic Macrocycles

Peptidomimetic macrocycles are first dissolved in neat N, N-dimethylacetamide (DMA, Sigma-Aldrich, 38840-1L-F) to make 20X stock solutions over a concentration range of 20-140 mg/mL. The DMA stock solutions are diluted 20-fold in an aqueous vehicle containing 2% Solutol-HS-15, 25 mM histidine, 45 mg/mL mannitol to obtain final concentrations of 1-7 mg/ml of the peptidomimetic macrocycles in 5% DMA, 2% Solutol-HS-15, 25 mM histidine, 45 mg/mL mannitol. The final solutions are mixed gently by repeat pipetting or light vortexing, and then the final solutions are sonicated for 10 min at room temperature in an ultrasonic water bath. Careful visual observation is then performed under hood light using a 7x visual amplifier to determine if precipitate exists on the bottom or as a suspension. Additional concentration ranges are tested as needed to determine the maximum solubility limit for each peptidomimetic macrocycle.

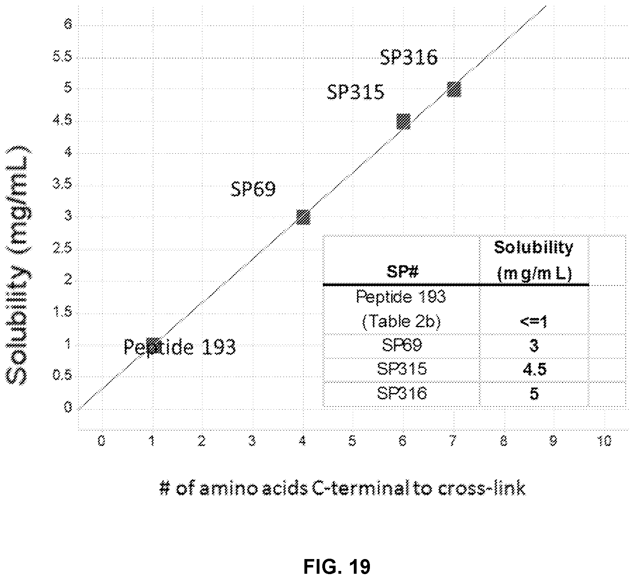

Results from this Example are shown in **Figure 19**.

### Example 17: Preparation of Peptidomimetic Macrocycles using a Boc-protected amino acid.

Peptidomimetic macrocycle precursors were prepared as described in Example 2 comprising an R8 amino acid at position "i" and an S5 amino acid at position "i+7". The amino acid at position "i+3" was a Boc-protected tryptophan which was incorporated during solid-phase synthesis. Specifically, the Boc-protected tryptophan amino acid shown below (and commercially available, for example, from Novabiochem) was using during solid phase synthesis:

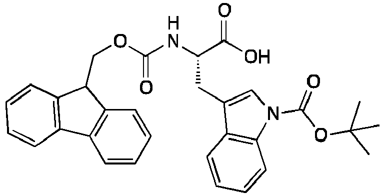

Metathesis was performed using a ruthenium catalyst prior to the cleavage and deprotection steps. The composition obtained following cyclization was determined by HPLC analysis to contain primarily peptidomimetic macrocycles having a crosslinker comprising a trans olefin ("iso2", comprising the double bond in an E configuration). Unexpectedly, a ratio of 90: 10 was observed for the *trans* and *cis* products, respectively.

### Example 18: Testing of peptidomimetic macrocycles for ability to reduce immune checkpoint protein expression or inhibit immune checkpoint protein activity

HCT-116 cells that are p53^{WT} (but not p53 null) upregulate p53 and down-regulate PD-L1 in response to dosing with Nutlin3. p53 effects on PD-L1 are mediated by transcription of miR-34a, -b and -c. The peptidomimetic macrocycles described herein can increase p53 levels in cancer cells. p53 expression is inversely correlated with PD-L1 in patients with NSCLC and PD-L1 expression is higher in patients with mutant p53 compared to p53^{WT}. Patients with low PD-L1 expression and high p53 expression have better survival compared to patients with high PD-L1 expression and low p53 expression. p53 regulates PD-L1 and the miR-34 family downregulates PD-L1 expression by directly repressing PD-L1. Furthermore, therapeutic delivery of miR-34a represses PD-L1 *in vivo* and therapeutic delivery of miR-34a alone or in combination with XRT increases CD8+ T cells. Therapeutic delivery of miR-34a also increases IFN-γ promoting tumor growth delay. miR-34a is directly transactivated by p53 to regulate several pathways in cancer, including tumor immune evasion.

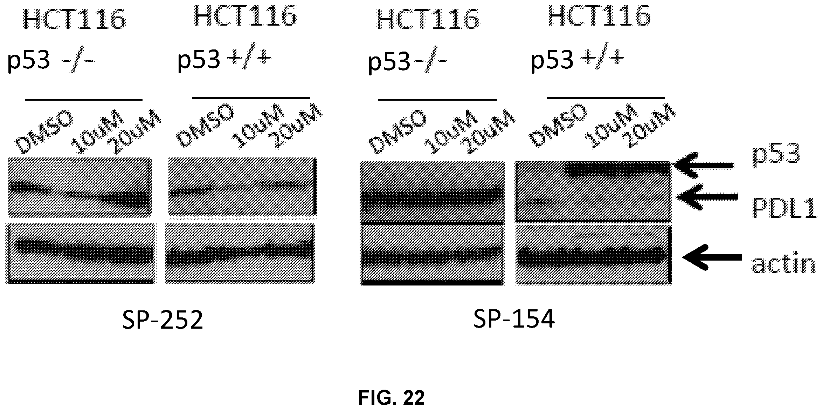

Assays were performed to determine whether the peptidomimetic macrocycles can diminish PD-L1 activity or expression. Briefly, HCT-1 16 p53^{+/+}cells and HCT-1 16 p53^{-/-} cells were treated with DMSO or 10µM SP or 20µM SP as indicated in **Figure 22**. As shown in **Figure 22**, SP treatment led to decreased PD-L1 expression in HCT-116 p53^{+/+}cells, but not HCT-116 p53^{-/-} cells. Similar assays will be performed in cell lines that express higher levels of PD-L1, such as A549 cells, H460 cells, and syngeneic mouse cell lines.

Assays will be performed to determine whether the peptidomimetic macrocycles can diminish PD-L1 activity or expression via miR-34a to enhance immune response against tumors. Assays will be performed to determine whether the peptidomimetic macrocycles of the invention mimic the immune-enhancing effects of anti-PD-1 and/or anti-PD-L1 agents (with added benefit of cell cycle arrest and apoptosis). Briefly, cancer cells from different lineages MCF-7 (breast), HCT-116 (large intestine), MV4-11 (leukemia), DOHH2, and A375 (melanoma) will be dosed with peptidomimetic macrocycles. These cell lines and others will be chosen to include cell lines that have high levels of PD-L1 expression and others that have low levels of PD-L1 expression. Changes in protein and mRNA levels of PD-1, PD-L1 and miR-34a (and p53 and p21 as controls) will be measured, for example, using flow cytometry. RT-PCR assays will be conducted to quantify miR-34a, miR-34b, and/or miR-34c levels in samples taken by FlowMetric in parallel with flow cytometry measurements. Full dose-response curves will be taken 24, 48, and 72 hours after dosing. Additionally, apoptosis measurements will be taken in parallel.

### Example 19: WST-1 Cell Proliferation Assays

The human tumor cell lines MCF-7 and MOLT-3 were obtained from American Type Culture Collection (ATCC) and grown in EMEM and RPMI1640, respectively. All media were supplemented with 10% (v/v) fetal calf serum, 100 units penicillin and 100 µg/ml streptomycin at 37 °C and 5% CO₂. Prior to dosing, MCF-7 cells were switched to serum free medium and grown at 37 °C overnight.

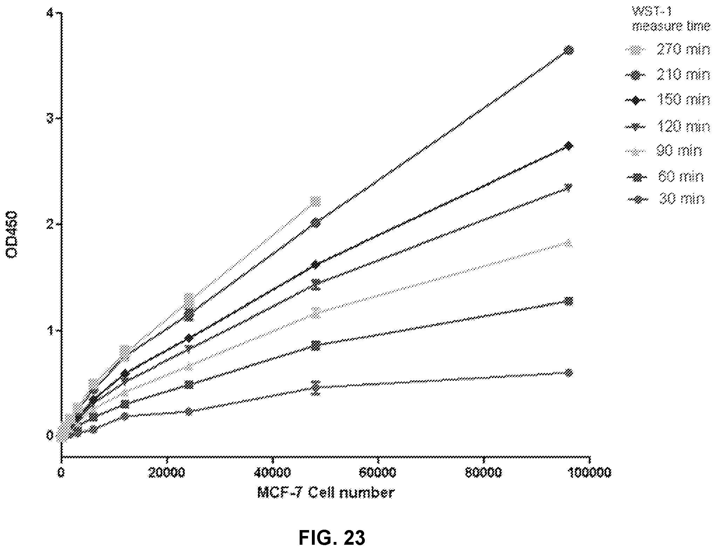

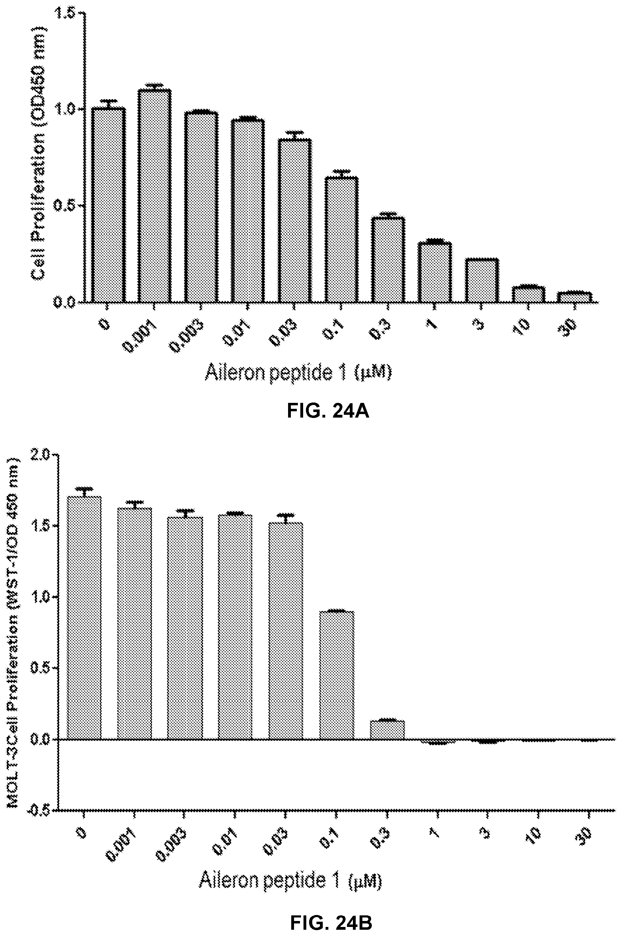

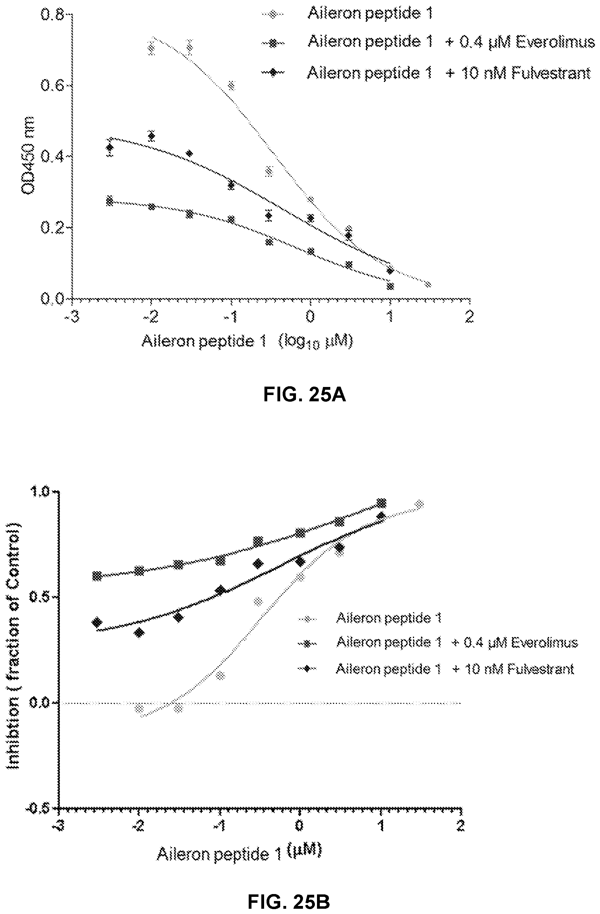

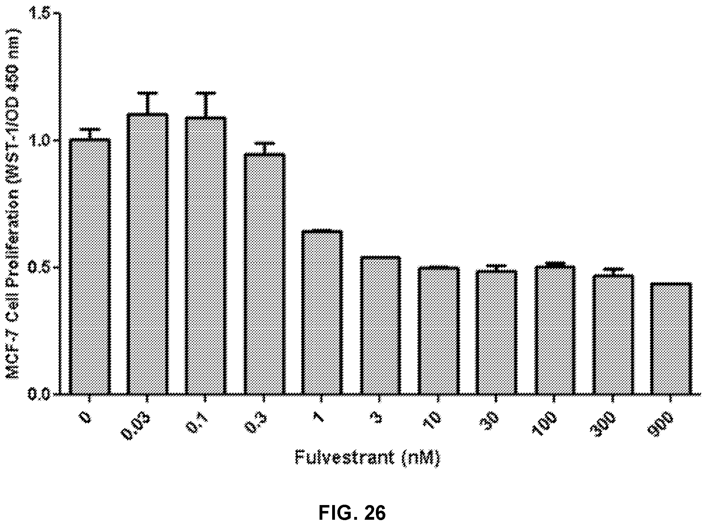

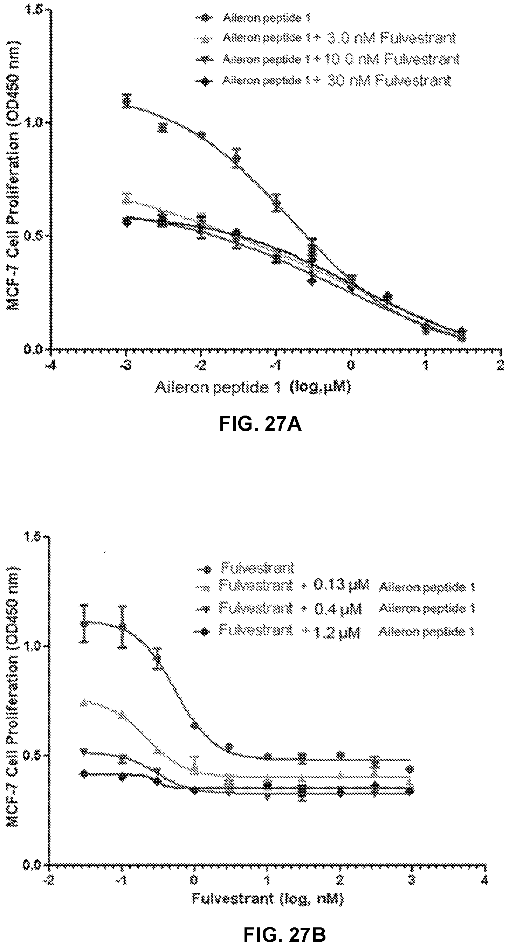

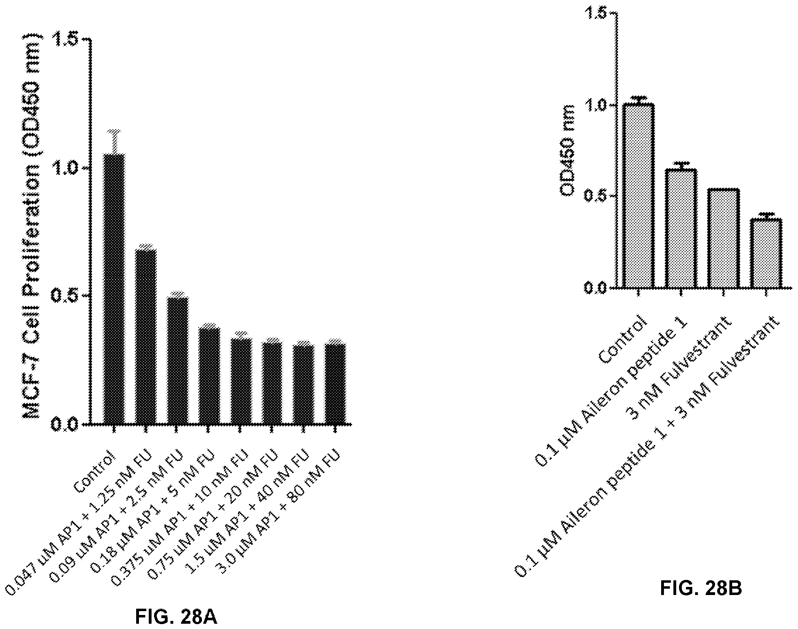

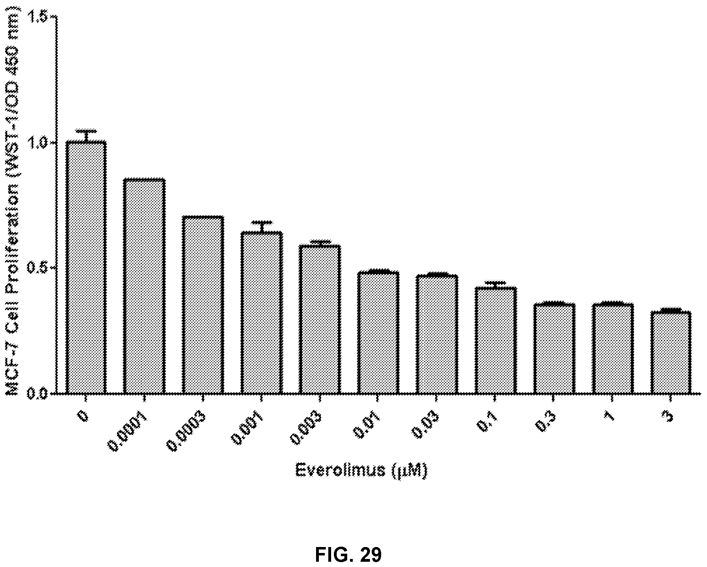

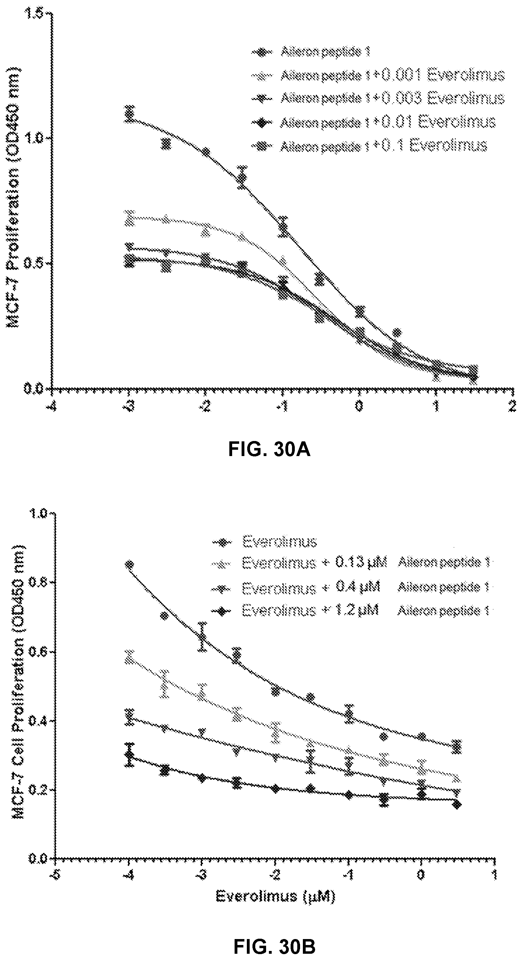

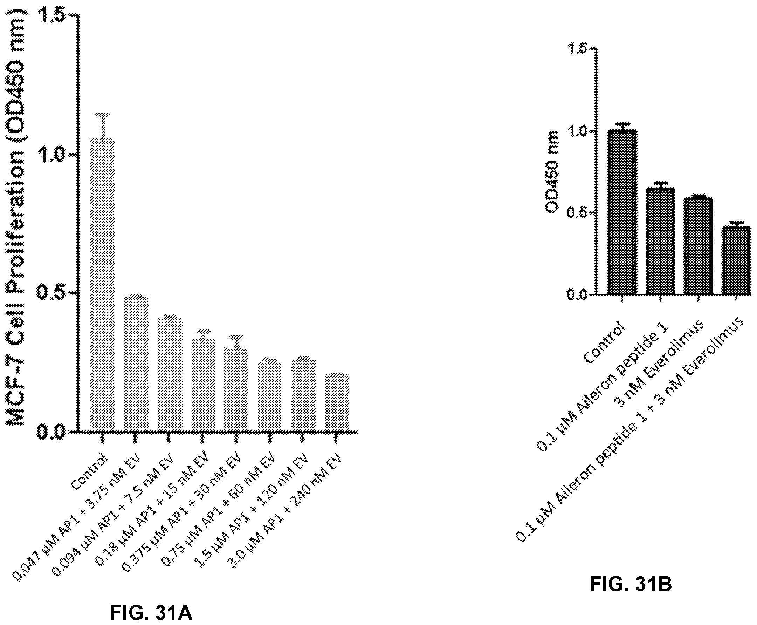

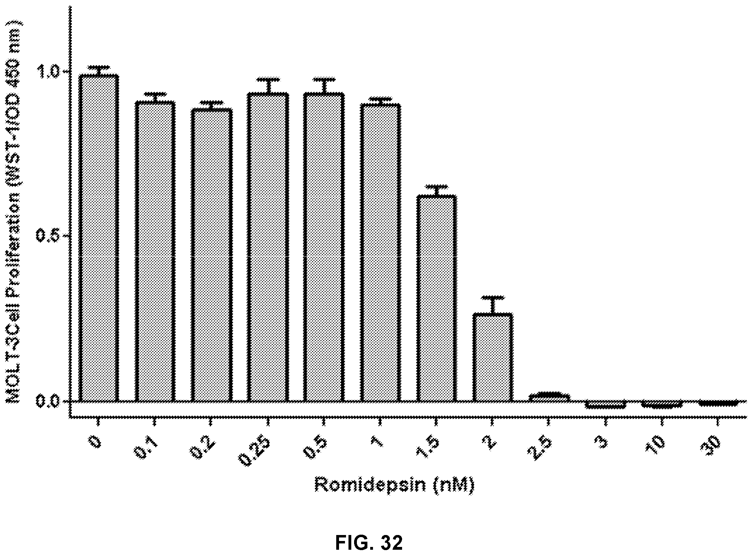

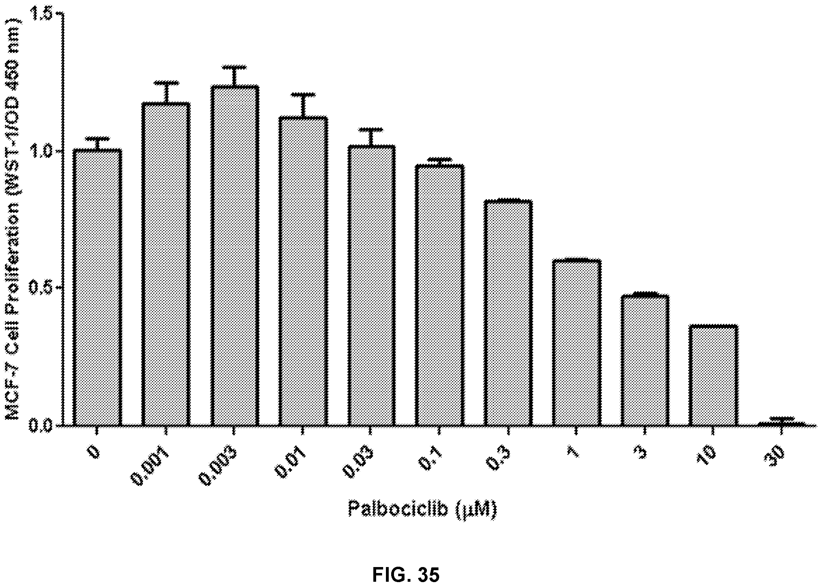

One day prior to assaying, cells were trypsinized, counted and seeded at pre-determined densities in 96-well plates as follows: MCF-7, 5000 cells/well/200 µl; MOLT-3, 30,000 cells/well/200 µl. Cells were dosed with Aileron peptide 1, palbociclib, everolimus, fulvestrant, or romidepsin alone or in combination with Aileron peptide 1 and incubated for three to five days. The WST-1 variant of the MTT assay was used to measure cell viability according to the manufacturer's protocol. WST-1 is a cell-impermeable, sulfonated tetrazolium salt that can be used to examine cell viability without killing the cells. Results can be seen in **Figure 23** (MCF-7 cells, no treatment), **Figure 24** (MCF-7 or MOLT-3 cells, Aileron peptide 1), **Figure 25** (fulvestrant or everolimus), **Figures 26-28** (fulvestrant), **Figures 29-31** (everolimus), **Figures 32-****34** (romidepsin), and **Figures 35-37** (palbociclib).

### Example 20: Synergism between PLX4032 and the peptidomimetic macrocycles of the disclosure in B-Raf-mutant melanoma cell line A375 and Mel-Ho (V600E) but not in Mel-Juso (H- & N-Ras mutations, COSMIC)

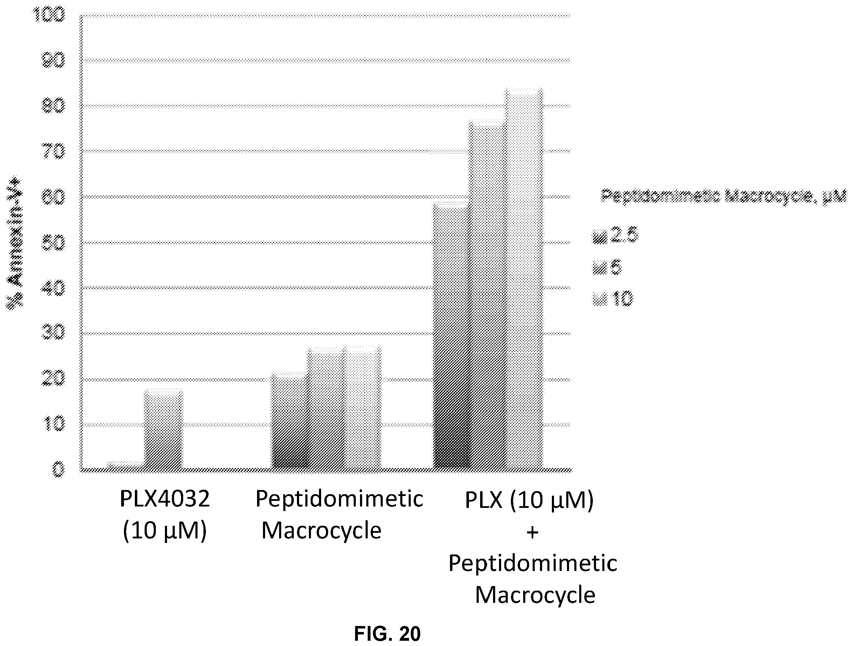

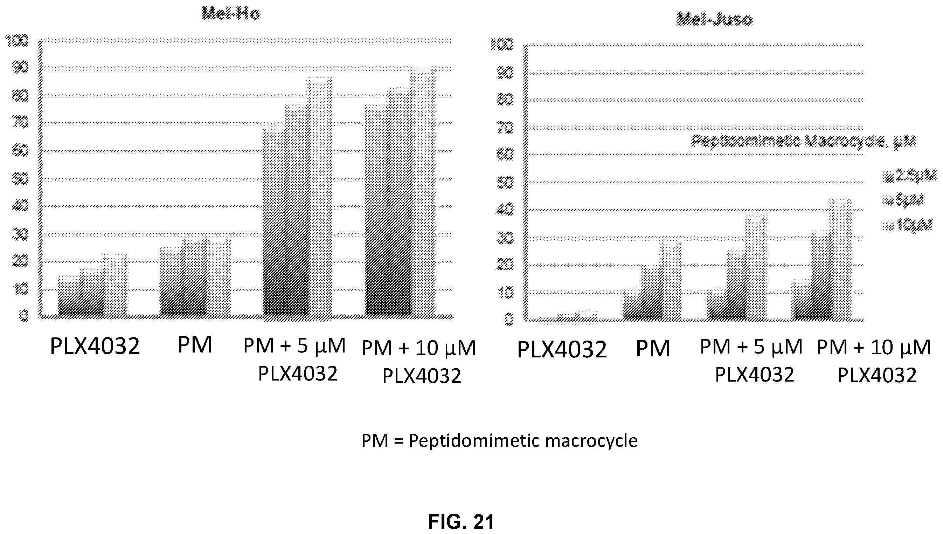

The combination of a representative peptidomimetic macrocycle of the disclosure (a p53 hydrocarbon cross-linked polypeptide macrocycle with an observed mass of 950-975 m/e) and commercially available targeted agent PLX4032 BRAF inhibitor was tested at various drug doses. The EC₅₀ of Aileron peptide 1 on A375 cells was determined to be 70 nM. As seen in **Figure 20**, the peptidomimetic macrocycle displayed synergy with PLX4032 in B-Raf-mutant melanoma cell line A375. As seen in **Figure 21**, the peptidomimetic macrocycle also displayed synergy with PLX4032 in Mel-Ho (V600E) but not in Mel-Juso (H- & N-Ras mutations, COSMIC).

### Example 21: Synergism between fluvestant and the peptidomimetic macrocycles of the disclosure in the MCF-7 breast cancer cell line

The combination of a representative peptidomimetic macrocycle of the disclosure (a p53 hydrocarbon cross-linked polypeptide macrocycle with an observed mass of 950-975 m/e) and commercially available targeted agent fluvestrant was tested at various drug doses. Initially, various MCF-7 cell numbers were plated and evaluated 3-7 days later to determine the optimal number of cells to be plated and treatment duration (**Figure 23**). Next, the optimal number of cells were plated and treated with various concentrations of Aileron peptide 1 or with various concentrations of fluvestrant alone. Cells were evaluated for viability by WST-1 assay or MTT assay 3-7 days after beginning treatment (**Figures 24A** and **26**). A number of concentrations around the IC₅₀ of the peptidomimetic macrocycle and a number of concentrations around the IC₅₀ of fluvestrant were then determined.

| | IC₅₀ (nM) |
|---|---|
| FUL | 0.768 |
| FUL+0.13 µM Aileron peptide 1 | 0.4428 |
| FUL+0.4 µM Aileron peptide 1 | 0.2609 |
| FUL+ 1.2 µM Aileron peptide 1 | 0.2621 |

The EC₅₀ of Aileron peptide 1 on MCF-7 cells was determined to be 410 nM. These chosen concentrations were tested on MCF-7 cells for Aileron peptide 1 in combination with fluvestrant. The optimal number of MCF-7 cells was plated and treated with Aileron peptide 1 and fluvestrant in combination. Aileron peptide 1 was added to the cells simultaneously with the fluvestrant. Cells were evaluated for viability by WST-1 assay or MTT assay 3-7 days after beginning the simultaneous treatments **(****Figures 25**, **27** and **28**). As seen in **Figure 26**, fulvestrant inhibited MCF-7 breast cancer cell proliferation with limited cell killing as a single agent. However, as seen in **Figures 25**, **27** and **28**, Aileron peptide 1 displayed synergy with fluvestant in the MCF-7 breast cancer cell line. Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism.

Exemplary cooperativity index calculations are shown in the table below:

| **Dose Aileron peptide 1 (µM)** | **Dose fulvestrant (nM)** | **Effect** | **CI** |
|---|---|---|---|
| 0.001 | 3.0 | 0.323 | 0.14159 |
| 0.003 | 3.0 | 0.402 | 0.09317 |
| 0.01 | 3.0 | 0.418 | 0.10712 |
| 0.03 | 3.0 | 0.482 | 0.12223 |
| 0.1 | 3.0 | 0.588 | 0.17027 |
| 0.3 | 3.0 | 0.644 | 0.34356 |
| 1.0 | 3.0 | 0.709 | 0.77439 |
| 3.0 | 3.0 | 0.755 | 1.74401 |
| 10.0 | 3.0 | 0.901 | 1.62697 |
| 30.0 | 3.0 | 0.92 | 3.70727 |
| 0.001 | 10.0 | 0.429 | 0.23789 |
| 0.003 | 10.0 | 0.414 | 0.26661 |
| 0.01 | 10.0 | 0.466 | 0.21426 |
| 0.03 | 10.0 | 0.519 | 0.19805 |
| 0.1 | 10.0 | 0.594 | 0.22753 |
| 0.3 | 10.0 | 0.701 | 0.28387 |
| 1.0 | 10.0 | 0.737 | 0.68105 |
| 3.0 | 10.0 | 0.786 | 1.43567 |
| 10.0 | 10.0 | 0.911 | 1.42122 |
| 30.0 | 10.0 | 0.946 | 2.26507 |
| 0.001 | 30.0 | 0.43 | 0.70343 |
| 0.003 | 30.0 | 0.418 | 0.76190 |
| 0.01 | 30.0 | 0.443 | 0.67686 |
| 0.03 | 30.0 | 0.478 | 0.60025 |
| 0.1 | 30.0 | 0.586 | 0.42426 |
| 0.3 | 30.0 | 0.6 | 0.66109 |
| 1.0 | 30.0 | 0.718 | 0.84264 |
| 3.0 | 30.0 | 0.758 | 1.79040 |
| 10.0 | 30.0 | 0.897 | 1.73116 |
| 30.0 | 30.0 | 0.917 | 3.89829 |
| 0.13 | 0.03 | 0.269 | 0.90978 |
| 0.13 | 0.1 | 0.321 | 0.68139 |
| 0.13 | 0.3 | 0.486 | 0.30536 |
| 0.13 | 1.0 | 0.552 | 0.23162 |
| 0.13 | 3.0 | 0.63 | 0.17212 |
| 0.13 | 10.0 | 0.61 | 0.24727 |
| 0.13 | 30.0 | 0.611 | 0.40656 |
| 0.13 | 100.0 | 0.594 | 1.07046 |
| 0.13 | 300.0 | 0.58 | 3.09815 |
| 0.13 | 900.0 | 0.627 | 6.70074 |
| 0.4 | 0.03 | 0.492 | 0.89889 |
| 0.4 | 0.1 | 0.524 | 0.77451 |
| 0.4 | 0.3 | 0.58 | 0.59564 |
| 0.4 | 1.0 | 0.666 | 0.39101 |
| 0.4 | 3.0 | 0.675 | 0.38341 |
| 0.4 | 10.0 | 0.693 | 0.38057 |
| 0.4 | 30.0 | 0.685 | 0.49815 |
| 0.4 | 100.0 | 0.66 | 0.98770 |
| 0.4 | 300.0 | 0.679 | 1.92173 |
| 0.4 | 900.0 | 0.667 | 5.45686 |

### Example 22: Synergism between everolimus and the peptidomimetic macrocycles of the disclosure in the MCF-7 breast cancer cell line

The combination of a representative peptidomimetic macrocycle of the disclosure (a p53 hydrocarbon cross-linked polypeptide macrocycle with an observed mass of 950-975 m/e) and commercially available targeted agent everolimus was tested at various drug doses. Initially, various MCF-7 cell numbers were plated and evaluated 3-7 days later to determine the optimal number of cells to be plated and treatment duration (**Figure 23**). Next, the optimal number of cells were plated and treated with various concentrations of Aileron peptide 1 or with various concentrations of everolimus alone. Cells were evaluated for viability by WST-1 assay or MTT assay 3-7 days after beginning treatment (**Figures 24A** and **29**). A number of concentrations around the IC₅₀ of the peptidomimetic macrocycle and a number of concentrations around the IC₅₀ of everolimus were then determined. The EC₅₀ of Aileron peptide 1 on MCF-7 cells was determined to be 410 nM. These chosen concentrations were tested on MCF-7 cells for Aileron peptide 1 in combination with everolimus. The optimal number of MCF-7 cells was plated and treated with Aileron peptide 1 and everolimus in combination. Aileron peptide 1 was added to the cells simultaneously with the everolimus. Cells were evaluated for viability by WST-1 assay or MTT assay 3-7 days after beginning the simultaneous treatments (**Figures 25**, **30** and **31**). As seen in **Figure 29**, everolimus inhibited MCF-7 breast cancer cell proliferation with limited cell killing as a single agent. However, as seen in **Figures 25**, **30** and **31**, Aileron peptide 1 displayed synergy with everolimus in the MCF-7 breast cancer cell line.

Exemplary cooperativity index calculations are shown in the table below:

| **Dose Aileron peptide 1 (µM)** | **Dose everolimus (µM)** | **Effect** | **CI** |
|---|---|---|---|
| 0.001 | 0.001 | 0.363 | 0.46998 |
| 0.003 | 0.001 | 0.365 | 0.45978 |
| 0.01 | 0.001 | 0.406 | 0.23282 |
| 0.03 | 0.001 | 0.429 | 0.21862 |
| 0.1 | 0.001 | 0.516 | 0.22558 |
| 0.3 | 0.001 | 0.703 | 0.23698 |
| 1.0 | 0.001 | 0.811 | 0.39235 |
| 3.0 | 0.001 | 0.864 | 0.74302 |
| 10.0 | 0.001 | 0.952 | 0.65211 |
| 30.0 | 0.001 | 0.964 | 1.37599 |
| 0.001 | 0.003 | 0.469 | 0.18255 |
| 0.003 | 0.003 | 0.495 | 0.11727 |
| 0.01 | 0.003 | 0.508 | 0.10758 |
| 0.03 | 0.003 | 0.557 | 0.08415 |
| 0.1 | 0.003 | 0.609 | 0.14138 |
| 0.3 | 0.003 | 0.722 | 0.21318 |
| 1.0 | 0.003 | 0.819 | 0.36874 |
| 3.0 | 0.003 | 0.871 | 0.69183 |
| 10.0 | 0.003 | 0.945 | 0.77158 |
| 30.0 | 0.003 | 0.952 | 1.95633 |
| 0.001 | 0.01 | 0.524 | 0.21623 |
| 0.003 | 0.01 | 0.537 | 0.17334 |
| 0.01 | 0.01 | 0.525 | 0.22955 |
| 0.03 | 0.01 | 0.554 | 0.17216 |
| 0.1 | 0.01 | 0.623 | 0.15213 |
| 0.3 | 0.01 | 0.716 | 0.22398 |
| 1.0 | 0.01 | 0.799 | 0.42963 |
| 3.0 | 0.01 | 0.854 | 0.81864 |
| 10.0 | 0.01 | 0.933 | 0.98709 |
| 30.0 | 0.01 | 0.953 | 1.90630 |
| 0.001 | 0.1 | 0.515 | 2.53851 |
| 0.003 | 0.1 | 0.541 | 1.56244 |
| 0.01 | 0.1 | 0.522 | 2.24431 |
| 0.03 | 0.1 | 0.563 | 1.07533 |
| 0.1 | 0.1 | 0.645 | 0.31323 |
| 0.3 | 0.1 | 0.735 | 0.22476 |
| 1.0 | 0.1 | 0.783 | 0.48900 |
| 3.0 | 0.1 | 0.844 | 0.89820 |
| 10.0 | 0.1 | 0.909 | 1.45716 |
| 30.0 | 0.1 | 0.925 | 3.41419 |
| 0.13 | 0.0001 | 0.477 | 0.31844 |
| 0.13 | 0.0003 | 0.548 | 0.22849 |
| 0.13 | 0.001 | 0.567 | 0.21454 |
| 0.13 | 0.003 | 0.626 | 0.16282 |
| 0.13 | 0.01 | 0.673 | 0.13216 |
| 0.13 | 0.03 | 0.699 | 0.12434 |
| 0.13 | 0.1 | 0.717 | 0.13805 |
| 0.13 | 0.3 | 0.743 | 0.15137 |
| 0.13 | 1.0 | 0.762 | 0.21739 |
| 0.13 | 3.0 | 0.789 | 0.27115 |
| 0.4 | 0.0001 | 0.633 | 0.45701 |
| 0.4 | 0.0003 | 0.664 | 0.38983 |
| 0.4 | 0.001 | 0.673 | 0.37246 |
| 0.4 | 0.003 | 0.723 | 0.28218 |
| 0.4 | 0.01 | 0.74 | 0.25644 |
| 0.4 | 0.03 | 0.746 | 0.25127 |
| 0.4 | 0.1 | 0.76 | 0.23938 |
| 0.4 | 0.3 | 0.8 | 0.18580 |
| 0.4 | 1.0 | 0.804 | 0.21110 |
| 0.4 | 3.0 | 0.828 | 0.20196 |
| 1.2 | 0.0001 | 0.703 | 0.94557 |
| 1.2 | 0.0003 | 0.746 | 0.73428 |
| 1.2 | 0.001 | 0.768 | 0.63825 |
| 1.2 | 0.003 | 0.783 | 0.57706 |
| 1.2 | 0.01 | 0.798 | 0.51924 |
| 1.2 | 0.03 | 0.798 | 0.52033 |
| 1.2 | 0.1 | 0.816 | 0.45611 |
| 1.2 | 0.3 | 0.832 | 0.40364 |
| 1.2 | 1.0 | 0.814 | 0.49378 |
| 1.2 | 3.0 | 0.845 | 0.39182 |

Analysis was performed according to Chou et al., Advances in Enzyme Regulation, 22:27-55 (1984) and Zhang et al., Am J Cancer Res., 6:97-104 (2016). Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism.

### Example 23: Treatment with romidepsin and the peptidomimetic macrocycles of the disclosure in the human MOLT-3 T-lymphoid cell line

The combination of Aileron peptide 1 and commercially available targeted agent romidepsin was tested at various drug doses. Initially, various MOLT-3 cell numbers were plated and evaluated 3-7 days later to determine the optimal number of cells to be plated and treatment duration. Next, the optimal number of cells were plated and treated with various concentrations of Aileron peptide 1 or with various concentrations of romidepsin alone. Cells were evaluated for viability by WST-1 assay or MTT assay 3-7 days after beginning treatment (**Figures 24B** and **32**). A number of concentrations around the IC₅₀ of the peptidomimetic macrocycle and a number of concentrations around the IC₅₀ of romidepsin were then determined.

| | IC₅₀ (µM) |
|---|---|
| Aileron peptide 1 | 0.088 |
| Aileron peptide 1+0.5 nM Romidepsin | 0.1014 |
| Aileron peptide 1+1.5 nM Romidepsin | 0.038 |
| Aileron peptide 1+3 nM Romidepsin | 0.028 |

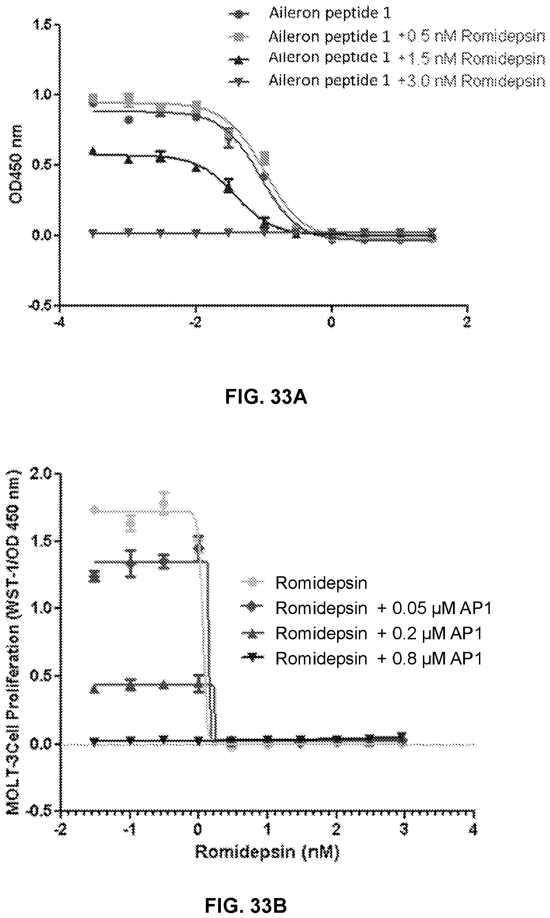

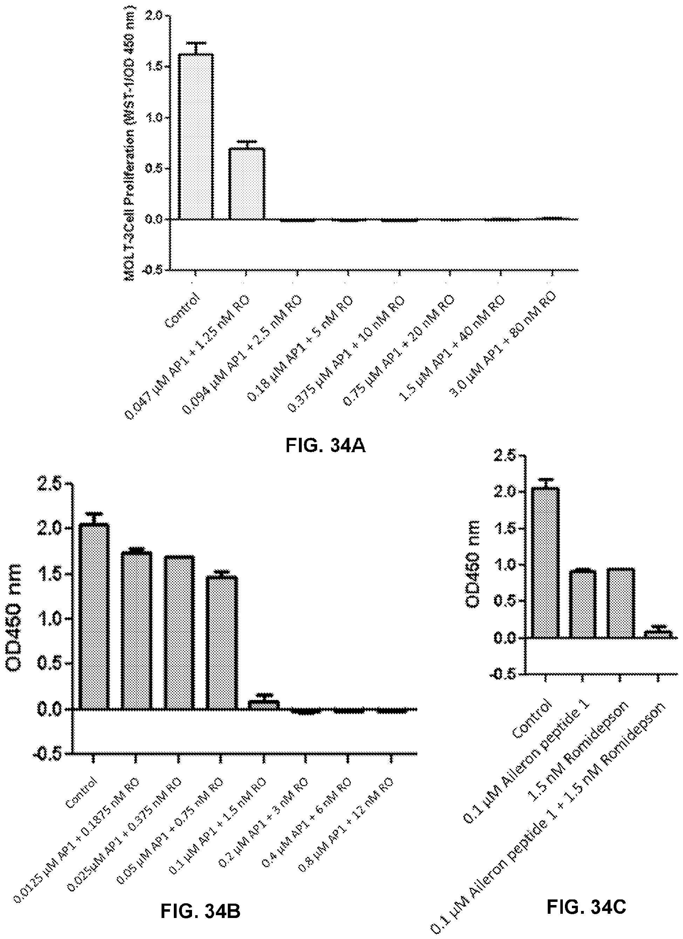

The **EC₅₀** of Aileron peptide 1 on MOLT-3 cells was determined to be 210 nM. These chosen concentrations were tested on MOLT-3 cells for the peptidomimetic macrocycle in combination with romidepsin. The optimal number of MOLT-3 cells was plated and treated with Aileron peptide 1 and romidepsin in combination. Aileron peptide 1 was added to the cells 2 hours prior to addition of the romidepsin. Cells were evaluated for viability by WST-1 assay or MTT assay 3-7 days after beginning the sequential treatment (**Figures 33** and **34**).

### Example 24: Treatment with palbociclib and the peptidomimetic macrocycles of the disclosure in the MCF-7 breast cancer cell line

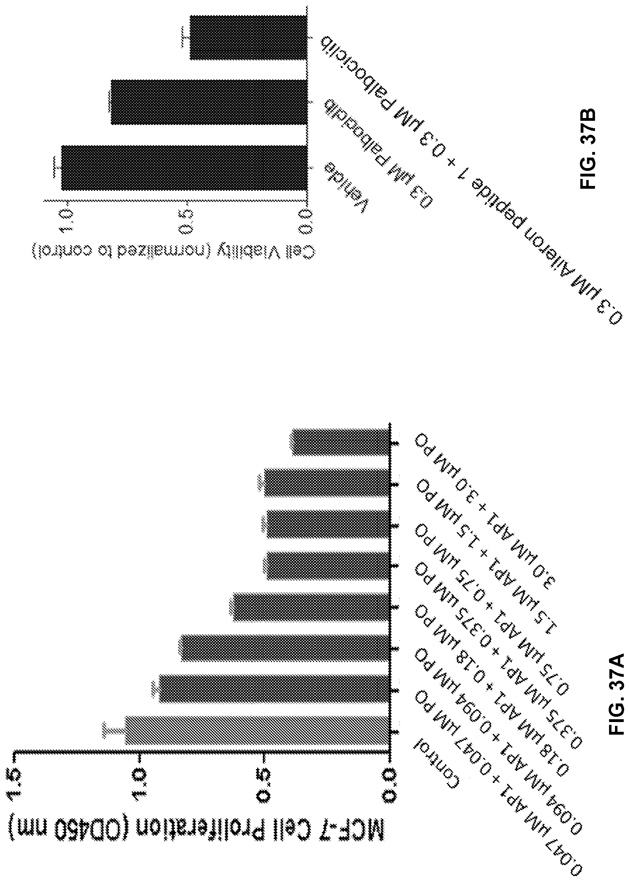

The combination of Aileron peptide 1 and commercially available targeted agent palbociclib was tested at various drug doses. Initially, various MCF-7 cell numbers were plated and evaluated 3-7 days later to determine the optimal number of cells to be plated and treatment duration (**Figure 23**). Next, the optimal number of cells were plated and treated with various concentrations of Aileron peptide 1 or with various concentrations of palbociclib alone. Cells were evaluated for viability by WST-1 assay or MTT assay 3-7 days or 120 hrs after beginning treatment (**Figures 24A** and **35**). A number of concentrations around the IC₅₀ of the peptidomimetic macrocycle and a number of concentrations around the IC₅₀ of palbociclib were then determined. The EC₅₀ of Aileron peptide 1 on MCF-7 cells was determined to be 410 nM. These chosen concentrations were tested on MCF-7 cells for Aileron peptide 1 in combination with palbociclib. The optimal number of MCF-7 cells was plated and treated with Aileron peptide 1 and palbociclib in combination. Aileron peptide 1 was added to the cells simultaneously with the palbociclib. Cells were evaluated for viability by WST-1 assay or MTT assay 3-7 days after beginning the simultaneous treatments **(****Figures 36** and **37****)**.

Exemplary cooperativity index calculations are shown in the table below:

| **Dose Aileron peptide 1 (µM)** | **Dose palbociclib (µM)** | **Effect** | **CI** |
|---|---|---|---|
| 0.001 | 0.3 | 0.178 | 0.59570 |
| 0.003 | 0.3 | 0.184 | 0.59898 |
| 0.01 | 0.3 | 0.223 | 0.54530 |
| 0.03 | 0.3 | 0.25 | 0.62998 |
| 0.1 | 0.3 | 0.325 | 0.79278 |
| 0.3 | 0.3 | 0.532 | 0.68885 |
| 1.0 | 0.3 | 0.65 | 1.13080 |
| 3.0 | 0.3 | 0.743 | 1.92593 |
| 10.0 | 0.3 | 0.924 | 1.17267 |
| 30.0 | 0.3 | 0.945 | 2.32597 |
| 0.4 | 0.001 | 0.585 | 0.57898 |
| 0.4 | 0.003 | 0.553 | 0.67550 |
| 0.4 | 0.01 | 0.55 | 0.68802 |
| 0.4 | 0.03 | 0.545 | 0.71276 |
| 0.4 | 0.1 | 0.556 | 0.70459 |
| 0.4 | 0.3 | 0.608 | 0.61579 |
| 0.4 | 1.0 | 0.592 | 0.90805 |
| 0.4 | 3.0 | 0.614 | 1.46501 |
| 0.4 | 10.0 | 0.698 | 2.61449 |
| 0.4 | 30.0 | 0.999 | 0.02893 |

Cells were also evaluated for viability using the CyQUANT method after beginning treatment (**Figures 78A** **and** **79A**). Cells were evaluated for viability using the CyQUANT method after beginning the simultaneous treatments (**Figures 78B** **and** **79B**). Analysis was performed according to Chou et al., Advances in Enzyme Regulation, 22:27-55 (1984) and Zhang et al., Am J Cancer Res., 6:97-104 (2016). Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism.

### Example 25: Treatment with dexamethasone and the peptidomimetic macrocycles of the disclosure in the DOHH-2 human lymphoma B-cell line

The combination of one or more representative peptidomimetic macrocycles of the disclosure and commercially available targeted agent dexamethasone are tested at various drug doses. Initially, various DOHH-2 cell numbers are plated and evaluated 3-7 days later to determine the optimal number of cells to be plated and treatment duration. Next, the optimal number of cells are plated and treated with various concentrations of a representative peptidomimetic macrocycle of the disclosure or with various concentrations of dexamethasone alone. Cells are evaluated for viability by WST-1 assay or MTT assay 3-7 days after beginning treatment. A number of concentrations around the IC₅₀ of the peptidomimetic macrocycle and a number of concentrations around the IC₅₀ of dexamethasone are then determined. The EC₅₀ of Aileron peptide 1 on DOHH-2 cells was determined to be 60 nM. These chosen concentrations are tested on DOHH-2 cells for the peptidomimetic macrocycle in combination with dexamethasone. The optimal number of DOHH-2 cells are plated and treated with the representative peptidomimetic macrocycle of the disclosure and dexamethasone in combination. In some cases, the peptidomimetic macrocycle are added to the cells simultaneously with the dexamethasone. In some cases, the peptidomimetic macrocycle are added to the cells prior to addition of the dexamethasone. In some cases, the peptidomimetic macrocycle are added to the cells after addition of the dexamethasone. Cells are evaluated for viability by WST-1 assay or MTT assay 3-7 days after beginning the simultaneous or sequential treatments.

### Example 26: Treatment with trametinib and the peptidomimetic macrocycles of the disclosure in the A375 human melanoma cell line

The combination of one or more representative peptidomimetic macrocycles of the disclosure and commercially available targeted agent trametinib are tested at various drug doses. Initially, various A375 cell numbers are plated and evaluated 3-7 days later to determine the optimal number of cells to be plated and treatment duration. Next, the optimal number of cells are plated and treated with various concentrations of a representative peptidomimetic macrocycle of the disclosure or with various concentrations of trametinib alone. Cells are evaluated for viability by WST-1 assay or MTT assay 3-7 days after beginning treatment. A number of concentrations around the IC₅₀ of the peptidomimetic macrocycle and a number of concentrations around the IC₅₀ of trametinib are then determined. The EC₅₀ of Aileron peptide 1 on A375 cells was determined to be 70 nM. These chosen concentrations are tested on A375 cells for the peptidomimetic macrocycle in combination with trametinib. The optimal number of A375 cells are plated and treated with the representative peptidomimetic macrocycle of the disclosure and trametinib in combination. In some cases, the peptidomimetic macrocycle are added to the cells simultaneously with the trametinib. In some cases, the peptidomimetic macrocycle are added to the cells prior to addition of the trametinib. In some cases, the peptidomimetic macrocycle are added to the cells after addition of the trametinib. Cells are evaluated for viability by WST-1 assay or MTT assay 3-7 days after beginning the simultaneous or sequential treatments.

### Example 27: Treatment with rituximab and the peptidomimetic macrocycles of the disclosure in the DOHH-2 human lymphoma B-cell line

The combination of one or more representative peptidomimetic macrocycles of the disclosure and commercially available targeted agent rituximab were tested at various drug doses. Initially, various DOHH-2 cell numbers were plated and evaluated 3-7 days later to determine the optimal number of cells to be plated and treatment duration. Next, the optimal number of cells were plated and treated with various concentrations of a representative peptidomimetic macrocycle of the disclosure or with various concentrations of rituximab alone. Cells were evaluated for viability by WST-1 assay or MTT assay 3-7 days after beginning treatment. A number of concentrations around the IC₅₀ of the peptidomimetic macrocycle and a number of concentrations around the IC₅₀ of rituximab were then determined. The EC₅₀ of Aileron peptide 1 on DOHH-2 cells was determined to be 60 nM. These chosen concentrations were tested on DOHH-2 cells for the peptidomimetic macrocycle in combination with rituximab. The optimal number of DOHH-2 cells were plated and treated with the representative peptidomimetic macrocycle of the disclosure and rituximab in combination. In some cases, the peptidomimetic macrocycle were added to the cells simultaneously with the rituximab. In some cases, the peptidomimetic macrocycle were added to the cells prior to addition of the rituximab. In some cases, the peptidomimetic macrocycle were added to the cells after addition of the rituximab. Cells were evaluated for viability by WST-1 assay or MTT assay 3-7 days after beginning the simultaneous or sequential treatments.

### Example 28: Treatment with obinutuzumab and the peptidomimetic macrocycles of the disclosure in the DOHH-2 human lymphoma B-cell line

The combination of one or more representative peptidomimetic macrocycles of the disclosure and commercially available targeted agent obinutuzumab are tested at various drug doses. Initially, various DOHH-2 cell numbers are plated and evaluated 3-7 days later to determine the optimal number of cells to be plated and treatment duration. Next, the optimal number of cells are plated and treated with various concentrations of a representative peptidomimetic macrocycle of the disclosure or with various concentrations of obinutuzumab alone. Cells are evaluated for viability by WST-1 assay or MTT assay 3-7 days after beginning treatment. A number of concentrations around the IC₅₀ of the peptidomimetic macrocycle and a number of concentrations around the IC₅₀ of obinutuzumab are then determined. The EC₅₀ of Aileron peptide 1 on DOHH-2 cells was determined to be 60 nM. These chosen concentrations are tested on DOHH-2 cells for the peptidomimetic macrocycle in combination with obinutuzumab. The optimal number of DOHH-2 cells are plated and treated with the representative peptidomimetic macrocycle of the disclosure and obinutuzumab in combination. In some cases, the peptidomimetic macrocycle are added to the cells simultaneously with the obinutuzumab. In some cases, the peptidomimetic macrocycle are added to the cells prior to addition of the obinutuzumab. In some cases, the peptidomimetic macrocycle are added to the cells after addition of the obinutuzumab. Cells are evaluated for viability by WST-1 assay or MTT assay 3-7 days after beginning the simultaneous or sequential treatments.

### Example 29: Treatment with dabrafenib and the peptidomimetic macrocycles of the disclosure in the A375 human melanoma cell line

The combination of one or more representative peptidomimetic macrocycles of the disclosure and commercially available targeted agent dabrafenib are tested at various drug doses. Initially, various A375 cell numbers are plated and evaluated 3-7 days later to determine the optimal number of cells to be plated and treatment duration. Next, the optimal number of cells are plated and treated with various concentrations of a representative peptidomimetic macrocycle of the disclosure or with various concentrations of dabrafenib alone. Cells are evaluated for viability by WST-1 assay or MTT assay 3-7 days after beginning treatment. A number of concentrations around the IC₅₀ of the peptidomimetic macrocycle and a number of concentrations around the IC₅₀ of dabrafenib are then determined. The EC₅₀ of Aileron peptide 1 on A375 cells was determined to be 70 nM. These chosen concentrations are tested on A375 cells for the peptidomimetic macrocycle in combination with dabrafenib. The optimal number of A375 cells are plated and treated with the representative peptidomimetic macrocycle of the disclosure and dabrafenib in combination. In some cases, the peptidomimetic macrocycle are added to the cells simultaneously with the dabrafenib. In some cases, the peptidomimetic macrocycle are added to the cells prior to addition of the dabrafenib. In some cases, the peptidomimetic macrocycle are added to the cells after addition of the dabrafenib. Cells are evaluated for viability by WST-1 assay or MTT assay 3-7 days after beginning the simultaneous or sequential treatments.

### Example 30: Treatment with vemurafenib and the peptidomimetic macrocycles of the disclosure in the A375 human melanoma cell line

The combination of one or more representative peptidomimetic macrocycles of the disclosure and commercially available targeted agent vemurafenib are tested at various drug doses. Initially, various A375 cell numbers are plated and evaluated 3-7 days later to determine the optimal number of cells to be plated and treatment duration. Next, the optimal number of cells are plated and treated with various concentrations of a representative peptidomimetic macrocycle of the disclosure or with various concentrations of vemurafenib alone. Cells are evaluated for viability by WST-1 assay or MTT assay 3-7 days after beginning treatment. A number of concentrations around the IC₅₀ of the peptidomimetic macrocycle and a number of concentrations around the IC₅₀ of vemurafenib are then determined. The EC₅₀ of Aileron peptide 1 on A375 cells was determined to be 70 nM. These chosen concentrations are tested on A375 cells for the peptidomimetic macrocycle in combination with vemurafenib. The optimal number of A375 cells are plated and treated with the representative peptidomimetic macrocycle of the disclosure and vemurafenib in combination. In some cases, the peptidomimetic macrocycle are added to the cells simultaneously with the vemurafenib. In some cases, the peptidomimetic macrocycle are added to the cells prior to addition of the vemurafenib. In some cases, the peptidomimetic macrocycle are added to the cells after addition of the vemurafenib. Cells are evaluated for viability by WST-1 assay or MTT assay 3-7 days after beginning the simultaneous or sequential treatments.

### Example 31: Treatment with dabrafenib, vemurafenib and the peptidomimetic macrocycles of the disclosure in the A375 human melanoma cell line

The combination of one or more representative peptidomimetic macrocycles of the disclosure and commercially available targeted agents dabrafenib and vemurafenib are tested at various drug doses. Initially, various A375 cell numbers are plated and evaluated 3-7 days later to determine the optimal number of cells plated and treatment duration. Next, the optimal number of cells are plated and treated with various concentrations of a representative peptidomimetic macrocycle of the disclosure or with various concentrations of vemurafenib alone or with various concentrations of dabrafenib alone. Cells are evaluated for viability by WST-1 assay or MTT assay 3-7 days after beginning treatment. A number of concentrations around the IC₅₀ of the peptidomimetic macrocycle and a number of concentrations around the IC₅₀ of dabrafenib and vemurafenib are then determined. The EC₅₀ of Aileron peptide 1 on A375 cells is determined to be 70 nM. These chosen concentrations are tested on A375 cells for the peptidomimetic macrocycle in combination with dabrafenib and vemurafenib. The optimal number of A375 cells are plated and treated with the representative peptidomimetic macrocycle of the disclosure and dabrafenib and vemurafenib in combination. In some cases, the peptidomimetic macrocycle are added to the cells simultaneously with the dabrafenib and vemurafenib. In some cases, the peptidomimetic macrocycle are added to the cells prior to addition of the dabrafenib and vemurafenib. In some cases, the peptidomimetic macrocycle are added to the cells after addition of the dabrafenib and vemurafenib. Cells are evaluated for viability by WST-1 assay or MTT assay 3-7 days after beginning the simultaneous or sequential treatments.

### Example 32: Treatment with cytarabine (Ara-C), azacitidine, decitabine, and midostaurin with the peptidomimetic macrocycles of the disclosure in the MV4-11 leukemia cancer cell line.

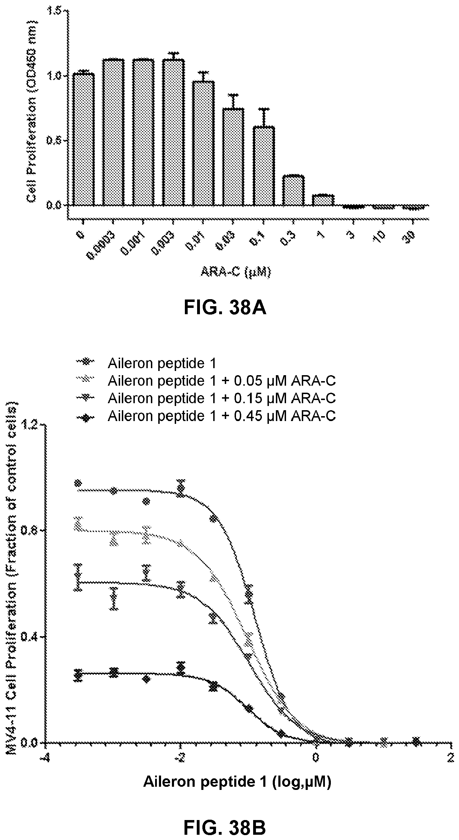

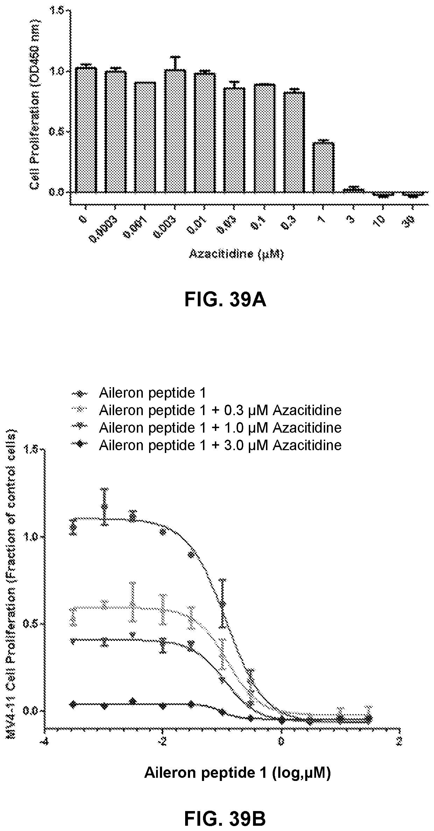

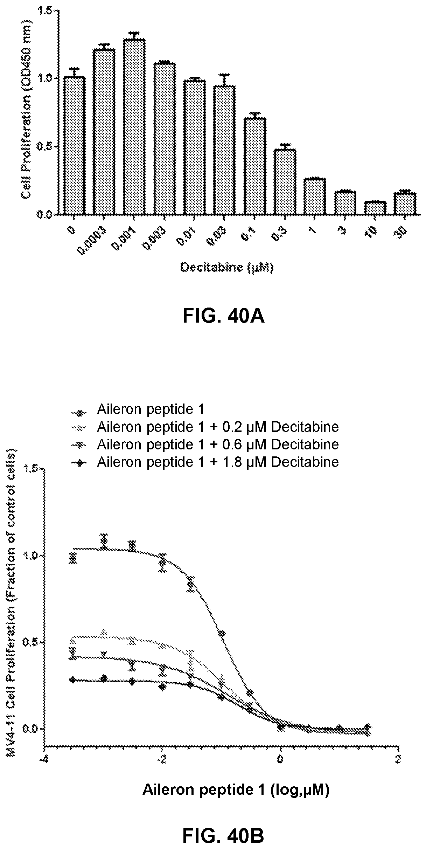

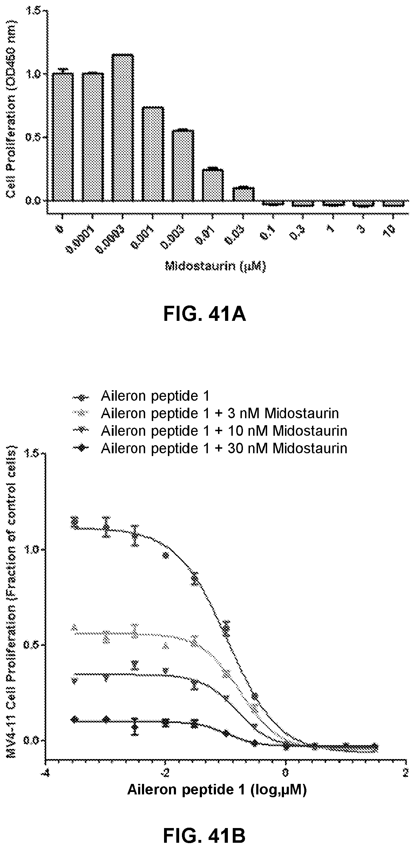

The combinations of Aileron peptide 1 (AP1) and commercially available Ara-C (**Figure 38A**), azacitidine (**Figure 39A**), decitabine (**Figure 40A**), and midostaurin (**Figure 41A**) were tested at various drug doses. Initially, various MV4-1 1 cell numbers were plated and evaluated 3-7 days later to determine the optimal number of cells to be plated and treatment duration.

Next, the optimal number of cells were plated and treated with various concentrations of AP1 or with various concentrations of Ara-C, azacitidine, decitabine, or midostaurin alone. Cells were evaluated for viability by WST-1 assay or MTT assay 3-7 days after beginning treatment. AP1 in combination with Ara-C (**Figure 38B**), azacitidine (**Figure 39B**), decitabine (**Figure 40B**), or midostaurin (**Figure 41B**). All showed complementary *in vitro* anticancer activity. Combination with Ara-C, azacitidine, decitabine, or midostaurin enhanced AP1 inhibition of cancer cell proliferation and cell killing.

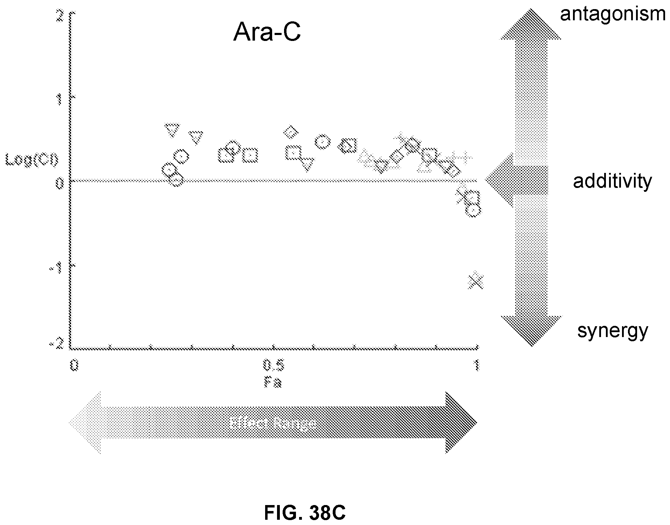

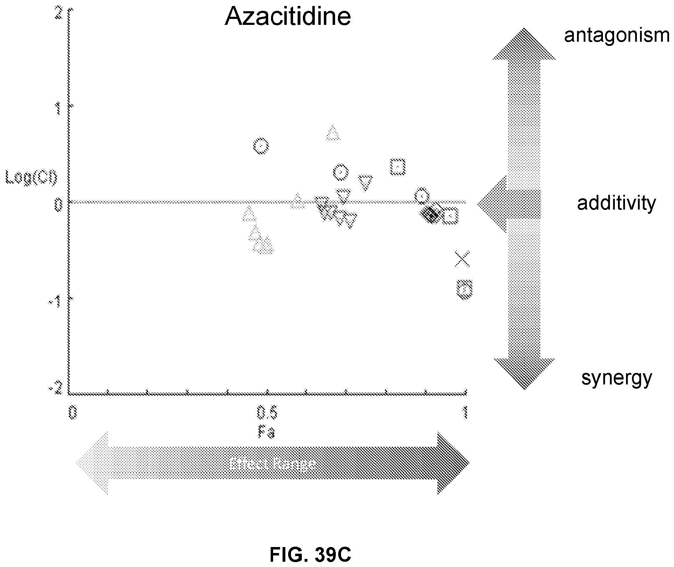

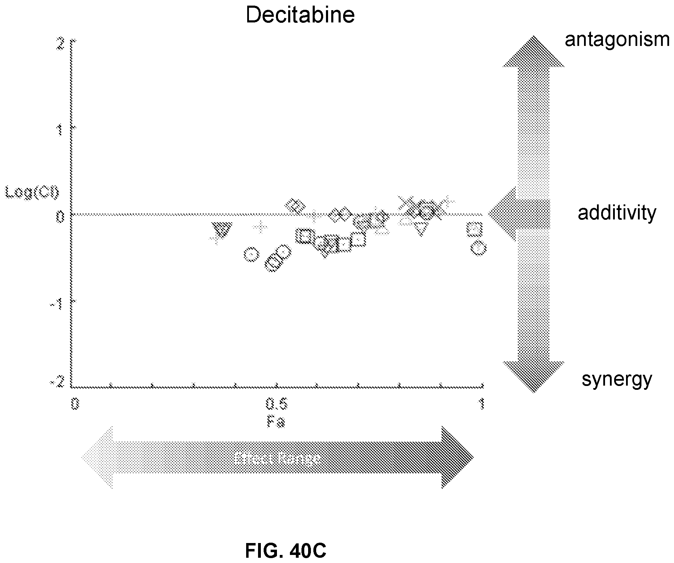

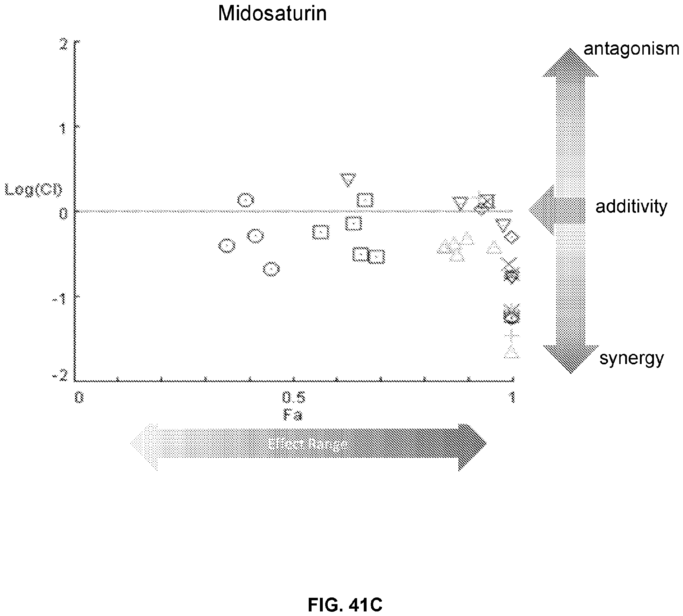

A drug combination index plot was used to assess the synergistic, additive, or antagonistic properties of each combination treatment. The anti-proliferative effect of the Ara-C and AP1 combination was mostly additive with some degree of synergy (**Figure 38C**). The anti-proliferative effect of the azacitidine and AP1 combination was mostly additive with some synergy (**Figure 39C**). The anti-proliferative effect of the decitabine and AP1 combination was mostly additive (**Figure 40C**). The anti-proliferative effect of the midostaurin and AP1 combination was mostly synergistic (**Figure 41C**). Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism.

### Example 33: Treatment with vincristine (VCR) and cyclophosphamide (CTX) with the peptidomimetic macrocycles of the disclosure in the DOHH-2 lymphoma B-cell cancer cell line.

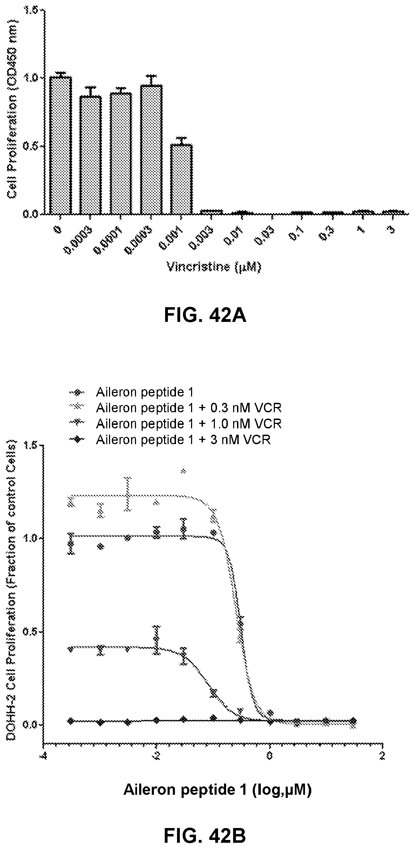

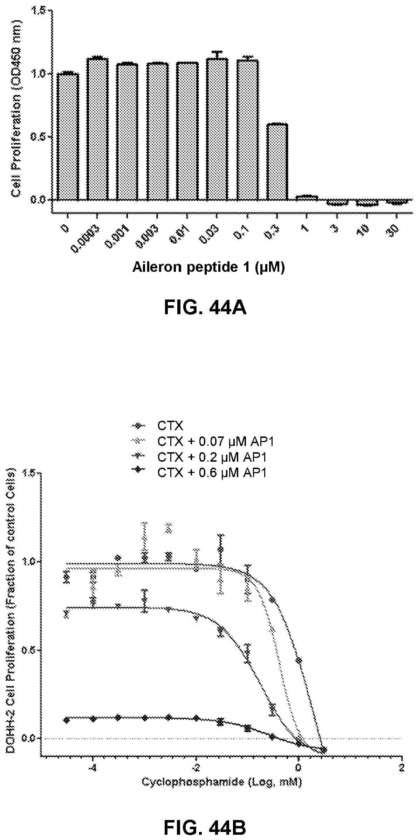

The combinations of AP1 and commercially available VCR (**Figure 42A**) and CTX (**Figure 44A**) were tested at various drug doses. Initially, various DOHH-2 cell numbers were plated and evaluated 3-7 days later to determine the optimal number of cells to be plated and treatment duration.

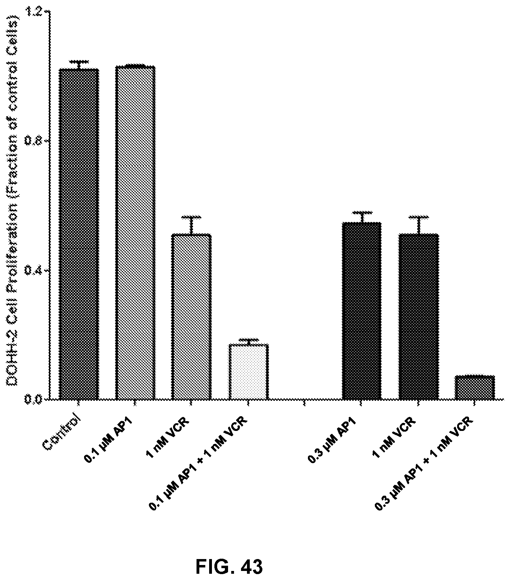

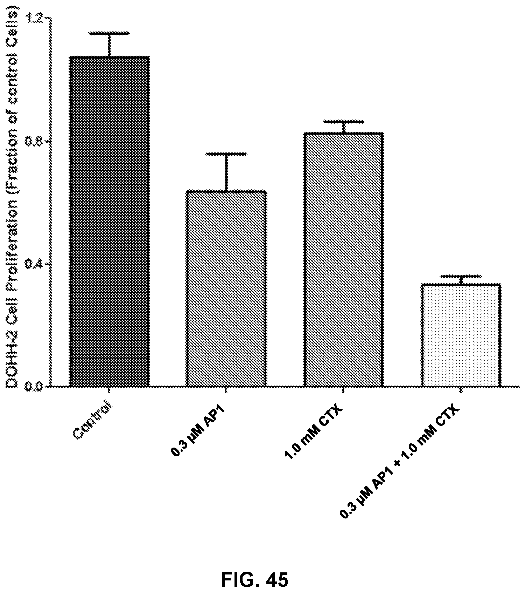

Next, the optimal number of cells were plated and treated with various concentrations of AP1 or with various concentrations of VCR or CTX alone. Cells were evaluated for viability by WST-1 assay or MTT assay 72 hours after beginning treatment with VCR (**Figure 42B**) or CTX (**Figure 44B**). AP1 in combination with VCR showed complementary *in vitro* anticancer activity (**Figure 43**). AP1 in combination with CTX showed complementary *in vitro* anticancer activity (**Figure 45**).

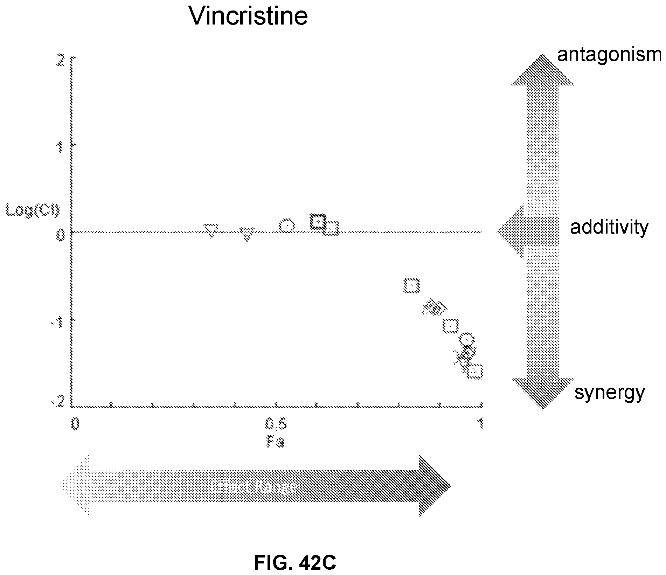

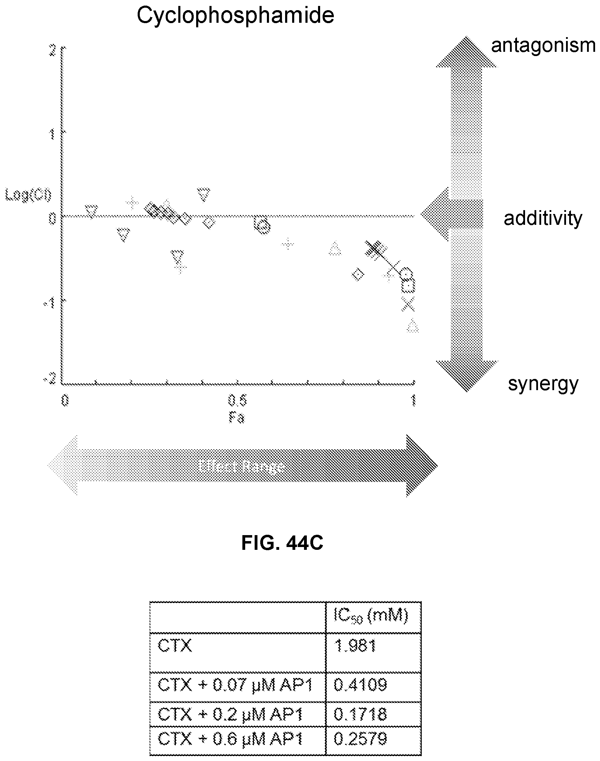

A drug combination index plot was used to assess the synergistic, additive, or antagonistic properties of each combination treatment. The anti-proliferative effect of the VCR and AP1 combination was mostly synergistic (**Figure 42C**). The anti-proliferative effect of the CTX and AP1 combination was synergistic (**Figure 44C**). Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism.

A number of concentrations around the IC₅₀ of the peptidomimetic macrocycle and a number of concentrations around the IC₅₀ of VCR were then determined.

| | IC₅₀ (µM) |
|---|---|
| AP1 | 0.3095 |
| AP1 + 0.3 nM VCR | 0.2520 |
| AP1 + 3 nM VCR | 0.082 |

A number of concentrations around the IC₅₀ of the peptidomimetic macrocycle and a number of concentrations around the IC₅₀ of CTX were then determined.

| | IC₅₀ (mM) |
|---|---|
| CTX | 1.981 |
| CTX + 0.07 µM AP1 | 0.4109 |
| CTX + 0.2 µM AP1 | 0.1718 |
| CTX + 0.6 µM AP1 | 0.2579 |

### Example 34: The order of addition effects on DOHH-2 cell viability using various concentrations of AP1 in combination with VCR.

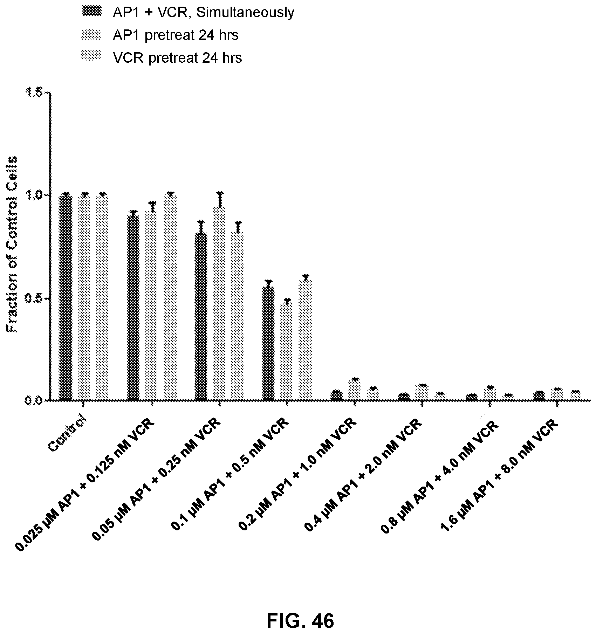

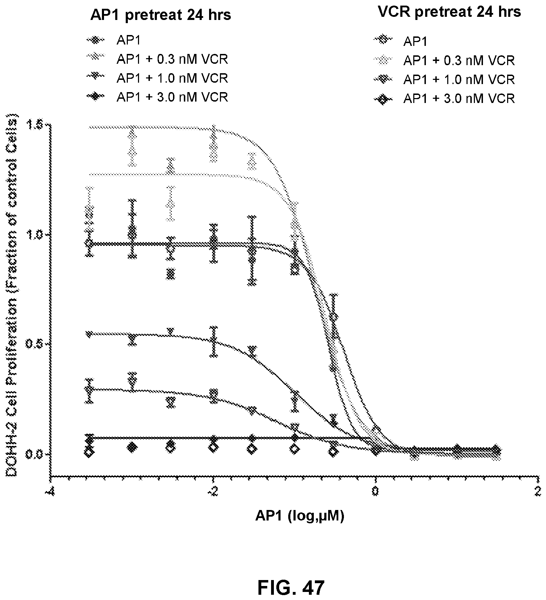

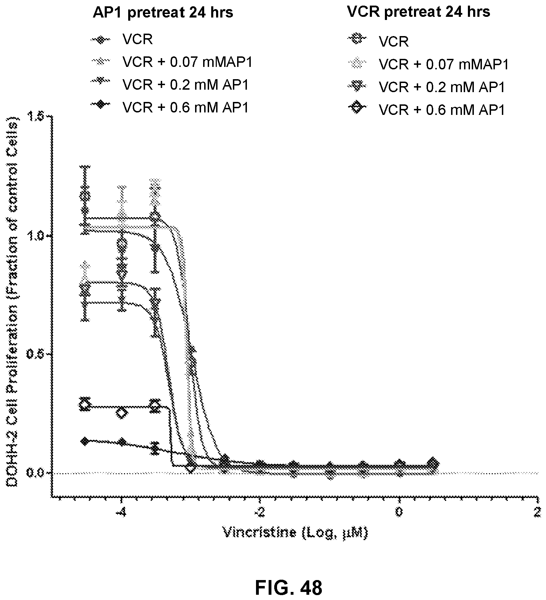

The anticancer activity of the combination treatment was assessed based on the order of addition of the drugs. DOHH-2 cells were sequentially treated by varying concentrations of AP1 and VCR for 72 hrs (**Figure 46**). AP1 suppressed DOHH-2 cell growth with or without VCR (**Figure 47**). Similiarly, VCR suppressed DOHH-2 cell growth with or without AP1 (**Figure 48**).

### Example 35: The order of addition effects on DOHH-2 cell viability using various concentrations of AP1 in combination with CTX.

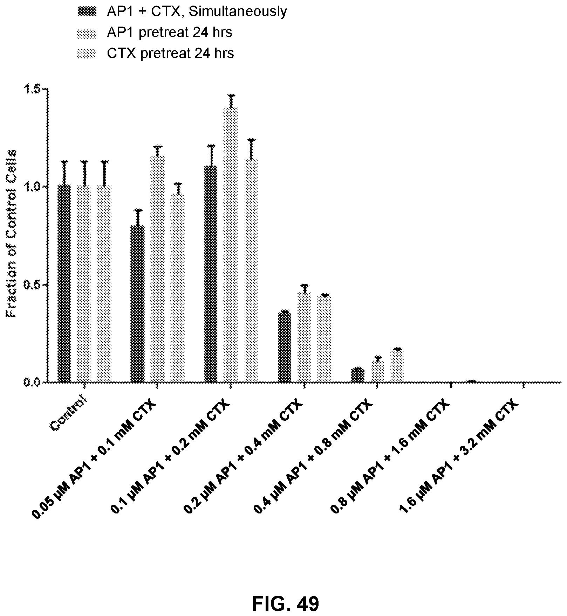

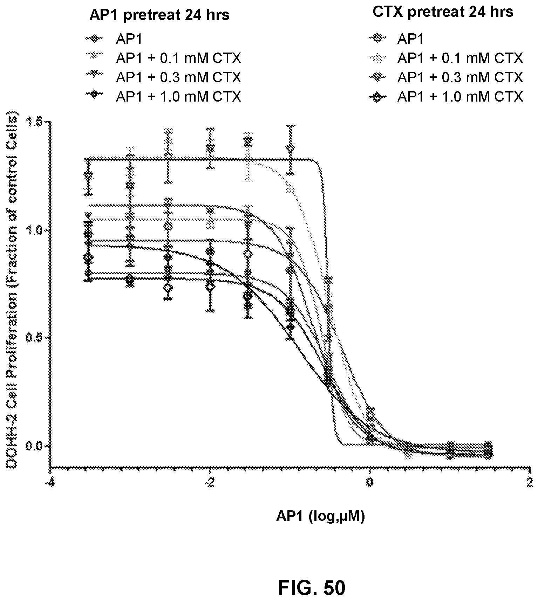

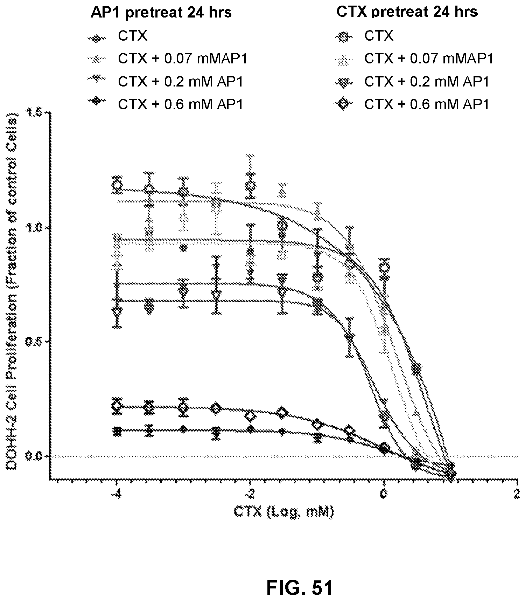

The anticancer activity of the combination treatment was assessed based on the order of addition of the drugs. DOHH-2 cells were sequentially treated by varying concentrations of AP1 and CTX for 72 hrs (**Figure 49**). AP1 suppressed DOHH-2 cell growth with or without CTX (**Figure 50**). Similiarly, CTX suppressed DOHH-2 cell growth with or without AP1 (**Figure 51**).

### Example 36: The order of addition effects on MV4-11 cell viability using various concentrations of AP1 in combination with midostaurin.

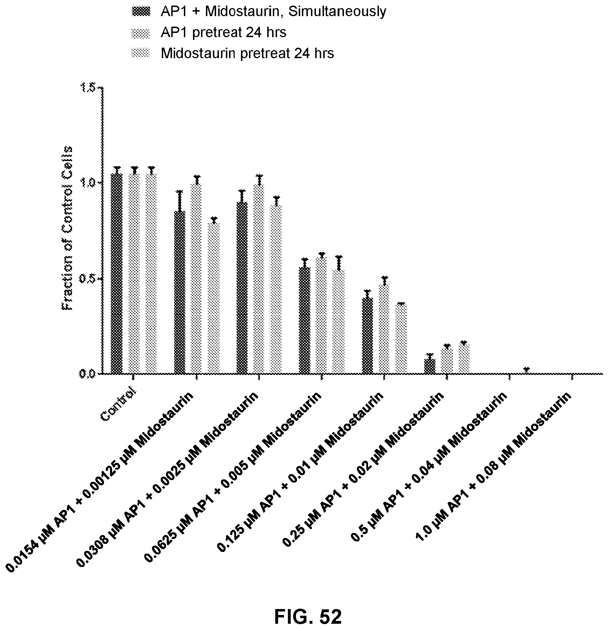

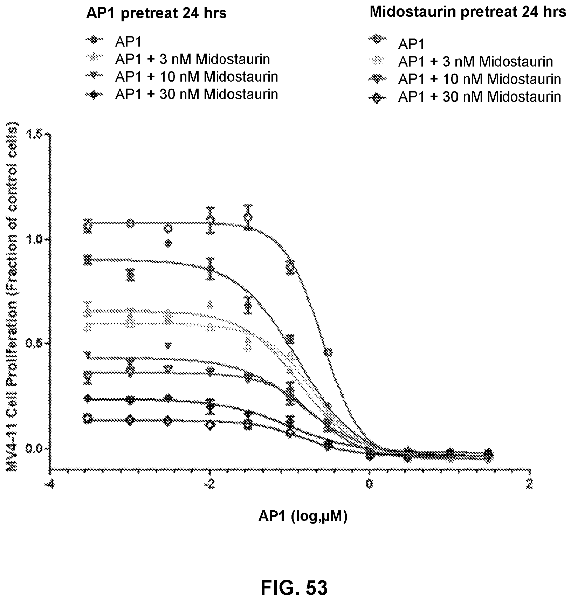

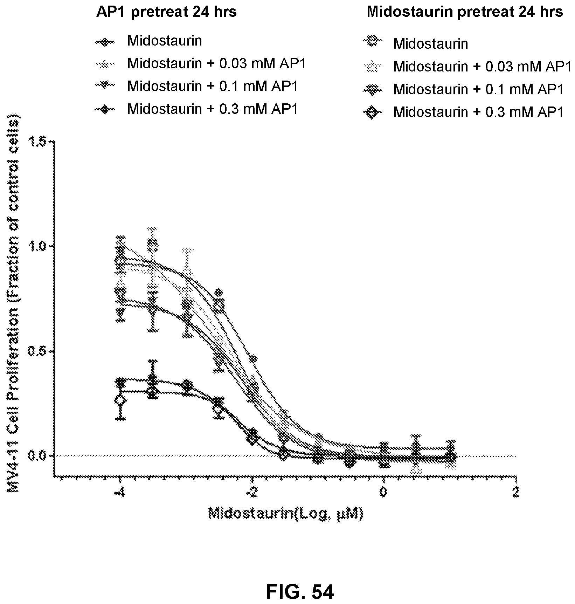

The anticancer activity of the combination treatment was assessed based on the order of addition of the drugs. MV4-1 1 cells were sequentially treated by varying concentrations of AP1 and midostaurin for 72 hrs (**Figure 52**). AP1 suppressed MV4-11 cell growth with or without midostaurin (**Figure 53**). Similiarly, midostaurin suppressed MV4-11 cell growth with or without AP1 (**Figure 54**).

### Example 37: The order of addition effects on MV4-11 cell viability using various concentrations of AP1 in combination with decitabine.

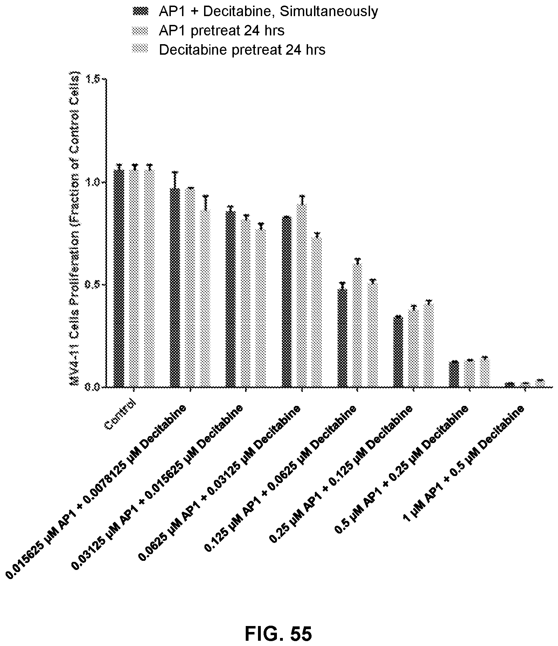

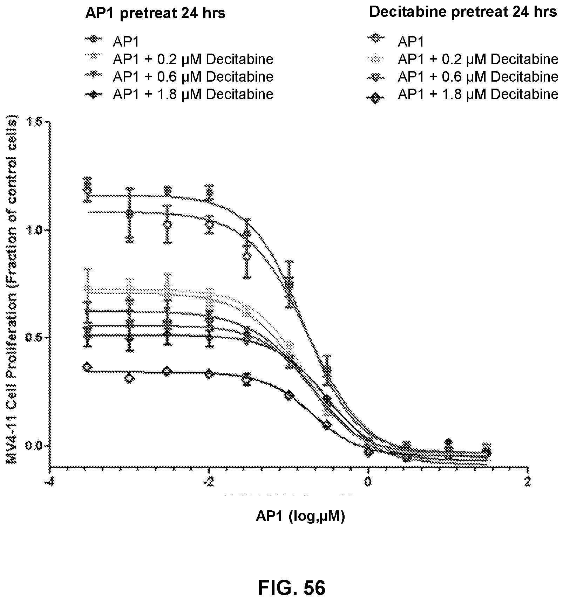

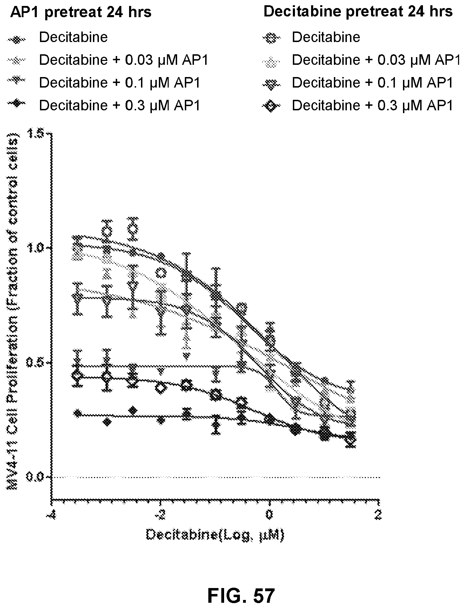

The anticancer activity of the combination treatment was assessed based on the order of addition of the drugs. MV4-1 1 cells were sequentially treated by varying concentrations of AP1 and decitabine for 72 hrs (**Figure 55**). AP1 suppressed MV4-11 cell growth with or without decitabine (**Figure 56**). Similiarly, decitabine suppressed MV4-11 cell growth with or without AP1 (**Figure 57**).

### Example 38: The order of addition effects on MV4-11 cell viability using various concentrations of AP1 in combination with Ara-C.

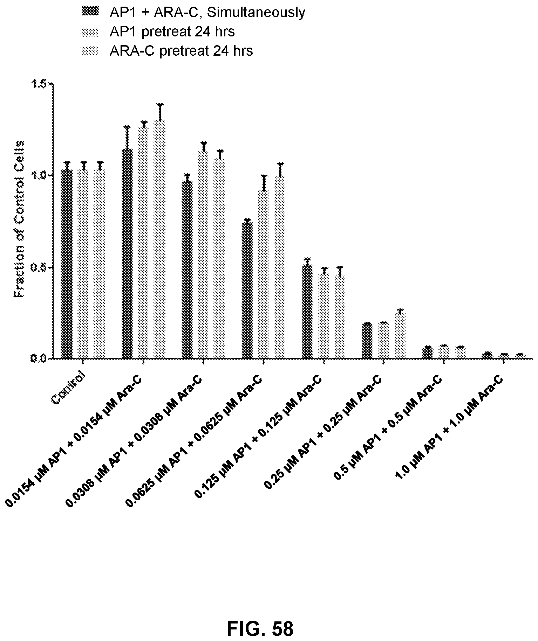

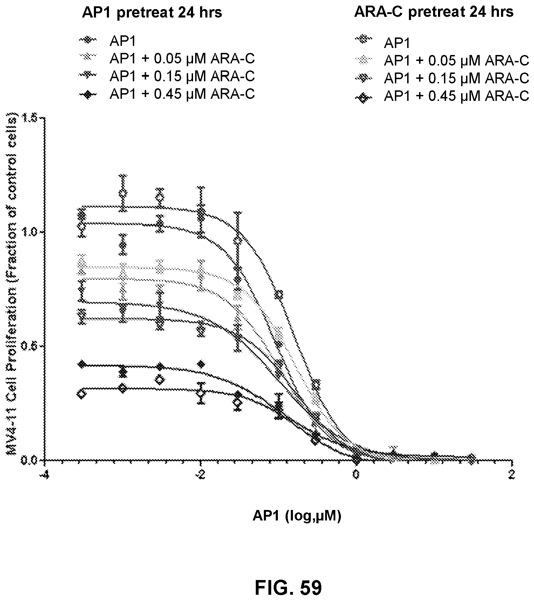

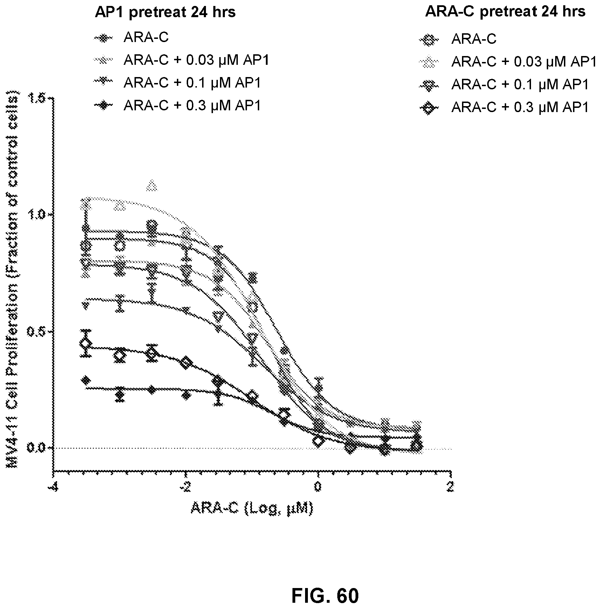

The anticancer activity of the combination treatment was assessed based on the order of addition of the drugs. MV4-1 1 cells were sequentially treated by varying concentrations of AP1 and Ara-C for 72 hrs (**Figure 58**). AP1 suppressed MV4-11 cell growth with or without Ara-C (**Figure 59**). Similiarly, Ara-C suppressed MV4-11 cell growth with or without AP1 (**Figure 60**).

### Example 39: The order of addition effects on MV4-11 cell viability using various concentrations of AP1 in combination with azacitidine.

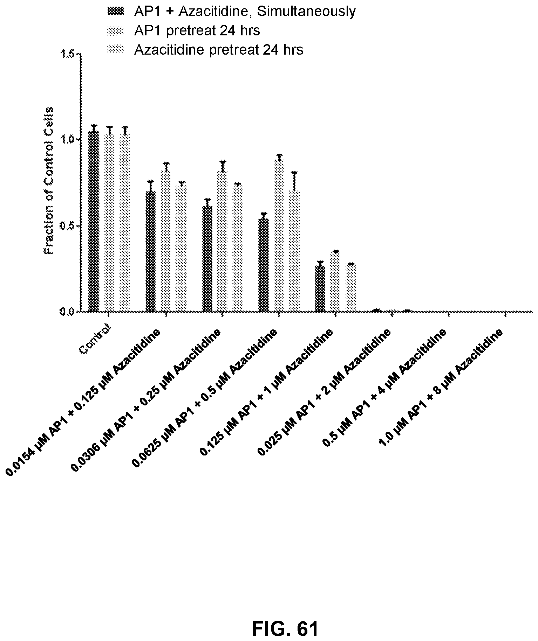

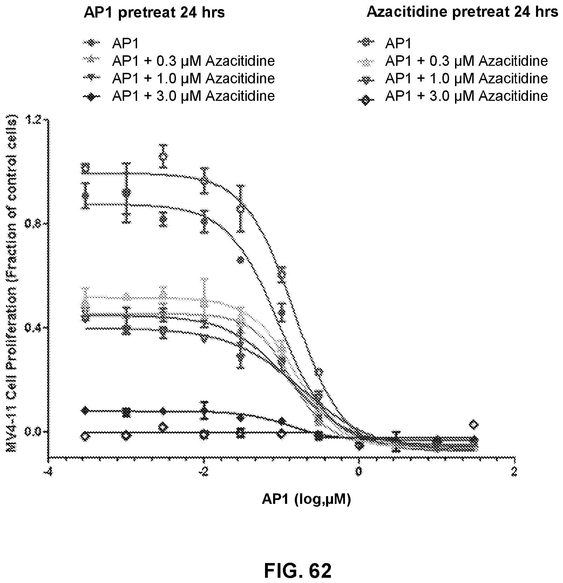

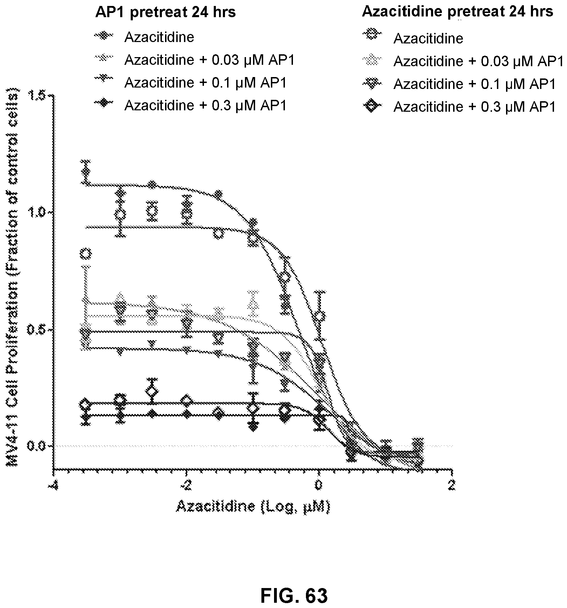

The anticancer activity of the combination treatment was assessed based on the order of addition of the drugs. MV4-1 1 cells were sequentially treated by varying concentrations of AP1 and azacitidine for 72 hrs (**Figure 61**). AP1 suppressed MV4-11 cell growth with or without azacitidine (**Figure 62**). Similiarly, azacitidine suppressed MV4-11 cell growth with or without AP1 (**Figure 63**).

### Example 40: Treatment with fulvestrant (FUL) and everolimus with the peptidomimetic macrocycles of the disclosure in the MCF-7 breast cancer cell line.

The combinations of AP1 and commercially available fulvestrant (**Figures 64A** **and** **65A**) and everolimus (**Figures 66A** **and** **67A**) were tested at various drug doses. Initially, various MCF-7 cell numbers were plated and evaluated 3-7 days later to determine the optimal number of cells to be plated and treatment duration.

Next, the optimal number of cells were plated and treated with various concentrations of AP1 or with various concentrations of fulvestrant or everolimus alone. Cells were evaluated for viability by WST-1 assay or MTT assay 120 hours after beginning treatment. AP1 suppressed MCF-7 cell growth with or without fulvestrant (**Figures 64B** **and** **65B**). AP1 suppressed MCF-7 cell growth with or without everolimus **(****Figures 66B** **and** **67B**).

A number of concentrations around the IC₅₀ of the peptidomimetic macrocycle and a number of concentrations around the IC₅₀ of FUL were then determined.

| | IC₅₀ (nM) |
|---|---|
| FUL | 0.768 |
| FUL + 0.13 µM AP1 | 0.4428 |
| FUL + 0.4 µM AP1 | 0.2609 |
| FUL + 1.2 µM AP1 | 0.2621 |

### Example 41: Treatment with rituximab and romidepsin with the peptidomimetic macrocycles of the disclosure in the MOLT-3 T-lymphoidcancer cell line.

The combinations of AP1 and commercially available rituximab (**Figures 68A** **and** **69A**) and romidepsin (**Figures 71A** **and** **72A**) were tested at various drug doses. Initially, various MOLT-3 cell numbers were plated and evaluated 3-7 days later to determine the optimal number of cells to be plated and treatment duration.

Next, the optimal number of cells were plated and treated with various concentrations of AP1 or with various concentrations of rituximab or romidepsin alone. Cells were evaluated for viability by WST-1 assay or MTT assay 3-7 days after beginning treatment with rituximab (**Figures 68B** **and** **69B**) or romidepsin (**Figure 71A** **and** **72B**). AP1 in combination with rituximab showed complementary *in vitro* anticancer activity (**Figure 70**). AP1 in combination with romidepsin showed complementary *in vitro* anticancer activity (**Figure 73**).

### Example 42: Treatment with rituximab and romidepsin with the peptidomimetic macrocycles of the disclosure in the MCF-7 breast cancer cell line.

The combinations of AP1 and commercially available ribociclib **(****Figures 74A** **and** **75A****)** and abemaciclib **(****Figures 76A** **and** **77A****)** were tested at various drug doses. Initially, various MCF-7 cell numbers were plated and evaluated 3-7 days later to determine the optimal number of cells to be plated and treatment duration.

Next, the optimal number of cells were plated and treated with various concentrations of AP1 or with various concentrations of ribociclib or abemaciclib alone. Cells were evaluated for viability by WST-1 assay or MTT assay 72 or 120 hours after beginning treatment with rituximab **(****Figures 74B** **and** **75B****)** or romidepsin **(****Figure 76A** **and** **77B****)**.

### Example 43: The order of addition effects on MCF-7 cell viability using various concentrations of AP 1 in combination with palbociclib using the CyQUANT method.

The anticancer activity of the combination treatment was assessed based on the order of addition of the drugs. MCF-7 cells were sequentially treated by varying concentrations of AP1 and palbociclib for 72 hrs (**Figure 80**). AP1 suppressed MCF-7 cell growth with or without palbociclib (**Figure 81**). Similiarly, palbociclib suppressed MCF-7 cell growth with or without AP1 (**Figure 82**).

### Example 44: Treatment with dexamethasone with the peptidomimetic macrocycles of the disclosure in the MCF-7 breast cancer cell line.

The combination of AP1 and commercially available dexamethasone was tested at various drug doses for 120 hrs. Cells were evaluated for viability by WST-1 assay. AP1 suppressed MCF-7 cell growth with or without dexamethasone (**Figure 83**).

### Example 45: Treatment with zelboraf, tafinlar, and mekinist with the peptidomimetic macrocycles of the disclosure in the A375 melanoma cancer cell line.

The combinations of AP1 and commercially available zelboraf (**Figures 84A** **and** **85A**), tafinlar (**Figures 86A** **and** **87A**), and mekinist (**Figures 88A** **and** **89A**) were tested at various drug doses. Initially, various A375 cell numbers were plated and evaluated 3-7 days later to determine the optimal number of cells to be plated and treatment duration.

Next, the optimal number of cells were plated and treated with various concentrations of AP1 or with various concentrations of zelboraf or tafinlar alone. Cells were evaluated for viability by WST-1 assay or MTT assay 72 hours after beginning treatment with zelboraf (**Figures 84B** **and** **85B**), tafinlar (**Figure 86A** **and** **87B**), or mekinist (**Figures 88B** **and** **89B**).

### Example 46: Combination index plots of fulvestrant, everolimus, palbociclib (WST-1), palbociclib (WST-1), and romidepsin in MCF-7 cells.

The combination index plots suggest additive or better complimentarity for AP1 in MCF-7 cells using fulvestrant (**Figure 90A**), everolimus (**Figure 90B**), palbociclib via WST-1 (**Figure 90C**), palbociclib via CyQUANT (**Figure 90D**), and romidepsin (**Figures 90E**). Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism.

### Example 47: Combination index plots of Ara-C, decitabine, azacitidine, and midostaurin in MV4-11 cells

The combination index plots suggest additive or better complimentarity for AP1 in MV4-1 1 cells using Ara-C (**Figure 91A**), decitabine (**Figure 91B**), azacitidine (**Figure 91C**), and midostaurin (**Figure 91D**). Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism

### Example 48: Combination index plots of vincristine, cyclophosphamide, and rituximab in DOHH-2 cells.

The combination index plots suggest additive or better complimentarity for AP1 in DOHH-2 cells using vincristine (**Figure 92A**), cyclophosphamide (**Figure 92B**), and rituximab (**Figure 92C**). Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism

### Example 49: Combination index plot of romidepsin in MOLT-3 cells.

The combination index plots suggest mostly additive complimentarity for AP1 in MOLT-3 cells using romidepsin (**Figure 93**). Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism

### Example 50: Combination index plots of vincristine, cyclophosphamide, and rituximab in A375 cells.

The combination index plots suggest additive or better complimentarity for AP1 in A375 cells using mekinist (**Figure 94A**), zelboraf (**Figure 94B**), and tafinlar (**Figure 94C**). Combination index (CI) values were calculated using the CompuSyn software. The data were expressed as log(CI). CI values: 0-0.1, very strong synergism; 0.1-0.3, strong synergism; 0.3-0.7, synergism; 0.7-0.85, moderate synergism; 0.85-0.90, slight synergism; 0.90-1.10, nearly additive; 1.10-1.20, slight antagonism; 1.20-1.45, moderate antagonism; 1.45-3.3, antagonism; 3.3-10, strong antagonism; 10, very strong antagonism

### Example 51: Aileron peptide 1 activation of the p5 3-pathway in AML cell lines

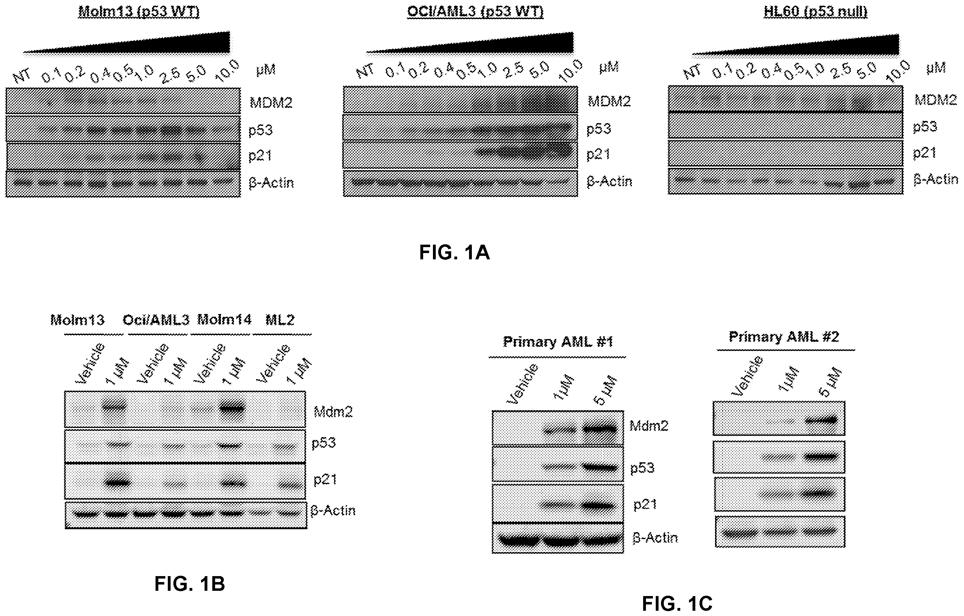

The Molm13 cell line was treated with increasing amounts of Aileron peptide 1 (0.1 µM, 0.2 µM, 0.4 µM, 0.5 µM, 1.0 µM, 2.5 µM, 5.0 µM, or 10.0 µM) (**Figure 1A**). Lysates were subjected to SDS-PAGE and probed by Western blotting with antibodies specific to MDM2, p53, p21, and β-Actin. The results demonstrate that Aileron peptide 1 activates the p53-pathway in the Molm13 cell line.

The OCI/AML3 cell line was treated with increasing amounts of Aileron peptide 1 (0.1 µM, 0.2 µM, 0.4 µM, 0.5 µM, 1.0 µM, 2.5 µM, 5.0 µM, or 10.0 µM) (**Figure 1B**). Lysates were subjected to SDS-PAGE and probed by Western blotting with antibodies specific to MDM2, p53, p21, and β-Actin. The results demonstrate that Aileron peptide 1 activates the p53-pathway in the OCI/AML3 cell line.

The HL60 cell line was treated with increasing amounts of Aileron peptide 1 (0.1 µM, 0.2 µM, 0.4 µM, 0.5 µM, 1.0 µM, 2.5 µM, 5.0 µM, or 10.0 µM) (**Figure 1C**). Lysates were subjected to SDS-PAGE and probed by Western blotting with antibodies specific to MDM2, p53, p21, and β-Actin. The results demonstrate that Aileron peptide 1 does not activate the p53-pathway in the p53 null HL60 cell line.

The Molm 13, OCI/AML3, Molml4 and ML2 cell lines were treated with vehicle or 1.0 µM Aileron peptide (**Figure 1B**). Lysates were subjected to SDS-PAGE and probed by Western blotting with antibodies specific to MDM2, p53, p21, and β-Actin. The results demonstrate that Aileron peptide 1 activates the p53-pathway in these cell lines.

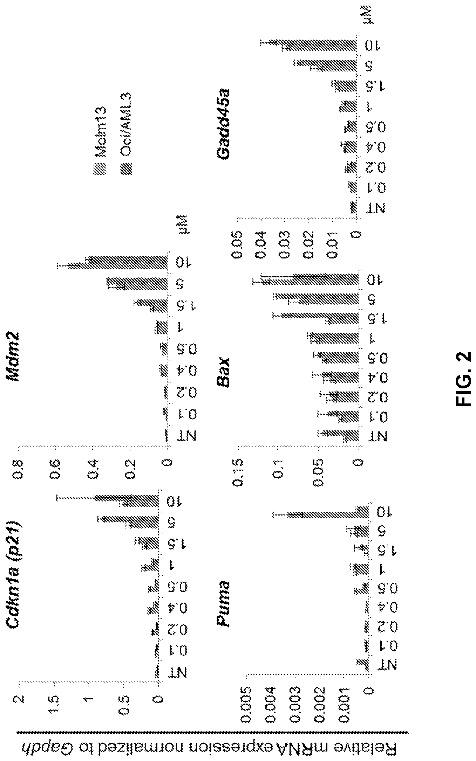

mRNA expression of p21, MDM2, Puma, Bax, and Gadd45a was also determined in Molm13 and Oci/AML3 cells lines folloing treatment with ncreasing amounts of Aileron peptide 1 (0.1 µM, 0.2 µM, 0.4 µM, 0.5 µM, 1.0 µM, 2.5 µM, 5.0 µM, or 10.0 µM) (**Figure 2**). Expression levels were normalized to GAPDH mRNA expression levels.The results demonstrate that Aileron peptide 1 activates the p53-pathway in these cell lines.

### Example 52: Aileron peptide 1 activation of the p53-pathway in primary AML cells

Two primary AML cell lines were treated with with vehicle or 1.0 µM Aileron peptide 1 or 5.0 µM Aileron peptide 1 (**Figure 1C**). Lysates were subjected to SDS-PAGE and probed by Western blotting with antibodies specific to MDM2, p53, p21, and β-Actin. The results demonstrate that Aileron peptide 1 activates the p53-pathway in primary AML cells.

### Example 53: Aileron peptide 1 stabilizes p53 in AML cells

The Molm 13, OCI/AML3, Molm 14, HL60 and ML2 cell lines were treated with vehicle or increasing amounts of Aileron peptide 1 (0.1 µM, 0.2 µM, 0.4 µM, 0.5 µM, 1.0 µM, 2.5 µM, 5.0 µM, or 10.0 µM)

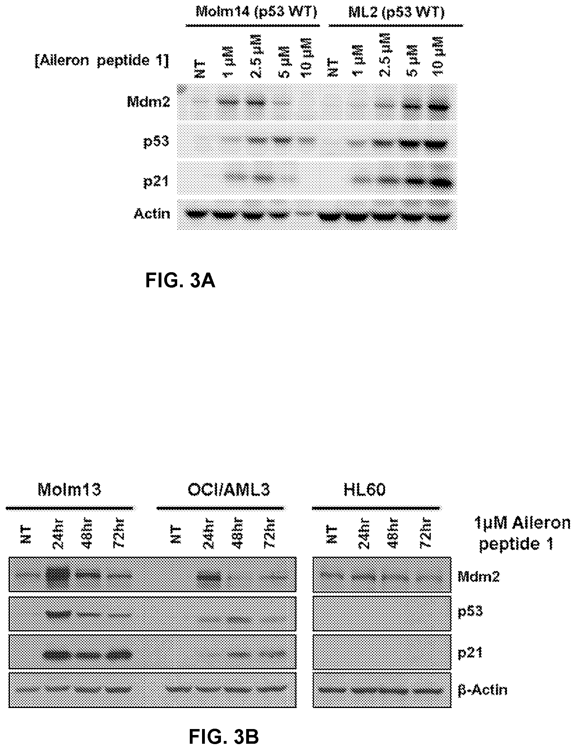

(**Figures 3A and 3B**) for 24 hrs, 48 hrs or 72 hrs. Lysates were subjected to SDS-PAGE and probed by Western blotting with antibodies specific to MDM2, p53, p21, and β-Actin. The results demonstrate that Aileron peptide 1 stabilizes p53 in a time and dose dependant manner the AML p53 wild type cell lines tested.

### Example 54: Immunoprecipitation Assays in AML Cells

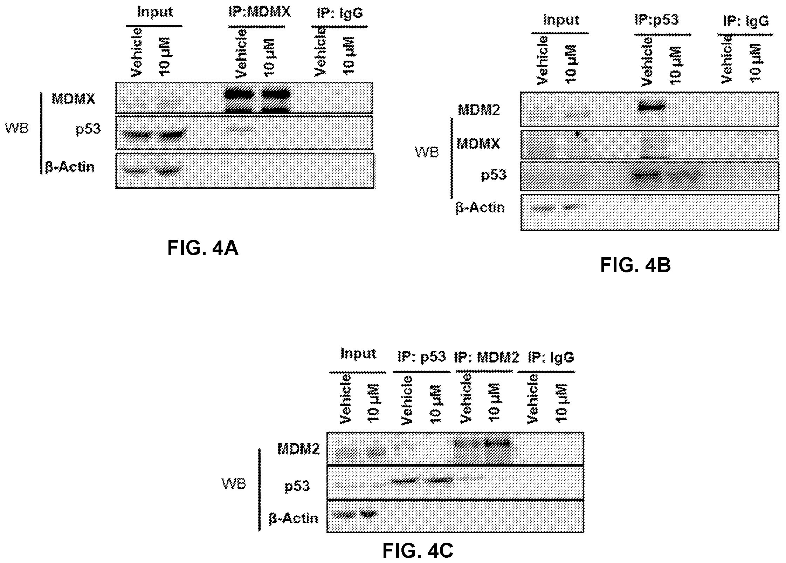

AML p53 wild type cell lines were treated with vehicle or 10.0 µM Aileron peptide **(****Figures 4A, 4B, and 4C**). Lysates were subjected to immunoprecipitation with a MDMX specific antibody (**Figure 4A**), a p53 specific antibody **(****Figure 4B****)**, or a MDM2 specific antibody **(****Figure 4C**). Immunoprecipitates were washed and subjected to SDS-PAGE and probed by Western blotting with antibodies specific to MDM2, MDMX, p53, and/or β-Actin. The results demonstrate that Aileron peptide 1 inhibits the p53-MDMX and the p53-MDM2 interaction.

### Example 55: Cellular Proliferation Assays of AML Cells

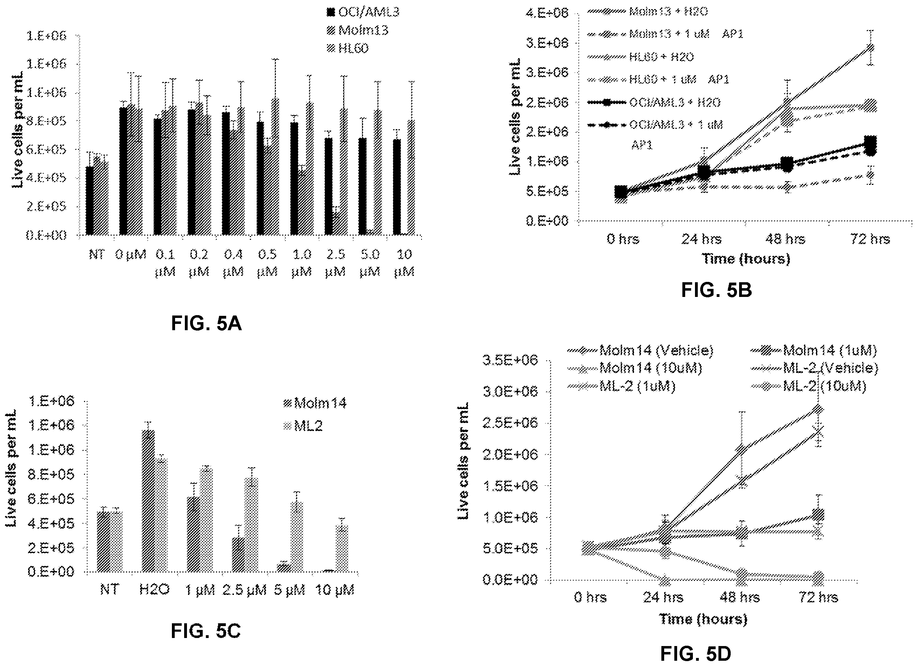

The Molm 13, OCI/AML3, Molm 14, HL60 and ML2 cell lines were plated at a known density (cells/mL) and treated with vehicle or increasing amounts of Aileron peptide 1 (0.1 µM, 0.2 µM, 0.4 µM, 0.5 µM, 1.0 µM, 2.5 µM, 5.0 µM, or 10.0 µM) (**Figures 5A-5D**). Cell proliferation was measured overtime by counting the number of live cells/mL at various time points and plotted. All p53 wild type cell lines treated with Aileron peptide 1 demonstrated reduced levels of cellular proliferation over time compared to vehicle alone.

### Example 56: Clonogenicity Assay on AML Cell Lines

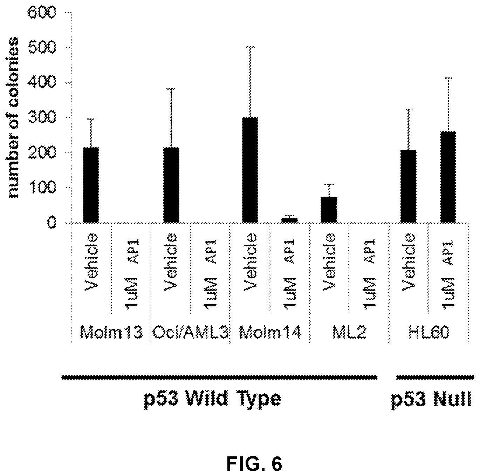

The Molm 13, OCI/AML3, Molm 14, HL60 and ML2 cell lines were treated with vehicle or 1.0 µM Aileron peptide 1. A clonogenicity assay was then performed and the number of clonies was counted **(****Figure 6****)**. The results demonstrated that Aileron peptide 1 treatment in the p53 wild type cell lines tested inhibited their clonogenic capacity.

### Example 57: Cellular Proliferation Assays of AML Cell Lines

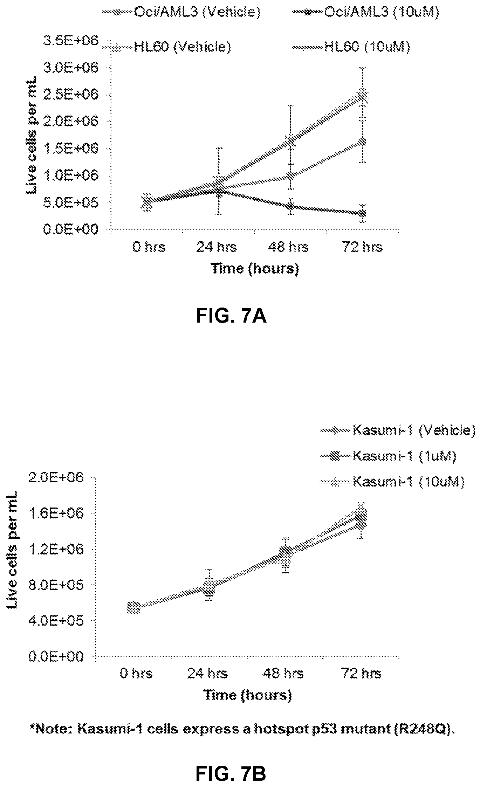

The OCI/AML3, HL60 and Kasumi-1 cell lines were plated at a known density (cells/mL) and treated with vehicle or 10.0 µM Aileron peptide 1 **(****Figures 7A and 7B****)**. Cell proliferation was measured over time by counting the number of live cells/mL at various time points and plotted. The p53 wild type OCI/AML3, but not the p53 null HL60 or the p53R248Q Kasumi-1 cell lines treated with Aileron peptide 1 demonstrated reduced levels of cellular proliferation over time compared to vehicle alone.

### Example 58: Apoptosis Assays of AML Cell Lines

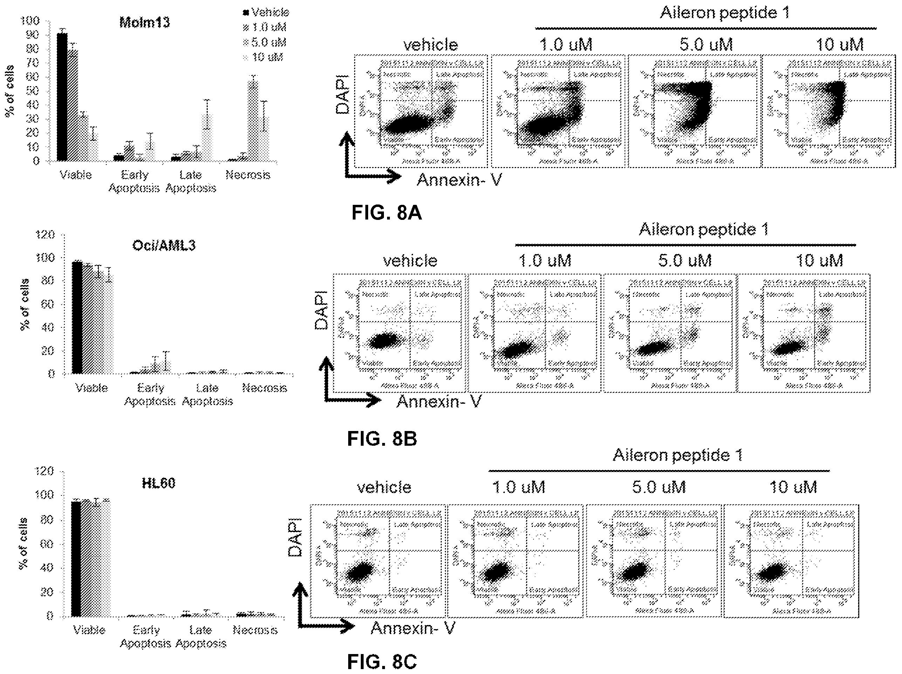

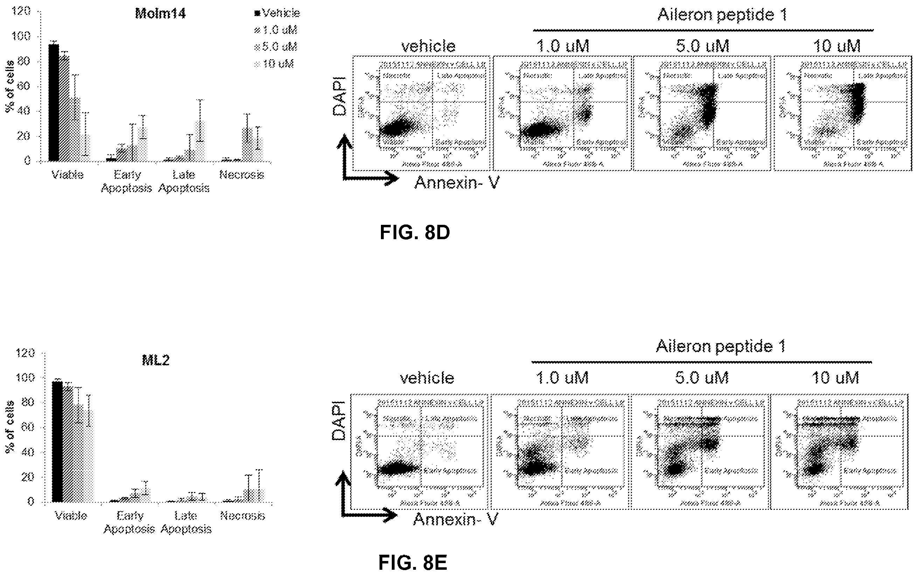

The Molm 13, OCI/AML3, Molm 14, HL60 and ML2 cell lines were treated with vehicle or increasing amounts of Aileron peptide 1 (1.0 µM, 5.0 µM, or 10.0 µM) (**Figures 8A-8E**). Cells were probed with DAPI and a FITC-labeled anti-Annexin-V antibody. FACS analysis was performed to determine the number of viable cells and the number of cells in early apoptosis, late apoptosis and undergoing necrosis and the results were plotted. The results demonstrate that Aileron peptide 1 induces apoptotic cell death in p53 wild type AML cell lines tested.

### Example 59: Cellular Proliferation in AML Cells Treated with Ara-C

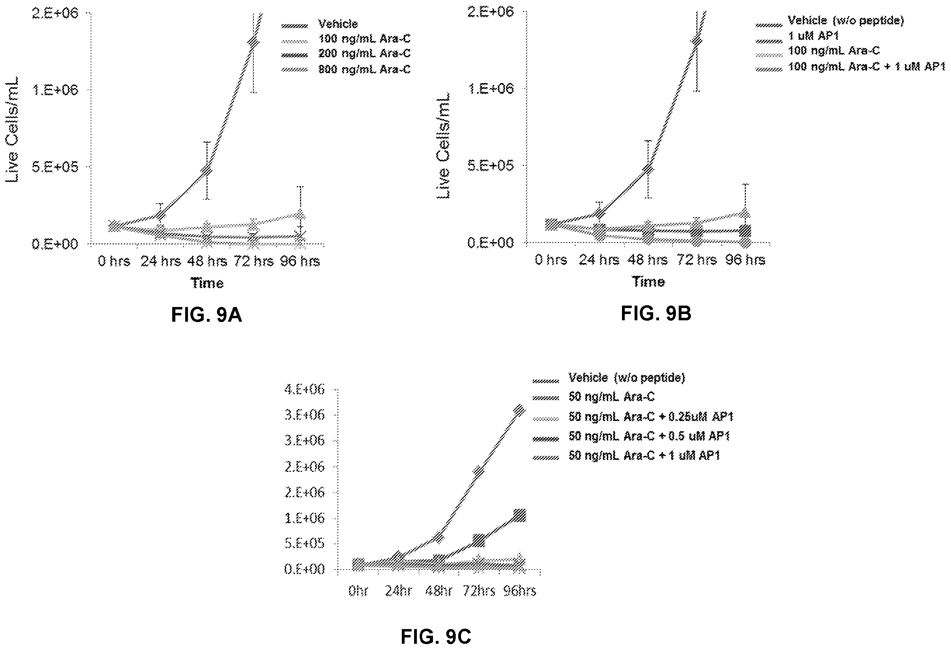

AML cell lines were plated at a known density (cells/mL) and were treated with vehicle or increasing amounts of Ara-C alone (**Figure 9A**); with vehicle, Aileron peptide 1 alone, or with Ara-C and Aileron peptide 1 (**Figure 9B**); or with vehicle, Ara-C alone, or with Ara-C and increasing amounts of Aileron peptide 1. Cell proliferation was measured over time by counting the number of live cells/mL at various time points and plotted. The results demonstrate that cytarabine (Ara-C) treatment inhibits proliferation of AML cell lines and that Ara-C synergizes with AP1 to inhibit proliferation of AML cell lines.

### Example 60: Cellular Proliferation Assays and Clonogenicity Assays of Primary AML Cells

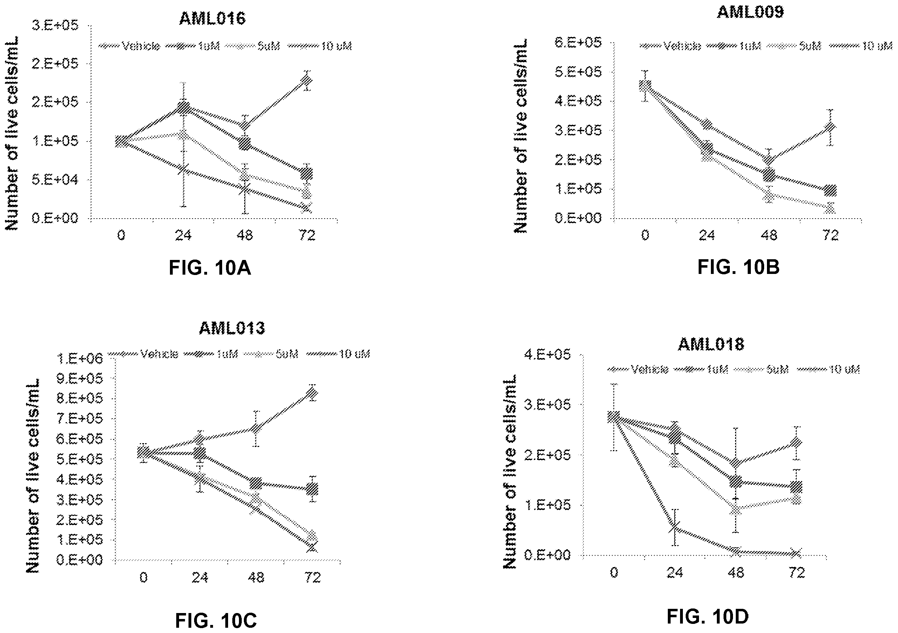

Primary AML cell lines were plated at a known density (cells/mL) and treated with vehicle or or increasing amounts of Aileron peptide 1 (1.0 µM, 5.0 µM, or 10.0 µM) (**Figures 10A-10D**). Cell proliferation was measured over time by counting the number of live cells/mL at various time points and plotted. The primary AML cell lines treated with Aileron peptide 1 demonstrated reduced levels of cellular proliferation over time compared to vehicle alone.

### Example 61: Clonogenicity Assay on Primary AML Cell Lines

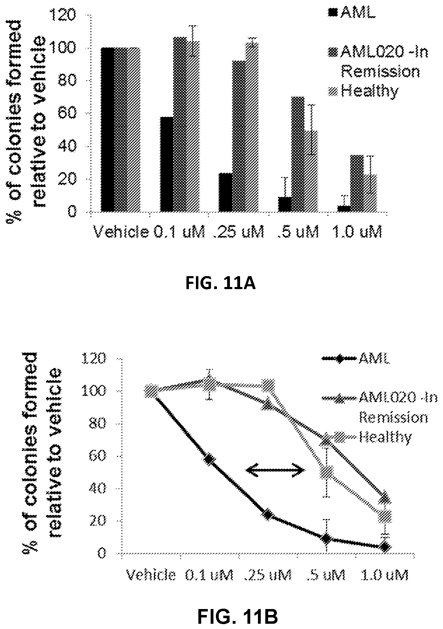

A primary AML cell line and a primary AML cell line from a patient in remission were treated with vehicle or increasing amounts of Aileron peptide 1 (0.1 µM, 0.25 µM, 0.5 µM, or 1.0 µM) **(****Figure 11A and 11B**). A clonogenicity assay was then performed and the number of clonies was counted. The results demonstrated that Aileron peptide 1 treatment in the primary AMl cell line tested inhibited its clonogenic capacity to a higher extent than the primary AML cell line from the patient in remission and cells from a healthy donor.

### Example 62: Apoptosis Assays on Primary AML Cell Lines

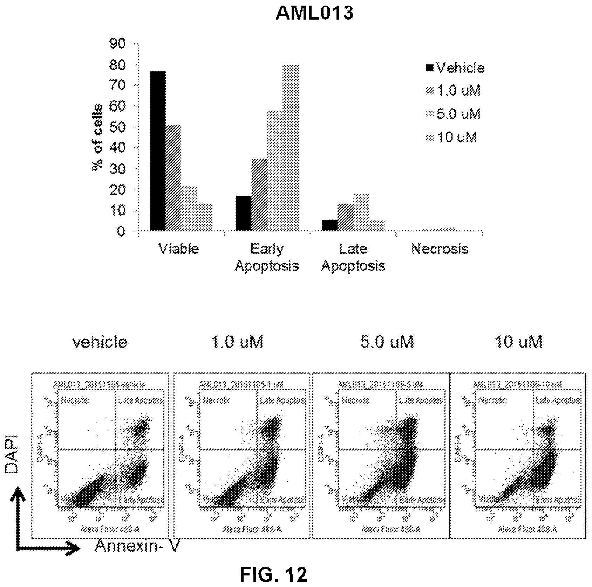

A primary AML cell line was treated with vehicle or increasing amounts of Aileron peptide 1 (1.0 µM, 5.0 µM, or 10.0 µM) (**Figure 12**). Cells were probed with DAPI and a FITC-labeled anti-Annexin-V antibody. FACS analysis was performed to determine the number of viable cells and the number of cells in early apoptosis, late apoptosis and undergoing necrosis and the results were plotted. The results demonstrate that Aileron peptide 1 induces apoptotic cell death in primary AML cells.

## Claims

1. A peptidomimetic macrocycle or a pharmaceutically acceptable salt thereof and at least one additional pharmaceutically active agent for use in the treatment of cancer in a subject in need thereof, wherein the peptidomimetic macrocycle or the pharmaceutically acceptable salt thereof antagonizes the activity of p53 and/or MDM2 and/or MDMX , and wherein the peptidomimetic macrocycle comprises an amino acid sequence which is at least 60% identical to an amino acid sequence in any of Table 1, Table 1a, Table 1b, and Table 1c, and wherein the peptidomimetic macrocycle has the formula: 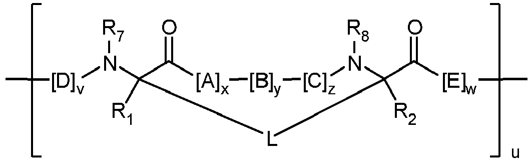 wherein:
each A, C, D, and E is independently an amino acid;
each B is independently an amino acid, 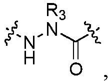 [-NH-L₃-CO-], [-NH-L₃-SO₂-], or [-NH-L₃-];
each R₁ and R₂ is independently hydrogen, alkyl, alkenyl, alkynyl, arylalkyl, cycloalkyl, cycloalkylalkyl, heteroalkyl, or heterocycloalkyl, unsubstituted or substituted with halo-; or forms a macrocycle-forming linker L' connected to the alpha position of one of said D or E amino acids;
each R₃ is independently hydrogen, alkyl, alkenyl, alkynyl, arylalkyl, heteroalkyl, cycloalkyl, heterocycloalkyl, cycloalkylalkyl, aryl, or heteroaryl, optionally substituted with R₅;
each L and L' is independently a macrocycle-forming linker of the formula -L₁-L₂-;
each L₁, L₂, and L₃ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
each R₄ is independently alkylene, alkenylene, alkynylene, heteroalkylene, cycloalkylene, heterocycloalkylene, arylene, or heteroarylene;
each K is independently O, S, SO, SO₂, CO, CO₂, or CONR₃;
each R₅ is independently halogen, alkyl, -OR₆, -N(R₆)₂, -SR₆, -SOR₆, -SO₂R₆, - CO₂R₆, a fluorescent moiety, a radioisotope or a therapeutic agent;
each R₆ is independently hydrogen, alkyl, alkenyl, alkynyl, arylalkyl, cycloalkylalkyl, heterocycloalkyl, a fluorescent moiety, a radioisotope or a therapeutic agent;
each R₇ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
each R₈ is independently hydrogen, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
each v is independently an integer from 1-1000;
each w is independently an integer from 3-1000;
u is an integer from 1-10;
each x, y and z is independently an integer from 0-10; and
each n is independently an integer from 1-5;
wherein the at least one additional pharmaceutically active agent is fulvestrant, everolimus, romidepsin, palbociclib, trametinib, rituximab, dabrafenib, vemurafenib, azacitidine, decitabine, midostaurin, vincristine, cyclophosphamide or cytarabine.

2. The compounds for use of claim 1, wherein the peptidomimetic macrocycle or the pharmaceutically acceptable salt thereof has a Formula: 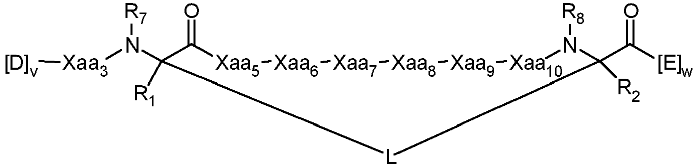 wherein:
each of Xaa₃, Xaa₅, Xaa₆, Xaa₇, Xaa₈, Xaa₉, and Xaa₁₀ is individually an amino acid, wherein at least three of Xaa₃, Xaa₅, Xaa₆, Xaa₇, Xaa₈, Xaa₉, and Xaa₁₀ are the same amino acid as the amino acid at the corresponding position of the sequence Phe₃-X₄-His₅-Tyr₆-Trp₇-Ala₈-Gln₉-Leu₁₀-X₁₁-Ser₁₂ or Phe₃-X₄-Glu₅-Tyr₆-Trp₇-Ala₈-Gln₉-Leu₁₀/Cba₁₀-X₁₁-Ala₁₂ wherein each X₄ and X₁₁ is independently an amino acid;
each D and E is independently an amino acid;
R₁ and R₂ are independently -H, alkyl, alkenyl, alkynyl, arylalkyl, cycloalkyl, cycloalkylalkyl, heteroalkyl, or heterocycloalkyl, unsubstituted or substituted with halo-; or at least one of R₁ and R₂ forms a macrocycle-forming linker L' connected to the alpha position of one of said D or E amino acids;
each L and L' is independently a macrocycle-forming linker of the formula -L₁-L₂-;
each L₁ and L₂ is independently alkylene, alkenylene, alkynylene, heteroalkylene, cycloalkylene, heterocycloalkylene, arylene, heteroarylene, or [-R₄-K-R₄-]ₙ, each being optionally substituted with R₅;
each R₄ is independently alkylene, alkenylene, alkynylene, heteroalkylene, cycloalkylene, heterocycloalkylene, arylene, or heteroarylene;
each K is independently O, S, SO, SO₂, CO, CO₂, or CONR₃;
each R₅ is independently halogen, alkyl, -OR₆, -N(R₆)₂, -SR₆, -SOR₆, -SO₂R₆, -CO₂R₆, a fluorescent moiety, a radioisotope or a therapeutic agent;
each R₆ is independently -H, alkyl, alkenyl, alkynyl, arylalkyl, cycloalkylalkyl, heterocycloalkyl, a fluorescent moiety, a radioisotope or a therapeutic agent;
R₇ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with a D residue;
R₈ is -H, alkyl, alkenyl, alkynyl, arylalkyl, cycloalkyl, heteroalkyl, cycloalkylalkyl, heterocycloalkyl, aryl, or heteroaryl, optionally substituted with R₅, or part of a cyclic structure with an E residue;
v is an integer from 1-1000;
w is an integer from 3-1000; and
n is an integer from 1-5.

3. The compounds for use of claim 1 or 2, wherein each E is independently an amino acid selected from Ala (alanine), D-Ala (D-alanine), Aib (α-aminoisobutyric acid), Sar (N-methyl glycine), and Ser (serine).

4. The compounds for use of claim 1 or 2, wherein:
v is an integer from 1-10;
w is an integer from 3-10;
each of the first two amino acids represented by E comprises an uncharged side chain or a negatively charged side chain;
L₁ and L₂ are independently alkylene or alkenylene; and
R₁ and R₂ are independently H or alkyl.

5. The compounds for use of any one of claims 1-4, wherein the at least one additional pharmaceutically active agent is palbociclib.

6. The compounds for use of any one of claims 1-5, wherein the cancer is selected from the group consisting of head and neck cancer, melanoma, lung cancer, breast cancer, colon cancer, ovarian cancer, NSCLC, stomach cancer, prostate cancer, leukemia, lymphoma, mesothelioma, renal cancer, non-Hodgkin lymphoma (NHL), and glioma.

7. The compounds for use of any one of claims 1-5, wherein the cancer comprises a cell that overexpresses PD-L1, PD-1, miR-34, or any combination thereof.

8. The compounds for use of any one of claims 1-2 and 5-7, wherein the peptidomimetic macrocycle is selected from the group consisting of: 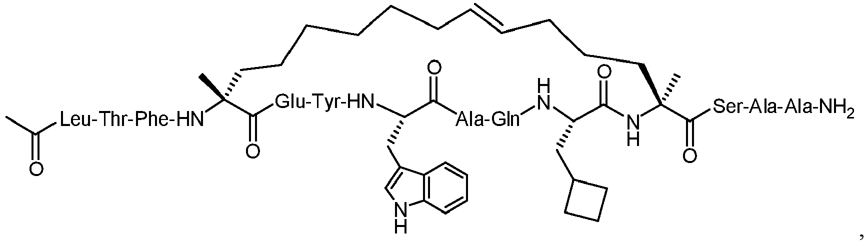 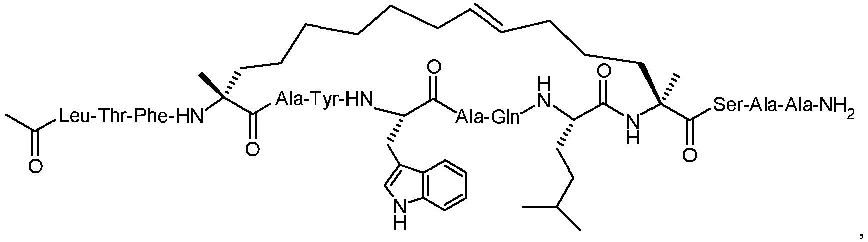 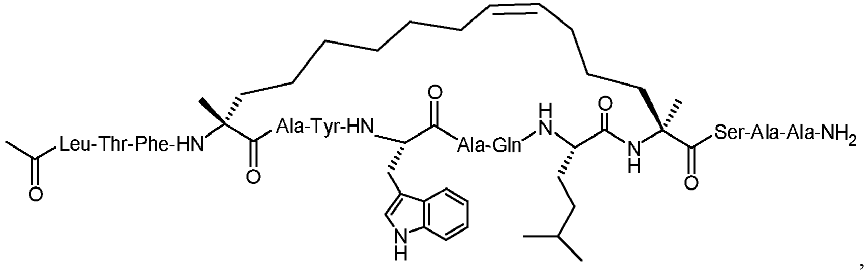 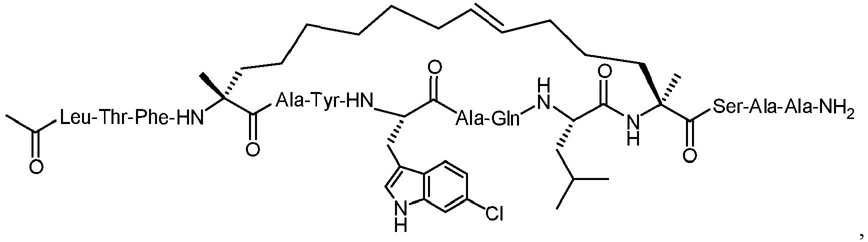 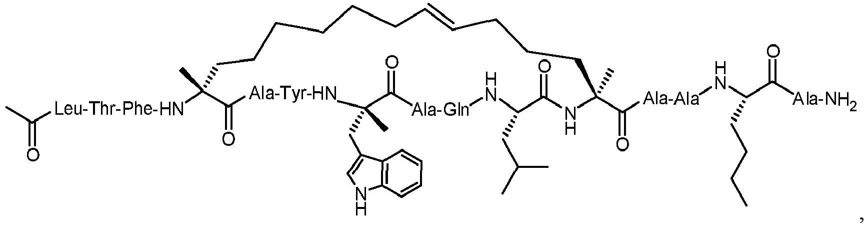 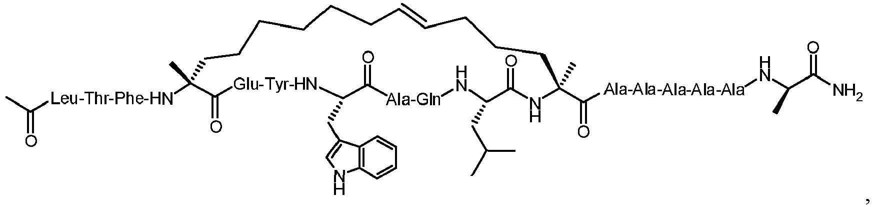 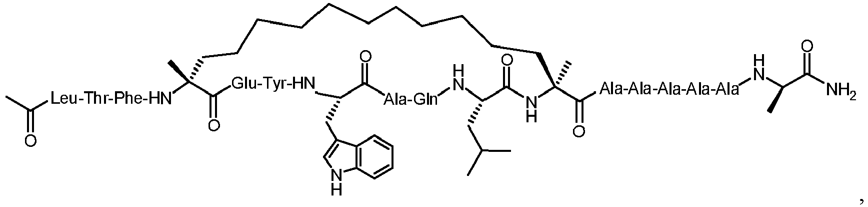 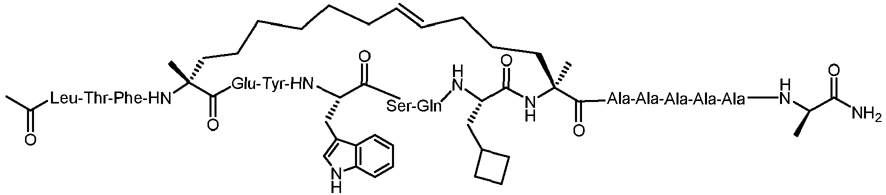 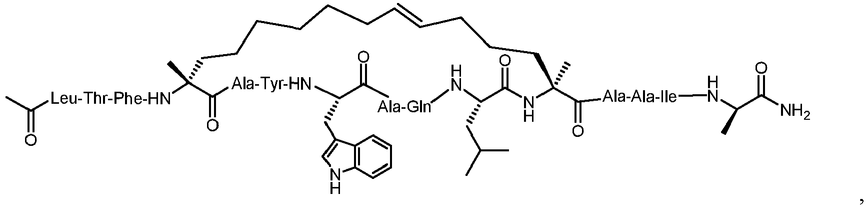 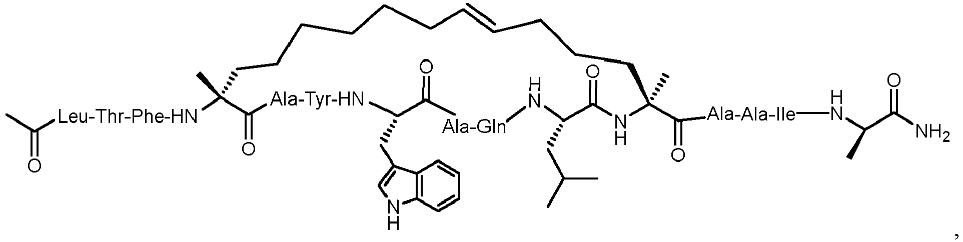 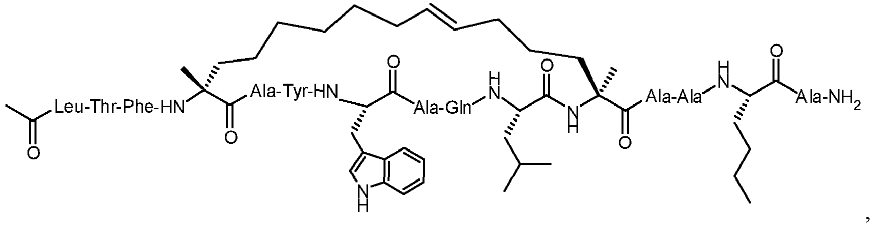 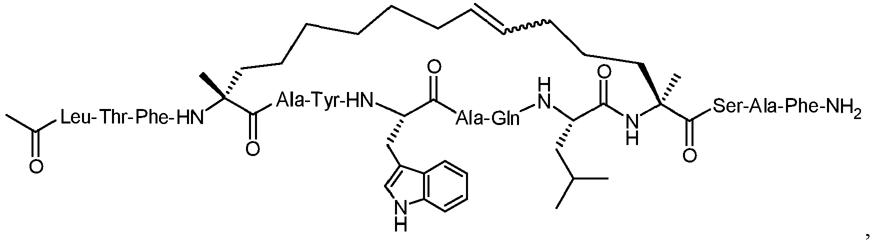 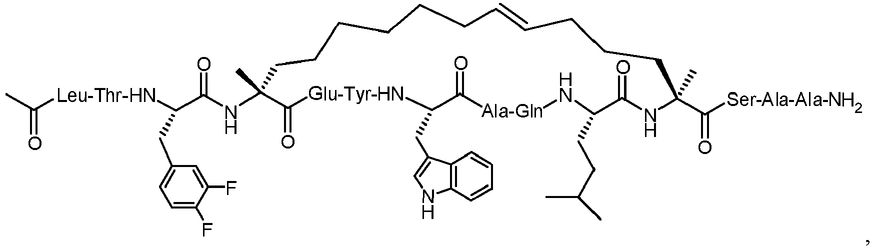 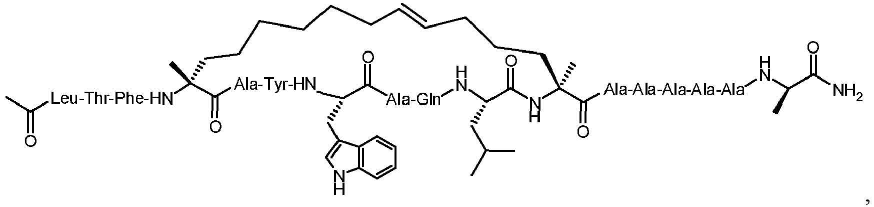 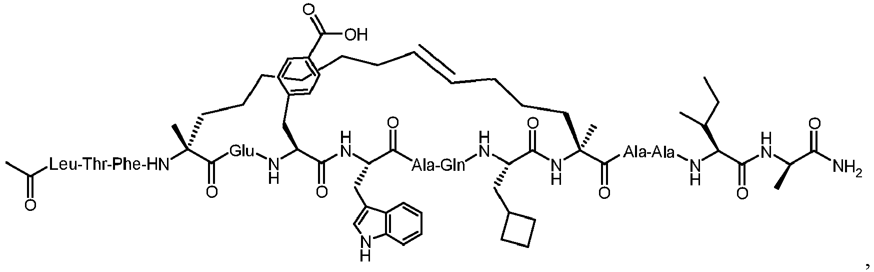 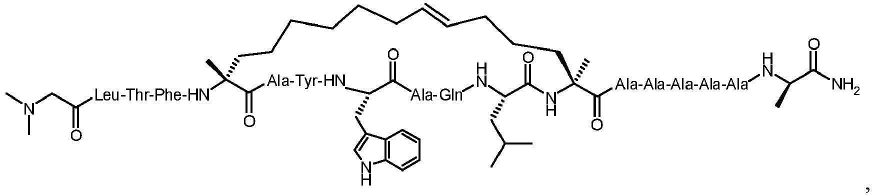 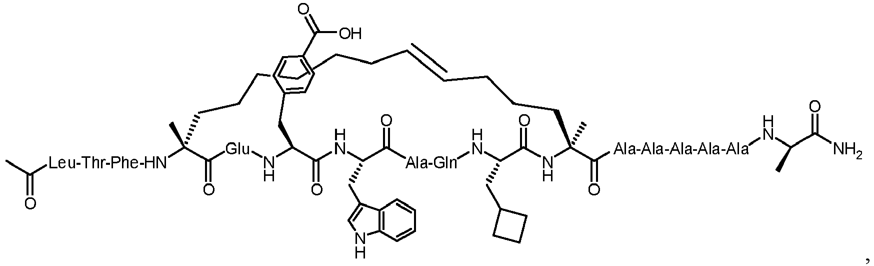 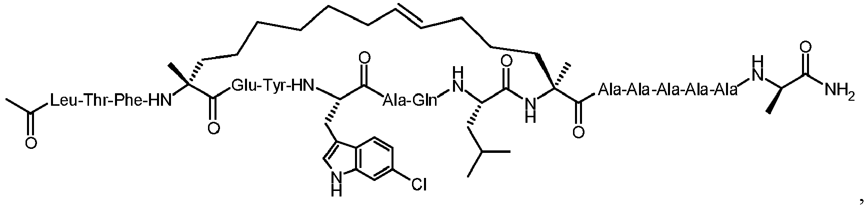 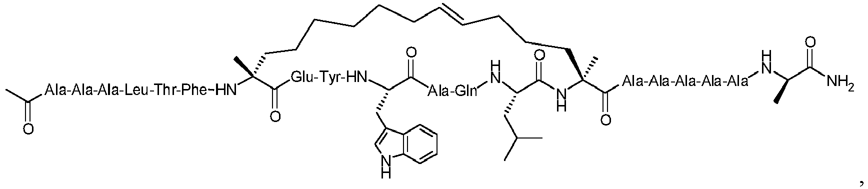 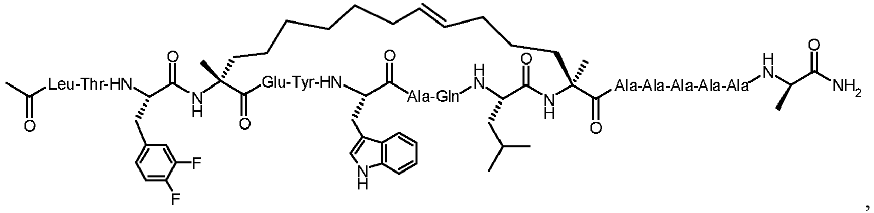 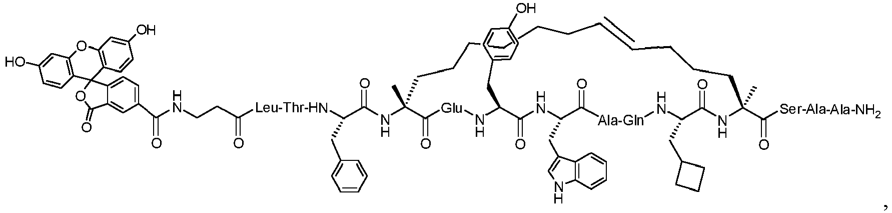 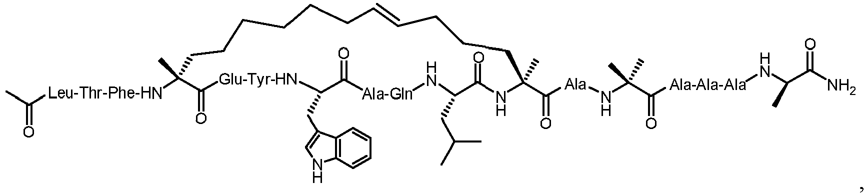 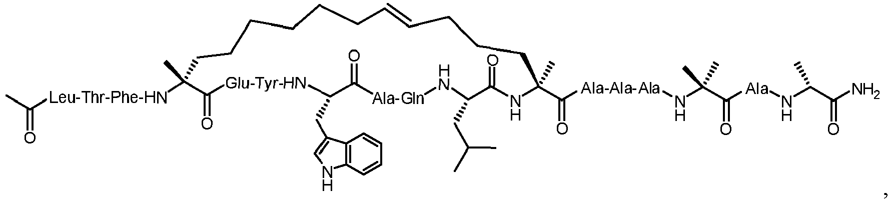 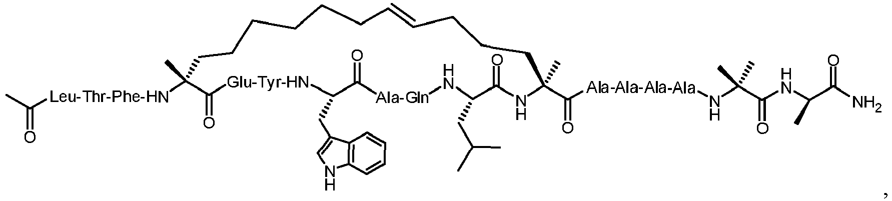 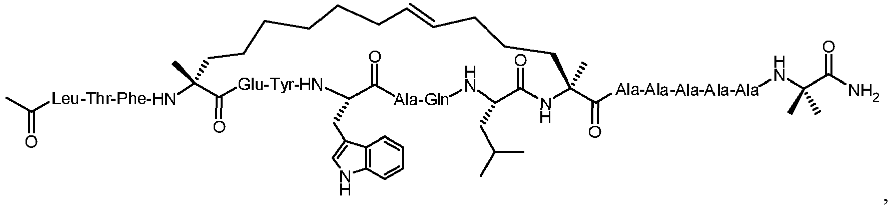 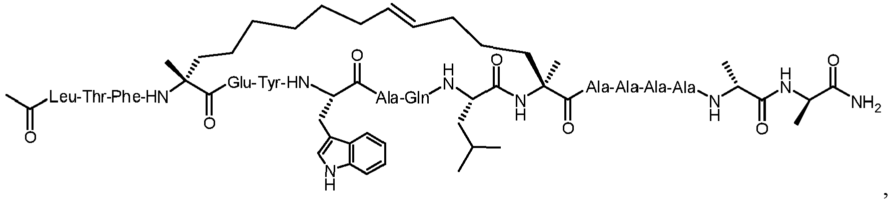 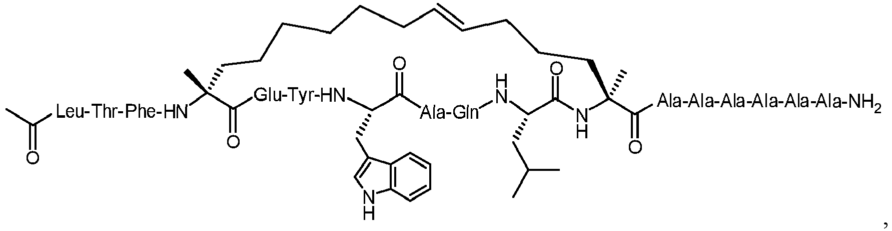 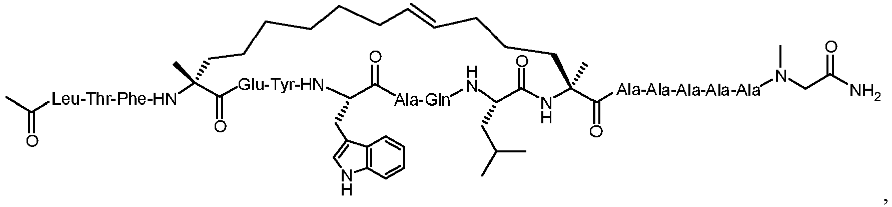 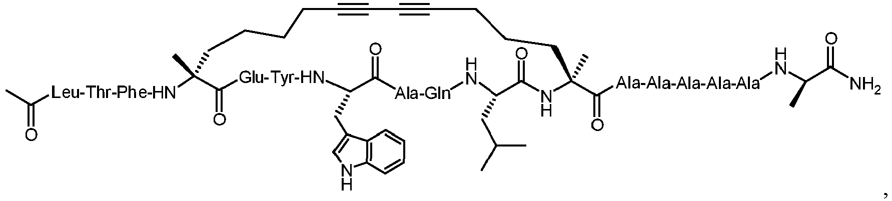 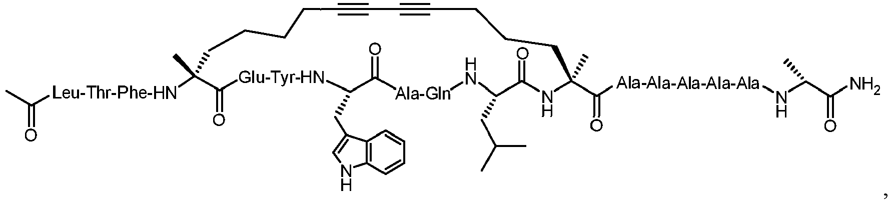 and

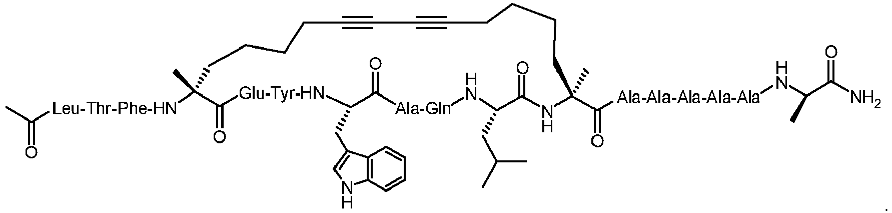

9. The compounds for use of any one of claims 1-8, wherein the cancer is AML.

10. The compounds for use of any one of claims 1-8, wherein the cancer is:
(i) myelodysplastic syndrome; or
(ii) peripheral T cell lymphoma; or
(iii) breast cancer.

11. The compounds for use of any one of claims 1-4, and 6-11, wherein the at least one additional pharmaceutically active agent is cytarabine.

12. The compounds for use of any one of claims 1-11, wherein combination of the peptidomimetic macrocycle or the pharmaceutically acceptable salt thereof and the at least one additional pharmaceutically active agent is synergistic.

## Patentansprüche

1. Peptidomimetischer Makrozyklus oder ein pharmazeutisch annehmbares Salz davon und mindestens ein zusätzlicher pharmazeutischer Wirkstoff zur Verwendung bei der Behandlung von Krebs in einem behandlungsbedürftigen Subjekt, wobei der peptidomimetische Makrozyklus oder das pharmazeutisch annehmbare Salz davon die Aktivität von p53 und/oder MDM2 und/oder MDMX antagonisiert, und wobei der peptidomimetische Makrozyklus eine Aminosäuresequenz umfasst, die mindestens 60 % identisch zu einer Aminosäuresequenz in einem aus Tabelle 1, Tabelle 1a, Tabelle 1b und Tabelle 1c ist, und wobei der peptidomimetische Makrozyklus die folgende Formel aufweist: 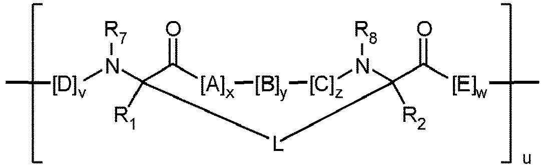 wobei:
jedes aus A, C, D und E unabhängig eine Aminosäure ist;
jedes B unabhängig eine Aminosäure, 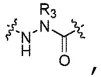 [-NH-L₃-CO-], [-NH-L₃-SO₂-] oder [-NH-L₃-] ist;
jedes R₁ und R₂ unabhängig Wasserstoff, Alkyl, Alkenyl, Alkynyl, Arylalkyl, Cycloalkyl, Cycloalkylalkyl, Heteroalkyl oder Heterocycloalkyl, unsubstituiert oder substituiert mit Halo- ist; oder einen Makrozyklus-bildenden Linker L' bildet, verbunden mit der Alpha-Position von einem der D- oder E-Aminosäuren;
jedes R₃ unabhängig Wasserstoff, Alkyl, Alkenyl, Alkynyl, Arylalkyl, Heteroalkyl, Cycloalkyl, Heterocycloalkyl, Cycloalkylalkyl, Aryl oder Heteroaryl ist, optional substituiert mit R₅;
jedes L und L' unabhängig ein Makrozyklus-bildender Linker der Formel -L₁-L₂- ist;
jedes L₁, L₂ und L₃ unabhängig Alkylen, Alkenylen, Alkynylen, Heteroalkylen, Cycloalkylen, Heterocycloalkylen, Arylen, Heteroarylen oder [-R₄-K-R₄-]ₙ ist, die jeweils optional mit R₅ substituiert sind;
jedes R₄ unabhängig Alkylen, Alkenylen, Alkynylen, Heteroalkylen, Cycloalkylen, Heterocycloalkylen, Arylen oder Heteroarylen ist;
jedes K unabhängig O, S, SO, SO₂, CO, CO₂ oder CONR₃ ist;
jedes R₅ unabhängig Halogen, Alkyl, -OR₆, -N(R₆)₂, -SR₆, - SOR₆, -SO₂R₆, -CO₂R₆, ein fluoreszierender Teil, ein Radioisotop oder ein therapeutisches Mittel ist;
jedes R₆ unabhängig Wasserstoff, Alkyl, Alkenyl, Alkynyl, Arylalkyl, Cycloalkylalkyl, Heterocycloalkyl, ein fluoreszierender Teil, ein Radioisotop oder ein therapeutisches Mittel ist;
jedes R₇ unabhängig Wasserstoff, Alkyl, Alkenyl, Alkynyl, Arylalkyl, Cycloalkyl, Heteroalkyl, Cycloalkylalkyl, Heterocycloalkyl, Aryl oder Heteroaryl ist, optional substituiert mit R₅ oder Teil einer cyclischen Struktur mit einem D-Rest;
jedes R₈ unabhängig Wasserstoff, Alkyl, Alkenyl, Alkynyl, Arylalkyl, Cycloalkyl, Heteroalkyl, Cycloalkylalkyl, Heterocycloalkyl, Aryl oder Heteroaryl ist, optional substituiert mit R5 oder Teil einer cyclischen Struktur mit einem E-Rest;
jedes v unabhängig eine Ganzzahl von 1-1000 ist;
jedes w unabhängig eine Ganzzahl von 3-1000 ist;
u eine Ganzzahl von 1-10 ist;
jedes x, y und z unabhängig eine Ganzzahl von 0-10 ist; und
jedes n unabhängig eine Ganzzahl von 1-5 ist;
wobei der mindestens eine zusätzliche pharmazeutische Wirkstoff Fulvestrant, Everolimus, Romidepsin, Palbociclib, Trametinib, Rituximab, Dabrafenib, Vemurafenib, Azacitidin, Decitabin, Midostaurin, Vincristin, Cyclophosphamid oder Cytarabin ist.

2. Verbindungen zur Verwendung nach Anspruch 1, wobei der peptidomimetische Makrozyklus oder das pharmazeutisch annehmbare Salz davon eine Formel aufweist: 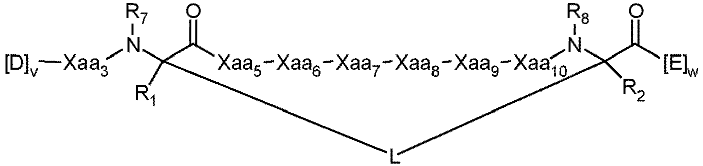 wobei:
jedes aus Xaa₃, Xaa₅, Xaa₆, Xaa₇, Xaa₈, Xaa₉ und Xaa₁₀ individuell eine Aminosäure ist,
wobei mindestens drei aus Xaa₃, Xaa₅, Xaa₆, Xaa₇, Xaa₈, Xaa₉ und Xaa₁₀ die gleiche Aminosäure wie die Aminosäure an der entsprechenden Position der Sequenz Phe₃-X₄-His₅-Tyr₆-Trp₇-Ala₈-Gln₉-Leu₁₀-X₁₁-Ser₁₂ oder Phe₃-X₄-Glu₅-Tyr₆-Trp₇-Ala₈-Gln₉-Leu₁₀/Cba₁₀-X₁₁-Ala₁₂ sind, wobei jedes X₄ und X₁₁ unabhängig eine Aminosäure ist;
jedes D und E unabhängig eine Aminosäure ist;
R₁ und R₂ unabhängig -H, Alkyl, Alkenyl, Alkynyl, Arylalkyl, Cycloalkyl, Cycloalkylalkyl, Heteroalkyl oder Heterocycloalkyl sind, unsubstituiert oder substituiert mit Halo-; oder mindestens eines aus R₁ und R₂ einen Makrozyklus-bildenden Linker L' bildet, verbunden mit der Alpha-Position von einer der D- oder E-Aminosäuren;
jedes L und L' unabhängig ein Makrozyklus-bildender Linker der Formel -L₁-L₂- ist;
jedes L₁ und L₂ unabhängig Alkylen, Alkenylen, Alkynylen, Heteroalkylen, Cycloalkylen, Heterocycloalkylen, Arylen, Heteroarylen oder [-R₄-K-R₄-]ₙ ist, die jeweils optional mit R₅ substituiert sind;
jedes R₄ unabhängig Alkylen, Alkenylen, Alkynylen, Heteroalkylen, Cycloalkylen, Heterocycloalkylen, Arylen oder Heteroarylen ist;
jedes K unabhängig O, S, SO, SO₂, CO, CO₂ oder CONR₃ ist;
jedes R₅ unabhängig Halogen, Alkyl, -OR₆, -N(R₆)₂, -SR₆, - SOR₆, -SO₂R₆, -CO₂R₆, ein fluoreszierender Teil, ein Radioisotop oder ein therapeutisches Mittel ist;
jedes R₆ unabhängig -H, Alkyl, Alkenyl, Alkynyl, Arylalkyl, Cycloalkylalkyl, Heterocycloalkyl, ein fluoreszierender Teil, ein Radioisotop oder ein therapeutisches Mittel ist;
R₇ -H, Alkyl, Alkenyl, Alkynyl, Arylalkyl, Cycloalkyl, Heteroalkyl, Cycloalkylalkyl, Heterocycloalkyl, Aryl oder Heteroaryl ist, optional substituiert mit R₅ oder Teil einer cyclischen Struktur mit einem D-Rest;
R₈ -H, Alkyl, Alkenyl, Alkynyl, Arylalkyl, Cycloalkyl, Heteroalkyl, Cycloalkylalkyl, Heterocycloalkyl, Aryl oder Heteroaryl ist, optional substituiert mit R₅ oder Teil einer cyclischen Struktur mit einem E-Rest;
v eine Ganzzahl von 1-1000 ist;
w eine Ganzzahl von 3-1000 ist; und
n eine Ganzzahl von 1-5 ist.

3. Verbindungen zur Verwendung nach Anspruch 1 oder 2, wobei jedes E unabhängig eine Aminosäure ist, ausgewählt aus Ala (Alanin), D-Ala (D-Alanin), Aib (α-Aminoisobuttersäure), Sar (N-Methylglycin) und Ser (Serin).

4. Verbindungen zur Verwendung nach Anspruch 1 oder 2, wobei:
v eine Ganzzahl von 1-10 ist;
w eine Ganzzahl von 3-10 ist;
jede der ersten zwei Aminosäuren, dargestellt durch E, eine ungeladene Seitenkette oder eine negativ geladene Seitenkette umfasst;
L₁ und L₂ unabhängig Alkylen oder Alkenylen sind; und
R₁ und R₂ unabhängig H oder Alkyl sind.

5. Verbindungen zur Verwendung nach einem der Ansprüche 1-4, wobei der mindestens eine zusätzliche pharmazeutische Wirkstoff Palbociclib ist.

6. Verbindungen zur Verwendung nach einem der Ansprüche 1-5, wobei der Krebs ausgewählt ist aus der Gruppe bestehend aus Kopf-Hals-Karzinom, Melanom, Lungenkrebs, Brustkrebs, Darmkrebs, Eierstockkrebs, NSCLC, Magenkrebs, Prostatakrebs, Leukämie, Lymphom, Mesotheliom, Nierenkrebs, Non-Hodgkin-Lymphom (NHL) und Gliom.

7. Verbindungen zur Verwendung nach einem der Ansprüche 1-5, wobei der Krebs eine Zelle umfasst, die PD-L1, PD-1, miR-34 oder eine beliebige Kombination davon überexprimiert.

8. Verbindungen zur Verwendung nach einem der Ansprüche 1-2 und 5-7, wobei der peptidomimetische Makrozyklus ausgewählt ist aus der Gruppe bestehend aus: 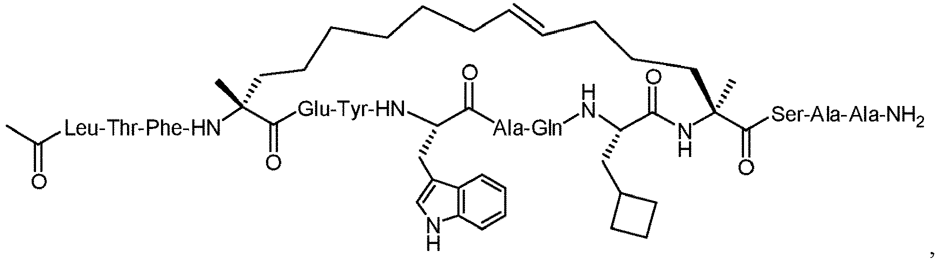 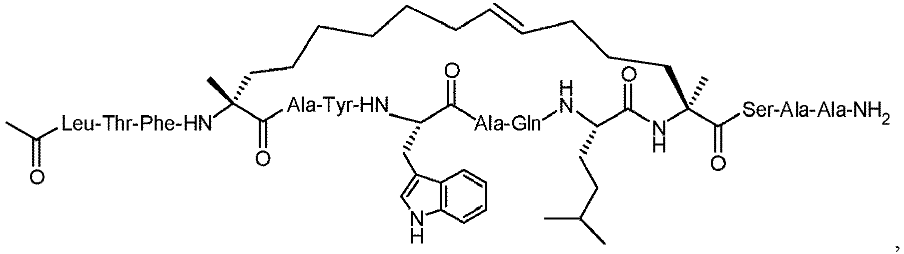 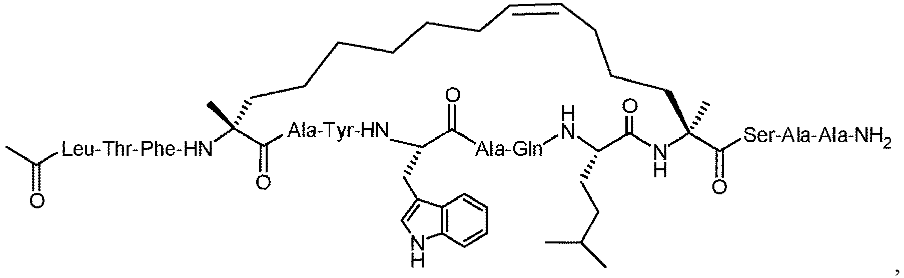 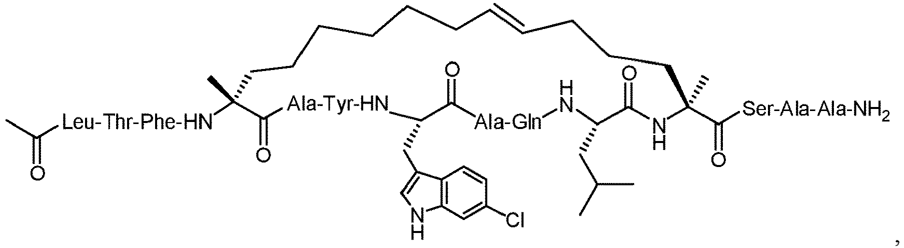 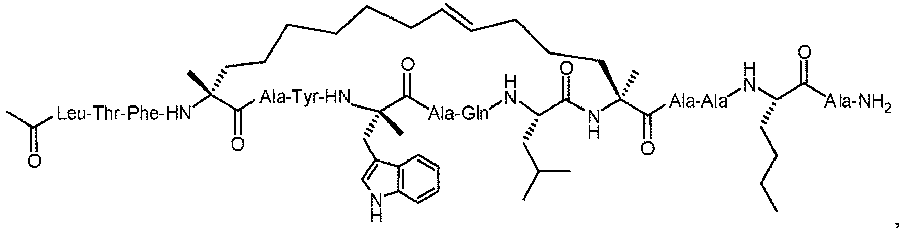 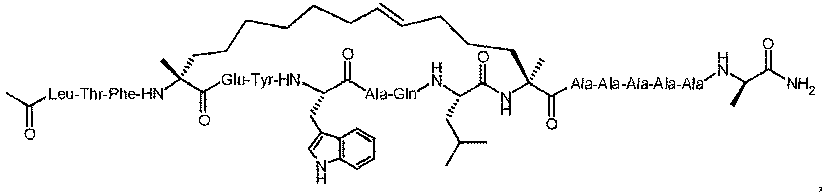 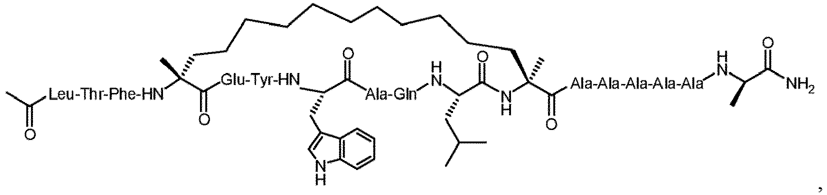 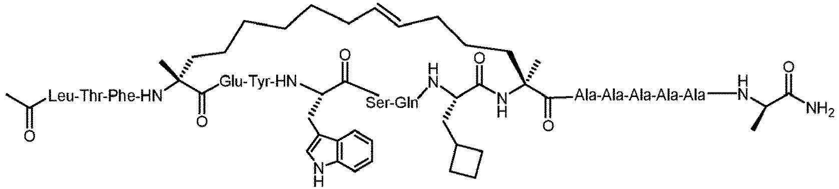 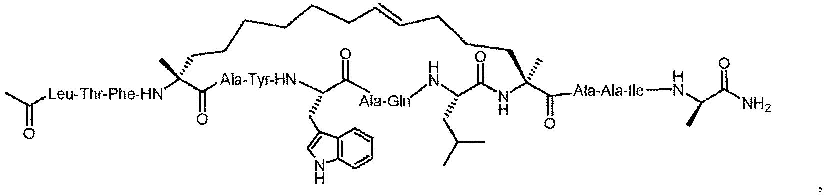 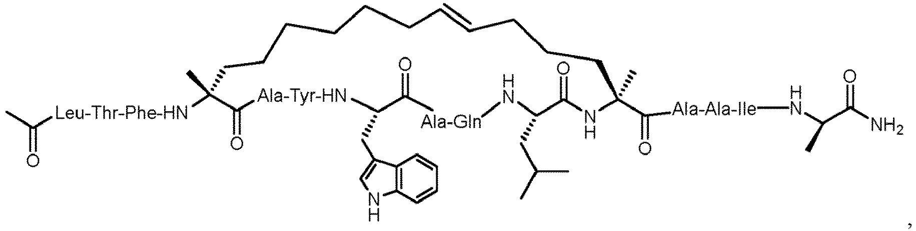 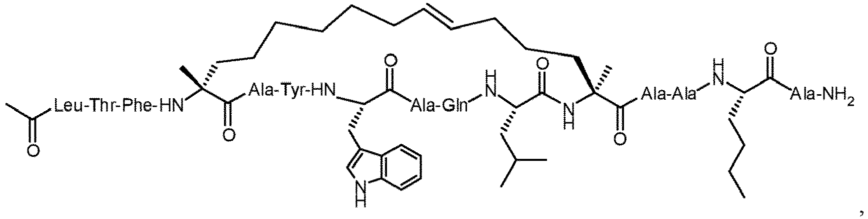 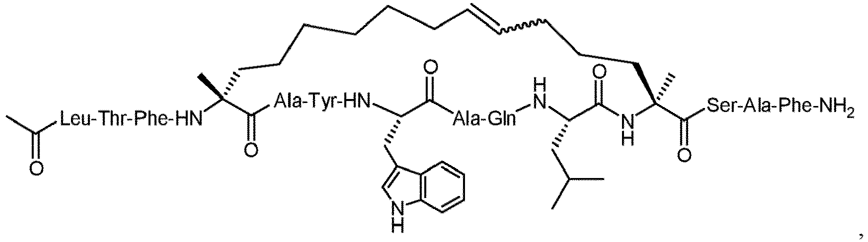 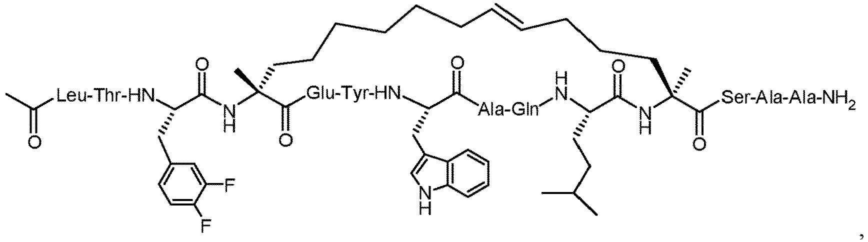 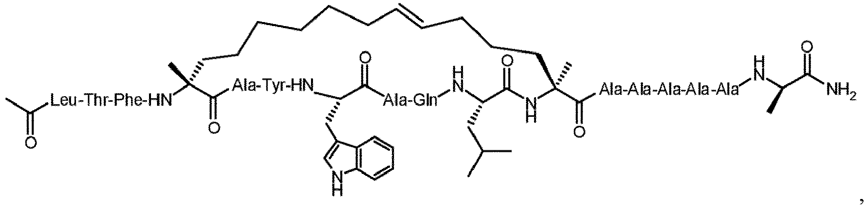 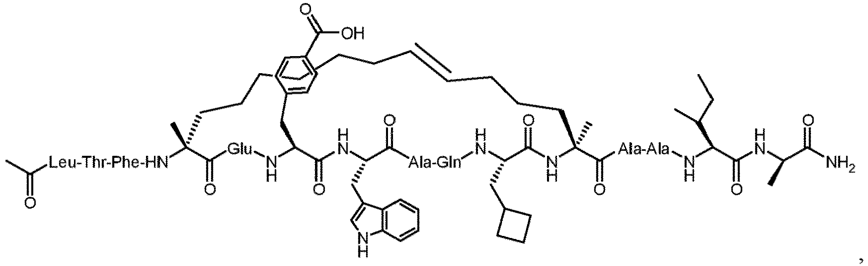 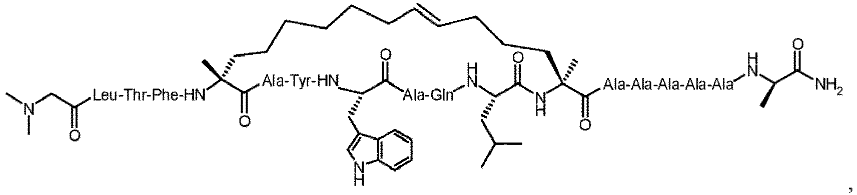 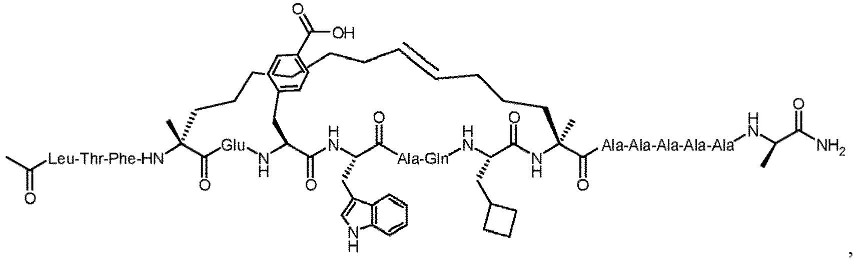 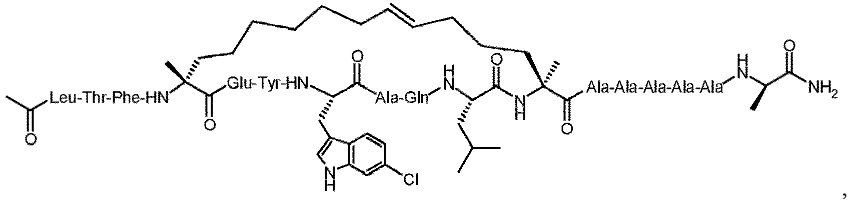 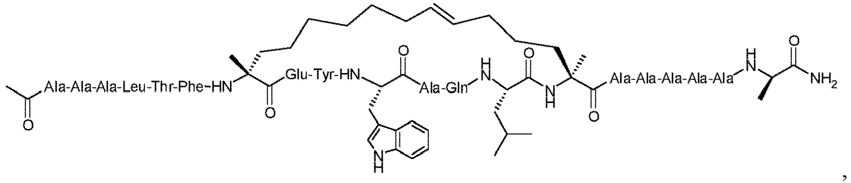 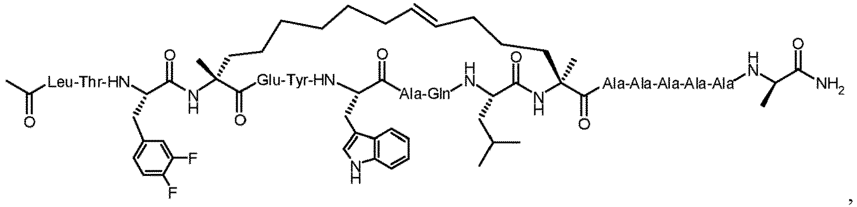 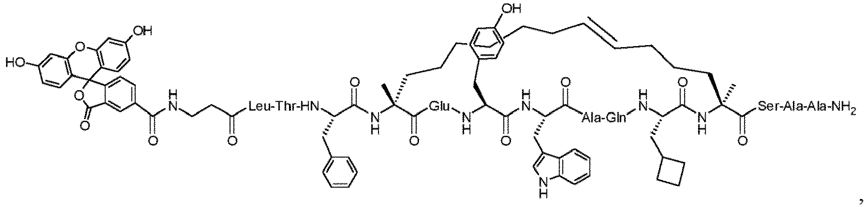 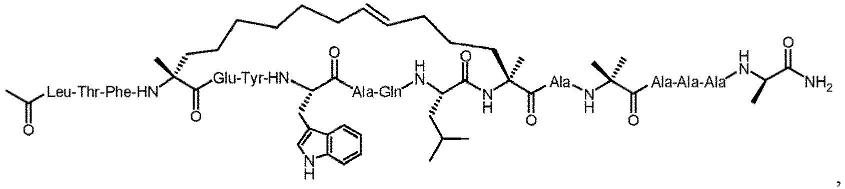 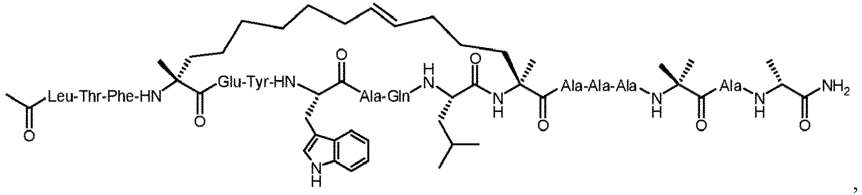 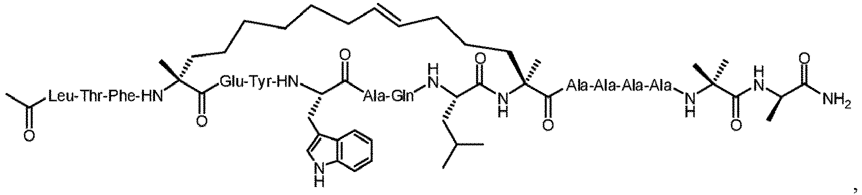 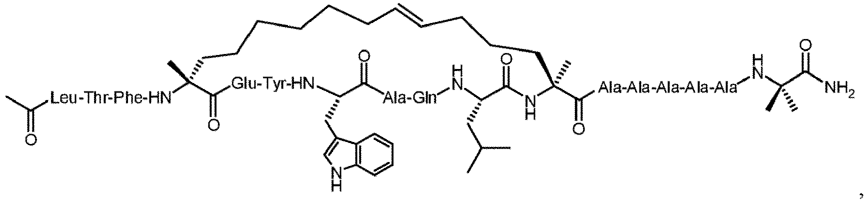 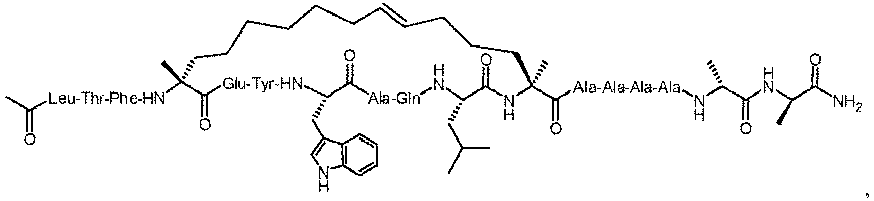 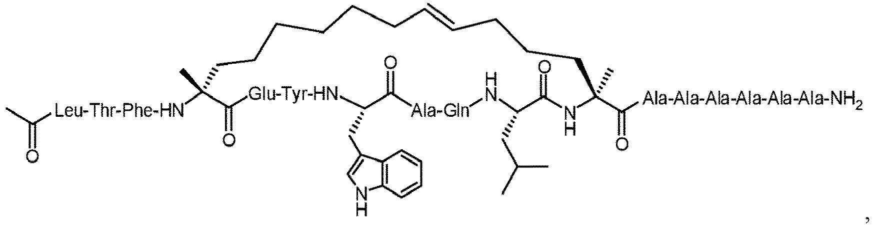 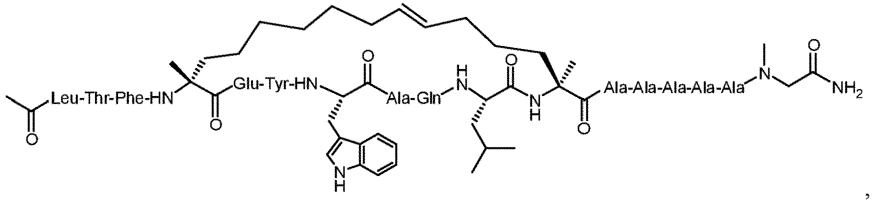 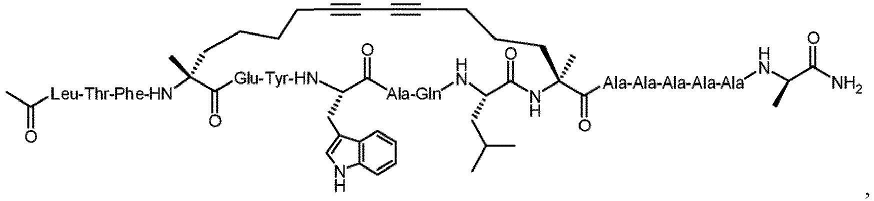 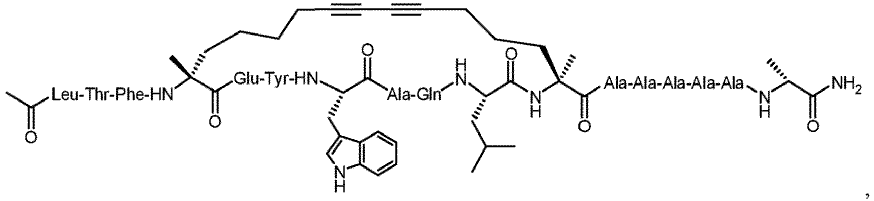 und

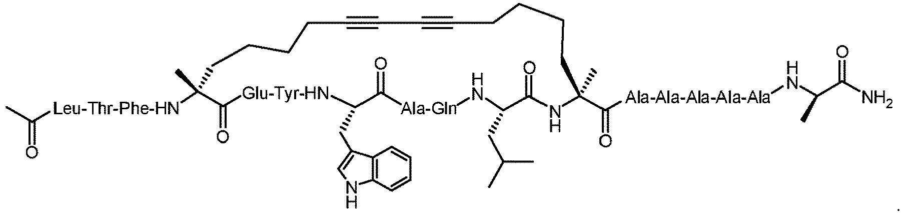

9. Verbindungen zur Verwendung nach einem der Ansprüche 1-8, wobei der Krebs AML ist.

10. Verbindungen zur Verwendung nach einem der Ansprüche 1-8, wobei der Krebs Folgendes ist:
(i) myelodysplastisches Syndrom; oder
(ii) periphere T-Zell-Lymphome; oder
(iii) Brustkrebs.

11. Verbindungen zur Verwendung nach einem der Ansprüche 1-4 und 6-11, wobei der mindestens eine zusätzliche pharmazeutische Wirkstoff Cytarabin ist.

12. Verbindungen zur Verwendung nach einem der Ansprüche 1-11, wobei eine Kombination aus dem peptidomimetischen Makrozyklus oder dem pharmazeutisch annehmbaren Salz davon und dem mindestens einen zusätzlichen pharmazeutischen Wirkstoff synergistisch ist.

## Revendications

1. Macrocycle peptidomimétique ou sel pharmaceutiquement acceptable de celui-ci et au moins un agent pharmaceutiquement actif supplémentaire pour une utilisation dans le traitement du cancer chez un sujet qui le nécessite, dans lequel le macrocycle peptidomimétique ou le sel pharmaceutiquement acceptable de celui-ci antagonise l'activité de p53 et/ou MDM2 et/ou MDMX, et dans lequel le macrocycle peptidomimétique comprend une séquence d'acides aminés qui est identique à au moins 60 % à une séquence d'acides aminés sur l'un quelconque parmi le tableau 1, le tableau 1a, le tableau 1b, et le tableau 1c, et dans lequel le macrocycle peptidomimétique est de formule : 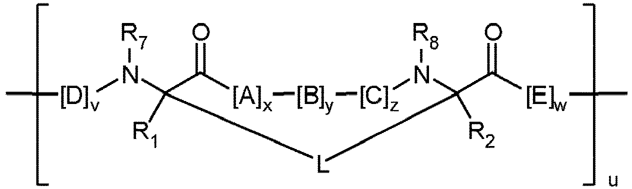 où :
chaque A, C, D, et E est indépendamment un acide aminé ;
chaque B est indépendamment un acide aminé, 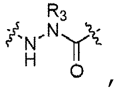 [-NH-L₃-CO-], [-NH-L₃-SO₂-], ou [-NH-L₃-] ;
chaque R₁ et R₂ est indépendamment hydrogène, alkyle, alcényle, alcynyle, arylalkyle, cycloalkyle, cycloalkylalkyle, hétéroalkyle, ou hétérocycloalkyle, non substitué ou substitué par halogène- ; ou forme un lieur formant macrocycle L' relié à la position alpha de l'un desdits acides aminés D ou E ;
chaque R₃ est indépendamment hydrogène, alkyle, alcényle, alcynyle, arylalkyle, hétéroalkyle, cycloalkyle, hétérocycloalkyle, cycloalkylalkyle, aryle, ou hétéroalkyle, éventuellement substitué par R₅ ;
chaque L et L' est indépendamment un lieur formant macrocycle de formule -L₁-L₂- ;
chaque L₁, L₂, et L₃ est indépendamment alkylène, alcénylène, alcynylène, hétéroalkylène, cycloalkylène, hétérocycloalkylène, arylène, hétéroarylène, ou [-R₄-K-R₄-]ₙ, chacun étant éventuellement substitué par R₅ ;
chaque R₄ est indépendamment alkylène, alcénylène, alcynylène, hétéroalkylène, cycloalkylène, hétérocycloalkylène, arylène, ou hétéroarylène ;
chaque K est indépendamment O, S, SO, SO₂, CO, CO₂, ou CONR₃ ;
chaque R₅ est indépendamment halogène, alkyle, -OR₆, - N(R₆)₂, -SR₆, -SOR₆, -SO₂R₆, -CO₂R₆, une fraction fluorescente, un radioisotope ou un agent thérapeutique ;
chaque R₆ est indépendamment hydrogène, alkyle, alcényle, alcynyle, arylalkyle, cycloalkylalkyle, hétérocycloalkyle, une fraction fluorescente, un radioisotope ou un agent thérapeutique ;
chaque R₇ est indépendamment hydrogène, alkyle, alcényle, alcynyle, arylalkyle, cycloalkyle, hétéroalkyle, cycloalkylalkyle, hétérocycloalkyle, aryle, ou hétéroaryle, éventuellement substitué par R₅, ou une partie d'une structure cyclique avec un résidu D ;
chaque R₈ est indépendamment hydrogène, alkyle, alcényle, alcynyle, arylalkyle, cycloalkyle, hétéroalkyle, cycloalkylalkyle, hétérocycloalkyle, aryle, ou hétéroaryle, éventuellement substitué par R₅, ou une partie d'une structure cyclique avec un résidu E ;
chaque v est indépendamment un entier de 1 à 1000 ;
chaque w est indépendamment un entier de 3 à 1000 ;
u est un entier de 1 à 10 ;
chaque x, y et z est indépendamment un entier de 0 à 10 ; et
chaque n est indépendamment un entier de 1 à 5 ;
dans lequel l'au moins un agent pharmaceutiquement actif supplémentaire est le fulvestrant, l'évérolimus, la romidepsine, le palbociclib, le tramétinib, le rituximab, le dabrafénib, le vémurafénib, l'azacitidine, la décitabine, la midostaurine, la vincristine, le cyclophosphamide ou la cytarabine.

2. Composés pour une utilisation selon la revendication 1, dans lesquels le macrocycle peptidomimétique ou le sel pharmaceutiquement acceptable de celui-ci est de formule : 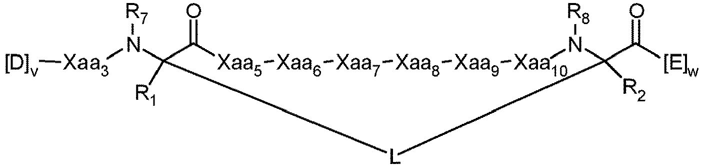 où :
chacun de Xaa₃, Xaa₅, Xaa₆, Xaa₇, Xaa₈, Xaa₉, et Xaa₁₀ est individuellement un acide aminé, dans lesquels au moins trois de Xaa₃, Xaa₅, Xaa₆, Xaa₇, Xaa₈, Xaa₉, et Xaa₁₀ sont le même acide aminé que l'acide aminé à la position correspondante de la séquence Phe₃-X₄-His₅-Tyr₆-Trp₇-Ala₈-Gln₉-Leu₁₀-X₁₁-Ser₁₂ ou Phe₃-X₄-Glu₅-Tyr₆-Trp₇-Ala₈-Gln₉-Leu₁₀/Cba₁₀-X₁₁-Ala₁₂ dans lesquels chaque X₄ et X₁₁ est indépendamment un acide aminé ;
chaque D et E est indépendamment un acide aminé ;
R₁ et R₂ sont indépendamment -H, alkyle, alcényle, alcynyle, arylalkyle, cycloalkyle, cycloalkylalkyle, hétéroalkyle, ou hétérocycloalkyle, non substitué ou substitué par halogène- ; ou au moins l'un de R₁ et R₂ forme un lieur formant macrocycle L' relié à la position alpha de l'un desdits acides aminés D ou E ;
chaque L et L' est indépendamment un lieur formant macrocycle de formule -L₁-L₂- ;
chaque L₁ et L₂ est indépendamment alkylène, alcénylène, alcynylène, hétéroalkylène, cycloalkylène, hétérocycloalkylène, arylène, hétéroarylène, ou [-R₄-K-R₄-]ₙ, chacun étant éventuellement substitué par R₅ ;
chaque R₄ est indépendamment alkylène, alcénylène, alcynylène, hétéroalkylène, cycloalkylène, hétérocycloalkylène, arylène, ou hétéroarylène ;
chaque K est indépendamment O, S, SO, SO₂, CO, CO₂, ou CONR₃ ;
chaque R₅ est indépendamment halogène, alkyle, -OR₆, - N(R₆)₂, -SR₆, -SOR₆, -SO₂R₆, -CO₂R₆, une fraction fluorescente, un radioisotope ou un agent thérapeutique ;
chaque R₆ est indépendamment -H, alkyle, alcényle, alcynyle, arylalkyle, cycloalkylalkyle, hétérocycloalkyle, une fraction fluorescente, un radioisotope ou un agent thérapeutique ;
R₇ est -H, alkyle, alcényle, alcynyle, arylalkyle, cycloalkyle, hétéroalkyle, cycloalkylalkyle, hétérocycloalkyle, aryle, ou hétéroaryle, éventuellement substitué par R₅, ou une partie d'une structure cyclique avec un résidu D ;
R₈ est -H, alkyle, alcényle, alcynyle, arylalkyle, cycloalkyle, hétéroalkyle, cycloalkylalkyle, hétérocycloalkyle, aryle, ou hétéroaryle, éventuellement substitué par R₅, ou une partie d'une structure cyclique avec un résidu E ;
v est un entier de 1 à 1000 ;
w est un entier de 3 à 1000 ; et
n est un entier de 1 à 5.

3. Composés pour une utilisation selon la revendication 1 ou 2, dans lesquels chaque E est indépendamment un acide aminé choisi parmi Ala (alanine), D-Ala (D-alanine), Aib (acide α-aminoisobutyrique), Sar (N-méthyl glycine), et Ser (sérine).

4. Composés pour une utilisation selon la revendication 1 ou 2, dans lesquels :
v est un entier de 1 à 10 ;
w est un entier de 3 à 10 ;
chacun des deux premiers acides aminés représentés par E comprend une chaîne latérale non chargée ou une chaîne latérale chargée négativement ;
L₁ et L₂ sont indépendamment alkylène ou alcénylène ; et
R₁ et R₂ sont indépendamment H ou alkyle.

5. Composés pour une utilisation selon l'une quelconque des revendications 1 à 4, dans lesquels l'au moins un agent pharmaceutiquement actif supplémentaire est le palbociclib.

6. Composés pour une utilisation selon l'une quelconque des revendications 1 à 5, dans lesquels le cancer est choisi dans le groupe constitué par le cancer de la tête et du cou, le mélanome, le cancer du poumon, le cancer du sein, le cancer du côlon, le cancer de l'ovaire, le NSCLC (cancer du poumon non à petites cellules), le cancer de l'estomac, le cancer de la prostate, la leucémie, le lymphome, le mésothéliome, le cancer du rein, le lymphome non hodgkinien (NHL), et le gliome.

7. Composés pour une utilisation selon l'une quelconque des revendications 1 à 5, dans lesquels le cancer comprend une cellule qui surexprime PD-L1, PD-1, miR-34, ou l'une quelconque de leurs combinaisons.

8. Composés pour une utilisation selon l'une quelconque des revendications 1 et 2 et 5 à 7, dans lesquels le macrocycle peptidomimétique est choisi dans le groupe constitué par : 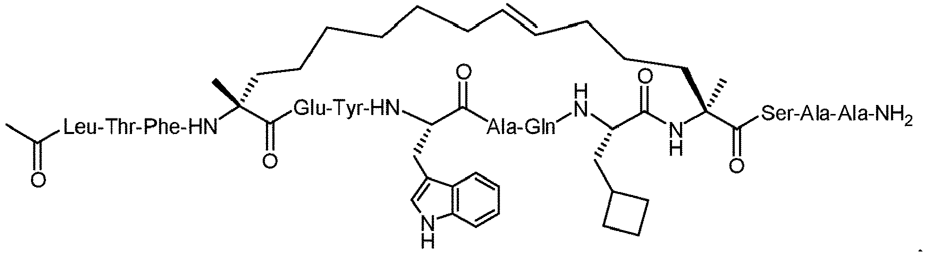 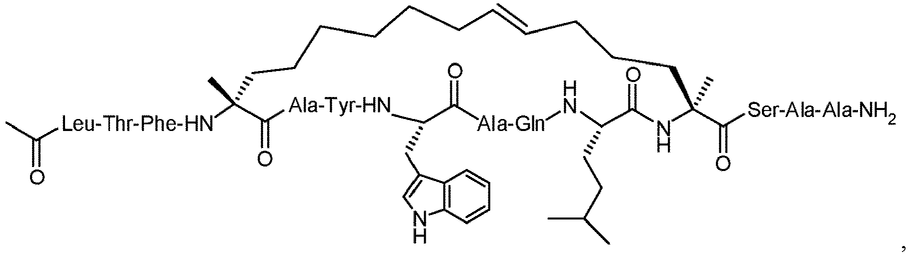 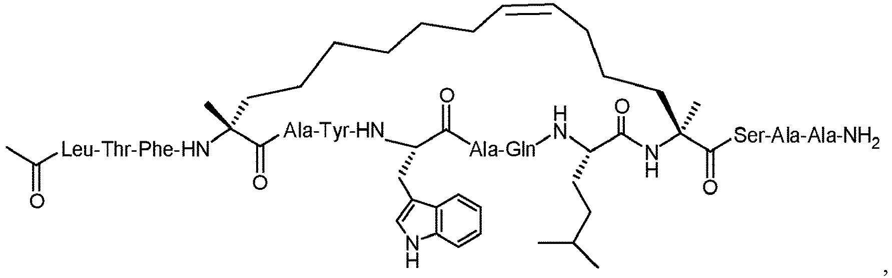 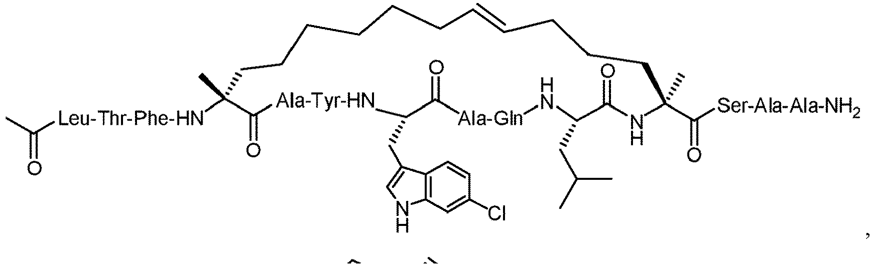 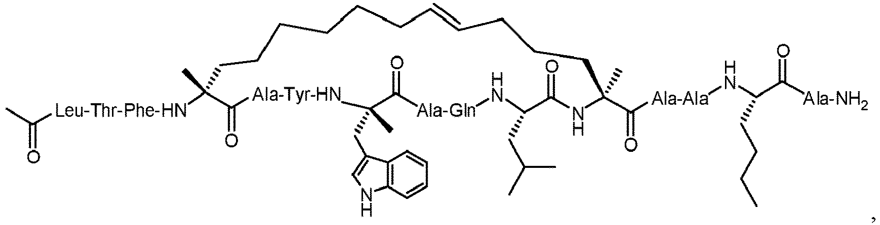 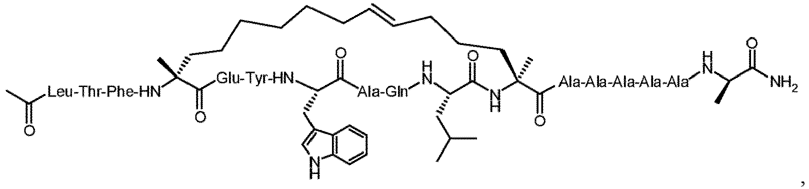 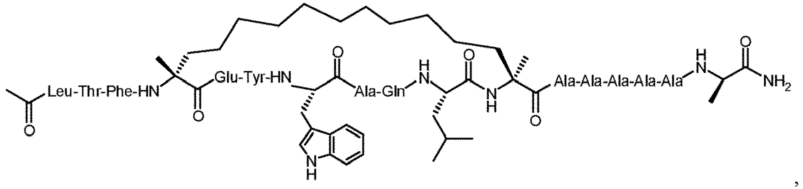 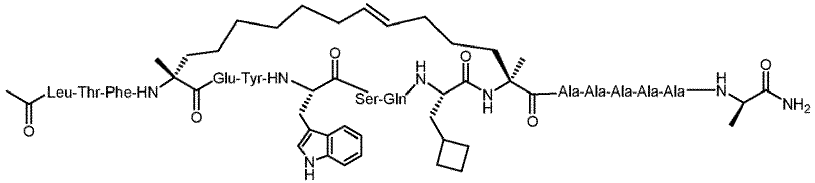 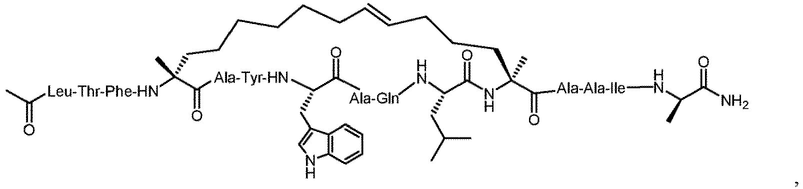 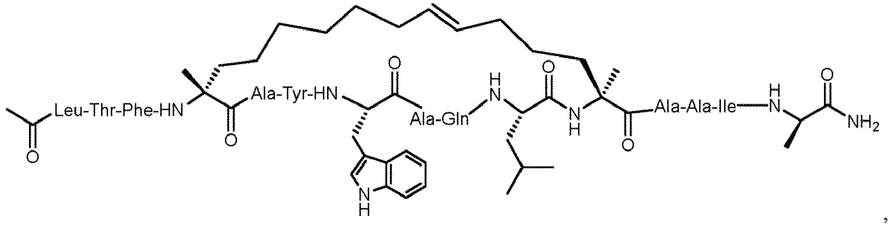 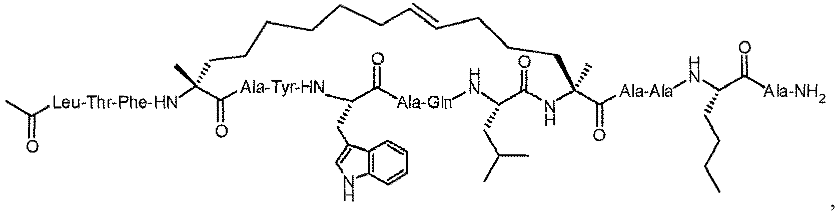 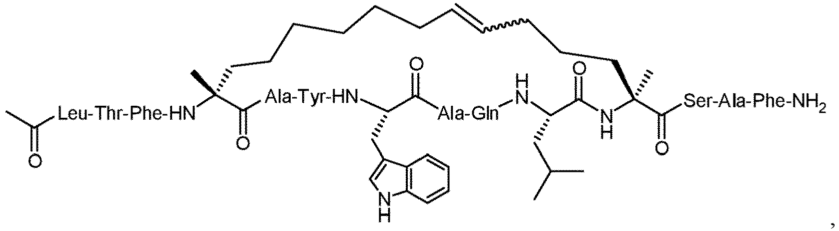 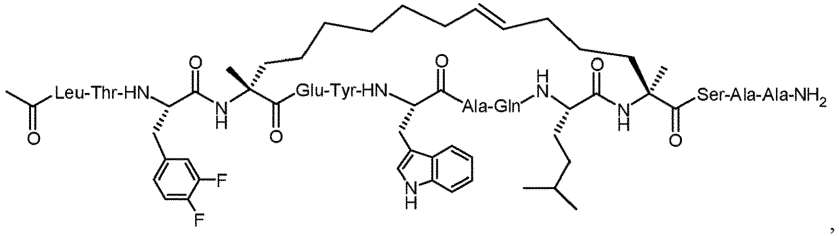 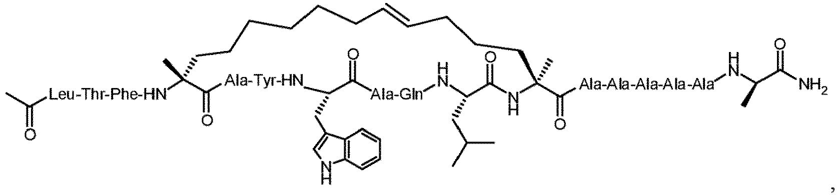 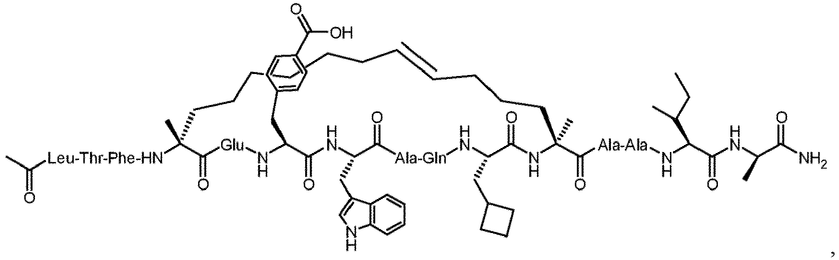 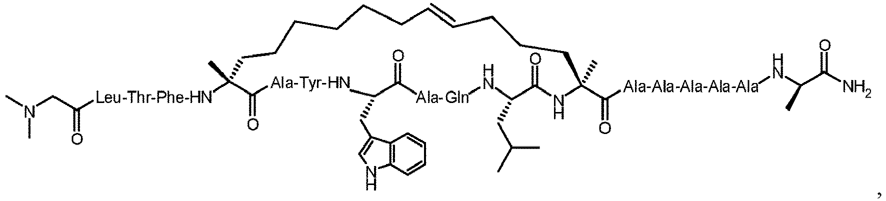 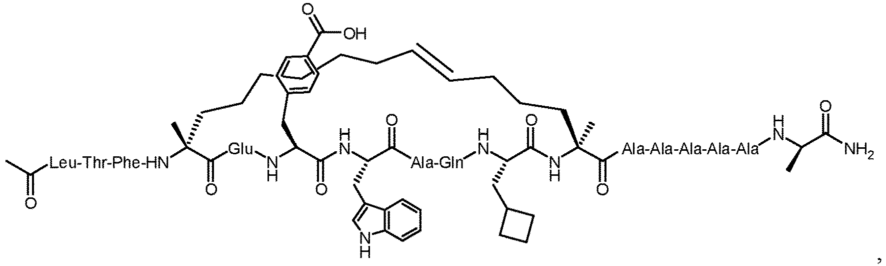 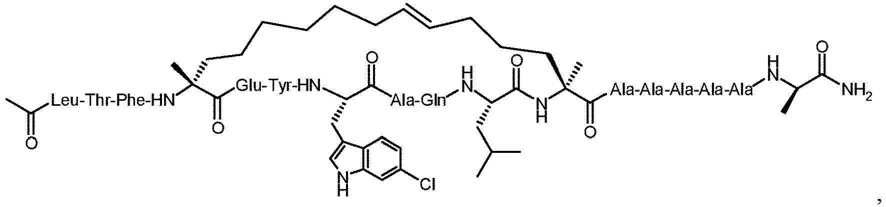 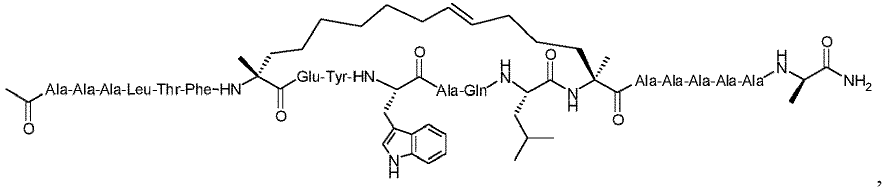 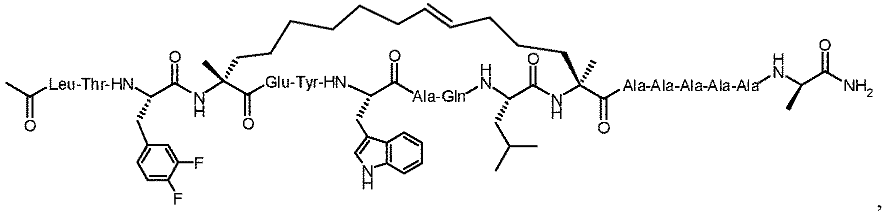 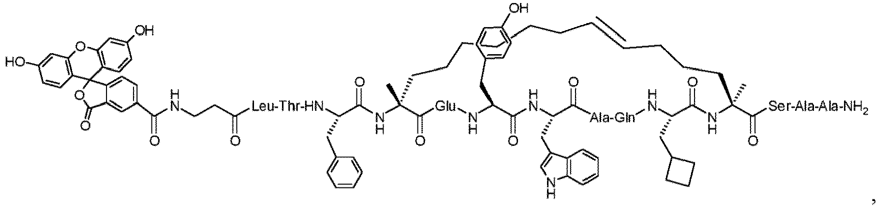 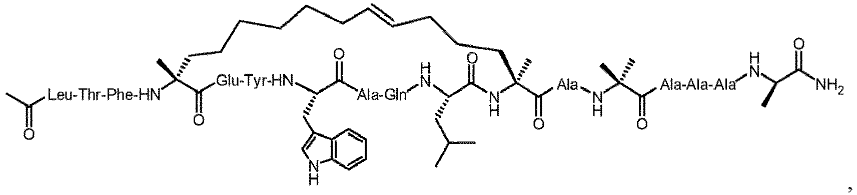 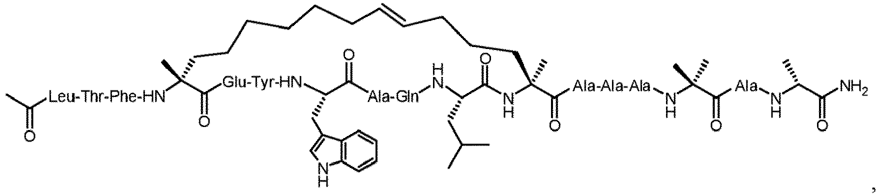 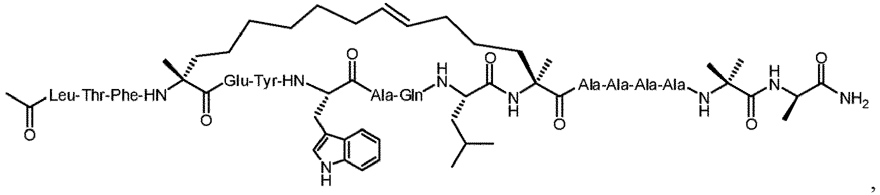 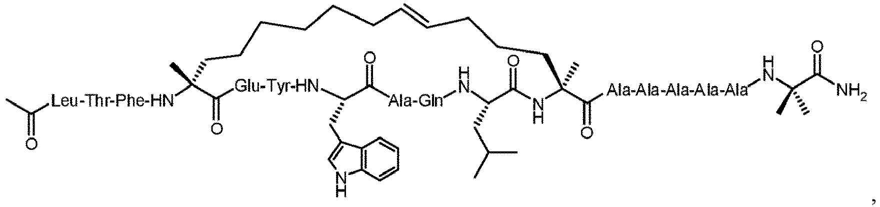 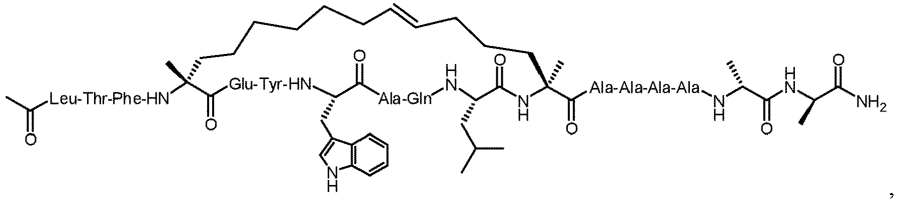 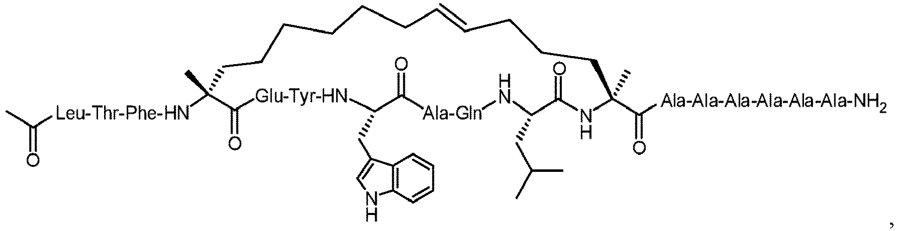 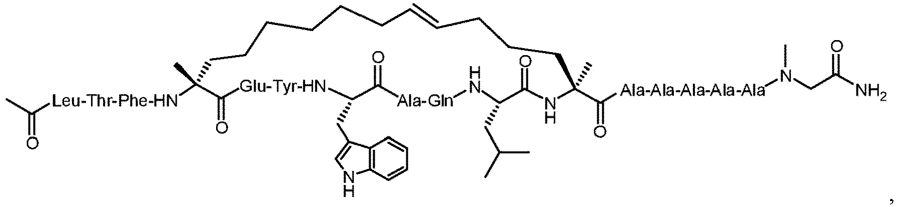 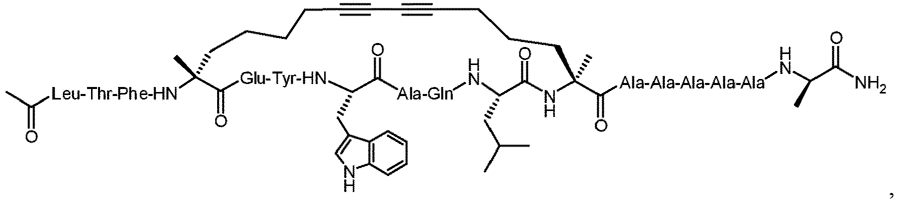 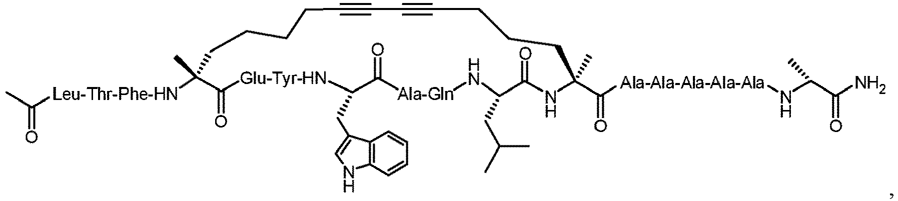 et

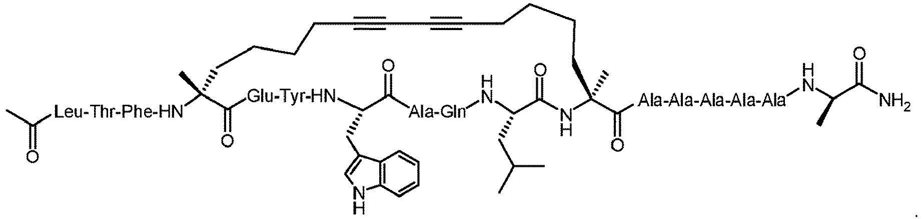

9. Composés pour une utilisation selon l'une quelconque des revendications 1 à 8, dans lesquels le cancer est la LMA.

10. Composés pour une utilisation selon l'une quelconque des revendications 1 à 8, dans lesquels le cancer est :
(i) le syndrome myélodysplasique ; ou
(ii) le lymphome périphérique à cellules T ; ou
(ii) le cancer du sein.

11. Composés pour une utilisation selon l'une quelconque des revendications 1 à 4, et 6 à 11, dans lesquels l'au moins un agent pharmaceutiquement actif supplémentaire est la cytarabine.

12. Composés pour une utilisation selon l'une quelconque des revendications 1 à 11, dans lesquels la combinaison du macrocycle peptidomimétique ou du sel pharmaceutiquement acceptable de celui-ci et de l'au moins un agent pharmaceutiquement actif supplémentaire est synergique.
